# EUROPEAN PATENT APPLICATION

(11) **EP 1 764 367 A1**
(43) Date of publication of application: **21.03.2007**
(21) Application number: 05728563.7
(22) Date of filing: 11.04.2005
(51) Int. Cl.: C07D 495/04, A61K 31/4365, A61K 31/4545, A61K 31/4725, A61K 31/496, A61K 31/5377, A61K 31/541, A61K 31/55, A61K 31/551, A61K 31/553, A61K 31/554, A61P 19/02, A61P 19/08, A61P 19/10, A61P 29/00

(54) **THIENOPYRIDINE DERIVATIVES**

(30) Priority: 12.04.2004 JP 2004116366
(71) Applicant: Sankyo Company, Limited, Tokyo 103-8426 (JP)
(72) Inventor: OIZUMI, Kiyoshi, c/o Sankyo Company Limited, Tokyo 140-8710 (JP); NAITO, Satoru, c/o Sankyo Company Limited, Tokyo 140-8710 (JP); NAKAO, Akira, c/o Sankyo Company Limited, Tokyo 140-8710 (JP); SHINOZUKA, Tsuyoshi, c/o Sankyo Company Limited, Tokyo, 140-8710 (JP); MATSUI, Satoshi, c/o Sankyo Company Limited, Tokyo 140-8710 (JP); SHIMADA, Kousei, c/o Sankyo Company Limited, Tokyo 140-8710 (JP)
(74) Representative: Gibson, Christian John Robert
(86) International application number: PCT/JP2005/007025
(87) International publication number: WO 2005/100365

(57) **Abstract**

The present invention provides a compound promoting osteogenesis. The present invention provides a compound having the following general formula (I) 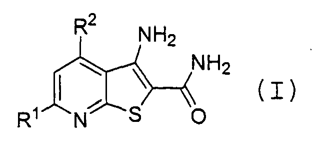 wherein R¹ is H or alkyl,
R² is R^{a}S-, R^{a}O-, R^{a}NH-, R^{a}(R^{b})N- or cyclic amino, and
R^{a} and R^{b} are alkyl which may be substituted, cycloalkyl which may be substituted, or the like, or a pharmacologically acceptable salt thereof.

## Description

### Technical Field

The present invention relates to a compound promoting osteogenesis.

### Background Art

It is known that thienopyridine derivatives have IκB kinase complex inhibitory effect (see Patent Document 1). In addition, 3-amino-4-(dimethylamino)thieno[2,3-b]pyridine-2-carboxamide and 3-amino-4-anilinothieno[2,3-b]pyridine-2-carboxamide are known compounds (see Non-Patent Document 1). However, the influence that these compounds give to bone has not been reported.

### [Patent Document 1]

WO03/103661

### [Non-Patent Document 1]

Pharm. Chem. J. (Engl. Transl.), 26, 870-874 (1992)

### Disclosure of the Invention

### Problems to be Solved by the invention

The present inventors have conducted intensive studies on compounds that promote osteogenesis, and consequently have found that thienopyridine derivatives have excellent pharmacological effect and thus completed the present invention. Means for Solving the Problems

The present invention relates to
(1) a compound having the following general formula (I) 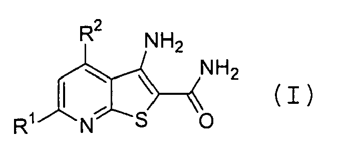 [wherein
   R¹ represents a hydrogen atom, the cyclopropyl group or a C₁-C₆ alkyl group,
   R² represents R^{a}S-, R^{a}O-, R^{a}NH-, R^{a}(R^{b})N- or a group 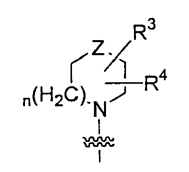 wherein R^{a} and R^{b} are the same or different and independently represent a C₁-C₆ alkyl group which may be substituted with one or more groups selected from Substituent Group α and Substituent Group γ; a C₃-C₈ cycloalkyl group which may be substituted with one or more groups selected from Substituent Group α, Substituent Group β and Substituent Group γ; a 5- to 7-membered heterocyclyl group which may be substituted with one or more groups from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms,
   R³ and R⁴ are the same or different and independently represent a hydrogen atom; a group selected from Substituent Group α, Substituent Group β and Substituent Group γ; a C₁-C₆ alkyl group substituted with one or more groups selected from Substituent Group γ; or a C₁-C₆ alkoxy group substituted with one or more groups selected from Substituent Group γ,
      or when R³ and R⁴ are bonded to adjacent carbon atoms, R³ and R⁴ together with the carbon atoms to which they are bonded may form a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and
      Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms,
   Z represents a single bond; a double bond; an oxygen atom; a sulfur atom; sulfinyl; sulfonyl; or a group having the formula R⁵N<;
   R⁵ represents a hydrogen atom; a C₁-C₆ alkyl group which may be substituted with one or more groups selected from Substituent Group α and Substituent Group γ; a C₂-C₆ alkenyl group which may be substituted with one or more groups selected from Substituent Group α and Substituent Group γ; a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and
      Substituent Group γ; a 5- to 7-membered heteroaryl group which may be substituted with one or more groups selected from Substituent Group α, Substituent Group β and
      Substituent Group γ and which contains 1 to 3 sulfur, oxygen and/or nitrogen atoms; a formyl group; a C₂-C₇ alkylcarbonyl group which may be substituted with one or more groups selected from Substituent Group α and Substituent Group γ; a 5- to 7-membered heterocyclylcarbonyl group which may be substituted with one or more groups selected from Substituent Group α, Substituent Group β and Substituent Group γ and which contains 1 to 3 sulfur, oxygen and/or nitrogen atoms; a C₇-C₁₁ arylcarbonyl group which may be substituted with one or more groups selected from Substituent Group α, Substituent Group β and Substituent Group γ; a 5- to 7-membered heteroarylcarbonyl group which may be substituted with one or more groups selected from Substituent Group α, Substituent Group β and Substituent Group γ and which contains 1 to 3 sulfur, oxygen and/or nitrogen atoms; a C₁-C₆ alkylsulfonyl group which may be substituted with one or more groups selected from Substituent Group α and Substituent Group γ; a C₆-C₁₀ arylsulfonyl group which may be substituted with one or more groups selected from Substituent Group α, Substituent Group β and Substituent Group γ; a 5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms; a C₂-C₇ alkoxycarbonyl group which may be substituted with one or more groups selected from Substituent Group α and
      Substituent Group γ; a C₇-C₁₁ aryloxycarbonyl group which may be substituted with one or more groups selected from Substituent Group α, Substituent Group β and
      Substituent Group γ; or one or more groups having the formula R^{c}(R^{d})N-CO-(wherein R^{c} and R^{d} are the same or different and independently represent a hydrogen atom or a C₁-C₆ alkyl group which may be substituted with one or more groups selected from Substituent Group α and Substituent Group γ), and
      n represents an integer of 1 to 4,
      Substituent Group α represents the group consisting of a halogen atom; a nitro group; a cyano group; a hydroxy group; a group having the formula R⁶-CO-, the formula R^{e}(R^{f})N-, the formula R^{e}(R^{f})N-CO- or the formula R^{e}(R^{f})N-SO₂- (wherein
   R⁶ represents a hydrogen atom, a C₁-C₆ alkyl group, a C₁-C₆ halogenated alkyl group, a C₃-C₈ cycloalkyl group, a hydroxy group, a C₁-C₆ alkoxy group, a C₆-C₁₀ aryl group or a C₆-C₁₀ aryloxy group and R^{e} and R^{f} are the same or different and independently represent a hydrogen atom;-a C₁-C₆ alkyl group; a C₁-C₆ alkoxy group; a C₆-C₁₀ aryl group; a 5- to 7-membered heteroaryl group which contains 1 to 3 sulfur, oxygen and/or nitrogen atoms; a formyl group; a C₂-C₇ alkylcarbonyl group; a C₂-C₇ alkoxycarbonyl group; a C₇-C₁₁ arylcarbonyl group; a 5- to 7-membered heteroarylcarbonyl group which contains 1 to 3 sulfur, oxygen and/or nitrogen atoms; a C₁-C₆ alkylsulfonyl group; a C₆-C₁₀ arylsulfonyl group; or a 5- to 7-membered heteroarylsulfonyl group which contains 1 to 3 sulfur, oxygen and/or nitrogen atoms, or alternatively R^{e} and R^{f} together with the nitrogen atom to which they are bonded form a 4- to 7-membered heterocylyl group which contains 1 to 3 sulfur, oxygen and/or nitrogen atoms (wherein the heterocylyl group may have 1 or 2 substituent groups selected from a hydroxy group and a methyl group)); a hydroxyimino group; a C₁-C₆ alkoxyimino group; a C₁-C₆ alkoxy group; a C₃-C₈ cycloalkyloxy group; a C₁-C₆ halogenated alkoxy group; a C₁-C₆ alkylthio group; a C₁-C₆ alkylsulfinyl group; and a C₁-C₆ alkylsulfonyl group,
      Substituent Group β represents the group consisting of a C₁-C₆ alkyl group which may be substituted with one or more groups selected from Substituent Group α; and a C₁-C₆ alkyl group substituted with a 5- to 7-membered heterocyclyl group which may be substituted with one or more groups selected from Substituent Group α and a C₁-C₆ alkyl group and which contains 1 to 3 sulfur, oxygen and/or nitrogen atoms, and
      Substituent Group γ represents the group consisting of a C₁-C₆ alkoxy group substituted with one or more groups selected from Substituent Group α; a C₁-C₆ alkylthio group substituted with one or more groups selected from Substituent Group α; a C₃-C₈ cycloalkyl group which may be substituted with one or more groups selected from Substituent Group α and Substituent Group β; a 5- to 7-membered heterocyclyl group which may be substituted with one or more groups selected from Substituent Group α and Substituent Group β and which contains 1 to 3 sulfur, oxygen and/or nitrogen atoms; a C₆-C₁₀ aryl group which may be substituted with one or more groups selected from Substituent Group α and Substituent Group β; a 5-to 7-membered heteroaryl group which may be substituted with one or more groups selected from Substituent Group α and Substituent Group β and which contains 1 to 3 sulfur, oxygen and/or nitrogen atoms; a C₃-C₈ cycloalkyloxy group which may be substituted with one or more groups selected from Substituent Group α and Substituent Group β; a 5- to 7-membered heterocyclyloxy group which may be substituted with one or more groups selected from Substituent Group α and Substituent Group β and which contains 1 to 3 sulfur, oxygen and/or nitrogen atoms; a C₆-C₁₀ aryloxy group which may be substituted with one or more groups selected from Substituent Group α and Substituent Group β; a 5- to 7-membered heteroaryloxy group which may be substituted with one or more groups selected from Substituent Group α and Substituent Group β and which contains 1 to 3 sulfur, oxygen and/or nitrogen atoms; and a C₆-C₁₀ aryl - C₁-C₆ alkoxy group in which the aryl moiety may be substituted with one or more groups selected from Substituent Group α and Substituent Group β] or a pharmacologically acceptable salt thereof.

Preferred examples of the above compound are
(2) compounds in which R¹ is a hydrogen atom, a cyclopropyl group or a C₁-C₄ alkyl group,
(3) compounds in which R¹ is a hydrogen atom, methyl, ethyl, propyl or cyclopropyl,
(4) compounds in which R¹ is a hydrogen atom or methyl,
(5) compounds in which R² is a group R^{a}(R^{b})N-, and R^{a} and R^{b} are the same or different and independently represent a C₁-C₆ alkyl group which may be substituted with one or more groups selected from Substituent Group α and Substituent Group γ,
(6) compounds in which R^{a} is a C₁-C₆ alkyl group which may be substituted with one group selected from Substituent Group α and Substituent Group γ, R^{b} is a C₁-C₆ alkyl group, and Substituent Group α is the group consisting of a C₁-C₆ alkoxy group, and Substituent Group γ is the group consisting of a C₁-C₆ alkoxy group substituted with one or more groups selected from Substituent Group α; a C₆-C₁₀ aryloxy group which may be substituted with one or more groups selected from Substituent Group α and Substituent Group β; and a 5- to 7-membered heteroaryloxy group which may be substituted with one or more groups selected from Substituent Group α and Substituent Group β and which contains 1 to 3 sulfur, oxygen and/or nitrogen atoms,
(7) compounds in which R² is a group 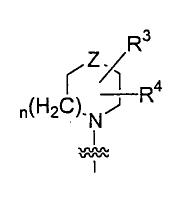 wherein R⁴ is a hydrogen atom or together with R³ forms a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms,
(8) compounds in which R² is a group 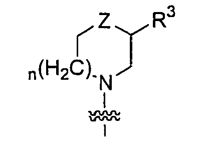 wherein Z represents a single bond, an oxygen atom, a sulfur atom or a group having the formula R⁵N<,
   R⁵ represents a C₆-C₁₀ aryl group which may be substituted with one or more groups selected from Substituent Group α, Substituent Group β and Substituent Group γ; a 5- to 7-membered heteroaryl group which may be substituted with one or more groups selected from Substituent Group α, Substituent Group β and Substituent Group γ and which contains 1 to 3 sulfur, oxygen and/or nitrogen atoms; a formyl group; a C₂-C₇ alkylcarbonyl group which may be substituted with one or more groups selected from Substituent Group α and Substituent Group γ; a C₁-C₆ alkylsulfonyl group which may be substituted with one or more groups selected from Substituent Group α and Substituent Group γ; a C₆-C₁₀ arylsulfonyl group which may be substituted with one or more groups selected from Substituent Group α, Substituent Group β and Substituent Group γ; a 5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms; a C₂-C₇ alkoxycarbonyl group which may be substituted with one or more groups selected from Substituent Group α and
   Substituent Group γ; or one or more groups having the formula R^{c}(R^{d})N-CO-, and n is an integer of 1 to 3,
(9) compounds in which R³ is a C₁-C₆ alkoxy group; a C₁-C₆ alkyl group which may be substituted with one or more groups selected from Substituent Group α; a C₁-C₆ alkoxy group substituted with one or more groups selected from Substituent Group α; a C₆-C₁₀ aryloxy group which may be substituted with one or more groups selected from Substituent Group α, Substituent Group β and Substituent Group γ; a C₁-C₆ alkyl group substituted with one or more groups selected from Substituent Group γ; or a C₁-C₆ alkoxy group substituted with one or more groups selected from Substituent Group γ,
   Z is a single bond, and n is 2,
(10) compounds in which R³ is a hydrogen atom, Z is a sulfur atom, and n is 1, and
(11) compounds in which R³ is a hydrogen atom, Z is a group having the formula R⁵N<, R⁵ represents a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and
   Substituent Group γ and which contains 1 to 3 sulfur, oxygen and/or nitrogen atoms:
   n is 2
   and pharmacologically acceptable salts thereof, and particularly preferred compounds are
(12) any of the compounds selected from the following:
   · 3-amino-4-[(3S)-3-(methoxymethyl)piperidin-1-yl]thieno[2,3-b]pyridine-2-carboxamide,
   · 3-amino-4-[(3S)-3-(methoxymethyl)piperidin-1-yl]-6-methylthieno[2,3-b]pyridine-2-carboxamide,
   · 3-amino-4-{3-[3-(2-hydroxyethoxy)propyl]piperidin-1-yl}thieno[2,3-b]pyridine-2-carboxamide,
   · 3-amino-4- {(3S)-[(2-methoxyethoxy)methyl]piperidin-1-yl} thieno[2,3-b]pyridine-2-carboxamide,
   · 3-amino-4-{(3S)-3-[(3-methoxypropoxy)methyl]piperidin-1-yl}thieno[2,3-b]pyridine-2-carboxamide,
   · 3-amino-4-(3-{[2-(dimethylamino)-2-oxoethoxy]methyl}piperidin-1-yl)thieno[2,3-b]pyridine-2-carboxamide,
   · 3-amino-4-(3-{3-[2-(dimethylamino)-2-oxoethoxy]propyl}piperidin-1-yl)thieno[2,3-b]pyridine-2-carboxamide,
   · 4-[4-(4-acetylphenyl)-1,4-diazepan-1-yl]-3-aminothieno[2,3-b]pyridine-2-carboxamide,
   · 3-amino-4-[4-(4-propionylphenyl)-1,4-diazepan-1-yl]thieno[2,3-b]pyridine-2-carboxamide,
   · 3-amino-4-{4-[4-(dimethylamino)phenyl]-1,4-diazepan-1-yl}thieno[2,3-b]pyridine-2-carboxamide,
   · 3-amino-4-(4-{4-[(dimethylamino)carbonyl]phenyl}-1,4-diazepan-1-yl)thieno[2,3-b]pyridine-2-carboxamide,
   · 4-[4-(5-acetylpyridin-2-yl)-1,4-diazepan-1-yl]-3-aminothieno[2,3-b]pyridine-2-carboxamide,
   · 3-amino-4-(4-{4-[(dimethylamino)carbonyl]phenyl}-1,4-diazepan-1-yl)-6-methylthieno[2,3-b]pyridine-2-carboxamide,
   · 3-amino-4-{4-[4-(2-methoxyethyl)phenyl]-1,4-diazepan-1-yl}thieno[2,3-b]pyridine-2-carboxamide,
   · 3-amino-4-(4-{4-[2-(dimethylamino)-2-oxoethyl]phenyl}-1,4-diazepan-1-yl)thieno[2,3-b]pyridine-2-carboxamide,
   · 3-amino-4-(4-{4-[3-(dimethylamino)-3-oxopropyl]phenyl}-1,4-diazepan-1-yl)thieno[2,3-b]pyridine-2-carboxamide,
   · 3-amino-4-{4-[4-(azetidin-1-ylcarbonyl)phenyl]-1,4-diazepan-1-yl}thieno [2,3-b]pyridine-2-carboxamide,
   · 3-amino-4-{4-[4-(morpholin-4-ylcarbonyl)phenyl]-1,4-diazepan-1-yl}thieno[2,3-b]pyridine-2-carboxamide,
   · 3-amino-4-(4-{4-[2-(dimethylamino)ethyl]phenyl}-1,4-diazepan-1-yl)thieno[2,3-b]pyridine-2-carboxamide,
   · 3-amino-4-{4-[4-(2-hydroxyethyl)phenyl]-1,4-diazepan-1-yl}thieno[2,3-b]pyridine-2-carboxamide,
   · 3-amino-4-{4-[3-(2-hydroxyethyl)phenyl]-1,4-diazepan-1-yl}thieno[2,3-b]pyridine-2-carboxamide,
   · 3-amino-4-{4-[4-(3-hydroxypropyl)phenyl]-1,4-diazepan-1-yl}thieno[2,3-b]pyridine-2-carboxamide,
   · 3-amino-4-{4-[4-(1-hydroxyethyl)phenyl]-1,4-diazepan-1-yl}thieno[2,3-b]pyridine-2-carboxamide,
   · 3-amino-4-{4-[4-(2-oxopropyl)phenyl]-1,4-diazepan-1-yl}thieno[2,3-b]pyridine-2-carboxamide,
   · 3-amino-4-{4-[4-(N-hydroxyethaneimidoyl)phenyl]-1,4-diazepan-1-yl}thieno[2,3-b]pyridine-2-carboxamide,
   · 3-amino-4-(4-{4-[(2-methyl-1,3-dioxolan-2-yl)methyl]phenyl}-1,4-diazepan-1-yl)thieno[2,3-b]pyridine-2-carboxamide,
   · 3-amino-4-[4-(2-methyl-1-oxo-2,3-dihydro-1H-isoindol-5-yl)-1,4-diazepan-1-yl]thieno[2,3-b]pyridine-2-carboxamide,
   · 3-amino-4-[4-(1,3-benzoxazol-6-yl)-1,4-diazepan-1-yl]thieno[2,3-b]pyridine-2-carboxamide,
   · 3-amino-4-[4-(4-hydroxyimino-3,4-dihydro-2H-chromen-7-yl)-1,4-diazepan-1-yl]thieno[2,3-b]pyridine-2-carboxamide,
   · 4-[4-(4-acetyl-1,3-thiazol-2-yl)-1,4-diazepan-1-yl]-3-aminothieno[2,3-b]pyridine-2-carboxamide,
   · 4-[4-(5-acetylthiophen-2-yl)-1,4-diazepan-1-yl]-3-aminothieno[2,3-b]pyridine-2-carboxamide, and
   · 3-amino-4-(4-{4-[(dimethylamino)carbonyl]-1,3-thiazol-2-yl}-1,4-diazepan-1-yl)thieno[2,3-b]pyridine-2-carboxamide or pharmacologically acceptable salts thereof.

The present invention further relates to
(13) a pharmaceutical composition which comprises a compound selected from any one of the above (1) to (12) or a pharmacologically acceptable salt thereof as an active ingredient {particularly a pharmaceutical composition for prevention or treatment of osteopathy [for example, osteoporosis (for example, postmenopausal osteoporosis, senile osteoporosis or secondary osteoporosis caused by the use of steroids or immunosuppressants), osteopenia or bone destruction associated with rheumatoid arthritis, Paget's disease of bone, bone fracture or dysostosis due to dwarfism] or osteoarthritis},
(14) use of a compound selected from any one of the above (1) to (12) or a pharmacologically acceptable salt thereof as an active ingredient for preparing a pharmaceutical composition {particularly a pharmaceutical composition for prevention or treatment of osteopathy [for example, osteoporosis (for example, postmenopausal osteoporosis, senile osteoporosis or secondary osteoporosis caused by the use of steroids or immunosuppressants, osteopenia or bone destruction associated with rheumatoid arthritis, Paget's disease of bone, bone fracture or dysostosis due to dwarfism] or osteoarthritis},
(15) a method for promoting osteogenesis, suppressing bone resorption and/or improving bone density comprising administering an effective amount of a compound selected from any one of the above (1) to (12) or a pharmacologically acceptable salt thereof to a mammal (for example, a human, horse, cow or pig, preferably a human) or a bird (preferably a chicken, more preferably a female chicken) {preferably a method for of prevention or treatment of osteopathy [for example, osteoporosis (for example, postmenopausal osteoporosis, senile osteoporosis or secondary osteoporosis caused by the use of steroids or immunosuppressants, osteopenia or bone destruction associated with rheumatoid arthritis, Paget's disease of bone, bone fracture or dysostosis due to dwarfism] or osteoarthritis}.

In the above general formula (I), the "C₁-C₆ alkyl group" in the definitions of R¹, R⁶, R^{e} and R^{f}; the C₁-C₆ alkyl group of "C₁-C₆ alkyl group which maybe substituted with one or more groups selected from Substituent Group α and Substituent Group γ" in the definitions of R^{a}, R^{b}, R⁵, R^{c} and R^{d}; the C₁-C₆ alkyl group of "C₁-C₆ alkyl group substituted with one or more groups selected from Substituent Group γ" in the definitions of R³ and R⁴; the alkyl moiety of "C₂-C₇ alkylcarbonyl group which may be substituted with one or more groups selected from Substituent Group α and Substituent Group γ" and "C₁-C₆ alkylsulfonyl group which may be substituted with one or more groups selected from Substituent Group α and Substituent Group γ" in the definition of R⁵; the alkyl moiety of "C₂-C₇ alkylcarbonyl group" and "C₁-C₆ alkylsulfonyl group" in the definition of R^{e} and R^{f}; the alkyl moiety of "C₁-C₆ alkylthio group", "C₁-C₆ alkylsulfinyl group" and "C₁-C₆ alkylsulfonyl group in the definition of Substituent Group α; the C₁-C₆ alkyl group of "C₁-C₆ alkyl group which may be substituted with one or more groups selected from Substituent Group α" in the definition of Substituent Group β; the C₁-C₆ alkyl moiety of "C₁-C₆ alkyl group substituted with a 5- to 7-membered heterocyclyl group which may be substituted with one or more groups selected from Substituent Group α and a C₁-C₆ alkyl group and which contains 1 to 3 sulfur, oxygen and/or nitrogen atoms" in the definition of Substituent Group β; and the alkyl moiety of "C₁-C₆ alkylthio group substituted with one or more groups selected from Substituent Group α" in the definition of Substituent Group γ can be a linear or branched chain alkyl group such as a methyl, ethyl, propyl, isopropyl, butyl, isobutyl, s-butyl, tert-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group, and preferably it is a C₁-C₄ linear or branched chain alkyl group, more preferably a C₁-C₃ linear or branched chain alkyl group and still more preferably a methyl, ethyl, propyl or isopropyl group.

The "C₃-C₈ cycloalkyl group" in the definition of R⁶; the C₃-C₈ cycloalkyl group of "C₃-C₈ cycloalkyl group which may be substituted with one or more groups selected from Substituent Group α, Substituent Group β and Substituent Group γ" in the definitions of R^{a}, R^{b} and R⁵; the C₃-C₈ cycloalkyl group of "C₃-C₈ cycloalkyl group which may be substituted with one or more groups selected from Substituent Group α, Substituent Group β and Substituent Group γ" which R³ and R⁴ form together with the carbon atoms to which they are bonded; the C₃-C₈ cycloalkyl group of "C₃-C₈ cycloalkyl group which may be substituted with one or more groups selected from Substituent Group α and Substituent Group β" in the definition of Substituent Group γ; the C₃-C₈ cycloalkyl moiety of "C₃-C₈ cycloalkyloxy group" in the definition of Substituent Group α; and the C₃-C₈ cycloalkyl moiety of "C₃-C₈ cycloalkyloxy group which may be substituted with one or more groups selected from Substituent Group α and Substituent Group β" in the definition of Substituent Group γ can be a cyclopropyl, cyclobutyl, cyclopentyl, cyclohexyl, cycloheptyl or cyclooctyl group and preferably it is a C₃-C₇ cycloalkyl group, and more preferably a cyclopropyl, cyclobutyl, cyclopentyl, cyclohexyl or cycloheptyl group.

The "4- to 7-membered heterocyclyl group which contains 1 to 3 sulfur, oxygen and/or nitrogen atoms" which R^{e} and R^{f} form together with the nitrogen atom to which they are bonded can be, for example, azetidinyl, pyrrolidinyl, pyrrolinyl, imidazolidinyl, imidazolinyl, pyrazolidinyl, pyrazolinyl, oxazolidinyl, thiazolidinyl, oxadiazolidinyl, piperidyl, tetrahydropyridyl, dihydropyridyl, piperazinyl, morpholinyl, thiomorpholinyl or homopiperidyl, and preferably it is a 4- to 6-membered heterocyclyl group which contains 1 or 2 sulfur, oxygen and/or nitrogen atoms and particularly preferably it is azetidinyl, pyrrolidinyl, piperidyl, piperazinyl or morpholinyl.

Here, the above mentioned "5- to 7-membered heterocyclyl group which contains 1 to 3 sulfur, oxygen and/or nitrogen atoms" may be condensed with another cyclic group (for example, a phenyl group) and such a group can be, for example, 1,2,3,4-tetrahydroquinolinyl, 1,2,3,4-tetrahydroisoquinolinyl, indolinyl or isoindolinyl.

The 5- to 7-membered heterocyclyl group of "5- to 7-membered heterocyclyl group which may be substituted with one or more groups selected from Substituent Group α, Substituent Group β and Substituent Group γ and which contains 1 to 3 sulfur, oxygen and/or nitrogen atoms" in the definitions of R^{a}, R^{b} and R⁵; the 5- to 7-membered heterocyclyl group of "5- to 7-membered heterocyclyl group which may be substituted with one or more groups selected from Substituent Group α, Substituent Group β and Substituent Group γ and which contains 1 to 3 sulfur, oxygen and/or nitrogen atoms" which R³ and R⁴ form together with the carbon atoms to which they are bonded; the 5- to 7-membered heterocyclyl moiety of "5- to 7-membered heterocyclylcarbonyl group which may be substituted with one or more groups selected from Substituent Group α, Substituent Group β and Substituent Group γ and which contains 1 to 3 sulfur, oxygen and/or nitrogen atoms" in the definition of R⁵; the 5- to 7-membered heterocyclyl moiety of "C₁-C₆ alkyl group substituted with a 5- to 7-membered heterocyclyl group which may be substituted with one or more groups selected from Substituent Group α and a C₁-C₆ alkyl group and which contains 1 to 3 sulfur, oxygen and/or nitrogen atoms" in the definition of Substituent Group β; the 5- to 7-membered heterocyclyl group of "5- to 7-membered heterocyclyl group which may be substituted with one or more groups selected from Substituent Group α and Substituent Group β and which contains 1 to 3 sulfur, oxygen and/or nitrogen atoms" in the definition of Substituent Group γ; and the 5- to 7-membered heterocyclyl moiety of "5- to 7-membered heterocyclyloxy group which may be substituted with one or more groups selected from Substituent Group α and Substituent Group β and which contains 1 to 3 sulfur, oxygen and/or nitrogen atoms" in the definition of Substituent Group γ can be, for example, pyrrolidinyl, pyrrolinyl, imidazolidinyl, imidazolinyl, pyrazolidinyl, pyrazolinyl, oxazolidinyl, dioxolanyl, thiazolidinyl, oxadiazolidinyl, piperidyl, tetrahydropyridyl, dihydropyridyl, 2H-pyranyl, 2-oxo-2H-pyranyl, tetrahydropyranyl, tetrahydrofuranyl, piperazinyl, morpholinyl, dioxanyl, thiomorpholinyl or homopiperidyl, and preferably it is a 5- to 6-membered heterocyclyl group which contains 1 or 2 sulfur, oxygen and/or nitrogen atoms, and particularly preferably it is dioxolanyl, pyrrolidinyl, piperidyl, piperazinyl or morpholinyl.

Here, the above mentioned "5- to 7-membered heterocyclyl group which contains 1 to 3 sulfur, oxygen and/or nitrogen atoms" may be condensed with another cyclic group (for example, a phenyl group) and such a group can be, for example, 1,2,3,4-tetrahydroquinolinyl, 1,2,3,4-tetrahydroisoquinolinyl, chromanyl, indolinyl or isoindolinyl.

The "C₆-C₁₀ aryl group" in the definition of R⁶; the C₆-C₁₀ aryl group of "C₆-C₁₀ aryl group which may be substituted with one or more groups selected from Substituent Group α, Substituent Group β and Substituent Group γ" in the definitions of R^{a}, R^{b} and R⁵; the C₆-C₁₀ aryl group of "C₆-C₁₀ aryl group which may be substituted with one or more groups selected from Substituent Group α, Substituent Group β and Substituent Group γ" which R³ and R⁴ form together with the carbon atoms to which they are bonded; the aryl moiety of "C₇-C₁₁ arylcarbonyl group which may be substituted with one or more groups selected from Substituent Group α, Substituent Group β and Substituent Group γ" in the definition of R⁵, the aryl moiety of "C₆-C₁₀ arylsulfonyl group which may be substituted with one or more groups selected from Substituent Group α, Substituent Group β and Substituent Group γ" and "C₇-C₁₁ aryloxycarbonyl group which may be substituted with one or more groups selected from Substituent Group α, Substituent Group β and Substituent Group γ"; the aryl moiety of "C₆-C₁₀ aryloxy group" in the definition of R⁶; the "C₆-C₁₀ aryl group" in the definition of R^{e} and R^{f}; the aryl moiety of "C₇-C₁₁ arylcarbonyl group" and "C₆-C₁₀ arylsulfonyl group" in the definition of R^{e} and R^{f}; the C₆-C₁₀ aryl group of "C₆-C₁₀ aryl group which may be substituted with one or more groups selected from Substituent Group α and Substituent Group β" in the definition of Substituent Group γ; the aryl moiety of "C₆-C₁₀ aryloxy group which may be substituted with one or more groups selected from Substituent Group α and Substituent Group β" in the definition of Substituent Group γ; and the aryl moiety of "C₆-C₁₀ aryl - C₁-C₆ alkoxy group in which the aryl moiety may be substituted with one or more groups selected from Substituent Group α and Substituent Group β" in the definition of Substituent Group γ can be, for example, phenyl or naphthyl and is preferably phenyl.

Here, the above mentioned "C₆-C₁₀ aryl group" may be condensed with a C₃-C₁₀ cycloalkyl group (preferably a C₅-C₆ cycloalkyl group), "heterocyclyl" mentioned above or "heteroaryl" mentioned below and examples of such a group include 5-indanyl, 5-isoindolinyl, 1-oxo-2-methyl-5-isoindolinyl, 1,3-benzoxazol-5-yl, 1,3-benzoxazol-6-yl, chroman-7-yl, 1,2-benzoisoxazol-6-yl, 1,3-benzodioxazol-5-yl, 2,3-dihydro-1-benzofuran-5-yl, quinolin-6-yl, isoquinolin-6-yl, 1,3-benzothiazol-6-yl and indol-5-yl.

The 5- to 7-membered heteroaryl group of "5- to 7-membered heteroaryl group which may be substituted with one or more groups selected from Substituent Group α, Substituent Group β and Substituent Group γ and which contains 1 to 3 sulfur, oxygen and/or nitrogen atoms" in the definitions of R^{a}, R^{b} and R⁵; the 5- to 7-membered heteroaryl group of "5- to 7-membered heteroaryl group which may be substituted with one or more groups selected from Substituent Group α, Substituent Group β and Substituent Group γ and which contains 1 to 3 sulfur, oxygen and/or nitrogen atoms" which R³ and R⁴ form together with the carbon atoms to which they are bonded; the 5- to 7-membered heteroaryl moiety of "5- to 7-membered heteroarylcarbonyl group which may be substituted with one or more groups selected from Substituent Group α, Substituent Group β and Substituent Group γ and which contains 1 to 3 sulfur, oxygen and/or nitrogen atoms" and "5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms" in the definition of R⁵; the "5-to 7-membered heteroaryl group which contains 1 to 3 sulfur, oxygen and/or nitrogen atoms" in the definitions of R^{e} and R^{f}; the 5- to 7-membered heteroaryl moiety of "5-to 7-membered heteroarylcarbonyl group which contains 1 to 3 sulfur, oxygen and/or nitrogen atoms" and "5- to 7-membered heteroarylsulfonyl group which contains 1 to 3 sulfur, oxygen and/or nitrogen atoms" in the definitions of R^{e} and R^{f}; the 5- to 7-membered heteroaryl group of "5- to 7-membered heteroaryl group which may be substituted with one or more groups selected from Substituent Group α and Substituent Group β and which contains 1 to 3 sulfur, oxygen and/or nitrogen atoms" in the definition of Substituent Group γ; and the 5- to 7-membered heteroaryl moiety of "5- to 7-membered heteroaryloxy group which may be substituted with one or more groups selected from Substituent Group α and Substituent Group β and which contains 1 to 3 sulfur, oxygen and/or nitrogen atoms" in the definition of Substituent Group γ can be, for example, furyl, thienyl, pyrrolyl, pyrazolyl, imidazolyl, oxazolyl, isoxazolyl, thiazolyl, isothiazolyl, triazolyl, thiadiazolyl, pyridyl, pyridazinyl, pyrimidinyl, pyrazinyl or azepinyl and preferably it is a 5- to 6-membered heteroaryl group which contains 1 or 2 sulfur, oxygen and/or nitrogen atoms such as furyl, thienyl, pyrrolyl, pyrazolyl, imidazolyl, oxazolyl, isoxazolyl, thiazolyl, isothiazolyl, pyridyl, pyridazinyl, pyrimidinyl or pyrazinyl.

Here, the above mentioned "5- to 7-membered heteroaryl group which contains 1 to 3 sulfur, oxygen and/or nitrogen atoms" may be condensed with another cyclic group [for example, a C₆-C₁₀ aryl or C₃-C₈ cycloalkyl group (preferably, a C₅-C₆ cycloalkyl group)], and such a group can be indolyl, benzofuranyl, benzothienyl, quinolyl, isoquinolyl, quinazolinyl, 5,6,7,8-tetrahydroquinolyl or 5,6,7,8-tetrahydroisoquinolyl.

The C₁-C₆ alkoxy group of "C₁-C₆ alkoxy group substituted with one or more groups selected from Substituent Group γ" in the definitions of R³ and R⁴; the alkoxy moiety of "C₂-C₇ alkoxycarbonyl group which may be substituted with one or more groups selected from Substituent Group α and Substituent Group γ" in the definition of R⁵; the "C₁-C₆ alkoxy group" in the definitions of R⁶ and Substituent Group α; the "C₁-C₆ alkoxy group" in the definitions of R^{e} and R^{f}; the C₁-C₆ alkoxy moiety of " C₁-C₆ alkoxyimino group" in the definition of Substituent Group α; and the C₁-C₆ alkoxy group of " C₁-C₆ alkoxy group substituted with one or more groups selected from Substituent Group α" in the definition of Substituent Group γ is one or more groups in which an oxygen atom is bonded to the above "C₁-C₆ alkyl group", more preferably it is a C₁-C₄ linear or branched chain alkoxy group, more preferably it is methoxy, ethoxy, propoxy, isopropoxy, butoxy, isobutoxy or tert-butoxy, still more preferably it is methoxy, ethoxy, propoxy, isopropoxy or butoxy, and particularly preferably it is methoxy or ethoxy.

The C₂-C₆ alkenyl group of "C₂-C₆ alkenyl group which may be substituted with one or more groups selected from Substituent Group α and Substituent Group γ" in the definition of R⁵ can be a linear or branched chain alkenyl group such as a vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl or 5-hexenyl group and preferably it is a C₂-C₄ linear or branched chain alkyl group, more preferably it is vinyl or 2-propenyl, and particularly preferably it is vinyl.

The "C₂-C₇ alkylcarbonyl group" in the definitions of R^{e} and R^{f}; and the C₂-C₇ alkylcarbonyl group of "C₂-C₇ alkylcarbonyl group which may be substituted with one or more groups selected from Substituent Group α and Substituent Group γ" in the definition of R⁵ is a group in which carbonyl is bonded to the above "C₁-C₆ alkyl group", and preferably it is a C₂-C₅ linear or branched chain alkylcarbonyl group, more preferably it is acetyl, propionyl, propylcarbonyl, isopropylcarbonyl, butylcarbonyl, isobutylcarbonyl or tert-butylcarbonyl, still more preferably it is acetyl, propionyl, propylcarbonyl, isopropylcarbonyl or butylcarbonyl, and particularly preferably it is acetyl or propionyl.

The 5- to 7-membered heterocyclylcarbonyl group of "5- to 7-membered heterocyclylcarbonyl group which may be substituted with one or more groups selected from Substituent Group α, Substituent Group β and Substituent Group γ and which contains 1 to 3 sulfur, oxygen and/or nitrogen atoms " in the definition of R⁵ is a group in which carbonyl is bonded to the above "5- to 7-membered heterocyclyl group", and preferably it is a 5- to 6-membered heterocyclylcarbonyl group which contains 1 or 2 sulfur, oxygen and/or nitrogen atoms, and particularly preferably it is piperidylcarbonyl, piperazinylcarbonyl or morpholinylcarbonyl.

The C₇-C₁₁ arylcarbonyl group" in the definitions of R^{e} and R^{f}; and the C₇-C₁₁ arylcarbonyl group of "C₇-C₁₁ arylcarbonyl group which may be substituted with one or more groups selected from Substituent Group α, Substituent Group β and Substituent Group γ" in the definition of R⁵ is a group in which carbonyl is bonded to the above "C₆-C₁₀ aryl group", and preferably it is benzoyl.

The "5- to 7-membered heteroarylcarbonyl group which contains 1 to 3 sulfur, oxygen and/or nitrogen atoms" in the definitions of R^{e} and R^{f}; and the "5- to 7-membered heteroarylcarbonyl group which may be substituted with one or more groups selected from Substituent Group α, Substituent Group β and Substituent Group γ and which contains 1 to 3 sulfur, oxygen and/or nitrogen atoms" in the definitions of R⁵ is a group in which carbonyl is bonded to the above "5- to 7-membered heteroaryl group", and preferably it is 5- to 6-membered heteroarylcarbonyl which contains 1 or 2 sulfur, oxygen and/or nitrogen atoms.

The "C₁-C₆ alkylsulfonyl group" in the definitions of Substituent Group α, R^{e} and R^{f}; and the C₁-C₆ alkylsulfonyl group of "C₁-C₆ alkylsulfonyl group which may be substituted with one or more groups selected from Substituent Group α and Substituent Group γ" in the definition of R⁵ is a group in which sulfonyl is bonded to the above "C₁-C₆ alkyl group", and preferably it is a C₁-C₄ linear or branched chain alkylsulfonyl group, more preferably it is methylsulfonyl, ethylsulfonyl, propylsulfonyl, isopropylsulfonyl, butylsulfonyl, isobutylsulfonyl or tert-butylsulfonyl, still more preferably it is methylsulfonyl, ethylsulfonyl, propylsulfonyl, isopropylsulfonyl or butylsulfonyl, and particularly preferably it is methylsulfonyl or ethylsulfonyl.

The "C₆-C₁₀ arylsulfonyl group" in the definitions of Substituent Group, R^{e} and R^{f}; and the C₆-C₁₀ arylsulfonyl group of "C₆-C₁₀ arylsulfonyl group which may be substituted with one or more groups selected from Substituent Group α, Substituent Group β and Substituent Group γ" in the definition of R⁵ is a group in which sulfonyl is bonded to the above "C₆-C₁₀ aryl group", and preferably it phenylsulfonyl.

The "5- to 7-membered heteroarylsulfonyl group which contains 1 to 3 sulfur, oxygen and/or nitrogen atoms" in the definitions of R^{e} and R^{f}; and the 5- to 7-membered heteroarylsulfonyl group of "5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms" in the definition R⁵ is a group in which sulfonyl is bonded to the above "5- to 7-membered heteroaryl group", and preferably it is 5- to 6-membered heteroarylsulfonyl which contains 1 or 2 sulfur, oxygen and/or nitrogen atoms.

The "C₂-C₇ alkoxycarbonyl group" in the definitions of R^{e} and R^{f}; and the C₂-C₇ alkoxycarbonyl group of "C₂-C₇ alkoxycarbonyl group which may be substituted with one or more groups selected from Substituent Group α and Substituent Group γ" in the definition of R⁵ is a group in which carbonyl is bonded to the above "C₁-C₆ alkoxy group", and preferably it is a C₂-C₅ linear or branched chain alkoxycarbonyl group, more preferably it is methoxycarbonyl, ethoxycarbonyl, propoxycarbonyl, isopropoxycarbonyl, butoxycarbonyl, isobutoxycarbonyl or tert-butoxycarbonyl, still more preferably it is methoxycarbonyl, ethoxycarbonyl, propoxycarbonyl, isopropoxycarbonyl or tert-butoxycarbonyl, and particularly preferably it is methoxycarbonyl, ethoxycarbonyl or tert-butoxycarbonyl.

The"C₆-C₁₀ aryloxy group" in the definition of R⁶; and the C₆-C₁₀ aryloxy group of "C₆-C₁₀ aryloxy group which may be substituted with one or more groups selected from Substituent Group α and Substituent Group β" in the definition of Substituent Group γ is a group in which an oxygen atom is bonded to the above "C₆-C₁₀ aryl group", and preferably it is phenoxy.

The C₇-C₁₁ aryloxycarbonyl group of "C₇-C₁₁ aryloxycarbonyl group which may be substituted with one or more groups selected from Substituent Group α, Substituent Group β and Substituent Group γ" in the definition of R⁵ is a group in which carbonyl is bonded to the above "C₆-C₁₀ aryloxy group", and preferably it is phenoxycarbonyl.

The "halogen atom" in the definition of Substituent Group α is a fluorine atom, a chlorine atom, a bromine atom or an iodine atom and preferably it is a fluorine atom or a chlorine atom.

The "C₁-C₆ halogenated alkyl group" in the definition of R⁶ is a group in which one or two or more hydrogen atoms in the above "C₁-C₆ alkyl group" is substituted with a "halogen atom" mentioned above, and preferably it is a C₁-C₄ alkyl halide group, more preferably it is trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl or 2,2-dibromoethyl, still more preferably it is trifluoromethyl, trichloromethyl, difluoromethyl or fluoromethyl, and the most preferably it is trifluoromethyl.

The "C₁-C₆ halogenated alkoxy group" in the definition of Substituent Group α is a group in which one or two or more hydrogen atoms in the above "C₁-C₆ alkoxy group" is substituted with a "halogen atom" mentioned above, and preferably it is a C₁-C₄ halogenated alkoxy group, more preferably it is trifluoromethyloxy, trichloromethyloxy, difluoromethyloxy, dichloromethyloxy, dibromomethyloxy, fluoromethyloxy, 2,2,2-trichloroethyloxy; 2,2,2-trifluoroethyloxy, 2-bromoethyloxy, 2-chloroethyloxy, 2-fluoroethyloxy or 2,2-dibromoethyloxy, and still more preferably it is trifluoromethyloxy, trichloromethyloxy, difluoromethyloxy or fluoromethyloxy.

The "C₁-C₆ alkylthio group" in the definition of Substituent Group α; and the C₁-C₆ alkylthio group of "C₁-C₆ alkylthio group substituted with one or more groups selected from Substituent Group α" in the definition of Substituent Group γ is a group in which a sulfur atom is bonded to the above "C₁-C₆ alkyl group", and preferably it is a C₁-C₄ linear or branched chain alkylthio group, more preferably it is methylthio, ethylthio, propylthio, isopropylthio, butylthio, isobutylthio or tert-butylthio, still more preferably it is methylthio, ethylthio, propylthio, isopropylthio or butylthio, and particularly preferably it is methylthio or ethylthio.

The "C₁-C₆ alkylsulfinyl group" in the definition of Substituent Group α is a group in which sulfinyl is bonded to the above "C₁-C₆ alkyl group", and preferably it is a C₁-C₄ linear or branched chain alkylsulfinyl group, more preferably it is methylsulfinyl, ethylsulfinyl, propylsulphinyl, isopropylsulphinyl, butylsulfinyl, isobutylsulfinyl or tert-butylsulfinyl, still more preferably it is methylsulfinyl, ethylsulfinyl, propylsulphinyl, isopropylsulfinyl or butylsulfinyl, and particularly preferably it is methylsulfinyl or ethylsulfinyl.

The "C₃-C₈ cycloalkyloxy group" in the definition of Substituent Group α; and the C₃-C₈ cycloalkyloxy group of "C₃-C₈ cycloalkyloxy group which may be substituted with one or more groups selected from Substituent Group α and Substituent Group β" in the definition of Substituent Group γ is a group in which an oxygen atom is bonded to the above "C₃-C₈ cycloalkyl group", and preferably it is a C₃-C₇ cycloalkyloxy group, and more preferably it is cyclopropyloxy, cyclobutyloxy, cyclopentyloxy, cyclohexyloxy or cycloheptyloxy.

The 5- to 7-membered heterocyclyloxy group of "5- to 7-membered heterocyclyloxy group which may be substituted with one or more groups selected from Substituent Group α and Substituent Group β and which contains 1 to 3 sulfur, oxygen and/or nitrogen atoms" in the definition of Substituent Group γ is a group in which an oxygen atom is bonded to the above "5- to 7-membered heterocyclyl group", and preferably it is a 5- to 6-membered heterocyclyloxy group which contains 1 or 2 sulfur, oxygen and/or nitrogen atoms, and particularly preferably it is piperidyloxy.

The 5- to 7-membered heteroaryloxy group of "5- to 7-membered heteroaryloxy group which may be substituted with one or more groups selected from Substituent Group α and Substituent Group β and which contains 1 to 3 sulfur, oxygen and/or nitrogen atoms" in the definition of Substituent Group γ is a group in which an oxygen atom is bonded to the above "5- to 7-membered heteroaryl which contains 1 to 3 sulfur, oxygen and/or nitrogen atoms", and preferably it is a 5- to 6-member heteroaryloxy group which contains 1 or 2 sulfur, oxygen and/or nitrogen atoms, and more preferably it is furyloxy, thienyloxy, pyrrolyloxy, pyrazolyloxy, imidazolyloxy, oxazolyloxy, isoxazolyloxy, thiazolyloxy, isothiazolyloxy, pyridyloxy, pyridazinyloxy, pyrimidinyloxy or pyrazinyloxy.

The C₆-C₁₀ aryl- C₁-C₆ alkoxy group of "C₆-C₁₀ aryl- C₁-C₆ alkoxy group in which the aryl moiety may be substituted with one or more groups selected from Substituent Group α and Substituent Group β" in the definition of Substituent Group γ is the above "C₁-C₆ alkoxy group" substituted with a "C₆-C₁₀ aryl group" mentioned above, and preferably it is benzyloxy, phenethyloxy or 3-phenylpropyloxy, and particularly preferably it is benzyloxy.

Since compound (I) of the present invention can be converted to a salt by reacting with an acid when it has a basic functional group such as an amino group or with a base when it has an acidic functional group such as a carboxyl group, the "pharmacologically acceptable salt thereof" refers to such a salt.

The salt based on a basic functional group can be, for example, an inorganic acid salt such as a hydrohalide such as hydrochloride, hydrobromide or hydriodide, nitrate, perchlorate, sulfate or phosphate; an organic salt such as a lower alkanesulfonate such as methanesulfonate, trifluoromethanesulfonate or ethanesulfonate, an aryl sulfonate such as benzenesulfonate or p-toluenesulfonate, or a carboxylate such as acetate, malate, fumarate, succinate, citrate, ascorbate, tartrate, oxalate or maleate; or it can be an amino acid salt such as glycinate, lysinate, argininate, ornithinate, glutamate or aspartate.

The salt based on an acid functional group can be, for example, a metal salt such as an alkali metal salt such as a sodium salt, potassium salt or lithium salt, an alkaline earth metal salt such as a calcium salt or magnesium salt, an aluminium salt or an iron salt; an ammonium salt; an organic amine salt such as a t-octylamine salt, dibenzylamine salt, morpholine salt, glucosamine salt, phenylglycine alkyl ester salt, diaminoethane salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt or tris(hydroxymethyl)aminomethane salt; or it can be an amino acid salt such as glycine salt, lysine salt, arginine salt, ornithine salt, glutamate or aspartate.

The compounds having general formula (I) of the present invention or pharmacologically acceptable salt thereof when allowed to stand in the atmosphere or to recrystallize may absorb water or adsorb water to form hydrates and such hydrates are also included in the present invention.

Among the compounds having general formula (I) of the present invention, there may be optical isomers due to an asymmetric centre in the molecule. For the compounds of the present invention, it should be noted that these isomers and mixtures thereof are represented by a single formula, i.e. general formula (I). Therefore, the present invention encompasses these isomers and mixtures of these isomers in any ratio.

Specific examples of the compound having general formula (I) of the present invention include compounds described in the following Table 1 to Table 3 for exemplified compounds.

Here, in Table 1 to Table 3, "Ac" represents acetyl, "Aze" represents azetidino, "perhydro-1-Azep" represents perhydroazepin-1-yl, "perhydro-1-Azoc" represents perhydroazocin-1-yl, "Bn" represents benzyl, "Bu" represents butyl, "iBu" represents isobutyl, "tBu" represents tert-butyl, "BzIox" represents 1,2-benzisoxazolyl, "BzDioxa" represents 1,4-benzodioxanyl, "BzDioxo" represents 1,3-benzodioxolanyl, "BzFur" represents 1-benzofuranyl, "Bzhy" represents benzhydryl, "BzOxaz" represents 1,3-benzoxazolyl, "BzThaz" represents 1,3-benzothiazolyl, "Chr" represents chromanyl, "Dioxa" represents 1,3-dioxanyl, "Dioxo" represents 1,3-dioxolanyl, "Et" represents ethyl, "Fur" represents furanyl, "cHep" represents cycloheptyl, "cHx" represents cyclohexyl, "IIndn" represents isoindolinyl, "Ind" represents indolyl, "Iqui" represents isoquinolyl, "decahydro-2-Iqui" represents 1,2,3,4,5,6,7,8,9,10-decahydroisoquinolin-2-yl, "1,2,3,4-tetrahydro-2-Iqui" represents 1,2,3,4-tetrahydroisoquinolin-2-yl, "Me" represents methyl, "Mor" represents morpholin-4-yl, "Oxaz" represents oxazolyl, "1,4-Oxazep" represents 1,4-oxazepin-4-yl, "Oxazn" represents oxazolynyl, "Ph" represents phenyl, "Phet" represents phenethyl, "Pip" represents piperidin-4-yl, "3,4-dehydro-Pip" represents 3,4-dehydropiperidin-4-yl, "Pipo" represents piperidino, "Pipra" represents piperazino, "cPn" represents cyclopentyl, "neoPn" represents neopentyl, "Pr" represents propyl, "iPr" represents isopropyl, "Py" represents pyridyl, "Pydz" represents pyridazinyl, "Pym" represents pyrimidyl, "Pyrld" represents pyrrolidin-1-yl, "Qui" represents quinolyl, "Qunz" represents quinazolinyl, "Thaz" represents thiazolyl, "Thazn" represents thiazolinyl, "Thi" represents thienyl, "1,4-Thiazep" represents 1,4-thiazepin-4-yl, and "Thmor" represents thiomorpholin-4-yl.

**Table 1**

| | | |
|---|---|---|
| 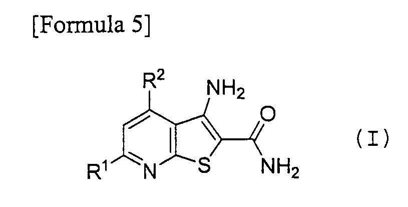 | | |

| Compound Number | R¹ | R² |
|---|---|---|
| 1-1 | H | MeO |
| 1-2 | H | EtO |
| 1-3 | H | iPrO |
| 1-4 | H | BnO |
| 1-5 | H | (HOOC)-CH₂O |
| 1-6 | H | cPnO |
| 1-7 | H | cHxO |
| 1-8 | H | cHepO |
| 1-9 | H | MeS |
| 1-10 | H | EtS |
| 1-11 | H | iPrS |
| 1-12 | H | BnS |
| 1-13 | H | cHxS |
| 1-14 | H | cHepS |
| 1-15 | H | Pyrld |
| 1-16 | H | Pip |
| 1-17 | H | 3-Me-Pip |
| 1-18 | H | 4-Me-Pip |
| 1-19 | H | 4-Bn-Pip |
| 1-20 | H | 3-(HO-CH₂)-Pip |
| 1-21 | H | 3-(MeO-CH₂)-Pip |
| 1-22 | H | 3-(EtO-CH₂)-Pip |
| 1-23 | H | 3-(PrO-CH₂)-Pip |
| 1-24 | H | 3-(BnO-CH₂)-Pip |
| 1-25 | H | 3-Ph-Pip |
| 1-26 | H | 4-Ph-Pip |
| 1-27 | H | 3-HO-Pip |
| 1-28 | H | 3-MeO-Pip |
| 1-29 | H | 3-EtO-Pip |
| 1-30 | H | 3-PrO-Pip |
| 1-31 | H | 3-BnO-Pip |
| 1-32 | H | 4-HO-Pip |
| 1-33 | H | 3-AcO-Pip |
| 1-34 | H | 4-AcO-Pip |
| 1-35 | H | 1,2,3,4-tetrahydro-2-Iqui |
| 1-36 | H | decahydro-2-Iqui |
| 1-37 | H | 3,4-dehydro-Pip |
| 1-38 | H | 4-Ph-3,4-dehydro-1-Pip |
| 1-39 | H | perhydro-1-Azep |
| 1-40 | H | perhydro-1-Azoc |
| 1-41 | H | Mor |
| 1-42 | H | 2,6-diMe-Mor |
| 1-43 | H | 1,4-Oxazep |
| 1-44 | H | Thmor |
| 1-45 | H | 1-Oxo-Thmor |
| 1-46 | H | 1,4-Thiazep |
| 1-47 | H | 1-Oxo-1,4-Thiazep |
| 1-48 | Me | MeO |
| 1-49 | Me | EtO |
| 1-50 | Me | iPrO |
| 1-51 | Me | BnO |
| 1-52 | Me | (HOOC)-CH₂O |
| 1-53 | Me | cPnO |
| 1-54 | Me | cHxO |
| 1-55 | Me | cHepO |
| 1-56 | Me | MeS |
| 1-57 | Me | EtS |
| 1-58 | Me | iPrS |
| 1-59 | Me | BnS |
| 1-60 | Me | cHxS |
| 1-61 | Me | cHepS |
| 1-62 | Me | Pyrld |
| 1-63 | Me | Pip |
| 1-64 | Me | 3-Me-Pip |
| 1-65 | Me | 4-Me-Pip |
| 1-66 | Me | 4-Bn-Pip |
| 1-67 | Me | 3-(HO-CH₂)-Pip |
| 1-68 | Me | 3-(MeO-CH₂)-Pip |
| 1-69 | Me | 3-(EtO-CH₂)-Pip |
| 1-70 | Me | 3-(PrO-CH₂)-Pip |
| 1-71 | Me | 3-(BnO-CH₂)-Pip |
| 1-72 | Me | 3-Ph-Pip |
| 1-73 | Me | 4-Ph-Pip |
| 1-74 | Me | 3-HO-Pip |
| 1-75 | Me | 3-MeO-Pip |
| 1-76 | Me | 3-EtO-Pip |
| 1-77 | Me | 3-PrO-Pip |
| 1-78 | Me | 3-BnO-Pip |
| 1-79 | Me | 4-HO-Pip |
| 1-80 | Me | 3-AcO-Pip |
| 1-81 | Me | 4-AcO-Pip |
| 1-82 | Me | 1,2,3,4-tetrahydro-2-Iqui |
| 1-83 | Me | decahydro-2-Iqui |
| 1-84 | Me | 3,4-dehydro-Pip |
| 1-85 | Me | 4-Ph-3,4-dehydro-1-Pip |
| 1-86 | Me | perhydro-1-Azep |
| 1-87 | Me | perhydro-1-Azoc |
| 1-88 | Me | Mor |
| 1-89 | Me | 2,6-diMe-Mor |
| 1-90 | Me | 1,4-Oxazep |
| 1-91 | Me | Thmor |
| 1-92 | Me | 1-Oxo-Thmor |
| 1-93 | Me | 1,4-Thiazep |
| 1-94 | Me | 1-Oxo-1,4-Thiazep |
| 1-95 | H | 3-[HO-(CH₂)₂]-Pip |
| 1-96 | H | 3-[HO-(CH₂)₃]-Pip |
| 1-97 | H | 3-[HO-(CH₂)₄]-Pip |
| 1-98 | H | 3-[HO-(CH₂)₅]-Pip |
| 1-99 | H | 3-[HO-(CH₂)₂-O-CH₂]-Pip |
| 1-100 | H | 3-[HO-(CH₂)₂-O-(CH₂)₂]-Pip |
| 1-101 | H | 3-[HO-(CH₂)₂-O-(CH₂)₃]-Pip |
| 1-102 | H | 3-[HO-(CH₂)₃-O-CH₂]-Pip |
| 1-103 | H | 3-[HO-(CH₂)₃-O-(CH₂)₂]-Pip |
| 1-104 | H | 3-[HO-(CH₂)₃-O-(CH₂)₃]-Pip |
| 1-105 | H | 3-[HO-(CH₂)₄-O-CH₂]-Pip |
| 1-106 | H | 3-[HO-(CH₂)₄-O-(CH₂)₂]-Pip |
| 1-107 | H | 3-[HO-(CH₂)₄-O-(CH₂)₃]-Pip |
| 1-108 | H | 3-[MeO-(CH₂)₂]-Pip |
| 1-109 | H | 3-[MeO-(CH₂)₃]-Pip |
| 1-110 | H | 3-[MeO-(CH₂)₄]-Pip |
| 1-111 | H | 3-[MeO-(CH₂)₅]-Pip |
| 1-112 | H | 3-[MeO-(CH₂)₂-O-CH₂]-Pip |
| 1-113 | H | 3-[MeO-(CH₂)₂-O-(CH₂)₂]-Pip |
| 1-114 | H | 3-[MeO-(CH₂)₂-O-(CH₂)₃]-Pip |
| 1-115 | H | 3-[MeO-(CH₂)₃-O-CH₂]-Pip |
| 1-116 | H | 3 -[MeO-(CH₂)₃-O-(CH₂)₂]-Pip |
| 1-117 | H | 3-[MeO-(CH₂)₃-O-(CH₂)₃]-Pip |
| 1-118 | H | 3-[MeO-(CH₂)₄-O-CH₂]-Pip |
| 1-119 | H | 3-[MeO-(CH₂)₄-O-(CH₂)₂]-Pip |
| 1-120 | H | 3-[MeO-(CH₂)₄-O-(CH₂)₃]-Pip |
| 1-121 | H | 3-[EtO-(CH₂)₂]-Pip |
| 1-122 | H | 3-[EtO-(CH₂)₃]-Pip |
| 1-123 | H | 3-[EtO-(CH₂)₂-O-CH₂]-Pip |
| 1-124 | H | 3-[EtO-(CH₂)₂-O-(CH₂)₂]-Pip |
| 1-125 | H | 3-[EtO-(CH₂)₂-O-(CH₂)₃]-Pip |
| 1-126 | H | 3-[PrO-(CH₂)₂]-Pip |
| 1-127 | H | 3-[PrO-(CH₂)₃]-Pip |
| 1-128 | H | 3-[BnO-(CH₂)₂]-Pip |
| 1-129 | H | 3-[BnO-(CH₂)₃]-Pip |
| 1-130 | H | 3-[PhO-(CH₂)₂-O-CH₂]-Pip |
| 1-131 | H | 3-[PhO-(CH₂)₂-O-(CH₂)₂]-Pip |
| 1-132 | H | 3-[PhO-(CH₂)₂-O-(CH₂)₃]-Pip |
| 1-133 | H | 3-[H₂N-(CH₂)₂-O-CH₂]-Pip |
| 1-134 | H | 3-[H₂N-(CH₂)₂-O-(CH₂)₂]-Pip |
| 1-135 | H | 3-[H₂N-(CH₂)₂-O-(CH₂)₃]-Pip |
| 1-136 | H | 3-[H₂N-(CH₂)₃-O-CH₂]-Pip |
| 1-137 | H | 3-[H₂N-(CH₂)₃-O-(CH₂)₂]-Pip |
| 1-138 | H | 3-[H₂N-(CH₂)₃-O-(CH₂)₃]-Pip |
| 1-139 | H | 3-[H₂N-(CH₂)₂-S-CH₂]-Pip |
| 1-140 | H | 3-[H₂N-(CH₂)₂-O-(CH₂)₂-O-CH₂]-Pip |
| 1-141 | H | 3-[Me₂N-(CH₂)₂-O-CH₂]-Pip |
| 1-142 | H | 3-[Me₂N-(CH₂)₂-O-(CH₂)₂]-Pip |
| 1-143 | H | 3-[Me₂N-(CH₂)₂-O-(CH₂)₃]-Pip |
| 1-144 | H | 3-[Me₂N-(CH₂)₃-O-CH₂]-Pip |
| 1-145 | H | 3-[Me₂N-(CH₂)₃-O-(CH₂)₂]-Pip |
| 1-146 | H | 3-[Me₂N-(CH₂)₃-O-(CH₂)₃]-Pip |
| 1-147 | H | 3-[Me₂N-(CH₂)₂-S-CH₂]-Pip |
| 1-148 | H | 3-[Me₂N-(CH₂)₂-O-(CH₂)₂-O-CH₂]-Pip |
| 1-149 | H | 3-(CN-CH₂-O-CH₂)-Pip |
| 1-150 | H | 3-[CN-CH₂-O-(CH₂)₂]-Pip |
| 1-151 | H | 3-[CN-CH₂-O-(CH₂)₃]-Pip |
| 1-152 | H | 3-[CN-(CH₂)₂-O-CH₂]-Pip |
| 1-153 | H | 3-[CN-(CH₂)₂-O-(CH₂)₂]-Pip |
| 1-154 | H | 3-[CN-(CH₂)₂-O-(CH₂)₃]-Pip |
| 1-155 | H | 3-(Me₂NCO-CH₂-O-CH₂)-Pip |
| 1-156 | H | 3-[Me₂NCO-CH₂-O-(CH₂)₂]-Pip |
| 1-157 | H | 3-[Me₂NCO-CH₂-O-(CH₂)₃]-Pip |
| 1-158 | H | 3-[Me₂NCO-(CH₂)₂-O-CH₂]-Pip |
| 1-159 | H | 3-[Me₂NCO-(CH₂)₂-O-(CH₂)₂]-Pip |
| 1-160 | H | 3-[Me₂NCO-(CH₂)₂-O-(CH₂)₃]-Pip |
| 1-161 | H | 3-[Me₂NCO-(CH₂)₃-O-CH₂]-Pip |
| 1-162 | H | 3-[Me₂NCO-(CH₂)₃-O-(CH₂)₂]-Pip |
| 1-163 | H | 3-[Me₂NCO-(CH₂)₃-O-(CH₂)₃]-Pip |
| 1-164 | H | 3-(Et₂NCO-CH₂-O-CH₂)-Pip |
| 1-165 | H | 3-[Et₂NCO-CH₂-O-(CH₂)₂]-Pip |
| 1-166 | H | 3-[Et₂NCO-CH₂-O-(CH₂)₃]-Pip |
| 1-167 | H | 3-[Et₂NCO-(CH₂)₂-O-CH₂]-Pip |
| 1-168 | H | 3-[Et₂NCO-(CH₂)₂-O-(CH₂)₂]-Pip |
| 1-169 | H | 3-[Et₂NCO-(CH₂)₂-O-(CH₂)₃]-Pip |
| 1-170 | H | 3-[Et₂NCO-(CH₂)₃-O-CH₂]-Pip |
| 1-171 | H | 3-[Et₂NCO-(CH₂)₃-O-(CH₂)₂]-Pip |
| 1-172 | H | 3-[Et₂NCO-(CH₂)₃-O-(CH₂)₃]-Pip |
| 1-173 | H | 3-[AcNH-(CH₂)₂-O-CH₂]-Pip |
| 1-174 | H | 3-[AcNH-(CH₂)₂-O-(CH₂)₂]-Pip |
| 1-175 | H | 3-[AcNH-(CH₂)₂-O-(CH₂)₃]-Pip |
| 1-176 | H | 3-[AcNH-(CH₂)₃-O-CH₂]-Pip |
| 1-177 | H | 3-[AcNH-(CH₂)₃-O-(CH₂)₂]-Pip |
| 1-178 | H | 3-[AcNH-(CH₂)₃-O-(CH₂)₃]-Pip |
| 1-179 | H | 3-[AcNH-(CH₂)₂-S-CH₂]-Pip |
| 1-180 | H | 3-[AcNH-(CH₂)₂-O-(CH₂)₂-O-CH₂]-Pip |
| 1-181 | H | 3-[Me-(CH₂)₄-CONH-(CH₂)₂-O-CH₂]-Pip |
| 1-182 | H | 3-[BocNH-(CH₂)₂-O-CH₂]-Pip |
| 1-183 | H | 3-[NC-(CH₂)₂-CONH-(CH₂)₂-O-CH₂]-Pip |
| 1-184 | H | 3-(H₂N-CH₂)-Pip |
| 1-185 | H | 3-[HO-(CH₂)₂-NH-CH₂]-Pip |
| 1-186 | H | 3-(BocNH-CH₂)-Pip |
| 1-187 | H | 3-(MeNH-CH₂)-Pip |
| 1-188 | H | 3-[Me-(CH₂)₃-NH-CH₂]-Pip |
| 1-189 | H | 3-(Me₂N-CH₂)-Pip |
| 1-190 | H | 2-(MeO-CH₂)-Mor |
| 1-191 | H | 3-[Et₂N-(CH₂)₂-O-CH₂]-Pip |
| 1-192 | H | 3-[Et₂N-(CH₂)₂-O-(CH₂)₂]-Pip |
| 1-193 | H | 3-[Et₂N-(CH₂)₂-O-(CH₂)₃]-Pip |
| 1-194 | H | 3-[Et₂N-(CH₂)₃-O-CH₂]-Pip |
| 1-195 | H | 3-[Et₂N-(CH₂)₃-O-(CH₂)₂]-Pip |
| 1-196 | H | 3-[Et₂N-(CH₂)₃-O-(CH₂)₃]-Pip |
| 1-197 | H | 3-[Et₂N-(CH₂)₂-S-CH₂]-Pip |
| 1-198 | H | 3-[Pyrld-(CH₂)₂-O-CH₂]-Pip |
| 1-199 | H | 3-[Pyrld-(CH₂)₂-O-(CH₂)₂]-Pip |
| 1-200 | H | 3-[Pyrld-(CH₂)₂-O-(CH₂)₃]-Pip |
| 1-201 | H | 3-[Pyrld-(CH₂)₃-O-CH₂]-Pip |
| 1-202 | H | 3-[Pyrld-(CH₂)₃-O-(CH₂)₂]-Pip |
| 1-203 | H | 3-[Pyrld-(CH₂)₃-O-(CH₂)₃]-Pip |
| 1-204 | H | 3-[Pyrld-(CH₂)₂-S-CH₂]-Pip |
| 1-205 | H | 3-[Mor-(CH₂)₂-O-CH₂]-Pip |
| 1-206 | H | 3-[Mor-(CH₂)₂-O-(CH₂)₂]-Pip |
| 1-207 | H | 3-[Mor-(CH₂)₂-O-(CH₂)₃]-Pip |
| 1-208 | H | 3-[Mor-(CH₂)₃-O-CH₂]-Pip |
| 1-209 | H | 3-[Mor-(CH₂)₃-O-(CH₂)₂]-Pip |
| 1-210 | H | 3-[Mor-(CH₂)₃-O-(CH₂)₃]-Pip |
| 1-211 | H | 3-[Mor-(CH₂)₂-S-CH₂]-Pip |
| 1-212 | H | 3-[Pipo-(CH₂)₂-O-CH₂]-Pip |
| 1-213 | H | 3-[Pipo-(CH₂)₂-O-(CH₂)₂]-Pip |
| 1-214 | H | 3-[Pipo-(CH₂)₂-O-(CH₂)₃]-Pip |
| 1-215 | H | 3-[Pipo-(CH₂)₃-O-CH₂] -Pip |
| 1-216 | H | 3-[Pipo-(CH₂)₃-O-(CH₂)₂]-Pip |
| 1-217 | H | 3-[Pipo-(CH₂)₃-O-(CH₂)₃]-Pip |
| 1-218 | H | 3-[Pipo-(CH₂)₂-S-CH₂]-Pip |
| 1-219 | H | 3-[(4-Me-Pipra)-(CH₂)₂-O-CH₂]-Pip |
| 1-220 | H | 3-[(4-Me-Pipra)-(CH₂)₂-O-(CH₂)₂]-Pip |
| 1-221 | H | 3-[(4-Me-Pipra)-(CH₂)₂-O-(CH₂)₃]-Pip |
| 1-222 | H | 3-[(4-Me-Pipra)-(CH₂)₃-O-CH₂]-Pip |
| 1-223 | H | 3-[(4-Me-Pipra)-(CH₂)₃-O-(CH₂)₂]-Pip |
| 1-224 | H | 3-[(4-Me-Pipra)-(CH₂)₃-O-(CH₂)₃]-Pip |
| 1-225 | H | 3-[(4-Me-Pipra)-(CH₂)₂-S-CH₂]-Pip |
| 1-226 | H | 3-(Pyrld-CO-CH₂-O-CH₂)-Pip |
| 1-227 | H | 3-[Pyrld-CO-CH₂-O-(CH₂)₂]-Pip |
| 1-228 | H | 3-[Pyrld-CO-CH₂-O-(CH₂)₃]-Pip |
| 1-229 | H | 3-(Mor-CO-CH₂-O-CH₂)-Pip |
| 1-230 | H | 3-[Mor-CO-CH₂-O-(CH₂)₂]-Pip |
| 1-231 | H | 3-[Mor-CO-CH₂-O-(CH₂)₃]-Pip |
| 1-232 | H | 3-(Pipo-CO-CH₂-O-CH₂)-Pip |
| 1-233 | H | 3-[Pipo-CO-CH₂-O-(CH₂)₂]-Pip |
| 1-234 | H | 3-[Pipo-CO-CH₂-O-(CH₂)₃]-Pip |
| 1-235 | H | 3-[(4-Me-Pipra)-CO-CH₂-O-CH₂]-Pip |
| 1-236 | H | 3-[(4-Me-Pipra)-CH₂-O-(CH₂)₂]-Pip |
| 1-237 | H | 3-[(4-Me-Pipra)-CH₂-O-(CH₂)₃]-Pip |
| 1-238 | Me | 3-[HO-(CH₂)₂]-Pip |
| 1-239 | Me | 3-[HO-(CH₂)₃]-Pip |
| 1-240 | Me | 3-[HO-(CH₂)₄]-Pip |
| 1-241 | Me | 3-[HO-(CH₂)₅]-Pip |
| 1-242 | Me | 3-[HO-(CH₂)₂-O-CH₂]-Pip |
| 1-243 | Me | 3-[HO-(CH₂)₂-O-(CH₂)₂]-Pip |
| 1-244 | Me | 3-[HO-(CH₂)₂-O-(CH₂)₃]-Pip |
| 1-245 | Me | 3-[HO-(CH₂)₃-O-CH₂]-Pip |
| 1-246 | Me | 3-[HO-(CH₂)₃-O-(CH₂)₂]-Pip |
| 1-247 | Me | 3-[HO-(CH₂)₃-O-(CH₂)₃]-Pip |
| 1-248 | Me | 3-[HO-(CH₂)₄-O-CH₂]-Pip |
| 1-249 | Me | 3-[HO-(CH₂)₄-O-(CH₂)₂]-Pip |
| 1-250 | Me | 3-[HO-(CH₂)₄-O-(CH₂)₃]-Pip |
| 1-251 | Me | 3-[MeO-(CH₂)₂]-Pip |
| 1-252 | Me | 3-[MeO-(CH₂)₃]-Pip |
| 1-253 | Me | 3-[MeO-(CH₂)₄]-Pip |
| 1-254 | Me | 3-[MeO-(CH₂)₅]-Pip |
| 1-255 | Me | 3-[MeO-(CH₂)₂-O-CH₂]-Pip |
| 1-256 | Me | 3-[MeO-(CH₂)₂-O-(CH₂)₂]-Pip |
| 1-257 | Me | 3-[MeO-(CH₂)₂-O-(CH₂)₃]-Pip |
| 1-258 | Me | 3-[MeO-(CH₂)₃-O-CH₂]-Pip |
| 1-259 | Me | 3-[MeO-(CH₂)₃-O-(CH₂)₂]-Pip |
| 1-260 | Me | 3-[MeO-(CH₂)₃-O-(CH₂)₃]-Pip |
| 1-261 | Me | 3-[MeO-(CH₂)₄-O-CH₂]-Pip |
| 1-262 | Me | 3-[MeO-(CH₂)₄-O-(CH₂)₂]-Pip |
| 1-263 | Me | 3-[MeO-(CH₂)₄-O-(CH₂)₃]-Pip |
| 1-264 | Me | 3-[EtO-(CH₂)₂]-Pip |
| 1-265 | Me | 3-[EtO-(CH₂)₃]-Pip |
| 1-266 | Me | 3-[EtO-(CH₂)₂-O-CH₂]-Pip |
| 1-267 | Me | 3-[EtO-(CH₂)₂-O-(CH₂)₂]-Pip |
| 1-268 | Me | 3-[EtO-(CH₂)₂-O-(CH₂)₃]-Pip |
| 1-269 | Me | 3-[PrO-(CH₂)₂]-Pip |
| 1-270 | Me | 3-[PrO-(CH₂)₃]-Pip |
| 1-271 | Me | 3-[BnO-(CH₂)₂]-Pip |
| 1-272 | Me | 3-[BnO-(CH₂)₃]-Pip |
| 1-273 | Me | 3-[PhO-(CH₂)₂-O-CH₂]-Pip |
| 1-274 | Me | 3-[PhO-(CH₂)₂-O-(CH₂)₂]-Pip |
| 1-275 | Me | 3-[PhO-(CH₂)₂-O-(CH₂)₃]-Pip |
| 1-276 | Me | 3-[H₂N-(CH₂)₂-O-CH₂]-Pip |
| 1-277 | Me | 3-[H₂N-(CH₂)₂-O-(CH₂)₂]-Pip |
| 1-278 | Me | 3-[H₂N-(CH₂)₂-O-(CH₂)₃]-Pip |
| 1-279 | Me | 3-[H₂N-(CH₂)₃-O-CH₂]-Pip |
| 1-280 | Me | 3-[H₂N-(CH₂)₃-O-(CH₂)₂]-Pip |
| 1-281 | Me | 3-[H₂N-(CH₂)₃-O-(CH₂)₃]-Pip |
| 1-282 | Me | 3-[H₂N-(CH₂)₂-S-CH₂]-Pip |
| 1-283 | Me | 3-[H₂N-(CH₂)₂-O-(CH₂)₂-O-CH₂]-Pip |
| 1-284 | Me | 3-[Me₂N-(CH₂)₂-O-CH₂]-Pip |
| 1-285 | Me | 3-[Me₂N-(CH₂)₂-O-(CH₂)₂]-Pip |
| 1-286 | Me | 3-[Me₂N-(CH₂)₂-O-(CH₂)₃]-Pip |
| 1-287 | Me | 3-[Me₂N-(CH₂)₃-O-CH₂]-Pip |
| 1-288 | Me | 3-[Me₂N-(CH₂)₃-O-(CH₂)₂]-Pip |
| 1-289 | Me | 3-[Me₂N-(CH₂)3-O-(CH₂)₃]-Pip |
| 1-290 | Me | 3-[Me₂N-(CH₂)₂-S-CH2]-Pip |
| 1-291 | Me | 3-[Me₂N-(CH₂)₂-O-(CH₂)₂-O-CH₂]-Pip |
| 1-292 | Me | 3-(CN-CH₂-O-CH₂)-Pip |
| 1-293 | Me | 3-[CN-CH₂-O-(CH₂)₂]-Pip |
| 1-294 | Me | 3-[CN-CH₂-O-(CH₂)₃]-Pip |
| 1-295 | Me | 3-[CN-(CH₂)₂-O-CH₂]-Pip |
| 1-296 | Me | 3-[CN-(CH₂)₂-O-(CH₂)₂]-Pip |
| 1-297 | Me | 3-[CN-(CH₂)₂-O-(CH₂)₃]-Pip |
| 1-298 | Me | 3-(Me₂NCO-CH₂-O-CH₂)-Pip |
| 1-299 | Me | 3-[Me₂NCO-CH₂-O-(CH₂)₂]-Pip |
| 1-300 | Me | 3-[Me₂NCO-CH₂-O-(CH₂)₃]-Pip |
| 1-301 | Me | 3-[Me₂NCO-(CH₂)₂-O-CH₂]-Pip |
| 1-302 | Me | 3-[Me₂NCO-(CH₂)₂-O-(CH₂)₂]-Pip |
| 1-303 | Me | 3-[Me₂NCO-(CH₂)₂-O-(CH₂)₃]-Pip |
| 1-304 | Me | 3-[Me₂NCO-(CH₂)₃-O-CH₂]-Pip |
| 1-305 | Me | 3-[Me₂NCO-(CH₂)₃-O-(CH₂)₂]-Pip |
| 1-306 | Me | 3-[Me₂NCO-(CH₂)₃-O-(CH₂)₃]-Pip |
| 1-307 | Me | 3-(Et₂NCO-CH₂-O-CH₂)-Pip |
| 1-308 | Me | 3-[Et₂NCO-CH₂-O-(CH₂)₂]-Pip |
| 1-309 | Me | 3-[Et₂NCO-CH₂-O-(CH₂)₃]-Pip |
| 1-310 | Me | 3-[Et₂NCO-(CH₂)₂-O-CH₂]-Pip |
| 1-311 | Me | 3-[Et₂NCO-(CH₂)₂-O-(CH₂)₂]-Pip |
| 1-312 | Me | 3-[Et₂NCO-(CH₂)₂-O-(CH₂)₃]-Pip |
| 1-313 | Me | 3-[Et₂NCO-(CH₂)₃-O-CH₂]-Pip |
| 1-314 | Me | 3-[Et₂NCO-(CH₂)₃-O-(CH₂)₂]-Pip |
| 1-315 | Me | 3-[Et₂NCO-(CH₂)₃-O-(CH₂)₃]-Pip |
| 1-316 | Me | 3-[AcNH-(CH₂)₂-O-CH₂]-Pip |
| 1-317 | Me | 3-[AcNH-(CH₂)₂-O-(CH₂)₂]-Pip |
| 1-318 | Me | 3-[AcNH-(CH₂)₂-O-(CH₂)₃]-Pip |
| 1-319 | Me | 3-[AcNH-(CH₂)₃-O-CH₂]-Pip |
| 1-320 | Me | 3-[AcNH-(CH₂)₃-O-(CH₂)₂]-Pip |
| 1-321 | Me | 3-[AcNH-(CH₂)₃-O-(CH₂)₃]-Pip |
| 1-322 | Me | 3-[AcNH-(CH₂)₂-S-CH₂]-Pip |
| 1-323 | Me | 3-[AcNH-(CH₂)₂-O-(CH₂)₂-O-CH₂]-Pip |
| 1-324 | Me | 3-[Me-(CH₂)₄-CONH-(CH₂)₂-O-CH₂]-Pip |
| 1-325 | Me | 3-[BocNH-(CH₂)₂-O-CH₂]-Pip |
| 1-326 | Me | 3-[NC-(CH₂)₂-CONH-(CH₂)₂-O-CH₂]-Pip |
| 1-327 | Me | 3-(H₂N-CH₂)-Pip |
| 1-328 | Me | 3-[HO-(CH₂)₂-NH-CH₂]-Pip |
| 1-329 | Me | 3-(BocNH-CH₂)-Pip |
| 1-330 | Me | 3-(MeNH-CH₂)-Pip |
| 1-331 | Me | 3-[Me-(CH₂)₃-NH-CH₂]-Pip |
| 1-332 | Me | 3-(Me₂N-CH₂)-Pip |
| 1-333 | Me | 2-(MeO-CH₂)-Mor |
| 1-334 | Me | 3-[Et₂N-(CH₂)₂-O-CH₂]-Pip |
| 1-335 | Me | 3-[Et₂N-(CH₂)₂-O-(CH₂)₂]-Pip |
| 1-336 | Me | 3-[Et₂N-(CH₂)₂-O-(CH₂)₃]-Pip |
| 1-337 | Me | 3-[Et₂N-(CH₂)₃-O-CH₂]-Pip |
| 1-338 | Me | 3-[Et₂N-(CH₂)₃-O-(CH₂)₂]-Pip |
| 1-339 | Me | 3-[Et₂N-(CH₂)₃-O-(CH₂)₃]-Pip |
| 1-340 | Me | 3-[Et₂N-(CH₂)₂-S-CH₂]-Pip |
| 1-341 | Me | 3-[Pyrld-(CH₂)₂-O-CH₂]-Pip |
| 1-342 | Me | 3-[Pyrld-(CH₂)₂-O-(CH₂)₂]-Pip |
| 1-343 | Me | 3-[Pyrld-(CH₂)₂-O-(CH₂)₃]-Pip |
| 1-344 | Me | 3-[Pyrld-(CH₂)₃-O-CH₂]-Pip |
| 1-345 | Me | 3-[Pyrld-(CH₂)₃-O-(CH₂)₂]-Pip |
| 1-346 | Me | 3-[Pyrld-(CH₂)₃-O-(CH₂)₃]-Pip |
| 1-347 | Me | 3-[Pyrld-(CH₂)₂-S-CH₂]-Pip |
| 1-348 | Me | 3-[Mor-(CH₂)₂-O-CH₂]-Pip |
| 1-349 | Me | 3-[Mor-(CH₂)₂-O-(CH₂)₂]-Pip |
| 1-350 | Me | 3-[Mor-(CH₂)₂-O-(CH₂)₃]-Pip |
| 1-351 | Me | 3-[Mor-(CH₂)₃-O-CH₂]-Pip |
| 1-352 | Me | 3-[Mor-(CH₂)₃-O-(CH₂)₂]-Pip |
| 1-353 | Me | 3-[Mor-(CH₂)₃-O-(CH₂)₃]-Pip |
| 1-354 | Me | 3-[Mor-(CH₂)₂-S-CH₂]-Pip |
| 1-355 | Me | 3-[Pipo-(CH₂)₂-O-CH₂]-Pip |
| 1-356 | Me | 3-[Pipo-(CH₂)₂-O-(CH₂)₂]-Pip |
| 1-357 | Me | 3-[Pipo-(CH₂)₂-O-(CH₂)₃]-Pip |
| 1-358 | Me | 3-[Pipo-(CH₂)₃-O-CH₂]-Pip |
| 1-359 | Me | 3-[Pipo-(CH₂)₃-O-(CH₂)₂]-Pip |
| 1-360 | Me | 3-[Pipo-(CH₂)₃-O-(CH₂)₃]-Pip |
| 1-361 | Me | 3-[Pipo-(CH₂)₂-S-CH₂]-Pip |
| 1-362 | Me | 3-[(4-Me-Pipra)-(CH₂)₂-O-CH₂]-Pip |
| 1-363 | Me | 3-[(4-Me-Pipra)-(CH₂)₂-O-(CH₂)₂]-Pip |
| 1-364 | Me | 3-[(4-Me-Pipra)-(CH₂)₂-O-(CH₂)₃]-Pip |
| 1-365 | Me | 3-[(4-Me-Pipra)-(CH₂)₃-O-CH₂]-Pip |
| 1-366 | Me | 3-[(4-Me-Pipra)-(CH₂)₃-O-(CH₂)₂]-Pip |
| 1-367 | Me | 3-[(4-Me-Pipra)-(CH₂)₃-O-(CH₂)₃]-Pip |
| 1-368 | Me | 3-[(4-Me-Pipra)-(CH₂)₂-S-CH₂]-Pip |
| 1-369 | Me | 3-(Pyrld-CO-CH₂-O-CH₂)-Pip |
| 1-370 | Me | 3-[Pyrld-CO-CH₂-O-(CH₂)₂]-Pip |
| 1-371 | Me | 3-[Pyrld-CO-CH₂-O-(CH₂)₃]-Pip |
| 1-372 | Me | 3-(Mor-CO-CH₂-O-CH₂)-Pip |
| 1-373 | Me | 3-[Mor-CO-CH₂-O-(CH₂)₂]-Pip |
| 1-374 | Me | 3-[Mor-CO-CH₂-O-(CH₂)₃]-Pip |
| 1-375 | Me | 3-(Pipo-CO-CH₂-O-CH₂)-Pip |
| 1-376 | Me | 3-[Pipo-CO-CH₂-O-(CH₂)₂]-Pip |
| 1-377 | Me | 3-[Pipo-CO-CH₂-O-(CH₂)₃]-Pip |
| 1-378 | Me | 3-[(4-Me-Pipra)-CO-CH₂-O-CH₂]-Pip |
| 1-379 | Me | 3-[(4-Me-Pipra)-CH₂-O-(CH₂)₂]-Pip |
| 1-380 | Me | 3-[(4-Me-Pipra)-CH₂-O-(CH₂)₃]-Pip |

**Table 2**

| | | | |
|---|---|---|---|
| 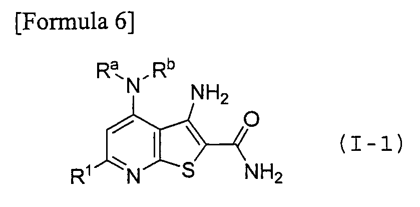 | | | |

| Compound Number | R¹ | R^{a} | R^{b} |
|---|---|---|---|
| 2-1 | H | H | Me |
| 2-2 | H | H | Et |
| 2-3 | H | H | Pr |
| 2-4 | H | H | iPr |
| 2-5 | H | H | Bu |
| 2-6 | H | H | iBu |
| 2-7 | H | H | neoPn |
| 2-8 | H | H | cPn |
| 2-9 | H | H | cHx |
| 2-10 | H | H | cHep |
| 2-11 | H | H | Bn |
| 2-12 | H | H | Phet |
| 2-13 | H | H | Ph-(CH₂)₃ |
| 2-14 | H | H | cHxCH₂ |
| 2-15 | H | H | MeOCH₂CH₂ |
| 2-16 | H | H | EtOCH₂CH₂ |
| 2-17 | H | Me | Me |
| 2-18 | H | Me | Et |
| 2-19 | H | Me | Pr |
| 2-20 | H | Me | iPr |
| 2-21 | H | Me | Bu |
| 2-22 | H | Me | iBu |
| 2-23 | H | Me | neoPn |
| 2-24 | H | Me | cPn |
| 2-25 | H | Me | cHx |
| 2-26 | H | Me | cHep |
| 2-27 | H | Me | Bn |
| 2-28 | H | Me | Phet |
| 2-29 | H | Me | Ph-(CH₂)₃ |
| 2-30 | H | Me | cHxCH₂ |
| 2-31 | H | Me | MeOCH₂CH₂ |
| 2-32 | H | Me | EtOCH₂CH₂ |
| 2-33 | H | Et | Et |
| 2-34 | H | Et | Pr |
| 2-35 | H | Et | iPr |
| 2-36 | H | Et | Bu |
| 2-37 | H | Et | iBu |
| 2-38 | H | Et | neoPn |
| 2-39 | H | Et | cPn |
| 2-40 | H | Et | cHx |
| 2-41 | H | Et | cHep |
| 2-42 | H | Et | Bn |
| 2-43 | H | Et | Phet |
| 2-44 | H | Et | Ph-(CH₂)₃ |
| 2-45 | H | Et | cHxCH₂ |
| 2-46 | H | Et | MeOCH₂CH₂ |
| 2-47 | H | Et | EtOCH₂CH₂ |
| 2-48 | H | Pr | Pr |
| 2-49 | H | Pr | iPr |
| 2-50 | H | Pr | Bu |
| 2-51 | H | Pr | iBu |
| 2-52 | H | Pr | neoPn |
| 2-53 | H | Pr | cPn |
| 2-54 | H | Pr | cHx |
| 2-55 | H | Pr | cHep |
| 2-56 | H | Pr | Bn |
| 2-57 | H | Pr | Phet |
| 2-58 | H | Pr | Ph-(CH₂)₃ |
| 2-59 | H | Pr | cHxCH₂ |
| 2-60 | H | Pr | MeOCH₂CH₂ |
| 2-61 | H | Pr | EtOCH₂CH₂ |
| 2-62 | H | MeOCH₂CH₂ | iPr |
| 2-63 | H | MeOCH₂CH₂ | Bu |
| 2-64 | H | MeOCH₂CH₂ | iBu |
| 2-65 | H | MeOCH₂CH₂ | neoPn |
| 2-66 | H | MeOCH₂CH₂ | cPn |
| 2-67 | H | MeOCH₂CH₂ | cHx |
| 2-68 | H | MeOCH₂CH₂ | cHep |
| 2-69 | H | MeOCH₂CH₂ | Bn |
| 2-70 | H | MeOCH₂CH₂ | Phet |
| 2-71 | H | MeOCH₂CH₂ | Ph-(CH₂)₃ |
| 2-72 | H | MeOCH₂CH₂ | cHxCH₂ |
| 2-73 | H | MeOCH₂CH₂ | MeOCH₂CH₂ |
| 2-74 | H | EtOCH₂CH₂ | iPr |
| 2-75 | H | EtOCH₂CH₂ | Bu |
| 2-76 | H | EtOCH₂CH₂ | iBu |
| 2-77 | H | EtOCH₂CH₂ | neoPn |
| 2-78 | H | EtOCH₂CH₂ | cPn |
| 2-79 | H | EtOCH₂CH₂ | cHx |
| 2-80 | H | EtOCH₂CH₂ | cHep |
| 2-81 | H | EtOCH₂CH₂ | Bn |
| 2-82 | H | EtOCH₂CH₂ | Phet |
| 2-83 | H | EtOCH₂CH₂ | Ph-(CH₂)₃ |
| 2-84 | H | EtOCH₂CH₂ | cHxCH₂ |
| 2-85 | H | EtOCH₂CH₂ | EtOCH₂CH₂ |
| 2-86 | Me | H | Me |
| 2-87 | Me | H | Et |
| 2-88 | Me | H | Pr |
| 2-89 | Me | H | iPr |
| 2-90 | Me | H | Bu |
| 2-91 | Me | H | iBu |
| 2-92 | Me | H | neoPn |
| 2-93 | Me | H | cPn |
| 2-94 | Me | H | cHx |
| 2-95 | Me | H | cHep |
| 2-96 | Me | H | Bn |
| 2-97 | Me | H | Phet |
| 2-98 | Me | H | Ph-(CH₂)₃ |
| 2-99 | Me | H | cHxCH₂ |
| 2-100 | Me | H | MeOCH₂CH₂ |
| 2-101 | Me | H | EtOCH₂CH₂ |
| 2-102 | Me | Me | Me |
| 2-103 | Me | Me | Et |
| 2-104 | Me | Me | Pr |
| 2-105 | Me | Me | iPr |
| 2-106 | Me | Me | Bu |
| 2-107 | Me | Me | iBu |
| 2-108 | Me | Me | neoPn |
| 2-109 | Me | Me | cPn |
| 2-110 | Me | Me | cHx |
| 2-111 | Me | Me | cHep |
| 2-112 | Me | Me | Bn |
| 2-113 | Me | Me | Phet |
| 2-114 | Me | Me | Ph-(CH₂)₃ |
| 2-115 | Me | Me | cHxCH₂ |
| 2-116 | Me | Me | MeOCH₂CH₂ |
| 2-117 | Me | Me | EtOCH₂CH₂ |
| 2-118 | Me | Et | Et |
| 2-119 | Me | Et | Pr |
| 2-120 | Me | Et | iPr |
| 2-121 | Me | Et | Bu |
| 2-122 | Me | Et | iBu |
| 2-123 | Me | Et | neoPn |
| 2-124 | Me | Et | cPn |
| 2-125 | Me | Et | cHx |
| 2-126 | Me | Et | cHep |
| 2-127 | Me | Et | Bn |
| 2-128 | Me | Et | Phet |
| 2-129 | Me | Et | Ph-(CH₂)₃ |
| 2-130 | Me | Et | cHxCH₂ |
| 2-131 | Me | Et | MeOCH₂CH₂ |
| 2-132 | Me | Et | EtOCH₂CH₂ |
| 2-133 | Me | Pr | Pr |
| 2-134 | Me | Pr | iPr |
| 2-135 | Me | Pr | Bu |
| 2-136 | Me | Pr | iBu |
| 2-137 | Me | Pr | neoPn |
| 2-138 | Me | Pr | cPn |
| 2-139 | Me | Pr | cHx |
| 2-140 | Me | Pr | cHep |
| 2-141 | Me | Pr | Bn |
| 2-142 | Me | Pr | Phet |
| 2-143 | Me | Pr | Ph-(CH₂)₃ |
| 2-144 | Me | Pr | cHxCH₂ |
| 2-145 | Me | Pr | MeOCH₂CH₂ |
| 2-146 | Me | Pr | EtOCH₂CH₂ |
| 2-147 | Me | MeOCH₂CH₂ | iPr |
| 2-148 | Me | MeOCH₂CH₂ | Bu |
| 2-149 | Me | MeOCH₂CH₂ | iBu |
| 2-150 | Me | MeOCH₂CH₂ | neoPn |
| 2-151 | Me | MeOCH₂CH₂ | cPn |
| 2-152 | Me | MeOCH₂CH₂ | cHx |
| 2-153 | Me | MeOCH₂CH₂ | cHep |
| 2-154 | Me | MeOCH₂CH₂ | Bn |
| 2-155 | Me | MeOCH₂CH₂ | Phet |
| 2-156 | Me | MeOCH₂CH₂ | Ph-(CH₂)₃ |
| 2-157 | Me | MeOCH₂CH₂ | cHxCH₂ |
| 2-158 | Me | MeOCH₂CH₂ | MeOCH₂CH₂ |
| 2-159 | Me | EtOCH₂CH₂ | iPr |
| 2-160 | Me | EtOCH₂CH₂ | Bu |
| 2-161 | Me | EtOCH₂CH₂ | iBu |
| 2-162 | Me | EtOCH₂CH₂ | neoPn |
| 2-163 | Me | EtOCH₂CH₂ | cPn |
| 2-164 | Me | EtOCH₂CH₂ | cHx |
| 2-165 | Me | EtOCH₂CH₂ | cHep |
| 2-166 | Me | EtOCH₂CH₂ | Bn |
| 2-167 | Me | EtOCH₂CH₂ | Phet |
| 2-168 | Me | EtOCH₂CH₂ | Ph-(CH₂)₃ |
| 2-169 | Me | EtOCH₂CH₂ | cHxCH₂ |
| 2-170 | Me | EtOCH₂CH₂ | EtOCH₂CH₂ |

**Table 3**

| | | | | |
|---|---|---|---|---|
| 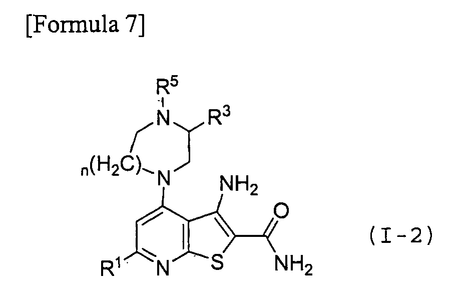 | | | | |

| Compound Number | R¹ | n | R³ | R⁵ |
|---|---|---|---|---|
| 3-1 | H | 1 | H | H |
| 3-2 | H | 1 | H | Me |
| 3-3 | H | 1 | H | Et |
| 3-4 | H | 1 | H | Pr |
| 3-5 | H | 1 | H | iPr |
| 3-6 | H | 1 | H | Bn |
| 3-7 | H | 1 | H | Bzhy |
| 3-8 | H | 1 | H | PhCH=CHCH₂ |
| 3-9 | H | 1 | H | Ph |
| 3-10 | H | 1 | H | 5-BzDioxo |
| 3-11 | H | 1 | H | 6-BzDioxa |
| 3-12 | H | 1 | H | 4-F-Ph |
| 3-13 | H | 1 | H | 3,4-diF-Ph |
| 3-14 | H | 1 | H | 3-Cl-4-F-Ph |
| 3-15 | H | 1 | H | 2-Cl-Ph |
| 3-16 | H | 1 | H | 3-Cl-Ph |
| 3-17 | H | 1 | H | 4-Cl-Ph |
| 3-18 | H | 1 | H | 4-Br-Ph |
| 3-19 | H | 1 | H | 3-NO₂-Ph |
| 3-20 | H | 1 | H | 4-NO₂-Ph |
| 3-21 | H | 1 | H | 4-CN-Ph |
| 3-22 | H | 1 | H | 4-Ac-Ph |
| 3-23 | H | 1 | H | 4-EtCO-Ph |
| 3-24 | H | 1 | H | 4-(HOOC)-Ph |
| 3-25 | H | 1 | H | 4-(MeOOC)-Ph |
| 3-26 | H | 1 | H | 4-(EtOOC)-Ph |
| 3-27 | H | 1 | H | 4-Me₂N-Ph |
| 3-28 | H | 1 | H | 4-(H₂NCO)-Ph |
| 3-29 | H | 1 | H | 4-(MeNHCO)-Ph |
| 3-30 | H | 1 | H | 4-(Me₂NCO)-Ph |
| 3-31 | H | 1 | H | 4-(Et₂NCO)-Ph |
| 3-32 | H | 1 | H | 2-Me-Ph |
| 3-33 | H | 1 | H | 3-Me-Ph |
| 3-34 | H | 1 | H | 4-Me-Ph |
| 3-35 | H | 1 | H | 4-Et-Ph |
| 3-36 | H | 1 | H | 4-Pr-Ph |
| 3-37 | H | 1 | H | 4-iPr-Ph |
| 3-38 | H | 1 | H | 4-tBu-Ph |
| 3-39 | H | 1 | H | 3,4-diMe-Ph |
| 3-40 | H | 1 | H | 3-F-4-Me-Ph |
| 3-41 | H | 1 | H | 4-F-3-Me-Ph |
| 3-42 | H | 1 | H | 3-NO₂-4-Me-Ph |
| 3-43 | H | 1 | H | 3-CF₃-Ph |
| 3-44 | H | 1 | H | 4-CF₃-Ph |
| 3-45 | H | 1 | H | 2-MeO-Ph |
| 3-46 | H | 1 | H | 3-MeO-Ph |
| 3-47 | H | 1 | H | 4-MeO-Ph |
| 3-48 | H | 1 | H | 3,4-diMeO-Ph |
| 3-49 | H | 1 | H | 3,4,5-triMeO-Ph |
| 3-50 | H | 1 | H | 4-EtO-Ph |
| 3-51 | H | 1 | H | 4-PrO-Ph |
| 3-52 | H | 1 | H | 4-iPrO-Ph |
| 3-53 | H | 1 | H | 4-CF₃O-Ph |
| 3-54 | H | 1 | H | 4-MeS-Ph |
| 3-55 | H | 1 | H | 4-MeSO-Ph |
| 3-56 | H | 1 | H | 4-MeSO₂-Ph |
| 3-57 | H | 1 | H | 4-BnO-Ph |
| 3-58 | H | 1 | H | 2-Oxaz |
| 3-59 | H | 1 | H | 2-Thaz |
| 3-60 | H | 1 | H | 2-BzOxaz |
| 3-61 | H | 1 | H | 2-BzThaz |
| 3-62 | H | 1 | H | 2-Py |
| 3-63 | H | 1 | H | 3-Py |
| 3-64 | H | 1 | H | 4-Py |
| 3-65 | H | 1 | H | 5-Ac-2-Py |
| 3-66 | H | 1 | H | 5-Me-2-Py |
| 3-67 | H | 1 | H | 6-MeO-3-Py |
| 3-68 | H | 1 | H | 2,3,5,6-tetraF-4-Py |
| 3-69 | H | 1 | H | 2-Pym |
| 3-70 | H | 1 | H | 4-Qunz |
| 3-71 | H | 1 | H | 6-Cl-3-Pydz |
| 3-72 | H | 1 | H | Ac |
| 3-73 | H | 1 | H | Ph-CO |
| 3-74 | H | 1 | H | MeSO₂ |
| 3-75 | H | 1 | H | tBuO-CO |
| 3-76 | Me | 1 | H | Ph |
| 3-77 | H | 1 | Me | 3-Me-Ph |
| 3-78 | H | 2 | H | H |
| 3-79 | H | 2 | H | Me |
| 3-80 | H | 2 | H | Et |
| 3-81 | H | 2 | H | Pr |
| 3-82 | H | 2 | H | iPr |
| 3-83 | H | 2 | H | Bn |
| 3-84 | H | 2 | H | Bzhy |
| 3-85 | H | 2 | H | PhCH=CHCH₂ |
| 3-86 | H | 2 | H | Ph |
| 3-87 | H | 2 | H | 5-BzDioxo |
| 3-88 | H | 2 | H | 6-BzDioxa |
| 3-89 | H | 2 | H | 4-F-Ph |
| 3-90 | H | 2 | H | 3,4-diF-Ph |
| 3-91 | H | 2 | H | 3-Cl-4-F-Ph |
| 3-92 | H | 2 | H | 2-Cl-Ph |
| 3-93 | H | 2 | H | 3-Cl-Ph |
| 3-94 | H | 2 | H | 4-Cl-Ph |
| 3-95 | H | 2 | H | 4-Br-Ph |
| 3-96 | H | 2 | H | 3-NO₂-Ph |
| 3-97 | H | 2 | H | 4-NO₂-Ph |
| 3-98 | H | 2 | H | 4-CN-Ph |
| 3-99 | H | 2 | H | 4-Ac-Ph |
| 3-100 | H | 2 | H | 4-EtCO-Ph |
| 3-101 | H | 2 | H | 4-(HOOC)-Ph |
| 3-102 | H | 2 | H | 4-(MeOOC)-Ph |
| 3-103 | H | 2 | H | 4-(EtOOC)-Ph |
| 3-104 | H | 2 | H | 4-Me₂N-Ph |
| 3-105 | H | 2 | H | 4-(H₂NCO)-Ph |
| 3-106 | H | 2 | H | 4-(MeNHCO)-Ph |
| 3-107 | H | 2 | H | 4-(Me₂NCO)-Ph |
| 3-108 | H | 2 | H | 4-(Et₂NCO)-Ph |
| 3-109 | H | 2 | H | 2-Me-Ph |
| 3-110 | H | 2 | H | 3-Me-Ph |
| 3-111 | H | 2 | H | 4-Me-Ph |
| 3-112 | H | 2 | H | 4-Et-Ph |
| 3-113 | H | 2 | H | 4-Pr-Ph |
| 3-114 | H | 2 | H | 4-iPr-Ph |
| 3-115 | H | 2 | H | 4-tBu-Ph |
| 3-116 | H | 2 | H | 3,4-diMe-Ph |
| 3-117 | H | 2 | H | 3-F-4-Me-Ph |
| 3-118 | H | 2 | H | 4-F-3-Me-Ph |
| 3-119 | H | 2 | H | 3-NO₂-4-Me-Ph |
| 3-120 | H | 2 | H | 3-CF₃-Ph |
| 3-121 | H | 2 | H | 4-CF₃-Ph |
| 3-122 | H | 2 | H | 2-MeO-Ph |
| 3-123 | H | 2 | H | 3-MeO-Ph |
| 3-124 | H | 2 | H | 4-MeO-Ph |
| 3-125 | H | 2 | H | 3,4-diMeO-Ph |
| 3-126 | H | 2 | H | 3,4,5-triMeO-Ph |
| 3-127 | H | 2 | H | 4-EtO-Ph |
| 3-128 | H | 2 | H | 4-PrO-Ph |
| 3-129 | H | 2 | H | 4-iPrO-Ph |
| 3-130 | H | 2 | H | 4-CF₃O-Ph |
| 3-131 | H | 2 | H | 4-MeS-Ph |
| 3-132 | H | 2 | H | 4-MeSO-Ph |
| 3-133 | H | 2 | H | 4-MeSO₂-Ph |
| 3-134 | H | 2 | H | 4-BnO-Ph |
| 3-135 | H | 2 | H | 2-Oxaz |
| 3-136 | H | 2 | H | 2-Thaz |
| 3-137 | H | 2 | H | 2-BzOxaz |
| 3-138 | H | 2 | H | 2-BzThaz |
| 3-139 | H | 2 | H | 2-Py |
| 3-140 | H | 2 | H | 3-Py |
| 3-141 | H | 2 | H | 4-Py |
| 3-142 | H | 2 | H | 5-Ac-2-Py |
| 3-143 | H | 2 | H | 5-Me-2-Py |
| 3-144 | H | 2 | H | 6-MeO-3-Py |
| 3-145 | H | 2 | H | 2,3,5,6-tetraF-4-Py |
| 3-146 | H | 2 | H | 2-Pym |
| 3-147 | H | 2 | H | 4-Qunz |
| 3-148 | H | 2 | H | 6-Cl-3-Pydz |
| 3-149 | H | 2 | H | Ac |
| 3-150 | H | 2 | H | Ph-CO |
| 3-151 | H | 2 | H | MeSO₂ |
| 3-152 | H | 2 | H | tBuO-CO |
| 3-153 | Me | 2 | H | H |
| 3-154 | Me | 2 | H | Me |
| 3-155 | Me | 2 | H | Et |
| 3-156 | Me | 2 | H | Pr |
| 3-157 | Me | 2 | H | iPr |
| 3-158 | Me | 2 | H | Bn |
| 3-159 | Me | 2 | H | Bzhy |
| 3-160 | Me | 2 | H | PhCH=CHCH₂ |
| 3-161 | Me | 2 | H | Ph |
| 3-162 | Me | 2 | H | 5-BzDioxo |
| 3-163 | Me | 2 | H | 6-BzDioxa |
| 3-164 | Me | 2 | H | 4-F-Ph |
| 3-165 | Me | 2 | H | 3,4-diF-Ph |
| 3-166 | Me | 2 | H | 3-Cl-4-F-Ph |
| 3-167 | Me | 2 | H | 2-Cl-Ph |
| 3-168 | Me | 2 | H | 3-Cl-Ph |
| 3-169 | Me | 2 | H | 4-Cl-Ph |
| 3-170 | Me | 2 | H | 4-Br-Ph |
| 3-171 | Me | 2 | H | 3-NO₂-Ph |
| 3-172 | Me | 2 | H | 4-NO₂-Ph |
| 3-173 | Me | 2 | H | 4-CN-Ph |
| 3-174 | Me | 2 | H | 4-Ac-Ph |
| 3-175 | Me | 2 | H | 4-EtCO-Ph |
| 3-176 | Me | 2 | H | 4-(HOOC)-Ph |
| 3-177 | Me | 2 | H | 4-(MeOOC)-Ph |
| 3-178 | Me | 2 | H | 4-(EtOOC)-Ph |
| 3-179 | Me | 2 | H | 4-Me₂N-Ph |
| 3-180 | Me | 2 | H | 4-(H₂NCO)-Ph |
| 3-181 | Me | 2 | H | 4-(MeNHCO)-Ph |
| 3-182 | Me | 2 | H | 4-(Me₂NCO)-Ph |
| 3-183 | Me | 2 | H | 4-(Et₂NCO)-Ph |
| 3-184 | Me | 2 | H | 2-Me-Ph |
| 3-185 | Me | 2 | H | 3-Me-Ph |
| 3-186 | Me | 2 | H | 4-Me-Ph |
| 3-187 | Me | 2 | H | 4-Et-Ph |
| 3-188 | Me | 2 | H | 4-Pr-Ph |
| 3-189 | Me | 2 | H | 4-iPr-Ph |
| 3-190 | Me | 2 | H | 4-tBu-Ph |
| 3-191 | Me | 2 | H | 3,4-diMe-Ph |
| 3-192 | Me | 2 | H | 3-F-4-Me-Ph |
| 3-193 | Me | 2 | H | 4-F-3-Me-Ph |
| 3-194 | Me | 2 | H | 3-NO₂-4-Me-Ph |
| 3-195 | Me | 2 | H | 3-CF₃-Ph |
| 3-196 | Me | 2 | H | 4-CF₃-Ph |
| 3-197 | Me | 2 | H | 2-MeO-Ph |
| 3-198 | Me | 2 | H | 3-MeO-Ph |
| 3-199 | Me | 2 | H | 4-MeO-Ph |
| 3-200 | Me | 2 | H | 3,4-diMeO-Ph |
| 3-201 | Me | 2 | H | 3,4,5-triMeO-Ph |
| 3-202 | Me | 2 | H | 4-EtO-Ph |
| 3-203 | Me | 2 | H | 4-PrO-Ph |
| 3-204 | Me | 2 | H | 4-iPrO-Ph |
| 3-205 | Me | 2 | H | 4-CF₃O-Ph |
| 3-206 | Me | 2 | H | 4-MeS-Ph |
| 3-207 | Me | 2 | H | 4-MeSO-Ph |
| 3-208 | Me | 2 | H | 4-MeSO₂-Ph |
| 3-209 | Me | 2 | H | 4-BnO-Ph |
| 3-210 | Me | 2 | H | 2-Oxaz |
| 3-211 | Me | 2 | H | 2-Thaz |
| 3-212 | Me | 2 | H | 2-BzOxaz |
| 3-213 | Me | 2 | H | 2-BzThaz |
| 3-214 | Me | 2 | H | 2-Py |
| 3-215 | Me | 2 | H | 3-Py |
| 3-216 | Me | 2 | H | 4-Py |
| 3-217 | Me | 2 | H | 5-Ac-2-Py |
| 3-218 | Me | 2 | H | 5-Me-2-Py |
| 3-219 | Me | 2 | H | 6-MeO-3-Py |
| 3-220 | Me | 2 | H | 2,3,5,6-tetraF-4-Py |
| 3-221 | Me | 2 | H | 2-Pym |
| 3-222 | Me | 2 | H | 4-Qunz |
| 3-223 | Me | 2 | H | 6-Cl-3-Pydz |
| 3-224 | Me | 2 | H | Ac |
| 3-225 | Me | 2 | H | Ph-CO |
| 3-226 | Me | 2 | H | MeSO₂ |
| 3-227 | Me | 2 | H | tBuO-CO |
| 3-228 | H | 2 | Me | H |
| 3-229 | H | 2 | Me | Me |
| 3-230 | H | 2 | Me | Et |
| 3-231 | H | 2 | Me | Pr |
| 3-232 | H | 2 | Me | iPr |
| 3-233 | H | 2 | Me | Bn |
| 3-234 | H | 2 | Me | Bzhy |
| 3-235 | H | 2 | Me | PhCH=CHCH₂ |
| 3-236 | H | 2 | Me | Ph |
| 3-237 | H | 2 | Me | 5-BzDioxo |
| 3-238 | H | 2 | Me | 6-BzDioxa |
| 3-239 | H | 2 | Me | 4-F-Ph |
| 3-240 | H | 2 | Me | 3,4-diF-Ph |
| 3-241 | H | 2 | Me | 3-Cl-4-F-Ph |
| 3-242 | H | 2 | Me | 2-Cl-Ph |
| 3-243 | H | 2 | Me | 3-Cl-Ph |
| 3-244 | H | 2 | Me | 4-Cl-Ph |
| 3-245 | H | 2 | Me | 4-Br-Ph |
| 3-246 | H | 2 | Me | 3-NO₂-Ph |
| 3-247 | H | 2 | Me | 4-NO₂-Ph |
| 3-248 | H | 2 | Me | 4-CN-Ph |
| 3-249 | H | 2 | Me | 4-Ac-Ph |
| 3-250 | H | 2 | Me | 4-EtCO-Ph |
| 3-251 | H | 2 | Me | 4-(HOOC)-Ph |
| 3-252 | H | 2 | Me | 4-(MeOOC)-Ph |
| 3-253 | H | 2 | Me | 4-(EtOOC)-Ph |
| 3-254 | H | 2 | Me | 4-Me₂N-Ph |
| 3-255 | H | 2 | Me | 4-(H₂NCO)-Ph |
| 3-256 | H | 2 | Me | 4-(MeNHCO)-Ph |
| 3-257 | H | 2 | Me | 4-(Me₂NCO)-Ph |
| 3-258 | H | 2 | Me | 4-(Et₂NCO)-Ph |
| 3-259 | H | 2 | Me | 2-Me-Ph |
| 3-260 | H | 2 | Me | 3-Me-Ph |
| 3-261 | H | 2 | Me | 4-Me-Ph |
| 3-262 | H | 2 | Me | 4-Et-Ph |
| 3-263 | H | 2 | Me | 4-Pr-Ph |
| 3-264 | H | 2 | Me | 4-iPr-Ph |
| 3-265 | H | 2 | Me | 4-tBu-Ph |
| 3-266 | H | 2 | Me | 3,4-diMe-Ph |
| 3-267 | H | 2 | Me | 3-F-4-Me-Ph |
| 3-268 | H | 2 | Me | 4-F-3-Me-Ph |
| 3-269 | H | 2 | Me | 3-NO₂-4-Me-Ph |
| 3-270 | H | 2 | Me | 3-CF₃-Ph |
| 3-271 | H | 2 | Me | 4-CF₃-Ph |
| 3-272 | H | 2 | Me | 2-MeO-Ph |
| 3-273 | H | 2 | Me | 3-MeO-Ph |
| 3-274 | H | 2 | Me | 4-MeO-Ph |
| 3-275 | H | 2 | Me | 3,4-diMeO-Ph |
| 3-276 | H | 2 | Me | 3,4,5-triMeO-Ph |
| 3-277 | H | 2 | Me | 4-EtO-Ph |
| 3-278 | H | 2 | Me | 4-PrO-Ph |
| 3-279 | H | 2 | Me | 4-iPrO-Ph |
| 3-280 | H | 2 | Me | 4-CF₃O-Ph |
| 3-281 | H | 2 | Me | 4-MeS-Ph |
| 3-282 | H | 2 | Me | 4-MeSO-Ph |
| 3-283 | H | 2 | Me | 4-MeSO₂-Ph |
| 3-284 | H | 2 | Me | 4-BnO-Ph |
| 3-285 | H | 2 | Me | 2-Oxaz |
| 3-286 | H | 2 | Me | 2-Thaz |
| 3-287 | H | 2 | Me | 2-BzOxaz |
| 3-288 | H | 2 | Me | 2-BzThaz |
| 3-289 | H | 2 | Me | 2-Py |
| 3-290 | H | 2 | Me | 3-Py |
| 3-291 | H | 2 | Me | 4-Py |
| 3-292 | H | 2 | Me | 5-Ac-2-Py |
| 3-293 | H | 2 | Me | 5-Me-2-Py |
| 3-294 | H | 2 | Me | 6-MeO-3-Py |
| 3-295 | H | 2 | Me | 2,3,5,6-tetraF-4-Py |
| 3-296 | H | 2 | Me | 2-Pym |
| 3-297 | H | 2 | Me | 4-Qunz |
| 3-298 | H | 2 | Me | 6-Cl-3-Pydz |
| 3-299 | H | 2 | Me | Ac |
| 3-300 | H | 2 | Me | Ph-CO |
| 3-301 | H | 2 | Me | MeSO₂ |
| 3-302 | H | 2 | Me | tBuO-CO |
| 3-303 | Me | 2 | Me | H |
| 3-304 | Me | 2 | Me | Me |
| 3-305 | Me | 2 | Me | Et |
| 3-306 | Me | 2 | Me | Pr |
| 3-307 | Me | 2 | Me | iPr |
| 3-308 | Me | 2 | Me | Bn |
| 3-309 | Me | 2 | Me | Bzhy |
| 3-310 | Me | 2 | Me | PhCH=CHCH₂ |
| 3-311 | Me | 2 | Me | Ph |
| 3-312 | Me | 2 | Me | 5-BzDioxo |
| 3-313 | Me | 2 | Me | 6-BzDioxa |
| 3-314 | Me | 2 | Me | 4-F-Ph |
| 3-315 | Me | 2 | Me | 3,4-diF-Ph |
| 3-316 | Me | 2 | Me | 3-Cl-4-F-Ph |
| 3-317 | Me | 2 | Me | 2-Cl-Ph |
| 3-318 | Me | 2 | Me | 3-Cl-Ph |
| 3-319 | Me | 2 | Me | 4-Cl-Ph |
| 3-320 | Me | 2 | Me | 4-Br-Ph |
| 3-321 | Me | 2 | Me | 3-NO₂-Ph |
| 3-322 | Me | 2 | Me | 4-NO₂-Ph |
| 3-323 | Me | 2 | Me | 4-CN-Ph |
| 3-324 | Me | 2 | Me | 4-Ac-Ph |
| 3-325 | Me | 2 | Me | 4-EtCO-Ph |
| 3-326 | Me | 2 | Me | 4-(HOOC)-Ph |
| 3-327 | Me | 2 | Me | 4-(MeOOC)-Ph |
| 3-328 | Me | 2 | Me | 4-(EtOOC)-Ph |
| 3-329 | Me | 2 | Me | 4-Me₂N-Ph |
| 3-330 | Me | 2 | Me | 4-(H₂NCO)-Ph |
| 3-331 | Me | 2 | Me | 4-(MeNHCO)-Ph |
| 3-332 | Me | 2 | Me | 4-(Me₂NCO)-Ph |
| 3-333 | Me | 2 | Me | 4-(Et₂NCO)-Ph |
| 3-334 | Me | 2 | Me | 2-Me-Ph |
| 3-335 | Me | 2 | Me | 3-Me-Ph |
| 3-336 | Me | 2 | Me | 4-Me-Ph |
| 3-337 | Me | 2 | Me | 4-Et-Ph |
| 3-338 | Me | 2 | Me | 4-Pr-Ph |
| 3-339 | Me | 2 | Me | 4-iPr-Ph |
| 3-340 | Me | 2 | Me | 4-tBu-Ph |
| 3-341 | Me | 2 | Me | 3,4-diMe-Ph |
| 3-342 | Me | 2 | Me | 3-F-4-Me-Ph |
| 3-343 | Me | 2 | Me | 4-F-3-Me-Ph |
| 3-344 | Me | 2 | Me | 3-NO₂-4-Me-Ph |
| 3-345 | Me | 2 | Me | 3-CF₃-Ph |
| 3-346 | Me | 2 | Me | 4-CF₃-Ph |
| 3-347 | Me | 2 | Me | 2-MeO-Ph |
| 3-348 | Me | 2 | Me | 3-MeO-Ph |
| 3-349 | Me | 2 | Me | 4-MeO-Ph |
| 3-350 | Me | 2 | Me | 3,4-diMeO-Ph |
| 3-351 | Me | 2 | Me | 3,4,5-triMeO-Ph |
| 3-352 | Me | 2 | Me | 4-EtO-Ph |
| 3-353 | Me | 2 | Me | 4-PrO-Ph |
| 3-354 | Me | 2 | Me | 4-iPrO-Ph |
| 3-355 | Me | 2 | Me | 4-CF₃O-Ph |
| 3-356 | Me | 2 | Me | 4-MeS-Ph |
| 3-357 | Me | 2 | Me | 4-MeSO-Ph |
| 3-358 | Me | 2 | Me | 4-MeSO₂-Ph |
| 3-359 | Me | 2 | Me | 4-BnO-Ph |
| 3-360 | Me | 2 | Me | 2-Oxaz |
| 3-361 | Me | 2 | Me | 2-Thaz |
| 3-362 | Me | 2 | Me | 2-BzOxaz |
| 3-363 | Me | 2 | Me | 2-BzThaz |
| 3-364 | Me | 2 | Me | 2-Py |
| 3-365 | Me | 2 | Me | 3-Py |
| 3-366 | Me | 2 | Me | 4-Py |
| 3-367 | Me | 2 | Me | 5-Ac-2-Py |
| 3-368 | Me | 2 | Me | 5-Me-2-Py |
| 3-369 | Me | 2 | Me | 6-MeO-3-Py |
| 3-370 | Me | 2 | Me | 2,3,5,6-tetraF-4-Py |
| 3-371 | Me | 2 | Me | 2-Pym |
| 3-372 | Me | 2 | Me | 4-Qunz |
| 3-373 | Me | 2 | Me | 6-Cl-3-Pydz |
| 3-374 | Me | 2 | Me | Ac |
| 3-375 | Me | 2 | Me | Ph-CO |
| 3-376 | Me | 2 | Me | MeSO₂ |
| 3-377 | Me | 2 | Me | tBuO-CO |
| 3-378 | H | 1 | H | 4-(MeO-CH₂)-Ph |
| 3-379 | H | 1 | H | 3-(MeO-CH₂)-Ph |
| 3-380 | H | 1 | H | 4-[MeO-(CH₂)₂]-Ph |
| 3-381 | H | 1 | H | 3-[MeO-(CH₂)₂]-Ph |
| 3-382 | H | 1 | H | 4-[MeO-(CH₂)₃]-Ph |
| 3-383 | H | 1 | H | 3-[MeO-(CH₂)₃]-Ph |
| 3-384 | H | 1 | H | 4-(EtO-CH₂)-Ph |
| 3-385 | H | 1 | H | 3-(EtO-CH₂)-Ph |
| 3-386 | H | 1 | H | 4-[EtO-(CH₂)₂]-Ph |
| 3-387 | H | 1 | H | 3-[EtO-(CH₂)₂]-Ph |
| 3-388 | H | 1 | H | 4-[EtO-(CH₂)₃]-Ph |
| 3-389 | H | 1 | H | 3-[EtO-(CH₂)₃]-Ph |
| 3-390 | H | 1 | H | 4-cPrO-Ph |
| 3-391 | H | 1 | H | 3-cPrO-Ph |
| 3-392 | H | 1 | H | 4-(cPrO-CH₂)-Ph |
| 3-393 | H | 1 | H | 3-(cPrO-CH₂)-Ph |
| 3-394 | H | 1 | H | 4-[cPrO-(CH₂)₂]-Ph |
| 3-395 | H | 1 | H | 3-[cPrO-(CH₂)₂]-Ph |
| 3-396 | H | 1 | H | 4-[cPrO-(CH₂)₃]-Ph |
| 3-397 | H | 1 | H | 3-[cPrO-(CH₂)₃]-Ph |
| 3-398 | H | 1 | H | 4-CHF₂O-Ph |
| 3-399 | H | 1 | H | 3-CHF₂O-Ph |
| 3-400 | H | 1 | H | 4-(CHF₂O-CH₂)-Ph |
| 3-401 | H | 1 | H | 3-(CHF₂O-CH₂)-Ph |
| 3-402 | H | 1 | H | 4-[CHF₂O-(CH₂)₂]-Ph |
| 3-403 | H | 1 | H | 3-[CHF₂O-(CH₂)₂]-Ph |
| 3-404 | H | 1 | H | 4-[CHF₂O-(CH₂)₃]-Ph |
| 3-405 | H | 1 | H | 3-[CHF₂O-(CH₂)₃]-Ph |
| 3-406 | H | 1 | H | 3-(H₂NCO)-Ph |
| 3-407 | H | 1 | H | 4-(H₂NCO-CH₂)-Ph |
| 3-408 | H | 1 | H | 3-(H₂NCO-CH₂)-Ph |
| 3-409 | H | 1 | H | 4-[H₂NCO-(CH₂)₂]-Ph |
| 3-410 | H | 1 | H | 3-[H₂NCO-(CH₂)₂]-Ph |
| 3-411 | H | 1 | H | 3-(MeNHCO)-Ph |
| 3-412 | H | 1 | H | 4-(MeNHCO-CH₂)-Ph |
| 3-413 | H | 1 | H | 3-(MeNHCO-CH₂)-Ph |
| 3-414 | H | 1 | H | 4-[MeNHCO-(CH₂)₂]-Ph |
| 3-415 | H | 1 | H | 3-[MeNHCO-(CH₂)₂]-Ph |
| 3-416 | H | 1 | H | 4-(iPrNHCO)-Ph |
| 3-417 | H | 1 | H | 3-(iPrNHCO)-Ph |
| 3-418 | H | 1 | H | 4-(iPrNHCO-CH₂)-Ph |
| 3-419 | H | 1 | H | 3-(iPrNHCO-CH₂)-Ph |
| 3-420 | H | 1 | H | 4-[iPrNHCO-(CH₂)₂]-Ph |
| 3-421 | H | 1 | H | 3-[iPrNHCO-(CH₂)₂]-Ph |
| 3-422 | H | 1 | H | 4-(EtNMeCO)-Ph |
| 3-423 | H | 1 | H | 3-(EtNMeCO)-Ph |
| 3-424 | H | 1 | H | 4-(EtNMeCO-CH₂)-Ph |
| 3-425 | H | 1 | H | 3-(EtNMeCO-CH₂)-Ph |
| 3-426 | H | 1 | H | 4-[EtNMeCO-(CH₂)₂]-Ph |
| 3-427 | H | 1 | H | 3-[EtNMeCO-(CH₂)₂]-Ph |
| 3-428 | H | 1 | H | 3-(Me₂NCO)-Ph |
| 3-429 | H | 1 | H | 4-(Me₂NCO-CH₂)-Ph |
| 3-430 | H | 1 | H | 3-(Me₂NCO-CH₂)-Ph |
| 3-431 | H | 1 | H | 4-[Me₂NCO-(CH₂)₂]-Ph |
| 3-432 | H | 1 | H | 3-[Me₂NCO-(CH₂)₂]-Ph |
| 3-433 | H | 1 | H | 3-(Et₂NCO)-Ph |
| 3-434 | H | 1 | H | 4-(Et₂NCO-CH₂)-Ph |
| 3-435 | H | 1 | H | 3-(Et₂NCO-CH₂)-Ph |
| 3-436 | H | 1 | H | 4-[Et₂NCO-(CH₂)₂]-Ph |
| 3-437 | H | 1 | H | 3-[Et₂NCO-(CH₂)₂]-Ph |
| 3-438 | H | 1 | H | 4-[(MeO)NMeCO]-Ph |
| 3-439 | H | 1 | H | 3-[(MeO)NMeCO]-Ph |
| 3-440 | H | 1 | H | 4-[(MeO)NMeCO-CH₂]-Ph |
| 3-441 | H | 1 | H | 3-[(MeO)NMeCO-CH₂]-Ph |
| 3-442 | H | 1 | H | 4-[(MeO)NMeCO-(CH₂)₂]-Ph |
| 3-443 | H | 1 | H | 3-[(MeO)NMeCO-(CH₂)₂]-Ph |
| 3-444 | H | 1 | H | 4-(Aze-CO)-Ph |
| 3-445 | H | 1 | H | 3-(Aze-CO)-Ph |
| 3-446 | H | 1 | H | 4-(Aze-CO-CH₂)-Ph |
| 3-447 | H | 1 | H | 3-(Aze-CO-CH₂)-Ph |
| 3-448 | H | 1 | H | 4-[Aze-CO-(CH₂)₂]-Ph |
| 3-449 | H | 1 | H | 3-[Aze-CO-(CH₂)₂]-Ph |
| 3-450 | H | 1 | H | 4-[(3-HO-1-Aze)-CO]-Ph |
| 3-451 1 | H | 1 | H | 3-[(3-HO-1-Aze)-CO]-Ph |
| 3-452 | H | 1 | H | 4-[(3-HO-Aze)-CO-CH₂]-Ph |
| 3-453 | H | 1 | H | 3-[(3-HO-Aze)-CO-CH₂]-Ph |
| 3-454 | H | 1 | H | 4-[(3-HO-Aze)-CO-(CH₂)₂]-Ph |
| 3-455 | H | 1 | H | 3-[(3-HO-Aze)-CO-(CH₂)₂]-Ph |
| 3-456 | H | 1 | H | 4-(Pyrld-CO)-Ph |
| 3-457 | H | 1 | H | 3-(Pyrld-CO)-Ph |
| 3-458 | H | 1 | H | 4-(Pyrld-CO-CH₂)-Ph |
| 3-459 | H | 1 | H | 3-(Pyrld-CO-CH₂)-Ph |
| 3-460 | H | 1 | H | 4-[Pyrld-CO-(CH₂)₂]-Ph |
| 3-461 | H | 1 | H | 3-[Pyrld-CO-(CH₂)₂]-Ph |
| 3-462 | H | 1 | H | 4-(Pipo-CO)-Ph |
| 3-463 | H | 1 | H | 3-(Pipo-CO)-Ph |
| 3-464 | H | 1 | H | 4-(Pipo-CO-CH₂)-Ph |
| 3-465 | H | 1 | H | 3-(Pipo-CO-CH₂)-Ph |
| 3-466 | H | 1 | H | 4-[Pipo-CO-(CH₂)₂]-Ph |
| 3-467 | H | 1 | H | 3-[Pipo-CO-(CH₂)₂]-Ph |
| 3-468 | H | 1 | H | 4-[(4-Me-Pipra)-CO]-Ph |
| 3-469 | H | 1 | H | 3-[(4-Me-Pipra)-CO]-Ph |
| 3-470 | H | 1 | H | 4-[(4-Me-Pipra)-CO-CH₂]-Ph |
| 3-471 | H | 1 | H | 3-[(4-Me-Pipra)-CO-CH₂]-Ph |
| 3-472 | H | 1 | H | 4-[(4-Me-Pipra)-CO-(CH₂)₂]-Ph |
| 3-473 | H | 1 | H | 3-[(4-Me-Pipra)-CO-(CH₂)₂]-Ph |
| 3-474 | H | 1 | H | 4-(Mor-CO)-Ph |
| 3-475 | H | 1 | H | 3-(Mor-CO)-Ph |
| 3-476 | H | 1 | H | 4-(Mor-CO-CH₂)-Ph |
| 3-477 | H | 1 | H | 3-(Mor-CO-CH₂)-Ph |
| 3-478 | H | 1 | H | 4-[Mor-CO-(CH₂)₂]-Ph |
| 3-479 | H | 1 | H | 3-[Mor-CO-(CH₂)₂]-Ph |
| 3-480 | H | 1 | H | 3-Me₂N-Ph |
| 3-481 | H | 1 | H | 4-(Me₂N-CH₂)-Ph |
| 3-482 | H | 1 | H | 3-(Me₂N-CH₂)-Ph |
| 3-483 | H | 1 | H | 4-[Me₂N-(CH₂)₂]-Ph |
| 3-484 | H | 1 | H | 3-[Me₂N-(CH₂)₂]-Ph |
| 3-485 | H | 1 | H | 4-[Me₂N-(CH₂)₃]-Ph |
| 3-486 | H | 1 | H | 3-[Me₂N-(CH₂)₃]-Ph |
| 3-487 | H | 1 | H | 4-Mor-Ph |
| 3-488 | H | 1 | H | 3-Mor-Ph |
| 3-489 | H | 1 | H | 4-(Mor-CH₂)-Ph |
| 3-490 | H | 1 | H | 3-(Mor-CH₂)-Ph |
| 3-491 | H | 1 | H | 4-[Mor-(CH₂)₂]-Ph |
| 3-492 | H | 1 | H | 3-[Mor-(CH₂)₂]-Ph |
| 3-493 | H | 1 | H | 4-[Mor-(CH₂)₃]-Ph |
| 3-494 | H | 1 | H | 3-[Mor-(CH₂)₃]-Ph |
| 3-495 | H | 1 | H | 4-Pipo-Ph |
| 3-496 | H | 1 | H | 3-Pipo-Ph |
| 3-497 | H | 1 | H | 4-(Pipo-CH₂)-Ph |
| 3-498 | H | 1 | H | 3-(Pipo-CH₂)-Ph |
| 3-499 | H | 1 | H | 4-[Pipo-(CH₂)₂]-Ph |
| 3-500 | H | 1 | H | 3-[Pipo-(CH₂)₂]-Ph |
| 3-501 | H | 1 | H | 4-[Pipo-(CH₂)₃]-Ph |
| 3-502 | H | 1 | H | 3-[Pipo-(CH₂)₃]-Ph |
| 3-503 | H | 1 | H | 4-HO-Ph |
| 3-504 | H | 1 | H | 3-HO-Ph |
| 3-505 | H | 1 | H | 4-(HO-CH₂)-Ph |
| 3-506 | H | 1 | H | 3-(HO-CH₂)-Ph |
| 3-507 | H | 1 | H | 4-[HO-(CH₂)₂]-Ph |
| 3-508 | H | 1 | H | 3-[HO-(CH₂)₂]-Ph |
| 3-509 | H | 1 | H | 4-[HO-(CH₂)₃]-Ph |
| 3-510 | H | 1 | H | 3-[HO-(CH₂)₃]-Ph |
| 3-511 | H | 1 | H | 4-[MeCH(OH)]-Ph |
| 3-512 | H | 1 | H | 3-[MeCH(OH)]-Ph |
| 3-513 | H | 1 | H | 4-[MeCH(OH)-CH₂]-Ph |
| 3-514 | H | 1 | H | 3-[MeCH(OH)-CH₂]-Ph |
| 3-515 | H | 1 | H | 4-[MeCH(OH)-(CH₂)₂]-Ph |
| 3-516 | H | 1 | H | 3-[MeCH(OH)-(CH₂)₂]-Ph |
| 3-517 | H | 1 | H | 3-CN-Ph |
| 3-518 | H | 1 | H | 4-(CN-CH₂)-Ph |
| 3-519 | H | 1 | H | 3-(CN-CH₂)-Ph |
| 3-520 | H | 1 | H | 4-[CN-(CH₂)₂]-Ph |
| 3-521 | H | 1 | H | 3-[CN-(CH₂)₂]-Ph |
| 3-522 | H | 1 | H | 3-Ac-Ph |
| 3-523 | H | 1 | H | 4-(Ac-CH₂)-Ph |
| 3-524 | H | 1 | H | 3-(Ac-CH₂)-Ph |
| 3-525 | H | 1 | H | 4-[Ac-(CH₂)₂]-Ph |
| 3-526 | H | 1 | H | 3-[Ac-(CH₂)₂]-Ph |
| 3-527 | H | 1 | H | 4-(CF₃CO)-Ph |
| 3-528 | H | 1 | H | 4-(EtCO)-Ph |
| 3-529 | H | 1 | H | 3-(EtCO)-Ph |
| 3-530 | H | 1 | H | 4-(EtCO-CH₂)-Ph |
| 3-531 | H | 1 | H | 3-(EtCO-CH₂)-Ph |
| 3-532 | H | 1 | H | 4-[EtCO-(CH₂)₂]-Ph |
| 3-533 | H | 1 | H | 3-[EtCO-(CH₂)₂]-Ph |
| 3-534 | H | 1 | H | 4-(iPrCO)-Ph |
| 3-535 | H | 1 | H | 4-(cBuCO)-Ph |
| 3-536 | H | 1 | H | 4-(cPrCO)-Ph |
| 3-537 | H | 1 | H | 4-(Ph-CO)-Ph |
| 3-538 | H | 1 | H | 4-Ac-3-MeO-Ph |
| 3-539 | H | 1 | H | 4-Ac-3-OH-Ph |
| 3-540 | H | 1 | H | 4-Ac-3-Cl-Ph |
| 3-541 | H | 1 | H | 4-[CH₃C(=N-OH)]-Ph |
| 3-542 | H | 1 | H | 3-[CH₃C(=N-OH)]-Ph |
| 3-543 | H | 1 | H | 4-[CH₃C(=N-OH)-CH₂]-Ph |
| 3-544 | H | 1 | H | 3-[CH₃C(=N-OH)-CH₂]-Ph |
| 3-545 | H | 1 | H | 4-[CH₃C(=N-OH)-(CH₂)₂]-Ph |
| 3-546 | H | 1 | H | 3-[CH₃C(=N-OH)-(CH₂)₂]-Ph |
| 3-547 | H | 1 | H | 4-[CH₃C(=N-OMe)]-Ph |
| 3-548 | H | 1 | H | 3-[CH₃C(=N-OMe)]-Ph |
| 3-549 | H | 1 | H | 4-[CH₃C(=N-OMe)-CH₂]-Ph |
| 3-550 | H | 1 | H | 3-[CH₃C(=N-OMe)-CH₂]-Ph |
| 3-551 | H | 1 | H | 4-[CH₃C(=N-OMe)-(CH₂)₂]-Ph |
| 3-552 | H | 1 | H | 3-[CH₃C(=N-OMe)-(CH₂)₂]-Ph |
| 3-553 | H | 1 | H | 4-(Me₂NSO₂)-P |
| 3-554 | H | 1 | H | 4-[(MeO)₂CH]-Ph |
| 3-555 | H | 1 | H | 3-[(MeO)₂CH]-Ph |
| 3-556 | H | 1 | H | 4-[(MeO)₂CH-CH₂]-Ph |
| 3-557 | H | 1 | H | 3-[(MeO)₂CH-CH₂]-Ph |
| 3-558 | H | 1 | H | 4-[(MeO)₂CH-(CH₂)₂]-Ph |
| 3-559 | H | 1 | H | 3-[(MeO)₂CH-(CH₂)₂]-Ph |
| 3-560 | H | 1 | H | 4-[Me(MeO)₂C]-Ph |
| 3-561 | H | 1 | H | 3-[Me(MeO)₂C]-Ph |
| 3-562 | H | 1 | H | 4-[Me(MeO)₂C-CH₂]-Ph |
| 3-563 | H | 1 | H | 3-[Me(MeO)₂C-CH₂]-Ph |
| 3-564 | H | 1 | H | 4-[Me(MeO)₂C-(CH₂)₂]-Ph |
| 3-565 | H | 1 | H | 3-[Me(MeO)₂C-(CH₂)₂]-Ph |
| 3-566 | H | 1 | H | 4-[(EtO)₂CH]-Ph |
| 3-567 | H | 1 | H | 3-[(EtO)₂CH]-Ph |
| 3-568 | H | 1 | H | 4-[(EtO)₂CH-CH_{2]}-Ph |
| 3-569 | H | 1 | H | 3-[(EtO)₂CH-CH_{2]}-Ph |
| 3-570 | H | 1 | H | 4-[(EtO)₂CH-(CH₂)₂]-Ph |
| 3-571 | H | 1 | H | 3-[(EtO)₂CH-(CH₂)₂]-Ph |
| 3-572 | H | 1 | H | 4-[Me(EtO)₂C]-Ph |
| 3-573 | H | 1 | H | 3-[Me(EtO)₂C]-Ph |
| 3-574 | H | 1 | H | 4-[Me(EtO)₂C-CH₂]-Ph |
| 3-575 | H | 1 | H | 3-[Me(EtO)₂C-CH₂]-Ph |
| 3-576 | H | 1 | H | 4-[Me(EtO)₂C-(CH₂)₂]-Ph |
| 3-577 | H | 1 | H | 3-[Me(EtO)₂C-(CH₂)₂]-Ph |
| 3-578 | H | 1 | H | 4-(2-Dioxo)-Ph |
| 3-579 | H | 1 | H | 3-(2-Dioxo)-Ph |
| 3-580 | H | 1 | H | 4-[(2-Dioxo)-CH₂]-Ph |
| 3-581 | H | 1 | H | 3-[(2-Dioxo)-CH₂]-Ph |
| 3-582 | H | 1 | H | 4-[(2-Dioxo)-(CH₂)₂]-Ph |
| 3-583 | H | 1 | H | 3-[(2-Dioxo)-(CH₂)₂]-Ph |
| 3-584 | H | 1 | H | 4-[2-Me-(2-Dioxo)]-Ph |
| 3-585 | H | 1 | H | 3-[2-Me-(2-Dioxo)]-Ph |
| 3-586 | H | 1 | H | 4-[2-Me-(2-Dioxo)-CH₂]-Ph |
| 3-587 | H | 1 | H | 3-[2-Me-(2-Dioxo)-CH₂]-Ph |
| 3-588 | H | 1 | H | 4-[2-Me-(2-Dioxo)-(CH₂)₂]-Ph |
| 3-589 | H | 1 | H | 3-[2-Me-(2-Dioxo)-(CH₂)₂]-Ph |
| 3-590 | H | 1 | H | 4-(2-Dioxa)-Ph |
| 3-591 | H | 1 | H | 3-(2-Dioxa)-Ph |
| 3-592 | H | 1 | H | 4-[(2-Dioxa)-CH₂]-Ph |
| 3-593 | H | 1 | H | 3-[(2-Dioxa)-CH₂]-Ph |
| 3-594 | H | 1 | H | 4-[(2-Dioxa)-(CH₂)₂]-Ph |
| 3-595 | H | 1 | H | 3-[(2-Dioxa)-(CH₂)₂]-Ph |
| 3-596 | H | 1 | H | 4-[2-Me-(2-Dioxa)]-Ph |
| 3-597 | H | 1 | H | 3-[2-Me-(2-Dioxa)]-Ph |
| 3-598 | H | 1 | H | 4-[2-Me-(2-Dioxa)-CH₂]-Ph |
| 3-599 | H | 1 | H | 3-[2-Me-(2-Dioxa)-CH₂]-Ph |
| 3-600 | H | 1 | H | 4-[2-Me-(2-Dioxa)-(CH₂)₂]-Ph |
| 3-601 | H | 1 | H | 3-[2-Me-(2-Dioxa)-(CH₂)₂]-Ph |
| 3-602 | H | 1 | H | 2-Me-1-oxo-5-IIndn |
| 3-603 | H | 1 | H | 6-BzOxaz |
| 3-604 | H | 1 | H | 4-(HO-N=)-7-Chr |
| 3-605 | H | 1 | H | 3-Me-6-BzIox |
| 3-606 | H | 1 | H | 2-Me-6-BzOxaz |
| 3-607 | H | 1 | H | 2-Me-5-BzOxaz |
| 3-608 | H | 1 | H | 2,3-dihydro-5-BzFur |
| 3-609 | H | 1 | H | 6-Qui |
| 3-610 | H | 1 | H | 6-Iqui |
| 3-611 | H | 1 | H | 3-(HO-N=)-2,3-dihydro-6-BzFur |
| 3-612 | H | 1 | H | 2-Me-6-BzThaz |
| 3-613 | H | 1 | H | 5-Ind |
| 3-614 | H | 1 | H | 4-Ac-2-Thaz |
| 3-615 | H | 1 | H | 5-Ac-2-Thi |
| 3-616 | H | 1 | H | 5-Ac-2-Fur |
| 3-617 | H | 1 | H | 5-Me₂NCO-2-Py |
| 3-618 | H | 1 | H | 5-(Me₂NCO-CH₂)-2-Py |
| 3-619 | H | 1 | H | 5-[Me₂NCO-(CH₂)₂]-2-Py |
| 3-620 | H | 1 | H | 4-Me₂NCO-2-Thaz |
| 3-621 | H | 1 | H | 5-Me₂NCO-2-Thaz |
| 3-622 | H | 1 | H | 4-(Me₂NCO-CH₂)-2-Thaz |
| 3-623 | H | 1 | H | 5-(Me₂NCO-CH₂)-2-Thaz |
| 3-624 | H | 1 | H | 2-Thazn |
| 3-625 | H | 1 | H | 2-Oxazn |
| 3-626 | Me | 1 | H | 4-(MeO-CH₂)-Ph |
| 3-627 | Me | 1 | H | 3-(MeO-CH₂)-Ph |
| 3-628 | Me | 1 | H | 4-[MeO-(CH₂)₂]-Ph |
| 3-629 | Me | 1 | H | 3-[MeO-(CH₂)₂]-Ph |
| 3-630 | Me | 1 | H | 4-[MeO-(CH₂)₃]-Ph |
| 3-631 | Me | 1 | H | 3-[MeO-(CH₂)₃]-Ph |
| 3-632 | Me | 1 | H | 4-(EtO-CH₂)-Ph |
| 3-633 | Me | 1 | H | 3-(EtO-CH₂)-Ph |
| 3-634 | Me | 1 | H | 4-[EtO-(CH₂)₂]-Ph |
| 3-635 | Me | 1 | H | 3-[EtO-(CH₂)₂]-Ph |
| 3-636 | Me | 1 | H | 4-[EtO-(CH₂)₃]-Ph |
| 3-637 | Me | 1 | H | 3-[EtO-(CH₂)₃]-Ph |
| 3-638 | Me | 1 | H | 4-cPrO-Ph |
| 3-639 | Me | 1 | H | 3-cPrO-Ph |
| 3-640 | Me | 1 | H | 4-(cPrO-CH₂)-Ph |
| 3-641 | Me | 1 | H | 3-(cPrO-CH₂)-Ph |
| 3-642 | Me | 1 | H | 4-[cPrO-(CH₂)₂]-Ph |
| 3-643 | Me | 1 | H | 3-[cPrO-(CH₂)₂]-Ph |
| 3-644 | Me | 1 | H | 4-[cPrO-(CH₂)₃]-Ph |
| 3-645 | Me | 1 | H | 3-[cPrO-(CH₂)₃]-Ph |
| 3-646 | Me | 1 | H | 4-CHF₂O-Ph |
| 3-647 | Me | 1 | H | 3-CHF₂O-Ph |
| 3-648 | Me | 1 | H | 4-(CHF₂O-CH₂)-Ph |
| 3-649 | Me | 1 | H | 3-(CHF₂O-CH₂)-Ph |
| 3-650 | Me | 1 | H | 4-[CHF₂O-(CH₂)₂]-Ph |
| 3-651 | Me | 1 | H | 3-[CHF₂O-(CH₂)₂]-Ph |
| 3-652 | Me | 1 | H | 4-[CHF₂O-(CH₂)₃]-Ph |
| 3-653 | Me | 1 | H | 3-[CHF₂O-(CH₂)₃]-Ph |
| 3-654 | Me | 1 | H | 3-(H₂NCO)-Ph |
| 3-655 | Me | 1 | H | 4-(H₂NCO-CH₂)-Ph |
| 3-656 | Me | 1 | H | 3-(H₂NCO-CH₂)-Ph |
| 3-657 | Me | 1 | H | 4-[H₂NCO-(CH₂)₂]-Ph |
| 3-658 | Me | 1 | H | 3-[H₂NCO-(CH₂)₂]-Ph |
| 3-659 | Me | 1 | H | 3-(MeNHCO)-Ph |
| 3-660 | Me | 1 | H | 4-(MeNHCO-CH₂)-Ph |
| 3-661 | Me | 1 | H | 3-(MeNHCO-CH₂)-Ph |
| 3-662 | Me | 1 | H | 4-[MeNHCO-(CH₂)₂]-Ph |
| 3-663 | Me | 1 | H | 3-[MeNHCO-(CH₂)₂]-Ph |
| 3-664 | Me | 1 | H | 4-(iPrNHCO)-Ph |
| 3-665 | Me | 1 | H | 3-(iPrNHCO)-Ph |
| 3-666 | Me | 1 | H | 4-(iPrNHCO-CH₂)-Ph |
| 3-667 | Me | 1 | H | 3-(iPrNHCO-CH₂)-Ph |
| 3-668 | Me | 1 | H | 4-[iPrNHCO-(CH₂)₂]-Ph |
| 3-669 | Me | 1 | H | 3-[iPrNHCO-(CH₂)₂]-Ph |
| 3-670 | Me | 1 | H | 4-(EtNMeCO)-Ph |
| 3-671 | Me | 1 | H | 3-(EtNMeCO)-Ph |
| 3-672 | Me | 1 | H | 4-(EtNMeCO-CH₂)-Ph |
| 3-673 | Me | 1 | H | 3-(EtNMeCO-CH₂)-Ph |
| 3-674 | Me | 1 | H | 4-[EtNMeCO-(CH₂)₂]-Ph |
| 3-675 | Me | 1 | H | 3-[EtNMeCO-(CH₂)₂]-Ph |
| 3-676 | Me | 1 | H | 3-(Me₂NCO)-Ph |
| 3-677 | Me | 1 | H | 4-(Me₂NCO-CH₂)-Ph |
| 3-678 | Me | 1 | H | 3-(Me₂NCO-CH₂)-Ph |
| 3-679 | Me | 1 | H | 4-[Me₂NCO-(CH₂)₂]-Ph |
| 3-680 | Me | 1 | H | 3-[Me₂NCO-(CH₂)₂]-Ph |
| 3-681 | Me | 1 | H | 3-(Et₂NCO)-Ph |
| 3-682 | Me | 1 | H | 4-(Et₂NCO-CH₂)-Ph |
| 3-683 | Me | 1 | H | 3-(Et₂NCO-CH₂)-Ph |
| 3-684 | Me | 1 | H | 4-[Et₂NCO-(CH₂)₂]-Ph |
| 3-685 | Me | 1 | H | 3-[Et₂NCO-(CH₂)₂]-Ph |
| 3-686 | Me | 1 | H | 4-[(MeO)NMeCO]-Ph |
| 3-687 | Me | 1 | H | 3-[(MeO)NMeCO]-Ph |
| 3-688 | Me | 1 | H | 4-[(MeO)NMeCO-CH₂]-Ph |
| 3-689 | Me | 1 | H | 3-[(MeO)NMeCO-CH₂]-Ph |
| 3-690 | Me | 1 | H | 4-[(MeO)NMeCO-(CH₂)₂]-Ph |
| 3-691 | Me | 1 | H | 3-[(MeO)NMeCO-(CH₂)₂]-Ph |
| 3-692 | Me | 1 | H | 4-(Aze-CO)-Ph |
| 3-693 | Me | 1 | H | 3-(Aze-CO)-Ph |
| 3-694 | Me | 1 | H | 4-(Aze-CO-CH₂)-Ph |
| 3-695 | Me | 1 | H | 3-(Aze-CO-CH₂)-Ph |
| 3-696 | Me | 1 | H | 4-[Aze-CO-(CH₂)₂]-Ph |
| 3-697 | Me | 1 | H | 3-[Aze-CO-(CH₂)₂]-Ph |
| 3-698 | Me | 1 | H | 4-[(3-HO-1-Aze)-CO]-Ph |
| 3-699 | Me | 1 | H | 3-[(3-HO-1-Aze)-CO]-Ph |
| 3-700 | Me | 1 | H | 4-[(3-HO-Aze)-CO-CH₂]-Ph |
| 3-701 | Me | 1 | H | 3-[(3-HO-Aze)-CO-CH₂]-Ph |
| 3-702 | Me | 1 | H | 4-[(3-HO-Aze)-CO-(CH₂)₂]-Ph |
| 3-703 | Me | 1 | H | 3-[(3-HO-Aze)-CO-(CH₂)₂]-Ph |
| 3-704 | Me | 1 | H | 4-(Pyrld-CO)-Ph |
| 3-705 | Me | 1 | H | 3-(Pyrld-CO)-Ph |
| 3-706 | Me | 1 | H | 4-(Pyrld-CO-CH₂)-Ph |
| 3-707 | Me | 1 | H | 3-(Pyrld-CO-CH₂)-Ph |
| 3-708 | Me | 1 | H | 4-[Pyrld-CO-(CH₂)₂]-Ph |
| 3-709 | Me | 1 | H | 3-[Pyrld-CO-(CH₂)₂]-Ph |
| 3-710 | Me | 1 | H | 4-(Pipo-CO)-Ph |
| 3-711 | Me | 1 | H | 3-(Pipo-CO)-Ph |
| 3-712 | Me | 1 | H | 4-(Pipo-CO-CH₂)-Ph |
| 3-713 | Me | 1 | H | 3-(Pipo-CO-CH₂)-Ph |
| 3-714 | Me | 1 | H | 4-[Pipo-CO-(CH₂)₂]-Ph |
| 3-715 | Me | 1 | H | 3-[Pipo-CO-(CH₂)₂]-Ph |
| 3-716 | Me | 1 | H | 4-[(4-Me-Pipra)-CO]-Ph |
| 3-717 | Me | 1 | H | 3-[(4-Me-Pipra)-CO]-Ph |
| 3-718 | Me | 1 | H | 4-[(4-Me-Pipra)-CO-CH₂]-Ph |
| 3-719 | Me | 1 | H | 3-[(4-Me-Pipra)-CO-CH₂]-Ph |
| 3-720 | Me | 1 | H | 4-[(4-Me-Pipra)-CO-(CH₂)₂]-Ph |
| 3-721 | Me | 1 | H | 3-[(4-Me-Pipra)-CO-(CH₂)₂]-Ph |
| 3-722 | Me | 1 | H | 4-(Mor-CO)-Ph |
| 3-723 | Me | 1 | H | 3-(Mor-CO)-Ph |
| 3-724 | Me | 1 | H | 4-(Mor-CO-CH₂)-Ph |
| 3-725 | Me | 1 | H | 3-(Mor-CO-CH₂)-Ph |
| 3-726 | Me | 1 | H | 4-[Mor-CO-(CH₂)₂]-Ph |
| 3-727 | Me | 1 | H | 3-[Mor-CO-(CH₂)₂]-Ph |
| 3-728 | Me | 1 | H | 3-Me₂N-Ph |
| 3-729 | Me | 1 | H | 4-(Me₂N-CH₂)-Ph |
| 3-730 | Me | 1 | H | 3-(Me₂N-CH₂)-Ph |
| 3-731 | Me | 1 | H | 4-[Me₂N-(CH₂)₂]-Ph |
| 3-732 | Me | 1 | H | 3-[Me₂N-(CH₂)₂]-Ph |
| 3-733 | Me | 1 | H | 4-[Me₂N-(CH₂)₃]-Ph |
| 3-734 | Me | 1 | H | 3-[Me₂N-(CH₂)₃]-Ph |
| 3-735 | Me | 1 | H | 4-Mor-Ph |
| 3-736 | Me | 1 | H | 3-Mor-Ph |
| 3-737 | Me | 1 | H | 4-(Mor-CH₂)-Ph |
| 3-738 | Me | 1 | H | 3-(Mor-CH₂)-Ph |
| 3-739 | Me | 1 | H | 4-[Mor-(CH₂)₂]-Ph |
| 3-740 | Me | 1 | H | 3-[Mor-(CH₂)₂]-Ph |
| 3-741 | Me | 1 | H | 4-[Mor-(CH₂)₃]-Ph |
| 3-742 | Me | 1 | H | 3-[Mor-(CH₂)₃]-Ph |
| 3-743 | Me | 1 | H | 4-Pipo-Ph |
| 3-744 | Me | 1 | H | 3-Pipo-Ph |
| 3-745 | Me | 1 | H | 4-(Pipo-CH₂)-Ph |
| 3-746 | Me | 1 | H | 3-(Pipo-CH₂)-Ph |
| 3-747 | Me | 1 | H | 4-[Pipo-(CH₂)_{2]}-Ph |
| 3-748 | Me | 1 | H | 3-[Pipo-(CH₂)₂]-Ph |
| 3-749 | Me | 1 | H | 4-[Pipo-(CH₂)₃]-Ph |
| 3-750 | Me | 1 | H | 3-[Pipo-(CH₂)₃]-Ph |
| 3-751 | Me | 1 | H | 4-HO-Ph |
| 3-752 | Me | 1 | H | 3-HO-Ph |
| 3-753 | Me | 1 | H | 4-(HO-CH₂)-Ph |
| 3-754 | Me | 1 | H | 3-(HO-CH₂)-Ph |
| 3-755 | Me | 1 | H | 4-[HO-(CH₂)₂]-Ph |
| 3-756 | Me | 1 | H | 3-[HO-(CH₂)₂]-Ph |
| 3-757 | Me | 1 | H | 4-[HO-(CH₂)₃]-Ph |
| 3-758 | Me | 1 | H | 3-[HO-(CH₂)₃]-Ph |
| 3-759 | Me | 1 | H | 4-[MeCH(OH)]-Ph |
| 3-760 | Me | 1 | H | 3-[MeCH(OH)]-Ph |
| 3-761 | Me | 1 | H | 4-[MeCH(OH)-CH₂]-Ph |
| 3-762 | Me | 1 | H | 3-[MeCH(OH)-CH₂]-Ph |
| 3-763 | Me | 1 | H | 4-[MeCH(OH)-(CH₂)₂]-Ph |
| 3-764 | Me | 1 | H | 3-[MeCH(OH)-(CH₂)₂]-Ph |
| 3-765 | Me | 1 | H | 3-CN-Ph |
| 3-766 | Me | 1 | H | 4-(CN-CH₂)-Ph |
| 3-767 | Me | 1 | H | 3-(CN-CH₂)-Ph |
| 3-768 | Me | 1 | H | 4-[CN-(CH₂)₂]-Ph |
| 3-769 | Me | 1 | H | 3-[CN-(CH₂)₂]-Ph |
| 3-770 | Me | 1 | H | 3-Ac-Ph |
| 3-771 | Me | 1 | H | 4-(Ac-CH₂)-Ph |
| 3-772 | Me | 1 | H | 3-(Ac-CH₂)-Ph |
| 3-773 | Me | 1 | H | 4-[Ac-(CH₂)₂]-Ph |
| 3-774 | Me | 1 | H | 3-[Ac-(CH₂)₂]-Ph |
| 3-775 | Me | 1 | H | 4-(CF₃CO)-Ph |
| 3-776 | Me | 1 | H | 4-(EtCO)-Ph |
| 3-777 | Me | 1 | H | 3-(EtCO)-Ph |
| 3-778 | Me | 1 | H | 4-(EtCO-CH₂)-Ph |
| 3-779 | Me | 1 | H | 3-(EtCO-CH₂)-Ph |
| 3-780 | Me | 1 | H | 4-[EtCO-(CH₂)₂]-Ph |
| 3-781 | Me | 1 | H | 3-[EtCO-(CH₂)₂]-Ph |
| 3-782 | Me | 1 | H | 4-(iPrCO)-Ph |
| 3-783 | Me | 1 | H | 4-(cBuCO)-Ph |
| 3-784 | Me | 1 | H | 4-(cPrCO)-Ph |
| 3-785 | Me | 1 | H | 4-(Ph-CO)-Ph |
| 3-786 | Me | 1 | H | 4-Ac-3-MeO-Ph |
| 3-787 | Me | 1 | H | 4-Ac-3-OH-Ph |
| 3-788 | Me | 1 | H | 4-Ac-3-Cl-Ph |
| 3-789 | Me | 1 | H | 4-[CH₃C(=N-OH)]-Ph |
| 3-790 | Me | 1 | H | 3-[CH₃C(=N-OH)]-Ph |
| 3-791 | Me | 1 | H | 4-[CH₃C(=N-OH)-CH₂]-Ph |
| 3-792 | Me | 1 | H | 3-[CH₃C(=N-OH)-CH₂]-Ph |
| 3-793 | Me | 1 | H | 4-[CH₃C(=N-OH)-(CH₂)₂]-Ph |
| 3-794 | Me | 1 | H | 3-[CH₃C(=N-OH)-(CH₂)₂]-Ph |
| 3-795 | Me | 1 | H | 4-[CH₃C(=N-OMe)]-Ph |
| 3-796 | Me | 1 | H | 3-[CH₃C(=N-OMe)]-Ph |
| 3-797 | Me | 1 | H | 4-[CH₃C(=N-OMe)-CH₂]-Ph |
| 3-798 | Me | 1 | H | 3-[CH₃C(=N-OMe)-CH₂]-Ph |
| 3-799 | Me | 1 | H | 4-[CH₃C(=N-OMe)-(CH₂)₂]-Ph |
| 3-800 | Me | 1 | H | 3-[CH₃C(=N-OMe)-(CH₂)₂]-Ph |
| 3-801 | Me | 1 | H | 4-(Me₂NSO₂)-P |
| 3-802 | Me | 1 | H | 4-[(MeO)₂CH]-Ph |
| 3-803 | Me | 1 | H | 3-[(MeO)₂CH]-Ph |
| 3-804 | Me | 1 | H | 4-[(MeO)₂CH-CH₂]-Ph |
| 3-805 | Me | 1 | H | 3-[(MeO)₂CH-CH₂]-Ph |
| 3-806 | Me | 1 | H | 4-[(MeO)₂CH-(CH₂)₂]-Ph |
| 3-807 | Me | 1 | H | 3-[(MeO)₂CH-(CH₂)₂]-Ph |
| 3-808 | Me | 1 | H | 4-[Me(MeO)₂C]-Ph |
| 3-809 | Me | 1 | H | 3-[Me(MeO)₂C]-Ph |
| 3-810 | Me | 1 | H | 4-[Me(MeO)₂C-CH₂]-Ph |
| 3-811 | Me | 1 | H | 3-[Me(MeO)₂C-CH₂]-Ph |
| 3-812 | Me | 1 | H | 4-[Me(MeO)₂C-(CH₂)₂]-Ph |
| 3-813 | Me | 1 | H | 3-[Me(MeO)₂C-(CH₂)₂]-Ph |
| 3-814 | Me | 1 | H | 4-[(EtO)₂CH]-Ph |
| 3-815 | Me | 1 | H | 3-[(EtO)₂CH]-Ph |
| 3-816 | Me | 1 | H | 4-[(EtO)₂CH-CH₂]-Ph |
| 3-817 | Me | 1 | H | 3-[(EtO)₂CH-CH₂]-Ph |
| 3-818 | Me | 1 | H | 4-[(EtO)₂CH-(CH₂)₂]-Ph |
| 3-819 | Me | 1 | H | 3-[(EtO)₂CH-(CH₂)₂]-Ph |
| 3-820 | Me | 1 | H | 4-[Me(EtO)₂C]-Ph |
| 3-821 | Me | 1 | H | 3-[Me(EtO)₂C]-Ph |
| 3-822 | Me | 1 | H | 4-[Me(EtO)₂C-CH₂]-Ph |
| 3-823 | Me | 1 | H | 3-[Me(EtO)₂C-CH₂]-Ph |
| 3-824 | Me | 1 | H | 4-[Me(EtO)₂C-(CH₂)₂]-Ph |
| 3-825 | Me | 1 | H | 3-[Me(EtO)₂C-(CH₂)₂]-Ph |
| 3-826 | Me | 1 | H | 4-(2-Dioxo)-Ph |
| 3-827 | Me | 1 | H | 3-(2-Dioxo)-Ph |
| 3-828 | Me | 1 | H | 4-[(2-Dioxo)-CH₂]-Ph |
| 3-829 | Me | 1 | H | 3-[(2-Dioxo)-CH₂]-Ph |
| 3-830 | Me | 1 | H | 4-[(2-Dioxo)-(CH₂)₂]-Ph |
| 3-831 | Me | 1 | H | 3-[(2-Dioxo)-(CH₂)₂]-Ph |
| 3-832 | Me | 1 | H | 4-[2-Me-(2-Dioxo)]-Ph |
| 3-833 | Me | 1 | H | 3-[2-Me-(2-Dioxo)]-Ph |
| 3-834 | Me | 1 | H | 4-[2-Me-(2-Dioxo)-CH₂]-Ph |
| 3-835 | Me | 1 | H | 3-[2-Me-(2-Dioxo)-CH₂]-Ph |
| 3-836 | Me | 1 | H | 4-[2-Me-(2-Dioxo)-(CH₂)₂]-Ph |
| 3-837 | Me | 1 | H | 3-[2-Me-(2-Dioxo)-(CH₂)₂]-Ph |
| 3-838 | Me | 1 | H | 4-(2-Dioxa)-Ph |
| 3-839 | Me | 1 | H | 3-(2-Dioxa)-Ph |
| 3-840 | Me | 1 | H | 4-[(2-Dioxa)-CH₂]-Ph |
| 3-841 | Me | 1 | H | 3-[(2-Dioxa)-CH₂]-Ph |
| 3-842 | Me | 1 | H | 4-[(2-Dioxa)-(CH₂)₂]-Ph |
| 3-843 | Me | 1 | H | 3-[(2-Dioxa)-(CH₂)₂]-Ph |
| 3-844 | Me | 1 | H | 4-[2-Me-(2-Dioxa)]-Ph |
| 3-845 | Me | 1 | H | 3-[2-Me-(2-Dioxa)]-Ph |
| 3-846 | Me | 1 | H | 4-[2-Me-(2-Dioxa)-CH₂]-Ph |
| 3-847 | Me | 1 | H | 3-[2-Me-(2-Dioxa)-CH₂]-Ph |
| 3-848 | Me | 1 | H | 4-[2-Me-(2-Dioxa)-(CH₂)₂]-Ph |
| 3-849 | Me | 1 | H | 3-[2-Me-(2-Dioxa)-(CH₂)₂]-Ph |
| 3-850 | Me | 1 | H | 2-Mc-1-oxo-5-IIndn |
| 3-851 | Me | 1 | H | 6-BzOxaz |
| 3-852 | Me | 1 | H | 4-(HO-N=)-7-Chr |
| 3-853 | Me | 1 | H | 3-Me-6-BzIox |
| 3-854 | Me | 1 | H | 2-Me-6-BzOxaz |
| 3-855 | Me | 1 | H | 2-Me-5-BzOxaz |
| 3-856 | Me | 1 | H | 2,3-dihydro-5-BzFur |
| 3-857 | Me | 1 | H | 6-Qui |
| 3-858 | Me | 1 | H | 6-Iqui |
| 3-859 | Me | 1 | H | 3-(HO-N=)-2,3-dihydro-6-BzFur |
| 3-860 | Me | 1 | H | 2-Me-6-BzThaz |
| 3-861 | Me | 1 | H | 5-Ind |
| 3-862 | Me | 1 | H | 4-Ac-2-Thaz |
| 3-863 | Me | 1 | H | 5-Ac-2-Thi |
| 3-864 | Me | 1 | H | 5-Ac-2-Fur |
| 3-865 | Me | 1 | H | 5-Me₂NCO-2-Py |
| 3-866 | Me | 1 | H | 5-(Me₂NCO-CH₂)-2-Py |
| 3-867 | Me | 1 | H | 5-[Me₂NCO-(CH₂)₂]-2-Py |
| 3-868 | Me | 1 | H | 4-Me₂NCO-2-Thaz |
| 3-869 | Me | 1 | H | 5-Me₂NCO-2-Thaz |
| 3-870 | Me | 1 | H | 4-(Me₂NCO-CH₂)-2-Thaz |
| 3-871 | Me | 1 | H | 5-(Me₂NCO-CH₂)-2-Thaz |
| 3-872 | Me | 1 | H | 2-Thazn |
| 3-873 | Me | 1 | H | 2-Oxazn |
| 3-874 | H | 2 | H | 4-(MeO-CH₂)-Ph |
| 3-875 | H | 2 | H | 3-(MeO-CH₂)-Ph |
| 3-876 | H | 2 | H | 4-[MeO-(CH₂)₂]-Ph |
| 3-877 | H | 2 | H | 3-[MeO-(CH₂)₂]-Ph |
| 3-878 | H | 2 | H | 4-[MeO-(CH₂)₃]-Ph |
| 3-879 | H | 2 | H | 3-[MeO-(CH₂)₃]-Ph |
| 3-880 | H | 2 | H | 4-(EtO-CH₂)-Ph |
| 3-881 | H | 2 | H | 3-(EtO-CH₂)-Ph |
| 3-882 | H | 2 | H | 4-[EtO-(CH₂)₂]-Ph |
| 3-883 | H | 2 | H | 3-[EtO-(CH₂)₂]-Ph |
| 3-884 | H | 2 | H | 4-[EtO-(CH₂)₃]-Ph |
| 3-885 | H | 2 | H | 3-[EtO-(CH₂)₃]-Ph |
| 3-886 | H | 2 | H | 4-cPrO-Ph |
| 3-887 | H | 2 | H | 3-cPrO-Ph |
| 3-888 | H | 2 | H | 4-(cPrO-CH₂)-Ph |
| 3-889 | H | 2 | H | 3-(cPrO-CH₂)-Ph |
| 3-890 | H | 2 | H | 4-[cPrO-(CH₂)₂]-Ph |
| 3-891 | H | 2 | H | 3-[cPrO-(CH₂)₂]-Ph |
| 3-892 | H | 2 | H | 4-[cPrO-(CH₂)₃]-Ph |
| 3-893 | H | 2 | H | 3-[cPrO-(CH₂)₃]-Ph |
| 3-894 | H | 2 | H | 4-CHF₂O-Ph |
| 3-895 | H | 2 | H | 3-CHF₂O-Ph |
| 3-896 | H | 2 | H | 4-(CHF₂O-CH₂)-Ph |
| 3-897 | H | 2 | H | 3-(CHF₂O-CH₂)-Ph |
| 3-898 | H | 2 | H | 4-[CHF₂O-(CH₂)₂]-Ph |
| 3-899 | H | 2 | H | 3-[CHF₂O-(CH₂₎₂]-Ph |
| 3-900 | H | 2 | H | 4-[CHF₂O-(CH₂)₃]-Ph |
| 3-901 | H | 2 | H | 3-[CHF₂O-(CH₂)₃]-Ph |
| 3-902 | H | 2 | H | 3-(H₂NCO)-Ph |
| 3-903 | H | 2 | H | 4-(H₂NCO-CH₂)-Ph |
| 3-904 | H | 2 | H | 3-(H₂NCO-CH₂)-Ph |
| 3-905 | H | 2 | H | 4-[H₂NCO-(CH₂)₂]-Ph |
| 3-906 | H | 2 | H | 3-[H₂NCO-(CH₂)₂]-Ph |
| 3-907 | H | 2 | H | 3-(MeNHCO)-Ph |
| 3-908 | H | 2 | H | 4-(MeNHCO-CH₂)-Ph |
| 3-909 | H | 2 | H | 3-(MeNHCO-CH₂)-Ph |
| 3-910 | H | 2 | H | 4-[MeNHCO-(CH₂)₂]-Ph |
| 3-911 | H | 2 | H | 3-[MeNHCO-(CH₂)₂]-Ph |
| 3-912 | H | 2 | H | 4-(iPrNHCO)-Ph |
| 3-913 | H | 2 | H | 3-(iPrNHCO)-Ph |
| 3-914 | H | 2 | H | 4-(iPrNHCO-CH₂)-Ph |
| 3-915 | H | 2 | H | 3-(iPrNHCO-CH₂)-Ph |
| 3-916 | H | 2 | H | 4-[iPrNHCO-(CH₂)₂]-Ph |
| 3-917 | H | 2 | H | 3-[iPrNHCO-(CH₂)₂]-Ph |
| 3-918 | H | 2 | H | 4-(EtNMeCO)-Ph |
| 3-919 | H | 2 | H | 3-(EtNMeCO)-Ph |
| 3-920 | H | 2 | H | 4-(EtNMeCO-CH₂)-Ph |
| 3-921 | H | 2 | H | 3-(EtNMeCO-CH₂)-Ph |
| 3-922 | H | 2 | H | 4-[EtNMeCO-(CH₂)₂]-Ph |
| 3-923 | H | 2 | H | 3-[EtNMeCO-(CH₂)₂]-Ph |
| 3-924 | H | 2 | H | 3-(Me₂NCO)-Ph |
| 3-925 | H | 2 | H | 4-(Me₂NCO-CH₂)-Ph |
| 3-926 | H | 2 | H | 3-(Me₂NCO-CH₂)-Ph |
| 3-927 | H | 2 | H | 4-[Me₂NCO-(CH₂)₂]-Ph |
| 3-928 | H | 2 | H | 3-[Me₂NCO-(CH₂)₂]-Ph |
| 3-929 | H | 2 | H | 3-(Et₂NCO)-Ph |
| 3-930 | H | 2 | H | 4-(Et₂NCO-CH₂)-Ph |
| 3-931 | H | 2 | H | 3-(Et₂NCO-CH₂)-Ph |
| 3-932 | H | 2 | H | 4-[Et₂NCO-(CH₂)₂]-Ph |
| 3-933 | H | 2 | H | 3-[Et₂NCO-(CH₂)₂]-Ph |
| 3-934 | H | 2 | H | 4-[(MeO)NMeCO]-Ph |
| 3-935 | H | 2 | H | 3-[(MeO)NMeCO]-Ph |
| 3-936 | H | 2 | H | 4-[(MeO)NMeCO-CH₂]-Ph |
| 3-937 | H | 2 | H | 3-[(MeO)NMeCO-CH₂]-Ph |
| 3-938 | H | 2 | H | 4-[(MeO)NMeCO-(CH₂)₂]-Ph |
| 3-939 | H | 2 | H | 3-[(MeO)NMeCO-(CH₂)₂]-Ph |
| 3-940 | H | 2 | H | 4-(Aze-CO)-Ph |
| 3-941 | H | 2 | H | 3-(Aze-CO)-Ph |
| 3-942 | H | 2 | H | 4-(Aze-CO-CH₂)-Ph |
| 3-943 | H | 2 | H | 3-(Aze-CO-CH₂)-Ph |
| 3-944 | H | 2 | H | 4-[Aze-CO-(CH₂)₂]-Ph |
| 3-945 | H | 2 | H | 3-[Aze-CO-(CH₂)₂]-Ph |
| 3-946 | H | 2 | H | 4-[(3-HO-1-Aze)-CO]-Ph |
| 3-947 | H | 2 | H | 3-[(3-HO-1-Aze)-CO]-Ph |
| 3-948 | H | 2 | H | 4-[(3-HO-Aze)-CO-CH₂]-Ph |
| 3-949 | H | 2 | H | 3-[(3-HO-Aze)-CO-CH₂]-Ph |
| 3-950 | H | 2 | H | 4-[(3-HO-Aze)-CO-(CH₂)₂]-Ph |
| 3-951 | H | 2 | H | 3-[(3-HO-Aze)-CO-(CH₂)₂]-Ph |
| 3-952 | H | 2 | H | 4-(Pyrld-CO)-Ph |
| 3-953 | H | 2 | H | 3-(Pyrld-CO)-Ph |
| 3-954 | H | 2 | H | 4-(Pyrld-CO-CH₂)-Ph |
| 3-955 | H | 2 | H | 3-(Pyrld-CO-CH₂)-Ph |
| 3-956 | H | 2 | H | 4-[Pyrld-CO-(CH₂)₂]-Ph |
| 3-957 | H | 2 | H | 3-[Pyrld-CO-(CH₂)₂]-Ph |
| 3-958 | H | 2 | H | 4-(Pipo-CO)-Ph |
| 3-959 | H | 2 | H | 3-(Pipo-CO)-Ph |
| 3-960 | H | 2 | H | 4-(Pipo-CO-CH₂)-Ph |
| 3-961 | H | 2 | H | 3-(Pipo-CO-CH₂)-Ph |
| 3-962 | H | 2 | H | 4-[Pipo-CO-(CH₂)₂]-Ph |
| 3-963 | H | 2 | H | 3-[Pipo-CO-(CH₂)₂]-Ph |
| 3-964 | H | 2 | H | 4-[(4-Me-Pipra)-CO]-Ph |
| 3-965 | H | 2 | H | 3-[(4-Me-Pipra)-CO]-Ph |
| 3-966 | H | 2 | H | 4-[(4-Me-Pipra)-CO-CH₂]-Ph |
| 3-967 | H | 2 | H | 3-[(4-Me-Pipra)-CO-CH₂]-Ph |
| 3-968 | H | 2 | H | 4-[(4-Me-Pipra)-CO-(CH₂)₂]-Ph |
| 3-969 | H | 2 | H | 3-[(4-Me-Pipra)-CO-(CH₂)₂]-Ph |
| 3-970 | H | 2 | H | 4-(Mor-CO)-Ph |
| 3-971 | H | 2 | H | 3-(Mor-CO)-Ph |
| 3-972 | H | 2 | H | 4-(Mor-CO-CH₂)-Ph |
| 3-973 | H | 2 | H | 3-(Mor-CO-CH₂)-Ph |
| 3-974 | H | 2 | H | 4-[Mor-CO-(CH₂)₂]-Ph |
| 3-975 | H | 2 | H | 3-[Mor-CO-(CH₂)₂]-Ph |
| 3-976 | H | 2 | H | 3-Me₂N-Ph |
| 3-977 | H | 2 | H | 4-(Me₂N-CH₂)-Ph |
| 3-978 | H | 2 | H | 3-(Me₂N-CH₂)-Ph |
| 3-979 | H | 2 | H | 4-[Me₂N-(CH₂)₂]-Ph |
| 3-980 | H | 2 | H | 3-[Me₂N-(CH₂)₂]-Ph |
| 3-981 | H | 2 | H | 4-[Me₂N-(CH₂)₃]-Ph |
| 3-982 | H | 2 | H | 3-[Me₂N-(CH₂)₃]-Ph |
| 3-983 | H | 2 | H | 4-Mor-Ph |
| 3-984 | H | 2 | H | 3-Mor-Ph |
| 3-985 | H | 2 | H | 4-(Mor-CH₂)-Ph |
| 3-986 | H | 2 | H | 3-(Mor-CH₂)-Ph |
| 3-987 | H | 2 | H | 4-[Mor-(CH₂)₂]-Ph |
| 3-988 | H | 2 | H | 3-[Mor-(CH₂)₂]-Ph |
| 3-989 | H | 2 | H | 4-[Mor-(CH₂)₃]-Ph |
| 3-990 | H | 2 | H | 3-[Mor-(CH₂)₃]-Ph |
| 3-991 | H | 2 | H | 4-Pipo-Ph |
| 3-992 | H | 2 | H | 3-Pipo-Ph |
| 3-993 | H | 2 | H | 4-(Pipo-CH₂)-Ph |
| 3-994 | H | 2 | H | 3-(Pipo-CH₂)-Ph |
| 3-995 | H | 2 | H | 4-[Pipo-(CH₂)_{2]}-Ph |
| 3-996 | H | 2 | H | 3-[Pipo-(CH₂)₂]-Ph |
| 3-997 | H | 2 | H | 4-[Pipo-(CH₂)₃]-Ph |
| 3-998 | H | 2 | H | 3-[Pipo-(CH₂)₃]-Ph |
| 3-999 | H | 2 | H | 4-HO-Ph |
| 3-1000 | H | 2 | H | 3-HO-Ph |
| 3-1001 | H | 2 | H | 4-(HO-CH₂)-Ph |
| 3-1002 | H | 2 | H | 3-(HO-CH₂)-Ph |
| 3-1003 | H | 2 | H | 4-[HO-(CH₂)₂]-Ph |
| 3-1004 | H | 2 | H | 3-[HO-(CH₂)₂]-Ph |
| 3-1005 | H | 2 | H | 4-[HO-(CH₂)₃]-Ph |
| 3-1006 | H | 2 | H | 3-[HO-(CH₂)₃]-Ph |
| 3-1007 | H | 2 | H | 4-[MeCH(OH)]-Ph |
| 3-1008 | H | 2 | H | 3-[MeCH(OH)]-Ph |
| 3-1009 | H | 2 | H | 4-[MeCH(OH)-CH₂]-Ph |
| 3-1010 | H | 2 | H | 3-[MeCH(OH)-CH_{2]}-Ph |
| 3-1011 | H | 2 | H | 4-[MeCH(OH)-(CH₂)₂]-Ph |
| 3-1012 | H | 2 | H | 3-[MeCH(OH)-(CH₂)₂]-Ph |
| 3-1013 | H | 2 | H | 3-CN-Ph |
| 3-1014 | H | 2 | H | 4-(CN-CH₂)-Ph |
| 3-1015 | H | 2 | H | 3-(CN-CH₂)-Ph |
| 3-1016 | H | 2 | H | 4-[CN-(CH₂)₂]-Ph |
| 3-1017 | H | 2 | H | 3-[CN-(CH₂)₂]-Ph |
| 3-1018 | H | 2 | H | 3-Ac-Ph |
| 3-1019 | H | 2 | H | 4-(Ac-CH₂)-Ph |
| 3-1020 | H | 2 | H | 3-(Ac-CH₂)-Ph |
| 3-1021 | H | 2 | H | 4-[Ac-(CH₂)₂]-Ph |
| 3-1022 | H | 2 | H | 3-[Ac-(CH₂)₂]-Ph |
| 3-1023 | H | 2 | H | 4-(CF₃CO)-Ph |
| 3-1024 | H | 2 | H | 4-(EtCO)-Ph |
| 3-1025 | H | 2 | H | 3-(EtCO)-Ph |
| 3-1026 | H | 2 | H | 4-(EtCO-CH₂)-Ph |
| 3-1027 | H | 2 | H | 3-(EtCO-CH₂)-Ph |
| 3-1028 | H | 2 | H | 4-[EtCO-(CH₂)₂]-Ph |
| 3-1029 | H | 2 | H | 3-[EtCO-(CH₂)₂]-Ph |
| 3-1030 | H | 2 | H | 4-(iPrCO)-Ph |
| 3-1031 | H | 2 | H | 4-(cBuCO)-Ph |
| 3-1032 | H | 2 | H | 4-(cPrCO)-Ph |
| 3-1033 | H | 2 | H | 4-(Ph-CO)-Ph |
| 3-1034 | H | 2 | H | 4-Ac-3-MeO-Ph |
| 3-1035 | H | 2 | H | 4-Ac-3-OH-Ph |
| 3-1036 | H | 2 | H | 4-Ac-3-Cl-Ph |
| 3-1037 | H | 2 | H | 4-[CH₃C(=N-OH)]-Ph |
| 3-1038 | H | 2 | H | 3-[CH₃C(=N-OH)]-Ph |
| 3-1039 | H | 2 | H | 4-[CH₃C(=N-OH)-CH₂]-Ph |
| 3-1040 | H | 2 | H | 3-[CH₃C(=N-OH)-CH₂]-Ph |
| 3-1041 | H | 2 | H | 4-[CH₃C(=N-OH)-(CH₂)₂]-Ph |
| 3-1042 | H | 2 | H | 3-[CH₃C(=N-OH)-(CH₂)₂]-Ph |
| 3-1043 | H | 2 | H | 4-[CH₃C(=N-OMe)]-Ph |
| 3-1044 | H | 2 | H | 3-[CH₃C(=N-OMe)]-Ph |
| 3-1045 | H | 2 | H | 4-[CH₃C(=N-OMe)-CH₂]-Ph |
| 3-1046 | H | 2 | H | 3-[CH₃C(=N-OMe)-CH₂]-Ph |
| 3-1047 | H | 2 | H | 4-[CH₃C(=N-OMe)-(CH₂)₂]-Ph |
| 3-1048 | H | 2 | H | 3-[CH₃C(=N-OMe)-(CH₂)₂]-Ph |
| 3-1049 | H | 2 | H | 4-(Me₂NSO₂)-Ph |
| 3-1050 | H | 2 | H | 4-[(MeO)₂CH]-Ph |
| 3-1051 | H | 2 | H | 3-[(MeO)₂CH]-Ph |
| 3-1052 | H | 2 | H | 4-[(MeO)₂CH-CH₂]-Ph |
| 3-1053 | H | 2 | H | 3-[(MeO)₂CH-CH₂]-Ph |
| 3-1054 | H | 2 | H | 4-[(MeO)₂CH-(CH₂)₂]-Ph |
| 3-1055 | H | 2 | H | 3-[(MeO)₂CH-(CH₂)₂]-Ph |
| 3-1056 | H | 2 | H | 4-[Me(MeO)₂C]-Ph |
| 3-1057 | H | 2 | H | 3-[Me(MeO)₂C]-Ph |
| 3-1058 | H | 2 | H | 4-[Me(MeO)₂C-CH₂]-Ph |
| 3-1059 | H | 2 | H | 3-[Me(MeO)₂C-CH₂]-Ph |
| 3-1060 | H | 2 | H | 4-[Me(MeO)₂C-(CH₂)₂]-Ph |
| 3-1061 | H | 2 | H | 3-[Me(MeO)₂C-(CH₂)₂]-Ph |
| 3-1062 | H | 2 | H | 4-[(EtO)₂CH]-Ph |
| 3-1063 | H | 2 | H | 3-[(EtO)₂CH]-Ph |
| 3-1064 | H | 2 | H | 4-[(EtO)₂CH-CH₂]-Ph |
| 3-1065 | H | 2 | H | 3-[(EtO)₂CH-CH₂]-Ph |
| 3-1066 | H | 2 | H | 4-[(EtO)₂CH-(CH₂)₂]-Ph |
| 3-1067 | H | 2 | H | 3-[(EtO)₂CH-(CH₂)₂]-Ph |
| 3-1068 | H | 2 | H | 4-[Me(EtO)₂C]-Ph |
| 3-1069 | H | 2 | H | 3-[Me(EtO)₂C]-Ph |
| 3-1070 | H | 2 | H | 4-[Me(EtO)₂C-CH₂]-Ph |
| 3-1071 | H | 2 | H | 3-[Me(EtO)₂C-CH₂]-Ph |
| 3-1072 | H | 2 | H | 4-[Me(EtO)₂C-(CH₂)₂]-Ph |
| 3-1073 | H | 2 | H | 3-[Me(EtO)₂C-(CH₂)₂]-Ph |
| 3-1074 | H | 2 | H | 4-(2-Dioxo)-Ph |
| 3-1075 | H | 2 | H | 3-(2-Dioxo)-Ph |
| 3-1076 | H | 2 | H | 4-[(2-Dioxo)-CH₂]-Ph |
| 3-1077 | H | 2 | H | 3-[(2-Dioxo)-CH₂]-Ph |
| 3-1078 | H | 2 | H | 4-[(2-Dioxo)-(CH₂)₂]-Ph |
| 3-1079 | H | 2 | H | 3-[(2-Dioxo)-(CH₂)₂]-Ph |
| 3-1080 | H | 2 | H | 4-[2-Me-(2-Dioxo)]-Ph |
| 3-1081 | H | 2 | H | 3-[2-Me-(2-Dioxo)]-Ph |
| 3-1082 | H | 2 | H | 4-[2-Me-(2-Dioxo)-CH₂]-Ph |
| 3-1083 | H | 2 | H | 3-[2-Me-(2-Dioxo)-CH₂]-Ph |
| 3-1084 | H | 2 | H | 4-[2-Me-(2-Dioxo)-(CH₂)₂]-Ph |
| 3-1085 | H | 2 | H | 3-[2-Me-(2-Dioxo)-(CH₂)₂]-Ph |
| 3-1086 | H | 2 | H | 4-(2-Dioxa)-Ph |
| 3-1087 | H | 2 | H | 3-(2-Dioxa)-Ph |
| 3-1088 | H | 2 | H | 4-[(2-Dioxa)-CH₂]-Ph |
| 3-1089 | H | 2 | H | 3-[(2-Dioxa)-CH₂]-Ph |
| 3-1090 | H | 2 | H | 4-[(2-Dioxa)-(CH₂)₂]-Ph |
| 3-1091 | H | 2 | H | 3-[(2-Dioxa)-(CH₂)₂]-Ph |
| 3-1092 | H | 2 | H | 4-[2-Me-(2-Dioxa)]-Ph |
| 3-1093 | H | 2 | H | 3-[2-Me-(2-Dioxa)]-Ph |
| 3-1094 | H | 2 | H | 4-[2-Me-(2-Dioxa)-CH₂]-Ph |
| 3-1095 | H | 2 | H | 3-[2-Me-(2-Dioxa)-CH₂]-Ph |
| 3-1096 | H | 2 | H | 4-[2-Me-(2-Dioxa)-(CH₂)₂]-Ph |
| 3-1097 | H | 2 | H | 3-[2-Me-(2-Dioxa)-(CH₂)₂]-Ph |
| 3-1098 | H | 2 | H | 2-Me-1-oxo-5-IIndn |
| 3-1099 | H | 2 | H | 6-BzOxaz |
| 3-1100 | H | 2 | H | 4-(HO-N=)-7-Chr |
| 3-1101 | H | 2 | H | 3-Me-6-BzIox |
| 3-1102 | H | 2 | H | 2-Me-6-BzOxaz |
| 3-1103 | H | 2 | H | 2-Me-5-BzOxaz |
| 3-1104 | H | 2 | H | 2,3-dihydro-5-BzFur |
| 3-1105 | H | 2 | H | 6-Qui |
| 3-1106 | H | 2 | H | 6-Iqui |
| 3-1107 | H | 2 | H | 3-(HO-N=)-2,3-dihydro-6-BzFur |
| 3-1108 | H | 2 | H | 2-Me-6-BzThaz |
| 3-1109 | H | 2 | H | 5-Ind |
| 3-1110 | H | 2 | H | 4-Ac-2-Thaz |
| 3-1111 | H | 2 | H | 5-Ac-2-Thi |
| 3-1112 | H | 2 | H | 5-Ac-2-Fur |
| 3-1113 | H | 2 | H | 5-Me₂NCO-2-Py |
| 3-1114 | H | 2 | H | 5-(Me₂NCO-CH₂)-2-Py |
| 3-1115 | H | 2 | H | 5-[Me₂NCO-(CH₂)₂]-2-Py |
| 3-1116 | H | 2 | H | 4-Me₂NCO-2-Thaz |
| 3-1117 | H | 2 | H | 5-Me₂NCO-2-Thaz |
| 3-1118 | H | 2 | H | 4-(Me₂NCO-CH₂)-2-Thaz |
| 3-1119 | H | 2 | H | 5-(Me₂NCO-CH₂)-2-Thaz |
| 3-1120 | H | 2 | H | 2-Thazn |
| 3-1121 | H | 2 | H | 2-Oxazn |
| 3-1122 | Me | 2 | H | 4-(MeO-CH₂)-Ph |
| 3-1123 | Me | 2 | H | 3-(MeO-CH₂)-Ph |
| 3-1124 | Me | 2 | H | 4-[MeO-(CH₂)₂]-Ph |
| 3-1125 | Me | 2 | H | 3-[MeO-(CH₂)₂]-Ph |
| 3-1126 | Me | 2 | H | 4-[MeO-(CH₂)₃]-Ph |
| 3-1127 | Me | 2 | H | 3-[MeO-(CH₂)₃]-Ph |
| 3-1128 | Me | 2 | H | 4-(EtO-CH₂)-Ph |
| 3-1129 | Me | 2 | H | 3-(EtO-CH₂)-Ph |
| 3-1130 | Me | 2 | H | 4-[EtO-(CH₂)₂]-Ph |
| 3-1131 | Me | 2 | H | 3-[EtO-(CH₂)₂]-Ph |
| 3-1132 | Me | 2 | H | 4-[EtO-(CH₂)₃]-Ph |
| 3-1133 | Me | 2 | H | 3-[EtO-(CH₂)₃]-Ph |
| 3-1134 | Me | 2 | H | 4-cPrO-Ph |
| 3-1135 | Me | 2 | H | 3-cPrO-Ph |
| 3-1136 | Me | 2 | H | 4-(cPrO-CH₂)-Ph |
| 3-1137 | Me | 2 | H | 3-(cPrO-CH₂)-Ph |
| 3-1138 | Me | 2 | H | 4-[cPrO-(CH₂)₂]-Ph |
| 3-1139 | Me | 2 | H | 3-[cPrO-(CH₂)₂]-Ph |
| 3-1140 | Me | 2 | H | 4-[cPrO-(CH₂)₃]-Ph |
| 3-1141 | Me | 2 | H | 3-[cPrO-(CH₂)₃]-Ph |
| 3-1142 | Me | 2 | H | 4-CHF₂O-Ph |
| 3-1143 | Me | 2 | H | 3-CHF₂O-Ph |
| 3-1144 | Me | 2 | H | 4-(CHF₂O-CH₂)-Ph |
| 3-1145 | Me | 2 | H | 3-(CHF₂O-CH₂)-Ph |
| 3-1146 | Me | 2 | H | 4-[CHF₂O-(CH₂)₂]-Ph |
| 3-1147 | Me | 2 | H | 3-[CHF₂O-(CH₂)₂]-Ph |
| 3-1148 | Me | 2 | H | 4-[CHF₂O-(CH₂)₃]-Ph |
| 3-1149 | Me | 2 | H | 3-[CHF₂O-(CH₂)₃]-Ph |
| 3-1150 | Me | 2 | H | 3-(H₂NCO)-Ph |
| 3-1151 | Me | 2 | H | 4-(H₂NCO-CH₂)-Ph |
| 3-1152 | Me | 2 | H | 3-(H₂NCO-CH₂)-Ph |
| 3-1153 | Me | 2 | H | 4-[H₂NCO-(CH₂)₂]-Ph |
| 3-1154 | Me | 2 | H | 3-[H₂NCO-(CH₂)₂]-Ph |
| 3-1155 | Me | 2 | H | 3-(MeNHCO)-Ph |
| 3-1156 | Me | 2 | H | 4-(MeNHCO-CH₂)-Ph |
| 3-1157 | Me | 2 | H | 3-(MeNHCO-CH₂)-Ph |
| 3-1158 | Me | 2 | H | 4-[MeNHCO-(CH₂)₂]-Ph |
| 3-1159 | Me | 2 | H | 3-[MeNHCO-(CH₂)₂]-Ph |
| 3-1160 | Me | 2 | H | 4-(iPrNHCO)-Ph |
| 3-1161 | Me | 2 | H | 3-(iPrNHCO)-Ph |
| 3-1162 | Me | 2 | H | 4-(iPrNHCO-CH₂)-Ph |
| 3-1163 | Me | 2 | H | 3-(iPrNHCO-CH₂)-Ph |
| 3-1164 | Me | 2 | H | 4-[iPrNHCO-(CH₂)₂]-Ph |
| 3-1165 | Me | 2 | H | 3-[iPrNHCO-(CH₂)₂]-Ph |
| 3-1166 | Me | 2 | H | 4-(EtNMeCO)-Ph |
| 3-1167 | Me | 2 | H | 3-(EtNMeCO)-Ph |
| 3-1168 | Me | 2 | H | 4-(EtNMeCO-CH₂)-Ph |
| 3-1169 | Me | 2 | H | 3-(EtNMeCO-CH₂)-Ph |
| 3-1170 | Me | 2 | H | 4-[EtNMeCO-(CH₂)₂]-Ph |
| 3-1171 | Me | 2 | H | 3-[EtNMeCO-(CH₂)₂]-Ph |
| 3-1172 | Me | 2 | H | 3-(Me₂NCO)-Ph |
| 3-1173 | Me | 2 | H | 4-(Me₂NCO-CH₂)-Ph |
| 3-1174 | Me | 2 | H | 3-(Me₂NCO-CH₂)-Ph |
| 3-1175 | Me | 2 | H | 4-[Me₂NCO-(CH₂)₂]-Ph |
| 3-1176 | Me | 2 | H | 3-[Me₂NCO-(CH₂)₂]-Ph |
| 3-1177 | Me | 2 | H | 3-(Et₂NCO)-Ph |
| 3-1178 | Me | 2 | H | 4-(Et₂NCO-CH₂)-Ph |
| 3-1179 | Me | 2 | H | 3-(Et₂NCO-CH₂)-Ph |
| 3-1180 | Me | 2 | H | 4-[Et₂NCO-(CH₂)₂]-Ph |
| 3-1181 | Me | 2 | H | 3-[Et₂NCO-(CH₂)₂]-Ph |
| 3-1182 | Me | 2 | H | 4-[(MeO)NMeCO]-Ph |
| 3-1183 | Me | 2 | H | 3-[(MeO)NMeCO]-Ph |
| 3-1184 | Me | 2 | H | 4-[(MeO)NMeCO-CH₂]-Ph |
| 3-1185 | Me | 2 | H | 3-[(MeO)NMeCO-CH₂]-Ph |
| 3-1186 | Me | 2 | H | 4-[(MeO)NMeCO-(CH₂)₂]-Ph |
| 3-1187 | Me | 2 | H | 3-[(MeO)NMeCO-(CH₂)₂]-Ph |
| 3-1188 | Me | 2 | H | 4-(Aze-CO)-Ph |
| 3-1189 | Me | 2 | H | 3-(Aze-CO)-Ph |
| 3-1190 | Me | 2 | H | 4-(Aze-CO-CH₂)-Ph |
| 3-1191 | Me | 2 | H | 3-(Aze-CO-CH₂)-Ph |
| 3-1192 | Me | 2 | H | 4-[Aze-CO-(CH₂)₂]-Ph |
| 3-1193 | Me | 2 | H | 3-[Aze-CO-(CH₂)₂]-Ph |
| 3-1194 | Me | 2 | H | 4-[(3-HO-1-Aze)-CO]-Ph |
| 3-1195 | Me | 2 | H | 3-[(3-HO-1-Aze)-CO]-Ph |
| 3-1196 | Me | 2 | H | 4-[(3-HO-Aze)-CO-CH₂]-Ph |
| 3-1197 | Me | 2 | H | 3-[(3-HO-Aze)-CO-CH₂]-Ph |
| 3-1198 | Me | 2 | H | 4-[(3-HO-Aze)-CO-(CH₂)₂]-Ph |
| 3-1199 | Me | 2 | H | 3-[(3-HO-Aze)-CO-(CH₂)₂]-Ph |
| 3-1200 | Me | 2 | H | 4-(Pyrld-CO)-Ph |
| 3-1201 | Me | 2 | H | 3-(Pyrld-CO)-Ph |
| 3-1202 | Me | 2 | H | 4-(Pyrld-CO-CH₂)-Ph |
| 3-1203 | Me | 2 | H | 3-(Pyrld-CO-CH₂)-Ph |
| 3-1204 | Me | 2 | H | 4-[Pyrld-CO-(CH₂)₂]-Ph |
| 3-1205 | Me | 2 | H | 3-[Pyrld-CO-(CH₂)₂]-Ph |
| 3-1206 | Me | 2 | H | 4-(Pipo-CO)-Ph |
| 3-1207 | Me | 2 | H | 3-(Pipo-CO)-Ph |
| 3-1208 | Me | 2 | H | 4-(Pipo-CO-CH₂)-Ph |
| 3-1209 | Me | 2 | H | 3-(Pipo-CO-CH₂)-Ph |
| 3-1210 | Me | 2 | H | 4-[Pipo-CO-(CH₂)₂]-Ph |
| 3-1211 | Me | 2 | H | 3-[Pipo-CO-(CH₂)₂]-Ph |
| 3-1212 | Me | 2 | H | 4-[(4-Me-Pipra)-CO]-Ph |
| 3-1213 | Me | 2 | H | 3-[(4-Me-Pipra)-CO]-Ph |
| 3-1214 | Me | 2 | H | 4-[(4-Me-Pipra)-CO-CH₂]-Ph |
| 3-1215 | Me | 2 | H | 3-[(4-Me-Pipra)-CO-CH₂]-Ph |
| 3-1216 | Me | 2 | H | 4-[(4-Me-Pipra)-CO-(CH₂)₂]-Ph |
| 3-1217 | Me | 2 | H | 3-[(4-Me-Pipra)-CO-(CH₂)₂]-Ph |
| 3-1218 | Me | 2 | H | 4-(Mor-CO)-Ph |
| 3-1219 | Me | 2 | H | 3=(Mor-CO)-Ph |
| 3-1220 | Me | 2 | H | 4-(Mor-CO-CH₂)-Ph |
| 3-1221 | Me | 2 | H | 3-(Mor-CO-CH₂)-Ph |
| 3-1222 | Me | 2 | H | 4-[Mor-CO-(CH₂)₂]-Ph |
| 3-1223 | Me | 2 | H | 3-[Mor-CO-(CH₂)₂]-Ph |
| 3-1224 | Me | 2 | H | 3-Me₂N-Ph |
| 3-1225 | Me | 2 | H | 4-(Me₂N-CH₂)-Ph |
| 3-1226 | Me | 2 | H | 3-(Me₂N-CH₂)-Ph |
| 3-1227 | Me | 2 | H | 4-[Me₂N-(CH₂)₂]-Ph |
| 3-1228 | Me | 2 | H | 3-[Me₂N-(CH₂)₂]-Ph |
| 3-1229 | Me | 2 | H | 4-[Me₂N-(CH₂)₃]-Ph |
| 3-1230 | Me | 2 | H | 3-[Me₂N-(CH₂)₃]-Ph |
| 3-1231 | Me | 2 | H | 4-Mor-Ph |
| 3-1232 | Me | 2 | H | 3-Mor-Ph |
| 3-1233 | Me | 2 | H | 4-(Mor-CH₂)-Ph |
| 3-1234 | Me | 2 | H | 3-(Mor-CH₂)-Ph |
| 3-1235 | Me | 2 | H | 4-[Mor-(CH₂)₂]-Ph |
| 3-1236 | Me | 2 | H | 3-[Mor-(CH₂)₂]-Ph |
| 3-1237 | Me | 2 | H | 4-[Mor-(CH₂)₃]-Ph |
| 3-1238 | Me | 2 | H | 3-[Mor-(CH₂)₃]-Ph |
| 3-1239 | Me | 2 | H | 4-Pipo-Ph |
| 3-1240 | Me | 2 | H | 3-Pipo-Ph |
| 3-1241 | Me | 2 | H | 4-(Pipo-CH₂)-Ph |
| 3-1242 | Me | 2 | H | 3-(Pipo-CH₂)-Ph |
| 3-1243 | Me | 2 | H | 4-[Pipo-(CH₂)₂]-Ph |
| 3-1244 | Me | 2 | H | 3-[Pipo-(CH₂)₂]-Ph |
| 3-1245 | Me | 2 | H | 4-[Pipo-(CH₂)₃]-Ph |
| 3-1246 | Me | 2 | H | 3-[Pipo-(CH₂)₃]-Ph |
| 3-1247 | Me | 2 | H | 4-HO-Ph |
| 3-1248 | Me | 2 | H | 3-HO-Ph |
| 3-1249 | Me | 2 | H | 4-(HO-CH₂)-Ph |
| 3-1250 | Me | 2 | H | 3-(HO-CH₂)-Ph |
| 3-1251 | Me | 2 | H | 4-[HO-(CH₂)₂]-Ph |
| 3-1252 | Me | 2 | H | 3-[HO-(CH₂)₂]-Ph |
| 3-1253 | Me | 2 | H | 4-[HO-(CH₂)₃]-Ph |
| 3-1254 | Me | 2 | H | 3-[HO-(CH₂)₃]-Ph |
| 3-1255 | Me | 2 | H | 4-[MeCH(OH)]-Ph |
| 3-1256 | Me | 2 | H | 3-[MeCH(OH)]-Ph |
| 3-1257 | Me | 2 | H | 4-[MeCH(OH)-CH₂]-Ph |
| 3-1258 | Me | 2 | H | 3-[MeCH(OH)-CH₂]-Ph |
| 3-1259 | Me | 2 | H | 4-[MeCH(OH)-(CH₂)₂]-Ph |
| 3-1260 | Me | 2 | H | 3-[MeCH(OH)-(CH₂)₂]-Ph |
| 3-1261 | Me | 2 | H | 3-CN-Ph |
| 3-1262 | Me | 2 | H | 4-(CN-CH₂)-Ph |
| 3-1263 | Me | 2 | H | 3-(CN-CH₂)-Ph |
| 3-1264 | Me | 2 | H | 4-[CN-(CH₂)₂]-Ph |
| 3-1265 | Me | 2 | H | 3-[CN-(CH₂)₂]-Ph |
| 3-1266 | Me | 2 | H | 3-Ac-Ph |
| 3-1267 | Me | 2 | H | 4-(Ac-CH₂)-Ph |
| 3-1268 | Me | 2 | H | 3-(Ac-CH₂)-Ph |
| 3-1269 | Me | 2 | H | 4-[Ac-(CH₂)₂]-Ph |
| 3-1270 | Me | 2 | H | 3-[Ac-(CH₂)₂]-Ph |
| 3-1271 | Me | 2 | H | 4-(CF₃CO)-Ph |
| 3-1272 | Me | 2 | H | 4-(EtCO)-Ph |
| 3-1273 | Me | 2 | H | 3-(EtCO)-Ph |
| 3-1274 | Me | 2 | H | 4-(EtCO-CH₂)-Ph |
| 3-1275 | Me | 2 | H | 3-(EtCO-CH₂)-Ph |
| 3-1276 | Me | 2 | H | 4-[EtCO-(CH₂)₂]-Ph |
| 3-1277 | Me | 2 | H | 3-[EtCO-(CH₂)₂]-Ph |
| 3-1278 | Me | 2 | H | 4-(iPrCO)-Ph |
| 3-1279 | Me | 2 | H | 4-(cBuCO)-Ph |
| 3-1280 | Me | 2 | H | 4-(cPrCO)-Ph |
| 3-1281 | Me | 2 | H | 4-(Ph-CO)-Ph |
| 3-1282 | Me | 2 | H | 4-Ac-3-MeO-Ph |
| 3-1283 | Me | 2 | H | 4-Ac-3-OH-Ph |
| 3-1284 | Me | 2 | H | 4-Ac-3-Cl-Ph |
| 3-1285 | Me | 2 | H | 4-[CH₃C(=N-OH)]-Ph |
| 3-1286 | Me | 2 | H | 3-[CH₃C(=N-OH)]-Ph |
| 3-1287 | Me | 2 | H | 4-[CH₃C(=N-OH)-CH₂]-Ph |
| 3-1288 | Me | 2 | H | 3-[CH₃C(=N-OH)-CH₂]-Ph |
| 3-1289 | Me | 2 | H | 4-[CH₃C(=N-OH)-(CH₂)₂]-Ph |
| 3-1290 | Me | 2 | H | 3-[CH₃C(=N-OH)-(CH₂)₂]-Ph |
| 3-1291 | Me | 2 | H | 4-[CH₃C(=N-OMe)]-Ph |
| 3-1292 | Me | 2 | H | 3-[CH₃C(=N-OMe)]-Ph |
| 3-1293 | Me | 2 | H | 4-[CH₃C(=N-OMe)-CH₂]-Ph |
| 3-1294 | Me | 2 | H | 3-[CH₃C(=N-OMe)-CH₂]-Ph |
| 3-1295 | Me | 2 | H | 4-[CH₃C(=N-OMe)-(CH₂)₂]-Ph |
| 3-1296 | Me | 2 | H | 3-[CH₃C(=N-OMe)-(CH₂)₂]-Ph |
| 3-1297 | Me | 2 | H | 4-(Me₂NSO₂)-Ph |
| 3-1298 | Me | 2 | H | 4-[(MeO)₂CH]-Ph |
| 3-1299 | Me | 2 | H | 3-[(MeO)₂CH]-Ph |
| 3-1300 | Me | 2 | H | 4-[(MeO)₂CH-CH₂]-Ph |
| 3-1301 | Me | 2 | H | 3-[(MeO)₂CH-CH₂]-Ph |
| 3-1302 | Me | 2 | H | 4-[(MeO)₂CH-(CH₂)₂]-Ph |
| 3-1303 | Me | 2 | H | 3-[(MeO)₂CH-(CH₂)₂]-Ph |
| 3-1304 | Me | 2 | H | 4-[Me(MeO)₂C]-Ph |
| 3-1305 | Me | 2 | H | 3-[Me(MeO)₂C]-Ph |
| 3-1306 | Me | 2 | H | 4-[Me(MeO)₂C-CH₂]-Ph |
| 3-1307 | Me | 2 | H | 3-[Me(MeO)₂C-CH₂]-Ph |
| 3-1308 | Me | 2 | H | 4-[Me(MeO)₂C-(CH₂)₂]-Ph |
| 3-1309 | Me | 2 | H | 3-[Me(MeO)₂C-(CH₂)₂]-Ph |
| 3-1310 | Me | 2 | H | 4-[(EtO)₂CH]-Ph |
| 3-1311 | Me | 2 | H | 3-[(EtO)₂CH]-Ph |
| 3-1312 | Me | 2 | H | 4-[(EtO)₂CH-CH₂]-Ph |
| 3-1313 | Me | 2 | H | 3-[(EtO)₂CH-CH₂]-Ph |
| 3-1314 | Me | 2 | H | 4-[(EtO)₂CH-(CH₂)₂]-Ph |
| 3-1315 | Me | 2 | H | 3-[(EtO)₂CH-(CH₂)₂]-Ph |
| 3-1316 | Me | 2 | H | 4-[Me(EtO)₂C]-Ph |
| 3-1317 | Me | 2 | H | 3-[Me(EtO)₂C]-Ph |
| 3-1318 | Me | 2 | H | 4-[Me(EtO)₂C-CH₂]-Ph |
| 3-1319 | Me | 2 | H | 3-[Me(EtO)₂C-CH₂]-Ph |
| 3-1320 | Me | 2 | H | 4-[Me(EtO)₂C-(CH₂)₂]-Ph |
| 3-1321 | Me | 2 | H | 3-[Me(EtO)₂C-(CH₂)₂]-Ph |
| 3-1322 | Me | 2 | H | 4-(2-Dioxo)-Ph |
| 3-1323 | Me | 2 | H | 3-(2-Dioxo)-Ph |
| 3-1324 | Me | 2 | H | 4-[(2-Dioxo)-CH₂]-Ph |
| 3-1325 | Me | 2 | H | 3-[(2-Dioxo)-CH₂]-Ph |
| 3-1326 | Me | 2 | H | 4-[(2-Dioxo)-(CH₂)₂]-Ph |
| 3-1327 | Me | 2 | H | 3-[(2-Dioxo)-(CH₂)₂]-Ph |
| 3-1328 | Me | 2 | H | 4-[2-Me-(2-Dioxo)]-Ph |
| 3-1329 | Me | 2 | H | 3-[2-Me-(2-Dioxo)]-Ph |
| 3-1330 | Me | 2 | H | 4-[2-Me-(2-Dioxo)-CH₂]-Ph |
| 3-1331 | Me | 2 | H | 3-[2-Me-(2-Dioxo)-CH₂]-Ph |
| 3-1332 | Me | 2 | H | 4-[2-Me-(2-Dioxo)-(CH₂)₂]-Ph |
| 3-1333 | Me | 2 | H | 3-[2-Me-(2-Dioxo)-(CH₂)₂]-Ph |
| 3-1334 | Me | 2 | H | 4-(2-Dioxa)-Ph |
| 3-1335 | Me | 2 | H | 3-(2-Dioxa)-Ph |
| 3-1336 | Me | 2 | H | 4-[(2-Dioxa)-CH₂]-Ph |
| 3-1337 | Me | 2 | H | 3-[(2-Dioxa)-CH₂]-Ph |
| 3-1338 | Me | 2 | H | 4-[(2-Dioxa)-(CH₂)₂]-Ph |
| 3-1339 | Me | 2 | H | 3-[(2-Dioxa)-(CH₂)₂]-Ph |
| 3-1340 | Me | 2 | H | 4-[2-Me-(2-Dioxa)]-Ph |
| 3-1341 | Me | 2 | H | 3-[2-Me-(2-Dioxa)]-Ph |
| 3-1342 | Me | 2 | H | 4-[2-Me-(2-Dioxa)-CH₂]-Ph |
| 3-1343 | Me | 2 | H | 3-[2-Me-(2-Dioxa)-CH₂]-Ph |
| 3-1344 | Me | 2 | H | 4-[2-Me-(2-Dioxa)-(CH₂)₂]-Ph |
| 3-1345 | Me | 2 | H | 3-[2-Me-(2-Dioxa)-(CH₂)₂]-Ph |
| 3-1346 | Me | 2 | H | 2-Me-1-oxo-5-IIndn |
| 3-1347 | Me | 2 | H | 6-BzOxaz |
| 3-1348 | Me | 2 | H | 4-(HO-N=)-7-Chr |
| 3-1349 | Me | 2 | H | 3-Me-6-BzIox |
| 3-1350 | Me | 2 | H | 2-Me-6-BzOxaz |
| 3-1351 | Me | 2 | H | 2-Me-5-BzOxaz |
| 3-1352 | Me | 2 | H | 2,3-dihydro-5-BzFur |
| 3-1353 | Me | 2 | H | 6-Qui |
| 3-1354 | Me | 2 | H | 6-Iqui |
| 3-1355 | Me | 2 | H | 3-(HO-N=)-2,3-dihydro-6-BzFur |
| 3-1356 | Me | 2 | H | 2-Me-6-BzThaz |
| 3-1357 | Me | 2 | H | 5-Ind |
| 3-1358 | Me | 2 | H | 4-Ac-2-Thaz |
| 3-1359 | Me | 2 | H | 5-Ac-2-Thi |
| 3-1360 | Me | 2 | H | 5-Ac-2-Fur |
| 3-1361 | Me | 2 | H | 5-Me₂NCO-2-Py |
| 3-1362 | Me | 2 | H | 5-(Me₂NCO-CH₂)-2-Py |
| 3-1363 | Me | 2 | H | 5-[Me₂NCO-(CH₂)₂]-2-Py |
| 3-1364 | Me | 2 | H | 4-Me₂NCO-2-Thaz |
| 3-1365 | Me | 2 | H | 5-Me₂NCO-2-Thaz |
| 3-1366 | Me | 2 | H | 4-(Me₂NCO-CH₂)-2-Thaz |
| 3-1367 | Me | 2 | H | 5-(Me₂NCO-CH₂)-2-Thaz |
| 3-1368 | Me | 2 | H | 2-Thazn |
| 3-1369 | Me | 2 | H | 2-Oxazn |

The compounds which are preferred in the above Table 1 to Table 3 for exemplified compounds are the exemplified compounds designated as numbers 1-7, 1-16, 1-17, 1-20, 1-21, 1-22, 1-23, 1-27, 1-28, 1-29, 1-36, 1-39, 1-40, 1-44, 1-68, 1-95, 1-96, 1-97, 1-99, 1-100, 1-101, 1-102, 1-105, 1-109, 1-112, 1-115, 1-118, 1-123, 1-133, 1-139, 1-140, 1-141, 1-143, 1-144, 1-146, 1-149, 1-152, 1-155, 1-156, 1-157, 1-164, 1-173, 1-181, 1-182, 1-183, 1-190, 1-229, 2-33, 2-102, 3-22, 3-30, 3-86, 3-87, 3-88, 3-89, 3-97, 3-98, 3-99, 3-100, 3-104, 3-105, 3-106, 3-107, 3-110, 3-111, 3-112, 3-116, 3-117, 3-123, 3-124, 3-125, 3-127, 3-131, 3-132, 3-136, 3-138, 3-139, 3-140, 3-142, 3-143, 3-161, 3-182, 3-429, 3-430, 3-431, 3-432, 3-876, 3-886, 3-894, 3-912, 3-918, 3-924, 3-925, 3-927, 3-934, 3-940, 3-946, 3-952, 3-970, 3-979, 3-1003, 3-1004, 3-1005, 3-1007, 3-1018, 3-1019, 3-1023, 3-1030, 3-1031, 3-1032, 3-1034, 3-1035, 3-1037, 3-1043, 3-1082, 3-1098, 3-1099, 3-1100, 3-1101, 3-1102, 3-1103, 3-1104, 3-1105, 3-1107, 3-1108, 3-1109, 3-1110, 3-1111, 3-1112, 3-1113, 3-1116, 3-1117, 3-1118 and 3-1120, and more preferred compounds are the exemplified compounds designated as numbers 1-17, 1-21, 1-22, 1-28, 1-29, 1-68, 1-99, 1-101, 1-102, 1-105, 1-109, 1-112, 1-115, 1-123, 1-143, 1-144, 1-146, 1-152, 1-155, 1-157, 1-164, 1-229, 3-30, 3-86, 3-87, 3-88, 3-99, 3-100, 3-104, 3-106, 3-107, 3-111, 3-116, 3-124, 3-127, 3-136, 3-142, 3-143, 3-161, 3-182, 3-429, 3-430, 3-876, 3-924, 3-925, 3-927, 3-934, 3-940, 3-952, 3-970, 3-979, 3-1003, 3-1004, 3-1005, 3-1007, 3-1018, 3-1019, 3-1031, 3-1034, 3-1035, 3-1037, 3-1043, 3-1082, 3-1098, 3-1099, 3-1100, 3-1101, 3-1102, 3-1104, 3-1110, 3-1111, 3-1113, 3-1116 and 3-1117.

Of these, particularly preferred compounds are
· 3-amino-4-[(3S)-3-(methoxymethyl)piperidin-1-yl]thieno[2,3-b]pyridine-2-carboxamide (exemplary compound No. 1-21),
· 3-amino-4-[(3S)-3-(methoxymethyl)piperidin-1-yl]-6-methylthieno[2,3-b]pyridine-2-carboxamide (exemplary compound No. 1-68),
· 3-amino-4-{3-[3-(2-hydroxyethoxy)propyl]piperidin-1-yl}thieno[2,3-b]pyridine-2-carboxamide (exemplary compound No. 1-101),
· 3-amino-4-{(35)-[(2-methoxyethoxy)methyl]piperidin-1-yl}thieno[2,3-b]pyridine-2-carboxamide hydrochloride (exemplary compound No. 1-112),
· 3-amino-4-{(3S)-3-[(3-methoxypropoxy)methyl]piperidin-1-yl}thieno[2,3-b]pyridine-2-carboxamide (exemplary compound No. 1-115),
· 3-amino-4-(3-{[2-(dimethylamino)-2-oxoethoxy]methyl}piperidin-1-yl)thieno[2,3-b]pyridine-2-carboxamide (exemplary compound No. 1-155),
· 3-amino-4-(3-{3-[2-(dimethylamino)-2-oxoethoxy]propyl}piperidin-1-yl)thieno[2,3-b]pyridine-2-carboxamide (exemplary compound No. 1-157),
· 4-[4-(4-acetylphenyl)-1,4-diazepan-1-yl]-3-aminothieno[2,3-b]pyridine-2-carboxamide (exemplary compound No. 3-99),
· 3-amino-4-[4-(4-propionylphenyl)-1,4-diazepan-1-yl]thieno[2,3-b]pyridine-2-carboxamide (exemplary compound No. 3-100),
· 3-amino-4-{4-[4-(dimethylamino)phenyl]-1,4-diazepan-1-yl}thieno[2,3-b]pyridine-2-carboxamide (exemplary compound No. 3-104),
· 3-amino-4-(4-{4-[(dimethylamino)carbonyl]phenyl}-1,4-diazepan-1-yl)thieno[2,3-b]pyridine-2-carboxamide (exemplary compound No. 3-107),
· 4-[4-(5-acetylpyridin-2-yl)-1,4-diazepan-1-yl]-3-aminothieno[2,3-b]pyridine-2-carboxamide (exemplary compound No. 3-142),
· 3-amino-4-(4-{4-[(dimethylamino)carbonyl]phenyl}-1,4-diazepan-1-yl)-6-methylthieno[2,3-b]pyridine-2-carboxamide (exemplary compound No. 3-182),
· 3-amino-4-{4-[4-(2-methoxyethyl)phenyl]-1,4-diazepan-1-yl}thieno[2,3-b]pyridine-2-carboxamide (exemplary compound No. 3-876),
· 3-amino-4-(4-{4-[2-(dimethylamino)-2-oxoethyl]phenyl}-1,4-diazepan-1-yl)thieno[2,3-b]pyridine-2-carboxamide (exemplary compound No. 3-925),
· 3-amino-4-(4-{4-[3-(dimethylamino)-2-oxopropyl]phenyl}-1,4-diazepan-1-yl)thieno[2,3-b]pyridine-2-carboxamide (exemplary compound No. 3-927),
· 3-amino-4-{4-[4-(azetidin-1-ylcarbonyl)phenyl]-1,4-diazepan-1-yl}thieno[2,3-b]pyridine-2-carboxamide (exemplary compound No. 3-940),
· 3-amino-4-{4-[4-(morpholin-4-ylcarbonyl)phenyl]-1,4-diazepan-1-yl}thieno[2,3-b]pyridine-2-carboxamide,
· 3-amino-4-(4-{4-[2-(dimethylamino)ethyl]phenyl}-1,4-diazepan-1-yl)thieno[2,3-b]pyridine-2-carboxamide (exemplary compound No. 3-979),
· 3-amino-4-{4-[4-(2-hydroxyethyl)phenyl]-1,4-diazepan-1-yl}thieno[2,3-b]pyridine-2-carboxamide (exemplary compound No. 3-1003),
· 3-amino-4-{4-[3-(2-hydroxyethyl)phenyl]-1,4-diazepan-1-yl}thieno[2,3-b]pyridine-2-carboxamide (exemplary compound No. 3-1004),
· 3-amino-4-{4-[4-(3-hydroxypropyl)phenyl]-1,4-diazepan-1-yl}thieno[2,3-b]pyridine-2-carboxamide (exemplary compound No. 3-1005),
· 3-amino-4-{4-[4-(1-hydroxyethyl)phenyl]-1,4-diazepan-1-yl}thieno[2,3-b]pyridine-2-carboxamide (exemplary compound No. 3-1007),
· 3-amino-4-{4-[4-(2-oxopropyl)phenyl]-1,4-diazepan-1-yl}thieno[2,3-b]pyridine-2-carboxamide (exemplary compound No. 3-1019),
· 3-amino-4-(4-{4-[(1E)-N-hydroxyethaneimidoyl]phenyl}-1,4-diazepan-1-yl)thieno[2,3-b]pyridine-2-carboxamide (exemplary compound No. 3-1037),
· 3-amino-4-(4-{4-[(2-methyl-1,3-dioxolan-2-yl)methyl]phenyl}-1,4-diazepan-1-yl)thieno[2,3-b]pyridine-2-carboxamide (exemplary compound No. 3-1082),
· 3-amino-4-[4-(2-methyl-1-oxo-2,3-dihydro-1H-isoindol-5-yl)-1,4-diazepan-1-yl]thieno[2,3-b]pyridine-2-carboxamide (exemplary compound No. 3-1098),
· 3-amino-4-[4-(1,3-benzoxazol-6-yl)-1,4-diazepan-1-yl]thieno[2,3-b]pyridine-2-carboxamide (exemplary compound No. 3-1099),
· 3-amino-4-{4-[(4E)-4-(hydroxyimino)-3,4-dihydro-2H-chromen-7-yl]-1,4-diazepan-1-yl}thieno[2,3-b]pyridine-2-carboxamide (exemplary compound No. 3-1100),
· 4-[4-(4-acetyl-1,3-thiazol-2-yl)-1,4-diazepan-1-yl]-3-aminothieno[2,3-b]pyridine-2-carboxamide (exemplary compound No. 3-1110),
· 4-[4-(5-acetylthiophen-2-yl)-1,4-diazepan-1-yl]-3-aminothieno[2,3-b]pyridine-2-carboxamide (exemplary compound No. 3-1111), and
· 3-amino-4-(4-{4-[(dimethylamino)carbonyl]-1,3-thiazol-2-yl}-1,4-diazepan-1-yl)thieno[2,3-b]pyridine-2-carboxamide (exemplary compound No. 3-1116) or pharmacologically acceptable salts thereof.

### Embodiments of the Invention

The compounds having a general formula (I) of the present invention can be produced by the processes mentioned below.

### <Process A>

The compound wherein R¹ is a hydrogen atom; R² is a group R^{a}NH-, R^{a}(R^{b})N- or 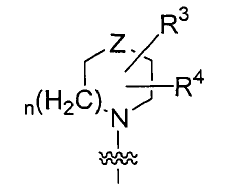 in general formula (I) can be produced according to Process A. 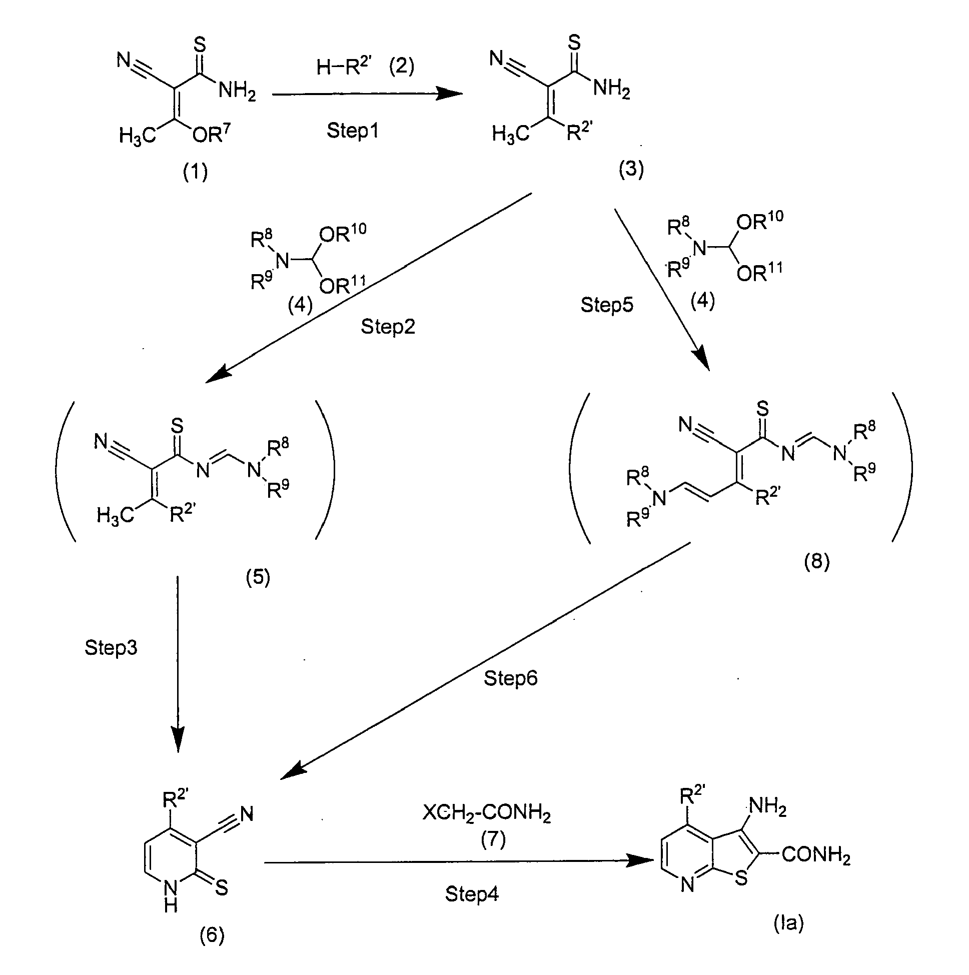 [wherein R^{2'} represents a group R^{a}NH-, R^{a}(R^{b})N- or 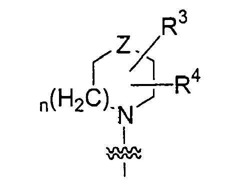 in the definition of R²,
R⁷ represents methyl or ethyl,
R⁸ and R⁹ independently represent a C₁-C₆ alkyl group (preferably methyl, ethyl or isopropyl, particularly preferably methyl) or they together with the nitrogen atom to which they are bonded represent a 4- to 7-membered heterocyclyl group which contains 1 to 2 sulfur, oxygen and/or nitrogen atoms (preferably pyrrolidinyl, piperidyl, morpholinyl), and
R¹⁰ and R¹¹ independently represent a C₁-C₆ alkyl group (preferably methyl, ethyl or isopropyl, particularly preferably methyl), and
X represents a halogen atom (preferably a chlorine atom or a bromine atom, particularly preferably a chlorine atom)].

Step 1 is a step for reacting compound (1) and amine compound (2) in an inert solvent to produce compound (3), and it can be carried out following a method described in J. Org. Chem, (1962) 27, 2433-2439.

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a halogenated hydrocarbon such as methylene chloride, chloroform, carbon tetrachloride, dichloroethane, chlorobenzene, o-dichlorobenzene, m-dichlorobenzene, fluorobenzene, trichloromethylbenzene or trifluoromethyl benzene, and preferably it is methanol, ethanol or N,N-dimethylformamide.

Reaction temperature varies depending on the raw material compounds or solvent used, but it is usually 0°C to reflux temperature of the reaction mixture and preferably it is room temperature to reflux temperature of the reaction mixture.

Reaction time differs according to the raw material compounds, solvent or reaction temperature used, but it is usually from 30 minutes to 96 hours, preferably from 30 minutes to 24 hours.

The deposit obtained by filtering the reaction liquid or the residue obtained by evaporating the solvent after the reaction terminates can be used in the next step (Step 2) without being particularly purified. In addition, the reaction solution can be used as it is in the next step when an amide is used as inert solvent.

Step 2 is a step for reacting compound (3) and N,N-dialkylformamide dialkyl acetal (4) in an inert solvent to produce amidine derivative (5).

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a halogenated hydrocarbon such as methylene chloride, chloroform, carbon tetrachloride, dichloroethane, chlorobenzene, o-dichlorobenzene, m-dichlorobenzene, fluorobenzene, trichloromethylbenzene or trifluoromethyl benzene, and preferably it is ethanol or N,N-dimethylformamide.

The amount of N,N-dialkylformamide dialkyl acetal (4) used for the reaction is preferably 1 to 2 equivalent for one equivalent of compound (3).

Reaction temperature varies depending on the raw material compounds or solvent used, but it is usually 0°C to reflux temperature of the reaction mixture, and preferably it is room temperature.

Reaction time differs according to the raw material compounds, solvent or reaction temperature used, but it is usually from 30 minutes to 96 hours, preferably from 30 minutes to 24 hours.

The deposit obtained by filtering the reaction liquid or the residue obtained by evaporating the solvent after the reaction terminates can be used in the next step (Step 3) without being particularly purified. In addition, the reaction solution can be used as it is in the next step when an amide is used as inert solvent.

Step 3 is a step for treating amidine derivative (5) in an inert solvent to produce thiopyridone derivatives (6).

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a halogenated hydrocarbon such as methylene chloride, chloroform, carbon tetrachloride, dichloroethane, chlorobenzene, o-dichlorobenzene, m-dichlorobenzene, fluorobenzene, trichloromethylbenzene or trifluoromethyl benzene, preferably it is ethanol or N,N-dimethylformamide, and particularly preferably it is N,N-dimethylformamide.

Reaction temperature varies depending on the raw material compounds or solvent used, but it is usually room temperature to reflux temperature of the reaction mixture and preferably it is 50°C to 120°C.

Reaction time differs according to the by raw material compounds, solvent or reaction temperature used, but it is usually from 10 minutes to six hours, preferably from 10 minutes to two hours.

After the reaction terminates, the object compound is collected from the reaction mixture according to a conventional method (extraction, column chromatography, filtration and concentration) as required. In addition, the reaction solution can be used as it is in the next step (Step 4) when an amide is used as inert solvent.

Step 4 is a step for reacting thiopyridone derivative (6) and α-haloacetamide (7) in the presence of a base in an inert solvent to produce thienopyridine derivatives (Ia).

The inert solvent to be used is, for example, an alcohol such as methanol, ethanol, propanol, 2-propanol or butanol; or an amide such as N,N-dimethylformamide, N,N-dimethylacetamide, N-methyl-2-pyrrolidone, N-methylpyrrolidinone or hexamethylphosphorotriamide, and preferably it is ethanol or N,N-dimethylformamide.

The base to be used is, for example, an organic base such as triethylamine or 1,8-diazabicyclo[5.4.0]-7-undecene (DBU); an alkali metal alkoxide such as sodium methoxide, sodium ethoxide, potassium tert-butoxide or lithium methoxide; an alkali metal hydroxide such as sodium hydroxide, potassium hydroxide or lithium hydroxide; or an aqueous solution of an alkali metal hydroxide, and preferably it is 1,8-diazabicyclo[5.4.0]-7-undecene (DBU), sodium ethoxide or an aqueous solution of sodium hydroxide.

Reaction temperature varies depending on the raw material compounds, solvent or base used, but it is usually 0°C to reflux temperature of the reaction mixture and preferably it is room temperature to reflux temperature of the reaction mixture.

Reaction time differs according to the raw material compounds, solvent, base or reaction temperature used, but it is usually from 10 minutes to six hours, preferably from 30 minutes to two hours.

After the reaction terminates, the object compound is collected from the reaction mixture according to a conventional method as required.

For example, the object compound can be obtained by adding water to the reaction mixture and filtering the separated object compound; or after neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 5 is a step for reacting compound (3) and N,N-dialkylformamide dialkyl acetal (4) in an inert solvent to produce amidine derivative (8).

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a halogenated hydrocarbon such as methylene chloride, chloroform, carbon tetrachloride, dichloroethane, chlorobenzene, o-dichlorobenzene, m-dichlorobenzene, fluorobenzene, trichloromethylbenzene or trifluoromethyl benzene, preferably it is an aromatic hydrocarbon, and particularly preferably it is toluene.

The amount of N,N-dialkylformamide dialkyl acetal (4) used for the reaction is preferably 2 to 3 equivalents for one equivalent of compound (3).

Reaction temperature varies depending on the raw material compounds or solvent used, but it is usually 0°C to reflux temperature of the reaction mixture and preferably it is room temperature to reflux temperature of the reaction mixture.

Reaction time is differs according to the raw material compounds, solvent or reaction temperature used, but it is usually from three minutes to six hours, preferably from three minutes to two hours.

The residue obtained by evaporating the solvent under reduced pressure after the reaction terminates can be used in the next step (Step 6) without being particularly purified.

Step 6 is a step for treating amidine derivative (8) with an alkaline aqueous solution to produce thiopyridone derivatives (6).

The alkaline aqueous solution to be used is, for example, an aqueous solution of an alkali metal hydroxide (for example, sodium hydroxide, potassium hydroxide or lithium hydroxide) and preferably an aqueous solution of sodium hydroxide.

Reaction temperature varies depending on the raw material compounds or solvent used, but it is usually room temperature to reflux temperature of the reaction mixture and preferably it is reflux temperature of the reaction mixture.

Reaction time differs according to the raw material compounds, solvent or reaction temperature used, but it is usually from 10 minutes to two hours, preferably from 30 minutes to one hour.

After the reaction terminates, the object compound is collected from the reaction mixture according to a conventional method (extraction, column chromatography, filtration and concentration) as required.

### <Process B>

The compound wherein R¹ is a C₁-C₆ alkyl group; R² is a group R^{a}NH-, R^{a}(R^{b})N- or 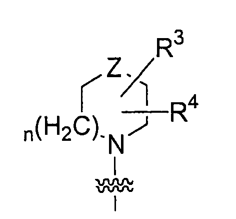 in general formula (I) can be produced according to Process B. 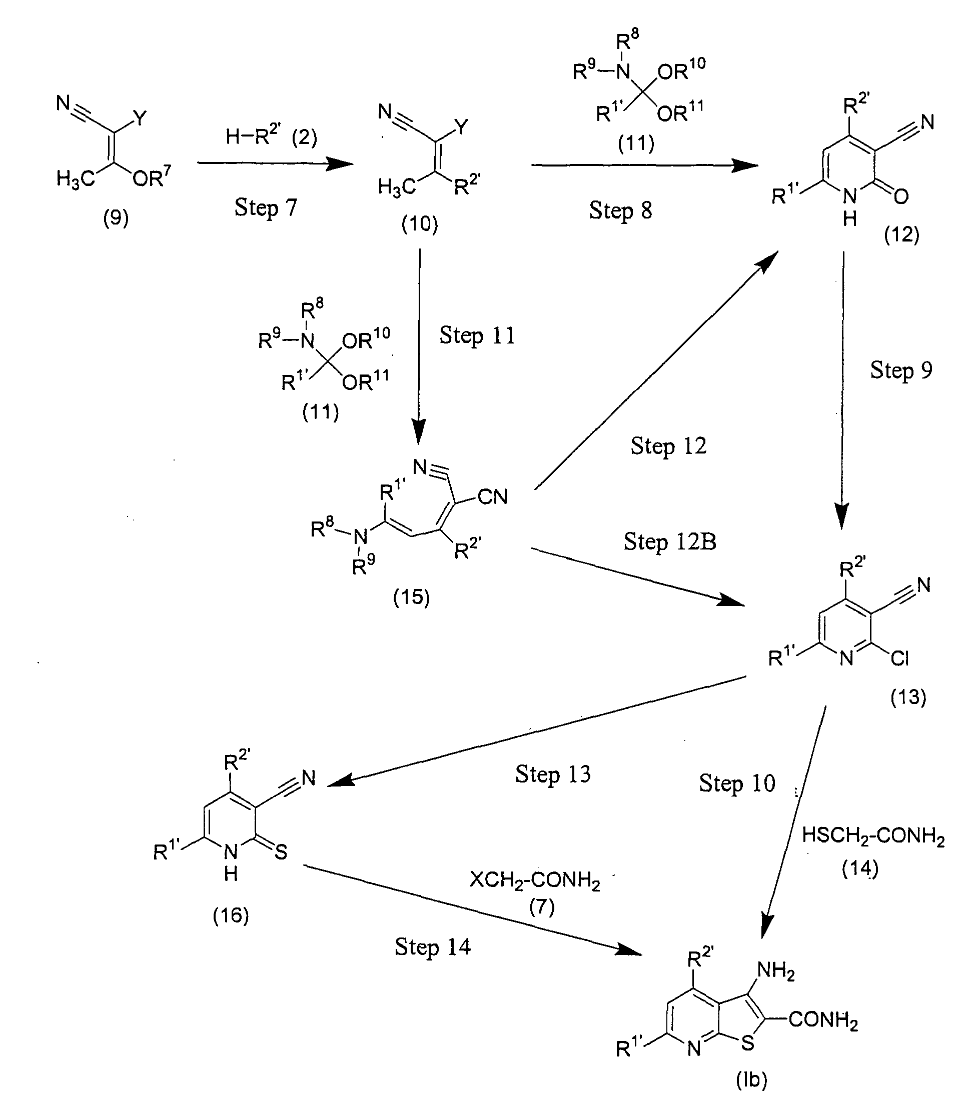 (wherein R^{2'}, R⁸, R⁹, R¹⁰, R¹¹ and X represent the same as defined above, and R^{1'} represents a C₁-C₆ alkyl group in the definition of R¹, and Y represents CONH₂ or CN.)

Step 7 is a step for reacting compound (9) and amine compound (2) in an inert solvent to produce compound (10), and it can be carried out following a similar method as described in Step 1.

Step 8 is a step for reacting a compound (10) in which Y is CONH₂ and (N-N-dialkyl)alkylamide dialkyl acetal (11) in an inert solvent to produce pyridone derivative (12), and it can be carried out following a method described in Pharm. Chem. J. (Engl. Transl.) 25, (1991), 623-628.

The inert solvent to be used is, for example, an aromatic hydrocarbon such as benzene, toluene or xylene; or an amide such as N,N-dimethylformamide, N,N-dimethylacetamide, N-methyl-2-pyrrolidone, N-methylpyrrolidinone or hexamethylphosphorotriamide, and preferably it is an amide and particularly preferably it is N,N-dimethylformamide.

Reaction temperature varies depending on the raw material compounds or solvent used, but it is usually room temperature to reflux temperature of the reaction mixture and preferably it is 50°C to reflux temperature of the reaction mixture.

Reaction time differs according to the raw material compounds, solvent or reaction temperature used, but it is usually from one hour to 24 hours, preferably from one hour to five hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 9 is a step for halogenating pyridone derivative (12) in the presence of a base with a halogenating agent to produce chloropyridine derivative (13).

When the reaction is carried out in an inert solvent, for example, an aromatic hydrocarbon such as benzene, toluene or xylene; or an ether such as diethyl ether, diisopropyl ether, tetrahydrofuran, dioxane or 1,2-dimethoxyethane, and preferably toluene or dioxane, is used as solvent.

The base to be used can be, for example, an organic amine such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) or 1,8-diazabicyclo[5.4.0]-7-undecene (DBU), and particularly preferably N,N-dimethylaniline.

The halogenating agent to be used can be, for example, a phosphorus chloride such as phosphorus trichloride, phosphorous pentachloride or phosphorus oxychloride; or thionyl chloride, and preferably it is phosphorous pentachloride, phosphorus oxychloride or thionyl chloride.

Reaction temperature varies depending on the raw material compounds, solvent, base or halogenating agent, but it is usually room temperature to reflux temperature of the reaction mixture, and preferably it is 50°C to reflux temperature of the reaction mixture.

Reaction time differs according to the raw material compounds, solvent, base, halogenating agent or reaction temperature, but it is usually from one hour to 24 hours, preferably from one hour to eight hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 10 is a step for reacting chloropyridine derivative (13) and 2-mercaptoacetamide (14) in the presence of a base in an inert solvent to produce thienopyridine derivative (Ib).

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; or an amide such as N,N-dimethylformamide, N,N-dimethylacetamide, N-methyl-2-pyrrolidone, N-methylpyrrolidinone or hexamethylphosphorotriamide, preferably it is an alcohol or an amide and more preferably it is ethanol or N,N-dimethylformamide.

The base to be used is, for example, an alkali metal alkoxide such as sodium methoxide, sodium ethoxide, potassium tert-butoxide or lithium methoxide; an alkali metal hydroxide such as sodium hydroxide, potassium hydroxide or lithium hydroxide; or an aqueous solution of an alkali metal hydroxide, and preferably it is sodium ethoxide or an aqueous solution of sodium hydroxide.

Reaction temperature varies depending on the raw material compounds, solvent or base used, but it is usually room temperature to reflux temperature of the reaction mixture.

Reaction time differs according to the raw material compounds, solvent, base or reaction temperature used, but it is usually from one hour to 24 hours, preferably from one hour to two hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

In addition, 2-mercaptoacetamide (14) can also be generated in the reaction system using 2-(acetylthio)acetamide.

Step 11 is a step for reacting a compound (10) in which Y is CN and (N,N-dialkyl)alkylamide dialkyl acetal (11) in an inert solvent to produce enamine derivative (15).

The inert solvent to be used is, for example, an alcohol such as methanol or ethanol; an aromatic hydrocarbon such as benzene, toluene or xylene; or an amide such as N,N-dimethylformamide, N,N-dimethylacetamide, N-methyl-2-pyrrolidone, N-methylpyrrolidinone or hexamethylphosphorotriamide, preferably it is an alcohol or an aromatic hydrocarbon, and particularly preferably it is ethanol, toluene or xylene.

Reaction temperature varies depending on the raw material compounds or solvent used, but it is usually room temperature to reflux temperature of the reaction mixture and preferably it is 100°C to reflux temperature of the reaction mixture.

Reaction time differs according to the raw material compounds, solvent or reaction temperature used, but it is usually from one hour to 24 hours, preferably from one hour to eight hours.

After the reaction terminates, the object compound is collected according to a conventional method as required from the reaction mixture.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 12 is a step for treating enamine derivative (15) with an acid to produce pyridone (12).

The acid to be used can be, for example, an organic acid such as formic acid, acetic acid, trifluoroacetic acid or polyphosphoric acid; or an inorganic acid such as hydrochloric acid, and preferably it is acetic acid or polyphosphoric acid.

When the reaction is carried out in an inert solvent, the solvent can be, for example, an aromatic hydrocarbon such as benzene, toluene or xylene; an amide such as N,N-dimethylformamide, N,N-dimethylacetamide, N-methyl-2-pyrrolidone, N-methylpyrrolidinone or hexamethylphosphorotriamide; an alcohol such as methanol or ethanol; or water or a mixed solvent of water and a solvent mentioned above, and particularly preferably it is water.

Reaction temperature varies depending on the raw material compounds or acid used, but it is usually room temperature to reflux temperature of the reaction mixture and preferably it is 50°C to reflux temperature of the reaction mixture.

Reaction time differs according to the raw material compounds, acid or reaction temperature used, but it is usually from one hour to 24 hours, preferably from one hour to eight hours.

Step 12B is a step for reacting an halogenating agent and enamine derivative (15) to produce chloropyridine derivative (13).

When the reaction is carried out in an inert solvent, for example, an aromatic hydrocarbon such as benzene, toluene or xylene; an ether such as diethyl ether, diisopropyl ether, tetrahydrofuran, dioxane or 1,2-dimethoxyethane; or an alcohol such as methanol, ethanol, propanol, 2-propanol or butanol can be used as solvent, and preferably methanol or ethanol is used.

The halogenating agent to be used can be, for example, a phosphorus chloride such as phosphorus trichloride, phosphorous pentachloride or phosphorus oxychloride; a sulfone chloride such as thionyl chloride; a chlorosilane such as trimethylsilane chloride, t-butyldimethylsilane chloride; an acid chloride such as oxalyl chloride; or an inorganic acid such as hydrochloric acid or hydrobromic acid, and preferably it is thionyl chloride, trimethylsilane chloride or oxalyl chloride.

However, a bromopyridine derivative is obtained when hydrobromic acid is used.

Reaction temperature varies depending on the raw material compounds, solvent or halogenating agent, but it is usually 0°C to reflux temperature of the reaction mixture, and preferably it is room temperature to reflux temperature of the reaction mixture.

Reaction time differs according to the raw material compounds, solvent or halogenating agent, but it is usually from 10 minutes to 24 hours, preferably from 30 minutes to two hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 13 is a step for reacting chloropyridine derivative (13) and thiourea or sodium sulfide (preferably thiourea) in an inert solvent to produce thiopyridone derivative (16).

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; or a mixture of solvents mentioned above, preferably it is an alcohol, an aromatic hydrocarbon or a mixture of an alcohol and aromatic hydrocarbon, and more preferably it is ethanol, toluene or a mixture of ethanol and toluene.

Reaction temperature varies depending on the raw material compounds or solvent used, but it is usually room temperature to reflux temperature of the reaction mixture and preferably it is 50°C to reflux temperature of the reaction mixture.

Reaction time differs according to the raw material compounds, solvent or reaction temperature used, but it is usually from one hour to 48 hours, preferably from one hour to 24 hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

Step 14 is a step for reacting thiopyridone derivative (16) and α-haloacetamide (7) in the presence of a base in an inert solvent to produce thienopyridine derivatives (Ib) and can be carried out by a similar method as described in Step 4.

### <Process C>

The compound wherein R² is R^{a}O- in general formula (I) can be produced according to Process C. 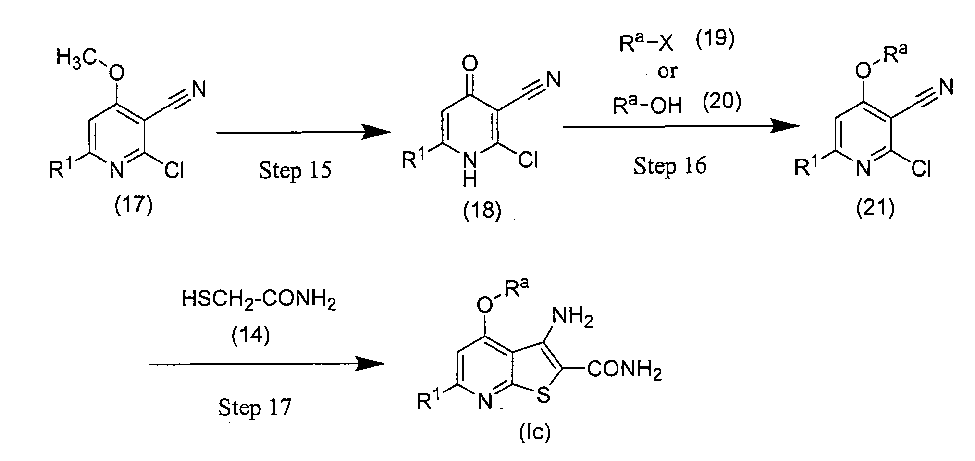 (wherein R¹, R^{a} and X represent the same as defined above.)

Step 15 is a step for demethylating methoxypyridine derivative (17) to produce compound (18) and, for example, it can be carried out by heating a methoxypyridine derivative (17) and concentrated hydrochloric acid under reflux in acetic acid solvent.

Step 16 is a step for (a) reacting compound (18) and halogen compound (19) in the presence of a base in an inert solvent or (b) performing a Mitsunobu reaction using compound (18) and alcohol derivative (20), to produce 4-alkoxypyridine derivative (21).

### (a) A method using halogen compound (19) (Etherification reaction)

The inert solvent to be used is, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a sulfoxide such as dimethylsulfoxide or sulfolane, preferably it is an ether or an amide, and particularly preferably it is tetrahydrofuran or N,N-dimethylacetamide.

The base to be used can be, for example, an alkali metal carbonate such as sodium carbonate or potassium carbonate; an alkali metal hydride such as lithium hydride, sodium hydride or hydrogenation potassium; an alkali metal alkoxide such as sodium methoxide, sodium ethoxide, potassium tert-butoxide or lithium methoxide; or an alkali metal hydroxide such as sodium hydroxide, potassium hydroxide or lithium hydroxide, preferably it is an alkali metal carbonate or alkali metal hydride, and more preferably it is potassium carbonate or sodium hydride.

Reaction temperature varies depending on the raw material compounds, solvent or base used, but it is usually 0°C to reflux temperature of the reaction mixture and preferably it is 0°C to room temperature.

Reaction time differs according to the raw material compounds, solvent, base or reaction temperature used, but it is usually from one hour to 48 hours, preferably from one hour to 24 hours.

### (b) A method using alcohol derivative (20) (Mitsunobu reaction)

The reaction is usually performed in an inert solvent, and the inert solvent used can be, for example, an aromatic hydrocarbon such as benzene, toluene or xylene; or an ether such as diethylether, diisopropylether, tetrahydrofuran, dioxane or 1,2-dimethoxyethane, and preferably it is toluene or tetrahydrofuran.

The reagent used for the Mitsunobu reaction is a combination of, for example, an azo compound such as a di(C₁-C₆ alkyl) azodicarboxylate such as diethyl azodicarboxylate or diisopropyl azodicarboxylate or an azodicarbonyl such as 1,1'-(azodicarbonyl)dipiperidine, and a phosphine such as a tri(C₆-C₁₀ aryl)phosphine such as triphenylphosphine or a tri(C₁-C₆ alkyl)phosphine such as tri-n-butylphosphine, more preferably it is a combinaticn of di(C₁-C₆ alkyl) azodicarboxylate and tri(C₆-C₁₀ aryl)phosphine, and most preferably it is a combination of diethyl azodicarboxylate and triphenylphosphine.

Reaction temperature varies depending on the raw material compounds, solvent or chemical reagent used, but it is usually 0°C to reflux temperature of the solvent and preferably it is 0°C to room temperature.

Reaction time differs according to the raw material compounds, solvent, reagent or reaction temperature used, but it is usually from one hour to 48 hours, preferably from one hour to 24 hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography. It is length of time.

Step 17 is a step for reacting 4-alkoxypyridine derivative (21) and 2-mercaptoacetamide (14) in the presence of a base in an inert solvent to produce thienopyridine derivative (Ic) and it can be carried out by a similar method as described in Step 10.

### <Process D>

The compound wherein R² is R^{a}S- in general formula (I) can be produced according to Process D. 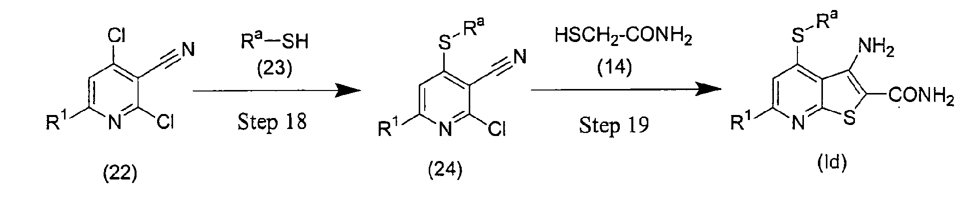 (wherein R¹, R^{a} and X represent the same as defined above.)

Step 18 is a step for reacting dichloropyridine compound (22) and thiol compound (23) or an alkali metal salt thereof (for example, sodium salt) in the presence or absence of a base in an inert solvent to produce 4-alkylthio pyridine derivative (24).

The inert solvent to be used can be, for example, an alcohol such as methanol, ethanol, propanol, 2-propanol or butanol; an aromatic hydrocarbon such as benzene, toluene or xylene; an ether such as diethyl ether, diisopropyl ether, tetrahydrofuran, dioxane or 1,2-dimethoxyethane; an amide such as N,N-dimethylformamide, N,N-dimethylacetamide, N-methyl-2-pyrrolidone, N-methylpyrrolidinone or hexamethylphosphorotriamide; or a sulfoxide such as dimethylsulfoxide or sulfolane, preferably it is an ether or an amide, and particularly preferably it is tetrahydrofuran or N,N-dimethylacetamide.

The base to be used can be, for example, an alkali metal hydride such as lithium hydride, sodium hydride or hydrogenation potassium; an alkali metal alkoxide such as sodium methoxide, sodium ethoxide, potassium tert-butoxide or lithium methoxide; or an alkali metal hydroxide such as sodium hydroxide, potassium hydroxide or lithium hydroxide, and preferably it is sodium hydride or sodium hydroxide.

Reaction temperature varies depending on the raw material compounds, solvent or base used, but it is usually 0°C to reflux temperature of the reaction mixture and preferably it is 0°C to room temperature.

Reaction time differs according to the raw material compounds, solvent, base or reaction temperature used, but it is usually from one hour to 24 hours, preferably from one hour to six hours.

After the reaction terminates, the object compound is collected from the reaction mixture according to a conventional method as required.

For example, the object compound can be obtained by neutralizing the reaction mixture appropriately and removing by filtration insolubles if present, adding water, extracting with a water-immiscible organic solvent such as ethyl acetate or toluene and washing with water and the like, and evaporating the solvent after drying over anhydrous magnesium sulfate and the like.

If necessary, the obtained compound can be separated and purified by a conventional method, for example by silica gel column chromatography.

In addition, 4-alkoxypyridine derivatives corresponding to compound (24) can be produced by substituting an alcohol derivative (20) for thiol compound (23).

Step 19 is a step for reacting 4-alkylthiopyridine derivative (24) and 2-mercaptoacetamide (14) in the presence of a base in an inert solvent to produce thienopyridine derivative (Id), and it is carried out by a method similar to the method described in Step 10.

Since the compounds having a general formula (I) of the present invention or pharmacologically acceptable salts thereof have effects of promoting osteogenesis, suppressing bone resorption and/or improving bone density, they are useful as a pharmaceutical composition {preferably a pharmaceutical composition for prevention or treatment of osteopathy [for example, osteoporosis (for example, postmenopausal osteoporosis, senile osteoporosis or secondary osteoporosis caused by use of steroid or immunosuppressant), osteopenia or bone destruction associated with rheumatoid arthritis, Paget's disease of bone, bone fracture or dysostosis due to dwarfism] or osteoarthritis} and can be administered to a mammal (for example, a human, horse, cow or pig, preferably a human) or a bird (preferably a chicken, more preferably a female chicken). The mode for administration can be, for example, oral administration by tablets, capsules, granules, powders or syrup, or parenteral administration by injection or suppository, etc., and preparations for those purposes can be produced by well-known methods using additives such as excipients, lubricants, binders, disintegrants, stabilizing agents, corrigents, diluents.

Excipients can be, for example, organic excipients such as a sugar derivative such as lactose, sucrose, glucose, mannitol or sorbitol, a starch derivative such as corn starch, potato starch, α-starch, dextrin or carboxymethyl starch, a cellulose derivative such as crystalline cellulose, low substituted hydroxypropyl cellulose, hydroxypropyl methyl cellulose, carboxymethyl cellulose, calcium carboxymethyl cellulose or internally crosslinked sodium carboxymethyl cellulose, gum arabic, dextran or pullulan; or inorganic excipients such as a silicate derivative such as light anhydrous silicic acid, synthetic aluminium silicate or magnesium aluminometasilicate, a phosphate such as calcium phosphate, a carbonate such as calcium carbonate, or calcium sulphate.

Lubricants can be, for example, stearic acid or a metal stearate such as calcium stearate or magnesium stearate; talc; colloidal silica; wax such as bee gum or spermaceti; boric acid; adipic acid; sulfate such as sodium sulfate; glycol; fumaric acid; sodium benzoate; DL-leucine; sodium salt of fatty acid; a lauryl sulfate such as sodium lauryl sulfate or magnesium lauryl sulfate; silicic acid such as silicic anhydride or silicic acid hydrate; or a starch derivative as mentioned above.

Binders can be, for example, polyvinylpyrrolidone or macrogol or a compound which is similar to the above excipients.

Disintegrants can be, for example, a compound which is similar to the above excipients or a chemically modified starch and/or cellulose such as cross sodium carmellose, sodium carboxymethyl starch or crosslinked polyvinylpyrrolidone.

Stabilizing agents can be, for example, a paraoxybenzoic acid ester such as methylparaben or propylparaben; an alcohol such as chlorobutanol, benzyl alcohol or phenylethyl alcohol; benzalkonium chloride; a phenol such as phenol or cresol; thimerosal; dehydroacetic acid; or sorbic acid.

Corrigents can be, for example, a sweetner, acidulant or flavouring agent usually used.

The amount of the compound having a general formula (I) of the present invention or its pharmacologically acceptable salt varies depending on the symptoms, age, administration method, but it is desirable to administer, for example, 0.0001 mg/kg (preferably, 0.001 mg/kg) for the lower limit and 100 mg/kg (preferably, 10 mg/kg) for the upper limit, in a single dose or divided into multiple doses per day, depending on the symptoms, in the case of oral administration. In the case of intravenous administration, it is desirable to administrate 0.0001 mg/kg (preferably, 0.001 mg/kg) for the lower limit and 1 mg/kg (preferably, 0.1 mg/kg) for the upper limit, in a single dose or divided into multiple doses, per day for an adult depending on the symptoms.

### Effects of the Invention

Since the compound having a general formula (I) of the present invention or pharmacologically acceptable salts thereof have effects of promoting osteogenesis, suppressing bone resorption and/or improving bone density, they are useful as a pharmaceutical composition {preferably a pharmaceutical composition for prevention or treatment of osteopathy [for example, osteoporosis (for example, postmenopausal osteoporosis, senile osteoporosis or secondary osteoporosis caused by the use of steroids or immunosuppressants), osteopenia or bone destruction associated with rheumatoid arthritis, Paget's disease of bone, bone fracture or dysostosis due to dwarfism] or osteoarthritis}.

### Best Mode for Carrying out the Invention

The present invention will be described in more detail by way of the Examples, Preparation Examples and Test Examples below but the present invention is not limited to these.

### (EXAMPLES)

### (Example 1) 3-amino-4-(dimethylamino)thieno[2,3-b]pyridine-2-carboxamide (Exemplified Compound No. 2-17)

The compound was produced by the following method with reference to a method described in Pharm.Chem.J.(Engl.Transl.),26,(1992),870-874. (1a) (2Z)-2-cyano-3-(dimethylamino)but-2-enethioamide

Cyanothioacetamide (1.00 g, 10 mmol) and N,N-dimethylacetamide dimethylacetal (1.73 g, 13 mmol) were dissolved in acetonitrile (5 mL) and the mixture was stirred at room temperature for one hour. The deposited crystal was filtered and the crystal was further washed with acetonitrile and 1.05 g (yield 62%) of the title compound was obtained.
Mp 155-158°C;
¹H NMR DMSO-d₆, 400MHz) δ 2.27 (3H, s), 3.03 (6H, s), 8.08 (1H, br), 8.83 (1H, br).
(1b) 4-(dimethylamino)-2-thioxo-1,2-dihydropyridine-3-carbonitrile

(2Z)-2-cyano-3-(dimethylamino)but-2-enethioamide (1.05 g, 6.2 mmol) produced in Example 1 (1a) and N,N-dimethylformamide dimethylacetal (2.22 g, 18.6 mmol) were dissolved in toluene (10 mL) and the mixture was stirred under heat reflux for two hours. The residue obtained by concentrating the mixture under reduced pressure was suspended in 1N aqueous solution of sodium hydroxide (10 mL) and heated under reflux for 30 minutes. After the reaction mixture was cooled to room temperature, 1N hydrochloric acid (15 mL) was added. The deposited solid was filtered and washed with water and ethanol and 0.87 g (yield 78%) of the title compound was obtained.
Mp 246-250°C;
¹H NMR (DMSO-d₆, 400MHz) δ 3.12 (6H, s), 6.25 (1H, d, J = 7.3 Hz), 7.29-7.33 (1H, m), 12.40 (1H, br).
(1c) 3-amino-4-(dimethylamino)thieno[2,3-b]pyridine-2-carboxamide

8N aqueous solution of sodium hydroxide (2 mL) and 2-chloroacetamide (0.54 g, 5.8 mmol) were added to an N,N-dimethylformamide (10 mL) solution of 4-(dimethylamino)-2-thioxo-1,2-dihydropyridine-3-carbonitrile (0.87 g) produced in Example 1 (1b), and the mixture was stirred at room temperature for one hour. Water (100 mL) was added to the reaction mixture, and the deposited crystal was filtered and further washed with water and ethanol and 0.79 g (yield 54%) of the title compound was obtained.
Mp 208-211 °C;
IR (KBr) νₘₐₓ 3430, 3296, 3132, 1673, 1582, 1372, 979 cm⁻¹;
¹H NMR (DMSO-d₆, 400MHz) δ 2.80 (6H, s), 6.96 (1H, d, J = 5.5 Hz), 6.97 (2H, br), 7.04 (2H, br), 8.36 (1H, d, J = 5.5 Hz);
MS (FAB) m/z: 237 [M+H]⁺;
Anal. Calcd for C₁₀H₁₂N₄SO: C, 50.83; H, 5.12; N, 23.71; S, 13.57. Found: C, 50.70; H, 4.98; N, 23.53; S, 13.38.

### (Example 2) 3-amino-4-(diethylamino)thieno[2,3-b]pyridine-2-carboxamide (Exemplified Compound No. 2-33)

### (2a) (2Z)-2-cyano-3-(diethylamino)but-2-enethioamide

(2Z)-2-cyano-3-ethoxybut-2-enethioamide (J. Org. Chem., (1962), 27, 2433-2439) (406 mg, 2.38 mmol) and diethylamine (0.36 mL, 3.53 mmol) were suspended in ethanol (5 mL) and the mixture was stirred at room temperature for two hours. After the solvent was evaporated, the obtained residue was purified by silica gel column chromatography (ethyl acetate/hexane =2:1) and the title compound was obtained (237 mg, yield 50%).
¹H NMR(CDCl₃, 400 MHz) δ 1.32 (6H, t, J = 7.04 Hz), 2.71 (3H, s), 3.65 (4H, q, J = 7.05 Hz), 6.69 (2H, br s).
(2b) 4-(diethylamino)-2-thioxo-1,2-dihydropyridine-3-carbonitrile

(2Z)-2-cyano-3-(diethylamino)but-2-enethioamide produced in Example 2 (2a) was used in place of (2Z)-2-cyano-3-(dimethylamino)but-2-enethioamide and reacted in a similar method as described in Example 1 (1b) and the title compound was obtained. Yield 54%.
¹H NMR(DMSO-d₆, 400 MHz) δ 1.19 (6H, t, J = 6.8 Hz), 3.21 (1H, s), 3.615 (4H, q, J = 6.8 Hz), 6.34 (1H, d, J = 7.8 Hz), 7.36 (1H, t, J = 7.8 Hz).
(2c) 3-amino-4-(diethylamino)thieno[2,3-b]pyridine-2-carboxamide

4-(diethylamino)-2-thioxo-1,2-dihydropyridine-3-carbonitrile produced in Example 2 (2b) was used in place of 4-(diethylamino)-2-thioxo-1,2-dihydropyridine-3-carbonitrile and reacted in a similar method as described in Example 1 (1c) and the title compound was obtained. Yield 65%.
Mp 127-129°C;
IR (KBr) νₘₐₓ 3426, 3304, 3143, 2974, 1672, 1647, 1581, 1504, 1376, 1345, 1262, 1158, 1050, 790, 616 cm⁻¹;
¹H NMR(DMSO-d₆, 400 MHz) δ 0.98 (6H, t, J = 7.0 Hz), 3.17 (4H, q, J = 7.0 Hz), 7.06 (1H, d, J = 5.1 Hz), 7.09 (2H, s), 7.36 (1H, d, J = 5.1 Hz); MS (FAB) m/z: 264.10 [M + H]⁺;
Anal. Calcd for C₁₂H₁₆N₄OS: C, 54.52; H, 6.10; N, 21.19; S, 12.13. Found: C, 54.18; H, 5.86; N, 21.34; S, 12.18.

### (Example 3) 3-amino-4-(dimethylamino)-6-methylthieno[2,3-b]pyridine-2-carboxamide (Exemplified Compound No. 2-102)

### (3a) 4-(dimethylamino) -6-methyl-2-thioxo-1,2-dihydropyridine-3-carbonitrile

2-chloro-4-(dimethylamino)-6-methylnicotinonitrile (Pharm.Chem.J., (Engl. Transl.),25,(1991),623-628.) (1.46 g, 7.5 mmol) and thiourea (0.74 g, 9.7 mmol) were suspended in toluene (25 mL) and the mixture was stirred under reflux for four hours. Ethanol (40 mL) was added to the reaction mixture and further heated under reflux for 30 minutes. The solid which deposited after allowing to stand overnight at room temperature was filtered and washed with ethanol, water, ethanol sequentially and the title compound was obtained as a crude product (0.64 g).
¹H NMR (DMSO-d₆, 400MHz) δ 2.20 (3H, s), 3.18 (6H, s), 6.23 (1H, s), 12.41 (1H, br).
(3b) 3-amino-4-(dimethylamino)-6-methylthieno[2,3-b]pyridine-2-carboxamide

4-(dimethylamino)-6-methyl-2-thioxo-1,2-dihydropyridine-3-carbonitrile (0.19 g, 1.0 mmol) produced in Example 3 (3a) was dissolved in N,N-dimethylformamide (3 mL) and 8N aqueous solution of sodium hydroxide (0.5 mL) and 2-chloroacetamide (0.11 g, 1.2 mmol) were added. After the mixture was stirred at room temperature for one hour, water (50 mL) was added. The aqueous layer was extracted with ethyl acetate (3x50 mL) and the extract was concentrated after drying over sodium sulfate under reduced pressure. The residue was purified by silica gel column chromatography (100% ethyl acetate) and 0.16 g of the title compound (yield 62%) was obtained.
Mp 167-170°C;
IR (KBr) νₘₐₓ 3442, 3327, 3170,1647,1580,1368, 992 cm⁻¹;
¹H NMR (DMSO-d₆, 400MHz) δ 2.48 (3H, s), 2.78 (6H, s), 6.84 (1H, s), 6.93 (2H, br), 6.95 (2H, br);
MS (EI) m/z: 250 [M⁺], 218, 205, 190;
Anal. Calcd for C₁₁H₁₄N₄SO·0.5 H₂O: C, 50.95; H, 5.83; N, 21.60; S, 12.36. Found: C, 50.84; H, 5.94; N, 21.51; S, 12.19.

### (Example 4) 4-(isobutylamino)-2-thioxo-1,2-dihydropyridine-3-carbonitrile (Exemplified Compound No. 3-3)

### (4a) (2Z)-2-cyano-3-(propylamino)but-2-enethioamide

(2Z)-2-cyano-3-ethoxybut-2-enethioamide (J.Org.Chem.,(1962),27,2433-2439) (0.34 g, 2.0 mmol) and propylamine (0.15 g, 2.5 mmol) were suspended in ethanol (5 mL) and the mixture was stirred at room temperature for 15 hours. The deposited solid was separated by filtration and further washed with ethanol and 0.29 g of the title compound (yield 79%) was obtained.
Mp 149-151 °C;
IR (KBr) νₘₐₓ 3400, 3287, 3187, 2190, 1612 cm⁻¹;
¹H NMR (DMSO-d₆, 400MHz) δ 0.97 (3H, t, J = 7.4 Hz), 1.55-1.64 (2H, m), 2.30 (3H, s), 3.35-3.40 (2H, m), 7.65 (1H, br), 8.45 (1H, br), 12.74 (1H, br);
MS (FAB) m/z: 184 [M+H]⁺;
Anal. Calcd for C₈H₁₃N₃S: C, 52.43; H, 7.15; N, 22.93; S, 17.49. Found: C, 52.59; H, 7.25; N, 22.83; S, 17.51.

### (4b) 3-amino-4-(propylamino)thieno[2,3-b]pyridine-2-carboxamide

(2Z)-2-cyano-3-(propylamino)but-2-enethioamide (0.29 g, 1.6 mmol) which was produced in Example 4 (4a) and N,N-dimethylformamide dimethylacetal (0.57 g, 4.7 mmol) were mixed with toluene (3 mL) and the mixture was stirred under heat reflux for two hours. 1N aqueous solution of sodium hydroxide (5 mL) was added to the residue which was obtained by evaporating the solvent under reduced pressure and the mixture was stirred under heat reflux for 30 minutes. After the reaction mixture was cooled to room temperature, ether (20 mL) was added and the liquid was partitioned. Furthermore, the organic layer was extracted with 1N aqueous solution of sodium hydroxide (5 mL). The combined aqueous layer was neutralized with 1N hydrochloric acid, and the deposited solid was separated by filtration and further washed with water and a little amount of ethanol and a solid (0.17 g) mainly containing 2-thioxo-1,2-dihydropyridine derivative was obtained.

The obtained solid was dissolved in N,N-dimethylformamide (3 mL) and 8N aqueous solution of sodium hydroxide (0.5 mL) and 2-chloroacetamide (0.10 g, 1.1 mmol) were added. After the mixture was stirred at room temperature for one hour, water (3 mL) was added. The deposited solid was separated by filtration and washed with water and ethanol and 106 mg of the title compound was obtained. Yield 27% from (2Z)-2-cyano-3-(propylamino)but-2-enethioamide.
Mp 214-215°C;
IR (KBr) νₘₐₓ 3348, 3319, 3189, 1650, 1592, 1504, 1364 cm⁻¹;
¹H NMR (DMSO-d₆, 400MHz) δ 0.95 (3H, t, J = 7.4 Hz), 1.59-1.68 (2H, m), 3.16-3.21 (2H, m), 6.41 (1H, d, J = 5.6 Hz), 6.44 (1H, brt, J = 5.4 Hz), 6.81 (2H, br), 7.02 (2H, br), 8.05 (1H, d, J = 5.6 Hz);
MS (EI) m/z: 250 [M⁺], 204;
Anal. Calcd for C₁₁H₁₄N₄OS·1.1H₂O: C, 48.91; H, 6.04; N, 20.74; S, 11.87. Found: C, 49.06; H, 5.92; N, 20.71; S, 11.90.

### (Example 5) 3-amino-4-(isobutylamino)thieno[2,3-b]pyridine-2-carboxamide (Exemplified Compound No. 2-6)

### (5a) (2Z)-2-cyano-3-(isobutylamino)but-2-enethioamide

(2Z)-2-cyano-3-ethoxybut-2-enethioamide (J.Org.Chem.,(1962),27,2433-2439) (340 mg, 2.0 mmol) and isobutylamine (183 mg, 2.5 mmol) were suspended in ethanol (3 mL) and the mixture was stirred at room temperature for six hours. After the deposited solid was separated by filtration, it was washed with ethanol and 378 mg of the title compound (96%) was obtained.
Mp 165-167°C;
IR (KBr) νₘₐₓ 3373, 3291, 3198, 2190, 1608 cm⁻¹;
¹H NMR (DMSO-d₆, 400MHz) δ 0.97 (6H, d, J = 6.7 Hz), 1.82-1.92 (1H, m), 2.30 (3H, s), 3.25-3.28 (2H, m), 7.67 (1H, br), 8.48 (1H, br), 12.76 (1H, br);
MS (FAB) m/z: 198 [M+H]⁺;
Anal. Calcd for C₉H₁₅N₃S: C, 54.79; H, 7.66; N, 21.30; S, 16.25. Found: C, 54.73; H, 7.84; N, 21.24; S, 16.18.

### (5b) 4-(isobutylamino)-2-thioxo-1,2-dihydropyridine-3-carbonitrile

(2Z)-2-cyano-3-(isobutylamino)but-2-enethioamide (360 mg, 1.8 mmol) which was produced in Example 5 (5a) and N,N-dimethylformamide dimethylacetal (652 mg, 5.5 mmol) were mixed with toluene (5 mL) and the mixture was stirred under reflux for two hours. 1N aqueous solution of sodium hydroxide (5 mL) was added to the residue which was obtained by evaporating the solvent under reduced pressure, and it was stirred under reflux for 30 minutes. After the reaction mixture was cooled to room temperature, the liquid was partitioned with ether (50 mL) and water (20 mL). The obtained aqueous layer was neutralized with 1N hydrochloric acid (5 mL) and the deposited solid was separated by filtration and further washed with water and a little amount of ethanol and 217 mg of a solid containing the title compound was obtained.
¹H NMR (DMSO-d₆, 400MHz) δ 0.87 (6H, d, J = 6.7 Hz), 1.78-1.88 (1H, m), 3.06-3.12 (2H, m), 6.33 (1H, d, J = 7.4 Hz), 7.41 (1H, br), 7.50 (1H, br), 12.35 (1H, br).

### (5c) 3-amino-4-(isobutylamino)thieno[2,3-b]pyridine-2-carboxamide

Crude product (215 mg, 1.0 mmol) of 4-(isobutylamino)-2-thioxo-1,2-dihydropyridine-3-carbonitrile which was produced in Example 5 (5b) was dissolved in N,N-dimethylformamide (3 mL) and 8N aqueous solution of sodium hydroxide (0.3 mL) and 2-chloroacetamide (126 mg, 1.3 mmol) were added. Water (3 mL) was added after the mixture was stirred at room temperature for one hour. The deposited solid was separated by filtration and washed with water and ethanol and 109 mg of the title compound was obtained. Yield 23% from 4-(isobutylamino)-2-thioxo-1,2-dihydropyridine-3-carbonitrile.
Mp 117-119°C;
IR (KBr) νₘₐₓ 3399, 3352, 3250, 3121, 1676, 1595, 860 cm⁻¹;
¹H NMR (DMSO-d₆, 400MHz) δ 0.95 (6H, d, J = 6.7 Hz), 1.90-2.01 (1H, m), 3.04-3.08 (2H, m), 6.43 (1H, d, J = 5.5 Hz), 6.52 (1H, br), 6.83 (2H, br), 7.08 (2H, br), 8.07 (1H, d, J = 5 .5 Hz);
MS (EI) m/z: 264 [M⁺], 204;
Anal. Calcd for C₁₂H₁₆N₄OS·0.1H₂O: C, 54.15; H, 6.14; N, 21.05; S, 12.05. Found: C, 54.11; H, 5.94; N, 21.06; S, 12.17.

### (Example 6) 3-amino-4-(neopentylamino)thieno[2,3-b]pyridine-2-carboxamide (Exemplified Compound No. 2-7)

### (6a) (2Z)-2-cyano-3-(neopentylamino)but-2-enethioamide

Neopentylamine was used in place of propylamine and the reaction was performed in a similar method as described in Example 4 (4a) and the title compound was obtained. Yield 54%.
Mp 143-144°C;
IR (KBr) νₘₐₓ 3376, 3297, 3202, 2188, 1607 cm⁻¹;
¹H NMR (DMSO-d₆, 400MHz) δ 0.99 (9H, s), 2.30 (3H, s), 3.23 (2H, d, J = 5.5 Hz), 7.67 (1H, br), 8.49 (1H, br), 12.78 (1H, br);
MS (FAB) m/z: 212 [M+H]⁺;
Anal. Calcd for C₁₀H₁₇N₃S: C, 56.84; H, 8.11; N, 19.88; S, 15.17. Found: C, 56.59; H, 8.09; N, 19.76; S, 14.90.

### (6b) 3-amino-4-(neopentylamino)thieno[2,3-b]pyridine-2-carboxamide

(2Z)-2-cyano-3-(neopentylamino)but-2-enethioamide which was produced in Example 6 (6a) was used in place of (2Z)-2-cyano-3-(propylamino)but-2-enethioamide and the reaction was performed in a similar method as described in Example 4 (4b) and the title compound was obtained. Yield 16%.
Mp 238-240°C;
IR (KBr) νₘₐₓ 3318, 3190, 1653, 1587, 1105 cm⁻¹;
¹H NMR (DMSO-d₆, 400MHz) δ 0.97 (9H, s), 3.08 (2H, d, J = 6.1 Hz), 6.46 (1H, brt, J = 6.1 Hz), 6.54 (1H, d, J = 5.7 Hz), 6.63 (2H, br), 7.14 (2H, br), 8.06 (1H, d, J = 5.7 Hz);
MS (FAB) m/z: 279 [M+H]⁺;
Anal. Calcd for C₁₃H₁₈N₄OS·1.2H₂O: C, 52.05; H, 6.85; N, 18.68; S, 10.69. Found: C, 52.31; H, 6.69; N, 18.68; S, 10.67.

### (Example 7) 3-amino-4-(benzylamino)thieno[2,3-b]pyridine-2-carboxamide (Exemplified Compound No. 2-11)

### (7a) (2Z)-3-(benzylamino) -2-cyanobut-2-enethioamide

Benzylamine was used in place of propylamine and the reaction was performed in a similar method as described in Example 4 (4a) and the title compound was obtained. Yield 97%.
Mp 157-159°C;
IR (KBr) νₘₐₓ 3355, 3287, 3193, 2193, 1627, 1608, 853, 739 cm⁻¹;
¹H NMR (DMSO-d₆, 400MHz) δ 2.34 (3H, s), 4.69 (2H, d, J = 5.6 Hz), 7.31-7.43 (5H, m), 7.74 (1H, br), 8.55 (1H, br), 13.02 (1H, br);
MS (FAB) m/z: 232 [M+H]⁺;
Anal. Calcd for C₁₂H₁₃N₃S: C, 62.31; H, 5.66; N, 18.17; S, 13.86. Found: C, 62.19; H, 5.88; N, 18.27; S, 13.66.

### (7b) 4-(benzylamino)-2-thioxo-1,2-dihydropyridine-3-carbonitrile

(2Z)-3-(benzylamino) -2-cyanobut-2-enethioamide (0.41 g) which was produced in Example 7 (7a) was used in place of (2Z)-2-cyano-3-(dimethylamino)but-2-enethioamide and the reaction was performed in a similar method as described in Example 1 (1b) and a crude product (0.38 g) of the title compound was obtained.
¹H NMR (DMSO-d₆, 400MHz) δ 4.54 (2H, d, J = 6.3 Hz), 6.19 (1H, d, J = 7.4 Hz), 7.24-7.44 (5H, m), 8.12 (1H, br), 12.45 (1H, br).

### (7c) 3-amino-4-(benzylamino)thieno[2,3-b]pyridine-2-carboxamide

A crude product (0.38 g) of 4-(benzylamino)-2-thioxo-1,2-dihydropyridine-3-carbonitrile which was produced in Example 7 (7b) was used in place of 4-(dimethylamino)-2-thioxo-1,2-dihydropyridine-3-carbonitrile and the reaction was performed in a similar method as described in Example 1 (1c), and 0.39 g of the title compound was obtained. Yield 73% from (2Z)-3-(benzylamino)-2-cyanobut-2-enethioamide.
Mp 260-262°C;
IR (KBr) νₘₐₓ 3460, 3394, 3351, 3112, 1654, 1627, 1598 cm⁻¹;
¹H NMR (DMSO-d₆, 400MHz) δ 4.52 (2H, d, J = 5.8 Hz), 6.28 (1H, d, J = 5.7 Hz), 6.95 (2H, br), 7.07 (2H, br), 7.16 (1H, brt, J = 5.8 Hz), 7.23-7.42 (5H, m), 8.00 (1H, d, J = 5.7 Hz);
MS (FAB) m/z: 299 [M+H]⁺;
Anal. Calcd for C₁₅H₁₄N₄OS: C, 60.38; H, 4.73; N, 18.78; S, 10.75. Found: C, 60.37; H, 4.85; N, 18.87; S, 10.65.

### (Example 8) 3-amino-4-[(2-phenethyl)amino]thieno[2,3-b]pyridine-2-carboxamide (Exemplified Compound No. 2-12)

### (8a) (2Z)-2-cyano-3-[(2-phenethyl)amino]but-2-enethioamide

Phenethylamine was used in place of propylamine, the reaction was performed in a similar method as described in Example 4 (4a) and the title compound was obtained. Yield 75%.
Mp 95-96°C;
IR (KBr) νₘₐₓ 3353, 3300, 3203, 2189, 1626, 1595, 749, 699 cm⁻¹;
¹H NMR (DMSO-d₆, 400MHz) δ 2.22 (3H, s), 2.89 (2H, t, J = 7.2 Hz), 3.64-3.69 (1H, m), 7.18-7.31 (5H, m), 7.60 (1H, br), 8.40 (1H, br), 12.70 (1H, br);
MS (FAB) m/z: 246 [M+H]⁺;
Anal. Calcd for C₁₃H₁₅N₃S: C, 63.64; H, 6.16; N, 17.13; S, 13.07. Found: C, 63.73; H, 6.11; N, 17.20; S, 13.14.

### (8b) 3-amino-4-[(2-phenethyl)amino]thieno[2,3-b]pyridine-2-carboxamide

(2Z)-2-cyano-3-[(2-phenethyl)amino]but-2-enethioamide (0.37 g, 1.5 mmol) which was produced in Example 8 (8a) was used in place of (2Z)-2-cyano-3-(dimethylamino)but-2-enethioamide and the reaction was performed in a similar method as described in Example 1 (1b) and 0.34 g of a crude product of 2-thioxo-1,2-dihydropyridine derivative was obtained.

The reaction was performed in a similar method as described in Example 1 (1c) using the obtained 2-thioxo-1,2-dihydropyridine derivative and 0.25 g of the title compound was obtained (yield 53%).
Mp 206-208°C;
IR (KBr) νₘₐₓ 3449, 3358, 3121, 1656, 1598, 1514, 1103, 753, 703 cm⁻¹;
¹H NMR (DMSO-d₆, 400MHz) δ 2.93 (2H, t, J = 7.5 Hz), 3.44-3.49 (2H, m), 6.49 (1H, d, J = 5.7 Hz), 6.50 (1H, br), 6.76 (2H, br), 7.04 (2H, br), 7.18-7.32 (5H, m), 8.07 (1H, d, J = 5.7 Hz);
MS (FAB) m/z: 313 [M+H]⁺;
Anal. Calcd for C₁₆H₁₆N₄OS: C, 61.52; H, 5.16; N, 17.93; S, 10.26. Found: C, 61.47; H, 5.25; N, 17.95; S, 10.33.

### (Example 9) 3-amino-4-[(3-phenylpropyl)amino]thieno[2,3-b]pyridine-2-carboxamide (Exemplified Compound No. 2-13)

### (9a) (2Z)-2-cyano-3-[(3-phenylpropyl)amino]but-2-enethioamide

3-phenyl propylamine was used in place of propylamine, the reaction was performed in a similar method as described in Example 4 (4a) and the title compound was obtained. Yield 94%.
Mp 125-126°C;
IR (KBr) νₘₐₓ 3399, 3285, 3173, 2189, 1612, 1603, 754, 531 cm⁻¹ ;
¹H NMR (DMSO-d₆, 400MHz) δ 1.85-1.92 (2H, m), 2.27 (3H, s), 2.70 (2H, t, J = 7.7 Hz), 3.37-3.42 (2H, m), 7.16-7.29 (5H, m), 7.64 (1H, br), 8.45 (1H, br), 12.79 (1H, br);
MS (FAB) m/z: 260 [M+H]⁺;
Anal. Calcd for C₁₄H₁₇N₃S: C, 64.85; H, 6.61; N, 16.20; S, 12.36. Found: C, 65.02; H, 6.52; N, 16.28; S, 12.37.

### (9b) 3-amino-4-[(3-phenylpropyl)amino]thieno[2,3-b]pyridine-2-carboxamide

(2Z)-2-cyano-3-[(3-phenylpropyl)amino]but-2-enethioamide (0.49 g, 1.9 mmol) which was produced in Example 9 (9a) was used in place of (2Z)-2-cyano-3-(dimethylamino)but-2-enethioamide and the reaction was performed in a similar method as described in Example 1 (1b) and a crude product of 2-thioxo-1,2-dihydropyridine derivative (0.45 g) was obtained.

The reaction was performed in a similar method as described in Example 1 (1c) using the obtained 2-thioxo-1,2-dihydropyridine derivative and 0.30 g of the title compound was obtained (yield 49%).
Mp 232-234°C;
IR (KBr) νₘₐₓ 3424, 3326, 3125, 1659, 1603, 1517, 1362 cm⁻¹;
¹H NMR (DMSO-d₆, 400MHz) δ 1.89-1.97 (2H, m), 2.69 (2H, t, J = 7.6 Hz), 3.21-3.26 (2H, m), 6.37 (1H, d, J = 5.6 Hz), 6.47 (1H, brt, J = 5.3 Hz), 6.81 (2H, br), 7.02 (2H, br), 7.15-7.29 (5H, m), 8.04 (1H, d, J = 5.6 Hz);
MS (FAB) m/z: 327 [M+H]⁺;
Anal. Calcd for C₁₇H₁₈N₄OS: C, 62.55; H, 5.56; N, 17.16; S, 9.82. Found: C, 62.33; H, 5.76; N, 17.32; S, 9.68.

### (Example 10) 3-amino-4-(cyclopentylamino)thieno[2,3-b]pyridine-2-carboxamide (Exemplified Compound No. 2-8)

### (10a) (2Z)-2-cyano-3-(cyclopentylamino)but-2-enethioamide

Cyclopentylamine was used in place of isobutylamine, the reaction was performed in a similar method as described in Example 5 (5a) and the title compound was obtained. Yield 89%.
Mp 162-163°C;
IR (KBr) νₘₐₓ 3368, 3287, 3196, 2192, 1612 cm⁻¹;
¹H NMR (DMSO-d₆, 400MHz) δ 1.53-1.74 (6H, m), 1.92-2.00 (2H, m), 2.33 (3H, s), 4.11-4.19 (1H, m), 7.63 (1H, br), 8.44 (1H, br), 12.94 (1H, brd, J = 7.5 Hz);
MS (FAB) m/z: 210 [M+H]⁺;
Anal. Calcd for C₁₀H₁₅N₃S: C, 57.38; H, 7.22; N, 20.08; S, 15.32. Found: C, 57.39; H, 7.21; N, 19.98; S, 15.26.

### (10b) 4-(cyclopentylamino)-2-thioxo-1,2-dihydropyridine-3-carbonitrile

(2Z)-2-cyano-3-(cyclopentylamino)but-2-enethioamide (358 mg) which was produced in Example 10 (10a) was used in place of (2Z)-2-cyano-3-(dimethylamino)but-2-enethioamide and the reaction was performed in a similar method as described in Example 1 (1b) and a crude product (318 mg) of the title compound was obtained.
¹H NMR (DMSO-d₆, 400MHz) δ 1.51-1.73 (6H, m), 1.90-1.98 (2H, m), 3.94-4.04 (1H, m), 6.37 (1H, d, J = 7.8 Hz), 7.44-7.49 (1H, m), 12.42 (1H, br).

### (10c) 3-amino-4-(cyclopentylamino)thieno[2,3-b]pyridine-2-carboxamide

A crude product (318 mg) of 4-(cyclopentylamino)-2-thioxo-1,2-dihydropyridine-3-carbonitrile which was produced in Example 10 (10b) was used in place of 4-(dimethylamino)-2-thioxo-1,2-dihydropyridine-3-carbonitrile, and the reaction was performed in a similar method as described in Example 1 (1c) and 261 mg of the title compound was obtained. Yield 55% from (2Z)-3-(cyclopentylamino)-2-cyanobut-2-enethioamide.
Mp 212-213°C;
IR (KBr) νₘₐₓ 3311, 3177, 1649, 1594, 1513, 1497 cm⁻¹;
¹H NMR (DMSO-d₆, 400MHz) δ 1.52-1.73 (6H, m), 2.01-2.08 (2H, m), 3.85-3.92 (1H, m), 6.21 (1H, brd, J = 6.4 Hz), 6.43 (1H, d, J = 5.6 Hz), 6.72 (2H, br), 7.07 (2H, br), 8.06 (1H, d, J = 5.6 Hz);
MS (EI) m/z: 276 [M⁺], 231;
Anal. Calcd for C₁₃H₁₆N₄OS: C, 56.50; H, 5.84; N, 20.27; S, 11.60. Found: C, 56.31; H, 5.73; N, 20.13; S, 11.33.

### (Example 11) 3-amino-4-(cyclohexylamino)thieno[2,3-b]pyridine-2-carboxamide (Exemplified Compound No. 2-9)

### (11a) (2Z)-2-cyano-3-(cyclohexylamino)but-2-enethioamide

Cyclohexylamine was used in place of isobutylamine, the reaction was performed in a similar method as described in Example 5 (5a) and the title compound was obtained. Yield 76%.
Mp 142-144°C;
IR (KBr) νₘₐₓ 3412, 3297, 3188, 2187, 1613, 1493, 1410 cm⁻¹;
¹H NMR (DMSO-d₆, 400MHz) δ 1.33-1.61 (6H, m), 1.81-1.93 (4H, m), 2.37 (3H, s), 3.55-3.64 (1H, m), 6.38 (1H, br), 6.69 (1H, br), 12.92 (1H, br);
MS (EI) m/z: 223 [M⁺], 190.

### (11b) 3-amino-4-(cyclohexylamino)thieno[2,3-b]pyridine-2-carboxamide

(2Z)-2-cyano-3-(cyclohexylamino)but-2-enethioamide (0.33 g, 1.5 mmol) which was produced in Example 11 (11a) was used in place of (2Z)-2-cyano-3-(dimethylamino)but-2-enethioamide and the reaction was performed in a similar method as described in Example 1 (1b) and a crude product (0.11 g) of 2-thioxo-1,2-dihydropyridine derivative was obtained.

The reaction was performed in a similar method as described in Example 1 (1c) using the obtained 2-thioxo-1,2-dihydropyridine derivative and 0.06 g of the title compound was obtained (yield 14%).
Mp 244-247°C;
IR (KBr) νₘₐₓ 3141, 1664, 1598, 1513, 1497, 1108 cm⁻¹;
¹H NMR (DMSO-d₆, 400MHz) δ 1.20-1.98 (10H, m), 3.43-3.50 (1H, m), 6.16 (1H, d, J = 7.5 Hz), 6.46 (1H, d, J = 5.7 Hz), 6.71 (2H, br), 7.08 (2H, br), 8.06 (1H, d, J = 5.7 Hz);
MS (FAB) m/z: 291 [M+H]⁺;
Anal. Calcd for C₁₄H₁₈N₄OS·0.3H₂O: C, 56.85; H, 6.34; N, 18.94; S, 10.85. Found: C, 57.00; H, 6.27; N, 18.95; S, 10.61.

### (Example 12) 3-amino-4-(cycloheptylamino)thieno[2,3-b]pyridine-2-carboxamide (Exemplified Compound No. 2-10)

### (12a) (2Z)-2-cyano-3-(cycloheptylamino)but-2-enethioamide

Cycloheptylamine was used in place of isobutylamine and the reaction was performed in a similar method as described in Example 5 (5a) and the title compound was obtained. Yield 74%.
Mp 132-134°C;
IR (KBr) νₘₐₓ 3380, 3295, 3200, 2189, 1609, 853 cm⁻¹;
¹H NMR (DMSO-d₆, 400MHz) δ 1.48-1.63 (10H, m), 1.85-1.92 (2H, m), 2.32 (3H, s), 3.90-3.97 (1H, m), 7.62 (1H, br), 8.43 (1H, br), 12.94 (1H, brd, J = 8.9 Hz);
MS (FAB) m/z: 238 [M+H]⁺;
Anal. Calcd for C₁₂H₁₉N₃S: C, 60.72; H, 8.07; N, 17.70; S, 13.51. Found: C, 60.69; H, 8.12; N, 17.68; S, 13.41.

### (12b) 4-(cycloheptylamino)-2-thioxo-1,2-dihydropyridine-3-carbonitrile

(2Z)-2-cyano-3-(cycloheptylamino)but-2-enethioamide (339 mg) which was produced in Example 12 (12a) was used in place of (2Z)-2-cyano-3-(dimethylamino)but-2-enethioamide and the reaction was performed in a similar method as described in Example 1 (1b) and a crude product (306 mg) of the title compound was obtained.
¹H NMR (DMSO-d₆, 400MHz) δ 1.40-1.72 (10H, m), 1.76-1.85 (2H, m), 3.61-3.73 (1H, m), 6.30 (1H, d, J = 7.4 Hz), 6.82 (1H, br), 7.43 (1H, br), 12.35 (1H, br).

### (12c) 3-amino-4-(cycloheptylamino)thieno[2,3-b]pyridine-2-carboxamide

A crude product (306 mg) of 4-(cycloheptylamino)-2-thioxo-1,2-dihydropyridine-3-carbonitrile which was produced in Example 12 (12b) was used in place of 4-(isobutylamino)-2-thioxo-1,2-dihydropyridine-3-carbonitrile and the reaction was performed in a similar method as described in Example 5 (5c) and 201 mg of the title compound was obtained. Yield 46% from (2Z)-2-cyano-3-(cycloheptylamino)but-2-enethioamide.
Mp 206-208°C;
IR (KBr) νₘₐₓ 3334, 1652, 1594, 1514, 1498, 1365 cm⁻¹;
¹H NMR (DMSO-d₆, 400MHz) δ 1.49-1.71 (10H, m), 1.93-1.98 (2H, m), 3.60-3.63 (1H, m), 6.21 (1H, d, J = 7.5 Hz), 6.35 (1H, d, J = 5.8 Hz), 6.70 (2H, br), 7.10 (2H, br), 8.08 (1H, d, J = 5.8 Hz);
MS (FAB) m/z: 305 [M+H]⁺;
Anal. Calcd for C₁₅H₂₀N₄OS·0.1H₂O: C, 58.84; H, 6.65; N, 18.30; S, 10.47. Found: C, 58.77; H, 6.48; N, 18.22; S, 10.34.

### (Example 13) 3-amino-4-pyrrolidin-1-ylthieno[2,3-b]pyridine-2-carboxamide (Exemplified Compound No. 1-15)

### (13a) (2Z)-2-cyano-3-pyrrolidin-1-ylbut-2-enethioamide

Pyrrolidine was used in place of propylamine, the reaction was performed in a similar method as described in Example 4 (4a) and the title compound was obtained.
Yield 86%
¹H NMR(CD₃OD, 400 MHz) δ 2.00 (4H, s), 2.47 (3H, s), 3.63 (4H, s).

### (13b) 4-pyrrolidin-1-yl-2-thioxo-1,2-dihydropyridine-3-carbonitrile

(2Z)-2-cyano-3-pyrrolidin-1-ylbut-2-enethioamide which was produced in Example 13 (13a) was used in place of (2Z)-2-cyano-3-(dimethylamino)but-2-enethioamide and the reaction was performed in a similar method as described in Example 1 (1b) and the title compound was obtained. Yield 80%.
¹H NMR(DMSO-d₆, 400 MHz) δ 1.91 (4H, s), 3.55-3.75 (4H, br s), 6.21 (1H, d, J = 7.3 Hz), 7.365 (1H, dd, J = 5.9, 7.3 Hz).

### (13c) 3-amino-4-pyrrolidin-1-yl thieno[2,3-b]pyridine-2-carboxamide

4-pyrrolidin-1-yl-2-thioxo-1,2-dihydropyridine-3-carbonitrile which was produced in Example 13 (13b) was used in place of 4-(dimethylamino)-2-thioxo-1,2-dihydropyridine-3-carbonitrile, and the reaction was performed in a similar method as described in Example 1 (1c) and the title compound was synthesized. Yield 85%.
Mp 275-283°C;
IR (KBr) νₘₐₓ 3429, 3297, 3138, 2999, 2877, 1673, 1611, 1584, 1503, 1372, 1341, 1273, 1113, 1056, 1002, 618, 486 cm⁻¹ ;
¹H NMR(DMSO-d₆, 400 MHz) δ 1.91 (4H, s), 3.30 (4H, s), 6.87 (1H, d, J = 5.8 Hz), 6.88 (2H, s), 7.02 (2H, br s), 8.255 (1H, d, J = 5.8 Hz);
MS (FAB) m/z: 262.08 [M+H]⁺;
Anal. Calcd for C₁₂H₁₄N₄OS: C,54.94; H,5.38; N,21.36; S,12.22. Found: C,54.54; H,5.15; N,21.10; S,12.03.

### (Example 14) 3-amino-4-piperidin-1-ylthieno[2,3-b]pyridine-2-carboxamide (Exemplified Compound No. 1-16)

### (14a) (2Z)-2-cyano-3-piperidin-1-ylbut-2-enethioamide

An ethanol (30 mL) solution of (2Z)-2-cyano-3-ethoxybut-2-enethioamide (1.70 g, 10 mmol) and piperidine (1.02 g, 12 mmol) was stirred for two hours under heat reflux and allowed to stand overnight at room temperature. After the deposited solid was washed with ethanol (3x3 mL) and 1.36 g of the title compound was obtained (yield 65%).
Mp 161-166°C;
IR (KBr) νₘₐₓ 3381, 3268, 3170, 2182, 1600, 1534, 873, 838, 636 cm⁻¹;
¹H NMR (DMSO-d₆, 400MHz) δ 1.58-1.63 (6H, m), 2.27 (3H, s), 3.31-3.36 (4H, m), 8.15 (1H, br), 8.86 (1H, br);
MS (EI) m/z: 209 [M⁺], 150, 44;
Anal. Calcd for C₁₀H₁₅N₃S: C, 57.38; H, 7.22; N, 20.08; S, 15.32. Found: C, 57.27; H, 7.16; N, 20.03; S, 15.47.
(14b) 4-piperidin-1-yl-2-thioxo-1,2-dihydropyridine-3-carbonitrile

(2Z)-2-cyano-3-piperidin-1-ylbut-2-enethioamide (0.42 g, 2.0 mmol) and N,N-dimethylformamide dimethylacetal (0.72 g, 6.0 mmol) which was produced in Example 14 (14a) were mixed toluene (3 mL) and the mixture was stirred under reflux for two hours.
1N aqueous solution of sodium hydroxide (3 mL) was added to the residue which was obtained by evaporating the solvent under reduced pressure and the mixture was stirred under heat reflux for 30 minutes. After the reaction mixture was cooled to room temperature, 1N hydrochloric acid (4 mL) was added and the deposited solid was separated by filtration and further washed with water and a little amount of ethanol and 0.30 g of a solid containing the title compound was obtained.
¹H NMR (DMSO-d₆, 400MHz) δ 1.50-1.55 (6H, m), 3.48-3.52 (4H, m), 6.38 (1H, d, J = 7.3 Hz), 7.35 (1H, d, J = 7.3 Hz).

### (14c) 3-amino-4-piperidin-1-ylthieno[2,3-b]pyridine-2-carboxamide

4-piperidin-1-yl-2-thioxo-1,2-dihydropyridine-3-carbonitrile (0.30 g) which was produced in Example 14 (14b) was dissolved in N,N-dimethylformamide (3 mL) and 8N aqueous solution of sodium hydroxide (0.6 mL) and 2-chloroacetamide (0.15 g, 1.6 mmol)were added. Water (5 mL) was added after the mixture was stirred at room temperature for one hour. The deposited solid was separated by filtration and washed with water and ethanol and 0.30 g of a solid was obtained. The obtained solid was purified by silica gel column chromatography (100% ethyl acetate) and 0.21 g of the title compound was obtained.
Yield 38% from (2Z)-2-cyano-3-piperidin-1-ylbut-2-enethioamide.
Mp 191-192°C;
IR (KBr) νₘₐₓ 3460, 3329, 3176, 1651, 1589, 1501, 1378, 963 cm⁻¹;
¹H NMR (DMSO-d₆, 400MHz) δ 1.72-1.79 (6H, m), 2.55-3.40 (4H, m), 6.93 (2H, br), 6.99 (1H, d, J = 5.1 Hz), 7.07 (2H, br), 8.40 (1H, d, J = 5.1 Hz);
MS (FAB) m/z: 277 [M+H]⁺;
Anal. Calcd for C₁₃H₁₆N₄SO·0.5 H₂O: C, 54.72; H, 6.00; N, 19.63; S, 11.24. Found: C, 54.68; H, 6.03; N, 19.67; S, 11.05.

### (Example 15) 3-amino-4-(4-methylpiperidin-1-yl)thieno[2,3-b]pyridine-2-carboxamide (Exemplified Compound No. 1-18)

### (15a) (2Z)-2-cyano-3-(4-methylpiperidin-1-yl)but-2-enethioamide

4-methylpiperidine was used in place of isobutylamine, the reaction was performed in a similar method as described in Example 5 (5a) and the title compound was obtained. Yield 87%.
Mp 156-159°C;
IR (KBr) νₘₐₓ 3387, 3262, 3162, 2177, 1604, 1540, 868 cm⁻¹;
¹H NMR (DMSO-d₆, 400MHz) δ 0.91 (3H, d, J = 6.1 Hz), 1.15-1.25 (2H, m), 1.62-1.73 (3H, m), 2.27 (3H, s), 3.04-3.10 (2H, m), 3.62 (2H, brd, J = 13.5 Hz), 8.14 (1H, br), 8.85 (1H, br);
MS (EI) m/z: 223 [M⁺], 190;
Anal. Calcd for C₁₁H₁₇N₃S·0.1 H₂O: C, 58.68; H, 7.70; N, 18.66; S, 14.27. Found: C, 58.92; H, 7.73; N, 18.77; S, 14.06.

### (15b) 4-(4-methylpiperidin-1-yl)-2-thioxo-1,2-dihydropyridine-3-carbonitrile

(2Z)-2-cyano-3-(4-methylpiperidin-1-yl)but-2-enethioamide (0.39 g) which was produced in Example 15 (15a) was used in place of (2Z)-2-cyano-3-(dimethylamino)but-2-enethioamide and the reaction was performed in a similar method as described in Example 1 (1b). After the reaction terminated, toluene was added and the liquid was partitioned, and then the aqueous layer was neutralized with 1N hydrochloric acid and the deposited solid was separated by filtration and further washed with water and ethanol and a crude product (0.24 g) of the title compound was obtained.
¹H NMR (DMSO-d₆, 400MHz) δ 0.93 (3H, d, J = 6.4 Hz), 1.14-1.24 (2H, m), 1.65-1.76 (3H, m), 3.10-3.16 (2H, m), 4.08 (2H, brd, J = 13.2 Hz), 6.49 (1H, d, J = 7.5 Hz), 7.44 (1H, d, J = 7.5 Hz), 12.60 (1H, br).

### (15c) 3-amino-4-(4-methylpiperidin-1-yl)thieno[2,3-b]pyridine-2-carboxamide

A crude product (0.24 g) of 4-(4-methylpiperidin-1-yl)-2-thioxo-1,2-dihydropyridine-3-carbonitrile which was produced in Example 15 (15b) was used in place of 4-(isobutylamino)-2-thioxo-1,2-dihydropyridine-3-carbonitrile, and the reaction was performed in a similar method as described in Example 5 (5c) and 0.24 g of the title compound was obtained. Yield 41% from (2Z)-2-cyano-3-(4-methylpiperidin-1-yl)but-2-enethioamide.
Mp 252-254°C;
IR (KBr) νₘₐₓ 3430, 3311, 3159, 1667, 1611, 1580, 1504, 1367, 1341 cm⁻¹;
¹H NMR (DMSO-d₆, 400MHz) δ 0.98 (3H, d, J = 6.0 Hz), 1.40-1.76 (5H, m), 2.66-2.80 (2H, m), 3.27-3.30 (2H, m), 6.91 (2H, br), 6.99 (1H, d, J = 5.2 Hz), 7.05 (2H, br), 8.39 (1H, d, J = 5.2 Hz);
MS (FAB) m/z: 291 [M+H]⁺;
Anal. Calcd for C₁₄H₁₈N₄SO: C, 57.91; H, 6.25; N, 19.29; S, 11.04. Found: C, 57.89; H, 6.31; N, 19.29; S, 11.06.

### (Example 16) 3-amino-4-(3-methylpiperidin-1-yl)Neno[2,3-b]pyridine-2-carboxamide (Exemplified Compound No. 1-17)

### (16a) 4-(3-methylpiperidin-1-yl)-2-thioxo-1,2-dihydropyridine-3-carbonitrile

(2Z)-2-cyano-3-ethoxybut-2-enethioamide (340 mg, 2.0 mmol) and 3-methylpiperidine (248 mg, 2.5 mmol) were mixed with ethanol (2 mL) and the mixture was stirred at room temperature for two hours. The residue which was obtained by concentrating the reaction mixture under reduced pressure was dissolved in toluene (6 mL) and was blended with N,N-dimethylformamide dimethylacetal (714 mg, 6.0 mmol) and the mixture was stirred under heat reflux for two hours. 1N aqueous solution of sodium hydroxide (4 mL) was added to the residue after the solvent was evaporated under reduced pressure. The mixture was stirred under heat reflux for one hour and after it was cooled, 1N hydrochloric acid (5 mL) was added. After the deposited solid was washed with water and ethanol and 211 mg of a solid mainly containing the title compound was obtained.
¹H NMR (DMSO-d₆, 400MHz) δ 0.89 (3H, d, J = 6.7 Hz), 1.14-1.25 (1H, m), 1.47-1.83 (4H, m), 2.82 (1H, dd, J = 10.6, 13.3 Hz), 3.08-3.15 (1H, m), 3.96-4.05 (2H, m), 6.50 (1H, d, J = 6.5 Hz), 7.42-7.45 (1H, m), 12.59 (1H, br).

### (16b) 3-amino-4-(3-methylpiperidin-1-yl)thieno[2,3-b]pyridine-2-carboxamide

A solid (211 mg) mainly containing 4-(3-methylpiperidin-1-yl)-2-thioxo-1,2-dihydropyridine-3-carbonitrile which was produced in Example 16 (16a) was dissolved in N,N-dimethylformamide (2 mL) and 8N aqueous solution of sodium hydroxide (0.2 mL) and 2-chloroacetamide (122 mg, 1.3 mmol) were added and the mixture was stirred at room temperature for one hour. Water (2 mL) was added to the reaction mixture and the deposited solid was separated by filtration and further washed with water and ethanol and 158 mg of the title compound was obtained. Yield 27% from (2Z)-2-cyano-3-ethoxybut-2-enethioamide.
Mp 201-204°C;
IR (KBr) νₘₐₓ 3422, 3325, 3162, 1657, 1580, 1501, 1371 cm⁻¹;
¹H NMR (DMSO-d₆, 400MHz) δ 0.91 (3H, d, J = 6.3 Hz), 1.70-1.95 (4H, m), 2.23-2.70 (2H, m), 3.20-3.30 (2H, m), 6.90 (2H, br), 6.99 (1H, d, J = 5.1 Hz), 7.08 (2H, br), 8.40 (1H, d, J = 5.1 Hz);
MS (FAB) m/z: 291 [M+H]⁺;
Anal. Calcd for C₁₄H₁₈N₄SO·0.1 H₂O: C, 57.55; H, 6.28; N, 19.17; S, 10.97. Found: C, 57.45; H, 6.10; N, 19.35; S, 11.12.

### (Example 17) 3-amino-4-[3-(hydroxymethyl)piperidin-1-yl]thieno[2,3-b]pyridine-2-carboxamide (Exemplified Compound No. 1-20)

(2Z)-2-cyano-3-ethoxybut-2-enethioamide (340 mg, 2.0 mmol) and 3-(hydroxymethyl)piperidine (288 mg, 2.5 mmol) were mixed with ethanol (3 mL) and the mixture was stirred at room temperature for 1.5 hours. The residue which was obtained by concentrating the reaction mixture under reduced pressure was dissolved in N,N-dimethylformamide (3 mL) and was blended with N,N-dimethylformamide dimethylacetal (250 mg, 2.1 mmol) and the mixture was stirred at room temperature for one hour and further at 100°C for one hour. The reaction mixture was cooled to room temperature and was blended with 8N aqueous solution of sodium hydroxide (0.4 mL) and 2-chloroacetamide (281 mg, 3.0 mmol) and the mixture was stirred at room temperature for one hour. Water (50 mL) and ethyl acetate (50 mL) were added to the reaction mixture and the liquid was partitioned, and further the aqueous layer was extracted with ethyl acetate (30 mL). Combined with the organic layer, the solvent was evaporated under reduced pressure after drying over sodium sulfate. The residue was purified by silica gel column chromatography (dichloromethane/methanol =10:1) and 146 mg of the title compound was obtained. Yield 24% from (2Z)-2-cyano-3-ethoxybut-2-enethioamide.
Mp 125-130°C;
IR (KBr) νₘₐₓ 3441, 3335, 3183, 1661, 1595, 1583, 1503, 1375, 1037 cm⁻¹;
¹H NMR (DMSO-d₆, 400MHz) δ 0.95-1.15 (1H, m), 1.70-1.99 (4H, m), 2.26-2.70 (2H, m), 3.21-3.43 (4H, m), 4.56 (1H, t, J = 5.3 Hz), 6.94 (2H, br), 7.02 (1H, d, J = 5.1 Hz), 7.11 (2H, br), 8.44 (1H, d, J = 5.1 Hz);
MS (FAB) m/z: 307 [M+H]⁺;
Anal. Calcd for C₁₄H₁₈N₄SO₂·1 H₂O: C, 51.84; H, 6.21; N, 17.27; S, 9.88. Found: C, 51.72; H, 6.15; N, 17.37; S, 9.70.

### (Example 18) 3-amino-4-(4-benzylpiperidin-1-yl)thieno[2,3-b]pyridine-2-carboxamide (Exemplified Compound No. 1-19)

### (18a) (2Z)-3-(4-benzylpiperidin-1-yl) -2-cyanobut-2-enethioamide

4-benzylpiperidine was used in place of isobutylamine, the reaction was performed in a similar method as described in Example 5 (5a) and the title compound was obtained. Yield 88%.
Mp 151-154°C;
IR (KBr) νₘₐₓ 3283, 3173, 2187, 1605, 1542, 867, 749, 702 cm⁻¹;
¹H NMR (DMSO-d₆, 400MHz) δ 1.26-1.32 (2H, m), 1.63-1.66 (2H, m), 1.80-1.90 (1H, m), 2.53 (2H, d, J = 7.2 Hz), 3.02-3.08 (2H, m), 3.65 (2H, brd, J = 13.6 Hz), 7.17-7.31 (1H, m), 8.18 (1H, br), 8.88 (1H, br);
MS (EI) m/z: 299 [M⁺], 240, 91;
Anal. Calcd for C₁₇H₂₁N₃S·0.3 H₂O: C, 66.98; H, 7.14; N, 13.78; S, 10.52. Found: C, 67.03; H, 6.97; N, 13.78; S, 10.36.

### (18b) 4-(4-benzylpiperidin-1-yl)-2-thioxo-1,2-dihydropyridine-3-carbonitrile

(2Z)-2-cyano-3-(4-benzylpiperidin-1-yl)but-2-enethioamide (0.53 g) which was produced in Example 18 (18a) was used in place of (2Z)-2-cyano-3-(dimethylamino)but-2-enethioamide and the reaction was performed in a similar method as described in Example 1 (1b). The aqueous layer was neutralized with 1N hydrochloric acid after was added toluene after the reaction terminated, the liquid was partitioned, and the deposited solid was separated by filtration and further washed with water and ethanol and a crude product (0.23 g) of the title compound was obtained.
¹H NMR (DMSO-d₆, 400MHz) δ 1.20-1.31 (2H, m), 1.69 (2H, brd, J = 10.9 Hz), 1.82-1.88 (1H, m), 2.54 (2H, d, J = 7.1 Hz), 3.05-3.11 (2H, m), 4.07 (2H, brd, J = 13.2 Hz), 6.45 (1H, d, J = 7.5 Hz), 7.40-7.44 (1H, m), 12.57 (1H, br).

### (18c) 3-amino-4-(4-benzylpiperidin-1-yl)thieno[2,3-b]pyridine-2-carboxamide

A crude product (0.53 g) of 4-(4-benzylpiperidin-1-yl)-2-thioxo-1,2-dihydropyridine-3-carbonitrile which was produced in Example 18 (18b) was used in place of 4-(isobutylamino)-2-thioxo-1,2-dihydropyridine-3-carbonitrile, the reaction was performed in a similar method as described in Example 5 (5c) and the title compound was obtained (0.20 g).
Yield 27% from (2Z)-2-cyano-3-(4-benzylpiperidin-1-yl)but-2-enethioamide.
Mp 220-221°C;
IR (KBr) νₘₐₓ 3446, 3330, 3156, 1649, 1579, 1502, 1367, 958, 748, 700 cm⁻¹;
¹H NMR (DMSO-d₆, 400MHz) δ 1.48-1.72 (5H, m), 2.52-2.73 (2H, m), 2.59 (2H, d, J = 6.4 Hz), 3.30-3.33 (2H, m), 6.94 (2H, br), 6.99 (1H, d, J = 5.3 Hz), 7.09 (2H, br), 7.18-7.32 (5H, m), 8.41 (1H, d, J = 5.3 Hz);
MS (FAB) m/z: 367 [M+H]⁺;
Anal. Calcd for C₂₀H₂₂N₄SO·0.1 H₂O: C, 65.23; H, 6.08; N, 15.21; S, 8.71. Found: C, 65.18; H, 6.15; N, 15.35; S, 8.54.

### (Example 19) 3-amino-4-(4-hydroxypiperidin-1-yl)thieno[2,3-b]pyridine-2-carboxamide (Exemplified Compound No. 1-32)

### (19a) (2Z)-2-cyano-3-(4-hydroxypiperidin-1-yl)but-2-enethioamide

4-hydroxypiperidine was used in place of isobutylamine, the reaction was performed in a similar method as described in Example 5 (5a) and the title compound was obtained. Yield 93%.
Mp 160-163°C;
IR (KBr) νₘₐₓ 3368, 3167, 2180, 1634, 1536, 1415 cm⁻¹;
¹H NMR (DMSO-d₆, 400MHz) δ 1.44-1.52 (2H, m), 1.78-1.83 (2H, m), 2.27 (3H, s), 3.15-3.22 (2H, m), 3.50-3.56 (2H, m), 3.75-3.79 (1H, m), 4.82 (1H, d, J = 3.8 Hz), 8.18 (1H, br), 8.88 (1H, br);
MS (FAB) m/z: 226 [M+H]⁺;
Anal. Calcd for C₁₀H₁₅N₃SO: C, 53.31; H, 6.71; N, 18.65; S, 14.23. Found: C, 53.14; H, 6.65; N, 18.54; S, 14.03.

### (19b) (22)-2-cyano-N-[(1E)-(dimethylamino)methylene]-3-(4-hydroxypiperidin-1-yl)but-2-enethioamide

(2Z)-2-cyano-3-(4-hydroxypiperidin-1-yl)but-2-enethioamide (0.22 g, 1.0 mmol) and N,N-dimethylformamide dimethylacetal (0.13 g, 1.1 mmol) which was produced in Example 19 (19a) were mixed with ethanol (3 mL) and the mixture was stirred at room temperature for three hours. The deposited solid was separated by filtration and further washed with ethanol and 0.27 g of the title compound was obtained (yield 96%).
¹H NMR (DMSO-d₆, 400MHz) δ 1.49-1.57 (2H, m), 1.83-1.90 (2H, m), 2.50 (3H, s), 2.99 (3H, s), 3.15 (3H, s), 3.32-3.39 (2H, m), 3.63-3.69 (2H, m), 3.80-3.86 (1H, m), 4.88 (1H, d, J = 3.9 Hz), 8.50 (1H, s);

### (19c) 3-amino-4-(4-hydroxypiperidin-1-yl)thieno[2,3-b]pyridine-2-carboxamide

(2Z)-2-cyano-N-[(1E)-(dimethylamino)methylene]-3-(4-hydroxypiperidin-1-yl)but-2-enethioamide (0.27 g) which was produced in Example 19 (19b) was dissolved in N,N-dimethylformamide (5 mL) and the mixture was stirred at 120°C for one hour. The reaction liquid was cooled to room temperature and 8N aqueous solution of sodium hydroxide (0.3 mL) and 2-chloroacetamide (0.11 g, 1.2 mmol) were added. Water (5 mL) was added after the mixture was stirred at room temperature for one hour. The deposited solid was separated by filtration and washed with water and ethanol and 0.05 g of the title compound was obtained (yield 18%).
Mp 239-241°C;
IR (KBr) νₘₐₓ 3445, 3425, 3330, 1645, 1592, 1376, 1045 cm⁻¹;
¹H NMR (DMSO-d₆, 400MHz) δ 1.63-1.71 (2H, m), 1.90-1.98 (2H, m), 2.70-3.30 (4H, m), 3.50-3.80 (1H, m), 4.77 (1H, br), 6.91 (2H, br), 7.00 (1H, d, J = 5.3 Hz), 7.06 (2H, br), 8.39 (1H, d, J = 5.3 Hz);
MS (FAB) m/z: 293 [M+H]⁺;
Anal. Calcd for C₁₃H₁₆N₄SO₂·0.1 H₂O: C, 53.08; H, 5.55; N, 19.05; S, 10.90. Found: C, 53.09; H, 5.37; N, 18.77; S, 10.99.

### (Example 20) 3-amino-4-(4-acetoxypiperidin-1-yl)thieno[2,3-b]pyridine-2-carboxamide (Exemplified Compound No. 1-34)

3-amino-4-(4-hydroxypiperidin-1-yl)thieno[2,3-b]pyridine-2-carboxamide (0.10 g, 0.3 mmol) which was produced in Example 19 (19c) and acetic acid anhydride (0.2 mL) was reacted overnight in tetrahydrofuran (5 mL) solvent at room temperature in the presence of a catalytic amount of N,N-dimethylaminopyridine. A saturated sodium bicarbonate aqueous solution (30 mL) was added to the reaction mixture and the aqueous layer was extracted with ethyl acetate (50 mL). The extract was dried over sodium sulfate and the solvent was evaporated under reduced pressure. The obtained solid was washed with ethyl acetate and 0.04 g of the title compound was obtained (yield 35%).
Mp 231-235°C;
IR (KBr) νₘₐₓ 3439, 3422, 3323, 1710, 1645, 1580, 1268, 1035, 963 cm⁻¹;
¹H NMR (DMSO-d₆, 400MHz) δ 1.82-2.10 (4H, m), 2.04 (3H, s), 2.70-3.45 (4H, m), 4.65-5.00 (1H, m), 6.91 (2H, br), 7.02 (1H, d, J = 5.3 Hz), 7.09 (2H, br), 8.41 (1H, d, J = 5.3 Hz);
MS (FAB) m/z: 335 [M+H]⁺;
Anal. Calcd for C₁₅H₁₈N₄SO₃·0.24 H₂O: C, 53.19; H, 5.50; N, 16.54; S, 9.47. Found: C, 53.47; H, 5.27; N, 16.20; S, 9.22.

### (Example 21) 3-amino-4-(4-phenyl-3,6-dihydropyridin-1(2H)-yl)thieno[2,3-b]pyridine-2-carboxamide (Exemplified Compound No. 1-38)

### (21 a) (2Z)-2-cyano-3-(4-phenyl-3,6-dihydropyridin-1 (2H)-yl)but-2-enethioamide

4-phenyl-1,2,3,6-tetrahydropyridine was used in place of isobutylamine, the reaction was performed in a similar method as described in Example 5 (5a) and the title compound was obtained. Yield 99%.
Mp 170-171°C;
IR (KBr) νₘₐₓ 3342, 3293, 3189, 2176, 1637, 1555, 1410, 872, 750 cm⁻¹;
¹H NMR (DMSO-d₆, 400MHz) δ 2.36 (3H, s), 2.64-2.71 (2H, m), 3.64 (2H, t, J = 5.5 Hz), 3.98-4.04 (2H, m), 6.16 (1H, brs), 7.24-7.45 (5H, m), 8.33 (1H, br), 9.02 (1H, br);
MS (FAB) m/z: 284 [M+H]⁺;
Anal. Calcd for C₁₆H₁₇N₃S·0.2 H₂O: C, 66.96; H, 6.11; N, 14.64; S, 11.17. Found: C, 67.02; H, 6.12; N, 14.62; S, 10.81.

### (21b) 4-phenyl-2'-thioxo-1',2',3,6-tetrahydro-2H-1,4'-bipyridine-3'-carbonitrile

(2Z)-2-cyano-3-(4-phenyl-3,6-dihydropyridine-1 (2H)-yl)but-2-enethioamide (1.13 g) which was produced in Example 21 (21a) was used in place of (2Z)-2-cyano-3-(dimethylamino)but-2-enethioamide and the reaction was performed in a similar method as described in Example 1 (1b) and a crude product (0.13 g) of the title compound was obtained.
¹H NMR (DMSO-d₆, 400MHz) δ 2.66-2.72 (2H, m), 3.88-3.91 (2H, m), 4.26-4.30 (2H, m), 6.24 (1H, brs), 6.56 (1H, d, J = 7.8 Hz), 7.27-7.53 (6H, m), 12.70 (1H, br).

### (21c) 3-amino-4-(4-phenyl-3,6-dihydropyridin-1(2H)-yl)thieno[2,3-b]pyridine-2-carboxamide

A crude product (0.13 g) of 4-phenyl-2'-thioxo-1',2',3,6-tetrahydro-2H-1,4'-bipyridine-3'-carbonitrile which was produced in Example 21 (21b) was used in place of 4-(isobutylamino)-2-thioxo-1,2-dihydropyridine-3-carbonitrile, and the reaction was performed in a similar method as described in Example 5 (5c) and the title compound (0.06 g) was obtained. Yield 4% from (2Z)-2-cyano-3-(4-phenyl-3,6-dihydropyridine-1 (2H)-yl)but-2-enethioamide
Mp 235°C (decomposition);
IR (KBr) νₘₐₓ 3435, 3325, 1646, 1583, 1369, 954, 747 cm⁻¹;
¹H NMR (DMSO-d₆, 400MHz) δ 2.62-2.76 (2H, m), 3.30-3.46 (2H, m), 3.77-3.86 (2H, m), 6.35 (1H, brs), 6.90 (2H, br), 7.09 (1H, d, J = 5.3 Hz), 7.10 (2H, br), 7.25-7.51 (5H, m), 8.43 (1H, d, J = 5.3 Hz);
MS (FAB) m/z: 351 [M+H]⁺;
Anal. Calcd for C₁₉H₁₈N₄SO·0.7 H₂O: C, 62.86; H, 5.39; N, 15.43; S, 8.87. Found: C, 63.16; H, 5.04; N, 15.23; S, 8.45.

### (Example 22)

### 3-amino-4-morpholin-4-ylthieno[2,3-b]pyridine-2-carboxamide (Exemplified Compound No. 1-41)

### (22a) (2Z)-2-cyano-3-morpholin-1-ylbut-2-enethioamide

Morpholine was used in place of propylamine, the reaction was performed in a similar method as described in Example 4 (4a) and the title compound was obtained. Yield 85%.
Mp 156-160°C;
IR (KBr) νₘₐₓ 3374, 3255, 3165, 2177, 1605, 1540, 1119, 985, 884 cm⁻¹;
¹H NMR (DMSO-d₆, 400MHz) δ 2.26 (3H, s), 3.36-3.39 (4H, m), 3.64-3.67 (4H, m), 8.38 (1H, br), 9.06 (1H, br);
MS (FAB) m/z: 212 [M+H]⁺;
Anal. Calcd for C₉H₁₃N₃SO: C, 51.16; H, 6.20; N, 19.89; S, 15.18. Found: C, 51.15; H, 6.14; N, 19.73; S, 15.17.

### (22b) 4-morpholin-1-yl-2-thioxo-1,2-dihydropyridine-3-carbonitrile

4-(dimethoxymethyl) morpholine (Nucleosides and Nucleotides, 12, 1033-1046, (1993)) was used in place of N,N-dimethylformamide dimethylacetal and (2Z)-2-cyano-3-morpholin-1-ylbut-2-enethioamide was used in place of (2Z)-2-cyano-3-piperidin-1-ylbut-2-enethioamide and the reaction was performed in a similar method as described in Example 14 (14b). The aqueous solution iobtained by removing insolubles by filtration from the reaction mixture was neutralized with 1N hydrochloric acid. The deposited solid was separated by filtration and washed with water and ethanol and a crude product of the title compound was obtained.
Yield 43%.
¹H NMR (DMSO-d₆, 400MHz) δ 3.62-3.65 (4H, m), 3.69-3.71 (4H, m), 6.51 (1H, d, J = 7.8 Hz), 7.52 (1H, d, J = 7.8 Hz) 12.75 (1H, br).

### (22c) 3-amino-4-morpholin-4-ylthieno[2,3-b]pyridine-2-carboxamide

4-morpholin-1-yl-2-thioxo-1,2-dihydropyridine-3-carbonitrile which was produced in Example 22 (22b) was used in place of 4-piperidin-1-yl-2-thioxo-1,2-dihydropyridine-3-carbonitrile, the reaction was performed in a similar method as described in Example 14 (14c) and the title compound was obtained. Yield 64%. Mp 232-234°C;
IR (KBr) νₘₐₓ 3427, 3377, 3170, 1670, 1579, 1501, 1373, 1112, 969 cm⁻¹;
¹H NMR (DMSO-d₆, 400MHz) δ 2.90-3.15 (4H, m), 3.84-3.86 (4H, m), 6.95 (2H, br), 7.05 (1H, d, J = 5.3 Hz), 7.13 (2H, br), 8.46 (1H, d, J = 5.3 Hz);
MS (FAB) m/z: 279 [M+H]⁺;
Anal. Calcd for C₁₂H₁₄N₄SO₂·0.3 H₂O: C, 50.80; H, 5.19; N, 19.75; S, 11.30. Found: C, 50.95; H, 4.86; N, 19.72; S, 11.28.

### (Example 23) 3-amino-4-(2,6-cis-dimethylmorpholin-4-yl)thieno[2,3-b]pyridine-2-carboxamide (Exemplified Compound No. 1-42)

(2Z)-2-cyano-3-ethoxybut-2-enethioamide (340 mg, 2.0 mmol) and 2,6-cis-dimethyhnorpholine (288 mg, 2.5 mmol) were mixed with ethanol (3 mL) and the mixture was stirred at room temperature for four hours. The obtained residue by evaporating the solvent under reduced pressure was dissolved in N,N-dimethylformamide (3 mL) and N,N-dimethylformamide dimethylacetal (262 mg, 2.2 mmol) was added. The reaction mixture was stirred at 100°C for one hour after stirring at room temperature for one hour. After cooled to room temperature, 8N aqueous solution of sodium hydroxide (0.4 mL) and 2-chloroacetamide (243 mg, 2.6 mmol) were added to the reaction mixture. The reaction mixture was partitioned with water (50 mL) and ethyl acetate (50 mL) after stirring at room temperature for one hour. The solvent was evaporated under reduced pressure after the organic layer was dried over sodium sulfate. Residue silica was purified by gel column chromatography (ethyl acetate/ethanol/triethylamine =30:1:1) and 100 mg of the title compound was obtained (16%).
Mp 242-244°C;
IR (KBr) νₘₐₓ 3441, 3326, 3172, 1650, 1584, 1502, 1373, 1082, 1010 cm⁻¹;
¹H NMR (DMSO-d₆, 400MHz) δ 1.13 (6H, d, J = 6.3 Hz), 2.40-2.50 (2H, m), 3.24 (2H, brd, J = 11.3 Hz), 3.90-3.98 (2H, m), 6.92 (2H, br), 7.03 (1H, d, J = 5.1 Hz), 7.16 (2H, br), 8.45 (1H, d, J = 5.1 Hz);
MS (EI) m/z: 306 [M⁺], 202;
Anal. Calcd for C₁₄H₁₈N₄SO₂: C, 54.88; H, 5.92; N, 18.29; S, 10.47. Found: C, 54.88; H, 5.99; N, 18.37; S, 10.43.

### (Example 24) 3-amino-4-thiomorpholin-4-ylthieno[2,3-b]pyridine-2-carboxamide (Exemplified Compound No. 1-44)

### (24a) (2Z)-2-cyano-3-thiomorpholin-4-ylbut-2-enethioamide

Thiomorpholine was used in place of isobutylamine, the reaction was performed in a similar method as described in Example 5 (5a) and the title compound was obtained. Yield 73%.
Mp 157-160°C (decomposition);
IR (KBr) νₘₐₓ 3346, 3285, 3180, 2183, 1534, 953, 865 cm⁻¹;
¹H NMR (DMSO-d₆, 400MHz) δ 2.26 (3H, s), 2.72-2.74 (4H, m), 3.58-3.61 (4H, m), 8.43(1H,br),9.11(1H,br);
MS (FAB) m/z: 228 [M+H]⁺;
Anal. Calcd for C₉H₁₃N₃S₂·0.1 H₂O: C, 47.17; H, 5.81; N, 18.34; S, 27.98. Found: C, 47.27; H, 5.82; N, 18.62; S, 27.84.

### (24b) 4-thiomorpholin-4-yl-2-thioxo-1,2-dihydropyridine-3-carbonitrile

(2Z)-2-cyano-3-thiomorpholin-4-ylbut-2-enethioamide (250 mg) which was produced in Example 24 (24a) was used in place of (2Z)-2-cyano-3-(dimethylamino)but-2-enethioamide and the reaction was performed in a similar method as described in Example 1 (1b) and a crude product (157 mg) of the title compound was obtained.
¹H NMR (DMSO-d₆, 400MHz) δ 2.74-2.77 (4H, m), 3.84-3.87 (4H, m), 6.49 (1H, d, J = 7.4 Hz), 7.48 (1H, brd, J = 7.4 Hz), 12.73 (1H, br).

### (24c) 3-amino-4-thiomorpholin-4-ylthieno[2,3-b]pyridine-2-carboxamide

A crude product (157 mg) of 4-thiomorpholin-4-yl-2-thioxo-1,2-dihydropyridine-3-carbonitrile which was produced in Example 24 (24b) was dissolved in N,N-dimethylformamide (2 mL) and 8N aqueous solution of sodium hydroxide (0.2 mL) and 2-chloroacetamide (74 mg, 0.8 mmol) were added. Water (2 mL) was added to the reaction mixture after stirring at room temperature one hour. The deposited solid was separated by filtration and washed with water and ethanol. The obtained crystal was further heated and the mixture was stirred in ethyl acetate. After cooling, crystal was separated by filtration and 37 mg of the title compound was obtained. Yield 11 % from (2Z)-2-cyano-3-thiomorpholin-4-ylbut-2-enethioamide.
Mp 255-258°C;
IR (KBr) νₘₐₓ 3439, 3322, 3166, 1656, 1582, 1502, 1374, 1344, 935 cm⁻¹;
¹H NMR (DMSO-d₆, 400MHz) δ 2.50-3.60 (8H, m), 6.93 (2H, br), 7.04 (1H, d, J = 5.3 Hz), 7.10 (2H, br), 8.43 (1H, d, J = 5.3 Hz);
MS (EI) m/z: 294 [M⁺], 202;
Anal. Calcd for C₁₂H₁₄N₄S₂O: C, 48.96; H, 4.79; N, 19.03; S, 21.78. Found: C, 48.90; H, 4.70; N, 19.04; S, 21.73.

### (Example 25) 3-amino-4-azepan-1-ylthieno[2,3-b]pyridine-2-carboxamide (Exemplified Compound No. 1-39)

### (25a) (2Z)-3-azepan-1-yl-2-cyanobut-2-enethioamide

Hexamethyleneimine was used in place of isobutylamine and the reaction was performed in a similar method as described in Example 5 (5a) and the title compound was obtained. Yield 94%.
Mp 158-161°C;
IR (KBr) νₘₐₓ 3405, 3292, 3184, 2192, 1607, 1517, 1009, 870 cm⁻¹;
¹H NMR (CDCl₃, 400MHz) δ 1.61-1.70 (4H, m), 1.87-1.95 (4H, m), 2.74 (3H, s), 3.70-3.73 (4H, m), 6.69 (2H, br);
MS (EI) m/z: 223 [M⁺], 190;
Anal. Calcd for C₁₁H₁₇N₃S: C, 59.16; H, 7.67; N, 18.81; S, 14.36. Found: C, 59.10; H, 7.72; N, 18.67; S, 14.07.

### (25b) 4-azepan-1-yl-2-thioxo-1,2-dihydropyridine-3-carbonitrile

(2Z)-3-azepan-1-yl-2-cyanobut-2-enethioamide (0.42 g) which was produced in Example 25 (25a) was used in place of (2Z)-2-cyano-3-(dimethylamino)but-2-enethioamide and the reaction was performed in a similar method as described in Example 1 (1b) and a crude product (0.38 g) of the title compound was obtained. ¹H NMR (DMSO-d₆, 400MHz) δ 1.49-1.54 (4H, m), 1.73-1.80 (4H, m), 3.75-3.78 (4H, m), 6.39 (1H, d, J = 7.8 Hz), 7.37 (1H, dd, J = 6.0, 7.8 Hz), 12.46 (1H, br). (25c) 3-amino-4-azepan-1-ylthieno[2,3-b]pyridine-2-carboxamide

A crude product (0.38 g) of 4-azepan-1-yl-2-thioxo-1,2-dihydropyridine-3-carbonitrile which was produced in Example 25 (25b) was used in place of 4-(isobutylamino)-2-thioxo-1,2-dihydropyridine-3-carbonitrile, the reaction was performed in a similar method as described in Example 5 (5c) and 0.39 g of the title compound was obtained. Yield 71% from (2Z)-3-azepan-1-yl-2-cyanobut-2-enethioamide.
Mp 219-221°C;
IR (KBr) νₘₐₓ 3436, 3298, 3131, 1673, 1582, 1371, 930 cm⁻¹;
¹H NMR (DMSO-d₆, 400MHz) δ 1.65-1.78 (8H, m), 3.25-3.28 (4H, m), 7.04 (4H, br), 7.05 (1H, d, J = 5.4 Hz), 8.38 (1H, d, J = 5.4 Hz);
MS (EI) m/z: 290 [M⁺], 202.

### (Example 26) 3-amino-4-azocan-1-ylthieno[2,3-b]pyridine-2-carboxamide (Exemplified Compound No. 1-40)

### (26a) (2Z)-3-azocan-1-yl-2-cyanobut-2-enethioamide

Heptamethyleneimine was used in place of isobutylamine, the reaction was performed in a similar method as described in Example 5 (5a) and the title compound was obtained. Yield 85%.
Mp 149-150°C;
IR (KBr) νₘₐₓ 3340, 3276, 3164, 2176,1631,1558, 848 cm⁻¹;
¹H NMR (DMSO-d₆, 400MHz) δ 1.45-1.52 (6H, m), 1.72-1.75 (4H, m), 2.34 (3H, s), 3.55-3.58 (4H, m), 8.30 (1H, br), 8.92 (1H, br);
MS (FAB) m/z: 238 [M+H]⁺;
Anal. Calcd for C₁₂H₁₉N₃S·0.06 H₂O: C, 60.45; H, 8.08; N, 17.62; S, 13.45. Found: C, 60.43; H, 7.93; N, 17.67; S, 13.58.

### (26b) 4-azocan-1-yl-2-thioxo-1,2-dihydropyridine-3-carbonitrile

(2Z)-3-azocan-1-yl-2-cyanobut-2-enethioamide (0.39 g) which was produced in Example 26 (26a) was used in place of (2Z)-2-cyano-3-(dimethylamino)but-2-enethioamide and the reaction was performed in a similar method as described in Example 1 (1b) and a crude product (0.35 g) of the title compound was obtained.
¹H NMR (DMSO-d₆, 400MHz) δ 1.44-1.57 (6H, m), 1.71-1.74 (4H, m), 3.80-3.83 (4H, m), 6.38 (1H, d, J = 7.7 Hz), 7.35-8.38 (1H, m), 12.48 (1H, br).

### (26c) 3-amino-4-azocan-1-ylthieno[2,3-b]pyridine-2-carboxamide

A crude product (0.35 g) of 4-azocan-1-yl-2-thioxo-1,2-dihydropyridine-3-carbonitrile which was produced in Example 26 (26b) was used in place of 4-(isobutylamino)-2-thioxo-1,2-dihydropyridine-3-carbonitrile, and the reaction was performed in a similar method as described in Example 5 (5c) and 0.36 g of the title compound was obtained.
Yield 72% from (2Z)-3-azocan-1-yl-2-cyanobut-2-enethioamide.
Mp 222-224°C;
IR (KBr) νₘₐₓ 3440, 3300, 3132, 1667, 1579, 1499, 1372, 990 cm⁻¹;
¹H NMR (DMSO-d₆, 400MHz) δ 1.60-1.75 (10H, m), 3.34-3.36 (4H, m), 7.03 (2H, br), 7.04 (2H, br), 7.08 (1H, d, J = 5.4 Hz), 8.35 (1H, d, J = 5.4 Hz);
MS (FAB) m/z: 305 [M+H]⁺;
Anal. Calcd for C₁₅H₂₀N₄SO: C, 58.84; H, 6.65; N, 18.30; S, 10.47. Found: C, 58.84; H, 6.53; N, 18.30; S, 10.37.

### (Example 27) 3-amino-4-(3,4-dihydroisoquinoline-2(1H)-yl)thieno[2,3-b]pyridine-2-carboxamide (Exemplified Compound No. 1-35)

### (27a) (2Z) 2-cyano-3-(3,4-dihydroisoquinoline-2(1H)-yl)but-2-enethioamide

1,2,3,4-tetras hydro-isoquinoline was used in place of isobutylamine, the reaction was performed in a similar method as described in Example 5 (5 a) and the title compound was obtained. Yield 93%.
IR (KBr) νₘₐₓ 3284, 3167, 2186, 1609, 1538, 878, 752 cm⁻¹;
¹H NMR (DMSO-d₆, 400MHz) δ 2.38 (3H, s), 2.95 (2H, t, J = 5.8 Hz), 3.62 (2H, t, J = 5.8 Hz), 4.54 (2H, s), 7.10-7.21 (4H, m), 8.32 (1H, br) 8.98 (1H, br);
MS (EI) m/z: 257 [M⁺], 224.

### (27b) 4-(3,4-dihydroisoquinoline-2(1H)-yl)-2-thioxo-1,2-dihydropyridine-3-carbonitrile

(2Z)-2-cyano-3-(3,4-dihydroisoquinoline-2(1H)-yl)but-2-enethioamide (470 mg) which was produced in Example 27 (27a) was used in place of (2Z)-2-cyano-3-(dimethylamino)but-2-enethioamide and the reaction was performed in a similar method as described in Example 1 (1b) and a crude product (175 mg) of the title compound was obtained.
¹H NMR (DMSO-d₆, 400MHz) δ 3.00 (2H, t, J = 5.9 Hz), 3.88 (2H, t, J = 5.9 Hz), 4.76 (2H, s), 6.58 (1H, d, J = 7.4 Hz), 7.22-7.25 (4H, m), 7.51-7.54 (1H, m) 12.72 (1H, br).

### (27c) 3-amino-4-(3,4-dihydroisoquinoline-2(1H)-yl)thieno[2,3-b]pyridine-2-carboxamide

A crude product (175 mg) of 4-(3,4-dihydroisoquinoline-2(1H)-yl)-2-thioxo-1,2-dihydropyridine-3-carbonitrile which was produced in Example 27 (27b) was used in place of 4-(isobutylamino)-2-thioxo-1,2-dihydropyridine-3-carbonitrile, and the reaction was performed in a similar method as described in Example 5 (5c) and the title compound was obtained (71 mg). Yield 12% from (2Z)-2-cyano-3-(3,4-dihydroisoquinoline-2(1H)-yl)but-2-enethioamide.
Mp 244-245°C;
IR (KBr) νₘₐₓ 3395, 3325, 3144, 1659, 1585, 1502, 1378, 739 cm⁻¹;
¹H NMR (DMSO-d₆, 400MHz) δ 2.94-3.10 (2H, m), 3.33-3.50 (2H, m), 4.28 (2H, brs), 6.85 (2H, br), 7.08 (1H, d, J = 5.5 Hz), 7.09 (2H, br), 7.17-7.19 (4H, m), 8.44 (1H, d, J = 5.5 Hz);
MS (FAB) m/z: 325 [M+H]⁺;
Anal. Calcd for C₁₇H₁₆N₄SO: C, 62.94; H, 4.97; N, 17.27; S, 9.88. Found: C, 62.69; H, 5.24; N, 17.21; S, 9.85.

### (Example 28) 3-amino-4-(trans-perhydroisoquinolin-2-yl)thieno[2,3-b]pyridine-2-carboxamide (Exemplified Compound No. 1-36)

### (28a) (2Z)-2-cyano-3-(trans-perhydroisoquinolin-2-yl)but-2-enethioamide

Trans-perhydroisoquinoline was used in place of isobutylamine, the reaction was performed in a similar method as described in Example 5 (5a) and the title compound was obtained. Yield 41 %.
Mp 142-147°C;
IR (KBr) νₘₐₓ 3375, 3277, 3163, 2184, 1606, 1537, 872 cm⁻¹;
¹H NMR (DMSO-d₆, 400MHz) δ 0.88-1.72 (12H, m), 2.27 (3H, s), 2.71-2.77 (1H, m), 3.04-3.11 (1H, m), 3.49-3.51 (1H, m), 3.64-3.68 (1H, m), 8.15 (1H, br), 8.87 (1H, br);
MS (FAB) m/z: 264 [M+H]⁺;
Anal. Calcd for C₁₄H₂₁N₃S: C, 63.84; H, 8.04; N, 15.95; S, 12.17. Found: C, 63.92; H, 7.98; N, 15.93; S, 11.91.

### (28b) 4-(trans-perhydroisoquinolin-2-yl)-2-thioxo-1,2-dihydropyridine-3-carbonitrile

(2Z)-2-cyano-3-(trans-perhydroisoquinolin-2-yl)but-2-enethioamide (210 mg) which was produced in Example 28 (28a) was used in place of (2Z)-2-cyano-3-(dimethylamino)but-2-enethioamide and the reaction was performed in a similar method as described in Example 1 (1b) and a crude product (153 mg) of the title compound was obtained.
¹H NMR (DMSO-d₆, 400MHz) δ 0.91-1.74. (12H, m), 2.75-2.81 (1H, m), 3.09-3.15 (1H, m), 3.94-4.00 (1H, m), 4.10-4.17 (1H, m), 6.48 (1H, d, J = 7.4 Hz), 7.39-7.43 (1H, m), 12.56 (1H, br).

### (28c) 3-amino-4-(trans-perhydroisoquinolin-2-yl)[2,3-thieno b]pyridine-2-carboxamide

A crude product (153 mg) of 4-(trans-perhydroisoquinolin-2-yl)-2-thioxo-1,2-dihydropyridine-3-carbonitrile which was produced in Example 28 (28b) was used in place of 4-(isobutylamino)-2-thioxo-1,2-dihydropyridine-3-carbonitrile and the reaction was performed in a similar method as described in Example 5 (5c) and the title compound was obtained (115 mg).
Yield 44% from (2Z)-2-cyano-3-(trans-perhydroisoquinolin-2-yl)but-2-enethioamide.
Mp 285-288°C;
IR (KBr) νₘₐₓ 3406, 3323, 3143, 1656, 1584, 1502, 1377, 951 cm⁻¹;
¹H NMR (DMSO-d₆, 400MHz) δ 0.92-1.76 (12H, m), 2.34-2.45 (1H, m), 2.66-2.79 (1H, m), 3.14-3.22 (1H, m), 3.30-3.38 (1H, m), 6.92 (2H, br), 7.01 (1H, d, J = 5.1 Hz), 7.10 (2H, br), 8.42 (1H, d, J = 5.1 Hz);
MS (FAB) m/z: 331 [M+H]⁺;
Anal. Calcd for C₁₇H₂₂N₄SO: C, 61.79; H, 6.71; N, 16.95; S, 9.70. Found: C, 61.63; H, 6.71; N, 16.94; S, 9.74.

### (Example 29) 3-amino-4-(cis-perhydroisoquinolin-2-yl)thieno[2,3-b]pyridine-2-carboxamide (Exemplified Compound No. 1-36)

### (29a) (2Z)-2-cyano-3-(cis-perhydroisoquinolin-2-yl)but-2-enethioamide

cis-perhydroisoquinoline was used in place of isobutylamine, the reaction was performed in a similar method as described in Example 5 (5a) and the title compound was obtained. Yield 95%.
Mp 173-175°C;
IR (KBr) νₘₐₓ 3444, 3250, 3160, 2188, 1603, 1542, 867 cm⁻¹;
¹H NMR (DMSO-d₆, 400MHz) δ 1.23-1.95 (12H, m), 2.27 (3H, s), 3.12-3.25 (2H, m), 3.41-3.54 (2H, m), 8.12 (1H, br), 8.85 (1H, br);
MS (FAB) m/z: 264 [M+H]⁺;
Anal. Calcd for C₁₄H₂₁N₃S: C, 63.84; H, 8.04; N, 15.95; S, 12.17. Found: C, 63.53; H, 8.10; N, 15.67; S, 12.32.

### (29b) 4-(cis-perhydroisoquinolin-2-yl)-2-thioxo-1,2-dihydropyridine-3-carbonitrile

(2Z)-2-cyano-3-(cis-perhydroisoquinolin-2-yl)but-2-enethioamide (490 mg) which was produced in Example 29 (29a) was used in place of (2Z)-2-cyano-3-(dimethylamino)but-2-enethioamide and the reaction was performed in a similar method as described in Example 1 (1b) and a crude product (370 mg) of the title compound was obtained.
¹H NMR (DMSO-d₆, 400MHz) δ 1.22-1.95 (12H, m), 3.10-3.43 (2H, m), 3.78-3.94 (2H, m), 6.47 (1H, d, J = 7.4 Hz), 7.40 (1H, d, J = 7.4 Hz), 12.40 (1H, br).

### (29c) 3-amino-4-(cis-perhydroisoquinolin-2-yl)thieno[2,3-b]pyridine-2-carboxamide

A crude product (370 mg) of 4-(cis-perhydroisoquinolin-2-yl)-2-thioxo-1,2-dihydropyridine-3-carbonitrile which was produced in Example 29 (29b) was used in place of 4-(isobutylamino)-2-thioxo-1,2-dihydropyridine-3-carbonitrile, the reaction was performed in a similar method as described in Example 5 (5c) and the title compound was obtained (200 mg). Yield 33% from (2Z)-2-cyano-3-(cis-perhydroisoquinolin-2-yl)but-2-enethioamide.
Mp 224-226°C;
IR (KBr) νₘₐₓ 3443, 3323, 3179, 1646, 1582, 1503, 1370, 957 cm⁻¹;
¹H NMR (DMSO-d₆, 400MHz) δ 1.20-2.25 (12H, m), 2.92-3.46 (4H, m), 6.93 (2H, br), 7.03 (1H, d, J = 5.1 Hz), 7.08 (2H, br), 8.40 (1H, d, J = 5.1 Hz);
MS (EI) m/z: 330 [M⁺], 285;
Anal. Calcd for C₁₇H₂₂N₄SO·0.1H₂O: C, 61.46; H, 6.73; N, 16.86; S, 9.65. Found: C, 61.37; H, 6.76; N, 16.80; S, 9.68.

### (Example 30) 3-amino-4-(1,4-oxazepan-4-yl)thieno[2,3-b]pyridine-2-carboxamide (Exemplified Compound No. 1-43)

### (30a) (2Z)-2-cyano-3-(1,4-oxazepan-4-yl)but-2-enethioamide

Homomorpholine was used in place of isobutylamine, the reaction was performed in a similar method as described in Example 5 (5a) and the title compound was synthesized.
Pale yellow powder
Mp 151-156°C;
IR (KBr) νₘₐₓ 3348, 3285, 3174, 2184, 1632, 1518, 1417, 1127, 1075, 878, 814 cm⁻¹; ¹H NMR(DMSO-d₆, 400 MHz) δ 1.86-1.92 (2H, m), 2.31 (3H, s), 3.57 (2H, t, J = 5.5 Hz), 3.62 (2H, t, J = 5.5 Hz), 3.66 (2H, t, J = 5.5 Hz), 3.77 (2H, t, J = 5.5 Hz), 8.29 (1H, brs), 8.94 (1H, brs);
HRMS m/z calcd for C₁₀H₁₆ON₃S 226.1014, found 226.1019;
MS (FAB) m/z: 226 [M+H]⁺, 209, 192, 165, 65, 51.

### (30b) 4-(1,4-oxazepan-4-yl)-2-thioxo-1,2-dihydropyridine-3-carbonitrile

(2Z)-2-cyano-3-(1,4-oxazepan-4-yl)but-2-enethioamide which was produced in Example 30 (30a) was used in place of (2Z)-2-cyano-3-(isobutylamino)but-2-enethioamide and the reaction was performed in a similar method as described in Example 5 (5b) and the title compound was obtained.
White powder
Mp 228-233°C (decomposition);
IR (KBr) νₘₐₓ 3119, 2952, 2210, 1625, 1520, 1252, 1117, 928, 780 cm⁻¹;
¹H NMR(DMSO-d₆, 400 MHz) δ 1.89-1.94 (2H, m), 3.67 (2H, t, J = 5.5 Hz), 3.77-3.79 (2H, m), 3.33-3.37 (4H, m), 6.44 (1H, d, J = 7.8 Hz), 7.42 (1H, d, J = 7.8 Hz), 12.59 (1H, brs);
HRMS m/z calcd for C₁₁H₁₃ON₃S 235.0779, found 235.0790;
MS (EI) m/z: 235 [M⁺], 204, 190, 177, 164, 150, 136, 108, 76, 70, 41.

### (30c) 3-amino-4-(1,4-oxazepan-4-yl)thieno[2,3-b]pyridine-2-carboxamide

4-(1,4-oxazepan-4-yl)-2-thioxo-1,2-dihydropyridine-3-carbonitrile which was produced in Example 30 (30b) was used in place of 4-(isobutylamino)-2-thioxo-1,2-dihydropyridine-3-carbonitrile and the reaction was performed in a similar method as described in Example 5 (5c) and the title compound was obtained.
White powder
Mp 175-176°C;
IR (KBr) νₘₐₓ 3445, 3301, 3141, 1671, 1585, 1497, 1370, 1153, 1060, 940 cm⁻¹;
¹H NMR(DMSO-d₆, 400 MHz) δ 1.99-2.05 (2H, m), 3.30-3.34 (4H, m), 3.78-3.84 (4H, m), 7.05 (2H, brs), 7.08-7.10 (3H, m), 8.41 (1H, d, J = 5.5 Hz);
HRMS m/z calcd for C₁₃H₁₇O₂N₄S 293.1073, found 293.1067;
MS (FAB) m/z: 293 [M+H]⁺, 276, 237, 183, 165, 120, 65;
Anal. Calcd for C₁₃H₁₆N₄O₂S·0.28H₂O: C, 52.50; H, 5.61; N, 18.84; S, 10.78. Found: C, 52.32; H, 5.41; N, 19.03, S, 10.77.

### (Example 31) 3-amino-4-(4-phenylpiperidin-1-yl)thieno[2,3-b]pyridine-2-carboxamide (Exemplified Compound No. 1-26)

### (31a) (2Z)-2-cyano-3-(4-phenylpiperidin-1-yl)but-2-enethioamide

4-phenylpiperidine was used in place of isobutylamine and the reaction was performed in a similar method as described in Example 5 (5a) and the title compound was synthesized.
Pale yellow powder
Mp 171-172°C;
IR (KBr) νₘₐₓ 3389, 3263, 3162, 2185, 1599, 1535, 1364, 1232, 980, 855, 764, 701 cm⁻¹;
¹H NMR(DMSO-d₆, 400 MHz) δ 1.69-1.86 (4H, m), 2.33 (3H, s), 2.83-2.91 (1H, m), 3.22 (2H, t, J = 12.1 Hz), 3.76 (2H, d, J = 12.1 Hz), 7.19-7.83 (5H, m), 8.28 (1H, brs), 8.98 (1H, brs);
HRMS m/z calcd for C₁₆H₂₀N₃S 286.1378, found 286.1372;
MS (FAB) m/z: 286 [M+H]⁺, 252, 227, 186, 80, 56, 41;
Anal. Calcd for C₁₆H₁₉N₃S: C, 67.33; H, 6.71; N, 14.72; S, 11.23. Found: C, 67.10; H, 6.75; N, 14.65, S, 11.17.

### (31b) (2Z)-2-cyano-N-[(1E)-(dimethylamino)methylene]-3-(4-phenylpiperidin-1-yl)but-2-enethioamide

(2Z)-2-cyano-3-(4-phenylpiperidin-1-yl)but-2-enethioamide (373 mg, 1.3 mmol) which was produced in Example 31 (31a) and N,N-dimethylformamide dimethylacetal (312 mg, 2.6 mmol) were mixed with ethanol (12 mL) and the mixture was stirred for 18 hours. The deposited solid was separated by filtration and 446 mg of the title compound was obtained (yield 70%).
Yellow powder
Mp 151-152°C;
IR (KBr) νₘₐₓ 2920, 2182, 1614, 1520, 1333, 1289, 1191, 975, 767, 704 cm⁻¹;
¹H NMR(DMSO-d₆, 500 MHz) δ 1.79-1.91 (4H, m), 2.55 (3H, s), 2.94-3.00 (1H, m), 3.02 (3H, s) 3.18 (3H, s), 3.30-3.36 (2H, m), 3.93 (2H, d, J = 13.7 Hz), 7.20-7.83 (5H, m), 8.55 (1H, s);
HRMS m/z calcd for C₁₉H₂₅N₄S 341.1800, found 341.1797;
MS (FAB) m/z: 341 [M+H]⁺, 273, 246, 200, 165, 63;
Anal. Calcd for C₁₉H₂₄N₄S·0.26H₂O: C, 66.11; H, 7.16; N, 16.23; S, 9.29. Found: C, 66.15; H, 6.97; N, 16.14, S, 8.99.

### (31c) 3-amino-4-(4-phenylpiperidin-1-yl)thieno[2,3-b]pyridine-2-carboxamide

N,N-dimethylformamide (1.8 mL) solution of (2Z)-2-cyano-N-[(1E)-(dimethylamino) methylene]-3-(4-phenylpiperidin-1-yl)but-2-enethioamide (299 mg, N of 0.88 mmol) which was produced in Example 31 (31b) was stirred at 80°C for 15 minutes. 8N aqueous solution of sodium hydroxide (0.37 mL) and 2-chloroacetamide (99 mg, 1.1 mmol) were added after the reaction mixture was cooled to room temperature. Water and ethyl acetate was added after the mixture was stirred at room temperature for one hour. The deposited solid was separated by filtration and 310 mg of the title compound was obtained (yield 58%).
Pale yellow crystal
Mp 226-233°C;
IR (KBr) νₘₐₓ 3445, 3315, 3130, 1647, 1580, 1501, 1371, 1229, 1052, 959, 701 cm⁻¹; ¹H NMR(DMSO-d₆, 500 MHz) δ 1.90 (2H, d, J = 11.7 Hz), 2.03 (2H, q, J = 11.7 Hz), 2.70 (1H, t, J = 11.7 Hz), 2.82-2.91 (2H, m), 3.45 (2H, d, J = 11.7 Hz), 7.02 (2H, brs), 7.07 (1H, d, J = 5 .4 Hz), 7.10 (2H, brs), 7.22 (1H, t, J = 7.3 Hz), 7.33 (2H, t, J = 7.3 Hz), 7.38 (2H, d, J = 7.3 Hz), 8.46 (1H, d, J = 5.4 Hz);
HRMS m/z calcd for C₁₉H₂₀ON₄S 352.1358, found 352.1358;
MS (FAB) m/z: 353 [M+H]⁺, 273, 246, 200, 165, 63.

### (Example 32) 3-amino-4-(1-oxothiomorpholine-4-yl)thieno[2,3-b]pyridine-2-carboxamide (Exemplified Compound No. 1-45)

3-amino-4-(thiomorpholine-4-yl)thieno[2,3-b]pyridine-2-carboxamide (73 mg, 0.25 mmol) which was produced in Example 24 (24c) was dissolved in methanol and an aqueous solution (1 mL) of sodium periodate (59 mg, 0.28 mmol) was added. A saturated saline solution was added to the reaction liquid after stirring at room temperature for one hour and the aqueous layer was extracted with a mixed solvent of chloromethane/2-propanol (4:1). The extract was concentrated after drying over sodium sulfate under reduced pressure.
The residue was washed with ether and 78 mg of the title compound was obtained (yield 92%).
Pale yellow powder
Mp 165-172°C;
IR (KBr) νₘₐₓ 3431, 3323, 3183, 1649, 1589, 1500, 1375, 1057, 933 cm⁻¹;
¹H NMR(DMSO-d₆, 500 MHz) δ 2.95-3.04 (2H, m), 3.21-3.32 (4H, m), 3.52-3.57 (2H, m), 6.99 (2H, brs), 7.15 (3H, brs), 8.48 (1H, d, J = 5.4 Hz);
HRMS m/z calcd for C₁₂H₁₄O₂N₄S₂ 310.0558, found 310.0555;
MS (EI) m/z: 310 [M⁺], 278, 261, 244, 230, 202, 189, 176, 148, 122, 101, 76.

### (Example 33) 3-amino-4-(1,4-thiazepan-4-yl)thieno[2,3-b]pyridine-2-carboxamide (Exemplified Compound No. 1-46)

### (33a) (2Z)-2-cyano-3-(1,4-thiazepan-4-yl)but-2-enethioamide

Homothiomorpholine (J. Org. Chem., 25, 1953-1956 (1960)) was used in place of isobutylamine and the reaction was performed in a similar method as described in Example 5 (5a) and the title compound was synthesized.
White powder
Mp 138-142°C;
IR (KBr) νₘₐₓ 3438, 23284, 3173, 1597, 1533, 1409, 1251, 867, 569 cm⁻¹;
¹H NMR(DMSO-d₆, 400 MHz) δ 1.95-2.01 (2H, m), 2.33 (3H, s), 2.67 (2H, t, J = 5.9 Hz), 2.94 (2H, t, J = 5.5 Hz), 3.65 (2H, t, J = 5.9 Hz), 3.70 (2H, t, J = 5.5 Hz), 8.46 (1H, brs), 9.07 (1H, brs);
HRMS m/z calcd for C₁₀H₁₅N₃S₂ 241.0707, found 241.0707;
MS (EI) m/z: 241 [M⁺], 208, 182, 142, 135, 121, 96, 68, 59, 43;
Anal. Calcd for C₁₀H₁₅N₃S₂: C, 49.76; H, 6.26; N, 17.41; S, 26.57. Found: C, 49.47; H, 6.32; N, 17.14, S, 26.48.

### (33b) 4-(1,4-thiazepan-4-yl)-2-thioxo-1,2-dihydropyridine-3-carbonitrile

(2Z)-2-cyano-3-(1,4-thiazepan-4-yl)but-2-enethioamide which was produced in Example 33 (33a) was used in place of (2Z)-2-cyano-3-(isobutylamino)but-2-enethioamide and the reaction was performed in a similar method as described in Example 5 (5b) and the title compound was obtained.
Yellow powder
Mp 265-270°C;
IR (KBr) νₘₐₓ 3115, 2949, 2207, 1625, 1524, 1256, 1136, 943, 788 cm⁻¹;
¹H NMR(DMSO-d₆, 400 MHz) δ 1.96 (2H, quint, J = 5.5 Hz), 2.62 (2H, t, J = 5.9 Hz), 2.90 (2H, t, J = 5.9 Hz), 3.97 (2H, t, J = 5.9 Hz), 4.04 (2H, t, J = 5.9 Hz), 6.42 (1H, d, J = 7.8 Hz), 7.37 (1H, d, J = 7.8 Hz), 12.48 (1H, brs);
HRMS m/z calcd for C₁₁H₁₃N₃S₂ 251.0551, found 251.0553;
MS (EI) m/z: 251 [M⁺], 236, 223, 204, 190, 177, 164, 150, 136, 108, 60;
Anal. Calcd for C₁₁H₁₃N₃S₂: C, 52.56; H, 5.21; N, 16.72; S, 25.51. Found: C, 52.41; H, 5.37; N, 17.01, S, 25.62.

### (33c) 3-amino-4-(1,4-thiazepan-4-yl)thieno[2,3-b]pyridine-2-carboxamide

4-(1,4-thiazepan-4-yl)-2-thioxo-1,2-dihydropyridine-3-carbonitrile which was produced in Example 33 (33b) was used in place of 4-(isobutylamino)-2-thioxo-1,2-dihydropyridine-3-carbonitrile and the reaction was performed in a similar method as described in Example 5 (5c) and the title compound was obtained.
Pale yellow powder
Mp 171-174°C;
IR (KBr) νₘₐₓ 3416, 3302, 3171, 1645, 1582, 1498, 1367, 1259, 1125, 481 cm⁻¹;
¹H NMR(DMSO-d₆, 500 MHz) δ 1.94-1.99 (2H, m), 2.76 (2H, t, J = 5.9 Hz), 2.98 (2H, t, J = 5.9 Hz), 3.29-3.32 (2H, m), 3.44-3.46 (2H, m), 7.07 (2H, brs), 7.20 (1H, d, J = 5.4 Hz), 7.41 (2H, brs), 8.46 (1H, d, J = 5.4 Hz);
HRMS m/z calcd for C₁₃H₁₆ON₄S₂ 308.0765, found 308.0767;
MS (EI) m/z: 308 [M⁺], 276, 244, 230, 202, 188, 176, 148, 122, 78, 45;
Anal. Calcd for C₁₃H₁₆N₄OS₂·0.66H₂O: C, 48.75; H, 5.45; N, 17.49. Found: C, 48.44; H, 5.38; N, 17.79.

### (Example 34) 3-amino-4-(1-oxo-1,4-thiazepan-4-yl)thieno[2,3-b]pyridine-2-carboxamide (Exemplified Compound No. 1-47)

3-amino-4-(1,4-thiazepan-4-yl)thieno[2,3-b]pyridine-2-carboxamide which was produced in Example 33 (33c) was used in place of 3-amino-4-(thiomorpholine-4-yl)thieno[2,3-b]pyridine-2-carboxamide and the reaction was performed in a similar method as described in Example 32 and the title compound was obtained. Pale yellow powder
Mp 111-119°C;
IR (KBr) νₘₐₓ 3432, 3321, 1648, 1590, 1499, 1368, 1035 cm⁻¹;
¹H NMR(CDCl₃, 500 MHz) δ 2.03-2.11 (1H, m), 2.59-2.67 (1H, m), 2.98-3.11 (2H, m), 3.19-3.30 (2H, m), 3.36-3.54 (3H, m), 3.91-3.96 (1H, m), 5.30 (2H, brs), 7.00 (1H, d, J = 4.9 Hz), 7.15 (2H, brs), 8.53 (1H, d, J = 4.9 Hz);
HRMS m/z calcd for C₁₃H₁₇O₂N₄S₂ 325.0793, found 325.0774;
MS (FAB) m/z: 325 [M+H]⁺, 273, 178, 165, 51;

### (Example 35) 3-amino-4-(3-phenylpiperidin-1-yl)thieno[2,3-b]pyridine-2-carboxamide (Exemplified Compound No. 1-25)

### (35a) (2Z)-2-cyano-3-(3-phenylpiperidin-1-yl)but-2-enethioamide

3-phenylpiperidine was used in place of isobutylamine and the reaction was performed in a similar method as described in Example 5 (5 a) and the title compound was synthesized.
Pale brown powder
Mp 159-162°C (decomposition);
IR (KBr) νₘₐₓ 3307, 3184, 2190, 1600, 1533, 1412, 1260, 978, 850, 703 cm⁻¹;
¹H NMR(DMSO-d₆, 500 MHz) δ 1.65-1.94 (4H, m), 2.32 (3H, s), 2.85-2.91 (1H, m), 3.12-3.19 (2H, m), 3.63 (1H, d, J = 11.2 Hz), 3.74 (1H, d, J = 11.2 Hz), 7.22-7.33 (5H, m), 8.30 (1H, brs), 8.99 (1H, brs);
HRMS m/z calcd for C₁₆H₁₉N₃S 285.1299, found 285.1286;
MS (EI) m/z: 285 [M⁺], 251, 226, 186, 160, 135, 109, 104, 91, 59;
Anal. Calcd for C₁₆H₁₉N₃S: C, 67.33; H, 6.71; N, 14.72; S, 11.23. Found: C, 67.25; H, 6.81; N, 14.53, S, 11.04.

### (35b) 4-(3-phenylpiperidin-1-yl)-2-thioxo-1,2-dihydropyridine-3-carbonitrile

(2Z)-2-cyano-3-(3-phenylpiperidin-1-yl)but-2-enethioamide which was produced in Example 35 (35a) was used in place of (2Z)-2-cyano-3-(isobutylamino)but-2-enethioamide and the reaction was performed in a similar method as described in Example 5 (5b) and the title compound was obtained.
Pale brown powder
Mp 236-238°C (decomposition);
IR (KBr) νₘₐₓ 3118, 2939, 2208, 1622, 1515, 1449, 1309, 1251, 1164, 974, 758, 700 cm⁻¹;
¹H NMR(DMSO-d₆, 500 MHz) δ 1.64-1.98 (4H, m), 2.84-2.90 (1H, m), 3.18-3.22 (2H, m), 4.12-4.15 (2H, m), 6.55 (1H, d, J = 7.3 Hz), 7.22-7.86 (5H, m), 7.46 (1H, d, J = 7.3 Hz), 12.66 (1H, brs);
HRMS m/z calcd for C₁₇H₁₈N₃S 296.1222, found 296.1196;
MS (FAB) m/z: 296 [M+H]⁺, 273, 242, 165, 65, 51;

### (35c) 3-amino-4-(3-phenylpiperidin-1-yl)thieno[2,3-b]pyridine-2-carboxamide

4-(3-phenylpiperidin-1-yl)-2-thioxo-1,2-dihydropyridine-3-carbonitrile which was produced in Example 35 (35b) was used in place of 4-(isobutylamino)-2-thioxo-1,2-dihydropyridine-3-carbonitrile and the reaction was performed in a similar method as described in Example 5 (5c) and the title compound was obtained.
Pale yellow powder
Mp 267-269°C;
IR (KBr) vₘₐₓ 3444, 3328, 3173, 2932, 1643, 1578, 1500, 1370, 1247, 1053, 966, 700cm⁻¹;
¹H NMR(DMSO-d₆, 500 MHz) δ 1.58-1.73 (1H, m), 1.86-2.02 (3H, m), 2.73-2.80 (2H, m), 3.09-3.16 (1H, m), 3.38-3.41 (2H, m), 6.99 (2H, brs), 7.06 (1H, d, J = 5.1 Hz), 7.12 (2H, brs), 7.20-7.33 (5H, m), 8.43 (1H, d, J = 5.1 Hz);
HRMS m/z calcd for C₁₉H₂₀ON₄S 352.1358, found 352.1360;
MS (EI) m/z: 352 [M⁺], 334, 307, 274, 252, 233, 202,176, 91, 77, 73;
Anal. Calcd for C₁₉H₂₀N₄OS·0.34H₂O: C, 63.64; H, 5.81; N, 15.62; S, 8.94. Found: C, 63.32; H, 5.57; N, 15.87, S, 8.69.

### (Example 36) 3-amino-4-(3-hydroxypiperidin-1-yl)thieno[2,3-b]pyridine-2-carboxamide (Exemplified Compound No. 1-27)

### (36a) (2Z)-2-cyano-3-(3-hydroxypiperidin-1-yl)but-2-enethioamide

3-hydroxypiperidine was used in place of isobutylamine and the reaction was performed in a similar method as described in Example 5 (5a) and the title compound was synthesized.
Pale yellow powder
Mp 159-162°C (decomposition);
IR (KBr) νₘₐₓ 3287, 2939, 2184, 1601, 1539, 1407, 1261, 1071, 859 cm⁻¹;
¹H NMR(DMSO-d₆, 400 MHz) δ 1.42-1.52 (2H, m), 1.74-1.86 (2H, m), 2.27 (3H, s), 3.03 (1H, dd, J = 7.8, 13.3 Hz), 3.11-3.16 (1H, m), 3.44 (1H, dd, J = 3.4, 13.3 Hz), 3.59-3.64 (1H, m), 5.01 (1H, d, J = 4.7 Hz), 8.14 (1H, brs), 8.91 (1H, brs);
HRMS m/z calcd for C₁₀H₁₆ON₃S 226.1014, found 226.1024;
MS (FAB) m/z: 226 [M+H]⁺, 192, 171, 65;
Anal. Calcd for C₁₀H₁₅N₃OS·0.04H₂O: C, 53.14; H, 6.72; N, 18.59; S, 14.19. Found: C, 53.00; H, 6.52; N, 18.47, S, 14.15.

### (36b) 4-(3-hydroxypiperidin-1-yl)-2-thioxo-1,2-dihydropyridine-3-carbonitrile

(2Z)-2-cyano-3-(3-hydroxypiperidin-1-yl)but-2-enethioamide which was produced in Example 36 (36a) was used in place of (2Z)-2-cyano-3-(isobutylamino)but-2-enethioamide and the reaction was performed in a similar method as described in Example 5 (5b) and the title compound was obtained.
Brown powder
Mp 202-206°C;
IR (KBr) νₘₐₓ 3119, 2945, 2208, 1625, 1522, 1251, 1173, 995, 962, 773 cm⁻¹;
¹H NMR(DMSO-d₆, 500 MHz) δ 1.46-1.53 (2H, m), 1.79-1.88 (2H, m), 3.29 (1H, dd, J = 7.8, 13.2 Hz), 3.39-3.44 (1H, m), 3.60-3.71 (2H, m), 3.80 (1H, dd, J = 3.4, 13.2 Hz), 6.48 (1H, d, J = 7.8 Hz), 7.44 (1H, d, J = 7.8 Hz), 12.59 (1H, brs).

### (36c) 3-amino-4-(3-hydroxypiperidin-1-yl)thieno[2,3-b]pyridine-2-carboxarnide

4-(3-hydroxypiperidin-1-yl)-2-thioxo-1,2-dihydropyridine-3-carbonitrile which was produced in Example 36 (36b) was used in place of 4-(isobutylamino)-2-thioxo-1,2-dihydropyridine-3-carbonitrile and the reaction was performed in a similar method as described in Example 5 (5c) and the title compound was obtained. Yellow powder
Mp 208-211°C;
IR (KBr) νₘₐₓ 3325, 1633, 1594, 1501, 1374, 1243, 959 cm⁻¹;
¹H NMR(DMSO-d₆, 400 MHz) δ 1.60-2.02 (4H, m), 3.16-3.96 (5H, m), 6.38 (2H, brs), 6.99 (1H, d, J = 5.1 Hz), 7.05 (2H, brs), 8.40 (1H, d, J = 5.1 Hz);
HRMS m/z calcd for C₁₃H₁₆O₂N₄S 292.0994, found 292.1013;
MS (EI) m/z: 292 [M⁺], 274, 256, 252, 218, 201, 176, 148, 128, 122, 43;
Anal. Calcd for C₁₃H₁₆N₄O₂S·0.16H₂O: C, 52.89; H, 5.57; N, 18.98; S, 10.86. Found:
C, 53.11; H, 5.55; N, 18.61, S, 10.86.

### (Example 37) tert-butyl 4-[3-amino-2-(aminocarbonyl)thieno[2,3-b]pyridin-4-yl]piperazine-1-carboxylate (Exemplified Compound No. 3-75)

### (37a) tert-butyl 4-[(1Z)-3-amino-2-cyano-1-methyl-3-thioxoprop-1-enyl]piperazine-1-carboxylate

tert-butyl piperazine-1-carboxylate was used in place of propylamine, the reaction was performed in a similar method as described in Example 4 (4a) and the title compound was obtained. Yield 40%.
¹H NMR(DMSO-d₆, 400 MHz) δ 1.41 (9H, s), 2.27 (3H, s), 3.36-3.46 (8H, m), 8.36 (1H, s), 9.06 (1H, s).

### (37b) tert-butyl 4-(3-cyano-2-thioxo-1,2-dihydropyridin-4-yl)piperazine-1-carboxylate

tert-butyl 4-[(1Z)-3-amino-2-cyano-1-methyl-3-thioxoprop-1-enyl]piperazine-1-carboxylate which was produced in Example 37 (37a) was used in place of (2Z)-2-cyano-3-(dimethylamino)but-2-enethioamide and the reaction was performed in a similar method as described in Example 1 (1b and the title compound was obtained.
Yield 69%.
¹H NMR(DMSO-d₆, 400 MHz) δ 2.49 (9H, s), 3.44-3.49 (4H, m), 3.61-3.66 (4H, m), 6.45 (1H, d, J = 7.4 Hz), 6.49 (1H, d, J = 7.4 Hz).
(37c) tert-butyl 4-[3-amino-2-(aminocarbonyl)thieno[2,3-b]pyridin-4-yl]piperazine-1-carboxylate

tert-butyl 4-(3-cyano-2-thioxo-1,2-dihydropyridin-4-yl)piperazine-1-carboxylate which was produced in Example 37 (37b) was used in place of 4-(dimethylamino)-2-thioxo-1,2-dihydropyridine-3-carbonitrile and the reaction was performed in a similar method as described in Example 1 (1c) and the title compound was synthesized. Yield 90%.
Mp 198-203°C;
IR (KBr) νₘₐₓ 3428, 3323, 3176, 2974, 1693, 1649, 1584, 1501, 1367, 1241, 1169, 1124, 976, 959, 825, 770 cm⁻¹;
¹H NMR(DMSO-d₆, 400 MHz) δ 1.42 (9H, s), 3.25(4H, s), 3.29 (4H, s), 6.95 (2H, br s), 7.03 (1H, d, J = 5.4 Hz), 7.12 (2H, br s), 8.45 (1H, d, J = 5.4 Hz);
MS (FAB) m/z: 378 [M+H]⁺;
Anal. Calcd for C₁₇H₂₃NₛO₃S·0.3H₂O: C,53.33; H,6.21; N,18.29; S,8.37. Found: C,53.14; H,5.86; N,18.06; S,8.41.

### (Example 38) 3-amino-4-piperazin-1-ylthieno[2,3-b]pyridine-2-carboxamide dihydrochloride (Exemplified Compound No. 3-1)

tert-butyl 4-[3-amino-2-(aminocarbonyl)thieno[2,3-b]pyridin-4-yl]piperazine-1-carboxylate (326 mg, 0.86 mmol) which was produced in Example 37 (37c) was suspended in 1,4-dioxane (10 mL) and was blended with 4N hydrochloric acid-dioxane (4 mL) and the mixture was stirred for two hours. After the solvent was evaporated, obtained yellow solid was dried under vacuum, and the title compound was obtained (316 mg, yield 100%).
Mp 270-280°C;
IR (KBr) νₘₐₓ 3320, 3180, 2925, 2770, 2717, 1648, 1604, 1446, 1395, 1259, 1059, 973, 906, 797, 556, 540, 516 cm⁻¹;
¹H NMR(DMSO-d₆, 400 MHz) δ 3.58-4.28 (8H, br s), 7.125 (1H, d, J = 5.1 Hz), 7.24 (2H, br s), 8.525 (1H, d, J = 5.1 Hz), 9.45 (2H, br s);
MS (FAB) m/z: 278 [M+H]⁺;
Anal. Calcd for C₁₂H₁₅N₅OS·2HCl·H₂O: C,39.14; H,5.20; N,19.02. Found: C,38.99; H,5.13; N,18.77.

### (Example 39) 3-amino-4-(4-methylpiperazin-1-yl)thieno[2,3-b]pyridine-2-carboxamide (Exemplified Compound No. 3-2)

### (39a) (2Z)-2-cyano-3-(4-methylpiperazin-1-yl)but-2-enethioamide

1-methylpiperazine was used in place of propylamine, the reaction was performed in a similar method as described in Example 4 (4a) and the title compound was obtained. Yield 85%.
¹H NMR(DMSO-d₆, 400 MHz) δ 2.18 (3H, s), 2.26 (3H, s), 2.38 (4H, m), 3.35 (4H, m), 8.29 (1H, br s), 8.99 (1H, br s).

### (39b) 3-amino-4-(4-methylpiperazin-1-yl)thieno[2,3-b]pyridine-2-carboxamide

(2Z)-2-cyano-3-(4-methylpiperazin-1-yl)but-2-enethioamide which was produced in Example 39 (39a) (301 mg, 1.34 mmol) and dimethylformamide dimethylacetal (0.53 mL, 4.01 mmol) were suspended in toluene (5 mL) and the mixture was stirred under reflux for three minutes. After cooling to room temperature, 1N aqueous solution of sodium hydroxide (3.50 mL) was added to the obtained residue by evaporating the solvent and it was heated for 30 minutes under reflux. After cooling to room temperature, 4N hydrochloric acid -dioxane solution (5 mL) was added and the pH value of solution was adjusted to around 3. The mixture was concentrated under reduced pressure and 2-chloroacetamide (126 mg, 1.35 mmol), 8N aqueous solution of sodium hydroxide (2.5 mL), dimethylformamide (5 mL) were added to the obtained residue and the mixture was stirred for three hours. Water (10 mL) was added to the reaction mixture and allowed to stand still for one week. The solid which resulted was filtered and the title compound was obtained (99 mg, yield 26%).
Mp 260-263°C;
IR (KBr) νₘₐₓ 3502, 3424, 3322, 3161, 2939, 2801, 1653, 1588, 1502, 1451, 1371, 1344, 1288, 1246, 1199, 973, 819, 737, 683, 625, 476 cm⁻¹;
¹H NMR(DMSO-d₆, 400 MHz) δ 2.26 (3H, s), 2.40-2.72 (4H, br s), 2.85-3.22 (4H, br s), 6.89 (2H, br s), 7.00 (1H, d, J = 5.5 Hz), 7.07 (2H, s), 8.41 (1H, d, J = 5.5 Hz); MS (FAB) m/z: 291 [M+H]⁺;
Anal. Calcd for C₁₃H₁₇N₅OS·0.3H₂O: C,52.61; H,5.98; N,23.60; S,10.80. Found: C,52.62; H,5.79; N,23.63; S,10.92.

### (Example 40) 3-amino-4-(4-ethyl piperazin-1-yl)thieno[2,3-b]pyridine-2-carboxamide (Exemplified Compound No. 3-3)

### (40a) (2Z)-2-cyano-3-(4-ethyl piperazin-1-yl)but-2-enethioamide

1-ethyl piperazine was used in place of propylamine and the title compound was obtained and the reaction was performed in a similar method as described in Example 4 (4a). Yield 67%.
¹H NMR(DMSO-d₆, 400 MHz) δ 1.00 (3H, t, J = 7.0 Hz), 2.25 (3H, s), 2.36 (2H, q, J = 7.0 Hz), 2.49 (4H, m), 3.36 (4H, m), 8.29 (1H, br s), 8.99 (1H, br s).

### (40b) 3-amino-4-(4-ethyl piperazin-1-yl)thieno[2,3-b]pyridine-2-carboxamide

(2Z)-2-cyano-3-(4-ethyl piperazin-1-yl)but-2-enethioamide (299 mg, 1.25 mmol) which was produced in Example 40 (40a) and dimethylformamide dimethylacetal (0.50 mL, 3.77 mmol) were suspended in toluene (5 mL) and the mixture was stirred under reflux for three minutes. After the reaction mixture was cooled to room temperature, 1N aqueous solution of sodium hydroxide (3.50 mL) was added to the obtained residue by evaporating the solvent, and heated under reflux for 40 minutes. After cooling to room temperature, 4N hydrochloric acid - dioxane solution (2 mL) was added and the pH value of solution was adjusted to around 4. The mixture was concentrated under reduced pressure and 2-chloroacetamide (118 mg, 1.27 mmol), 8N aqueous solution of sodium hydroxide (2.5 mL), dimethylformamide (5 mL) were added to the obtained residue and it was stirred for three hours. Water (10 mL) was added to the reaction mixture and the solid which resulted after allowing to stand still for one day was filtered and the title compound was obtained (123 mg, yield 32%).
Mp 218-219°C;
IR (KBr) νₘₐₓ 3425, 3316, 3173, 2968, 2821, 1644, 1581, 1503, 1448, 1375, 1346, 1243, 1136, 975, 826, 770, 735, 542, 478 cm⁻¹;
¹H NMR(DMSO-d₆, 400 MHz) δ 1.04 (3H, t, J = 7.0 Hz), 2.415 (2H, q, J = 7.0 Hz), 2.46-3.24 (8H, br s), 6.89 (2H, s), 7.015 (1H, d, J = 5.5 Hz), 7.08 (2H, s), 8.415 (1H, d, J = 5.5 Hz);
MS (FAB) m/z: 306 [M+H]⁺;
Anal. Calcd for C₁₄H₁₉N₅OS·0.5H₂O: C,53.48; H,6.41; N,22.27; S,10.20. Found: C,53.54; H,6.05; N,22.17; S,10.27.

### (Example 41) 3-amino-4-(4-isopropyl piperazin-1-yl)thieno[2,3-b]pyridine-2-carboxamide (Exemplified Compound No. 3-5)

### (41a) (2Z)-2-cyano-3-(4-isopropyl piperazin-1-yl)but-2-enethioamide

1-isopropyl piperazine was used in place of propylamine, the reaction was performed in a similar method as described in Example 4 (4a) and the title compound was obtained. Yield 67%.
¹H NMR(DMSO-d₆, 400 MHz) δ 0.955 (6H, d, J = 6.7 Hz), 2.25 (3H, s), 2.36 (2H, q, J = 7.0 Hz), 2.48 (4H, m), 2.65 (1H, m), 3.34 (4H, m), 8.25 (1H, br s), 8.94 (1H, br s).

### (41b) 3-amino-4-(4-isopropyl piperazin-1-yl)thieno[2,3-b]pyridine-2-carboxamide

(2Z)-2-cyano-3-(4-isopropyl piperazin-1-yl)but-2-enethioamide (299 mg, 1.18 mmol) which was produced in Example 41 (41 a) and dimethylformamide dimethylacetal (0.47 mL, 3.56 mmol) were suspended in toluene (5 mL) and the mixture was stirred under reflux for three minutes. After the reaction mixture was cooled to room temperature, 1N aqueous solution of sodium hydroxide (3.50 mL) was added to the obtained residue by evaporating the solvent, and heated under reflux for 40 minutes. After cooling to room temperature, 1N hydrochloric acid (5 mL) was added and the pH value of solution was adjusted to around 4. The solid which resulted was removed by filtration and the obtained filtrate was concentrated under reduced pressure. 2-chloroacetamide (153 mg, 1.64 mmol), 8N aqueous solution of sodium hydroxide (5 mL), dimethylformamide (1.5 mL) were added to the residue and the mixture was stirred for three hours. Water (10 mL) was added to the reaction mixture and the solid which resulted was filtered and washed with diisopropyl ether (4x3 mL) and the title compound was obtained (137 mg, yield 28%).
Mp 240-243°C;
IR (KBr) νₘₐₓ 3413, 3322, 3121, 2964, 2827, 1660, 1584, 1502, 1448, 1376, 1345, 1245, 1176, 1134, 982, 820, 766, 741, 646, 486 cm⁻¹;
¹H NMR(DMSO-d₆, 400 MHz) δ 1.015 (6H, d, J = 6.3 Hz), 2.40-3.40 (9H, br s), 6.90 (2H, br s), 7.00 (1H, d, J = 5.5 Hz), 7.06 (2H, s), 8.40 (1H, d, J = 5.1 Hz);
MS (FAB) m/z: 319 [M+H]⁺;
Anal. Calcd for C₁₅H₂₁N₅OS·0.19H₂O: C,55.80; H,6.67; N,21.69; S,9.93. Found: C,55.82; H,6.53; N,21.63; S,10.06.

### (Example 42) 3-amino-4-(4-phenylpiperazin-1-yl)thieno[2,3-b]pyridine-2-carboxamide (Exemplified Compound No. 3-9)

### (42a) (2Z)-2-cyano-3-(4-phenylpiperazin-1-yl)but-2-enethioamide

1-phenylpiperazine was used in place of propylamine, the reaction was performed in a similar method as described in Example 4 (4a) and the title compound was obtained. Yield 60%.
¹H NMR(DMSO-d₆, 400 MHz) δ 2.31 (3H, s), 3.27 (4H, m), 3.51 (4H, m), 6.78 (1H, t, J = 7.1 Hz), 6.92 (2H, d, J = 7.8 Hz), 7.21 (2H, m), 8.36 (1H, s), 9.05 (1H, s).

### (42b) 4-(4-phenylpiperazin-1-yl)-2-thioxo-1,2-dihydropyridine-3-carbonitrile

(2Z)-2-cyano-3-(4-phenylpiperazin-1-yl)but-2-enethioamide which was produced in Example 42 (42a) was used in place of (2Z)-2-cyano-3-(dimethylamino)but-2-enethioamide and the reaction was performed in a similar method as described in Example 1 (1b) and the title compound was obtained. Yield 77%.
¹H NMR(DMSO-d₆, 400 MHz) δ 3.31 (4H, m), 3.30 (4H, m), 6.535 (1H, d, J = 6.6 Hz), 6.79 (1H, t, J = 7.4 Hz), 6.93 (2H, d, J = 7.8 Hz), 7.14-7.25 (2H, m), 7.50 (2H, dd, J = 6.9, 7.4 Hz).

### (42c) 3-amino-4-(4-phenylpiperazin-1-yl)thieno[2,3-b]pyridine-2-carboxamide

4-(4-phenylpiperazin-1-yl)-2-thioxo-1,2-dihydropyridine-3-carbonitrile which was produced in Example 42 (42b) was used in place of 4-(dimethylamino)-2-thioxo-1,2-dihydropyridine-3-carbonitrile and the reaction was performed in a similar method as described in Example 1 (1c) and the title compound was synthesized.
Yield 75%.
Mp 250-252°C;
IR (KBr) νₘₐₓ 3438, 3318, 3176, 2832, 1645, 1596, 1579, 1447, 1377, 1343, 1238, 1136, 1135, 978, 914, 831, 762, 733, 693, 626, 484 cm⁻¹;
¹H NMR(DMSO-d₆, 400 MHz) δ 2.89-3.71 (8H, br s), 6.70 (1H, t, J = 7.1 Hz), 6.93 (2H, s), 6.99 (2H, d, J = 9.0 Hz), 7.07 (2H, d, J = 5.5 Hz), 7.10 (2H, s), 7.22 (2H, dd, J = 7.4, 8.6 Hz), 8.44 (1H, d, J = 5.5 Hz);
MS (FAB) m/z: 353 [M+H]⁺;
Anal. Calcd for C₁₈H₁₉N₅OS·0.26H₂O: C,60.37; H,5.49; N,19.56; S,8.95. Found: C, 60.18; H,5.33; N,19.55; S,9.02.

### (Example 43) 3-amino-4-(4-benzylpiperazin-1-yl)thieno[2,3-b]pyridine-2-carboxamide (Exemplified Compound No. 3-6)

### (43a) (2Z)-3-(4-benzylpiperazin-1-yl) -2-cyanobut-2-enethioamide

1-benzylpiperazine was used in place of propylamine, the reaction was performed in a similar method as described in Example 4 (4a) and the title compound was obtained. Yield 33%.
¹H NMR(DMSO-d₆, 400 MHz) δ 2.25 (3H, s), 2.44 (4H, m), 3.37 (4H, m), 3.50 (2H, s), 7.22-7.34 (5H, m), 8.30 (1H, s), 8.98 (1H, s).

### (43b) 4-(4-benzylpiperazin-4-yl)-2-thioxo-1,2-dihydropyridine-3-carbonitrile

(2Z)-3-(4-benzylpiperazin-1-yl)-2-cyanobut-2-enethioamide which was produced in Example 42 (42a) was used in place of (2Z)-2-cyano-3-(dimethylamino)but-2-enethioamide and the reaction was performed in a similar method as described in Example 1 (1b) and the title compound was obtained. Yield 78%.
¹H NMR(DMSO-d₆, 400 MHz) δ 2.49 (4H, m), 3.51 (2H, s), 3.61 (4H, m), 6.45 (1H, d, J = 7.8 Hz), 7.20-7.34 (5H, m), 7.50 (1H, d, J = 7.4 Hz).

### (43c) 3-amino-4-(4-benzylpiperazin-1-yl)thieno[2,3-b]pyridine-2-carboxamide

4-(4-phenylpiperazin-1-yl)-2-thioxo-1,2-dihydropyridine-3-carbonitrile which was produced in Example 42 (42b) was used in place of 4-(dimethylamino)-2-thioxo-1,2-dihydropyridine-3-carbonitrile and the reaction was performed in a similar method as described in Example 1 (1c) and the title compound was synthesized.
Yield 75%.
Mp 241-242°C;
IR (KBr) νₘₐₓ 3435, 3398, 3322, 3128, 2828, 1656, 1585, 1503, 1452, 1373, 1348, 1250, 1232, 1132, 1061, 1009, 973, 826, 742, 699, 627, 551, 487 cm⁻¹;
¹H NMR(DMSO-d₆, 400 MHz) δ 2.42-3.22 (8H, br s), 3.55 (1H, s), 6.89 (2H, s), 7.02 (1H, d, J = 5.5 Hz), 7.07 (2H, s), 7.20-7.34 (5H, s), 8.415 (1H, d, J = 5.1 Hz); MS (FAB) m/z: 367 [M+H]⁺;
Anal. Calcd for C₁₉H₂₁N₅OS: C,62.10; H,5.76; N,19.06; S,8.73. Found: C,61.96; H,5.63; N,18.76; S,8.75.

### (Example 44) 3-amino-4-(4-pyrimidin-2-ylpiperazin-1-yl)thieno[2,3-b]pyridine-2-carboxamide (Exemplified Compound No. 3-69)

### (44a) (2Z)-2-cyano-3-(4-pyrimidin-2-ylpiperazin-1-yl)but-2-enethioamide

1-pyrimidin-2-ylpiperazine was used in place of propylamine, the reaction was performed in a similar method as described in Example 4 (4a) and the title compound was obtained. Yield 87%.
¹H NMR(DMSO-d₆, 400 MHz) δ 2.31 (3H, s), 3.51 (4H, m), 3.83 (4H, m), 6.66 (1H, t, J = 4.7 Hz), 8.32 (1H, s), 6.92 (2H, d, J = 4.7 Hz), 9.03 (1H, s).

### (44b) 4-(4-pyrimidin-2-ylpiperazin-1-yl)-2-thioxo-1,2-dihydropyridine-3-carbonitrile

(2Z)-2-cyano-3-(4-pyrimidin-2-ylpiperazin-1-yl)but-2-enethioamide which was produced in Example 44 (44a) was used in place of (2Z)-2-cyano-3-(dimethylamino)but-2-enethioamide and the reaction was performed in a similar method as described in Example 1 (1b) and the title compound was obtained. Yield 77%.
¹H NMR(DMSO-d₆, 400 MHz) δ 3.785 (4H, m), 3.89 (4H, m), 6.53 (1H, d, J = 7.4 Hz), 6.69 (1H, t, J = 4.3 Hz), 7.52 (1H, t, J = 7.8 Hz), 7.50 (2H, d, J = 4.7 Hz), 12.74 (1H, br s).

### (44c) 3-amino-4-(4-pyrimidin-2-ylpiperazin-1-yl)thieno[2,3-b]pyridine-2-carboxamide

(2Z)-2-cyano-3-(4-pyrimidin-2-ylpiperazin-1-yl)but-2-enethioamide which was produced in Example 44 (44b) was used in place of 4-(dimethylamino)-2-thioxo-1,2-dihydropyridine-3-carbonitrile and the reaction was performed in a similar method as described in Example 1 (1c) and the title compound was synthesized.
Yield 69%.
Mp 280°C (decomposition);
IR (KBr) νₘₐₓ 3439, 3317, 3141, 2835, 1648, 1582, 1548, 1500, 1466, 1359, 1241, 1133, 974, 797, 486 cm⁻¹;
¹H NMR(DMSO-d₆, 400 MHz) δ 2.72-3.65 (8H, br s), 6.66 (1H, t, J = 4.7 Hz), 6.99 (2H, br s), 7.05 (2H, d, J = 5.1 Hz), 7.11 (2H, br s), 8.39 (2H, d, J = 4.7 Hz), 8.43 (1H, d, J = 5.5 Hz);
MS (FAB) m/z: 356 [M+H]⁺;
Anal. Calcd for C₁₆H₁₇N₇OS·0.24H₂O: C,54.07; H,4.82; N,27.59; S,9.02. Found: C,53.59; H,4.81; N,27.03; S,8.99.

### (Example 45) 3-amino-4-(4-pyridin-2-ylpiperazin-1-yl)thieno[2,3-b]pyridine-2-carboxamide (Exemplified Compound No. 3-62)

### (45a) (2Z)-2-cyano-3-(4-pyridin-2-ylpiperazin-1-yl)but-2-enethioamide

1-pyridin-2-ylpiperazine was used in place of propylamine, the reaction was performed in a similar method as described in Example 4 (4a) and the title compound was obtained. Yield 92%.
¹H NMR(DMSO-d₆, 400 MHz) δ 2.32 (3H, s), 3.53 (4H, m), 3.65 (4H, m), 6.67 (1H, t, J = 4.7 Hz), 6.83 (1H, d, J = 8.9 Hz), 7.57 (1H, t, J = 8.9 Hz), 8.14 (1H, d, J = 4.7 Hz), 8.36 (1H, br s), 9.07 (1H, br s).

### (45b) 4-(4-pyridin-2-ylpiperazin-1-yl)-2-thioxo-1,2-dihydropyridine-3-carbonitrile

(2Z)-2-cyano-3-(4-pyridin-2-ylpiperazin-1-yl)but-2-enethioamide which was produced in Example 45 (45a) was used in place of (2Z)-2-cyano-3-(dimethylamino)but-2-enethioamide and the reaction was performed in a similar method as described in Example 1 (1b) and the title compound was obtained. Yield 43%.
¹H NMR(DMSO-d₆, 400 MHz) δ 3.70 (4H, m), 3.82 (4H, m), 6.53 (1H, d, J = 7.4 Hz), 6.72 (1H, t, J = 5.9 Hz), 6.90 (1H, d, J = 8.2 Hz), 7.52 (1H, t, J = 6.7 Hz), 7.64 (1H, t, J = 7.4 Hz), 8.13 (1H, d, J = 5.1 Hz), 12.73 (1H, br s).

### (45c) 3-amino-4-(4-pyridin-2-ylpiperazin-1-yl)thieno[2,3-b]pyridine-2-carboxamide

4-(4-pyridin-2-ylpiperazin-1-yl)-2-thioxo-1,2-dihydropyridine-3-carbonitrile which was produced in Example 45 (45b) was used in place of 4-(dimethylamino)-2-thioxo-1,2-dihydropyridine-3-carbonitrile and the reaction was performed in a similar method as described in Example 1 (1c) and the title compound was synthesized. Yield 77%.
Mp 266-270°C;
IR (KBr) νₘₐₓ 3438, 3317, 3142, 2834, 1674, 1590, 1582, 1501, 1435, 1369, 1345, 1133, 974, 774, 741, 620, 487 cm⁻¹;
¹H NMR(DMSO-d₆, 400 MHz) δ 2.80-3.60 (8H, br s), 6.67 (1H, dd, J = 5.1, 6.3 Hz), 6.89 (1H, d, J = 8.2 Hz), 6.97 (2H, s), 7.06 (1H, d, J = 5.1 Hz), 7.11 (2H, s), 7.55 (1H, t, J = 5.1 Hz), 8.39 (1H, d, J = 3.5 Hz), 8.44 (1H, d, J = 5.1 Hz);
MS (FAB) m/z: 355 [M+H]⁺;
Anal. Calcd for C₁₇H₁₈N₆OS: C,57.61; H,5.12; N,23.71; S,9.05. Found: C,57.25; H,5.01; N,23.35; S,9.04.

### (Example 46) 3-amino-4-[4-(4-methylphenyl)piperazin-1-yl]thieno[2,3-b]pyridine-2-carboxamide (Exemplified Compound No. 3-34)

### (46a) (2Z)-2-cyano-3-[4-(4-methylphenyl)piperazin-1-yl]but-2-enethioamide

1-(4-methylphenyl)piperazine was used in place of propylamine, the reaction was performed in a similar method as described in Example 4 (4a) and the title compound was obtained. Yield 70%.
¹H NMR(DMSO-d₆, 400 MHz) δ 2.20 (3H, s), 2.30 (3H, s), 3.20 (4H, m), 3.51 (4H, m), 6.83 (2H, d, J = 8.6 Hz), 7.02 (2H, d, J = 8.2 Hz), 8.35 (1H, s), 9.04 (1H, s).

### (46b) 4-[4-(4-methylphenyl)piperazin-1-yl]-2-thioxo-1,2-dihydropyridine-3-carbonitrile

(2Z)-2-cyano-3-[4-(4-methylphenyl)piperazin-1-yl]but-2-enethioamide which was produced in Example 46 (46a) was used in place of (2Z)-2-cyano-3-(dimethylamino)but-2-enethioamide and the reaction was performed in a similar method as described in Example 1 (1b) and the title compound was obtained. Yield 77%.
¹H NMR(DMSO-d₆, 400 MHz) δ 2.21 (3H, s), 3.22 (4H, m), 3.79 (4H, m), 6.45 (1H, d, J = 7.4 Hz), 6.86 (2H, d, J = 8.6 Hz), 7.05 (2H, d, J = 8.2 Hz), 7.52 (1H, t, J = 7.0 Hz), 12.73 (1H, br s).

### (46c) 3-amino-4-[4-(4-methylphenyl)piperazin-1-yl]thieno[2,3-b]pyridine-2-carboxamide

4-[4-(4-methylphenyl)piperazin-1-yl]-2-thioxo-1,2-dihydropyridine-3-carbonitrile which was produced in Example 46 (46b) was used in place of 4-(dimethylamino)-2-thioxo-1,2-dihydropyridine-3-carbonitrile, the reaction was performed in a similar method as described in Example 1 (1c) and the title compound was synthesized. Yield 72%.
Mp 270°C (decomposition);
IR (KBr) νₘₐₓ 3433, 3310, 3143, 2829, 1667, 1611, 1580, 1513, 1449, 1371, 1345, 1237, 1134, 1124, 974, 956, 812, 617, 482 cm⁻¹;
¹H NMR(DMSO-d₆, 400 MHz) δ 2.20 (3H, s), 2.91-3.60 (8H, br s), 6.86-7.12 (9H, m), 8.42 (1H, d, J = 5.5 Hz);
MS (FAB) m/z: 367 [M+H]⁺;
Anal. Calcd for C₁₉H₂₁N₅OS·0.24H₂O: C,61.38; H,5.82; N,18.84; S,8.62. Found: C, 61.58; H,5.58; N,18.84; S,8.63.

### (Example 47) 3-amino-4-[4-(4-fluorophenyl)piperazin-1-yl]thieno[2,3-b]pyridine-2-carboxamide (Exemplified Compound No. 3-12)

### (47a) (2Z)-2-cyano-3-[4-(4-fluorophenyl)piperazin-1-yl]but-2-enethioamide

1-(4-fluorophenyl) piperazine was used in place of propylamine, the reaction was performed in a similar method as described in Example 4 (4a) and the title compound was obtained. Yield 73%.
¹H NMR(DMSO-d₆, 400 MHz) δ 2.31 (3H, s), 3.21 (4H, m), 3.52 (4H, m), 6.83 (2H, dd, J = 4.7, 9.0 Hz), 7.02 (2H, t, J = 8.6 Hz), 8.37 (1H, s), 9.06 (1H, s).

### (47b) 4-[4-(4-fluorophenyl)piperazin-1-yl]-2-thioxo-1,2-dihydropyridine-3-carbonitrile

(2Z)-2-cyano-3-[4-(4-fluorophenyl)piperazin-1-yl]but-2-enethioamide which was produced in Example 47 (47a) was used in place of (2Z)-2-cyano-3-(dimethylamino)but-2-enethioamide and the reaction was performed in a similar method as described in Example 1 (1b) and the title compound was obtained. Yield 74%.
¹H NMR(DMSO-d₆, 400 MHz) δ 3.24 (4H, m), 3.80 (4H, m), 6.56 (1H, d, J = 7.4 Hz), 6.98 (2H, dd, J = 4.7, 9.4 Hz), 7.08 (2H, t, J = 8.9 Hz), 7.52 (1H, d, J = 7.4 Hz).

### (47c) 3-amino-4-[4-(4-fluorophenyl) piperazin-1-yl]thieno[2,3-b]pyridine-2-carboxamide

4-[4-(4-fluorophenyl)piperazin-1-yl]-2-thioxo-1,2-dihydropyridine-3-carbonitrile which was produced in Example 47 (47b) was used in place of 4-(dimethylamino)-2-thioxo-1,2-dihydropyridine-3-carbonitrile, and the reaction was performed in a similar method as described in Example 1 (1c) and the title compound was synthesized. Yield 62%.
Mp 275°C (decomposition);
IR (KBr) νₘₐₓ 3443, 3324, 3180, 2832, 1646, 1583, 1558, 1509, 1450, 1373, 1345, 1234, 1136, 977, 959, 826, 716, 553 cm⁻¹;
¹H NMR(DMSO-d₆, 400 MHz) δ 2.93-3.75 (8H, br s), 6.96 (2H, s), 7.00-7.19 (7H, m), 8.48 (1H, d, J = 5.1 Hz);
MS (FAB) m/z: 371 [M+H]⁺;
Anal. Calcd for C₁₈H₁₈FN₅OS·0.19H₂O: C,57.67; H,4.94; N,18.68; S,8.55. Found: C, 57.72; H,4.72; N,18.S8; S,8.53.

### (Example 48) 3-amino-4-(4-benzhydrylpiperazin-1-yl)thieno[2,3-b]pyridine-2-carboxamide (Exemplified Compound No. 3-7)

### (48a) (2Z)-3-(4-benzhydrylpiperazin-1-yl)-2-cyanobut-2-enethioamide

1-benzhydrylpiperazine was used in place of propylamine, the reaction was performed in a similar method as described in Example 4 (4a) and the title compound was obtained. Yield 90%.
¹H NMR(DMSO-d₆, 400 MHz) δ 2.22 (3H, s), 2.38 (4H, m), 3.41 (4H, m), 4.34 (1H, s), 7.18 (2H, t, J = 7.4 Hz), 7.28 (4H, t, J = 7.8 Hz), 7.42 (4H, d, J = 7.0 Hz), 8.31 (1H, s), 8.98 (1H, s).

### (48b) 4-(4-benzhydrylpiperazin-1-yl)-2-thioxo-1,2-dihydropyridine-3-carbonitrile

(2Z)-3-(4-benzhydrylpiperazin-1-yl)-2-cyanobut-2-enethioamide which was produced in Example 48 (48a) was used in place of (2Z)-2-cyano-3-(dimethylamino)but-2-enethioamide and the reaction was performed in a similar method as described in Example 1 (1b) and the title compound was obtained. Yield 84%.
¹H NMR(DMSO-d₆, 400 MHz) δ 2.44 (4H, m), 3.66 (4H, m), 4.38 (1H, s), 6.47 (1H, d, J = 7.0 Hz), 7.23 (1H, d, J = 7.0 Hz), 7.27-7.66 (10H, m).

### (48c) 3-amino-4-(4-benzhydrylpiperazin-1-yl)thieno[2,3-b]pyridine-2-carboxamide

4-(4-benzhydrylpiperazin-1-yl)-2-thioxo-1,2-dihydropyridine-3-carbonitrile which was produced in Example 48 (48b) was used in place of 4-(dimethylamino)-2-thioxo-1,2-dihydropyridine-3-carbonitrile and the reaction was performed in a similar method as described in Example 1 (1c) and the title compound was synthesized. Yield 63%.
Mp 225-233°C;
IR (KBr) νₘₐₓ 3450, 3381, 3171, 2827, 1649, 1580, 1501, 1449, 1366, 1242, 1137, 979, 957, 835, 758, 706, 626, 473 cm⁻¹;
¹H NMR(DMSO-d₆, 400 MHz) δ 2.20-3.40 (8H, br s), 4.38 (1H, s), 6.89 (1H, s), 7.07 (2H, m), 7.20 (2H, t, J = 7.4 Hz), 7.31 (5H, t, J = 7.4 Hz), 7.47 (5H, d, J = 7.4 Hz), 8.44 (1H, d, J = 5.5 Hz);
MS (FAB) m/z: 444 [M+H]⁺;
Anal. Calcd for C₂₅H₂₅N₅OS·0.26H₂O: C,66.99; H,5.74; N,15.62; S,7.15. Found: C, 67.07; H,5.57; N,15.36; S,7.09.

### (Example 49) 3-amino-4-[4-(4-methoxyphenyl)piperazin-1-yl]thieno[2,3-b]pyridine-2-carboxamide (Exemplified Compound No. 3-47)

### (49a) (2Z)-2-cyano-3-[4-(4-methoxyphenyl)piperazin-1-yl]but-2-enethioamide

1-(4-methoxyphenyl) piperazine was used in place of propylamine, the reaction was performed in a similar method as described in Example 4 (4a) and the title compound was obtained. Yield 73%.
¹H NMR(DMSO-d₆, 400 MHz) δ 2.31 (3H, s), 3.13 (4H, m), 3.52 (4H, m), 3.69 (3H, s), 6.84 (2H, d, J = 9.0 Hz), 6.92 (2H, d, J = 9.3 Hz), 8.39 (1H, s), 9.07 (1H, s).

### (49b) 4-[4-(4-methoxyphenyl)piperazin-1-yl]-2-thioxo-1,2-dihydropyridine-3-carbonitrile

(2Z)-2-cyano-3-[4-(4-methoxyphenyl)piperazin-1-yl]but-2-enethioamide which was produced in Example 49 (49a) was used in place of (2Z)-2-cyano-3-(dimethylamino)but-2-enethioamide and the reaction was performed in a similar method as described in Example 1 (1b) and the title compound was obtained. Yield 59%.
¹H NMR(DMSO-d₆, 400 MHz) δ 3.14 (4H, m), 3.67 (3H, s), 3.77 (4H, m), 6.45 (1H, d, J = 7.1 Hz), 6.86 (2H, d, J = 9.4 Hz), 7.05 (2H, d, J = 9.4 Hz), 7.52 (1H, t, J = 7.4 Hz).

### (49c) 3-amino-4-[4-(4-methoxyphenyl)-piperazin-1-yl]thieno[2,3-b]pyridine-2-carboxamide

4-[4-(4-methoxyphenyl)piperazin-1-yl]-2-thioxo-1,2-dihydropyridine-3-carbonitrile which was produced in Example 49 (49b) was used in place of 4-(dimethylamino)-2-thioxo-1,2-dihydropyridine-3-carbonitrile, the reaction was performed in a similar method as described in Example 1 (1c) and the title compound was synthesized. Yield 79%.
Mp 275°C (decomposition);
IR (KBr) νₘₐₓ 3435, 3311, 3160, 2957, 2831, 1664, 1609, 1511, 1449, 1373, 1346, 1242, 1182, 1136, 1035, 976, 959, 826, 740, 605, 480 cm⁻¹;
¹H NMR(DMSO-d₆, 400 MHz) δ 2.95-3.56 (8H, br s), 3.69 (1H, s), 6.3 (1H, d, J = 9.0 Hz), 6.93 (2H, s), 6.95 (1H, d, J = 9.4 Hz), 7.07 (1H, d, J = 5.5 Hz), 7.09 (2H, s), 8.45 (1H, d, J = 5.5 Hz);
MS (FAB) m/z: 384 [M+H]⁺;
Anal. Calcd for C₁₉H₂₁N₅O₂S·0.28H₂O: C,58.74; H,5.59; N,18.03; S,8.25. Found: C, 58.57; H,5.42;N,18.03; S,8.03.

### (Example 50) 3-amino-4-{4-[(2E)-3-phenylprop-2-enyl]piperazin-1-yl}thieno[2,3-b]pyridine-2-carboxamide (Exemplified Compound No. 3-8)

### (50a) (2Z)-2-cyano-3-{4-[(2E)-3-phenylpropane-2-enyl]piperazin-1-yl}but-2-enethioamide

1-[(2E)-3-phenylpropane-2-enyl]piperazine was used in place of propylamine, the reaction was performed in a similar method as described in Example 4 (4a) and the title compound was obtained. Yield 38%.
¹H NMR(DMSO-d₆, 400 MHz) δ 2.27 (3H, s), 2.50 (4H, m), 3.14 (2H, d, J = 5.9 Hz), 3.40 (4H, m), 3.69 (3H, s), 6.30 (1H, dt, J = 6.6, 16.0 Hz), 6.56 (1H, d, J = 16.0 Hz), 7.24 (1H, t, J = 7.4 Hz), 7.32 (2H, t, J = 7.0 Hz), 7.44 (2H, d, J = 7.0 Hz), 8.32 (1H, s), 9.01 (1H, s).

### (50b) 4-{4-[(2E)-3-phenylpropane-2-enyl]piperazin-1-yl}-2-thioxo-1,2-dihydropyridine-3-carbonitrile

(2Z)-2-cyano-3- {4-[(2E)-3-phenylpropane-2-enyl]piperazin-1-yl} but-2-enethioamide which was produced in Example 50 (50a) was used in place of (2Z)-2-cyano-3-(dimethylamino)but-2-enethioamide and the reaction was performed in a similar method as described in Example 1 (1b) and the title compound was obtained.
Yield 84%.
¹H NMR(DMSO-d₆, 400 MHz) δ 2.85-4.44 (10H, m), 6.28 (1H, dt, J = 7.8, 14.8 Hz), 6.55 (1H, d, J = 7.4 Hz), 6.79 (1H, d, J = 14.9 Hz), 7.31 (1H, d, J = 7.4 Hz), 7.37 (2H, t, J = 7.4 Hz), 7.48 (2H, d, J = 7.4 Hz), 7.57 (1H, t, J = 6.7 Hz), 12.91 (1H, s).

### (50c) 3-amino-4-{4-[(2E)-3-phenylprop-2-enyl]piperazin-1-yl}thieno[2,3-b]pyridine-2-carboxamide

4-{4-[(2E)-3-phenylpropane-2-enyl]piperazin-1-yl}-2-thioxo-1,2-dihydropyridine-3-carbonitrile which was produced in Example 50 (50b) was used in place of 4-(dimethylamino)-2-thioxo-1,2-dihydropyridine-3-carbonitrile and the reaction was performed in a similar method as described in Example 1 (1c) and the title compound was synthesized. Yield 60%.
Mp 225-227°C;
IR (KBr) νₘₐₓ 3433, 3319, 3184, 3024, 2832, 2811, 1649, 1578, 1501, 1448, 1369, 1347, 1129, 971, 823, 736, 693, 627, 470 cm⁻¹;
¹H NMR(DMSO-d₆, 400 MHz) δ 2.42-3.81 (8H, br s), 3.19 (1H, d, J = 6.3 Hz), 6.32 (1H, dt, J = 6.6, 16.0 Hz), 6.55 (1H, d, J = 16.0 Hz), 6.90 (2H, s), 7.02 (1H, d, J = 5.0 Hz), 7.07 (2H, s), 7.21 (1H, t, J = 7.4 Hz), 7.30 (2H, t, J = 7.4 Hz), 7.43 (2H, d, J = 7.0 Hz), 8.41 (1H, d, J = 5.1 Hz);
MS (FAB) m/z: 394 [M+H]⁺;
Anal. Calcd for C₂₁H₂₃N₅OS·0.46H₂O: C,62.78; H,6.00; N,17.43; S,7.98. Found: C, 62.45; H, 5.70; N,17.32; S,7.94.

### (Example 51) 3-amino-4-(4-benzoylpiperazin-1-yl)thieno[2,3-b]pyridine-2-carboxamide (Exemplified Compound No. 3-73)

3-amino-4-piperazin-1-ylthieno[2,3-b]pyridine-2-carboxamide dihydrochloride (99.7 mg, 0.28 mmol) which was produced in Example 38 was suspended in 1,4-dioxane (5 mL) and was blended with benzoyl chloride (37 µL, 0.31 mmol) and triethylamine (44 µL, 0.31 mmol) and the mixture was stirred for 12 hours. After evaporating the solvent, water (15 mL) was added and allowed to stand for several hours, a brown solid which was generated was filtrated, dried under vacuum and the title compound was obtained (31.8 mg, yield 29%).
Mp >300°C;
IR (KBr) νₘₐₓ 3437, 3325, 3182, 2923, 2856, 1635, 1598, 1567, 1495, 1411, 1284, 1244, 1013, 974, 720, 675, 482 cm⁻¹;
¹H NMR(DMSO-d₆, 400 MHz) δ 2.95-3.66 (8H, br s), 6.99 (2H, s), 7.07 (1H, s), 7.15 (2H, s), 7.42-7.57 (3H, m), 7.96 (2H, m), 8.48 (1H, s);
MS (FAB) m/z: 382 [M+H]⁺.

### (Example 52) 3-amino-4-[4-(4-chlorophenyl)piperazin-1-yl]thieno[2,3-b]pyridine-2-carboxamide (Exemplified Compound No. 3-17)

### (52a) (2Z)-3-[4-(4-chlorophenyl)piperazin-1-yl]-2-cyanobut-2-enethioamide

1-(4-chlorophenyl) piperazine was used in place of propylamine, the reaction was performed in a similar method as described in Example 4 (4a) and the title compound was obtained. Yield 73%.
¹H NMR(DMSO-d₆, 400 MHz) δ 2.31 (3H, s), 3.29 (4H, m), 3.53 (4H, m), 6.95 (2H, d, J = 9.0 Hz), 7.25 (2H, d, J = 9.0 Hz), 8.39 (1H, s), 9.10 (1H, s).

### (52b) 4-[4-(4-chlorophenyl)piperazin-1-yl]-2-thioxo-1,2-dihydropyridine-3-carbonitrile

(2Z)-3-[4-(4-chlorophenyl)piperazin-1-yl]-2-cyanobut-2-enethioamide which was produced in Example 52 (52a) was used in place of (2Z)-2-cyano-3-(dimethylamino)but-2-enethioamide and the reaction was performed in a similar method as described in Example 1 (1b) and the title compound was obtained. Yield 74%.
¹H NMR(DMSO-d₆, 400 MHz) δ 3.31 (4H, m), 3.79 (4H, m), 6.52 (1H, d, J = 7.4 Hz), 6.93 (2H, d, J = 9.4 Hz), 7.23 (2H, d, J = 9.0 Hz), 7.50 (1H, m);

### (52c) 3-amino-4-[4-(4-chlorophenyl) piperazin-1-yl]thieno[2,3-b]pyridine-2-carboxamide

4-[4-(4-chlorophenyl)piperazin-1-yl]-2-thioxo-1,2-dihydropyridine-3-carbonitrile which was produced in Example 52 (52b) was used in place of 4-(dimethylamino)-2-thioxo-1,2-dihydropyridine-3-carbonitrile, and the reaction was performed in a similar method as described in Example 1 (1c) and the title compound was synthesized. Yield 50%.
Mp 297°C (decomposition);
IR (KBr) νₘₐₓ 3423, 3317, 3164, 2975, 2832, 1669, 1649, 1580, 1496, 1449, 1370, 1343, 1238, 1137, 1106, 976, 962, 819, 609, 487 cm⁻¹;
¹H NMR(DMSO-d₆, 400 MHz) δ 2.95-3.66 (8H, br s), 6.94 (2H, s), 7.01 (2H, d, J = 9.0 Hz), 7.065 (1H, d, J = 5.1 Hz), 7.11 (2H, s), 7.25 (2H, d, J = 9.0 Hz), 8.445 (1H, d, J = 5.4 Hz);
MS (FAB) m/z: 388 [M+H]⁺;
Anal. Calcd for C₁₈H₁₈ClN₅OS: C, 55.74; H, 4.68; N, 18.06; S, 8.27. Found: C, 55.43; H, 4.75; N, 17.98; S, 8.12.

### (Example 53) 3-amino-4-[4-(2-methoxyphenyl)piperazin-1-yl]thieno[2,3-b]pyridine-2-carboxamide (Exemplified Compound No. 3-45)

### (53a) (2Z)-2-cyano-3-[4-(2-methoxyphenyl)piperazin-1-yl]but-2-enethioamide

1-(2-methoxyphenyl) piperazine was used in place of propylamine, the reaction was performed in a similar method as described in Example 4 (4a) and the title compound was obtained. Yield 68%.
¹H NMR(DMSO-d₆, 400 MHz) δ 2.31 (3H, s), 3.05 (4H, m), 3.51 (4H, m), 3.80 (3H, s), 6.85-7.04 (4H, m), 8.39 (1H, s), 9.08 (1H, s).

### (53b) 4-[4-(2-methoxyphenyl)piperazin-1-yl]-2-thioxo-1,2-dihydropyridine-3-carbonitrile

(2Z)-2-cyano-3-[4-(2-methoxyphenyl)piperazin-1-yl]but-2-enethioamide which was produced in Example 53 (53a) was used in place of (2Z)-2-cyano-3-(dimethylamino)but-2-enethioamide and the reaction was performed in a similar method as described in Example 1 (1b) and the title compound was obtained. Yield 76%.
¹H NMR(DMSO-d₆, 400 MHz) δ 3.08 (4H, m), 3.79 (7H, m), 6.44 (1H, d, J = 7.4 Hz), 6.86 (2H, s), 7.05 (2H, s), 7.49 (1H, t, J = 6.6 Hz), 12.96 (1H, s).

### (53c) 3-amino-4-[4-(2-methoxyphenyl) piperazin-1-yl]thieno[2,3-b]pyridine-2-carboxamide

4-[4-(2-methoxyphenyl)piperazin-1-yl]-2-thioxo-1,2-dihydropyridine-3-carbonitrile which was produced in Example 53 (53b) was used in place of 4-(dimethylamino)-2-thioxo-1,2-dihydropyridine-3-carbonitrile and the reaction was performed in a similar method as described in Example 1 (1c) and the title compound was synthesized. Yield 71 %.
Mp 294°C (decomposition);
IR (KBr) νₘₐₓ 3435, 3311, 3160, 2957, 2831, 1664, 1609, 1511, 1449, 1373, 1346, 1242, 1182, 1136, 1035, 976, 959, 826, 740, 605, 480 cm⁻¹;
¹H NMR(DMSO-d₆, 400 MHz) δ 2.96-3.56 (8H, br s), 3.79 (3H, s), 6.86-7.00 (6H, m), 7.10 (3H, d, J = 5.1 Hz), 8.445 (1H, d, J = 5.1 Hz);
MS (FAB) m/z: 384 [M+H]⁺;
Anal. Calcd for C₁₉H₂₁N₅O₂S·0.1H₂O: C, 59.23; H, 5.55; N, 18.18; S,8.32. Found: C, 58.94; H, 5.25; N, 18.29; S, 8.21.

### (Example 54) 3-amino-4-[4-(2-chlorophenyl)piperazin-1-yl]thieno[2,3-b]pyridine-2-carboxamide (Exemplified Compound No. 3-15)

### (54a) (2Z)-3-[4-(2-chlorophenyl)piperazin-1-yl]-2-cyanobut-2-enethioamide

1-(2-chlorophenyl) piperazine was used in place of propylamine, the reaction was performed in a similar method as described in Example 4 (4a) and the title compound was obtained. Yield 71 %.
¹H NMR(DMSO-d₆, 400 MHz) δ 2.31 (3H, s), 3.05 (4H, m), 3.51 (4H, m), 3.80 (3H, s), 6.85-7.04 (4H, m), 8.39 (1H, s), 9.08 (1H, s).

### (54b) 4-[4-(2-chlorophenyl)-piperazin-1-yl]-2-thioxo-1,2-dihydropyridine-3-carbonitrile

(2Z)-3-[4-(2-chlorophenyl)piperazin-1-yl]-2-cyanobut-2-enethioamide which was produced in Example 54 (54a) was used in place of (2Z)-2-cyano-3-(dimethylamino)but-2-enethioamide and the reaction was performed in a similar method as described in Example 1 (1b) and the title compound was obtained. Yield 37%.
¹H NMR(DMSO-d₆, 400 MHz) δ 3.11 (4H, m), 3.79 (4H, m), 6.44 (1H, d, J = 7.4 Hz), 7.06 (1H, t, J = 7.4 Hz), 7.16 (1H, d, J = 7.8 Hz), 7.30 (1H, t, J = 7.4 Hz), 7.42 (1H, d, J = 7.8 Hz), 7.51 (1H, t, J = 7.0 Hz).

### (54c) 3-amino-4-[4-(2-chlorophenyl) piperazin-1-yl]thieno[2,3-b]pyridine-2-carboxamide

4-[4-(2-chlorophenyl)-piperazin-1-yl]-2-thioxo-1,2-dihydropyridine-3-carbonitrile which was produced in Example 54 (54b) was used in place of 4-(dimethylamino)-2-thioxo-1,2-dihydropyridine-3-carbonitrile and the reaction was performed in a similar method as described in Example 1 (1c) and the title compound was synthesized. Yield 79%.
Mp 294-298°C (decomposition);
IR (KBr) vₘₐₓ 3449, 3327, 3131, 2842, 1665, 1606, 1581, 1501, 1447, 1375, 1284, 1245, 1230, 1182, 1135, 1038, 976, 957, 826, 766, 627, 483 cm⁻¹ ;
¹H NMR(DMSO-d₆, 400 MHz) δ 2.95-3.62 (8H, br s), 6.98 (2H, s), 7.03-7.14 (4H, m), 7.25 (1H, d, J = 7.8 Hz), 7.33 (1H, t, J = 7.0 Hz), 7.42 (1H, d, J = 7.8 Hz), 8.45 (1H, d, J = 5.1 Hz);
MS (FAB) m/z: 388 [M+H]⁺;
Anal. Calcd for C₁₈H₁₈ClN₅OS: C, 55.74; H, 4.68; N, 18.06; S, 8.27. Found: C, 55.48; H, 4.75; N, 17.95; S, 8.04.

### (Example 55) tert-butyl 4-[3-amino-2-(aminocarbonyl)thieno[2,3-b]pyridin-4-yl]-1,4-diazepane-1-carboxylate (Exemplified Compound No. 3-152)

### (55a) tert-butyl 4-[(1Z)-3-amino-2-cyano-1-methyl-3-thiooxoprop-1-enyl]-1,4-diazepane-1-carboxylate]

N-Boc-homopiperazine was used in place of isobutylamine and the reaction was performed in a similar method as described in Example 5 (5a) and the title compound was synthesized.
Yellow powder
Mp 189-190°C;
IR (KBr) νₘₐₓ 3284, 3196, 2188, 1686, 1526, 1415, 1280, 1167, 884 cm⁻¹;
¹H NMR(DMSO-d₆, 400 MHz) δ 1.38 (9H, s), 1.75-1.82 (2H, m), 2.27 (3H, s), 3.29-3.52 (8H, m), 8.80 (1H, brs), 9.02 (1H, brs);
HRMS m/z calcd for C₁₅H₂₅O₂N₄S 325.1798, found 325.1715;
MS (FAB) m/z: 325 [M⁺], 269, 252, 235, 201, 191, 65, 57;
Anal. Calcd for C₁₅H₂₄N₄O₂S: C, 55.53; H, 7.46; N, 12.27; S, 9.88. Found: C, 55.40; H, 7.48; N, 17.15, S, 9.68.

### (55b) tert-butyl 4-(3-cyano-2-thioxo-1,2-dihydropyridin-4-yl)-1,4-diazepane-1-carboxylate

tert-butyl 4-[(1Z)-3-amino-2-cyano-1-methyl-3-thiooxoprop-1-enyl]-1,4-diazepane-1-carboxylate] which was produced in Example 55 (55a) was used in place of (2Z)-2-cyano-3-(isobutylamino)but-2-enethioamide and the reaction was performed in a similar method as described in Example 5 (5b), and the title compound was obtained.
White powder
Mp 203-205°C;
IR (KBr) νₘₐₓ 2974, 2205, 1692, 1625, 1537, 1478, 1246, 1152, 929, 771 cm⁻¹;
¹H NMR(DMSO-d₆, 400 MHz) δ 1.27 (4.5 H, s), 1.33 (4.5 H, s), 1.71-1.85 (2H, m), 3.31-3.38 (2H, m), 3.50-3.60 (2H, m), 3.74-3.84 (2H, m), 3.87-4.04 (2H, m), 6.42 (1H, t, J = 7.4 Hz), 7.88 (1H, d, J = 7.4 Hz), 12.44 (1H, brs);
HRMS m/z calcd for C₁₆H₂O₂N₄S 335.1542, found 335.1541;
MS (FAB) m/z: 335 [M+H]⁺, 289, 279, 233, 200, 176, 165, 93, 83;
Anal. Calcd for C₁₆H₂₂N₄O₂S: C, 57.46; H, 6.63; N, 16.75; S, 9.59. Found: C, 57.10; H, 6.24; N, 16.66, S, 9.82.

### (55c) tert-butyl 4-[3-amino-2-(aminocarbonyl)thieno[2,3-b]pyridin-4-yl]-1,4-diazepane-1-carboxylate

tert-butyl 4-(3-cyano-2-thioxo-1,2-dihydropyridin-4-yl)-1,4-diazepane-1-carboxylate which was produced in Example 55 (55b) was used in place of 4-(isobutylamino)-2-thioxo-1,2-dihydropyridine-3-carbonitrile and the reaction was performed in a similar method as described in Example 5 (5c) and the title compound was obtained.
Pale yellow powder
Mp 177-178°C;
IR (KBr) νₘₐₓ 3431, 3316, 3145, 2973, 1686, 1581, 1502, 1411, 1366, 1170, 929, 769 cm⁻¹;
¹H NMR(DMSO-d₆, 400 MHz) δ 1.40 (4.5H, s), 1.43 (4.5H, s), 1.93-2.02 (2H, m), 3.17-3.23 (4H, m), 3.47 (2H, t, J = 6.3 Hz), 3.58-3.63 (2H, m), 7.00 (2H, brs), 7.06 (1H, d, J = 5.1 Hz), 7.08 (2H, brs), 8.39 (1H, d, J = 5.1 Hz);
HRMS m/z calcd for C₁₈H₂₆O₃N₅S 392.1757, found 392.1761;
MS (FAB) m/z: 391 [M⁺], 336, 319, 289, 230, 218, 202, 190, 176, 93;
Anal. Calcd for C₁₈H₂₅N₅O₃S: C, 55.22; H, 6.44; N, 17.89; S, 8.19. Found: C, 54.90; H, 6.40; N, 17.85; S, 8.26.

### (Example 56) 3-amino-4-(4-benzyl-1,4-diazepan-1-yl)thieno[2,3-b]pyridine-2-carboxamide (Exemplified Compound No. 3-83)

### (56a) (2Z)-3-(4-benzyl-1,4-diazepan-1-yl)-2-cyanobut-2-enethioamide

1-benzylhomopiperazine was used in place of propylamine, the reaction was performed in a similar method as described in Example 4 (4a) and the title compound was obtained. Yield 88%.
¹H NMR(DMSO-d₆, 400 MHz) δ 1.86 (2H, m), 2.30 (3H, s), 2.57 (2H, t, J = 5.1 Hz), 2.67-2.73 (2H, m), 3.50-3.54 (4H, m), 3.57, (2H, s), 7.21-7.32 (5H, m), 8.18 (1H, s), 8.84 (1H, s).

### (56b) 4-(4- benzyl-1,4-diazepan-1-yl)-2-thioxo-1,2-dihydropyridine-3-carbonitrile

(2Z)-3-(4-benzyl-1,4-diazepan-1-yl)-2-cyanobut-2-enethioamide which was produced in Example 56 (56a) was used in place of (2Z)-2-cyano-3-(dimethylamino)but-2-enethioamide and the reaction was performed in a similar method as described in Example 1 (1b) and the title compound was obtained. Yield 70%.
¹H NMR(DMSO-d₆, 400 MHz) δ 1.80-4.43 (12H, m), 6.38 (1H, d, J = 7.4 Hz), 7.15-7.61 (6H, m).

### (56c) 3-amino-4-(4-benzyl-1,4-diazepan-1-yl)thieno [2,3-b]pyridine-2-carboxamide

4-(4-benzyl-1,4-diazepan-1-yl)-2-thioxo-1,2-dihydropyridine-3-carbonitrile which was produced in Example 56 (56b) was used in place of 4-(dimethylamino)-2-thioxo-1,2-dihydropyridine-3-carbonitrile and the reaction was performed in a similar method as described in Example 1 (1c) and the title compound was synthesized. Yield 75%.
Mp 194-195°C;
IR (KBr) νₘₐₓ 3429, 3306, 3142, 2937, 2828, 1674, 1646, 1583, 1501, 1452, 1368, 1344, 1262, 1157, 1108, 1026, 925, 740, 699, 483 cm⁻¹;
¹H NMR(DMSO-d₆, 400 MHz) δ 1.86 (2H, m), 2.70 (2H, m), 2.79 (2H, m), 3.29 (2H, m), 3.35 (2H, m), 3.67 (2H, s), 7.04 (2H, s), 7.06 (2H, s), 7.15 (2H, s), 7.25 (1H, m), 7.31-7.37 (4H, m), 8.365 (1H, d, J = 5.5 Hz);
MS (FAB) m/z: 382 [M+H]⁺;
Anal. Calcd for C₂₀H₂₃N₅OS: C, 62.97; H, 6.08; N, 18.36; S, 8.41. Found: C, 62.94; H, 5.75; N, 18.33; S, 8.30.

### (Example 57) 3-amino-4-(4-phenyl-1,4-diazepan-1-yl)thieno[2,3-b]pyridine-2-carboxamide (Exemplified Compound No. 3-86)

### (57a) tert-butyl 4-phenyl-1,4-diazepane-1-carboxylate

The title compound was synthesized by performing a reaction in a similar method as described in Org.Lett.,4,581-584(2002).
Brown liquid
IR (film) νₘₐₓ 2974, 1694, 1599, 1506, 1415, 1237, 1169, 930, 748, 692 cm⁻¹;
¹H NMR(CDCl₃, 400 MHz) δ 1.37 (4.5H, s), 1.44 (4.5H, s), 1.93-2.00 (2H, m), 3.19 (1H, t, J = 5.9 Hz), 3.80 (1H, t, J = 5.9 Hz), 3.51-3.57 (6H, m), 6.63-6.69 (3H, m), 7.19 (2H, t, J = 7.1 Hz);
HRMS m/z calcd for C₁H₂₄O₂N₂ 276.1835, found 276.1833;
MS (EI) m/z: 276 [M⁺], 220, 205, 175, 146, 132, 120, 94, 57;
Anal. Calcd for C₁₆H₂₄N₂O₂·0.14H₂O: C, 68.90; H, 8.77; N, 10.04. Found: C, 68.96; H, 8.75; N, 9.85.

### (57b) 1-phenyl-1,4-diazepane

4N hydrochloric acid-dioxane solution (75 mL) was added to methanol (20 mL) solution of tert-butyl 4-phenyl-1,4-diazepane-1-carboxylate (16.58 g, 60.0 mmol) which was produced in Example 57 (57a) and the mixture was stirred at room temperature for one hour. The reaction liquid was concentrated, and a sodium hydrogen carbonate aqueous solution (100 mL) was added to the obtained residual substance, and extracted with a mixed solvent (3x100 mL) of methylene chloride/2-methylene chloride propanol (4:1) and after the extracted solvent was dried over sodium sulfate, the solvent was evaporated under reduced pressure and the title compound (10.57 g, yield 100%) was obtained.
Yellow liquid
IR (film) νₘₐₓ 2931, 1598, 1506, 1394, 1245, 1035, 748, 692 cm⁻¹;
¹H NMR(CDCl₃, 500 MHz) δ 1.87-1.92 (2H, m), 2.83 (2H, t, J = 5.9 Hz), 3.03 (2H, t, J = 5.9 Hz), 3.54-3.59 (4H, m), 6.65 (1H, t, J = 8.3 Hz), 6.70 (2H, d, J = 8.3 Hz), 7.21 (2H, t, J = 8.3 Hz);
HRMS m/z calcd for C₁₁H₁₆N₂ 176.1313, found 176.1318;
MS (EI) m/z: 176 [M⁺], 146, 134, 120, 106, 94, 77, 69, 43.

### (57c) (2Z)-2-cyano-3-(4-phenyl-1,4-diazepan-1-yl)but-2-enethioamide

1-phenyl-1,4-diazepane which was produced in Example 57 (57b) was used in place of isobutylamine and the reaction was performed in a similar method as described in Example 5 (5a) and the title compound was synthesized.
Pale yellow powder
Mp 151-153°C;
IR (KBr) νₘₐₓ 3290, 2185, 1599, 1538, 1503, 1397, 1369, 1011, 873, 754 cm⁻¹;
¹H NMR(DMSO-d₆, 500 MHz) δ 1.91-1.96 (2H, m), 2.23 (3H, s), 3.50-3.55 (4H, m), 3.61-3.63 (2H, m), 3.71-3.73 (2H, m), 6.61 (1H, t, J = 7.3 Hz), 6.76 (2H, d, J = 7.3 Hz), 7.16 (2H, t, J = 7.3 Hz), 8.33 (1H, brs), 9.00 (1H, brs);
HRMS m/z calcd for C₁₆H₂₁N₄S 301.1487, found 301.1465;
MS (FAB) m/z: 300 [M⁺], 267, 242, 195, 175;
Anal. Calcd for C₁₆H₂₀N₄S: C, 63.97; H, 6.71; N, 18.65; S, 10.67. Found: C, 63.76; H, 6.47; N, 18.75, S, 10.62.

### (57d) 4-(4-phenyl-1,4-diazepan-1-yl)-2-thioxo-1,2-dihydropyridine-3-carbonitrile

(2Z)-2-cyano-3-(4-phenyl-1,4-diazepan-1-yl)but-2-enethioamide was used in place of (2Z)-2-cyano-3-(isobutylamino)but-2-enethioamide which was produced in Example 57 (57c) and the reaction was performed in a similar method as described in Example 5 (5b) and the title compound was synthesized.
Brown powder
Mp 128-132°C;
IR (KBr) νₘₐₓ 2954, 2205, 1626, 1504, 1248, 1136, 928, 750, 617 cm⁻¹;
¹H NMR(DMSO-d₆, 400 MHz) δ 1.90-1.96 (2H, m), 3.51 (2H, t, J = 5.9 Hz), 3.71 (4H, q, J = 5.9 Hz), 3.93 (2H, t, J = 5.9 Hz), 6.40 (1H, d, J = 7.4 Hz), 6.56 (1H, t, J = 7.1 Hz), 6.74 (2H, d, J = 7.1 Hz), 7.12 (2H, t, J = 7.1 Hz), 7.83 (1H, d, J = 7.4 Hz), 12.47 (1H, brs);
HRMS m/z calcd for C₁₇H₁₈N₄S 310.1252, found 310.1224;
MS (EI) m/z: 310 [M⁺], 281, 251, 204, 165, 132;
Anal. Calcd for C₁₇H₁₈N₄S: C, 65.78; H, 5.84; N, 18.05; S, 10.33. Found: C, 65.58; H, 5.51; N, 18.03, S, 10.24.

### (57e) 3-amino-4-(4-phenyl-1,4-diazepan-1-yl)thieno[2,3-b]pyridine-2-carboxamide

4-(4-phcnyl-1,4-diazepan-1-yl)-2-thioxo-1,2-dihydropyridinc-3 -carbonitrile which was produced in Example 57 (57d) was used in place of 4-(isobutylamino)-2-thioxo-1,2-dihydropyridine-3-carbonitrile and the reaction was performed in a similar method as described in Example 5 (5c) and the title compound was synthesized.
White powder
Mp 215-218°C;
IR (KBr) νₘₐₓ 3340, 3316, 3143, 1645, 1598, 1504, 1369, 1233, 939, 752, 694 cm⁻¹; ¹H NMR(DMSO-d₆, 400 MHz) δ 2.10-2.15 (2H, m), 3.16-3.19 (2H, m), 3.26-3.29 (2H, m), 3.52 (2H, t, J = 6.3 Hz), 3.75 (2H, t, J = 6.3 Hz), 6.58 (1H, t, J = 8.2 Hz), 6.74 (2H, d, J = 8.2 Hz), 6.95 (2H, brs), 7.05 (1H, d, J = 5.5 Hz), 7.05 (2H, brs), 7.14 (2H, t, J = 8.2 Hz), 8.3 6 (1H, d, J = 5.5 Hz);
HRMS m/z calcd for C₁₉H₂₁N₅OS 367.1467, found 367.1462;
MS (EI) m/z: 367 [M⁺], 335, 324, 275, 259, 242, 216, 202, 175, 146, 120;
Anal. Calcd for C₁₉H₂₁N₅OS·0.24H₂O: C, 61.38; H, 5.82; N, 18.84; S, 8.62. Found: C, 61.22; H, 5.64; N, 18.94; S, 8.49.

### (Example 58) 3-amino-4-[4-(4-methoxyphenyl)-1,4-diazepan-1-yl]thieno[2,3-b]pyridine-2-carboxamide (Exemplified Compound No. 3-124)

### (58a) tert-butyl 4-(4-methoxyphenyl)-1,4-diazepane-1-carboxylate

The title compound was synthesized by performing a reaction in a similar method as described in Org.Lett.,4,581-584(2002).
Brown liquid
IR (film) νₘₐₓ 2974, 1693, 1513, 1415, 1242, 1169, 1041, 930, 815 cm⁻¹;
¹H NMR(CDC1₃, 500 MHz) δ 1.37 (4.5H, s), 1.44 (4.5H, s), 1.92-1.99 (2H, m), 3.20 (1H, t, J = 5.9 Hz), 3.31 (1H, t, J = 5.9 Hz), 3.47-3.57 (6H, m), 3.74 (3H, s), 6.65 (2H, d, J = 9.3 Hz), 6.81 (2H, d, J = 9.3 Hz);
HRMS m/z calcd for C₁₇H₂₆O₃N₂ 306.1943, found 306.1935;
MS (EI) m/z: 306 [M⁺], 250, 235, 205, 189, 162, 150, 121, 70, 57;
Anal. Calcd for C₁₇H₂₆N₂O₃·0.36H2O: C, 65.26; H, 8.61; N, 8.95. Found: C, 64.92; H, 8.29; N, 8.87.

### (58b) 1-(4-methoxyphenyl)-1,4-diazepane

tert-butyl 4-(4-methoxyphenyl)-1,4-diazepane-1-carboxylate which was produced in Example 58 (58a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2934, 1513, 1241, 1040, 814 cm⁻¹;
¹H NMR(CDCl₃, 400 MHz) δ 1.89 (2H, quint, J = 5.1 Hz), 2.89 (2H, t, J = 5.1 Hz), 3.02 (2H, t, J = 5.1 Hz), 3.48-3.54 (4H, m), 3.75 (3H, s), 6.65 (2H, d, J = 9.0 Hz), 6.82 (2H, d, J = 9.0 Hz);
HRMS m/z calcd for C₁₂H₁₈ON₂ 206.1419, found 206.1424;
MS (EI) m/z: 206 [M⁺], 176, 164, 150, 136, 121, 109, 92, 77, 70, 43.

### (58c) (2Z)-2-cyano-3-[4-(4-methoxyphenyl)-1,4-diazepan-1-yl]but-2-enethioamide

1-(4-methoxyphenyl)-1,4-diazepane which was produced in Example 58 (58b) was used in place of isobutylamine and the reaction was performed in a similar method as described in Example 5 (5a) and the title compound was synthesized.
Pale yellow powder
Mp 157-158°C;
IR (KBr) νₘₐₓ 3284, 3154, 2179, 1512, 1361, 1243, 1037, 821 cm⁻¹;
¹H NMR(DMSO-d₆, 400 MHz) δ 1.89-1.96 (2H, m), 2.24 (3H, s), 3.44 (2H, t, J = 5.9 Hz), 3.49 (2H, t, J =5.9 Hz), 3.57-3.63 (4H, m), 3.64 (3H, s), 6.70 (2H, d, J = 9.0 Hz), 6.76 (2H, d, J = 9.0 Hz), 8.27 (1H, brs), 8.95 (1H, brs);
HRMS m/z calcd for C₁₇H₂₃ON₄S 331.1593, found 331.1589;
MS (FAB) m/z: 331 [M+H]⁺, 246, 228, 182, 63;
Anal. Calcd for C₁₇H₂₂N₄OS: C, 61.79; H, 6.71; N, 16.95; S, 9.70. Found: C, 61.51; H, 6.69; N, 16.99, S, 9.71.

### (58d) (2Z)-2-cyano-N-[(1E)-(dimethylamino) methylene]-3-[4-(4-methoxyphenyl)-1,4-diazepan-1-yl]but-2-enethioamide

(2Z)-2-cyano-3-[4-(4-methoxyphenyl)-1,4-diazepan-1-yl]but-2-enethioamide (476 mg, 1.44 mmol) which was produced in Example 58 (58c) was suspended in ethanol (15 mL) and was blended with N,N-dimethylformamide dimethylacetal (382 µL, 2.88 mmol) and the mixture was stirred at room temperature for one day. After the deposited solid was washed with ethanol, the title compound (452 mg, yield 81 %) was obtained.
Yellow powder
Mp 145-146°C;
IR (KBr) νₘₐₓ 2926, 2178, 1610, 1512, 1324, 1294, 1187, 1012, 923, 669, 514 cm⁻¹; ¹H NMR(DMSO-d₆, 400 MHz) δ 1.94-2.00 (2H, m), 2.50 (3H, s), 2.93 (3H, s), 3.14 (3H, s), 3.45-3.49 (2H, m), 3.65 (3H, s), 3.69-3.79 (6H, m), 6.73 (2H, d, J = 7.8 Hz), 6.78 (2H, d, J = 7.8 Hz), 8.46 (1H, s);
HRMS m/z calcd for C₂₀H₂₈ON₅S 386.2014, found 386.2007;
MS (FAB) m/z: 386 [M+H]⁺, 369, 352, 273, 242, 196, 165, 65, 55;
Anal. Calcd for C₂₀H₂₇N₅OS: C, 62.31; H, 7.06; N, 18.17; S, 8.32. Found: C, 62.03; H, 6.88; N, 18.02; S, 8.50.

### (58e) 3-amino-4-[4-(4-methoxyphenyl)-1,4-diazepan-1-yl]thieno[2,3-b]pyridine-2-carboxamide

N,N-dimethylformamide (2.3 mL) solution of (2Z)-2-cyano-N-[(1E)-(dimethylamino)methylene]-3-[4-(4-methoxyphenyl)-1,4-diazepan-1-yl]but-2-enethioamide (443 mg, 1.15 mmol) which was produced in Example 58 (58d) was stirred at 80°C for one hour. The reaction liquid was cooled to room temperature and was blended with 2-chloroacetamide (129 mg, 1.38 mmol) and 8M aqueous solution of sodium hydroxide (0.49 mL) and the mixture was stirred at room temperature for one hour.
Water (5 mL) was added to the reaction liquid and the resulted solid was separated by filtration and the title compound (378 mg, yield 83%) was obtained.
Pale yellow powder
Mp 206-208°C;
IR (KBr) νₘₐₓ 3446, 3328, 3168, 1578, 1511, 1370, 1240, 1037, 937, 816 cm⁻¹;
¹H NMR (DMSO-d₆, 400 MHz) δ 1.78-1.86 (2H, m), 2.89-2.95 (2H, m), 2.97-3.01 (2H, m), 3.19 (2H, t, J = 5.9 Hz), 3.37 (3H, s), 3.37-3.42 (2H, m), 6.43 (2H, d, J = 9.4 Hz), 6.51 (2H, d, J = 9.4 Hz), 6.69 (2H, brs), 6.77-6.79 (2H, m), 8.10 (1H, d, J = 5.5 Hz);
HRMS m/z calcd for C₂₀H₂₄O₂N₅S 398.1651, found 398.1635;
MS (FAB) m/z: 398 [M+H]⁺, 273, 257, 242, 226, 200, 165, 63;
Anal. Calcd for C₂₀H₂₃N₅O₂S·0.52H₂O: C, 59.04; H, 5.96; N, 17.21. Found: C, 58.73; H, 6.09; N, 17.52.

### (Example 59) 3-amino-4-[4-(4-nitrophenyl)-1,4-diazepan-1-yl]thieno[2,3-b]pyridine-2-carboxamide (Exemplified Compound No. 3-97)

### (59a) tert-butyl 4-(4-nitrophenyl)-1,4-diazepane-1-carboxylate

4-fluoro nitrobenzene (282 mg, 2 mmol) and tert-butoxycarbonyl homopiperazine (801 mg, 4 mmol) were dissolved in dimethylsulfoxide (5 mL) and the mixture was stirred at 80°C for one hour. Ethyl acetate (20 mL) and water (20 mL) was added to the reaction liquid and, after partitioning, the organic layer was washed with water (20 mLx5) and a saline solution (20 mL), and after drying over sodium sulfate, the solvent was evaporated under reduced pressure. After the obtained residue was blended with hexane and solidified, it was separated by filtration and purified and the title compound (620 mg, yield 96%) was obtained. Yellow powder
IR (KBr) νₘₐₓ 1683, 1600, 1482, 1422, 1310, 1253, 1114, 983, 824 cm⁻¹;
¹H NMR(DMSO-d₆, 400 MHz) δ 1.16 (4.5H, s), 1.27 (4.5H, s), 1.71-1.83 (2H, m), 3.22 (1H, t, J = 5.5 Hz), 3.29 (1H, t, J = 7.4 Hz), 3.50 (1H; t, J = 5.9 Hz), 3.56 (1H, t, J = 5.9 Hz), 3.62-3.65 (2H, m), 3.70-3.77 (2H, m), 6.87 (2H, d, J = 9.4 Hz), 8.00-8.04 (2H, m);
HRMS m/z calcd for C₁₆H₂₃N₃O₄ 321.1689, found 321.1682;
MS (FAB) m/z: 321 [M⁺], 266, 220, 200, 120.

### (59b) 1-(4-nitrophenyl)-1,4-diazepane

tert-butyl 4-(4-nitrophenyl)-1,4-diazepane-1-carboxylate which was produced in Example 59 (59a) was used in place of tert-butyl 4-phenyl-1,4-diazepane-1-carboxylate and the reaction was performed in a similar method as described in Example 57 (57b) and the title compound was obtained.
Yellow liquid
¹H NMR(DMSO-d₆, 400 MHz) δ 1.71-1.77 (2H, m), 2.61 (2H, t, J = 5.9 Hz), 2.83 (2H, t, J = 5.9 Hz), 3.56 (2H, t, J = 5.9 Hz), 3.63 (2H, t, J = 5.9 Hz), 6.79 (2H, d, J = 9.4 Hz), 7.99 (2H, d, J = 9.4 Hz).

### (59c) (2Z)-2-cyano-3-[4-(4-nitrophenyl)-1,4-diazepan-1-yl]but-2-enethioamide

1-(4-nitrophenyl)-1,4-diazepane which was produced in Example 59 (59b) was used in place of isobutylamine and the reaction was performed in a similar method as described in Example 5 (5a) and the title compound was synthesized. Yellow powder
IR (KBr) νₘₐₓ 3190, 2183, 1596, 1516, 1315, 1115 cm⁻¹;
¹H NMR(DMSO-d₆, 400 MHz) δ 1.91-1.96 (2H, m), 2.22 (3H, s), 3.54-3.57 (2H, m), 3.52-3.65 (2H, m), 3.69-3.72 (2H, m), 3.90-3.92 (2H, m), 6.91 (2H, d, J = 8.2 Hz), 8.03 (2H, d, J = 8.2 Hz), 8.45 (1H, brs), 9.08 (1H, brs);
HRMS m/z calcd for C₁₆H₂₀O₂N₅S 346.1337, found 346.1342;
MS (FAB) m/z: 346 [M+H]⁺, 329, 273, 200, 165.

### (59d) 4-[4-(4-nitrophenyl)-1,4-diazepan-1-yl)-2-thioxo-1,2-dihydropyridine-3-carbonitrile

(2Z)-2-cyano-3-[4-(4-nitrophenyl)-1,4-diazepan-1-yl]but-2-enethioamide which was produced in Example 59 (59c) was used in place of (2Z)-2-cyano-3-(isobutylamino)but-2-enethioamide and the reaction was performed in a similar method as described in Example 5 (5b) and the title compound was obtained.
Brown powder
IR (KBr) νₘₐₓ 2204, 1596, 1515, 1312, 1114, 927, 752 cm⁻¹;
¹H NMR(DMSO-d₆, 400 MHz) δ 1.91-1.97 (2H, m), 3.65-3.70 (2H, m), 3.75-3.78(2H, m), 3.88-3.90 (2H, m), 3.97-3.99 (2H, m), 6.43 (1H, d, J = 7.8 Hz), 6.92 (1H, t, J = 9.4 Hz), 7.86 (1H, d, J = 7.8 Hz), 8.01 (2H, d, J = 9.4 Hz);
HRMS m/z calcd for C₁₇H₁₈N₅O₂S 356.1182, found 356.1165;
MS (EI) m/z: 356 [M+H]⁺, 273, 246, 182, 120.

### (59e) 3-amino-4-[4-(4-nitrophenyl)-1,4-diazepan-1-yl]thieno[2,3-b]pyridine-2-carboxamide

4-[4-(4-nitrophenyl)-1,4-diazepan-1-yl)-2-thioxo-1,2-dihydropyridine-3-carbonitrile which was produced in Example 59 (59d) was used in place of 4-(isobutylamino)-2-thioxo-1,2-dihydropyridine-3-carbonitrile and the reaction was performed in a similar method as described in Example 5 (5c) and the title compound was obtained.
Yellow powder
Mp 244-248°C;
IR (KBr) νₘₐₓ 3439, 1646, 1596, 1509, 1312, 1114, 824, 7534 cm⁻¹;
¹H NMR(DMSO-d₆, 400 MHz) δ 2.17-2.23 (2H, m), 3.18-3.24 (2H, m), 3.31-3.36 (2H, m), 3.72 (2H, t, J = 5.9 Hz), 3.91-3.95 (2H, m), 6.93 (2H, d, J = 9.4 Hz), 7.00 (2H, brs), 7.08 (1H, d, J = 5.5 Hz), 7.12 (2H, brs), 8.07 (2H, t, J = 9.4 Hz), 8.41 (1H, d, J = 5.5 Hz);
HRMS m/z calcd for C₁₉H₂₁N₆O₃S 413.1396, found 413.1400;
MS (FAB) m/z: 413 [M+H]⁺, 338, 246, 182;
Anal. Calcd for C₁₉H₂₀N₆O₃S·0.76H₂O: C, 53.55; H, 5.09; N, 19.72; S, 7.52. Found: C, 53.85; H, 4.86; N, 19.54, S, 7.14.

### (Example 60) 3-amino-4-[4-(4-chlorophenyl)-1,4-diazepan-1-yl]thieno[2,3-b]pyridine-2-carboxamide (Exemplified Compound No. 3-94)

### (60a) tert-butyl 4-(4-chlorophenyl)-1,4-diazepane-1-carboxylate

The reaction was performed in a similar method as described in Org.Lett.,4,581-584(2002) and the title compound was synthesized.
Brown liquid
¹H NMR(DMSO-d₆, 400 MHz) δ 1.36 (4.5H, s), 1.43 (4.5H, s), 1.91-1.97 (2H, m), 3.19 (1H, t, J = 6.3 Hz), 3.30 (1H, t, J = 6.3 Hz), 3.48-3.56 (6H, m), 6.59 (2H, d, J = 9.0 Hz), 7.121 (2H, d, J = 3.5, 9.0 Hz).

### (60b) 1-(4-chlorophenyl)-1,4-diazepane

tert-butyl 4-(4-chlorophenyl)-1,4-diazepane-1-carboxylate which was produced in Example 60 (60a) was used in place of tert-butyl 4-phenyl-1,4-diazepane-1-carboxylate and the reaction was performed in a similar method as described in Example 57 (57b) and the title compound was obtained.
Yellow liquid
¹H NMR(CDCl₃, 400 MHz) δ 1.87 (2H, quint, J = 5.9 Hz), 2.80 (2H, t, J = 5.9 Hz), 3.00 (2H, t, J = 5.9 Hz), 3.49 (2H, t, J = 5.9 Hz), 3.53 (2H, t, J = 5.9 Hz), 6.58 (2H, t, J = 9.4 Hz), 7.11 (2H, d, J = 9.4 Hz).

### (60c) (2Z)-3-[4-(4-chlorophenyl)-1,4-diazepan-1-yl]-2-cyanobut-2-enethioamide

1-(4-chlorophenyl)-1,4-diazepane which was produced in Example 60 (60b) was used in place of isobutylamine and the reaction was performed in a similar method as described in Example 5 (5a) and the title compound was synthesized. Pale yellow powder
Mp 170-172°C (decomposition);
IR (KBr) νₘₐₓ 3370, 3268, 3175, 2187, 1536, 1498, 1397, 813, 510 cm⁻¹;
¹H NMR(DMSO-d₆, 400 MHz) δ 1.88-1.94 (2H, m), 2.22 (3H, s), 3.48-3.53 (4H, m), 3.58-3.60 (2H, m), 3.70-3.72 (2H, m), 6.75 (2H, d, J = 9.0 Hz), 7.14 (2H, d, J = 9.0 Hz), 8.34 (1H, brs), 8.98 (1H, brs);
HRMS m/z calcd for C₁₆H₂₀N₄ClS 335.1097, found 335.1095;
MS (FAB) m/z: 335 [M+H]⁺, 273, 246, 211, 165, 63;
Anal. Calcd for C₁₆H₁₉ClN₄S : C, 57.39; H, 5.47; N, 16.73; Cl, 10.59; S, 9.58. Found: C, 57.23; H, 5.47; N, 17.77; Cl, 10.59; S, 9.54.

### (60d) (2Z)-3-[4-(4-chlorophenyl)-1,4-diazepan-1-yl]-2-cyano-N-[(1E)-(dimethylamino)methylene]but-2-enethioamide

(2Z)-3-[4-(4-chlorophenyl)-1,4-diazepan-1-yl]-2-cyanobut-2-enethioamide which was produced in Example 60 (60c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 259-264°C;
IR (KBr) νₘₐₓ 2926, 2176, 1610, 1499, 1328, 1291, 1194, 1012, 923, 820 cm⁻¹;
¹H NMR(DMSO-d₆, 400 MHz) δ 1.94-1.97 (2H, m), 2.46 (3H, s), 2.90 (3H, s) 3.13 (3H, s), 3.54 (2H, t, J = 5.9 Hz), 3.69-3.76(4H, m), 3.79-3.81 (2H, m), 6.75 (2H, d, J = 9.0 Hz), 7.11 (2H, d, J = 9.0 Hz), 8.42 (1H, s);
HRMS m/z calcd for C₁₉H₂₅N₅ClS 390.1519, found 390.1524;
MS (FAB) m/z: 390 [M+H]⁺, 356, 318, 273, 208, 196, 180, 166, 90;
Anal. Calcd for C₁₉H₂₄ClN₅S: C, 58.42; H, 6.20; N, 17.96; Cl, 9.09; S, 8.22. Found: C, 58.41; H, 6.17; N, 17.66, Cl, 8.91; S, 8.12.

### (60e) 3-amino-4-[4-(4-chlorophenyl)-1,4-diazepan-1-yl]thieno[2,3-b]pyridine-2-carboxamide

(2Z)-3-[4-(4-chlorophenyl)-1,4-diazepan-1-yl]-2-cyano-N-[(1E)-(dimethylamino)methylene]but-2-enethioamide which was produced in Example 60 (60d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder
Mp 92-96°C;
IR (KBr) νₘₐₓ 3441, 3323, 2948, 1646, 1594, 1499, 1368, 939, 810 cm⁻¹;
¹H NMR(DMSO-d₆, 500 MHz) δ 2.11-2.16 (2H, m), 3.15-3.20 (2H, m), 3.26-3.28 (2H, m), 3.53 (2H, t, J = 5.9 Hz), 3.75 (2H, t, J = 4.4 Hz), 6.76 (2H, d, J = 8.8 Hz), 6.99 (2H, brs), 7.07 (1H, d, J = 5.4 Hz), 7.08 (2H, brs), 7.17 (2H, t, J = 8.8 Hz), 8.40 (1H, d, J = 5.4 Hz);
HRMS m/z calcd for C₁₉H₂₁N₅OClS 402.1155, found 402.1158;
MS (FAB) m/z: 402 [M+H]⁺, 385, 273, 258, 246, 211, 200, 93;
Anal. Calcd for C₁₉H₂₀ClN₅OS·0.51H₂O: C, 55.51; H, 5.15; N, 17.04; Cl, 8.62. Found: C, 55.60; H, 5.09; N, 16.84; Cl, 8.76.

### (Example 61) 3-amino-4-[4-(2-chlorophenyl)-1,4-diazepan-1-yl]thieno[2,3-b]pyridine-2-carboxamide (Exemplified Compound No. 3-92)

### (61a) tert-butyl 4-(2-chlorophenyl)-1,4-diazepane-1-carboxylate

The reaction was performed in a similar method as described in Org. Lett., 4, 581-584 (2002) and the title compound was synthesized.
Brown liquid
IR (film) νₘₐₓ 2976, 1692, 1482, 1366, 1160, 751 cm⁻¹;
¹H NMR(CDCl₃, 400 MHz) δ 1.46 (4.5H, s), 1.47 (4.5H, s), 1.97-2.04 (2H, m), 3.14-3.21 (4H, m), 3.53-3.64 (4H, m), 6.90 (1H, t, J = 7.1 Hz), 7.04 (1H, d, J = 7.1 Hz), 7.14 (1H, t, J = 7.1 Hz), 7.32 (1H, d, J = 7.1 Hz);
HRMS m/z calcd for C₁₆H₂₃N₂O₂³⁵Cl 310.1448, found 310.1472;
MS (EI) m/z: 310 [M⁺], 282, 253, 237, 209, 193, 180, 166, 154, 138, 125, 111, 70, 57.

### (61b) 1-(2-chlorophenyl)-1,4-diazepane

tert-butyl 4-(2-chlorophenyl)-1,4-diazepane-1-carboxylate which was produced in Example 61 (61a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2941, 2836, 1588, 1481, 1295, 1040, 752 cm⁻¹;
¹H NMR(CDC1₃, 400 MHz) δ 1.93 (2H, quint, J = 5.9 Hz), 2.18 (1H, brs), 3.03-3.08 (4H, m), 3.23-3.29 (4H, m), 6.87 (1H, t, J = 5.9 Hz), 7.07 (1H, d, J = 5.9 Hz), 7.14 (1H, t, J = 5.9 Hz), 7.31 (1H, d, J = 5.9 Hz);
HRMS m/z calcd for C₁₁H₁₅N₂Cl 210.0924, found 210.0916;
MS (EI) m/z: 210 [M⁺], 180, 175, 168, 154, 146, 128, 111, 92, 77, 64.

### (61c) (2Z)-3-[4-(2-chlorophenyl)-1,4-diazepan-1-yl]-2-cyanobut-2-enethioamide

1-(2-chlorophenyl)-1,4-diazepane which was produced in Example 61 (61b) was used in place of isobutylamine and the reaction was performed in a similar method as described in Example 5 (5a) and the title compound was synthesized.
Pale yellow powder
Mp 123-124°C;
IR (KBr) νₘₐₓ 3367, 3265, 3186, 2184, 1611, 1525, 1402, 1289, 1041, 766, 673 cm⁻¹; ¹H NMR(CDCl₃, 400 MHz) δ 2.12-2.17 (2H, m), 2.69 (3H, s), 3.23 (2H, t, J = 5.9 Hz), 3.34-3.37 (2H, m), 3.91 (2H, t, J = 5.9 Hz), 3.97-4.00 (2H, m), 6.70 (2H, brs), 6.97 (1H, dt, J = 1.2, 8.2 Hz), 7.05 (1H, dd, J = 1.2, 8.2 Hz), 7.19 (1H, dt, J = 1.2, 8.2 Hz), 7.35 (1H, dd, J = 1.2, 8.2 Hz);
HRMS m/z calcd for C₁₆H₂₀N₄ClS 335.1097, found 335.1098;
MS (FAB) m/z: 335 [M+H]⁺, 273, 246, 211;
Anal. Calcd for C₁₆H₁₉ClN₄S·0.64H₂O: C, 55.48; H, 5.90; N, 16.17; Cl, 10.23; S, 9.26. Found: C, 55.69; H, 5.81; N, 16.19; Cl, 9.97; S, 9.35.

### (61d) 3-amino-4-[4-(2-chlorophenyl)-1,4-diazepan-1-yl]thieno[2,3-b]pyridine-2-carboxamide

(2Z)-3-[4-(2-chlorophenyl)-1,4-diazepan-1-yl]-2-cyanobut-2-enethioamide (64 mg, 0.19 mmol) which was produced in Example 61 (61c) and N,N-dimethylformamide dimethylacetal (54 mg, 0.45 mmol) were dissolved in ethanol (2 mL) and after stirring at room temperature for one hour, the solvent was evaporated. The residue was dissolved in N,N-dimethylformamide (0.3 mL) and the mixture was stirred at 80°C for 15 minutes. The reaction mixture was cooled to room temperature and 8N aqueous solution of sodium hydroxide (0.08 mL) and 2-chloroacetamide (21 mg, 0.22 mmol) were added. After stirring at room temperature one hour, the reaction mixture was partitioned with water and ethyl acetate and the organic solvent was concentrated under reduced pressure after drying over sodium sulfate. A solid was separated by filtration after the residue was solidified with hydrous ethanol and the title compound was obtained 76 mg (yield 31%).
Pale yellow powder
Mp 206-210°C;
IR (KBr) νₘₐₓ 3435, 3318, 3168, 1645, 1584, 1501, 1369, 1041, 937, 761 cm⁻¹;
¹H NMR(DMSO-d₆, 400 MHz) δ 2.05-2.11 (2H, m), 3.28-3.31 (2H, m), 3.41-3.48(6H, m), 6.97-7.01 (1H, m), 7.07 (2H, brs), 7.11 (2H, brs), 7.11-7.12 (1H, m), 7.25-7.27 (2H, m), 7.40 (1H, d, J = 7.1 Hz), 8.40 (1H, d, J = 5.5 Hz);
HRMS m/z calcd for C₁₉H₂₁N₅OClS 402.1155, found 402.1153;
MS (FAB) m/z: 402 [M+H]⁺, 246, 189, 182;
Anal. Calcd for C₁₉H₂₀ClN₅OS·0.22H₂O: C, 56.23; H, 5.08; N, 17.25; Cl, 8.73; S, 7.90. Found: C, 56.18; H, 5.11; N, 17.08; Cl, 8.66; S, 7.62.

### (Example 62) 3-amino-4-[4-(4-cyanophenyl)-1,4-diazepan-1-yl]thieno[2,3-b]pyridine-2-carboxamide (Exemplified Compound No. 3-98)

### (62a) tert-butyl 4-(4-cyanophenyl)-1,4-diazepane-1-carboxylate

The reaction was performed in a similar method as described in Example 59 (59a) using 4-cyano fluorobenzene (242 mg, 2 mmol) and tert-butoxycarbonyl homopiperazine (801 mg, 4 mmol) and the title compound (364 mg, yield 60%) was obtained.
Brown liquid
IR (film) νₘₐₓ 2975, 2214, 1691, 1606, 1521, 1417, 1365, 1240, 1178, 929, 819, 544 cm⁻¹;
¹H NMR(DMSO-d₆, 400 MHz) δ 1.16 (4.5H, s), 1.28 (4.5H, s), 1.68-1.85 (2H, m), 3.17 (1H, t, J = 5.9 Hz), 3.25 (1H, t, J = 5.5 Hz), 3.44-3.68 (6H, m), 6.81 (2H, d, J = 9.4 Hz), 7.48 (2H, dd, J = 3.5, 9.4 Hz);
HRMS m/z calcd for C₁₇H₂₃N₃O₂ 301.1790, found 301.1784;
MS (FAB) m/z: 302 [M+H]⁺, 301, 246, 228, 200, 120.

### (62b) 1-(4-cyanophenyl)-1,4-diazepane

tert-butyl 4-(4-cyanophenyl)-1,4-diazepane-1-carboxylate which was produced in Example 62 (62a) was used in place of tert-butyl 4-phenyl-1,4-diazepane-1-carboxylate and the reaction was performed in a similar method as described in Example 57 (57b) and the title compound was obtained.
Brown liquid
IR (film) νₘₐₓ 2935, 2211, 1606, 1521, 1404, 1178, 817, 544 cm⁻¹;
¹H NMR(CDCl₃, 400 MHz) δ 1.87 (2H, quint, J = 5.9 Hz), 2.81 (2H, t, J = 5.9 Hz), 3.01 (2H, t, J = 5.9 Hz), 3.55 (2H, t, J = 5.9 Hz), 3.61 (2H, t, J = 5.9 Hz), 6.65 (2H, t, J = 9.0 Hz), 8.43 (2H, d, J = 9.0 Hz);
HRMS m/z calcd for C₁₂H₁₅N₃ 201.1266, found 201.1268;
MS (EI) m/z: 201 [M⁺], 171, 159, 145, 131, 116, 102.

### (62c) (2Z)-2-cyano-3-[4-(4-cyanophenyl)-1,4-diazepan-1-yl]but-2-enethioamide

1-(4-cyanophenyl)-1,4-diazepane which was produced in Example 62 (62b) was used in place of isobutylamine and the reaction was performed in a similar method as described in Example 5 (5a) and the title compound was synthesized.
Pale yellow powder
Mp 169-171°C (decomposition);
IR (KBr) νₘₐₓ 3290, 2214, 217, 1604, 1519, 1408, 1179, 819, 543 cm⁻¹;
¹H NMR(DMSO-d₆, 400 MHz) δ 1.88-1.94 (2H, m), 2.21 (3H, s), 3.52 (2H, t, J = 5.5 Hz), 3.58-3.63 (4H, m), 3.82 (2H, t, J = 5.5 Hz), 6.85 (2H, d, J = 9.0 Hz), 7.51 (2H, d, J = 9.0 Hz), 8.38 (1H, brs), 9.02 (1H, brs);
HRMS m/z calcd for C₁₇H₂₀N₅S 326.1440, found 326.1436;
MS (FAB) m/z: 326 [M+H]⁺, 202, 171, 120.

### (62d) (2Z)-2-cyano-3-[4-(4-cyanophenyl)-1,4-diazepan-1-yl]-N-[(1E)-(dimethylamino)methylene]but-2-enethioamide

(2Z)-3-[4-(4-cyanophenyl)-1,4-diazepan-1-yl]-2-cyanobut-2-enethioamide which was produced in Example 62 (62c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 120-126°C;
IR (KBr) νₘₐₓ 2926, 2211, 2180, 1606, 1519, 1178, 821, 546 cm⁻¹;
¹H NMR(DMSO-d₆, 400 MHz) δ 1.94-1.97 (2H, m), 2.44 (3H, s), 2.90 (3H, s) 3.14 (3H, s), 3.64 (2H, t, J = 5.5 Hz), 3.71-3.77(4H, m), 3.90 (2H, t, J = 5.5 Hz), 6.85 (2H, d, J = 9.0 Hz), 7.48 (2H, d, J = 9.0 Hz), 8.41 (1H, s);
HRMS m/z calcd for C₂₀H₂₅N₆S 381.1861, found 381.1856;
MS (FAB) m/z: 381 [M+H]⁺, 336, 257, 230, 202, 180, 90, 65;
Anal. Calcd for C₂₀H₂₄N₆S·0.62H₂O: C, 61.33; H, 6.50; N, 21.46; S, 8.19. Found: C, 61.36; H, 6.35; N, 21.25, S, 8.11.

### (62e) 3-amino-4-[4-(4-cyanophenyl)-1,4-diazepan-1-yl]thieno[2,3-b]pyridine-2-carboxamide

(2Z)-2-cyano-3-[4-(4-cyanophenyl)-1,4-diazepan-1-yl]-N-[(1E)-(dimethylamino)methylene]but-2-enethioamide which was produced in Example 62 (62d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Yellow powder
Mp 151-153°C;
IR (KBr) νₘₐₓ 3439, 3327, 2211, 1605, 1519, 1366, 1178, 938, 818, 544 cm⁻¹;
¹H NMR(DMSO-d₆, 400 MHz) δ 2.13-2.18 (2H, m), 3.17-3.19 (2H, m), 3.61 (2H, t, J = 6.3 Hz), 3.84 (2H, t, J = 5.1 Hz), 6.86 (2H, d, J = 9.0 Hz), 6.96 (2H, brs), 7.05 (1H, d, J = 5.5 Hz), 7.07 (2H, brs), 7.52 (2H, t, J = 9.0 Hz), 8.37 (1H, d, J = 5.5 Hz); HRMS m/z calcd for C₂₀H₂₁N₆OS 393.1497, found 393.1501;
MS (FAB) m/z: 393 [M+H]⁺, 273, 246;
Anal. Calcd for C₂₀H₂₀N₆OS·0.94H₂O: C, 58.67; H, 5.39; N, 20.53. Found: C, 58.99; H, 5.51; N, 20.22.

### (Example 63) 3-amino-4-[4-(4-trifluoromethylphenyl)-1,4-diazepan-1-yl]thieno[2,3-b]pyridine-2-carboxamide (Exemplified Compound No. 3-121)

### (63a) tert-butyl 4-(4-trifluoromethylphenyl)-1,4-diazepane-1-carboxylate

The reaction was performed in a similar method as described in Org. Lett., 4, 581-584 (2002) and the title compound was synthesized.
White powder
IR (KBr) νₘₐₓ 2974, 1673, 1422, 1332, 1197, 1100, 987, 829 cm⁻¹;
¹H NMR(CDCl₃, 400 MHz) δ 1.33 (4.5H, s), 1.41 (4.5H, s), 1.93-1.99 (2H, m), 3.20 (1H, t, J = 6.3 Hz), 3.31 (1H, t, J = 5.9 Hz), 3.54-3.62 (6H, m), 6.67 (2H, d, J = 9.0 Hz), 7.40 (2H, d, J = 9.0 Hz);
HRMS m/z calcd for C₁₇H₂₃N₂O₂F₃ 344.1711, found 344.1718;
MS (FAB) m/z: 345 [M+H]⁺, 344, 289, 243, 214, 174, 120, 57;
Anal. Calcd for C₁₇H₂₃F₃N₂O₂: C, 59.29; H, 6.73; N, 8.13. Found: C, 59.38; H, 6.45; N, 7.98.

### (63b) 1-(4-trifluoromethylphenyl)-1,4-diazepane

tert-butyl 4-(4-trifluoromethylphenyl)-1,4-diazepane-1-carboxylate which was produced in Example 63 (63a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2936, 1616, 1531, 1402, 1330, 1197, 1106, 818 cm⁻¹;
¹H NMR(CDCl₃, 400 MHz) δ 1.88 (2H, quint, J = 6.3 Hz), 2.81 (2H, t, J = 5.9 Hz), 3.01 (2H, t, J = 5.1 Hz), 3.54-3.61 (4H, m), 6.67 (2H, t, J = 9.0 Hz), 8.39 (2H, d, J = 9.0 Hz);
HRMS m/z calcd for C₁₂H₁₅N₂F₃ 244.1187, found 244.1182;
MS (EI) m/z: 244 [M⁺], 225, 214, 202, 188, 174, 159, 145, 69, 43.

### (63c) (2Z)-2-cyano-3-[4-(4-trifluoromethylphenyl)-1,4-diazepan-1-yl]but-2-enethioamide

1-(4-trifluoromethylphenyl)-1,4-diazepane which was produced in Example 63 (63b) was used in place of isobutylamine and the reaction was performed in a similar method as described in Example 5 (5a) and the title compound was synthesized.
Pale yellow powder
Mp 266-269°C (decomposition);
IR (KBr) νₘₐₓ 3373, 3267, 3171, 2185, 1614, 1527, 1330, 1104, 822 cm⁻¹;
¹H NMR(DMSO-d₆, 400 MHz) δ 1.91-1.95 (2H, m), 2.23 (3H, s), 3.51 (2H, t, J = 5.5 Hz), 3.59-3.63 (4H, m), 3.79 (2H, t, J = 5.1 Hz), 6.87 (2H, d, J = 8.6 Hz), 7.42 (2H, d, J = 8.6 Hz), 8.37 (1H, brs), 9.01 (1H, brs);
HRMS m/z calcd for C₁₇H₂₀N₄F₃S 369.1361, found 369.1359;
MS (FAB) m/z: 369 [M+H]⁺, 335, 245, 227, 200, 166, 63;
Anal. Calcd for C₁₇H₁₉F₃N₄S·0.10H₂O: C, 55.15; H, 5.23; N, 15.13; F, 15.39. Found: C, 54.92; H, 5.26; N, 15.25, F, 15.59.

### (63d) (2Z)-2-cyano-N-[(lE)-(dimethylamino)methylene]-3-[4-(4-trifluoromethylphenyl)-1,4-diazepan-1-yl]but-2-enethioamide

(2Z)-3-[4-(4-trifluoromethylphenyl)-1,4-diazepan-1-yl]-2-cyanobut-2-enethioamide which was produced in Example 63 (63c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 251-253°C;
IR (KBr) νₘₐₓ 2927, 2177, 1614, 1400, 1332, 1201, 1107, 925, 832 cm⁻¹;
¹H NMR(DMSO-d₆, 400 MHz) δ 1.95-1.99 (2H, m), 2.47 (3H, s), 2.89 (3H, s), 3.12 (3H, s), 3.63 (2H, t, J = 6.3 Hz), 3.70-3.79(4H, m), 3.89 (2H, t, J = 5.1 Hz), 6.90 (2H, d, J = 9.0 Hz), 7.42 (2H, d, J = 9.0 Hz), 8.44 (1H, s);
HRMS m/z calcd for C₂₀H₂₅N₆F₃S 424.1783, found 424.1783;
MS (FAB) m/z: 424 [M+H]⁺, 390, 379, 352, 245, 227, 200, 188, 90, 73;
Anal. Calcd for C₂₀H₂₄F₃N₅S: C, 56.72; H, 5.71; N, 16.54; F, 13.46; S, 7.57. Found: C, 56.46; H, 5.60; N, 16.36, F, 13.32; S, 7.49.

### (63e) 3-amino-4-[4-(4-trifluoromethylphenyl)-1,4-diazepan-1-yl]thieno[2,3-b]pyridine-2-carboxamide

(2Z)-2-cyano-N-[(1E)-(dimethylamino)methylene]-3-[4-(4-trifluoromethylphenyl)-1,4-diazepan-1-yl]but-2-enethioamide which was produced in Example 63 (63d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder
Mp 204-208°C;
IR (KBr) νₘₐₓ 3326, 1614, 1502, 1330, 1199, 1104, 939, 817 cm⁻¹;
¹H NMR(DMSO-d₆, 400 MHz) δ 2.15-2.19 (2H, m), 3.17-3.22 (2H, m), 3.30-3.32 (2H, m), 3.62 (2H, t, J = 5.5 Hz), 3.84-3.86 (2H, m), 6.91 (2H, d, J = 9.0 Hz), 7.00 (2H, brs), 7.09 (1H, d, J = 5.5 Hz), 7.11 (2H, brs), 7.47 (2H, t, J = 9.0 Hz), 8.41 (1H, d, J = 5.5 Hz);
HRMS m/z calcd for C₂₀H₂₁ON₅F₃S 436.1419, found 436.1418;
MS (FAB) m/z: 436 [M+H]⁺, 419, 246, 214;
Anal. Calcd for C₂₀H₂₀F₃N₅OS·0.98H₂O: C, 53.01; H, 4.88; N, 15.46; F, 12.58; S,7.08. Found: C, 52.79; H, 4.61; N, 15.21; F, 12.97; S, 7.12.

### (Example 64) 3-amino-4-{4-[4-(methylthio)phenyl]-1,4-diazepan-1-yl}thieno[2,3-b]pyridine-2-carboxamide (Exemplified Compound No. 3-131)

### (64a) tert-butyl 4-[4-(methylthio)phenyl]-1,4-diazepane-1-carboxylate

The reaction was performed in a similar method as described in Org. Lett., 4, 581-584 (2002) and the title compound was synthesized.
Brown liquid
IR (film) νₘₐₓ 2974, 1693, 1595, 1503, 1416, 1365, 1237, 1168, 1125, 930, 810 cm⁻¹; ¹H NMR(CDCl₃, 400 MHz) δ 1.35 (4.5H, s), 1.42 (4.5H, s), 1.91-1.96 (2H, m), 2.39 (3H, s), 3.19 (1H, t, J = 5.9 Hz), 3.30 (1H, t, J = 5.9 Hz), 3.49-3.54 (6H, m), 6.61 (2H, d, J = 8.6 Hz), 7.22 (2H, d, J = 8.6 Hz);
HRMS m/z calcd for C₁₇H₂₆O₂N₂S 322.1715, found 322.1714;
MS (FAB) m/z: 322 [M⁺], 266, 221, 178, 57;
Anal. Calcd for C₁₇H₂₆N₂O₂S.0.28H₂O: C, 62.34; H, 8.17; N, 8.55. Found: C, 62.38; H, 8.41; N, 8.38.

### (64b) 1-[4-(methylthio)phenyl]-1,4-diazepane

tert-butyl 4-[4-(methylthio)phenyl]-1,4-diazepane-1-carboxylate which was produced in Example 64 (64a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2919, 1595, 1503, 1395, 1195, 809 cm⁻¹;
¹H NMR(CDCl₃, 400 MHz) δ 1.88 (2H, quint, J = 5.9 Hz), 2.40 (3H, s), 2.82 (2H, t, J = 5.9 Hz), 3.01 (2H, t, J = 5.1 Hz), 3.47-3.57 (4H, m), 6.64 (2H, d, J = 8.6 Hz), 8.25 (2H, d, J = 8.6 Hz);
HRMS m/z calcd for C₁₂H₁₈N₂S 222.1191, found 222.1178;
MS (EI) m/z: 222 [M⁺], 192, 180, 166, 151, 137, 108, 91, 77, 70, 56, 43.

### (64c) (2Z)-2-cyano-3-{4-[4-(methylthio)phenyl]-1,4-diazepan-1-yl}but-2-enethioamide

1-(4-(methylsulphanylphenyl)-1,4-diazepane which was produced in Example 64 (64b) was used in place of isobutylamine and the reaction was performed in a similar method as described in Example 5 (5a) and the title compound was synthesized.
Pale yellow powder
Mp 159-160°C (decomposition);
IR (KBr) νₘₐₓ 3341, 3154, 2178, 1594, 1501, 1392, 1244, 1011, 879, 811 cm⁻¹;
¹H NMR(DMSO-d₆, 400 MHz) δ 1.90-1.94 (2H, m), 2.23 (3H, s), 2.36 (3H, s), 3.49-3.54 (4H, m), 3.59-3.61 (2H, m), 3.70-3.73 (2H, m), 6.75 (2H, d, J = 8.6 Hz), 7.16 (2H, d, J = 8.6 Hz), 8.35 (1H, brs), 9.01 (1H, brs);
HRMS m/z calcd for C₁₇H₂₂N₄S₂ 346.1286, found 346.1245;
MS (EI) m/z: 346 [M⁺], 272, 247, 222, 205, 192, 178, 166, 151, 137, 123, 96, 68, 59, 42;
Anal. Calcd for C₁₇H₂₂N₄S₂·0.08H₂O: C, 56.68; H, 6.42; N, 16.10; S, 18.43. Found: C, 58.47; H, 6.32; N, 15.98, S, 18.39.

### (64d) (2Z)-2-cyano-N-[(1E)-(dimethylamino)methylene]-3-{4-[4-(methylthio)phenyl]-1,4-diazepan-1-yl}but-2-enethioamide

(2Z)-3-{4-[4-(methylthio)phenyl]-1,4-diazepan-1-yl}-2-cyanobut-2-enethioamide which was produced in Example 64 (64c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 133-136°C;
IR (KBr) νₘₐₓ 2921, 2177, 1610, 1501, 1396, 1325, 1291, 1195, 1011, 923, 815 cm⁻¹; ¹H NMR(DMSO-d₆, 400 MHz) δ 1.93-1.98 (2H, m), 2.35 (3H, s), 2.49 (3H, s), 2.92 (3H, s), 3.14 (3H, s), 3.53 (2H, t, J = 5.9 Hz), 3.69-3.81 (6H, m), 6.75 (2H, d, J = 9.0 Hz), 7.14 (2H, d, J = 9.0 Hz), 8.46 (1H, s);
HRMS m/z calcd for C₂₀H₂₈N₅S₂ 402.1786, found 402.1770;
MS (FAB) m/z: 402 [M+H]⁺, 368, 330, 221, 192, 178, 166;
Anal. Calcd for C₂₀H₂₇N₅S₂·0.2H₂O: C, 59.28; H, 6.82; N, 17.28; S, 15.83. Found: C, 59.53; H, 6.80; N, 17.00; S, 15.96.

### (64e) 3-amino-4-{4-[4-(methylthio)phenyl]-1,4-diazepan-1-yl}thieno[2,3-b]pyidine-2-carboxamide

(2Z)-2-cyano-N-[(1E)-(dimethylamino)methylene]-3- {4-[4-(methylthio)phenyl]-1,4-diazepan-1-yl}but-2-enethioamide which was produced in Example 64 (64d) was used in place of (2Z)-2-cyano-N-[(lE)-(dimethylamino)methylene]-3-[4-(4-methoxyphenyl)-1,4-diazepan-1-yl]but-2-enethioamide and the reaction was performed in a similar method as described in 58 embodiment (58e) and the title compound was obtained.
Pale yellow powder
Mp 186-188°C;
IR (KBr) νₘₐₓ 3433, 3326, 3163, 1657, 1592, 1500, 1367, 1232, 940, 81 cm⁻¹; ¹H NMR(DMSO-d₆, 400 MHz) δ 2.09-2.14 (2H, m), 2.36 (3H, s), 3.16-3.18 (2H, m), 3.25-3.28 (2H, m), 3.52 (2H, t, J = 6.3 Hz), 3.74 (2H, t, J = 4.7 Hz), 6.73 (2H, d, J = 9.0 Hz), 6.94 (2H, brs), 7.05 (1H, d, J = 5.1 Hz), 7.06 (2H, brs), 7.17 (2H, t, J = 9.0 Hz), 8.37 (1H, d, J = 5.1 Hz);
HRMS m/z calcd for C₂₀H₂₄ON₅S₂ 414.1422, found 414.1414;
MS (FAB) m/z: 414 [M+H]⁺, 413, 397, 275, 230, 218, 192, 178, 166;
Anal. Calcd for C₂₀H₂₃N₅OS₂: C, 58.08; H, 5.61; N, 16.93; S, 15.51. Found: C, 57.91; H, 5.66; N, 16.68; S, 15.42.

### (Example 65) 3-amino-4-[4-(3-toluyl)-1,4-diazepan-1-yl]thieno[2,3-b]pyridine-2-carboxamide (Exemplified Compound No. 3-110)

### (65a) tert-butyl 4-(3-toluyl)-1,4-diazepane-1-carboxylate

The reaction was performed in a similar method as described in Org.Lett.,4,581-584(2002) and the title compound was synthesized.
Brown liquid
IR (film) νₘₐₓ 2974, 1695, 1602, 1498, 1415, 1175, 930, 692 cm⁻¹;
¹H NMR(CDCl₃, 400 MHz) δ 1.36 (4.5H, s), 1.44 (4.5H, s), 1.92-1.98 (2H, m), 2.28 (3H, s), 3.19 (1H, t, J = 6.3 Hz), 3.29 (1H, t, J = 6.3 Hz), 3.49-3.54 (6H, m), 6.46-6.48 (3H, m), 7.06 (1H, t, J = 7.1 Hz);
HRMS m/z calcd for C₁₇H₂₆O₂N₂ 290.1994, found 290.1981;
MS (FAB) m/z: 290 [M⁺], 235, 217, 189, 120, 91, 70, 57;
Anal. Calcd for C₁₇H₂₆N₂O₂·0.16H2O: C, 69.62; H, 9.05; N, 9.55. Found: C, 69.71; H, 9.36; N, 9.28.

### (65b) 1-(3-toluyl)-1,4-diazepane

tert-butyl 4-(3-toluyl)-1,4-diazepane-1-carboxylate which was produced in Example 65 (65a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2928, 1601, 1498, 1363, 1178, 765, 692 cm⁻¹;
¹H NMR(CDCl₃, 400 MHz) δ 1.88 (2H, quint, J = 4.3 Hz), 2.29 (3H, s), 2.82 (2H, t, J = 5.9 Hz), 3.02 (2H, t, J = 5.1 Hz), 3.52-3.57 (4H, m), 6.47-6.51 (3H, m), 7.09 (2H, dd, J = 1.9, 9.4 Hz);
HRMS m/z calcd for C₁₂H₁₈N₂ 190.1470, found 190.1456;
MS (EI) m/z: 190 [M⁺], 160, 148, 134, 122, 105, 91, 77, 65, 43.

### (65c) (2Z)-2-cyano-3-[4-(3-toluyl)-1,4-diazepan-1-yl]but-2-enethioamide

1-(3-toluyl)-1,4-diazepane which was produced in Example 65 (65b) was used in place of isobutylamine and the reaction was performed in a similar method as described in Example 5 (5a) and the title compound was synthesized.
Pale yellow powder
Mp 144-148°C;
IR (KBr) νₘₐₓ 3151, 2188, 1601, 1542, 1345, 1174, 912, 766 cm⁻¹;
¹H NMR(DMSO-d₆, 400 MHz) δ 1.91-1.95 (2H, m), 2.22 (3H, s), 2.23 (3H, s), 3.48-3.54 (4H, m), 3.60-3.63 (2H, m), 3.68-3.71 (2H, m), 6.44 (1H, d, J = 7.0 Hz), 6.55-6.57 (2H, m), 7.03 (1H, t, J = 7.0 Hz), 8.34 (1H, brs), 9.01 (1H, brs);
HRMS m/z calcd for C₁₇H₂₃N₄S 315.1644, found 315.1645;
MS (FAB) m/z: 315 [M+H]⁺, 281, 256, 246, 173;
Anal. Calcd for C₁₇H₂₂N₄S: C, 64.93; H, 7.05; N, 17.82; S, 10.20. Found: C, 64.65; H, 7.05; N, 17.73, S, 10.13.

### (65d) (2Z)-2-cyano-N-[(1E)-(dimethylamino)methylene]-3-[4-(3-toluyl)-1,4-diazepan-1-yl]but-2-enethioamide

(2Z)-3-[4-(3-toluyl)-1,4-diazepan-1-yl]-2-cyanobut-2-enethioamide which was produced in Example 65 (65c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 256-260°C (decomposition);
IR (KBr) νₘₐₓ 2178, 1608, 1394, 1292, 1181, 1118, 773, 692 cm⁻¹;
¹H NMR(DMSO-d₆, 400 MHz) δ 1.94-1.99 (2H, m), 2.21 (3H, s), 2.49 (3H, s), 2.92 (3H, s), 3.14 (3H, s), 3.54 (2H, t, J = 5.9 Hz), 3.68-3.77(6H, m), 6.43 (1H, d, J = 7.8 Hz), 6.55-6.58 (2H, m), 7.02 (1H, t, J = 7.8 Hz), 8.45 (1H, s);
HRMS m/z calcd for C₂₀H₂₈N₅S 370.2065, found 370.2057;
MS (FAB) m/z: 370 [M+H]⁺, 354, 336, 325, 298, 281, 235, 208, 196, 180, 173, 160, 134,115,90,58;
Anal. Calcd for C₂₀H₂₇NₛS·0.06H₂O: C, 64.82; H, 7.38; N, 18.90; S, 8.65. Found: C, 64.85; H, 7.22; N, 18.61; S, 8.95.

### (65e) 3-amino-4-[4-(3-toluyl)-1,4-diazepan-1-yl]thieno[2,3-b]pyridine-2-carboxamide

(2Z)-2-cyano-N-[(1E)-(dimethylamino)methylene]-3-[4-(3-toluyl)-1,4-diazepan-1-yl]but-2-enethioamide which was produced in Example 65 (65d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder
Mp 209-212°C;
IR (KBr) νₘₐₓ 3327, 3169, 2830, 1637, 1579, 1498, 1373, 1234, 1182, 942, 767 cm⁻¹; ¹HNMR(DMSO-d₆, 400 MHz) δ 2.10-2.15 (2H, m), 2.22 (3H, s), 3.14-3.18 (2H, m), 3.26-3.28 (2H, m), 3.52 (2H, t, J = 6.3 Hz), 3.73 (2H, t, J = 4.7 Hz), 6.41 (1H, d, J = 7.8 Hz), 6.53-6.56 (2H, m), 6.97 (2H, brs), 7.01 (1H, t, J = 7.8 Hz), 7.05 (1H, d, J = 5.5 Hz), 7.06 (2H, brs), 8.37 (1H, d, J = 5.5 Hz);
HRMS m/z calcd for C₂₀H₂₄ON₅S 382.1702, found 382.1699;
MS (FAB) m/z: 382 [M+H]⁺, 365, 248, 230, 218, 202, 176;
Anal. Calcd for C₂₀H₂₃N₅OS·0.08H₂O: C, 62.73; H, 6.10; N, 18.29; S, 8.37. Found: C, 62.56; H, 6.08; N, 18.14; S, 8.32.

### (Example 66) 3-amino-4-{4-[4-(methylsulfinyl)phenyl]-1,4-diazepan-1-yl}thieno[2,3-b]pyridine-2-carboxamide (Exemplified Compound No. 3-132)

3-amino-4-{4-[4-(methylthio)phenyl]-1,4-diazepan-1-yl}thieno [2,3-b]pyridine-2-carboxamide (57 mg, 0.14 mmol) which was produced in Example 64 (64e) was dissolved in methanol (5 mL), and was blended with an aqueous solution (1 mL) of sodium periodate (33 mg, 0.15 mmol) and heated under reflux for one hour. Water (10 mL) was added to the reaction liquid and after stirring for two hours, generated powder was separated by filtration and the title compound (53 mg, yield 89%) was obtained.
White powder
Mp 153-157°C;
IR (KBr) νₘₐₓ 3439, 3324, 3182, 1646, 1592, 1505, 1367, 1093, 1037, 939, 814 cm⁻¹;
¹H NMR(DMSO-d₆, 400 MHz) δ 2.13-2.17 (2H, m), 2.67 (3H, s), 3.18-3.22 (2H, m), 3.29-3.31 (2H, m), 3.61 (2H, t, J = 6.3 Hz), 3.84 (2H, t, J = 6.3 Hz), 6.93 (2H, d, J = 9.0 Hz), 6.94 (2H, brs), 7.09 (1H, d, J = 5.1 Hz), 7.09 (2H, brs), 7.49 (2H, t, J = 9.0 Hz), 8.40 (1H, d, J = 5.1 Hz);
HRMS m/z calcd for C₂₀H₂₄O₂N₅S₂ 430.1371, found 430.1386;
MS (FAB) m/z: 430 [M+H]⁺, 412, 395, 242, 230, 204, 166, 65;
Anal. Calcd for C₂₀H₂₃N₅O₂S₂·2.1H₂O: C, 51.40; H, 5.87; N, 14.98; S, 13.72. Found: C, 51.61; H, 5.87; N, 15.00; S, 13.52.

### (Example 67) 3-amino-4-[4-(2-methoxyphenyl)-1,4-diazepan-1-yl]thieno[2,3-b]pyridine-2-carboxamide (Exemplified Compound No. 3-122)

### (67a) tert-butyl 4-(2-methoxyphenyl)-1,4-diazepane-1-carboxylate

The reaction was performed in a similar method as described in Org.Lett., 4,581-584(2002) and the title compound was synthesized.
Brown liquid
IR (film) vₘₐₓ 2975, 1694, 1503, 1415, 1242, 1165, 1029, 745 cm⁻¹;
¹H NMR(CDCl₃, 400 MHz) δ 1.14-1.46 (9H, m), 1.89-2.00 (2H, m), 3.18-3.31 (4H, m), 3.48-3.62 (4H, m), 3.83 (3H, s), 6.81-6.93 (4H, m);
HRMS m/z calcd for C₁₇H₂₆O₃N₂ 306.1943, found 306.1972;
MS (EI) m/z: 306 [M⁺], 249, 233, 205, 188, 176, 162, 150, 134, 120, 57.

### (67b) 1-(2-methoxyphenyl)-1,4-diazepane

tert-butyl 4-(2-methoxyphenyl)-1,4-diazepane-1-carboxylate which was produced in Example 67 (67a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2938, 1593, 1502, 1242, 1028, 744 cm⁻¹;
¹H NMR(CDCl₃,400 MHz) δ 1.96 (2H, quint, J = 5.9 Hz), 3.05 (2H, t, J = 5.5 Hz), 3.11 (2H, t, J = 5.5 Hz), 3.30-3.34 (4H, m), 3.84 (3H, s), 6.83-6.96 (4H, m);
HRMS m/z calcd for C₁₂H₁₈ON₂ 206.1419, found 206.1417;
MS (EI) m/z: 206 [M⁺], 176, 164, 150, 136, 120, 109, 91, 77, 43.

### (67c) (2Z)-2-cyano-3-[4-(2-methoxyphenyl)-1,4-diazepan-1-yl]but-2-enethioamide

1-(2-methoxyphenyl)-1,4-diazepane which was produced in Example 67 (67b) was used in place of isobutylamine and the reaction was performed in a similar method as described in Example 5 (5a) and the title compound was synthesized. Yellow amorphous
IR (KBr) νₘₐₓ 3287, 3171, 2918, 2184, 1607, 1533, 1452, 1238, 1024, 751 cm⁻¹;
¹H NMR(DMSO-d₆, 400 MHz) δ 1.93-1.98 (2H, m), 2.33 (3H, s), 3.14-3.17 (2H, m), 3.25-3.37 (2H, m), 3.60-3.66 (4H, m), 3.75 (3H, s), 6.79-6.90 (4H, m), 8.23 (1H, brs), 8.91 (1H, brs);
HRMS m/z calcd for C₁₇H₂₃ON₄S 331.1593, found 331.1587;
MS (FAB) m/z: 331 [M+H]⁺, 315, 273, 200, 165, 63.

### (67d) 3-amino-4-[4-(2-methoxyphenyl)-1,4-diazepan-1-yl]thieno[2,3-b]pyridine-2-carboxamide

(2Z)-2-cyano-3-[4-(2-methoxyphenyl)-1,4-diazepan-1-yl]but-2-enethioamide which was produced in Example 67 (67c) was used in place of (2Z)-3-[4-(2-chlorophenyl)-1,4-diazepan-1-yl]-2-cyanobut-2-enethioamide and the reaction was performed in a similar method as described in Example 61 (61d) and the title compound was obtained.
Pale yellow powder
Mp 179-184°C;
IR (KBr) νₘₐₓ 3440, 3314, 3141, 1581, 1500, 1371, 1235, 937, 751 cm⁻¹;
¹H NMR(DMSO-d₆, 400 MHz) δ 2.04-2.10 (2H, m), 3.25-3.40 (8H, m), 3.75 (3H, s), 6.80-6.93 (4H, m), 7.05 (2H, brs), 7.08 (2H, brs), 7.11 (1H, d, J = 5.5 Hz), 8.39 (1H, d, J = 5.5 Hz);
HRMS m/z calcd for C₂₀H₂₄O₂N₅S 398.1651, found 398.1653;
MS (FAB) m/z: 398 [M+H]⁺, 273, 246, 200, 63;
Anal. Calcd for C₂₀H₂₃N₅O₂S.0.14H₂O: C, 60.05; H, 5.87; N, 17.51; S, 8.02. Found: C, 59.87; H, 5.87; N, 17.43; S, 7.85.

### (Example 68) 3-amino-4-[4-(3-methoxyphenyl)-1,4-diazepan-1-yl]thieno[2,3-b]pyridine-2-carboxamide (Exemplified Compound No. 3-123)

### (68a) tert-butyl 4-(3-methoxyphenyl)-1,4-diazepane-1-carboxylate

The reaction was performed in a similar method as described in Org. Lett., 4, 581-584 (2002) and the title compound was synthesized.
Brown liquid
IR (film) νₘₐₓ 2973, 1694, 1612, 1500, 1416, 1165, 930, 753 cm⁻¹;
¹H NMR(CDCl₃, 400 MHz) δ 1.37 (4.5H, s), 1.44 (4.5H, s), 1.93-2.00 (2H, m), 3.19 (1H, t, J = 6.3 Hz), 3.28-3.30 (1H, m), 3.49-3.55 (6H, m), 3.77 (3H, s), 6.23-6.25 (2H, m), 6.32 (1H, d, J = 7.0 Hz), 7.11 (1H, t, J = 7.0 Hz);
HRMS m/z calcd for C₁₇H₂₆O₃N₂ 306.1943, found 306.1937;
MS (EI) m/z: 306 [M⁺], 250, 235, 205, 188, 176, 162, 150, 121, 70, 57;
Anal. Calcd for C₁₇H₂₆N₂O₃·0.38H2O: C, 65.18; H, 8.61; N, 8.94. Found: C, 65.22; H, 8.59; N, 8.79.

### (68b) 1-(3-methoxyphenyl)-1,4-diazepane

tert-butyl 4-(3-methoxyphenyl)-1,4-diazepane-1-carboxylate which was produced in Example 68 (68a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2935, 1611, 1500, 1166, 1054, 923, 752, 688 cm⁻¹;
¹H NMR(CDCl₃, 400 MHz) δ 1.88 (2H, quint, J = 5.9 Hz), 2.82 (2H, t, J = 5.9 Hz), 3.01 (2H, t, J = 5.9 Hz), 3.51-3.57 (4H, m), 3.78 (3H, s), 6.21-6.24 (2H, m), 6.32 (1H, dd, J =1.9, 9.4 Hz), 7.11 (1H, t, J = 9.4 Hz);
HRMS m/z calcd for C₁₂H₁₈ON₂ 206.1420, found 206.1403;
MS (EI) m/z: 206 [M⁺], 164, 150, 138, 121, 70, 56.

### (68c) (2Z)-2-cyano-3-[4-(3-methoxyphenyl)-1,4-diazepan-1-yl]but-2-enethioamide

1-(3-methoxyphenyl)-1,4-diazepane which was produced in Example 68 (68b) was used in place of isobutylamine and the reaction was performed in a similar method as described in Example 5 (5a) and the title compound was synthesized.
Pale yellow powder
Mp 129-131°C;
IR (KBr) νₘₐₓ 3349, 3154, 2939, 2187, 1611, 1541, 1344, 1167, 1058, 914, 749 cm⁻¹; ¹H NMR(DMSO-d₆, 400 MHz) δ 1.91-1.94 (2H, m), 2.23 (3H, s), 3.49-3.54 (4H, m), 3.59-3.62 (2H, m), 3.69 (3H, s), 3.69-3.73 (2H, m), 6.21 (1H, d, J = 9.0 Hz), 6.25 (1H, s), 6.35 (1H, d, J = 9.0 Hz), 7.05 (1H, t, J = 9.0 Hz), 8.35 (1H, brs), 9.02 (1H, brs);
HRMS m/z calcd for C₁₇H₂₃ON₄S 331.1592, found 331.1575;
MS (FAB) m/z: 331 [M+H]⁺, 246, 200, 165, 63;
Anal. Calcd for C₁₇H₂₂N₄OS: C, 61.79; H, 6.71; N, 16.95; S, 9.70. Found: C, 61.72; H, 6.66; N, 16.85, S, 9.44.

### (68d) 4-[4-(3-methoxyphenyl)-1,4-diazepan-1-yl]-2-thioxo-1,2-dihydropyridine-3-carbonitrile

(2Z)-2-cyano-3-[4-(3-methoxyphenyl)-1,4-diazepan-1-yl]but-2-enethioamide (476 mg, 1.44 mmol) which was produced in Example 68 (68c) and N,N-dimethylformamide dimethylacetal (344 mg, 2.88 mmol) were mixed with ethanol (15 mL) and the mixture was stirred at room temperature for 18 hours. The crystal which was deposited was separated by filtration and the title compound was obtained 490 mg (yield 27%).
Pale yellow powder
Mp 224-227°C (decomposition);
IR (KBr) νₘₐₓ 2955, 2208, 1608, 1499, 1256, 1166, 1052, 929, 756, 687 cm⁻¹;
¹H NMR(DMSO-d₆, 500 MHz) δ 1.90-1.95 (2H, m), 3.51 (2H, t, J = 5.4 Hz), 3.69 (3H, s), 3.71-3.73 (4H, m), 3.95 (2H, t, J = 5.4 Hz), 6.20 (1H, d, J = 7.8 Hz), 6.26 (1H, s), 6.37 (1H, t, J = 7.8 Hz), 6.43 (1H, d, J = 7.8 Hz), 7.05 (1H, t, J = 7.8 Hz), 7.37 (1H, d, J = 7.8 Hz), 12.51 (1H, brs);
HRMS m/z calcd for C₁₈H₂₁ON₄S 341.1436, found 341.1445;
MS (FAB) m/z: 341 [M+H]⁺, 273, 246, 200, 165, 63;
Anal. Calcd for C₁₈H₂₀N₄OS·0.12H₂O: C, 63.10; H, 5.95; N, 16.35; S, 9.36. Found: C, 62.84; H, 5.97; N, 16.35, S, 9.36.

### (68e) 3-amino-4-[4-(3-methoxyphenyl)-1,4-diazepan-1-yl]thieno[2,3-b]pyridine-2-carboxamide

4-[4-(3-methoxyphenyl)-1,4-diazepan-1-yl]-2-thioxo-1,2-dihydropyridine-3-carbonitrile which was produced in Example 68 (68d) was used in place of 4-(isobutylamino)-2-thioxo-1,2-dihydropyridine-3-carbonitrile and the reaction was performed in a similar method as described in Example 5 (5c) and the title compound was obtained.
Pale yellow powder
Mp 193-195°C;
IR (KBr) νₘₐₓ 3426, 3319, 3145, 1611, 1499, 1372, 1228, 1167, 1054, 943, 822 cm⁻¹; ¹H NMR(DMSO-d₆, 400 MHz) δ 2.11-2.16 (2H, m), 3.16-3.21 (2H, m), 3.26-3.30 (2H, m), 3.53 (2H, t, J = 6.7 Hz), 3.70 (3H, s), 3.75 (2H, t, J = 4.7 Hz), 6.22 (1H, dd, J = 2.4, 8.2 Hz), 6.26 (1H, t, J = 2.4 Hz), 6.37 (1H, dd, J = 2.4, 8.2 Hz), 7.00 (2H, brs), 7.04-7.08 (2H, m), 7.09 (2H, brs), 8.40 (1H, d, J = 5.5 Hz);
HRMS m/z calcd for C₂₀H₂₄O₂N₅S 398.1651, found 398.1639;
MS (FAB) m/z: 398 [M+H]⁺, 273, 246, 200, 63;
Anal. Calcd for C₂₀H₂₃N₅O₂S·0.16H₂O: C, 60.00; H, 5.87; N, 17.49; S, 8.01. Found: C, 59.86; H, 5.67; N, 17.29; S, 7.83.

### (Example 69) 3-amino-4-[4-(4-fluoro-3-methylphenyl)-1,4-diazepan-1-yl]thieno[2,3-b]pyridine-2-carboxamide (Exemplified Compound No. 3-118)

### (69a) tert-butyl 4-(4-fluoro-3-methylphenyl)-1,4-diazepane-1-carboxylate

The reaction was performed in a similar method as described in Org. Lett., 4, 581-584 (2002) and the title compound was synthesized.
Brown liquid
IR (film) νₘₐₓ 2975, 1694, 1509, 1416, 1366, 1232, 1170, 931, 762 cm⁻¹;
¹H NMR(CDCl₃, 400 MHz) δ 1.34 (4.5H, s), 1.42 (4.5H, s), 1.91-1.95 (2H, m), 2.20 (3H, s), 3.18 (1H, t, J = 5.9 Hz), 3.29 (1H, t, J = 5.9 Hz), 3.44-3.55 (6H, m), 6.37-6.44 (2H, m), 6.81 (1H, t, J = 8.6 Hz);
HRMS m/z calcd for C₁₇H₂₅O₂N₂F 308.1900, found 308.1892;
MS (EI) m/z: 308 [M⁺], 253, 251, 207, 205, 164, 152, 138, 123, 109, 57;
Anal. Calcd for C₁₇H₂₅FN₂O₂: C, 66.21; H, 8.17; N, 9.08; F, 6.16. Found: C, 66.50; H, 7.29; N, 8.88; F, 6.18.

### (69b) 1-(4-fluoro-3-methylphenyl)-1,4-diazepane

tert-butyl 4-(4-fluoro-3-methylphenyl)-1,4-diazepane-1-carboxylate which was produced in Example 69 (69a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2929, 1615, 1509, 1228, 839, 797, 761 cm⁻¹;
¹H NMR(CDCl₃, 400 MHz) δ 1.88 (2H, quint, J = 6.3 Hz), 2.23 (3H, s), 2.83 (2H, t, J = 5.9 Hz), 3.02 (2H, t, J = 5.5 Hz), 3.48-3.54 (4H, m), 6.42-6.48 (2H, m), 6.85 (1H, t, J = 9.0 Hz);
HRMS m/z calcd for C₁₂H₁₇N₂F 208.1575, found 208.1353;
MS (EI) m/z: 208 [M⁺], 178, 166, 152, 138, 123, 109, 43;

### (69c) (2Z)-2-cyano-3-[4-(4-fluoro-3-methylphenyl)-1,4-diazepan-1-yl]but-2-enethioamide

1-(4-fluoro-3-methylphenyl)-1,4-diazepane which was produced in Example 69 (69b) was used in place of isobutylamine and the reaction was performed in a similar method as described in Example 5 (5a) and the title compound was synthesized.
Pale yellow powder
Mp 146-150°C (decomposition);
IR (KBr) νₘₐₓ 3328, 3152, 2189, 1627, 1543, 1508, 1389, 1220, 1056, 917, 868, 801 cm⁻¹;
¹H NMR(DMSO-d₆, 400 MHz) δ 1.88-1.96 (2H, m), 2.18 (3H, s), 2.24 (3H, s), 3.48-3.53 (4H, m), 3.60-3.62 (2H, m), 3.66-3.69 (2H, m), 6.54-6.60 (1H, m), 6.64-6.67 (1H, m), 6.92 (1H, t, J = 9.0 Hz), 8.35 (1H, brs), 9.01 (1H, brs);
HRMS m/z calcd for C₁₇H₂₂N₄FS 333.1536, found 333.1534;
MS (FAB) m/z: 333 [M+H]⁺, 299, 274, 207, 191, 178, 164, 123, 65, 51;
Anal. Calcd for C₁₇H₂₁FN₄S: C, 61.42; H, 6.37; N, 16.85; S, 9.65. Found: C, 61.18; H, 6.29; N, 16.71, S, 9.74.

### (69d) (2Z)-2-cyano-N-[(1E)-(dimethylamino)methylene]-3-[4-(4-fluoro-3-methylphenyl)-1,4-diazepan-1-yl]but-2-enethioamide

(2Z)-3-[4-(4-fluoro-3-methylphenyl)-1,4-diazepan-1-yl]-2-cyanobut-2-enethioamide which was produced in Example 69 (69c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 148-150°C;
IR (KBr) νₘₐₓ 2923, 2178, 1609, 1509, 1324, 1292, 1118, 1015, 512 cm⁻¹;
¹H NMR(DMSO-d₆, 400 MHz) δ 1.94-1.99 (2H, m), 2.15 (3H, s), 2.47 (3H, s), 2.92 (3H, s), 3.14 (3H, s), 3.51 (2H, t, J = 5.9 Hz), 3.72 (2H, t, J = 5.4 Hz), 3.73-3.77 (4H, m), 6.55-6.59 (1H, m), 6.64-6.66 (1H, m), 6.80 (1H, t, J = 9.3 Hz), 8.45 (1H, s); HRMS m/z calcd for C₂₀H₂₇N₅FS 388.1971, found 388.1962;
MS (FAB) m/z: 388 [M+H]⁺, 354, 299, 273, 165, 120, 65, 51;
Anal. Calcd for C₂₀H₂₆FN₅S: C, 61.99; H, 6.76; N, 18.07; F, 4.90; S, 8.27. Found: C, 61.75; H, 6.57; N, 17.81; F, 4.77; S, 8.31.

### (69e) 3-amino-4-[4-(4-fluoro-3-methylphenyl)-1,4-diazepan-1-yl]thieno[2,3-b]pyridine-2-carboxamide

(2Z)-2-cyano-N-[(1E)-(dimethylamino)methylene]-3-[4-(4-fluoro-3-methylphenyl)-1,4-diazepan-1-yl]but-2-enethioamide which was produced in Example 69 (69d) was used in place of (2Z)-2-cyano-N-[(lE)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
White powder
Mp 194-197°C;
IR (KBr) νₘₐₓ 3414, 3326, 3172, 1637, 1579, 1507, 1374, 1209, 943 cm⁻¹;
¹H NMR(DMSO-d₆, 400 MHz) δ 2.10-2.18 (2H, m), 2.18 (3H, s), 3.15-3.21 (2H, m), 3.27-3.29 (2H, m), 3.50 (2H, t, J = 6.3 Hz), 3.71 (2H, t, J = 4,7 Hz), 6.52-6.56 (1H, m), 6.62-6.64 (1H, m), 6.91 (1H, t, J = 9.0 Hz), 6.98 (2H, brs), 7.06 (1H, d, J = 5.5 Hz), 7.07 (2H, brs), 8.38 (1H, d, J = 5.5 Hz);
HRMS m/z calcd for C₂₀H₂₃ON₅FS 400.1607, found 400.1632;
MS (FAB) m/z: 400 [M+H]⁺, 383, 275, 236, 209;
Anal. Calcd for C₂₀H₂₂FN₅OS·0.14H₂O: C, 59.75; H, 5.59; N, 17.42; F, 4.73; S, 7.98. Found: C, 59.82; H, 5.58; N, 17.42; F, 4.45; S, 7.71.

### (Example 70) 3-amino-4-[4-(2-toluyl)-1,4-diazepan-1-yl]thieno[2,3-b]pyridine-2-carboxamide (Exemplified Compound No. 3-109)

### (70a) tert-butyl 4-(2-toluyl)-1,4-diazepane-1-carboxylate

The reaction was performed in a similar method as described in J. Org. Chem. 68, 452-459 (2003) and the title compound was synthesized.
Brown liquid
IR (film) νₘₐₓ 2975, 1695, 1492, 1413, 1159, 762, 725 cm⁻¹;
¹HNMR(CDCl₃, 400 MHz) δ 1.47-1.48 (9H, m), 1.87-1.97 (2H, m), 2.29 (3H, s), 3.00-3.10 (4H, m), 3.52-3.60 (4H, m), 6.92 (1H, t, J = 7.8 Hz), 7.01 (1H, d, J = 7.8 Hz), 7.08-7.14 (2H, m);
HRMS m/z calcd for C₁₇H₂₆O₂N₂ 290.1994, found 290.1977;
MS (FAB) m/z: 290 [M⁺], 249, 233, 189, 166, 146, 130, 95, 51.

### (70b) 1-(2-toluyl)-1,4-diazepane

tert-butyl 4-(2-toluyl)-1,4-diazepane-1-carboxylate which was produced in Example 70 (70a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2938, 2831, 1598, 1492, 1458, 1213, 1163, 1114, 759, 724 cm⁻¹;
¹H NMR(CDCl₃, 400 MHz) δ 1.89 (2H, quint, J = 5.9 Hz), 2.32 (3H, s), 3.02 (2H, t, J = 4.3 Hz), 3.07 (2H, t, J = 5.9 Hz), 3.12-3.17 (4H, m), 6.94 (1H, t, J = 7.8 Hz), 7.07 (1H, d, J = 7.8 Hz), 7.12-7.17 (2H, m);
HRMS m/z calcd for C₁₂H₁₈N₂ 190.1470, found 190.1443;
MS (EI) m/z: 190 [M⁺], 160, 148, 134, 118, 105, 91, 77, 65, 43.

### (70c) (2Z)-2-cyano-3-[4-(2-toluyl)-1,4-diazepan-1-yl]but-2-enethioamide

1-(2-toluyl)-1,4-diazepane which was produced in Example 70 (70b) was used in place of isobutylamine and the reaction was performed in a similar method as described in Example 5 (5a) and the title compound was synthesized.
Pale yellow powder
Mp 99-100°C;
IR (KBr) νₘₐₓ 3286, 3173, 2184, 1599, 1521, 1410, 1294, 1220, 881, 765 cm⁻¹;
¹H NMR(DMSO-d₆, 500 MHz) δ 2.00-2.04 (2H, m), 2.24 (3H, s), 2.36 (3H, s), 3.01 (2H, t, J = 5.4 Hz), 3.16-3.17 (2H, m), 3.66 (2H, t, J = 5.4 Hz), 3.70-3.72 (2H, m), 6.95 (1H, t, J = 6.8 Hz), 7.07 (1H, d, J = 7.3 Hz), 7.12-7.17 (2H, m), 8.27 (1H, brs), 8.93 (1H, brs);
HRMS m/z calcd for C₁₇H₂₃N₄S 315.1644, found 315.1643;
MS (FAB) m/z: 315 [M+H]⁺, 281, 256, 189, 173, 65, 39;
Anal. Calcd for C₁₇H₂₂N₄S·0.56H₂O: C, 62.92; H, 7.18; N, 17.26; S, 9.88. Found: C, 62.65; H, 6.88; N, 17.11, S, 9.13.

### (70d) 3-amino-4-[4-(2-toluyl)-1,4-diazepan-1-yl]thieno[2,3-b]pyridine-2-carboxamide

(2Z)-2-cyano-3-[4-(2-toluyl)-1,4-diazepan-1-yl]but-2-enethioamide which was produced in Example 70 (70c) was used in place of (2Z)-3-[4-(2-chlorophenyl)-1,4-diazepan-1-yl]-2-cyanobut-2-enethioamide and the reaction was performed in a similar method as described in Example 61 (61d) and the title compound was obtained.
Pale yellow powder
Mp 206-209°C (decomposition);
IR (KBr) νₘₐₓ 3427, 3308, 3142, 1583, 1493, 1374, 1228, 1053, 944, 767 cm⁻¹;
¹H NMR(DMSO-d₆, 400 MHz) δ 1.97-2.04 (2H, m), 2.23 (3H, s), 3.11 (2H, t, J = 5.9 Hz), 3.22-3.25 (2H, m), 3.38-3.42 (2H, m), 6.84-6.88 (1H, m), 7.00-7.10 (7H, m), 8.34 (1H, d, J = 5.5 Hz);
HRMS m/z calcd for C₂₀H₂₄ON₅S 382.1702, found 382.1701;
MS (FAB) m/z: 382 [M+H]⁺, 246, 185, 107;
Anal. Calcd for C₂₀H₂₃N₅OS: C, 62.97; H, 6.08; N, 18.36. Found: C, 62.79; H, 6.27; N, 18.20.

### (Example 71) 3-amino-4-[4-(3-fluoro-4-methylphcnyl)-1,4-diazepan-1-yl]thieno[2,3-b]pyridine-2-carboxamide (Exemplified Compound No. 3-117)

### (71a) tert-butyl 4-(3-fluoro-4-methylphenyl)-1,4-diazepane-1-carboxylate

The reaction was performed in a similar method as described in Org. Lett., 4, 581-584 (2002) and the title compound was synthesized.
Brown liquid
IR (film) νₘₐₓ 2975, 1695, 1634, 1518, 1416, 1366, 1246, 1171, 1124, 930 cm⁻¹;
¹H NMR(CDCl₃, 400 MHz) δ 1.38 (4.5H, s), 1.45 (4.5H, s), 1.93-1.99 (2H, m), 2.14 (3H, s), 3.20 (1H, t, J = 5.9 Hz), 3.30 (1H, t, J = 5.9 Hz), 3.45-3.55 (6H, m), 6.33-6.36 (2H, m), 6.96 (1H, t, J = 8.6 Hz);
HRMS m/z calcd for C₁₇H₂₅O₂N₂F 308.1900, found 308.1877;
MS (EI) m/z: 308 [M⁺], 253, 207, 178, 164, 152, 138, 123, 109, 57.

### (71b) 1-(3-fluoro-4-methylphenyl)-1,4-diazepane

tert-butyl 4-(3-fluoro-4-methylphenyl)-1,4-diazepane-1-carboxylate which was produced in Example 71 (71a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2929, 1634, 1518, 1459, 1119, 927, 822 cm⁻¹;
¹H NMR(CDCl₃, 400 MHz) δ 1.88 (2H, quint, J = 5.9 Hz), 2.14 (3H, s), 2.82 (2H, t, J = 5.9 Hz), 3.01 (2H, t, J = 5.9 Hz), 3.50 (2H, t, J = 5.9 Hz), 5.53 (2H, t, J = 5.9 Hz), 6.34-6.38 (2H, m), 6.97 (1H, t, J = 9.0 Hz);
HRMS m/z calcd for C₁₂H₁₇N₂F 208.1576, found 208.1360;
MS (EI) m/z: 208 [M⁺], 178, 166, 152, 138, 123, 109, 44.

### (71c) (2Z)-2-cyano-3-[4-(3-fluoro-4-methylphenyl)-1,4-diazepan-1-yl]but-2-enethioamide

1-(3-fluoro-4-methylphenyl)-1,4-diazepane which was produced in Example 71 (71b) was used in place of isobutylamine and the reaction was performed in a similar method as described in Example 5 (5a) and the title compound was synthesized.
Yellow powder
Mp 155-157°C (decomposition);
IR (KBr) νₘₐₓ 3163, 2184, 1632, 1516, 1350, 1173, 1120, 872 cm⁻¹;
¹H NMR(DMSO-d₆, 400 MHz) δ 1.90-1.94 (2H, m), 2.08 (3H, s), 2.25 (3H, s), 3.51-3.53 (4H, m), 3.59-3.61 (2H, m), 3.70-3.72 (2H, m), 6.50 (1H, dd, J = 2.4, 8.8 Hz), 6.56 (1H, dd, J = 2.4, 13.8 Hz), 7.02 (1H, t, J = 8.8 Hz), 8.35 (1H, brs), 9.01 (1H, brs);
HRMS m/z calcd for C₁₇H₂₁N₄FSNa 355.1369, found 355.1354;
MS (ESI) m/z: 355.14 [M+Na]⁺;
Anal. Calcd for C₁₇H₂₁FN₄S: C, 61.42; H, 6.37; N, 16.85; F, 5.71. Found: C, 61.07; H, 6.21; N, 16.66, F, 6.06.

### (71d) (2Z)-2-cyano-N-[(1E)-(dimethylamino)methylene]-3-[4-(3-fluoro-4-methylphenyl)-1,4-diazepan-1-yl]but-2-enethioamide

(2Z)-3-[4-(3-fluoro-4-methylphenyl)-1,4-diazepan-1-yl]-2-cyanobut-2-enethioamide which was produced in Example 71 (71c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 254-256°C;
IR (KBr) vₘₐₓ 2925, 2177, 1608, 1517, 1397, 1291, 1120, 1013, 906, 512 cm⁻¹;
¹H NMR(DMSO-d₆, 500 MHz) δ 1.94-1.98 (2H, m), 2.07 (3H, s), 2.49 (3H, s), 2.93 (3H, s), 3.15 (3H, s), 3.53 (2H, t, J = 5.9 Hz), 3.71 (2H, t, J = 5.4 Hz), 3.77-3.81 (4H, m), 6.51 (1H, dd, J = 2.4, 8.3 Hz), 6.56 (1H, dd, J = 2.4, 13.7 Hz), 7.01 (1H, t, J = 8.3 Hz), 8.46 (1H, s);
HRMS m/z calcd for C₂₀H₂₇N₅FS 388.1971, found 388.1986;
MS (FAB) m/z: 388 [M+H]⁺, 354, 273, 242, 209, 166;
Anal. Calcd for C₂₀H₂₆FN₅S: C, 61.99; H, 6.76; N, 18.07; F, 4.90; S, 8.27. Found: C, 61.73; H, 6.87; N, 18.08; F, 4.92; S, 8.29.

### (71e) 3-amino-4-[4-(3-fluoro-4-methylphenyl)-1,4-diazepan-1-yl]thieno[2,3-b]pyridine-2-carboxamide

(2Z)-2-cyano-N-[(1E)-(dimethylamino)methylene]-3-[4-(3-fluoro-4-methylphenyl)-1,4-diazepan-1-yl]but-2-enethioamide which was produced in Example 71 (71d) was used in place of (2Z)-2-cyano-N-[(lE)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder
Mp 79-83°C;
IR (KBr) νₘₐₓ 3440, 3323, 3177, 2926, 1633, 1578, 1517, 1368, 1118, 943, 824 cm⁻¹; ¹H NMR(DMSO-d₆, 400 MHz) δ 2.07-2.15 (2H, m), 2.09 (3H, s), 3.15-3.20 (2H, m), 3.23-3.27 (2H, m), 3.51 (2H, t, J = 6.3 Hz), 3.73 (2H, t, J = 4,7 Hz), 6.47-6.54 (2H, m), 6.97 (2H, brs), 7.02 (1H, t, J = 8.7 Hz), 7.06 (1H, d, J = 5.5 Hz), 7.08 (2H, brs), 8.38 (1H, d, J = 5.5 Hz);
HRMS m/z calcd for C₂₀H₂₃ON₅FS 400.1608, found 400.1559;
MS (FAB) m/z: 400 [M+H]⁺, 278, 246, 185, 83, 57;
Anal. Calcd for C₂₀H₂₂FN₅OS·0.56H₂O: C, 58.65; H, 5.69; N, 17.10; F, 4.64. Found: C, 58.93; H, 6.04; N, 17.04; F, 4.89.

### (Example 72) 3-amino-4-[4-(2,3-dihydro-1,4-benzodioxin-6-yl)-1,4-diazepan-1-yl]thieno[2,3-b]pyridine-2-carboxamide (Exemplified Compound No. 3-88)

### (72a) tert-butyl 4-(2,3-dihydro-1,4-benzodioxin-6-yl)-1,4-diazepane-1-carboxylate

The reaction was performed in a similar method as described in Org.Lett.,4,581-584(2002) and the title compound was synthesized.
Brown liquid
IR (film) νₘₐₓ 2974, 1692, 1511, 1416, 1284, 1170, 1072, 931 cm⁻¹;
¹H NMR(CDCl₃, 400 MHz) δ 1.39 (4.5H, s), 1.45 (4.5H, s), 1.92-1.99 (2H, m), 3.20 (1H, t, J = 6.3 Hz), 3.30 (1H, t, J = 5.9 Hz), 3.43-3.49 (4H, m), 3.53-3.55 (2H, m), 4.17-4.18 (2H, m), 4.22-4.23 (2H, m), 6.18-6.22 (2H, m), 6.72 (1H, d, J = 9.8 Hz); HRMS m/z calcd for C₁₈H₂₇O₄N₂ 335.1971, found 335.1974;
MS (FAB) m/z: 334 [M⁺], 278, 246, 235, 189, 145, 139, 83, 57;
Anal. Calcd for C₁₈H₂₆N₃O₄·0.26H₂O: C, 63.76; H, 7.88; N, 8.27. Found: C, 63.75; H, 7.66; N, 8.02.

### (72b) 1-(2,3-dihydro-1,4-benzodioxin-6-yl)-1,4-diazepane

tert-butyl 4-(2,3-dihydro-1,4-benzodioxin-6-yl)-1,4-diazepane-1-carboxylate which was produced in Example 72 (72a) was used in place of tert-butyl 4-phenyl-1,4-diazepane-1-carboxylate and the reaction was performed in a similar method as described in Example 57 (57b) and the title compound was obtained.
Yellow liquid
IR (film) νₘₐₓ 2929, 1626, 1510, 1284, 1071, 822, 789 cm⁻¹;
¹H NMR(CDCl₃, 400 MHz) δ 1.87 (2H, quint, J = 6.3 Hz), 2.82 (2H, t, J = 5.9 Hz), 3.00 (2H, t, J = 5.5 Hz), 3.45-3.50 (4H, m), 4.17-4.19 (2H, m), 4.22-4.24 (2H, m), 6.19-6.22 (2H, m), 6.72 (1H, d, J = 9.4 Hz);
HRMS m/z calcd for C₁₃H₁₈O₂N₂ 234.1368, found 234.1373;
MS (EI) m/z: 234 [M⁺], 204, 192, 178, 166, 149, 136, 117, 79, 56, 43.

### (72c) (2Z)-2-cyano-3-[4-(2,3-dihydro-1,4-benzodioxin-6-yl)-1,4-diazepan-1-yl]but-2-enethioamide

1-(2,3-dihydro-1,4-benzodioxin-6-yl)-1,4-diazepane which was produced in Example 72 (72b) was used in place of isobutylamine and the reaction was performed in a similar method as described in Example 5 (5a) and the title compound was synthesized.
Yellow powder
Mp 114-116°C;
IR (KBr) νₘₐₓ 3314, 3155, 2185, 1542, 1510, 1286, 1211, 1068, 869 cm⁻¹;
¹H NMR(DMSO-d₆, 400 MHz) δ 1.88-1.84 (2H, m), 2.25 (3H, s), 3.40-3.45 (2H, m), 3.46-3.52 (2H, m), 3.59 (4H, brs), 4.10-4.12 (2H, m), 4.16-4.18 (2H, m), 6.24-6.27 (2H, m), 6.65 (1H, d, J = 9.5 Hz), 8.30 (1H, brs), 8.97 (1H, brs);
HRMS m/z calcd for C₁₈H₂₃O₂N₄S 359.1542, found 359.1540;
MS (FAB) m/z: 359 [M+H]⁺, 338, 273, 226, 182, 165, 120, 63;
Anal. Calcd for C₁₈H₂₂N₄O₂S: C, 59.30; H, 6.27; N, 15.37. Found: C, 59.04; H, 6.18; N, 15.71.

### (72d) (2Z)-2-cyano-3-[4-(2,3-dihydro-1,4-benzodioxin-6-yl)-1,4-diazepan-1-yl]-N-[(1E)-(dimethylamino)methylene]but-2-enethioamide

(2Z)-2-cyano-3-[4-(2,3-dihydro-1,4-benzodioxin-6-yl)-1,4-diazepan-1-yl]but-2-enethioamide which was produced in Example 72 (72c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder
Mp 127-129°C;
IR (KBr) νₘₐₓ 3429, 2925, 2179, 1610, 1410, 1293, 1209, 1069, 513 cm⁻¹;
¹H NMR(DMSO-d₆, 400 MHz) δ 1.92-1.98 (2H, m), 2.50 (3H, s), 2.95 (3H, s), 3.15 (3H, s), 3.45 (2H, t, J = 5.9 Hz), 3.67-3.72 (4H, m), 3.75-3.77 (2H, m), 4.11-4.13 (2H, m), 4.16-4.18 (2H, m), 6.26-6.29 (2H, m), 6.66 (1H, d, J = 9.4 Hz), 8.48 (1H, s); HRMS m/z calcd for C₂₁H₂₈O₂N₅S 414.1964, found 414.1974;
MS (FAB) m/z: 413 [M+H]⁺, 380, 342, 273, 235, 178, 65, 39;
Anal. Calcd for C₂₁H₂₇N₅O₂S: C, 60.99; H, 6.58; N, 16.94; S, 7.75. Found: C, 60.86; H, 6.47; N, 16.79; S, 7.62.

### (72e) 3-amino-4-[4-(2,3-dihydro-1,4-benzodioxin-6-yl)-1,4-diazepan-1-yl]thieno[2,3-b]pyridine-2-carboxamide

(2Z)-2-cyano-3-[4-(2,3-dihydro-1,4-benzodioxin-6-yl)-1,4-diazepan-1-yl]-N-[(1E)-(dimethylamino)methylene]but-2-enethioamide which was produced in Example 72 (72d) was used in place of (2Z)-2-cyano-N-[(lE)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder
Mp 104-107°C;
IR (KBr) vₘₐₓ 3440, 3324, 1645, 1580, 1510, 1368, 1069, 625 cm⁻¹;
¹H NMR(DMSO-d₆, 500 MHz) δ 2.07-2.13 (2H, m), 3.17-3.23 (2H, m), 3.24-3.29 (2H, m), 3.46 (2H, t, J = 5.9 Hz), 3.67 (2H, t, J = 4.9 Hz), 4.13-4.14 (2H, m), 4.18-4.20 (2H, m), 6.25-6.29 (2H, m), 6.69 (1H, d, J = 8.8 Hz), 6.98 (2H, brs), 7.08 (1H, d, J = 5.4 Hz), 7.09 (2H, brs), 8.41 (1H, d, J = 5.4 Hz);
HRMS m/z calcd for C₂₁H₂₄O₃N₅S 426.1600, found 426.1619;
MS (FAB) m/z: 426 [M+H]⁺, 409, 182, 165, 120, 63;
Anal. Calcd for C₂₁H₂₃N₅O₃S·0.50H₂O: C, 58.05; H, 5.57; N, 16.12. Found: C, 57.96; H, 5.85; N, 15.92.

### (Example 73) 3-amino-4-{4-[4-(methylsulsulfonyl)phenyl]-1,4-diazepan-1-yl}thieno[2,3-b]pyridine-2-carboxamide (Exemplified Compound No. 3-133)

3-amino-4-{4-[4-(methylthio)phenyl]-1,4-diazepan-1-yl}thieno[2,3-b]pyridine-2-carboxamide (68 mg, 0.16 mmol) which was produced in Example 64 (64e) was dissolved in methanol (5 mL) and was blended with an aqueous solution (5 mL) of oxone (216 mg, 0.35 mmol) and the mixture was stirred at room temperature for 18 hours. Water (10 mL) was added to the reaction liquid and the aqueous layer was extracted with a mixed solvent (3x10 mL) of methylene chloride/2-methylene chloride propanol (4:1). After the extract was dried over sodium sulfate, the solvent was evaporated under reduced pressure. The obtained residue was purified by silica gel column chromatography (ethyl acetate/methanol =20:1) and the title compound (16 mg, yield 22%) was obtained.
White powder
Mp 89-96°C;
IR (KBr) νₘₐₓ 3330, 1694, 1593, 1557, 1293, 1139, 772, 537 cm⁻¹;
¹H NMR(CDCl₃, 400 MHz) δ 2.11-2.17 (2H, m), 3.00 (3H, s), 3.47 (2H, dd, J = 5.9, 7.4 Hz), 3.55-3.61 (4H, m), 3.68 (2H, t, J = 5.9 Hz), 5.83 (1H, brs), 6.69 (2H, d, J = 9.0 Hz), 6.77 (1H, d, J = 5.5 Hz), 6.88 (1H, brs), 7.70 (2H, t, J = 9.0 Hz), 8.42 (1H, d, J = 5.5 Hz);
HRMS m/z calcd for C₂₀H₂₄O₃N₅S₂ 446.1321, found 446.1307;
MS (FAB) m/z: 446 [M+H]⁺, 415, 273, 242, 165, 65.

### (Example 74) 3-amino-4-{4-[4-(dimethylamino)phenyl]-1,4-diazepan-1-yl}thieno[2,3-b]pyridine-2-carboxamide (Exemplified Compound No. 3-104)

### (74a) tert-butyl 4-(4-dimethylaminophenyl)-1,4-diazepane-1-carboxylate

The reaction was performed in a similar method as described in J.Org.Chem. 65,1158- (2000) and the title compound was synthesized.
Pale brown liquid
IR (film) νₘₐₓ 2973, 1694, 1519, 1415, 1168, 930, 810 cm⁻¹;
¹H NMR(CDCl₃, 400 MHz) δ 1.38 (4.5H, s), 1.44 (4.5H, s), 1.93-1.98 (2H, m), 2.81 (6H, s), 3.20 (1H, t, J = 6.3 Hz), 3.31 (1H, t, J = 6.3 Hz), 3.45-3.57 (6H, m), 6.67 (2H, d, J = 9.0 Hz), 6.76 (2H, d, J = 9.0 Hz);
HRMS m/z calcd for C₁₈H₃₀O₂NS 320.2338, found 320.2338;
MS (FAB) m/z: 320 [M⁺], 263, 182, 165, 120, 63.

### (74b) 1-(4-dimethylaminophenyl)-1,4-diazepane

tert-butyl 4-(4-dimethylaminophenyl)-1,4-diazepane-1-carboxylate which was produced in Example 74 (74a) was used in place of tert-butyl 4-phenyl-1,4-diazepane-1-carboxylate and the reaction was performed in a similar method as described in Example 57 (57b) and the title compound was obtained.
Pale yellow powder
Mp 214-217°C (decomposition);
IR (film) νₘₐₓ 2934, 1520, 1472, 1323, 1194, 945, 813 cm⁻¹;
¹H NMR(CDCl₃, 500 MHz) δ 2.32 (2H, quint, J = 5.4 Hz), 2.84 (6H, s), 3.24 (2H, t, J = 5.9 Hz), 3.33 (2H, t, J = 4.9 Hz), 3.52 (2H, t, J = 6.8 Hz), 3.73 (2H, t, J = 4.9 Hz), 6.68 (2H, d, J = 9.3 Hz), 6.76 (2H, d, J = 9.3 Hz);
HRMS m/z calcd for C₁₃H₂₁N₃ 219.1737, found 219.1752;
MS (EI) m/z: 219 [M⁺], 189, 176, 163, 148, 134, 120.

### (74c) (2Z)-2-cyano-3-[4-(4-dimethylaminophenyl)-1,4-diazepan-1-yl]but-2-enethioamide

1-(4-dimethylaminophenyl)-1,4-diazepane which was produced in Example 74 (74b) was used in place of isobutylamine and the reaction was performed in a similar method as described in Example 5 (5a) and the title compound was synthesized.
Pale yellow powder
Mp 161-164°C (decomposition);
IR (KBr) νₘₐₓ 3439, 3314, 3154, 2178, 1601, 1518, 1442, 1292, 1218, 1015, 877, 816 cm⁻¹;
¹H NMR(DMSO-d₆, 500 MHz) δ 1.91-1.95 (2H, m), 2.26 (3H, s), 2.74 (6H, s), 3.41 (2H, t, J = 5.9 Hz), 3.51 (2H, t, J = 5.9 Hz), 3.58-3.62 (4H, m), 6.67 (2H, d, J = 9.8 Hz), 6.71 (2H, d, J = 9.8 Hz), 8.28 (1H, brs), 8.96 (1H, brs);
HRMS m/z calcd for C₁₈H₂₆N₅S 344.1909, found 344.1901;
MS (EI) m/z: 343 [M⁺], 309, 218, 182, 65;
Anal. Calcd for C₁₈H₂₅N₅S: C, 62.94; H, 7.34; N, 20.39; S, 9.34. Found: C, 62.66; H, 7.06; N, 20.28, S, 9.11.

### (74d) 4-{4-[4-(dimethylaminophenyl]-1,4-diazepan-1-yl}-2-thioxo-1,2-dihydropyridine-3-carbonitrile

(2Z)-2-cyano-3-[4-(4-dimethylaminophenyl)-1,4-diazepan-1-yl]but-2-enethioamide which was produced in Example 74 (74c) was used in place of (2Z)-2-cyano-3-(isobutylamino)but-2-enethioamide and the reaction was performed in a similar method as described in Example 5 (5b) and the title compound was obtained. Pale brown powder
Mp 186-188°C;
IR (KBr) νₘₐₓ 2948, 2204, 1625, 1517, 1243, 1142, 929, 810 cm⁻¹;
¹H NMR(DMSO-d₆, 500 MHz) δ 1.92-1.95 (2H, m), 2.73 (6H, s), 3.42 (2H, t, J = 5.9 Hz), 3.63 (2H, t, J = 4.9 Hz), 3.71 (2H, t, J = 5.4 Hz), 3.93 (2H, t, J = 5.4 Hz), 6.42 (1H, d, J = 7.8 Hz), 6.66 (2H, d, J = 9.3 Hz), 6.69 (2H, d, J = 9.3 Hz), 7.35 (1H, d, J = 7.8 Hz), 12.52 (1H, brs);
HRMS m/z calcd for C₁₉H₂₄N₅S 354.1753, found 354.1772;
MS (FAB) m/z: 353 [M⁺], 182, 165, 65.

### (74e) 3-amino-4-{4-[4-(dimethylamino)phenyl]-1,4-diazepan-1-yl}thieno[2,3-b]pyridine-2-carboxamide

4-{4-[4-(dimethylaminophenyl]-1,4-diazepan-1-yl}-2-thioxo-1,2dihydropyridine-3-carbonitrile which was produced in Example 74 (74d) was used in place of 4-(isobutylamino)-2-thioxo-1,2-dihydropyridine-3-carbonitrile and the reaction was performed in a similar method as described in Example 5 (5c) and the title compound was obtained.
White powder
Mp 174-176°C;
IR (KBr) νₘₐₓ 3438, 3324, 2944, 1644, 1579, 1517, 1368, 1230, 940, 811 cm⁻¹;
¹H NMR(DMSO-d₆, 500 MHz) δ 2.07-2.12 (2H, m), 2.75 (6H, s), 3.20-3.25 (2H, m), 3.26-3.30 (2H, m), 3.47 (2H, t, J = 5.9 Hz), 3.67 (2H, t, J = 4.4 Hz), 6.71 (4H, s), 6.97 (2H, brs), 7.07-7.08 (3H, m), 8.40 (1H, d, J = 5.4 Hz);
HRMS m/z calcd for C₂₁H₂₇ON₆S 411.1967, found 411.1945;
MS (FAB) m/z: 411 [M+H]⁺, 394, 273, 242, 200, 189, 175, 93;
Anal. Calcd for C₂₁H₂₆N₆OS·0.16H₂O: C, 61.01; H, 6.42; N, 20.33; S, 7.76. Found: C, 60.75; H, 6.34; N, 20.20; S, 7.94.

### (Example 75) 3-amino-6-methyl-4-(4-phenylpiperazin-1-yl)thieno[2,3-b]pyridine-2-carboxamide (Exemplified Compound No. 3-76)

### (75 a) (1-ethoxyethylidene)malononitrile

An acetic acid (1 mL) solution of malononitrile (4.41 g, 66.8 mmol) and orthotriethyl acetate (14.7 mL, 80.2 mmol) was stirred at 80°C for one hour. The reaction liquid was cooled to room temperature and generated powder was separated by filtration and the title compound (7.14 g, yield 79%) was obtained.
White powder
¹H NMR(DMSO-d₆, 400 MHz) δ 1.32 (3H, t, J = 7.0 Hz), 2.45 (3H, s), 4.42 (2H, q, J = 7.0 Hz).

### (75b) [1-(4-phenylpiperazin-1-yl)ethylidene]malononitrile

1-phenylpiperazine (1.68 mL, 11 mmol) was added to an ethanol (40 mL) suspension of (1-ethoxyethylidene)malononitrile (1.36 g, 10 mmol) which was produced in Example 75 (75a) and the mixture was stirred at room temperature for 15 hours. The powder which was generated was separated by filtration and the title compound (1.74 g, yield 69%) was obtained.
White powder
Mp 188-190°C;
IR (KBr) νₘₐₓ 2199, 1560, 1450, 1236, 998, 763 cm⁻¹;
¹H NMR(DMSO-d₆, 500 MHz) δ 2.32 (3H, s), 3.32-3.34 (4H, m), 3.88 (4H, brs), 6.78 (1H, t, J = 8.6 Hz), 6.91 (2H, d, J = 8.6 Hz), 7.22 (2H, t, J = 8.6 Hz);
HRMS m/z calcd for C₁₅H₁₆N₄ 252.1375, found 252.1372;
MS (EI) m/z: 252 [M⁺], 210, 160, 146, 132, 126, 119, 105, 91, 77, 65;
Anal. Calcd for C₁₅H₁₆N₄: C, 71.40; H, 6.39; N, 22.21. Found: C, 71.16; H, 6.41; N, 22.25.

### (75c) [(2E)-3-(dimethylamino)-1-(4-phenylpiperazin-1-yl)but-2-enylidene]malononitrile

A xylene (10 mL) suspension of [1-(4-phenylpiperazin-1-yl)ethylidene]malononitrile which was produced in Example 75 (75b) (1.74 g, 6.9 mmol) was blended with dimethylacetamide dimethylacetal (5.0 mL, 34.5 mmol) and heated for four hours under reflux. The reaction liquid was concentrated and the obtained residue was purified by silica gel column chromatography (100% ethyl acetate) and the obtained solid was further washed with ether and the title compound (1.23 g, yield 55%) was obtained.
Yellow powder
Mp 181-184°C;
IR (KBr) νₘₐₓ 2193, 1557, 1511, 1440, 1375, 1258, 1023, 765, 551 cm⁻¹;
¹H NMR(DMSO-d₆, 400 MHz) δ 2.18 (3H, s), 3.04 (6H, s), 3.24 (4H, t, J = 5.1 Hz), 3.68 (4H, t, J = 5.1 Hz), 4.53 (1H, s), 6.81 (1H, t, J = 7.8 Hz), 6.95 (2H, d, J = 7.8 Hz), 7.24 (2H, t, J = 7.8 Hz);
HRMS m/z calcd for C₁₉H₂₃N₅ 321.1953, found 321.1938;
MS (EI) m/z: 321 [M⁺], 277, 256, 189, 160, 132, 72;
Anal. Calcd for C₁₉H₂₃N₅: C, 71.00; H, 7.21; N, 21.79. Found: C, 71.03; H, 7.21; N, 21.60.

### (75d) 6-methyl-2-oxo-4-(4-phenylpiperazin-1-yl)-1,2-dihydropyridine-3-carbonitrile

[(2E)-3-(dimethylamino)-1-(4-phenylpiperazin-1-yl)but-2-enylidene]malononitrile (1.22 g, 3.8 mmol) which was produced in Example 75 (75c) was dissolved in a mixed solvent (10 mL) of acetic acid/water (4:1) and heated for one hour under reflux. The reaction liquid was cooled to room temperature and water (10 mL) was added and powder which was generated was separated by filtration and the title compound (0.87 g, yield 85%) was obtained.
Yellow powder
Mp >270°C;
IR (KBr) νₘₐₓ 2837, 2202, 1618, 1497, 1447, 1230, 1003, 755 cm⁻¹;
¹H NMR(DMSO-d₆, 400 MHz) δ 2.14 (3H, s), 3.27 (4H, t, J = 5.1 Hz), 3.76 (4H, t, J = 5.1 Hz), 6.02 (1H, s), 6.81 (1H, t, J = 7.8 Hz), 6.96 (2H, d, J = 7.8 Hz), 7.24 (2H, t, J = 7.8 Hz), 11.40 (1H, brs);
HRMS m/z calcd for C₁₇H₁₈ON₄ 294.1481, found 294.1477;
MS (EI) m/z: 294 [M⁺], 276, 252, 189, 162, 132, 105, 91, 77;
Anal. Calcd for C₁₇H₁₈N₄O·0.16H₂O: C, 68.69; H, 6.21; N, 18.85. Found: C, 69.00; H, 6.16; N, 18.49.

### (75e) 2-chloro-6-methyl-4-(4-phenylpiperazin-1-yl)nicotinonitrile

N,N-dimethylaniline (75 µL, 0.53 mmol) and phosphorus oxychloride (1.6 mL, 17.4 mmol) were added to 1,4-dioxane (3 mL) solution of 6-methyl-2-oxo-4-(4-phenylpiperazin-1-yl)-1,2-dihydropyridine-3-carbonitrile (223 mg, 0.76 mmol) which was produced in Example 75 (75d), and heated under reflux for two hours. After the reaction liquid was concentrated and a sodium hydrogen carbonate aqueous solution (10 mL) to the obtained residual substance, the aqueous layer was extracted with ethyl acetate (3x10 mL) and after the extract was dried over sodium sulfate, the solvent was evaporated under reduced pressure. The obtained residue was purified by silica gel column chromatography (hexane/ethyl acetate =2:1) and the title compound (117 mg, yield 49%) was obtained.
Yellow powder
Mp 139-140°C;
IR (KBr) νₘₐₓ 2830, 2216, 1582, 1502, 1447, 1229, 989, 758, 695 cm⁻¹;
¹H NMR(CDCl₃, 400 MHz) δ 2.49 (3H, s), 3.36 (4H, t, J = 5.1 Hz), 3.68 (4H, t, J = 5.1 Hz), 6.57 (1H, s), 6.89-6.95 (3H, m), 7.29 (2H, dd, J = 7.1, 8.6 Hz);
HRMS m/z calcd for C₁₇H₁₇N₄Cl 312.1142, found 312.1147;
MS (EI) m/z: 312 [M⁺], 294, 270, 206, 194, 179, 152, 132, 105, 91, 77;
Anal. Calcd for C₁₇H₁₇ClN₄: C, 65.28; H, 5.48; N, 17.91. Found: C, 65.11; H, 5.14; N, 17.69.

### (75f) 3-amino-6-methyl-4-(4-phenylpiperazin-1-yl)thieno[2,3-b]pyridine-2-carboxamide

2-mercaptoacetamide (about 70% purity) (48 mg, 0.52 mmol) and 8N aqueous solution of sodium hydroxide (0.1 mL) were added to N,N-dimethylformamide (0.6 mL) solution of 2-chloro-6-methyl-4-(4-phenylpiperazin-1-yl)nicotinonitrile (92 mg, 0.29 mmol) which was produced in Example 75 (75e) and the mixture was stirred at room temperature for one hour. Water was added to the reaction solution and the deposited crystal was separated by filtration and the title compound was obtained 90 mg (yield 84%).
Pale yellow crystal
Mp 247-250°C;
IR (KBr) νₘₐₓ 3435, 2828, 1651, 1581, 1494, 1365, 1223, 993, 762, 694 cm⁻¹;
¹H NMR(DMSO-d₆, 400 MHz) δ 2.50 (3H, s), 2.88-3.70(8H, m), 6.80 (1H, t, J = 7.4 Hz), 6.90 (2H, brs), 6.95 (1H, s), 7.00 (2H, d, J = 7.4 Hz), 7.04 (2H, brs), 7.23 (2H, t, J = 7.4 Hz);
HRMS m/z calcd for C₁₉H₂₁ON₅S 367.1467, found 367.1450;
MS (EI) m/z: 367 [M⁺], 262, 244, 230, 218, 190, 175, 132, 120, 104, 91, 77;
Anal. Calcd for C₁₉H₂₁N₅OS: C, 62.10; H, 5.76; N, 19.06. Found: C, 62.38; H, 5.84; N, 18.84.

### (Example 76) 3-amino-4-[4-(4-fluorophenyl)-1,4-diazepan-1-yl]thieno[2,3-b]pyridine-2-carboxamide (Exemplified Compound No. 3-89)

### (76a) tert-butyl 4-(4-fluorophenyl)-1,4-diazepane-1-carboxylate

The reaction was performed in a similar method as described in Org.Lett. 4,581-584 (2002) and the title compound was synthesized.
White powder (yield 45%)
Mp 88-89°C;
IR (KBr) νₘₐₓ 2968, 2921,1682, 1515, 1418, 1244, 828 cm⁻¹;
¹H NMR(CDCl₃, 400 MHz) δ 1.36 (4.5H, s), 1.43 (4.5H, s), 1.90-2.01 (2H, m), 3.21 (1H, t, J = 5.9 Hz), 3.32 (1H, t, J = 5.9Hz), 3.46-3.63 (6H, m), 6.61 (2H, dd, J = 9.4, 4.3 Hz), 6.92 (2H, t, J = 8.6 Hz);
MS (EI) m/z: 294 [M⁺], 238, 223, 193;
Anal. Calcd for C₁₆H₂₃FN₂O₂·0.14H₂O: C, 64.73; H, 7.90; N, 9.44; F, 6.40. Found: C, 64.77; H, 7.92; N, 9.36; F, 6.37.

### (76b) 1-(4-fluorophenyl)-1,4-diazepane

tert-butyl 4-(4-fluorophenyl)-1,4-diazepane-1-carboxylate which was produced in Example 76 (76a) was used in place of tert-butyl 4-phenyl-1,4-diazepane-1-carboxylate and the reaction was performed in a similar method as described in Example 57 (57b) and the title compound was obtained.
Pale yellow oil (yield 81%)
IR (film) νₘₐₓ 3322, 2935, 1611, 1513, 1228, 814 cm⁻¹;
¹H NMR(CDCl₃, 500 MHz) δ 1.89 (2H, quint, J = 5.9 Hz), 2.83 (2H, t, J = 5.9 Hz), 3.02 (2H, t, J = 5.4 Hz), 3.51 (2H, t, J = 5.4 Hz), 3.54 (2H, t, J = 6.3 Hz), 6.61 (2H, dd, J = 9.3, 4.4 Hz), 6.91 (2H, t, J = 9.3 Hz);
MS (EI) m/z: 194 [M⁺], 164, 152, 138.

### (76c) (2Z)-2-cyano-3-[4-(4-fluorophenyl)-1,4-diazepan-1-yl]but-2-enethioamide

1-(4-fluorophenyl)-1,4-diazepane which was produced in Example 76 (76b) was used in place of isobutylamine and the reaction was performed in a similar method as described in Example 5 (5a) and the title compound was synthesized. Yellow ocher powder (yield 30%)
Mp 150-152°C;
IR (KBr) νₘₐₓ 3356, 3269, 3178, 2177, 1615, 1537, 1507 cm⁻¹;
¹H NMR(DMSO-*d₆*, 400 MHz) δ 1.89-1.98 (2H, m), 2.23 (3H, s), 3.47-3.54 (4H, m), 3.57-3.63 (2H, m), 3.66-3.72 (2H, m), 6.74 (2H, dd, J = 9.0, 4.3 Hz), 6.97 (2H, t, J = 9.0 Hz), 8.31 (1H, bs), 8.96 (1H, bs);
MS (FAB) m/z: 319 [M+H]⁺, 273, 259, 242;
Anal. Calcd for C₁₆H₁₉FN₄S·0.4H₂O: C, 59.02; H, 6.13; N, 17.21. Found: C, 58.73; H, 5.84; N, 17.20.

### (76d) (2Z)-2-cyano-N-[(1E)-(dimethylamino)methylene]-3-[4-(4-fluorophenyl)-1,4-diazepan-1-yl]but-2-enethioamide

(2Z)-2-cyano-3-[4-(4-fluorophenyl)-1,4-diazepan-1-yl]but-2-enothioamide which was produced in Example 76 (76c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder (yield 73%)
Mp 127-128°C (decomposition);
IR (KBr) νₘₐₓ 2924, 2182, 1610, 1510, 1421, 1395, 1324, 1293 cm⁻¹;
¹H NMR(DMSO-*d₆*, 400 MHz) δ 1.93-2.02 (2H, m), 2.47 (3H, s), 2.92 (3H, s), 3.14 (3H, s), 3.53 (2H, t, J = 5.9 Hz), 3.73 (2H, t, J = 5.9 Hz), 3.78 (4H, bs), 6.76 (2H, dd, J = 9.0, 4.3 Hz), 6.97 (2H, t, J = 9.0 Hz), 8.45 (2H, s);
MS (FAB) m/z: 374 [M+H]⁺, 340,273, 195;
Anal. Calcd for C₁₉H₂₄FN₅S: C, 61.10; H, 6.48; N, 18.75; F, 5.09; S, 8.59. Found: C, 61.01; H, 6.49; N, 18.50; F, 5.17; S, 8.56.

### (76e) 3-amino-4-[4-(4-fluorophenyl)-1,4-diazepan-1-yl]thieno[2,3-b]pyridine-2-carboxamide

(2Z)-2-cyano-N-[(1E)-(dimethylamino)methylene]-3-[4-(4-fluorophenyl)-1,4-diazepan-1-yl]but-2-enethioamide which was produced in Example 76 (76d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale yellow powder (yield 64%)
Mp 203-205°C;
IR (KBr) νₘₐₓ 3453, 3324, 3179, 2948, 2838, 1646, 1579, 1510, 1369 cm⁻¹;
¹H NMR(DMSO-*d₆*, 500 MHz) δ 2.10-2.19 (2H, m), 3.16-3.24 (2H, m), 3.30 (2H, s), 3.49-3.56 (2H, m), 3.71-3.78 (2H, m), 6.76 (2H, dd, J = 9.0, 4.4 Hz), 6.96-7.05 (4H, m), 7.06-7.12 (3H, m), 8.40 (1H, d, J = 4.9 Hz);
MS (FAB) m/z: 386 [M+H]⁺, 369, 273;
Anal. Calcd for C₁₉H₂₀FN₅OS·1.35H₂O: C, 57.95; H, 5.81; N, 17.78; F, 4.82; S, 8.14. Found: C, 57.58; H, 5.41; N, 17.76; F, 4.73; S, 7.99.

### (Example 77) 3-amino-4-[4-(4-methylphenyl)-1,4-diazepan-1-yl]thieno[2,3-b]pyridine-2-carboxamide (Exemplified Compound No. 3-111)

### (77a) tert-butyl 4-(4-methylphenyl)-1,4-diazepane-1-carboxylate

The reaction was performed in a similar method as described in Org. Lett., 4, 581-584 (2002) and the title compound was synthesized.
Colourless oil (yield 36%)
IR (film) νₘₐₓ, 2974, 2929, 1695, 1619, 1521, 1415, 1365, 1237, 1169 cm⁻¹;
¹H NMR(CDCl₃, 400 MHz) δ 1.38 (4.5H, s), 1.44 (4.5H, s), 1.91-2.01 (2H, m), 2.23 (3H, s), 3.18 (1H, t, J = 5.9 Hz), 3.20 (1H, t, J = 5.9 Hz), 3.46-3.59 (6H, m), 6.60 (2H, d, J = 8.2 Hz), 7.00 (2H, d, J = 8.2 Hz);
MS (EI) m/z: 290 [M⁺], 234, 146.

### (77b) 1-(4-methylphenyl)-1,4-diazepane

tert-butyl 4-(4-methylphenyl)-1,4-diazepane-1-carboxylate which was produced in Example 77 (77a) was used in place of tert-butyl 4-phenyl-1,4-diazepane-1-carboxylate and the reaction was performed in a similar method as described in Example 57 (57b) and the title compound was obtained.
Pale yellow oil (yield 100%)
IR (film) νₘₐₓ 3318, 2923, 1618, 1520, 1394, 1363, 1189, 802 cm⁻¹;
¹H NMR(CDCl₃, 400 MHz) δ 1.89 (2H, quint, J = 5.9 Hz), 2.23 (3H, s), 2.79-2.85 (2H, m), 2.99-3.05 (2H, m), 3.49-3.58 (4H, m), 6.60 (2H, d, J = 8.2 Hz), 7.00 (2H, d, J = 8.2 Hz);
MS (EI) m/z: 190 [M⁺], 160, 148, 134.

### (77c) 4-[4-(4-methylphenyl)-1,4-diazepan-1-yl]-2-thioxo-1,2-dihydropyridine-3-carbonitrile

1-(4-methylphenyl)-1,4-diazepane (463 mg, 2.39 mmol) which was produced in Example 77 (77b) and (2Z)-2-cyano-3-ethoxybut-2-enethioamide (J. Org.Chem. (1962), 27,2433-2439) (386 mg, 2.27 mmol) were dissolved in N,N-dimethylformamide (4.78 mL) and the mixture was stirred at room temperature for 30 minutes. Subsequently N,N-dimethylformamide dimethylacetal (302 µL, 2.27 mmol) was added and the reaction mixture was stirred at 60°C after stirring at room temperature for 30 minutes for one hour. After cooling to room temperature, the reaction mixture was blended with ethyl acetate (10 mL) and water (50 mL) and the mixture was stirred. The deposited solid was separated by filtration and washed with ethyl acetate and water sequentially and 125 mg of the title compound was obtained. Yield 28% from 1-(4-methylphenyl)-1,4-diazepane.
Pale brown powder
Mp 220-223°C;
IR (KBr) νₘₐₓ 3115, 2940, 2205, 1626, 1518, 1250, 928,798, 775 cm⁻¹;
¹H NMR(CDCl₃, 500 MHz) δ 2.11 (2H, quint, J = 5.9 Hz), 2.25 (3H, s), 3.56 (2H, t, J = 5.9 Hz), 3.71-3.76 (2H, m), 3.76-3.80 (2H, m), 4.10-4.15 (2H, m), 6.19 (1H, d, J = 7.8 Hz), 6.65 (2H, d, J = 8.2 Hz), 7.05 (2H, d, J = 8.2 Hz), 7.20 (1H, d, J = 7.8 Hz), 11.22 (1H, bs);
MS (EI) m/z: 324 [M⁺], 160, 146;
Anal. Calcd for C₁₈H₂₀N₄S·0.1H₂O: C, 66.27; H, 6.24; N, 17.17; S, 9.83. Found: C, 66.07; H, 6.19; N, 17.10; S, 9.60.

### (77d) 3-amino-4-[4-(4-methylphenyl)-1,4-diazepan-1-yl]thieno[2,3-b]pyridine-2-carboxamide

4-[4-(4-methylphenyl)-1,4-diazepan-1-yl]-2-thioxo-1,2-dihydropyridine-3-carbonitrile which was produced in Example 77 (77c) was used in place of 4-(isobutylamino)-2-thioxo-1,2-dihydropyridine-3-carbonitrile and the reaction was performed in a similar method as described in Example 5 (5c) and the title compound was obtained.
Pale brown powder (yield 28%)
Mp 175-176°C;
IR (KBr) νₘₐₓ 3429,3317, 3170, 3093, 2942, 2833, 1635, 1579, 1520, 1372 cm⁻¹; ¹H NMR(DMSO-*d₆*, 400 MHz) δ 2.07-2.16 (2H, m), 2.18 (3H, s), 3.15-3.22 (2H, m), 3.23-3.30 (2H, m), 3.51 (2H, t, J = 6.3 Hz), 3.72 (2H, t, J = 4.7 Hz), 6.66 (2H, d, J = 8.2 Hz), 6.92-6.99 (4H, m), 7.02-7.09 (3H, m), 8.37 (1H, d, J = 5.1 Hz);
MS (FAB) m/z: 382 [M+H]⁺, 365, 275;
Anal. Calcd for C₂₀H₂₃N₅OS: C, 62.97; H, 6.08; N, 18.36; S, 8.41. Found: C, 62.58; H, 6.02; N, 18.23; S, 8.29.

### (Example 78) 3-amino-4-[4-(3-chlorophenyl)-1,4-diazepan-1-yl]thieno[2,3-b]pyridine-2-carboxamide (Exemplified Compound No. 3-93)

### (78a) tert-butyl 4-(3-chlorophenyl)-1,4-diazepane-1-carboxylate

The reaction was performed in a similar method as described in Org.Lett.,4,581-584(2002) and the title compound was synthesized.
Colourless oil (yield 30%)
IR (film) νₘₐₓ 2975, 1694, 1594, 1493, 1416, 1365, 1237,1168 cm⁻¹;
¹H NMR(CDCl₃, 400 MHz) δ 1.36 (4.5H, s), 1.44 (4.5H, s), 1.95 (2H, quint, J = 6.3 Hz), 3.21 (1H, t, J = 5.9 Hz), 3.31 (1H, t, J = 5.9 Hz), 3.46-3.60 (6H, m), 6.54 (1H, dd, J = 9.0, 2.0 Hz), 6.58-6.65 (2H, m), 7.08 (1H, t, J = 8.6 Hz);
MS (EI) m/z: 310 [M⁺], 253, 166.

### (78b) 1-(3-chlorophenyl)-1,4-diazepane

tert-butyl 4-(3-chlorophenyl)-1,4-diazepane-1-carboxylate which was produced in Example 78 (78c) was used in place of tert-butyl 4-phenyl-1,4-diazepane-1-carboxylate and the reaction was performed in a similar method as described in Example 57 (57b) and the title compound was obtained.
Pale brown oil (yield 100%)
IR (film) νₘₐₓ 3323, 2933, 1593,1494, 1362, 1102, 984, 759, 683 cm⁻¹;
¹H NMR(CDCl₃, 400 MHz) δ 1.89 (2H, quint, J = 5.9 Hz), 2.79-2.86 (2H, m), 2.98-3.06 (2H, m), 3.52 (2H, t, J = 5.5 Hz), 3.56 (2H, t, J = 6.3 Hz), 6.56 (1H, dd, J = 8.6, 2.7 Hz), 5.59-6.63 (1H, m), 6.67-6.64 (1H, m), 7.10 (1H, t, J = 8.6 Hz);
MS (EI) m/z: 210 [M⁺], 168, 154.

### (78c) (2Z)-3-[4-(3-chlorophenyl)-1,4-diazepan-1-yl]-2-cyanobut-2-enethioamide

1-(3-chlorophenyl)-1,4-diazepane which was produced in Example 78 (78b) was used in place of isobutylamine and the reaction was performed in a similar method as described in Example 5 (5a) and the title compound was synthesized. Slightly brown powder (yield 62%)
Mp 133-134°C;
IR (KBr) νₘₐₓ 3320, 3152, 2964, 2187, 1593, 1541, 1488, 1390, 1346 cm⁻¹;
¹H NMR(DMSO-d₆, 400 MHz) δ 1.88-1.97 (2H, m), 2.24 (3H, s), 3.48-3.57 (4H, m), 3.58-3.63 (2H, m), 3.70-3.75 (2H, m), 6.60 (1H, dd, J = 8.2, 1.6 Hz), 6.70 (1H, dd, J = 8.2, 2.3 Hz), 6.75-6.78 (1H, m), 7.14 (1H, t, J = 8.2 Hz), 8.36 (1H, bs), 9.01 (1H, s);
MS (FAB) m/z: 335 [M+H]⁺, 246,200.

### (78d) (2Z)-3-[4-(3-chlorophenyl)-1,4-diazepan-1-yl]-2-cyano-N-[(1E)-(dimethylamino)methylene]but-2-enethioamide

(2Z)-3-[4-(3-chlorophenyl)-1,4-diazepan-1-yl]-2-cyanobut-2-enethioamide which was produced in Example 78 (78c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Yellow powder (yield 89%)
Mp 129-130°C (decomposition);
IR (KBr) νₘₐₓ 2924, 2925, 2177, 1609, 1397, 1325, 1290 cm⁻¹;
¹H NMR(DMSO-d₆, 400 MHz) δ 1.92-2.01 (2H, m), 2.49 (3H, s), 3.15 (3H, s), 3.32 (3H, s), 3.57 (2H, t, J = 5.5 Hz), 3.72 (2H, t, J = 5.5 Hz), 3.75-3.80 (2H, m), 3.80-3.84 (2H, m), 6.61 (1H, d, J = 8.3 Hz), 6.73 (1H, dd, J = 8.3, 2.0 Hz), 6.79 (1H, s), 7.14 (1H, t, J = 8.3 Hz), 8.45 (1H, s);
MS (FAB) m/z: 390 [M+H]⁺, 356, 211, 180;
Anal. Calcd for C₁₉H₂₄ClN₅S: C, 58.52; H, 6.20; N, 17.96; Cl, 9.09; S, 8.22. Found: C, 58.26; H, 6.18; N, 17.83; F, 8.95; S, 8.22.

### (78e) 3-amino-4-[4-(3-chlorophenyl)-1,4-diazepan-1-yl]thieno[2,3-b]pyridine-2-carboxamide

(2Z)-3-[4-(3-chlorophenyl)-1,4-diazepan-1-yl]-2-cyano-N-[(1E)-(dimethylamino)methylene]but-2-enethioamide which was produced in Example 78 (78d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Slightly brown powder (yield 61%)
Mp 213-215°C;
IR (KBr) νₘₐₓ 3442, 3324, 3183, 2950, 2836, 1644, 1593, 1494, 1369 cm-¹;
¹H NMR(DMSO-d₆, 400 MHz) δ 2.10-2.20 (2H, m), 3.13-3.24 (2H, m), 3.25-3.31 (2H, m), 3.51-3.58 (2H, m), 3.77 (2H, t, J = 4.4 Hz), 6.62 (1H, dd, J = 8.3, 2.0 Hz), 6.73 (1H, dd, J = 8.3, 2.0 Hz), 7.01 (1H, bs), 7.06-7.12 (3H, m), 7.17 (1H, t, J = 8.3 Hz), 8.40 (1H, d, J = 5.4 Hz);
MS (FAB) m/z: 402 [M+H]⁺, 385, 273;
Anal. Calcd for C₁₉H₂₀N₅ClOS·0.18H₂O: C, 56.33; H, 5.07; N, 17.29; Cl, 8.75; S, 7.91. Found: C, 56.33; H, 5.01; N, 17.38; Cl, 8.77; S, 7.68.

### (Example 79) 3-amino-4- {4-[4-(benzyloxy)phenyl]-1,4-diazepan-1-yl}thieno[2,3-b]pyridine-2-carboxamide (Exemplified Compound No. 3-134)

### (79a) tert-butyl 4-[4-(benzyloxy)phenyl]-1,4-diazepane-1-carboxylate

The reaction was performed in a similar method as described in Org. Lett., 4, 581-584 (2002) and the title compound was synthesized.
Colourless oil (yield 15%)
IR (film) νₘₐₓ 2974, 1693, 1512, 1416, 1237, 1168 cm⁻¹;
¹H NMR(CDCl₃, 400 MHz) δ 1.37 (4.5H, s), 1.44 (4.5H, s), 1.90-2.00 (1H, m), 3.17-3.23 (1H, m), 3.27-3.35 (1H, m), 3.41-3.61 (6H, m), 4.97 (2H, s), 6.62 (2H, d, J = 8.8 Hz), 6.86 (2H, d, J = 8.8 Hz), 7.26-7.44 (5H, m);
MS (EI) m/z: 382 [M⁺], 327, 291, 235.

### (79b) 1-[4-(benzyloxy)phenyl]-1,4-diazepane

tert-butyl 4-[4-(benzyloxy)phenyl]-1,4-diazepane-1-carboxylate which was produced in Example 79 (79a) was used in place of tert-butyl 4-phenyl-1,4-diazepane-1-carboxylate and the reaction was performed in a similar method as described in Example 57 (57b) and the title compound was obtained.
Pale brown oil (yield 100%)
IR (film) νₘₐₓ 3329, 3033, 1512, 1455, 1237, 1025, 812 cm⁻¹;
¹H NMR(CDCl₃, 400 MHz) δ 1.88 (2H, quint, J = 5.9 Hz), 2.82 (2H, t, J = 5.9 Hz), 3.01 (2H, t, J = 5.5 Hz), 3.46-3.54 (4H, m), 4.98 (2H, s), 6.62 (2H, d, J = 9.0 Hz), 6.86 (2H, d, J = 9.0 Hz), 7.25-7.43 (5H, m);
MS (EI) m/z: 282 [M⁺], 191, 148.

### (79c) 4-{4-[4-(benzyloxy)phenyl]-1,4-diazepan-1-yl}-2-thieno-1,2-dihydropyridine-3-carbonitrile

1-[4-(benzyloxy)phenyl]-1,4-diazepane which was produced in Example 79 (79b) was used in place of 1-(4-methylphenyl)-1,4-diazepane and the reaction was performed in a similar method as described in Example 77 (77c) and the title compound was obtained.
Pale brown powder (yield 30%)
Mp 190-191°C;
IR (KBr) νₘₐₓ 3129, 3045, 2953, 2205, 1625, 1511, 1455, 1240 cm⁻¹;
¹H NMR(DMSO-d₆, 400 MHz) δ 1.87-1.98 (2H, m), 3.45 (2H, t, J = 5.9 Hz), 3.62-3.75 (4H, m), 3.89-3.96 (2H, m), 4.96 (2H, s), 6.41 (1H, d, J = 7.8 Hz), 6.70 (2H, d, J - 9.0 Hz), 6.83 (2H, d, J = 9.0 Hz), 7.26-7.42 (6H, m);
MS (FAB) m/z: 417 [M+H]⁺, 325, 200;
Anal. Calcd for C₂₄H₂₄N₄OS·0.54H₂O: C, 67.62; H, 5.93; N, 13.14; S, 7.52. Found: C, 67.30; H, 5.97; N, 13.50; S, 7.39.

### (79d) 3-amino-4-{4-[4-(benzyloxy)phenyl]-1,4-diazepan-1-yl}thieno[2,3-b]pyridine-2-carboxamide

4- {4-[4-(benzyloxy)phenyl]-1,4-diazepan-1-yl}-2-thieno-1,2-dihydropyridine-3-carbonitrile which was produced in Example 79 (79c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 89%)
Mp 211-213°C;
IR (KBr) νₘₐₓ 3430, 3320, 3167, 2945, 1646, 1579, 1511, 1367, 1230 cm⁻¹;
¹H NMR(DMSO-*d₆*, 500 MHz) δ 2.06-2.16 (2H, m), 3.15-3.22 (2H, m), 3.24-3.30 (2H, m), 3.44-3.51 (2H, m), 3.65-3.72 (2H, m), 4.99 (2H, s), 6.69 (2H, d, J = 9.0 Hz), 6.86 (2H, d, J = 9.0 Hz), 6.98 (2H, bs), 7.05 (1H, d, J = 5.1 Hz), 7.07 (2H, bs), 7.26-7.43 (5H, m), 8.37 (1H, d, J = 5.1 Hz);
MS (FAB) m/z: 474 [M+H]⁺, 242, 200;
Anal. Calcd for C₂₆H₂₇N₅O₂S·0.26H₂O: C, 65.29; H, 5.80; N, 14.64; S, 6.70. Found: C, 65.46; H, 6.02; N, 14.59; S, 6.51.

### (Example 80) 3-amino-4-[4-(3-nitrophenyl)-1,4-diazepan-1-yl]thieno[2,3-b]pyridine-2-carboxamide (Exemplified Compound No. 3-96)

### (80a) tert-butyl 4-(3-nitrophenyl)-1,4-diazepane-1-carboxylate

The reaction was performed in a similar method as described in Org. Lett., 4, 581-584 (2002) and the title compound was synthesized.
Orange oil (yield 25%)
IR (film) νₘₐₓ 2975, 1692, 1528, 1347, 1166, 735 cm⁻¹;
¹H NMR(CDCl₃, 400 MHz) δ 1.33 (4.5H, s), 1.41 (4.5H, s), 1.98 (2H, quint, J = 5.9 Hz), 3.21-3.26 (1H, m), 3.31-3.37 (1H, m), 3.56-3.66 (6H, m), 6.94 (1H, dd, J = 8.2, 2.0 Hz), 7.30 (1H, t, J = 8.2 Hz), 7.44-7.50;
MS (FAB) m/z: 321 [M⁺], 266, 248, 222.

### (80b) 1-(3-nitrophenyl)-1,4-diazepane

tert-butyl 4-(3-nitrophenyl)-1,4-diazepane-1-carboxylate which was produced in Example 80 (80a) was used in place of tert-butyl 4-phenyl-1,4-diazepane-1-carboxylate and the reaction was performed in a similar method as described in Example 57 (57b) and the title compound was obtained.
Reddish orange oil (yield 100%)
IR (film) νₘₐₓ 2935, 2855, 1618, 1525, 1347, 735 cm⁻¹;
¹H NMR(CDCl₃, 500 MHz) δ 1.93 (2H, quint, J = 5.9 Hz), 2.85 (2H, t, J = 5.9 Hz), 3.06 (2H, t, J = 5.4 Hz, 3.60 (2H, t, J = 5.4 Hz), 3.64 (2H, t, J = 5.9 Hz), 6.95 (1H, dd, J = 8.3, 2.4 Hz), 7.31 (1H, t, J = 8.3 Hz), 7.44-7.51 (2H, m);
MS (EI) m/z: 221 [M⁺], 179, 165.

### (80c) (2Z)-2-cyano-3-[4-(3-nitrophenyl)-1,4-diazepan-1-yl]but-2-enethioamide

1-(3-nitrophenyl)-1,4-diazepane which was produced in Example 80 (80b) was used in place of isobutylamine and the reaction was performed in a similar method as described in Example 5 (5a) and the title compound was synthesized. Orange powder (yield 71 %)
Mp 157-158°C;
IR (KBr) νₘₐₓ 3443, 3353, 3277, 3176, 2926, 2182, 1617, 1525, 1346 cm⁻¹;
¹H NMR(DMSO-*d₆*, 500 MHz) δ 1.92-2.01 (2H, m), 2.24 (3H, s), 3.53-3.58 (2H, m), 3.62-3.69 (4H, m), 3.81-3.88 (2H, m), 7.20-7.26 (1H, m), 7.40-7.50 (3H, m), 8.40 (1H, bs), 9.05 (1H, bs);
MS (FAB) m/z: 346 [M+H]⁺, 246, 200.

### (80d) 3-amino-4-[4-(3-nitrophenyl)-1,4-diazepan-1-yl]thieno[2,3-b]pyridine-2-carboxamide

(2Z)-2-cyano-3-[4-(3-nitrophenyl)-1,4-diazepan-1-yl]but-2-enethioamide which was produced in Example 80 (80c) was used in place of (2Z)-2-cyano-3-(propylamino)but-2-enethioamide and the reaction was performed in a similar method as described in Example 4 (4b) and the title compound was obtained.
Pale yellow powder (yield 6%)
Mp 180-185°C;
IR (KBr) νₘₐₓ 3450, 3324, 3169, 2953, 2842, 1646, 1579, 1524,1368, 1345 cm⁻¹;
¹H NMR(DMSO-*d₆*, 500 MHz) δ 2.16-2.24 (2H, m), 3.15-3.24 (2H, m), 3.29-3.38 (2H, m), 3.64 (2H, t, J = 5.9 Hz), 3.84-3.89 (2H, m), 7.01 (2H, bs), 7.09 (1H, d, J = 5.4 Hz), 7.10 (2H, bs), 7.21-7.26 (1H. m), 7.44 (2H, d, J = 5.4 Hz), 8.41 (1H, d, J = 5.4 Hz);
MS (FAB) m/z: 413 [M+H]⁺, 246, 200.

### (Example 81) 3-amino-4-[4-(3-chloro-4-fluorophenyl)-1,4-diazepan-1-yl]thieno[2,3-b]pyridine-2-carboxamide (Exemplified Compound No. 3-91)

### (81a) tert-butyl 4-(3-chloro-4-fluorophenyl)-1,4-diazepane-1-carboxylate

The reaction was performed in a similar method as described in Org.Lett.,4,581-584(2002) and the title compound was synthesized.
Colourless oil (yield 36%)
IR (film) νₘₐₓ 2976, 1693, 1508, 1417, 1237, 1168 cm⁻¹;
¹H NMR(CDCl₃, 400 MHz) δ 1.36 (4.5H, s), 1.43 (4.5H, s), 1.89-1.98 (2H, m), 3.21 (1H, t, J = 5.9 Hz), 3.32 (1H, t, J = 5.9 Hz), 3.41-3.61 (6H, m), 6.48 (1H, dt, J = 9.0, 3.3 Hz), 6.59-6.65 (1H, m), 6.96 (1H, t, J = 9.0 Hz);
MS (FAB) m/z: 328 [M⁺], 273, 229, 189.

### (81b) 1-(3-chloro-4-fluorophenyl)-1,4-diazepane

tert-butyl 4-(3-chloro-4-fluorophenyl)-1,4-diazepane-1-carboxylate which was produced in Example 81 (81a) was used in place of tert-butyl 4-phenyl-1,4-diazepane-1-carboxylate and the reaction was performed in a similar method as described in Example 57 (57b) and the title compound was obtained.
Slightly brown oil (yield 100%)
IR (film) νₘₐₓ 2934, 1611, 1508, 1240, 1047, 797 cm⁻¹;
¹H NMR(CDCl₃, 500 MHz) δ 1.88 (2H, quint, J = 5.9 Hz), 2.83 (2H, t, J = 5.9 Hz), 3.01 (2H, t, J = 5.5 Hz), 3.48 (2H, t, J = 5.5 Hz), 3.52 (2H, t, J = 5.9 Hz), 6.49 (1H, dt, J = 9.0, 3.1 Hz), 6.64 (1H, q, J = 3.1 Hz), 6.97 (1H, t, J = 9.0 Hz);
MS (EI) m/z: 228 [M⁺], 186, 172.

### (81c) (2Z)-3-[4-(3-chloro-4-fluorophenyl)-1,4-diazepan-1-yl]-2-cyanobut-2-enethioamide

1-(3-chloro-4-fluorophenyl)-1,4-diazepane which was produced in Example 81 (81b) was used in place of isobutylamine and the reaction was performed in a similar method as described in Example 5 (5a) and the title compound was synthesized.
Slightly yellow powder (yield 63%)
Mp 148-150°C;
IR (KBr) νₘₐₓ 3358, 3265, 3170, 2177, 1615, 1526, 1505, 1408 cm⁻¹;
¹H NMR(DMSO-*d₆*, 500 MHz) δ 1.89-1.96 (2H, m), 2.25 (3H, s), 3.49-3.56 (4H, m), 3.58-3.64 (2H, m), 3.68-3.75 (2H, m), 6.73 (1H, dt, J = 9.0, 3.1 Hz), 6.90 (1H, q, J = 3.1 Hz), 7.18 (1H, t, J = 9.0 Hz), 8.37 (1H, bs), 9.02 (1H, bs);
MS (FAB) m/z: 353 [M+H]⁺, 200, 165.

### (81d) (2Z)-3-[4-(3-chloro-4-fluorophenyl)-1,4-diazepan-1-yl]-2-cyano-N-[(1E)-(dimethylamino)methylene]but-2-enethioamide

(2Z)-3-[4-(3-chloro-4-fluorophenyl)-1,4-diazepan-1-yl]-2-cyanobut-2-enethioamide which was produced in Example 81 (81c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Single yellow powder (yield 45%)
Mp 133-135°C (decomposition);
IR (KBr) vₘₐₓ 2924, 2177, 1609, 1506, 1398, 1326, 1289 cm⁻¹;
¹H NMR(DMSO-*d₆*, 400 MHz) δ 1.91-2.00 (2H, m), 2.47 (3H, s), 2.91 (3H, s), 3.14 (3H, s) 3.50-3.58 (2H, m), 3.68-3.87 (6H, m), 6.73 (1H, dt, J = 9.0, 3.1 Hz), 6.90 (1H, q, J = 3.1 Hz), 7.15 (1H, t, J = 9.0 Hz), 8.43 (1H, s);
MS (FAB) m/z: 408 [M+H]⁺, 374,273.

### (81e) 3-amino-4-[4-(3-chloro-4-fluorophenyl)-1,4-diazepan-1-yl]thieno[2,3-b]pyridine-2-carboxamide

(2Z)-3-[4-(3-chloro-4-fluorophenyl)-1,4-diazepan-1-yl]-2-cyano-N-[(1E)-(dimethylamino)methylene]but-2-enethioamide which was produced in Example 81 (81d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Pale brown powder (yield 54%)
Mp 181-184°C;
IR (KBr) νₘₐₓ 3326, 3095, 2834, 1636, 1579, 1506, 1373 cm⁻¹;
¹H NMR(DMSO-*d₆*, 400 MHz) δ 2.09-2.19 (2H, d), 3.13-3.22 (2H, m), 3.24-3.32 (2H, m), 3.51 (2H, t, J = 5.9 Hz), 3.71-3.77 (2H, m), 6.71 (1H, dt, J = 9.0, 3.1 Hz), 6.85 (1H, q, J = 3.1 Hz), 6.99 (2H, bs), 7.06 (1H, d, J = 5.5 Hz), 7.09 (2H, bs), 7.18 (1H, t, J = 9.0 Hz), 8.38 (1H, d, J = 5.5 Hz);
MS (FAB) m/z: 420 [M+H]⁺, 273, 176;
Anal. Calcd for C₁₉H₁₉ClFN₅OS: C, 54.35; H, 4.65; N, 16.68; Cl, 8.44; F, 4.52; S, 7.64. Found: C, 54.04; H, 4.43; N, 16.30; Cl, 8.33; F, 4.86; S, 8.03.

### (Example 82) 3-amino-4-[4-(4-bromophenyl)-1,4-diazepan-1-yl]thieno[2,3-b]pyridine-2-carboxamide (Exemplified Compound No. 3-95)

### (82a) tert-butyl 4-(4-bromophenyl)-1,4-diazepane-1-carboxylate

The reaction was performed in a similar method as described in Org.Lett.,4,581-584(2002) and the title compound was synthesized.
Colourless oil (yield 27%)
IR (film) νₘₐₓ 2975, 1693, 1591, 1498, 1416, 1237, 1167 cm⁻¹;
¹H NMR(CDCl₃, 500 MHz) δ 1.37 (4.5H, s), 1.43 (4.5H, s), 1.90-1.99 (2H, m), 3.20 (1H, t, J = 5.9 Hz), 3.31 (1H, t, J = 5.9 Hz), 3.48-3.58 (6H, m), 6.56 (2H, d, J = 8.8 Hz), 7.27 (2H, d, J = 8.8 Hz);
MS (FAB) m/z: 355 [M+H]⁺, 255, 189.

### (82b) 1-(4-bromophenyl)-1,4-diazepane

tert-butyl 4-(4-bromophenyl)-1,4-diazepane-1-carboxylate which was produced in Example 82 (82a) was used in place of tert-butyl 4-phenyl-1,4-diazepane-1-carboxylate and the reaction was performed in a similar method as described in Example 57 (57b) and the title compound was obtained.
Slightly brown oil (yield 90%)
IR (film) νₘₐₓ 2931, 1591, 1498, 1396, 1362, 1190, 806 cm⁻¹;
¹H NMR(CDCl₃, 500 MHz) δ 1.88 (2H, quint, J = 5.9 Hz), 2.82 (2H, t, J = 5.9 Hz), 3.01 (2H, t, J = 5.4 Hz), 3.51, t, J = 5.4 Hz), 3.55 (2H, t, J = 5.9 Hz), 6.56 (2H, d, J = 8.8 Hz), 7.26 82H, d, J = 8.8 Hz);
MS (EI) m/z: 254 [M⁺], 212, 198.

### (82c) (2Z)-3-[4-(4-bromophenyl)-1,4-diazepan-1-yl]-2-cyanobut-2-enethioamide

1-(4-bromophenyl)-1,4-diazepane which was produced in Example 82 (82b) was used in place of isobutylamine and the reaction was performed in a similar method as described in Example 5 (5a) and the title compound was synthesized. Slightly yellow powder (yield 72%)
Mp 152-155°C;
IR (KBr) νₘₐₓ 3371, 3276, 3174, 2957, 2185, 1589, 1536,1496, 1408, 1357 cm⁻¹;
¹H NMR(DMSO-*d₆*, 500 MHz) δ 1.87-1.97 (2H, m), 2.24 (3H, s), 3.48-3.57 (4H, m), 3.58-3.64 (2H, m), 3.70-3.77 (2H, m), 6.74 (2H, d, J = 8.8 Hz), 7.28 (2H, d, J = 8.8 Hz), 8.38 (1H, bs), 9.03 (1H, bs);
MS (FAB) m/z: 379 [M+H]⁺, 273, 182.

### (82d) (2Z)-3-[4-(4-bromophenyl)-1,4-diazepan-1-yl]-2-cyano-N-[(1E)-(dimethylamino)methylene]but-2-enethioamide

(2Z)-3-[4-(4-bromophenyl)-1,4-diazepan-1-yl]-2-cyanobut-2-enethioamide which was produced in Example 82 (82c) was used in place of (2Z)-2-cyano-3-[4-(4-methoxyphenyl)-1,4-diazepan-1-yl]but-2-enethioamide and the reaction was performed in a similar method as described in Example 58 (58d) and the title compound was obtained.
Single yellow powder (yield 84%)
Mp 143-146°C (decomposition);
IR (KBr) νₘₐₓ 2925, 2176, 1611, 1497, 1397, 1328, 1290 cm⁻¹;
¹H NMR(DMSO-*d₆*, 400 MHz) δ 1.91-2.00 (2H, m), 2.47 (3H, s), 2.92 (3H, s), 3.15 (3H, s), 3.55 (2H, t, J = 5.9 Hz), 3.69-3.85 (6H, m), 6.74 (2H, d, J = 8.8 Hz), 7.26 (2H, d, J = 8.8 Hz), 8.46 (1H, s);
MS (FAB) m/z: 434 [M+H]⁺, 400, 273.

### (82e) 3-amino-4-[4-(4-bromophenyl)-1,4-diazepan-1-yl]thieno[2,3-b]pyridine-2-carboxamide

(2Z)-3-[4-(4-bromophenyl)-1,4-diazepan-1-yl]-2-cyano-N-[(1E)-(dimethylamino)methylene]but-2-enethioamide which was produced in Example 82 (82d) was used in place of (2Z)-2-cyano-N-[(1E)-(dimethylamino)methylene]-3-[4-(4-methoxyphenyl)-1,4-diazepan-1-yl]but-2-enethioamide and the reaction was performed in a similar method as described in Example 58 (58e) and the title compound was obtained.
Slightly brown powder (yield 59%)
Mp 170-173°C;
IR (KBr) νₘₐₓ 3442, 3322, 3180, 2948, 2838, 1645, 1588, 1498, 1367 cm⁻¹;
¹H NMR(DMSO-*d₆*, 400 MHz) δ 2.13 (2H, quint, J = 5.5 Hz), 3.13-3.21 (2H, m), 3.23-3.30 (2H, m), 3.52 (2H, t, J = 6.3 H), 3.71-3.77 (2H, m), 6.72 (2H, d, J = 9.0 Hz), 6.98 (2H, bs), 7.05 81H, d, J = 5.5 Hz), 7.08 (2H, bs), 7.27 (2H, d, J = 9.0 Hz), 8.38 (1H, d, J = 5.5 Hz);
MS (FAB) m/z: 446 [M+H]⁺, 429, 273.

### (Example 83) 3-amino-4-[4-(4-ethoxyphenyl)-1,4-diazepan-1-yl]thieno[2,3-b]pyridine-2-carboxamide (Exemplified Compound No. 3-127)

### (83a) tert-butyl 4-(4-ethoxyphenyl)-1,4-diazepane-1-carboxylate

The reaction was performed in a similar method as described in Org.Lett.,4,581-584(2002) and the title compound was synthesized.
Colourless oil (yield 27%)
IR (film) νₘₐₓ 2976, 1694, 1513, 1416, 1240,1169 cm⁻¹;
¹H NMR(CDCl₃, 500 MHz) δ 1.37 (3H, t, J = 6.8 Hz), 1.38(4.5H, s), 1.44 (4.5H, s), 1.91-2.00 (2H, m), 3.21 (1H, t, J = 5.9 Hz), 3.32 (1H, t, J = 5.9 Hz), 3.45-3.60 (6H, m), 3.96 (2H, q, J = 6.8 Hz), 6.64 (2H, d, J = 9.3 Hz), 6.81 (2H, d, J = 9.3 Hz);
MS (FAB) m/z: 320 [M⁺], 264, 219.

### (83b) 1-(4-ethoxyphenyl)-1,4-diazepane

tert-butyl 4-(4-ethoxyphenyl)-1,4-diazepane-1-carboxylate which was produced in Example 83 (83a) was used in place of tert-butyl 4-phenyl-1,4-diazepane-1-carboxylate and the reaction was performed in a similar method as described in Example 57 (57b) and the title compound was obtained.
Brown oil (yield 98%)
IR (film) νₘₐₓ 2931, 1513, 1239, 1050, 812 cm⁻¹;
¹H NMR(CDCl₃, 400 MHz) δ 1.37 (3H, t, J = 7.0 Hz), 1.88 (2H, quint, J = 5.9 Hz), 2.82 (2H, t, J = 5.5H), 3.01 (2H, t, J = 5.5 Hz), 3.46-3.55 (4H, m), 3.95 (2H, q, J = 7.0 Hz), 6.62 (2H, d, J = 9.4 Hz), 6.79 (2H, d, J = 9.4 Hz);
MS (EI) m/z: 220 [M⁺], 178, 164.

### (83c) 4-[4-(4-ethoxyphenyl)-1,4-diazepan-1-yl]-2-thioxa-1,2-dihydropyridine-3-carbonitrile

1-(4-ethoxyphenyl)-1,4-diazepane which was produced in Example 83 (83b) was used in place of 1-(4-methylphenyl)-1,4-diazepane and the reaction was performed in a similar method as described in Example 77 (77c) and the title compound was obtained.
Slightly brown powder (yield 25%)
Mp 204-208°C;
IR (KBr) νₘₐₓ 3118, 2970, 2207, 1625, 1511, 1244 cm⁻¹;
¹H NMR(CDCl₃, 400 MHz) δ 1.38 (3H, t, J = 7.0 Hz), 2.11 (2H, quint, J = 5.9 Hz), 3.50 (2H, t, J = 5.9 Hz), 3.69-3.78 (4H, m), 3.96 (2H, q, J = 7.0 Hz), 4.07-4.13 (2H, m), 6.20 (1H, d, J = 7.6 Hz), 6.66 (2H, d, J = 9.2 Hz), 6.81 (2H, d, J = 9.2 Hz), 7.22 (1H, d, J = 7.6 Hz), 11.97 (1H, bs);
MS (FAB) m/z: 355 [M+H]⁺, 273;
Anal. Calcd for C₁₉H₂₂N₄OS·0.16H₂O: C, 63.86; H, 6.38; N, 15.68; S, 8.97. Found: C, 63.86; H, 6.26; N, 15.66; S, 8.85.

### (83d) 3-amino-4-[4-(4-ethoxyphenyl)-1,4-diazepan-1-yl]thieno[2,3-b]pyridine-2-carboxamide

4-[4-(4-ethoxyphenyl)-1,4-diazepan-1-yl]-2-thioxa-1,2-dihydropyridine-3-carbonitrile which was produced in Example 83 (83c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 84%)
Mp 181-183°C;
IR (KBr) νₘₐₓ 3423, 3330, 3116, 2973, 1586, 1512, 1369, 1234 cm⁻¹;
¹H NMR(DMSO-*d₆*, 500 MHz) δ 1.28 (3H, t, J = 7.0 Hz), 2.08-2.16 (2H, m), 3.18-3.24 (2H, m), 3.26-3.31 (2H, m), 3.40 82H, t, J = 5.9 Hz), 3.70 82H, t, J = 4.7 hz), 3.92 (2H, q, J = 7.0 Hz), 6.71 (2H, d, J = 8.8 Hz), 6.80 (2H, d, J = 8.8 Hz), 6.99 (2H, bs), 7.05-7.12 (3H, m), 8.40 (1H, d, J = 5.4 Hz);
MS (FAB) m/z: 412 [M+H]⁺, 75;
Anal. Calcd for C₂₁H₂₅N₅O₂S·0.42H₂O: C, 60.19; H, 6.21; N, 16.71; S, 7.65. Found: C, 60.49; H, 6.32; N, 16.93; S, 7.35.

### (Example 84) 3-amino-4-[4-(3,4-dimethylphenyl)-1,4-diazepan-1-yl]thieno[2,3-b]pyridine-2-carboxamide (Exemplified Compound No. 3-116)

### (84a) tert-butyl 4-(3,4-dimethylphenyl)-1,4-diazepane-1-carboxylate

The reaction was performed in a similar method as described in Org. Lett., 4, 581-584 (2002) and the title compound was synthesized.
Colourless oil (yield 17%)
IR (film) νₘₐₓ 3366, 2972, 2931, 1695, 1616, 1511,1415,1242, 1169 cm⁻¹;
¹H NMR(CDCl₃, 500 MHz) δ 1.36 (4.5H, s), 1.45 (4.5H, s), 1.93-2.02 (2H, m), 2.15 (3H, s), 2.22 (3H, s), 3.20 (1H, t, J = 5.9 Hz), 3.30 (1H, d, J = 5.9 Hz), 3.47-3.59 (6H, m), 6.46 (1H, d, J = 8.3 Hz), 6.51 (1H, s), 6.96 (1H, d);
MS (FAB) m/z: 304 [M⁺], 249, 203.

### (84b) 1-(3,4-dixnethylphenyl)-1,4-diazepane

tert-butyl 4-(3,4-dimethylphenyl)-1,4-diazepane-1-carboxylate which was produced in Example 84 (84a) was used in place of tert-butyl 4-phenyl-1,4-diazepane-1-carboxylate and the reaction was performed in a similar method as described in Example 57 (57b) and the title compound was obtained.
Pale brown prism crystal (yield 100%)
IR (KBr) νₘₐₓ 2939, 2860, 2384, 1615, 1512, 1459, 1411, 1280 cm⁻¹;
¹H NMR(CDCl₃, 400 MHz) δ 1.88 (2H, quint, J = 5.9 Hz), 2.15 (3H, s), 2.21 (3H, s), 2.82 (2H, t, J = 5.9 Hz), 3.01 (2H, t, J = 5.5 Hz), 3.49-3.56 (4H, m), 6.45 (1H, dd, J = 8.2, 2.7 Hz), 6.50 (1H, d, J = 2.7 Hz), 6.94 (1H, d, J - 8.2 Hz);
MS (EI) m/z: 204 [M⁺], 174, 162, 148.

### (84c) 4-[4-(3,4-dimethylphenyl)-1,4-diazepan-1-yl]-2-thioxa-1,2-dihydropyridine-3-carbonitrile

1-(3,4-dimethylphenyl)-1,4-diazepane which was produced in Example 84 (84b) was used in place of 1-(4-methylphenyl)-1,4-diazepane and the reaction was performed in a similar method as described in Example 77 (77c) and the title compound was obtained.
Pale brown powder (yield 31 %)
Mp 251-253°C;
IR (KBr) νₘₐₓ 3121, 2937, 2205, 1625, 1511, 1459, 1254 cm⁻¹;
¹H NMR(DMSO-*d₆*, 400 MHz) δ 1.93 (2H, quint, J = 5.5 Hz), 2.07 (3H, s), 2.14 (3H, s), 3.48 (2H, t, J = 5.9 Hz), 3.65-3.74 (4H, m), 3.91-3.98 (2H, m), 6.43 (1H, d, J = 7.6 Hz), 6.50 (1H, dd, J = 8.4, 2.7 Hz), 6.60 (1H, d, J = 2.7 Hz), 6.90 (1H, d, J = 8.4 Hz), 7.37 (1H, d, J = 7.6 Hz), 12.44 (1H, bs);
MS (FAB) m/z: 339 [M+H]⁺, 273, 174;
Anal. Calcd for C₁₉H₂₂N₄S·0.24H₂O: C, 66.57; H, 6.61; N, 16.34; S, 9.35. Found: C, 66.47; H, 6.44; N, 16.34; S, 9.15.

### (84d) 3-amino-4-[4-(3,4-dimethylphenyl)-1,4-diazepan-1-yl]thieno[2,3-b]pyridine-2-carboxamide

4-[4-(3,4-dimethylphenyl)-1,4-diazepan-1-yl]-2-thioxa-1,2-dihydropyridine-3-carbonitrile which was produced in Example 84 (84c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 82%)
Mp 195-197°C;
IR (KBr) νₘₐₓ 3432, 3324, 3174, 2934, 2829, 1642, 1579, 1507, 1369 cm⁻¹;
¹H NMR(DMSO-d₆, 500 MHz) δ 2.08-2.15 (2H, m), 2.10 (3H,s), 2.16 (3H, s), 3.15-3.22 (2H, m), 3.25-3.31 (2H, m), 3.52 (2H, t, J = 5.9 Hz), 3.69-3.76 (2H, m), 6.50 (1H, dd, J = 8.3, 2.4 Hz), 6.60 (1H, d, J = 2.4 Hz), 6.92 (1H, d, J = 8.3 Hz), 6.99 (2H, bs), 7.04-7.13 (3H, m), 8.40 (1H, d, J = 5.4 Hz);
MS (FAB) m/z: 396 [M+H]⁺, 246, 185;
Anal. Calcd for C₂₁H₂₅N₅OS: C, 63.77; H, 6.37; N, 17.71; S, 8.11. Found: C, 63.45; H, 6.33; N, 17.54; S, 8.11.

### (Example 85) 3-amino-4-[4-(3,4-difluorophenyl)-1,4-diazepan-1-yl]thieno[2,3-b]pyridine-2-carboxamide (Exemplified Compound No. 3-90)

### (85a) tert-butyl 4-(3,4-difluorophenyl)-1,4-diazepane-1-carboxylate

The reaction was performed in a similar method as described in Org. Lett., 4, 581-584 (2002) and the title compound was synthesized.
Colourless oil (yield 29%)
IR (film) νₘₐₓ 2976, 1691, 1521, 1417, 1234, 1168, 777 cm⁻¹;
¹H NMR(CDCl₃, 400 MHz) δ 1.36 (4.5H, s), 1.43 (4.5H, s), 1.89-1.98 (2H, m), 3.21 (1H, t, J = 5.9 Hz), 3.31 (1H, t, J = 5.9 Hz), 3.41-3.58 (6H, m), 6.27-6.33 (1H, m), 6.38-6.47 (1H, m), 6.96 (1H, q, J = 9.0 Hz);
MS (FAB) m/z: 312 [M⁺], 257, 211.

### (85b) 1-(3,4-difluorophenyl)-1,4-diazepane

tert-butyl 4-(3,4-difluorophenyl)-1,4-diazepane-1-carboxylate which was produced in Example 85 (85a) was used in place of tert-butyl 4-phenyl-1,4-diazepane-1-carboxylate and the reaction was performed in a similar method as described in Example 57 (57b) and the title compound was obtained.
Brown oil (yield 98%)
IR (film) νₘₐₓ 2935, 2838, 1631, 1597, 1520, 1275, 777 cm⁻¹;
¹H NMR(CDCl₃, 500 MHz) δ 1.88 (2H, m, J = 5.9 Hz), 2.83 (2H, t, J = 5.9 Hz), 3.01 1 (2H, t, J = 5.4 Hz), 3.48 (2H, t, J = 5.4 Hz), 3.52 (2H, t, J = 5.9 Hz), 6.28-6.34 (1H, m), 6.44 (1H, ddd, J = 14.2, 6.8, 2.9 Hz), 6.98 (1H, q, J = 9.3 Hz);
MS (EI) m/z: 212 [M⁺], 170, 156.

### (85c) 4-[4-(3,4-difluorophenyl)-1,4-diazepan-1-yl]-2-thioxo-1,2-dihydropyridine-3-carbonitrile

1-(3,4-difluorophenyl)-1,4-diazepane which was produced in Example 85 (85b) was used in place of 1-(4-methylphenyl)-1,4-diazepane and the reaction was performed in a similar method as described in Example 77 (77c) and the title compound was obtained.
Slightly brown powder (yield 27%)
Mp 250-251°C;
IR (KBr) νₘₐₓ 3122, 2959, 2206, 1626, 1520, 1247, 777 cm⁻¹;
¹H NMR(DMSO-*d*₆, 500 MHz) δ 1.93 (2H, quint, J = 5.9 Hz), 3.51 (2H, m), 3.68-3.76 (4H, m), 3.92-3.98 (2H, m), 6.43 (1H, d, J = 7.8 Hz), 6.52-6.57 (1H, m), 6.83 (1H, ddd, J = 14.7, 6.8, 2.9 Hz), 7.17 (1H, q, J = 9.8 Hz), 7.37 (1H, d, J = 7.8 Hz), 12.15 (1H, bs);
MS (FAB) m/z: 347 [M+H]⁺, 273,242;
Anal. Calcd for C₁₇H₁₆F₂N₄S·0.2H₂O: C, 58.34; H, 4.72; F, 10.86; N, 16.01; S, 9.16. Found: C, 58.48; H, 4.73; F, 10.59; N, 16.13; S, 9.04.

### (85d) 3-amino-4-[4-(3,4-difluorophenyl)-1,4-diazepan-1-yl]thieno[2,3-b]pyridine-2-carboxamide

4-[4-(3,4-difluorophenyl)-1,4-diazepan-1-yl]-2-thioxo-1,2-dihydropyridine-3-carbonitrile which was produced in Example 85 (85c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 83%)
Mp 199-201°C;
IR (KBr) νₘₐₓ 3454, 3324, 3171, 2953, 2839, 1650, 1582, 1519, 1369 cm⁻¹;
¹H NMR(DMSO-d₆, 500 MHz) δ 2.10-2.18 (2H, m), 3.14-3.23 (2H, m), 3.24-3.32 (2H, m), 3.52 (2H, t, J = 6.4 Hz), 3.75 (2H, t, J = 5.0 Hz), 6.49-6.56 (1H, m), 6.78 (1H, ddd, J = 14.7, 6.8, 2.9 Hz), 7.00 (2H, bs), 7.08 (1H, d, J = 5.2 Hz), 7.10 (2H, bs), 7.20 (1H, q, J = 9.8 Hz), 8.41 (1H, d, J = 5.2 Hz);
MS (FAB) m/z: 404 [M+H]⁺, 387, 246, 200;
Anal. Calcd for C₁₉H₁₉F₂N₅O₂S·0.24H₂O: C, 55.96; H, 4.82; F, 9.32; N, 17.17; S, 7.86. Found: C, 55.97; H, 4.76; F, 9.25; N, 17.24; S, 7.76.

### (Example 86) 3-amino-4-{4-[3-(trifluoromethyl)phenyl]-1,4-diazepan-1-yl}thieno[2,3-b]pyridine-2-carboxamide (Exemplified Compound No. 3-120)

### (86a) tert-butyl 4-[3-(trifluoromethyl)phenyl]-1,4-diazepane-1-carboxylate

The reaction was performed in a similar method as described in Org.Lett.,4,581-584(2002) and the title compound was synthesized.
Colourless oil (yield 13%)
IR (film) νₘₐₓ 2976, 1695, 1612, 1463, 1417, 1321, 1164, 1124 cm⁻¹;
¹H NMR(CDCl₃, 500 MHz) δ 1.33 (4.5H, s), 1.43 (4.5H, s), 1.93-2.01 (2H, m), 3.23 81H, t, J = 5.9 Hz), 3.34 (1H, t, J = 5.9 Hz), 3.54-3.64 (6H, m), 6.80-6.94 (3H, m), 7:23-7.32 (1H, m);
MS (FAB) m/z: 344 [M⁺], 289, 243.

### (86b) 1-[3-(trifluoromethyl)phenyl]-1,4-diazepane

tert-butyl 4-[3-(trifluoromethyl)phenyl]-1,4-diazepane-1-carboxylate which was produced in Example 86 (86a) was used in place of tert-butyl 4-phenyl-1,4-diazepane-1-carboxylate and the reaction was performed in a similar method as described in Example 57 (57b) and the title compound was obtained.
Brown oil (yield 89%)
IR (film) νₘₐₓ 2938, 1691, 1611, 1506, 1457, 1321, 1162, 1121 cm⁻¹;
¹H NMR(CDCl₃, 500 MHz) δ 1.91 (2H, t, J = 5.9 Hz), 2.84 (2H, t, J = 5.9 Hz), 3.04 (2H, t, J = 5.4 Hz), 3.57 (2H, t, J = 5.4 Hz), 3.60 (2H, t, J = 6.4 Hz), 6.80-6.91 (3H, m), 7.25-7.31 (1H, m);
MS (EI) m/z: 244 [M⁺], 202, 188.

### (86c) 2-thioxo-4-{4-[3-(trifluoromethyl)phenyl]-1,4-diazepan-1-yl}-1,2-dihydropyridine-3-carbonitrile

1-[3-(trifluoromethyl)phenyl]-1,4-diazepane which was produced in Example 86 (86b) was used in place of 1-(4-methylphenyl)-1,4-diazepane and the reaction was performed in a similar method as described in Example 77 (77c) and the title compound was obtained.
Slightly brown powder (yield 27%)
Mp 247-248°C;
IR (KBr) νₘₐₓ 2414, 3129, 2964, 2207, 1625, 1523, 1320, 1120 cm⁻¹;
¹H NMR(DMSO-*d₆*, 500 MHz) δ 1.92-2.00 (2H, m), 3.60 (2H, t, J = 5.9 Hz), 3.75 (2H, t, J = 5.9 hz), 3.80 (2H, t, J = 5.4 Hz), 3.08 (2H, t, J = 5.4 Hz), 6.45 (1H, d, J = 7.8 Hz), 6.90 (1H, d, J = 7.3 Hz), 6.99 (1H, s), 7.07 (1H, dd, J = 8.3, 2.0 Hz), 7.33-7.40 (2H, m), 11.53 (1H, bs);
MS (FAB) m/z: 379 [M+H]⁺, 273, 226;
Anal. Calcd for C₁₈H₁₇F₃N₄S·0.2H₂O: C, 56.69; H, 4.59; N, 14.67; S, 8.39. Found: C, 56.60; H, 4.58; N, 14.67; S, 8.39.

### (86d) 3-amino-4-{4-[3-(trifluoromethyl)phenyl]-1,4-diazepan-1-yl}thieno[2,3-b]pyridine-2-carboxamide

2-thioxo-4-{4-[3-(trifluoromethyl)phenyl]-1,4-diazepan-1-yl}-1,2-dihydropyridine-3-carbonitrile which was produced in Example 86 (86c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 84%)
Mp 195-197°C;
IR (KBr) νₘₐₓ 3427, 3326, 3167, 1639, 1580, 1504, 1371, 1121 cm⁻¹;
¹H NMR(DMSO-*d₆*, 500 MHz) δ 2.14-2.23 (2H, m), 3.15-3.25 (2H, m), 3.26-3.36 (2H, m), 3.60 (2H, t, J = 5.9 Hz), 3.84 (2H, t, J = 4.6 Hz), 6.91 (1H, d, J = 7.3 Hz), 6.95-6.99 (1H, m), 7.01 (1H, bs), 7.06 (1H, dd, J = 8.3, 2.0 Hz), 7.09 (1H, d, J = 5.2 Hz), 7.10 (1H, bs), 7.38 81H, t, J = 8.3 Hz), 8.41 (1H, d, J = 5.2 Hz);
MS (FAB) m/z: 436 [M+H]⁺, 419, 240.

### (Example 87) 3-amino-4-{4-[4-(trifluoromethoxy)phenyl]-1,4-diazepan-1-yl}thieno[2,3-b]pyridine-2-carboxamide (Exemplified Compound No. 3-130)

### (87a) tert-butyl 4-[4-(trifluoromethoxy)phenyl]-1,4-diazepane-1-carboxylate

The reaction was performed in a similar method as described in Org. Lett., 4, 581-584 (2002) and the title compound was synthesized.
Slightly brown oil (yield 41 %)
IR (film) νₘₐₓ 2976, 1692, 1516, 1417, 1268, 1163 cm⁻¹;
¹H NMR(CDCl₃, 400 MHz) δ 1.33 (4.5H, s), 1.42 (4.5H, s), 1.90-1.98 (2H, m), 3.22 (1H, t, J = 5.9 Hz), 3.32 (1H, t, J = 5.5 Hz), 3.48-3.60 (6H, m), 6.61 (2H, d, J = 9.2 Hz), 7.04 (2H, d, J - 9.2 Hz);
MS (FAB) m/z: 360 [M⁺], 305, 259.

### (87b) 1-[4-(trifluoromethoxy)phenyl]-1,4-diazepane

tert-butyl 4-[4-(trifluoromethoxy)phenyl]-1,4-diazepane-1-carboxylate which was produced in Example 87 (87a) was used in place of tert-butyl 4-phenyl-1,4-diazepane-1-carboxylate and the reaction was performed in a similar method as described in Example 57 (57b) and the title compound was obtained.
Green oil (yield 57%)
IR (film) νₘₐₓ 2936, 1610, 1516, 1265, 1231, 1205, 1156, 805 cm⁻¹;
¹H NMR(CDCl₃, 500 MHz) δ 1.89 (2H, quint, J = 5.9 Hz), 2.83 (2H, t, J = 5.9 Hz), 3.03 (2H, t, J = 5.4 Hz), 3.53 (2H, t, J = 5.4 Hz), 3.56 (2H, t, J = 5.9 Hz), 6.63 (2H, d, J = 9.3 Hz), 7.05 (2H, d, J = 9.3 Hz);
MS (EI) m/z: 260 [M⁺], 218, 204.

### (87c) 2-thioxa-4-{4-[4-(trifluoromethoxy)phenyl]-1,4-diazepan-1-yl}-1,2-dihydropyridine-3-carbonitrile

1-[4-(trifluoromethoxy)phenyl]-1,4-diazepane was used in place of 1-(4-methylphenyl)-1,4-diazepane which was produced in Example 87 (87b) and the reaction was performed in a similar method as described in Example 77 (77c) and the title compound was obtained.
Slightly brown powder (yield 19%)
Mp 240-242°C;
IR (KBr) νₘₐₓ 3122, 2957, 2210, 1627, 1514, 1245, 1150 cm⁻¹;
¹H NMR(DMSO-*d*₆, 500 MHz) δ 1.91-1.98 (2H, m), 3.55 (2H, t, J = 5.9 Hz), 3.70-3.78 (4H, m), 3.97 (2H, t, J = 5.4 Hz), 6.44 (1H, d, J = 7.8 Hz), 6.83 (2H, d, J = 9.3 Hz), 7.13 (2H, d, J = 9.3 Hz), 7.37 (1H, d, J = 7.8 Hz), 12.53 (1H, bs);
MS (FAB) m/z: 395 [M+H]⁺, 175;
Anal. Calcd for C₁₈H₁₇F₃N₄OS: C, 54.81; H, 4.34; N, 14.21; S, 8.13. Found: C, 54.74; H, 4.06; N, 14.12; S, 8.08.

### (87d) 3-amino-4-{4-[4-(trifluoromethoxy)phenyl]-1,4-diazepan-1-yl}thieno[2,3-b]pyridine-2-carboxamide

2-thioxa-4-{4-[4-(trifluoromethoxy)phenyl]-1,4-diazepan-1-yl}-1,2-dihydropyridine-3-carbonitrile which was produced in Example 87 (87c) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 79%)
Mp 163-165°C;
IR (KBr) νₘₐₓ 3413, 3325, 3176, 2947, 2836, 1637, 1579, 1515, 1372 cm⁻¹;
¹H NMR(DMSO-*d₆*, 500 MHz) δ 2.11-2.19 (2H, m), 3.16-3.24 (2H, m), 3.26-3.33 (2H, m), 3.55 (2H, t, J = 5.9 Hz), 3.79 (2H, t, J = 4.6 Hz), 6.82 (2H, d, J = 9.3 Hz), 7.00 (2H, bs), 7.08 (1H, d, J = 5.4 Hz), 7.10 (2H, bs), 7.15 (2H, d, J = 9.3 Hz), 8.40 (1H, d, J = 5.4 Hz);
MS (FAB) m/z: 452 [M+H]⁺, 435,216;
Anal. Calcd for C₂₀H₂₀F₃N₅O₂S: C, 53.21; H, 4.47; F, 12.62; N, 15.51; S, 7.10. Found: C, 53.06; H, 4.13; F, 12.53; N, 15.44; S, 6.93.

### (Example 88) 3-amino-4-[4-(2,3,5,6-tetrafluoropyridin-4-yl)-1,4-diazepan-1-yl]thieno[2,3-b]pyridine-2-carboxamide (Exemplified Compound No. 3-145)

### (88a) tert-butyl 4-(2,3,5,6-tetrafluoropyfidin-4-yl)-1,4-diazepane-1-carboxylate

Pentafluoropyridine (878 µL, 8 mmol), 1,4-tert-butyl diazepane-1-carboxylate (1,577 µL, 8 mmol) and triethylamine (1,227 µL, 8.8 mmol) were dissolved in methylene chloride (40 mL) and the mixture was stirred at room temperature for one hour. The reaction liquid was blended with a saturated sodium hydrogen carbonate aqueous solution (50 mL) and partitioned and the aqueous layer was extracted with methylene chloride (2x25 mL) and then the organic layer was combined and the solvent was evaporated under reduced pressure after drying over anhydrous sodium sulfate. Residual substance was purified by silica gel column chromatography (eluent: hexane/ethyl acetate =5/1) and 2.54 g (91%) of the title compound was obtained.
White powder
Mp 55-59°C;
IR (KBr) νₘₐₓ 2985, 1690, 1636, 1523, 1476, 1170, 1143 cm⁻¹;
¹H NMR(CDCl₃, 400 MHz) δ 1.44 (4.5H, s), 1.46 (4.5H, s), 1.90-2.03 (1H, m), 3.47 (1H, m, J = 5.9 Hz, 3.53)1H, t, J = 5.9 Hz), 3.71-3.56 (6H, m);
MS (FAB) m/z: 350 [M+H]⁺, 294, 250.

### (88b) 1-(2,3,5,6-tetrafluoropyridin-4-yl)-1,4-diazepane

tert-butyl 4-(2,3,5,6-tetrafluoropyridin-4-yl)-1,4-diazepane-1-carboxylate which was produced in Example 88 (88a) was used in place of tert-butyl 4-phenyl-1,4-diazepane-1-carboxylate and the reaction was performed in a similar method as described in Example 57 (57b) and the title compound was obtained.
White powder (yield 97%)
Mp 58-61°C;
IR (KBr) νₘₐₓ 3356, 2932, 2852, 1638, 1534, 1473, 1126, 1068, 932 cm⁻¹;
¹H NMR(CDCl₃, 400 MHz) δ 1.92 (2H, quint, J = 5.9 Hz), 2.96 (2H, t, J = 5.9 Hz), 3.05 (2H, t, J = 5.5 Hz), 3.62-3.71 (6H, m);
MS (EI) m/z: 249 [M⁺], 207, 193.

### (88c) 4-[4-(2,3,5,6-tetrafluoropyridin-4-yl)-1,4-diazepan-1-yl]-2-thioxa-1,2-dihydropyridine-3-carbonitrile

1-(2,3,5,6-tetrafluoropyridin-4-yl)-1,4-diazepane which was produced in Example 88 (88b) was used in place of 1-(4-methylphenyl)-1,4-diazepane and the reaction was performed in a similar method as described in Example 77 (77c) and the title compound was obtained.
Pale brown powder (yield 22%)
Mp 267-269°C;
IR (KBr) νₘₐₓ 3121, 2961, 2210, 1628, 1522, 1471, 1250, 1135, 962 cm⁻¹;
¹H NMR(DMSO-*d₆*, 400 MHz) δ 1.97-2.08 (2H, m), 3.58-3.66 (2H, m), 3.76-3.85 (2H, m), 3.89-3.96 (2H, m), 3.97-4.05 (2H, m), 6.50 (1H, d, J = 7.6 Hz), 7.42 (1H, d, J = 7.6 Hz), 12.51 (1H, bs);
MS (FAB) m/z: 384 [M+H]⁺, 200;
Anal. Calcd for C₁₆H₁₃F₄N₅S: C, 50.13; H, 3.42; N, 18.27; S, 8.36. Found: C, 50.08; H, 3.66; N, 18.35; S, 8.37.

### (88d) 3-amino-4-[4-(2,3,5,6-tetrafluoropyridin-4-yl)-1,4-diazepan-1-yl]thieno[2,3-b]pyridine-2-carboxamide

4-[4-(2,3,5,6-tetrafluoropyridin-4-yl)-1,4-diazepan-1-yl]-2-thioxa-1,2-dihydropyridine-3-carbonitrile which was produced in Example 88 (88c) was used in place of 4-(isobutylamino)-2-thioxo-1,2-dihydropyridine-3-carbonitrile and the reaction was performed in a similar method as described in Example 5 (5c) and the title compound was obtained.
Slightly yellow powder (yield 85%)
Mp 228-231 °C;
IR (KBr) νₘₐₓ 3441, 3325, 3175, 2924, 1641, 1583, 1469, 1371, 1121, 962 cm⁻¹;
¹H NMR(DMSO-*d₆*, 500 MHz) δ 2.14-2.23 (2H, m), 3.26-3.35 (2H, m), 3.39-3.48 (2H, m), 3.75-3.82 (2H, m), 3.87-3.94 (2H, m), 7.04 (2H, bs), 7.10 (1H, d, J = 5.4 Hz), 7.11 (2H, bs), 8.44 (1H, d, J = 5.4 Hz);
MS (FAB) m/z: 441 [M+H]⁺, 329, 273;
Anal. Calcd for C₁₈H₁₆F₄N₆OS: C, 49.09; H, 3.66; F, 17.25; N, 19.08; S, 7.28. Found: C, 49.02; H, 3.72; F, 16.85; N, 19.13; S, 7.21.

### (Example 89) 3-amino-4-(4-quinazolin-4-yl-1,4-diazepan-1-yl)thieno[2,3-b]pyridine-2-carboxamide (Exemplified Compound No. 3-147)

### (89a) tert-butyl 4-quinazolin-4-yl-1,4-diazepane-1-carboxylate

4-chloroquinazoline (Helv. Chim. Acta, (2001), 84,1112-1118) was used in place of pentafluoropyridine and the reaction was performed in a similar method as described in Example 88 (88a) and the title compound was obtained.
Colourless oil (yield 91 %)
Mp 55-59°C;
IR (film) νₘₐₓ 2975, 2929, 2869, 1693, 1566, 1506, 1346, 1167 cm⁻¹;
¹H NMR(CDCl₃, 400 MHz) δ 1.34 (4.5H, s), 1.42 (4.5H, s), 2.03-2.21 (2H, m), 3.47 (1H, t, J = 5.9 Hz), 3.56 (1H, t, J = 5.5 Hz), 3.65-3.77 (2H, m), 3.90-4.10 (4H, m), 7.41 (1H, t, J = 8.2 Hz), 7.71 (1H, t, J = 8.2 Hz), 7.86 (1H, d, J = 8.2 Hz), 7.95 (1H, d, J = 8.2 Hz), 8.64 (1H,s);
MS (FAB) m/z: 329 [M+H]⁺, 273.

### (89b) 4-(1,4-diazepan-1-yl) quinazoline

tert-butyl 4-quinazolin-4-yl-1,4-diazepane-1-carboxylate which was produced in Example 89 (89a) was used in place of tert-butyl 4-phenyl-1,4-diazepane-1-carboxylate and the reaction was performed in a similar method as described in Example 57 (57b) and the title compound was obtained.
Slightly brown oil (yield 76%)
IR (film) νₘₐₓ 3296, 2937, 1684, 1613, 1567, 1505, 1346 cm⁻¹;
¹H NMR(CDCl₃, 400 MHz) δ 2.03 (2H, m), 2.96-3.02 (2H, m), 3.17-3.22 (2H, m), 3.98-4.04 (4H, m), 7.33-7.39 (1H, m), 7.64-7.70 (1H, m), 7.80-7.84 (1H, m), 7.92-7.97 (1H, m), 8.60 (1H,s);
MS (EI) m/z: 228 [M⁺], 185, 172, 159.

### (89c) 4-(4-quinazolin-4-yl-1,4-diazepan-1-yl)-2-thioxo-1,2-dihydropyridine-3-carbonitrile

4-(1,4-diazepan-1-yl)quinazoline produced in Example 89 (89b) was used in place of 1-(4-methylphenyl)-1,4-diazepane and the reaction was performed in a similar method as described in Example 77 (77c) and the title compound was obtained.
Brown powder (yield 19%)
Mp 221-223°C;
IR (KBr) νₘₐₓ 3431, 3111, 2926, 2204, 1623, 1566, 1504, 1344, 1242 cm⁻¹;
¹H NMR(DMSO-*d*₆, 400 MHz) δ 2.14-2.22 (2H, m), 3.93 (2H, t, J = 5.9 Hz), 3.98 (2H, t, J = 5.5 Hz), 4.13 (4H, bs), 6.47 (1H, d, J = 7.4 Hz), 7.35 (1H, d, J = 8.2 Hz), 7.45-7.52 (1H, m), 7.70-7.79 (2H, m), 8.02 (1H, d, J = 8.2 Hz), 8.50 (1H, s),12.49 (1H, bs);
MS (FAB) m/z: 363 [M+H]⁺, 257, 229;
Anal. Calcd for C₁₉H₁₈N₆S: C, 60.09; H, 5.29; N, 22.13. Found: C, 60.18; H, 5.38; N, 22.09.

### (89d) 3-amino-4-(4-quinazolin-4-yl-1,4-diazepan-1-yl)thieno[2,3-b]pyridine-2-carboxamide

4-(4-quinazolin-4-yl-1,4-diazepan-1-yl)-2-thioxo-1,2-dihydropyridine-3-carbonitrile which was produced in Example 89 (89c) was used in place of 4-(isobutylamino)-2-thioxo-1,2-dihydropyridine-3-carbonitrile and the reaction was performed in a similar method as described in Example 5 (5c) and the title compound was obtained.
Slightly yellow powder (yield 85%)
Mp 228-231°C;
IR (KBr) νₘₐₓ 3439, 3321, 3181, 2957, 1648, 1567, 1501, 1344 cm⁻¹;
¹H NMR(DMSO-*d*₆, 400 MHz) δ 2.24-2.36 (2H, m), 3.17-3.30 (2H, m), 3.50-3.59 (2H, m), 4.03-4.13 (2H, m), 4.18-4.29 (2H, m), 7.00 (2H, bs), 7.06 (1H, d, J = 5.1 Hz), 7.10 (2H, bs), 7.46 (1H, ddd, J = 8.6, 6.0, 2,3 Hz), 7.70-7.78 (2H, m), 8.10 (1H, d, J = 8.2 Hz), 8.39 (1H, d, J = 5.1 Hz), 8.50 (1H, s);
MS (FAB) m/z: 420 [M+H]⁺, 273, 246, 200.

### (Example 90) 3-amino-4-[4-(4-methyl-3-nitrophenyl)-1,4-diazepan-1-yl]thieno[2,3-2b]pyridine-2-carboxamide (Exemplified Compound No. 3-119)

### (90a) tert-butyl 4-(4-methyl-3-nitrophenyl)-1,4-diazepane-1-carboxylate

The reaction was performed in a similar method as described in Org. Lett., 4, 581-584 (2002) and the title compound was synthesized.
Orange powder (yield 41 %)
Mp 103-106°C;
IR (KBr) νₘₐₓ 2978, 1685, 1528, 1415, 1334 cm⁻¹;
¹H NMR (CDCl₃, 500 MHz) δ 1.35 (4.5H, s), 1.42 (4.5H, s), 1.93-2.00 (2H, m), 2.45 (3H, s), 3.23 (1H, t, J = 5.9 Hz), 3.33 (1H, t, J = 5.9 Hz), 3.53-3.62 (6H, m), 6.82 (1H, dd, J = 8.8, 2.9 Hz, 7.13 (1H, d, J = 8.8 Hz, 7.26 (1H, d, J = 2.9 Hz);
MS (FAB) m/z: 336 [M+H]⁺, 280, 234, 189;
Anal. Calcd for C₁₇H₂₅N₃O₄·0.3H₂O: C, 59.91; H, 7.57; N, 12.33. Found: C, 59.94; H, 7.29; N, 12.01.

### (90b) 1-(4-methyl-3-nitrophenyl)-1,4-diazepane

tert-butyl 4-(4-methyl-3-nitrophenyl)-1,4-diazepane-1-carboxylate which was produced in Example 90 (90a) was used in place of tert-butyl 4-phenyl-1,4-diazepane-1-carboxylate and the reaction was performed in a similar method as described in Example 57 (57b) and the title compound was obtained.
Red oil (yield 100%)
IR (liquid) vₘₐₓ 2930, 1628, 1527, 1346 cm⁻¹;
¹H NMR(CDCl₃, 500 MHz) δ 1.91 (2H, t, J = 5.9 Hz), 2.45 (3H, s), 2.83 (2H, t, J = 5.9 Hz), 3.04 (2H, t, J = 5.5 Hz), 3.55 (2H, t, J = 5.5 Hz), 3.59 (2H, t, J = 5.9 Hz), 6.81 (1H, dd, J = 8.3, 2.9 Hz), 7.12 (1H, d, J = 8.3 Hz), 7.26 (1H, d, J = 2.9 Hz);
MS (EI) m/z: 235 [M⁺], 193, 179.

### (90c) 4-[4-(4-methyl-3-nitrophenyl)-1,4-diazepan-1-yl]-2-thioxo-1,2-dihydropyridine-3-carbonitrile

1-(4-methyl-3-nitrophenyl)-1,4-diazepane which was produced in Example 90 (90b) was used in place of 1-(4-methylphenyl)-1,4-diazepane and the reaction was performed in a similar method as described in Example 77 (77c) and the title compound was obtained.
Brown powder (yield 31 %)
Mp 277-278°C;
IR (KBr) νₘₐₓ 3437, 3124, 2961, 2206, 1625, 1525, 1341, 1252 cm⁻¹;
¹H NMR(DMSO-*d₆*, 400 MHz) δ 1.94 (2H, m), 2.32 (3H, s), 3.57 (2H, t, J = 5.9 Hz), 3.70-3.81 (4H, m), 3.92-3.99 (2H, m), 6.42 (1H, d, J = 7.8 Hz), 7.05 (1H, dd, J = 8.6, 2.7 Hz), 7.22 (1H, d, J = 8.6 Hz), 7.24 (1H, d, J = 2.7 Hz), 7.35 (1H, d, J = 7.8 Hz), 12.47 (1H, bs);
MS (FAB) m/z: 370 [M+H]⁺, 259, 242;
Anal. Calcd for C₁₈H₁₉N₅O₂S·0.26H₂O: C, 57.79; H, 5.26; N, 18.72; S, 8.57. Found: C, 57.78; H, 5.16; N, 18.73; S, 8.51.

### (90d) 3-amino-4-[4-(4-methyl-3-nitrophenyl)-1,4-diazepan-1-yl]thieno[2,3-b]pyridine-2-carboxamide

4-[4-(4-methyl-3-nitrophenyl)-1,4-diazepan-1-yl]-2-thioxo-1,2-dihydropyridine-3-carbonitrile which was produced in Example 90 (90c) was used in place of 4-(isobutylamino)-2-thioxo-1,2-dihydropyridine-3-carbonitrile and the reaction was performed in a similar method as described in Example 5 (5c) and the title compound was obtained.
Orange powder (yield 91%)
Mp 114-117°C;
IR (KBr) νₘₐₓ 3439, 3323, 3181, 2927, 2846, 1650, 1579, 1525, 1366 cm⁻¹;
¹H NMR(DMSO-*d₆*, 500 MHz) δ 2.13-2.21 (2H, m), 2.36 (3H,s), 3.14-3.23 (2H, m), 3.27-3.34 (2H, m), 3.59 (2H, t, J = 5.9 Hz), 3.81 (2H, t, J = 4.8 Hz), 7.01 (2H, bs), 7.0-7.15 84H, m), 7.24-7.29 (2H, m), 8.41 (1H, d, J = 5.4 Hz);
MS (FAB) m/z: 427 [M+H]⁺, 410, 273, 246;
Anal. Calcd for C₂₀H₂₂N₆O₃S·0.5H₂O: C, 55.16; H, 5.32; N, 19.30. Found: C, 54.87; H, 5.49; N, 19.58.

### (Example 91) 3-amino-4-[4-(4-isopropoxyphenyl)-1,4-diazepan-1-yl]thieno[2,3-b]pyridine-2-carboxamide (Exemplified Compound No. 3-129)

### (91a) tert-butyl 4-(4-isopropoxyphenyl)-1,4-diazepane-1-carboxylate

The reaction was performed in a similar method as described in Org. Lett., 4, 581-584 (2002) and the title compound was synthesized.
Colourless oil (yield 14%)
IR (film) νₘₐₓ 2974, 1694, 1511, 1415, 1366, 1237, 1168, 1122 cm⁻¹;
¹H NMR(CDCl₃, 400 MHz) δ 1.28 (6H, d, J = 5.9 Hz), 1.37 (4.5H, s), 1.43 (4.5H, s), 1.90-2.00 (2H, m), 3.20 (2H, t, J = 5.9 Hz), 3.31 (2H, t, J = 5.5 Hz), 3.40-3.61 (6H, m), 4.35 (1H, sept, J = 5.9 Hz), 6.61 (2H, d, J = 9.0 Hz), 6.78 (2H, d, J = 9.0 Hz); MS (EI) m/z: 334 [M⁺], 278, 236.

### (91b) 1-(4-isopropoxy phenyl)-1,4-diazepane

tert-butyl 4-(4-isopropoxy phenyl)-1,4-diazepane-1-carboxylate which was produced in Example 91 (91 a) was used in place of tert-butyl 4-phenyl-1,4-diazepane-1-carboxylate and the reaction was performed in a similar method as described in Example 57 (57b) and the title compound was obtained.
Slightly orange prism crystal (yield 89%)
Mp 62-64°C;
IR (film) νₘₐₓ 2974, 2932, 1511, 1237, 1114, 957, 815 cm⁻¹;
¹H NMR(CDCl₃, 400 MHz) δ 1.29 (6H, d, J = 5.9 Hz), 1.88 (2H, quint, J = 5.9 Hz), 2.83 (2H, t, J = 5.9 Hz), 3.01 (2H, t, J = 5.5 Hz), 3.49 (2H, t, J = 5.5 Hz), 3.52 (2H, t, J = 5.9 Hz), 4.35 (1H, sept, J = 5.9 Hz), 6.61 (2H, d, J = 9.0 Hz), 6.79 (2H, d, J = 9.0 Hz);
MS (EI) m/z: 234 [M⁺], 191, 178.

### (91c) 4-[4-(4-isopropoxyphenyl)-1,4-diazepan-1-yl]-2-thioxo-1,2-dihydropyridine-3-carbonitrile

1-(4-isopropoxy phenyl)-1,4-diazepane which was produced in Example 91 (91b) was used in place of 1-(4-methylphenyl)-1,4-diazepane and the reaction was performed in a similar method as described in Example 77 (77c) and the title compound was obtained.
Slightly brown powder (yield 32%)
Mp 216-218°C;
IR (KBr) νₘₐₓ 3440, 3128, 3046, 2974, 2205, 1625, 1511, 1511, 1241 cm⁻¹;
¹H NMR(CDCl₃, 500 MHz) δ 1.30 (6H, d, J = 5.9 Hz), 2.11 (2H, quint, J = 5.9 Hz), 3.51 (2H, t, J = 5.9 Hz), 3.73 (2H, t, J = 5.4 Hz), 3.76 (2H, t, J = 5.9 Hz), 4.12 (2H, t, J = 5.4 Hz), 4.39 (1H, sept, J = 5.9 Hz), 6.21 (1H, d, J = 7.8 Hz), 6.67 (2H, d, J = 8.8 Hz), 6.82 (2H, d, J = 8.8 Hz), 7.23 (1H, d, J = 7.8 Hz), 1184 (1H, bs);
MS (EI) m/z: 368 [M⁺], 325, 148;
Anal. Calcd for C₂₀H₂₄N₄OS·0.15H₂O: C, 64.71; H, 6.60; N, 15.09; S, 8.64. Found: C, 64.59; H, 6.46; N, 15.09; S, 8.47.

### (91d) 3-amino-4-[4-(4-isopropoxyphenyl)-1,4-diazepan-1-yl]thieno[2,3-b]pyridine-2-carboxamide

4-[4-(4-isopropoxyphenyl)-1,4-diazepan-1-yl]-2-thioxo-1,2-dihydropyridine-3-carbonitrile which was produced in Example 91 (91c) was used in place of 4-(isobutylamino)-2-thioxo-1,2-dihydropyridine-3-carbonitrile and the reaction was performed in a similar method as described in Example 5 (5c) and the title compound was obtained.
Slightly yellow powder 79%
Mp 173-175°C;
IR (KBr) νₘₐₓ 3441, 3324, 2973, 1644, 1579, 1510, 1370, 1235 cm⁻¹;
¹H NMR(DMSO-*d₆*, 400 MHz) δ 1.21 (6H, d, J = 5.9 Hz), 2.07-2.16 (2H, m), 3.16-3.24 (2H, m), 3.25-3.33 (2H, m), 3.47 (2H, t, J = 5.9 Hz), 3.69 (2H, t, J = 4.7 Hz), 4.37 (1H, sept, J = 5.9 Hz), 6.68 (2H, d, J = 9.0 Hz), 6.76 (2H, d, J = 9.0 Hz), 6.97 (2H, bs), 7.03-7.11 (3H, m), 8.37 (1H, d, J = 5.5 Hz);
MS (EI) m/z: 425 [M⁺], 382, 275, 183;
Anal. Calcd for C₂₂H₂₇N₅O₂S: C, 62.09; H, 6.40; N, 16.46; S, 7.54. Found: C, 61.83; H, 6.23; N, 16.32; S, 7.38.

### (Example 92) 3-amino-4-[4-(4-tert-butylphenyl)-1,4-diazepan-1-yl]thieno[2,3-b]pyridine-2-carboxamide (Exemplified Compound No. 3-115)

### (92a) tert-butyl 4-(4-tert-butylphenyl)-1,4-diazepane-1-carboxylate

The reaction was performed in a similar method as described in Org.Lett.,4,581-584(2002) and the title compound was synthesized.
White powder (yield 9%)
Mp 62-64°C;
IR (KBr) νₘₐₓ 2962, 1686, 1520, 1420, 1364, 1246, 1170 cm⁻¹;
¹H NMR(CDCl₃, 400 MHz) δ 1.27 (9H, s), 1.35 (4.5H, s), 1.43 (4.5H, s), 1.91-2.00 (1H, m), 3.21 (1H, t, J = 6.3 Hz), 3.32 (1H, t, J = 5.9 Hz), 3.48-3.59 (6H, m), 6.62 (2H, d, J = 8.8 Hz), 7.20 (2H, d, J = 8.8 Hz);
MS (EI) m/z: 332 [M⁺], 276, 261.

### (92b) 1-(4-tert-butylphenyl)-1,4-diazepane

tert-butyl 4-(4-tert-butylphenyl)-1,4-diazepane-1-carboxylate which was produced in Example 92 (92a) was used in place of tert-butyl 4-phenyl-1,4-diazepane-1-carboxylate and the reaction was performed in a similar method as described in Example 57 (57b) and the title compound was obtained.
Pale brown oil (yield 97%)
IR (film) νₘₐₓ 2959, 1614, 1520, 1363, 1201, 812, 552 cm⁻¹;
¹H NMR(CDCl₃, 400 MHz) δ 1.28 (9H, s), 1.89 (2H, quint, J = 5.9 Hz), 2.84 (2H, t, J = 5.9 Hz), 3.02 (2H, t, J = 5.5 Hz), 3.52 (2H, t, J = 5.5 Hz), 3.54 (2H, t, J = 5.9 Hz), 6.63 (2H, d, J = 8.4 Hz), 7.21 (2H, d, J = 8.4 Hz);
MS (EI) m/z: 232 [M⁺], 217, 190, 176.

### (92c) 4-[4-(4-tert-butylphenyl)-1,4-diazepan-1-yl]-2-thioxo-1,2-dihydropyridine-3-carbonitrile

1-(4-tert-butylphenyl)-1,4-diazepane which was produced in Example 92 (92b) was used in place of 1-(4-methylphenyl)-1,4-diazepane and the reaction was performed in a similar method as described in Example 77 (77c) and the title compound was obtained.
Pale brown powder (yield 21 %)
Mp 257-258°C;
IR (KBr) νₘₐₓ 3121, 3041, 2958, 2205, 1625, 1519, 1459, 1247 cm⁻¹;
¹H NMR(CDCl₃, 400 MHz) δ 1.28 (9H, s), 2.12 (2H, quint, J = 5.9 Hz), 3.56 (2H, t,
J = 5.9 Hz), 3.71-3.81 (4H, m), 4.13 (2H, t, J = 5.1 Hz), 6.20 (1H, d, J = 7.6 Hz), 6.66 (2H, d, J = 8.6 Hz), 7.22 (1H, d, J = 7.6 Hz), 7.24 (2H, d, J = 8.6 Hz), 11.94 (1H, bs); MS (EI) m/z: 366 [M⁺], 351, 188.

### (92d) 3-amino-4-[4-(4-tert-butylphenyl)-1,4-diazepan-1-yl]thieno[2,3-b]pyridine-2-carboxamide

4-[4-(4-tert-butylphenyl)-1,4-diazepan-1-yl]-2-thioxo-1,2-dihydropyridine-3-carbonitrile which was produced in Example 92 (92c) was used in place of 4-(isobutylamino)-2-thioxo-1,2-dihydropyridine-3-carbonitrile and the reaction was performed in a similar method as described in Example 5 (5c) and the title compound was obtained.
Slightly yellow powder (yield 72%)
Mp 231-232°C;
IR (KBr) νₘₐₓ 3440, 3324, 3182, 2957, 1645, 1579, 1579, 1519, 1364 cm⁻¹;
¹H NMR(DMSO-*d₆*, 400 MHz) δ 1.21 (9H, s), 2.06-2.17 (2H, m), 3.22-3.16 (4H, m), 6.69 (2H, d, J = 9.0), 6.89 (2H, bs), 7.03-7.09 (3H, m), 7.17 (2H, d, J = 9.0 Hz), 8.38 (1H, d, J = 5.5 Hz);
MS (EI) m/z: 423 [M⁺], 391, 275;
Anal. Calcd for C₂₃H₂₉NₛOS·0.27H₂O: C, 64.48; H, 6.95; N, 16.35; S, 7.48. Found: C, 64.17; H, 6.64; N, 16.31; S, 7.43.

### (Example 93) 3-amino-4-[4-(4-ethylphenyl)-1,4-diazepan-1-yl]thieno[2,3-b]pyridine-2-carboxamide (Exemplified Compound No. 3-112)

### (93a) tert-butyl 4-(4-ethylphenyl)-1,4-diazepane-1-carboxylate

The reaction was performed in a similar method as described in Org.Lett.,4,581-584(2002) and the title compound was synthesized.
Colourless oil (yield 11 %)
IR (film) νₘₐₓ 2964, 1695, 1519, 1415, 1236, 1169 cm⁻¹;
¹H NMR(CDCl₃, 400 MHz) δ 1.19 (3H, t, J = 7.6 Hz), 1.37 (4.5H, s), 1.44 (4.5H, s), 1.92-2.01 (2H, m), 2.53 (2H, q, J = 7.6 Hz), 3.19 (1H, t, J = 6.3 Hz), 3.30 (1H, t, J = 5.9 Hz), 3.47-3.59 (6H, m), 6.62 (2H, d, J = 8.4 Hz), 7.02 (2H, d, J = 8.4 Hz);
MS (FAB) m/z: 304 [M⁺], 248, 160.

### (93b) 1-(4-ethylphenyl)-1,4-diazepane

tert-butyl 4-(4-ethylphenyl)-1,4-diazepane-1-carboxylate which was produced in Example 93 (93a) was used in place of tert-butyl 4-phenyl-1,4-diazepane-1-carboxylate and the reaction was performed in a similar method as described in Example 57 (57b) and the title compound was obtained.
Pale brown oil (yield 100%)
IR (film) νₘₐₓ 2929, 1616, 1519, 1189, 813 cm⁻¹;
¹H NMR(CDCl₃, 400 MHz) δ 1.19 (3H, t, J = 7.6 Hz), 1.88 (2H, quint, J = 5.9 Hz), 2.54 (2H, q, J = 7.6 Hz), 2.82 (2H, t, J = 5.9 Hz), 3.01 (2H, t, J - 5.5 Hz), 3.49-3.57 (2H, m), 6.62 (2H, d, J = 8.8 Hz), 7.03 (2H, d, J = 8.8 Hz);
MS (EI) m/z: 204 [M⁺], 162, 148.

### (93c) 4-[4-(4-ethylphenyl)-1,4-diazepan-1-yl]-2-thioxo-1,2-dihydropyridine-3-carbonitrile

1-(4-ethylphenyl)-1,4-diazepane which was produced in Example 93 (93b) was used in place of 1-(4-methylphenyl)-1,4-diazepane and the reaction was performed in a similar method as described in Example 77 (77c) and the title compound was obtained.
Slightly brown powder (yield 22%)
Mp 226-229°C;
IR (KBr) νₘₐₓ 3122, 2959, 2205, 1625, 1517, 1457, 1246 cm⁻¹;
¹H NMR(CDCl₃, 400 MHz) δ 1.20 (2H, t, J = 7.6 Hz), 2.11 (2H, quint, J = 5.9 Hz), 2.55 (2H, q, J = 7.6 Hz), 3.55 (2H, t, J = 5.9 Hz), 3.74 (2H, t, J = 5.9 Hz), 3.77 (2H, t, J = 5.1 Hz), 4.12 (2H, t, J = 5.1 Hz), 6.19 (1H, d, J = 7.6 Hz), 6.65 (2H, d, J = 8.6 Hz), 7.06 (2H, d, J = 8.6 Hz), 7.20 (1H, d, J = 7.6 Hz), 11.58 (1H, bs);
MS (EI) m/z: 338 [M⁺], 323, 174, 160.

### (93d) 3-amino-4-[4-(4-ethylphenyl)-1,4-diazepan-1-yl]thieno[2,3-b]pyridine-2-carboxamide

4-[4-(4-ethylphenyl)-1,4-diazepan-1-yl]-2-thioxo-1,2-dihydropyridine-3-carbonitrile which was produced in Example 93 (93c) was used in place of 4-(isobutylamino)-2-thioxo-1,2-dihydropyridine-3-carbonitrile, and the reaction was performed in a similar method as described in Example 5 (5c) and the title compound was obtained.
White powder (yield 82%)
Mp 186-188°C;
IR (KBr) νₘₐₓ 3439, 3324, 3178, 2958, 1643, 1579, 1518, 1369 cm⁻¹;
¹H NMR(DMSO-*d₆*, 400 MHz) δ 1.14 (3H, t, J = 7.4 Hz), 2.07-2.17 (2H, m), 2.49 (2H, q, J = 7.4 Hz), 3.15-3.35 (4H, m), 3.53 (2H, t, J = 5.9 Hz), 3.74 (2H, t, J = 4.7 Hz), 6.70 (2H, d, J = 8.4 Hz), 6.96 (1H, bs), 7.02 (2H, d, J = 8.4 Hz), 7.04-7.14 (3H, m), 8.40 (1H, d, J = 5.5 Hz);
MS (EI) m/z: 395 [M⁺], 377, 189;
Anal. Calcd for C₂₁H₂₅N₅OS: C, 63.77; H, 6.37; N, 17.71; S, 8.11. Found: C, 63.56; H, 6.25; N, 17.68; S, 8.10.

### (Example 94) 4-[4-(4-acetylphenyl)-1,4-diazepan-1-yl]-3-aminothieno[2,3-b]pyridine-2-carboxamide (Exemplified Compound No. 3-99)

### (94a) tert-butyl 4-(4-acetylphenyl)-1,4-diazepane-1-carboxylate

4-fluoro acetophenone was used in place of 4-fluoronitrobenzene and the reaction was performed in a similar method as described in Example 59 (59a) and the title compound was obtained.
Yellow liquid
IR (film) νₘₐₓ 2974, 1596, 1523, 1416, 1363, 1284, 1237, 1191, 929, 819, 756 cm⁻¹;
¹H NMR(CDCl₃, 400 MHz) δ 1.36 (4.5H, s), 1.42 (4.5H, s), 1.94-2.01 (2H, m), 2.50 (1H, s), 3.21 (1H, t, J = 5.9 Hz), 3.32 (1H, t, J = 5.9 Hz), 3.58-3.64 (6H, m), 6.66 (2H, d, J = 8.6 Hz), 7.83 (2H, t, J = 8.6 Hz);
HRMS m/z calcd for C₁₈H₂₆O₃N₂ 318.1944, found 318.1934;
MS (EI) m/z: 318 [M⁺], 261, 247, 217, 188, 174, 162, 132, 105, 91, 57, 41.

### (94b) 1-[4-(1,4-diazepan-1-yl)phenyl]ethanone

tert-butyl 4-(4-acetylphenyl)-1,4-diazepane-1-carboxylate which was produced in Example 94 (94a) was used in place of tert-butyl 4-phenyl-1,4-diazepane-1-carboxylate and the reaction was performed in a similar method as described in Example 57 (57b) and the title compound was obtained.
Brown liquid
IR (KBr) νₘₐₓ 2931, 1658, 1597, 1523, 1403, 1360, 1285, 1191, 820 cm⁻¹;
¹H NMR(CDCl₃, 500 MHz) δ 1.91 (2H, quint, J = 5.9 Hz), 2.50 (3H, s), 2.83 (2H, t, J = 5.9 Hz), 3.04 (2H, t, J = 5.4 Hz), 3.61 (2H, t, J = 5.4 Hz), 3.66 (2H, t, J = 6.4 Hz), 6.68 (2H, d, J = 8.8 Hz), 7.85 (2H, d, J = 8.8 Hz);
HRMS m/z calcd for C₁₃H₁₈ON₂ 218.1420.1313, found 218.1422;
MS (EI) m/z: 218 [M⁺], 203, 176, 162, 148, 132, 70, 43.

### (94c) (2Z)-3-[4-(4-acetylphenyl)-1,4-diazepan-1-yl]-2-cyanobut-2-enethioamide

1-[4-(1,4-diazepan-1-yl)phenyl]ethanone which was produced in Example 94 (94b) was used in place of isobutylamine and the reaction was performed in a similar method as described in Example 5 (5a) and the title compound was obtained.
Yellow powder
Mp 173-177°C;
IR (KBr) νₘₐₓ 3310, 3178, 2183, 1593, 1524, 1407, 1354, 1289, 1194, 825, 594 cm⁻¹; ¹H NMR(DMSO-d₆, 400 MHz) δ 1.89-1.96 (2H, m), 2.23 (3H, s), 2.42 (3H, s), 3.49-3.54 (2H, m), 3.60-3.66 (4H, m), 3.83-3.86 (2H, m), 6.81 (2H, d, J = 9.0 Hz), 7.76 (2H, d, J = 9.0 Hz), 7.16 (2H, t, J = 7.3 Hz), 8.39 (1H, brs), 9.04 (1H, brs);
HRMS m/z calcd for C₁₈H₂₃ON₄S 343.1592, found 343.1595;
MS (FAB) m/z: 343 [M+H]⁺, 326, 273, 246, 219, 165, 120, 65;
Anal. Calcd for C₁₈H₂₂N₄OS·0.40H₂O: C, 61.83; H, 6.57; N, 16.02; S, 9.17. Found: C, 61.52; H, 6.35; N, 15.90, S, 9.52.

### (94d) 4-[(4-acetylphenyl)-1,4-diazepan-1-yl]-2-thioxo-1,2-dihydropyridine-3-carbonitrile

(2Z)-3-[4-(4-acetylphenyl)-1,4-diazepan-1-yl]-2-cyanobut-2-enethioamide which was produced in Example 94 (94c) (504 mg, 1.47 mmol) and N,N-dimethylformamide dimethylacetal (175 mg, 1.47 mmol) were dissolved in N,N-dimethylformamide (2 mL) and the mixture was stirred at 60°C for 80 minutes. After 1N hydrochloric acid was added to make the reaction solution slightly acidic, ethyl acetate (10 mL) and water (20 mL) were added. The deposited solid was separated by filtration and the title compound was obtained (518 mg, 38%).
Pale brown powder
Mp 265-270°C;
IR (KBr) νₘₐₓ 2940, 2206, 1625, 1593, 1521, 1355, 1288, 1248, 1238, 1192, 1143, 929, 817 cm⁻¹;
¹H NMR(DMSO-d₆, 500 MHz) δ 1.91-1.97 (2H, m), 2.42 (3H, s), 3.64 (2H, t, J = 5.9 Hz), 3.76 (2H, t, J = 5.9 Hz), 3.86 (2H, t, J = 5.4 Hz), 3.98 (2H, t, J = 5.4 Hz), 6.44 (1H, d, J = 7.8 Hz), 6.85 (1H, d, J = 8.8 Hz), 7.87 (1H, t, J = 7.8 Hz), 7.77 (2H, t, J = 8.8 Hz), 12.57 (1H, brs);
HRMS m/z calcd for C₁₉H₂₁ON₄S 353.1436, found 353.1425;
MS (EI) m/z: 352 [M⁺], 337, 323, 218, 204, 174, 162, 132, 105, 91, 77, 43;
Anal. Calcd for C₁₉H₂₀N₄OS·0.33H₂O: C, 63.66; H, 5.81; N, 15.63; S, 8.95. Found: C, 63.58; H, 5.80; N, 15.63, S, 8.74.

### (94e) 4-[4-(4-acetylphenyl)-1,4-diazepan-1-yl]-3-aminothieno[2,3-b]pyridine-2-carboxamide

4-[(4-acetylphenyl)-1,4-diazepan-1-yl]-2-thioxo-1,2-dihydropyridine-3-carbonitrile which was produced in Example 94 (94d) was used in place of 4-(isobutylamino)-2-thioxo-1,2-dihydropyridine-3-carbonitrile and the reaction was performed in a similar method as described in Example 5 (5c), and the title compound was obtained.
Pale brown powder
Mp 279-282°C (decomposition);
IR (KBr) vₘₐₓ 3439, 3326, 3183, 1653, 1595, 1364, 1279, 1193, 939, 820 cm⁻¹;
¹H NMR(DMSO-d₆, 500 MHz) δ 2.16-2.18 (2H, m), 2.44 (3H, s), 3.19-3.20 (2H, m), 3.30-3.32 (2H, m), 3.66 (2H, t, J = 6.3 Hz), 3.87 (2H, t, J = 4.9 Hz), 6.84 (2H, d, J = 8.8 Hz), 6.99 (2H, brs), 7.08 (1H, d, J = 5.4 Hz), 8.10 (2H, brs), 7.80 (2H, d, J = 8.8 Hz), 8.41 (1H, d, J = 5.4 Hz);
MS (EI) m/z: 409 [M⁺].

### (Example 95) 3-amino-4-[3-methyl-4-(3-methylphenyl)piperazin-1-yl]thieno[2,3-b]pyridine-2-carboxamide (Exemplified Compound No. 3-77)

### (95a) 4-[3-methyl-4-(3-methylphenyl)piperazin-1-yl]-2-thioxo-1,2-dihydropyridine-3-carbonitrile

2-methyl-1-(3-methylphenyl) piperazine was used in place of 1-(4-methylphenyl)-1,4-diazepane and the reaction was performed in a similar method as described in Example 77 (77c) and the title compound was obtained.
Pale brown powder (yield 23%)
Mp 250-251 °C;
IR (KBr) νₘₐₓ 3122, 3039, 2970, 2208, 1621, 1496, 1447, 1248, 1011 cm⁻¹ ;
¹H NMR(DMSO-*d₆*, 400 MHz) δ 0.99 (3H, d, J = 6.3 Hz), 2.25 (3H, s), 3.14-3.23 (1H, m), 3.37-3.52 (2H, m), 3.67 (1H, dd, J = 13.3, 3.5 Hz), 3.91-3.99 (1H, m), 4.01-4.15 82H, m), 6.53 (2H, d, J = 7.6 Hz), 6.58 (1H, d, J = 7.4 Hz), 6.65-6.71 (2H, m), 7.09 (1H, t, J = 7.4 Hz), 7.49 (1H, d, J = 7.6 Hz);
MS (FAB) m/z: 325 [M+H]⁺, 200;
Anal. Calcd for C₁₈H₂₀N₄S·0.32H₂O: C, 65.47; H, 6.30; N, 16.97; S, 9.71. Found: C, 65.24; H, 6.38; N, 17.19; S, 9.78.

### (95b) 3-amino-4-[3-methyl-4-(3-methylphenyl)piperazin-1-yl]thieno[2,3-b]pyridine-2-carboxamide

4-[3-methyl-4-(3-methylphenyl)piperazin-1-yl]-2-thioxo-1,2-dihydropyridine-3-carbonitrile which was produced in Example 95 (95a) was used in place of 4-(isobutylamino)-2-thioxo-1,2-dihydropyridine-3-carbonitrile and the reaction was performed in a similar method as described in Example 5 (5c) and the title compound was obtained.
Slightly brown powder (yield 81 %)
Mp 215-217°C;
IR (KBr) νₘₐₓ 3449, 3325, 3179, 2972, 2836, 1644, 1580, 1501, 1370 cm⁻¹;
¹H NMR(DMSO-*d₆*, 400 MHz) δ 0.99-1.14 (3H, m), 2.27 (3H, s), 2.62-3.51 (7H, m), 6.58-6.69 (1H, m), 6.74-6.85 (2H, m), 6.96 (2H, bs), 7.06-7.19 (4H, m), 8.46 (1H, d, J = 5.5 Hz);
MS (EI) m/z: 382 [M+H]⁺, 200;
Anal. Calcd for C₂₀H₂₃N₅OS·0.16: C, 62.50; H, 6.12; N, 18.22; S, 8.34. Found: C, 62.49; H, 6.19; N, 18.15; S, 8.16.

### (Example 96) 3-amino-4-(4-pyridin-3-yl-1,4-diazepan-1-yl)thieno[2,3-b]pyridine-2-carboxamide (Exemplified Compound No. 3-140)

### (96a) (2Z)-2-cyano-3-(4-pyridin-3-yl-1,4-diazepan-1-yl)but-2-enethioamide

1-pyridin-3-yl-1,4-diazepane (J. Med. Chem. (2000), 43, 2217-2226) was used in place of isobutylamine and the reaction was performed in a similar method as described in Example 5 (5a) and the title compound was obtained. Yield 83%.
Mp 162-164°C;
IR (KBr) νₘₐₓ 3300, 3164, 2187, 1583, 1530, 796, 707 cm⁻¹;
¹H NMR (DMSO-d₆, 400MHz) δ 1.91-1.97 (2H, m), 2.24 (3H, s), 3.51-3.63 (6H, m), 3.76-3.79 (2H, m), 7.11-7.15 (2H, m), 7.82-7.84 (1H, m), 8.14 (1H, brs), 8.35 (1H, br), 9.00 (1H, br);
MS (FAB) m/z: 302 [M+H]⁺;
Anal. Calcd for C₁₅H₁₉N₅S·0.1 H₂O: C, 59.42; H, 6.38; N, 23.10; S, 10.57. Found: C, 59.53; H, 6.48; N, 22.88; S, 10.57.

### (96b) 3-amino-4-(4-pyridin-3-yl-1,4-diazepan-1-yl)thieno[2,3-b]pyridine-2-carboxamide

(2Z)-2-cyano-3-(4-pyridin-3-yl-1,4-diazepan-1-yl)but-2-enethioamide which was produced in Example 96 (96a) (485 mg, 1.61 mmol) and N,N-dimethylformamide dimethylacetal (211 mg, 1.77 mmol) were dissolved in N,N-dimethylformamide (3 mL) and the mixture was stirred at room temperature for one hour. Furthermore, it was stirred from 100°C to 120°C for 1.5 hours. The reaction solution was cooled to room temperature and 8N aqueous solution of sodium hydroxide (0.3 mL) and 2-chloroacetamide (196 mg, 2.09 mmol) were added. Water (3 mL) was added after the mixture was stirred at room temperature for three hours. The deposited solid was separated by filtration and washed with water and ethanol and 239 mg of a solid was obtained. The obtained solid was recrystallized from ethanol (2 mL) and 195 mg of the title compound was obtained (yield 33%).
Mp 214-216°C;
IR (KBr) νₘₐₓ 3444, 3324, 3166, 1650, 1579, 1495, 1368, 794, 709 cm⁻¹;
¹H NMR (DMSO-d₆, 400MHz) δ 2.14-2.19 (2H, m), 3.16-3.22 (2H, m), 3.27-3.81 (2H, m), 3.55-3.58 (2H, m), 3.79-3.81 (2H, m), 6.97 (2H, brs), 7.07 (1H, d, J = 5.5 Hz), 7.09 (2H, brs), 7.10-7.16 (2H, m), 7.83 (1H, dd, J = 2.0, 4.3 Hz), 8.16 (1H, d, J = 2.0 Hz), 8.35 (1H, d, J = 5.5 Hz);
MS (FAB) m/z: 369 [M+H]⁺.

### (Example 97) 3-amino-4-[4-(1,3-thiazol-2-yl)-1,4-diazepan-1-yl]thieno[2,3-b]pyridine-2-carboxamide (Exemplified Compound No. 3-136)

### (97a) 1-(1,3-thiazol-2-yl)-1,4-diazepane dihydrochloride

2-bromothiazole (8.20 g, 50 mmol) and homopiperazine (10.00 g, 100 mmol) were stirred in N-butanol (100 mL) under heat reflux for 24 hours. The reaction mixture was allowed to stand at room temperature for 24 hours and the deposited solid was separated by filtration. 1N aqueous solution of sodium hydroxide (30 mL) was added to the residue which was obtained by concentrating the filtrate under reduced pressure and the aqueous layer was extracted with methylene chloride (3x100 mL). The extract was concentrated after drying over sodium sulfate under reduced pressure. The residue was dissolved in methanol (30 mL) and 4N hydrochloric acid-1,4-dioxane solution (30 mL) was added. The deposited hydrochloride was separated by filtration and washed with 1,4-dioxane and acetone and 9.43 g (yield 74%) of the title compound was obtained.
IR (neat) νₘₐₓ 1603, 1581, 743 cm⁻¹ ;
¹H NMR (CDCl₃, 400MHz) δ 2.13-2.19 (2H, m), 3.18-3.25 (2H, m), 3.30-3.36 (2H, m), 3.64-3.67 (2H, m), 3.96-3.99 (2H, m), 6.98 (1H, d, J = 3.9 Hz), 7.35 (1H, d, J = 3.9 Hz);
MS (EI) m/z: 183 [M⁺], 83;
Anal. Calcd for C₈H₁₃N₃S·2(HCl)·0.1(H₂O): C, 37.24; H, 5.94; N, 16.29; S, 12.43; Cl, 27.48. Found: C, 37.33; H, 5.88; N, 16.24; S, 12.37; Cl, 27.27.

### (97b) 1-(1,3-thiazol-2-yl)-1,4-diazepane

After 1-(1,3-thiazol-2-yl)-1,4-diazepane dihydrochloride (5.11 g, 19.9 mmol) which was produced in Example 97 (97a) was mixed with 1N aqueous solution of sodium hydroxide (60 mL), the mixture was extracted with methylene chloride (3x50 mL). The extract was dried over sodium sulfate and concentrated under reduced pressure and 3.60 g (yield 99%) of the title compound was obtained.
IR (neat) νₘₐₓ 3304, 1534, 1139, 614 cm⁻¹;
¹H NMR (CDCl₃, 400MHz) δ 1.90-1.96 (2H, m), 2.89-2.91 (2H, m), 3.04-3.07 (2H, m), 3.66-3.70 (4H, m), 6.44 (1H, d, J = 3.5 Hz), 7.15 (1H, d, J = 3.5 Hz);
MS (EI) m/z: 183 [M⁺], 83;
Anal. Calcd for C₈H₁₃N₃S: C, 52.43; H, 7.15; N, 22.93; S, 17.50. Found: C, 52.15; H, 7.46; N, 22.66; S, 10.50.

### (97c) (2Z)-2-cyano-3-[4-(1,3-thiazol-2-yl)-1,4-diazepan-1-yl]but-2-enethioamide

1-(1,3-thiazol-2-yl)-1,4-diazepane which was produced in Example 97 (97b) was used in place of isobutylamine and the reaction was performed in a similar method as described in Example 5 (5a) and the title compound was obtained (4.56 g).
Yield 91 %.
Mp 149-152°C;
IR (KBr) νₘₐₓ 3276, 3130, 2182, 1531, 885, 615 cm⁻¹;
¹H NMR (DMSO-d₆, 400MHz) δ 1.95-2.00 (2H, m), 2.27 (3H, s), 3.57-3.69 (6H, m), 3.80-3.84 (2H, m), 6.77 (1H, d, J = 3.5 Hz), 7.12 (1H, d, J = 3.5 Hz), 8.42 (1H, br), 9.06 (1H, br);
MS (FAB) m/z: 308 [M+H]⁺;
Anal. Calcd for C₁₃H₁₇N₅S₂: C, 50.79; H, 5.57; N, 22.78; S, 20.86. Found: C, 50.52; H, 5.64; N, 22.44; S, 21.10.

### (97d) 3-amino-4-[4-(1,3-thiazol-2-yl)-1,4-diazepan-1-yl]thieno[2,3-b]pyridine-2-carboxamide

(2Z)-2-cyano-3-[4-(1,3-thiazol-2-yl)-1,4-diazepan-1-yl]but-2-enethioamide (394 mg, 1.28 mmol) which was produced in Example 97 (97c) and N,N-dimethylformamide dimethylacetal (168 mg, 1.41 mmol) were dissolved in N,N-dimethylformamide (2 mL) and the mixture was stirred at room temperature for one hour. Furthermore, it was stirred at 100°C for one hour. The reaction solution was cooled to room temperature and 8N aqueous solution of sodium hydroxide (0.3 mL) and 2-chloroacetamide (156 mg, 1.66 mmol) were added. Water (2 mL) was added after the mixture was stirred at room temperature overnight. The deposited solid was separated by filtration and 215 mg of a solid was obtained. The obtained solid was recrystallized from 95% ethanol (15 mL) and 150 mg of the title compound was obtained (yield 31 %).
Mp 243-245°C;
IR (KBr) νₘₐₓ 3432, 3310, 3154, 1648, 1580, 1509, 1375, 933, 614 cm⁻¹;
¹H NMR (DMSO-d₆, 400MHz) δ 2.48-2.50 (2H, m), 3.19-3.37 (4H, m), 3.62 (2H, t, J = 5 9 Hz), 3.93-3.95 (2H, m), 6.73 (1H, d, J = 3.5 Hz), 7.00 (2H, brs), 7.06 (1H, d, J = 5.5 Hz), 7.10 (2H, brs), 7.13 (1H, d, J = 3.5 Hz), 8.39 (1H, d, J = 3.5 Hz);
MS (EI) m/z: 374 [M⁺];
Anal. Calcd for C₁₆H₁₈N₆OS₂·1.1 H₂O: C, 48.74; H, 5.16; N, 21.31; S, 16.26. Found: C, 48.98; H, 5.03; N, 21.22; S, 15.89.

### (Example 98) 3-amino-4-[4-(6-methoxypyridin-3-yl)-1,4-diazepan-1-yl]thieno[2,3-b]pyridine-2-carboxamide (Exemplified Compound No. 3-144)

### (98a) (2Z)-2-cyano-3-[4-(6-methoxypyridin-3-yl)-1,4-diazepan-1-yl]but-2-enethioamide

1-(6-methoxypyridin-3-yl)-1,4-diazepane (J. Med.Chem. (2000), 43,2217-2226) was used in place of isobutylamine and the reaction was performed in a similar method as described in Example 5 (5a) and the title compound was obtained. Yield 66%.
Mp 159-161°C;
IR (KBr) νₘₐₓ 3306, 3186, 2187, 1643, 1530, 1504, 1041 cm⁻¹;
¹H NMR (DMSO-d₆, 400MHz) δ 1.90-1.97 (2H, m), 2.25 (3H, s), 3.44-3.45 (4H, m), 3.60-3.68 (4H, m), 3.74 (3H, s), 6.66 (1H, d, J = 9.0 Hz), 7.28 (1H, dd, J = 3.1, 9.0 Hz), 7.67 (1H, d, J = 3.1 Hz), 8.31 (1H, br), 8.97 (1H, br);
MS (EI) m/z: 331 [M⁺];
Anal. Calcd for C₁₆H₂₁N₅SO·0.1 H₂O: C, 57.67; H, 6.41; N, 21.02; S, 9.62. Found: C, 57.74; H, 6.50; N, 20.84; S, 9.49.

### (98b) 3-amino-4-[4-(6-methoxypyridin-3-yl)-1,4-diazepan-1-yl]thieno[2,3-b]pyridine-2-carboxamide

(2Z)-2-cyano-3-[4-(6-methoxypyridin-3-yl)-1,4-diazepan-1-yl]but-2-enethioamide which was produced in Example 98 (98a) (220 mg, 0.66 mmol) and N,N-dimethylformamide dimethylacetal (83 mg, 0.70 mmol) were dissolved in N,N-dimethylformamide (2 mL) and the mixture was stirred at room temperature for two hours. Furthermore, it was stirred at 100°C for one hour. The reaction solution was cooled to room temperature and 8N aqueous solution of sodium hydroxide (0.2 mL) and 2-chloroacetamide (80 mg, 0.86 mmol) were added. The mixture was partitioned with water (50 mL) and ethyl acetate (50 mL) after stirring at room temperature for one hour. The organic layer was washed with a saturated saline solution (30 mL) and the solvent was evaporated under reduced pressure after drying over sodium sulfate. The residue was purified by silica gel chromatography (100% ethyl acetate) and was recrystallized from ethanol and 96 mg of the title compound was obtained (yield 36%).
Mp 170-172°C;
IR (KBr) νₘₐₓ 3440, 3323, 3183, 1645, 1579, 1499, 1370, 1033, 939, 819 cm⁻¹;
¹H NMR (DMSO-d₆, 400MHz) δ 2.10-2.16 (2H, m), 3.17-3.31 (4H, m), 3.49-3.52 (2H, m), 3.70-3.72 (2H, m), 3.75 (3H, s), 6.67 (1H, d, J = 9.0 Hz), 6.98 (2H, br), 7.06 (1H, d, J = 5.5 Hz), 7.07 (2H, br), 7.27 (1H, dd, J = 3.1, 9.0 Hz), 7.67 (1H, d, J = 3.1 Hz), 8.3 (1H, d, J = 5.5 Hz);
MS (FAB) m/z: 399 [M+H]⁺;
Anal. Calcd for C₁₉H₂₂N₆SO₂·0.4 H₂O: C, 56.25; H, 5.66; N, 20.72; S, 7.90. Found: C, 55.97; H, 5.44; N, 21.11; S, 7.86.

### (Example 99) 3-amino-4-(4-pyridin-2-yl-1,4-diazepan-1-yl)thieno[2,3-b]pyridine-2-carboxamide (Exemplified Compound No. 3-139)

### (99a) tert-butyl 2-pyridin-2-yl-1,4-diazepane-1-carboxylate

Synthesis was performed with reference to a method described in J. Org. Chem. (2001), 66, 7729-7737 as follows. Potassium tert-butoxide (1.68 g, 15 mmol), tris(dibenzylideneacetone)dipalladium (0 valent) (92 mg, 0.1 mmol), 1,3-bis(2,6-diisopropylphenyl)glyoxalin-2-ylidene) hydrochloride (85 mg, 0.2 mmol) were mixed in 1,4-dioxane (20 mL), and the mixture was added dropwise to 1,4-dioxane (10 mL) solution of 2-bromopyridine (2.37 g, 15 mmol) and N-Boc-homopiperazine (2.00 g, 10 mmol). After stirring at room temperature for 12 hours, water (100 mL) and ethyl acetate (100 mL) were added to the reaction mixture and insolubles were removed with Celite. After the liquid was partitioned into the organic layer and aqueous layer, the organic layer was washed with a saturated saline solution (50 mL) and dried over sodium sulfate. The residue which was obtained by evaporating the solvent under reduced pressure was purified by silica gel column chromatography (hexane/ethyl acetate =4:1) and 2.27 g of the title compound was obtained (yield 82%).
IR (neat) νₘₐₓ 1694, 1597, 1494, 1240, 1169, 928, 770 cm⁻¹;
¹H NMR (DMSO-d₆, 400MHz) δ 1.38, (4.5H, s), 1.43, (4.5H, s), 1.92-1.99 (2H, m), 3.21-3.34 (2H, m), 3.54-3.78 (6H, m), 6.47-6.52 (2H, m), 7.38-7.42 (1H, m), 8.10-8.12 (1H, m);
MS (FAB) m/z: 278 [M+H]⁺.

### (99b) 1-pyridin-2-yl-1,4-diazepane

tert-butyl 2-pyridin-2-yl-1,4-diazepane-1-carboxylate (2.17 g, 7.8 mmol) which was produced in Example 99 (99a) was dissolved in methylene chloride (8 mL) and trifluoroacetic acid (8 mL) was added under ice-cooling. The reaction mixture was concentrated under reduced pressure after stirring at room temperature for three hours. 1N aqueous solution of sodium hydroxide (30 mL) was added to the residue and the aqueous layer was extracted with methylene chloride (3x40 mL). The extract was dried over sodium sulfate and the solvent was evaporated under reduced pressure and 1.34 g (yield 97%) of the title compound was obtained as oil. IR (neat) νₘₐₓ 3308, 1597, 1496, 1440, 769 cm⁻¹;
¹H NMR (CDCl₃, 400MHz) δ 1.86-1.92 (2H, m), 2.83-2.86 (2H, m), 3.01-3.04 (2H, m), 3.69-3.74 (4H, m), 6.46-6.50 (2H, m), 7.40 (1H, ddd, J = 2.0, 7.1, 8.6 Hz), 8.12 (1H, ddd, J = 0.8, 2.0, 5.1 Hz);
MS (EI) m/z: 177 [M⁺], 121.

### (99c) (2Z)-2-cyano-3-(4-pyridin-2-yl-1,4-diazepan-1-yl)but-2-enethioamide

1-pyridin-2-yl-1,4-diazepane which was produced in Example 99 (99b) was used in place of isobutylamine and the reaction was performed in a similar method as described in Example 5 (5a) and the title compound was obtained. Yield 79%.
Mp 155-157°C;
IR (KBr) νₘₐₓ 3398, 3288, 3184, 2185, 1598, 1540, 1494, 1439, 773 cm⁻¹
¹H NMR (DMSO-d₆, 400MHz) δ 1.88-1.94 (2H, m), 2.24 (3H, s), 3.55-3.70 (6H, m), 3.89-3.92 (2H, m), 6.57 (1H, dd, J = 5.0, 7.0 Hz), 6.72 (1H, d, J = 8.6 Hz), 7.50 (1H, ddd, J = 2.0, 7.0, 8.6 Hz), 8.07 (1H, dd, J = 2.0, 5.0 Hz), 8.34 (1H, br), 9.01 (1H, br); MS (FAB) m/z: 302 [M+H]⁺.

### (99d) 4-(4-pyridin-2-yl-1,4-diazepan-1-yl)-2-thioxo-1,2-dihydropyridine-3-carbonitrile

(2Z)-2-cyano-3-(4-pyridin-2-yl-1,4-diazepan-1-yl)but-2-enethioamide (0.90 g, 3.0 mmol) which was produced in Example 99 (99c) and N,N-dimethylformamide dimethylacetal (0.39 g, 3.3 mmol) were dissolved in N,N-dimethylformamide (5 mL) and the mixture was stirred at room temperature for one hour and further at 100°C for one hour. The reaction solution was cooled to room temperature and partitioned with isopropyl ether (20 mL) and 1N aqueous solution of sodium hydroxide (10 mL). The aqueous layer was made acidic (pH =4) with 1N hydrochloric acid and was neutralized by adding a saturated sodium bicarbonate aqueous solution. The deposited crystal was separated by filtration and washed with water and ethanol and a crude product of the title compound (0.37 g) was obtained.
Mp 220-224°C;
¹H NMR (DMSO-d₆, 400MHz) δ 1.88-1.94 (2H, m), 3.63-3.66 (2H, m), 3.76-3.79 (2H, m), 3.88-3.98 (4H, m), 6.41 (1H, d, J = 7.8 Hz), 6.53 (1H, dd, J = 5.0, 7.0 Hz), 6.71 (1H, d, J = 8.6 Hz), 7.81-7.35 (1H, m), 7.46 (1H, ddd, J = 2.0, 7.0, 8.6 Hz), 8.03 (1H, dd, J = 2.0, 5.0 Hz), 12.50 (1H, br).

### (99e) 3-amino-4-(4-pyridin-2-yl-1,4-diazepan-1-yl)thieno[2,3-b]pyridine-2-carboxamide

A crude product (0.37 g) of 4-(4-pyridin-2-yl-1,4-diazepan-1-yl)-2-thioxo-1,2-dihydropyridine-3-carbonitrile which was produced in Example 99 (99d) was dissolved in N,N-dimethylformamide (4 mL) and 8N aqueous solution of sodium hydroxide (0.4 mL) and 2-chloroacetamide (0.14 g, 1.5 mmol) were added. Water (4 mL) was added after the mixture was stirred at room temperature for one hour.

The deposited solid was separated by filtration and washed with water and ethanol and 0.33 g of a solid was obtained. The obtained solid was heated in 80% ethanol (5 mL) and the mixture was stirred, and after it was cooled, the solid was separated by filtration and 0.30 g of the title compound was obtained. Yield 27% from (2Z)-2-cyano-3-(4-pyridin-2-yl-1,4-diazepan-1-yl)but-2-enethioamide.
Mp 237-239°C;
IR (KBr) νₘₐₓ 3444, 3325, 3167, 1650, 1596, 1496, 1371, 942, 770 cm⁻¹;
¹H NMR (DMSO-d₆, 400MHz) δ 2.11-2.17 (2H, m), 3.17-3.32 (4H, m), 3.70 (2H, t, J = 6.3 Hz), 3.96-4.03 (2H, m), 6.57 (1H, dd, J = 5.1, 7.0 Hz), 6.69 (1H, d, J = 8.6 Hz), 7.01 (2H, brs), 7.07 (1H, d, J = 5.1 Hz), 7.10 (2H, brs), 7.51 (1H, ddd, J = 2.0, 7.0, 8.6 Hz), 8.09 (1H, dd, J = 2.0, 5.1 Hz), 8.39 (1H, d, J = 5.1 Hz);
MS (FAB) m/z: 369 [M+H]⁺;
Anal. Calcd for C₁₈H₂₀N₆SO·0.3 H₂O: C, 57.83; H, 5.55; N, 22.48; S, 8.58. Found: C, 57.99; H, 5.35; N, 22.43; S, 8.59.

### (Example 100) 3-amino-4-(4-pyridin-4-yl-1,4-diazepan-1-yl)thieno[2,3-b]pyridine-2-carboxamide (Exemplified Compound No. 3-141)

### (100a) tert-butyl 4-pyridin-4-yl-1,4-diazepane-1-carboxylate

4-bromopyridine hydrochloride was used in place of 2-bromopyridine with 2.1 equivalent of potassium tert-butoxide, and the reaction was performed in a similar method as described in Example 99 (99a). The residual substance which was obtained by post-treatment of the reaction was purified by silica gel column chromatography (Chromatorex NH, Fuji Silysia) (100% ethyl acetate) and the title compound was obtained. Yield 34%.
Mp 124-129°C;
IR (KBr) νₘₐₓ 1674, 1597, 1167, 987 cm⁻¹;
¹H NMR (CDCl₃, 400MHz) δ 1.36 (4.5H, s), 1.42 (4.5H, s), 1.92-1.99 (2H, m), 3.21-3.34 (2H, m), 3.52-3.59 (4H, m), 6.50 (2H, brd, J = 6.5 Hz), 8.18-8.20 (2H, m);
MS (EI) m/z: 277 [M⁺], 220;
Anal. Calcd for C₁₅H₂₃N₃O₂: C, 64.95; H, 8.36; N, 15.15. Found: C, 64.78; H, 8.46; N, 15.03.

### (100b) Pyridin-4-yl-1,4-diazepane

tert-butyl 4-pyridin-4-yl-1,4-diazepane-1-carboxylate (0.42 g, 1.5 mmol) which was produced in Example 100 (100a) was dissolved in methanol (3 mL) and 1,4-4N hydrochloric acid -dioxane solution (3 mL) was added. The reaction mixture was concentrated under reduced pressure after stirring at room temperature for two hours. 1N aqueous solution of sodium hydroxide (10 mL) was added to the residue and the aqueous layer was extracted with methylene chloride (3x20 mL). The extract was dried over sodium sulfate and the solvent was evaporated under reduced pressure and 0.26 g (yield 97%) of the title compound was obtained as oil.
IR (neat) νₘₐₓ 3270, 1600, 1518, 804 cm⁻¹;
¹H NMR (CDCl₃, 400MHz) δ 1.86-1.92 (2H, m), 2.83 (2H, t, J = 5.9 Hz), 3.02 (2H, t, J = 5.3 Hz), 3.53-3.61 (4H, m), 6.51 (2H, d, J = 6.6 Hz), 8.20 (2H, d, J = 6.6 Hz);
MS (FAB) m/z: 178 [M+H]⁺.

### (100c) (2Z)-2-cyano-3-(4-pyridin-4-yl-1,4-diazepan-1-yl)but-2-enethioamide

Pyridin-4-yl-1,4-diazepane which was produced in Example 100 (100b) was used in place of isobutylamine and the reaction was performed in a similar method as described in Example 5 (5a) and the title compound was obtained. Yield 57%. Amorphous solid
IR (KBr) νₘₐₓ 3172, 2185, 1600, 1538, 1520, 1411 cm⁻¹;
¹H NMR (DMSO-d₆, 400MHz) δ 1.89-1.94 (2H, m), 2.23 (3H, s), 3.52-3.62 (6H, m), 3.78-3.80 (2H, m), 6.70 (2H, d, J = 6.7 Hz), 8.09 (2H, d, J = 6.7 Hz), 8.41 (1H, br), 9.04 (1H, br);
MS (FAB) m/z: 302 [M+H]⁺.

### (100d) 3-amino-4-(4-pyridin-4-yl-1,4-diazepan-1-yl)thieno[2,3-b]pyridine-2-carboxamide

(2Z)-2-cyano-3-(4-pyridin-4-yl-1,4-diazepan-1-yl)but-2-enethioamide which was produced in Example 100 (100c) (200 mg, 0.66 mmol) and N,N-dimethylformamide dimethylacetal (87 mg, 0.73 mmol) were dissolved in N,N-dimethylformamide (3 mL) and after stirring at room temperature for two hours, further stirred at 100°C for one hour. The reaction solution was cooled to room temperature and 8N aqueous solution of sodium hydroxide (0.2 mL) and 2-chloroacetamide (93 mg, 0.99 mmol) were added. Water (4 mL) was added after the mixture was stirred at room temperature for one hour. The deposited solid was separated by filtration after allowing to stand at room temperature for 15 hours and washed with water and then dried, and 55 mg of a crude product was obtained. The crude product was suspended in 80% aqueous ethanol (3 mL), and heated and the mixture was stirred and after it was cooled, the solid was separated by filtration and purified and 30 mg of the title compound was obtained (yield 12%).
Mp 288-291°C (decomposition);
IR (KBr) νₘₐₓ 3334, 1650, 1597, 1573, 1510, 1367 cm⁻¹;
¹H NMR (DMSO-d₆, 400MHz) δ 2.13-2.18 (2H, m), 3.15-3.30 (4H, m), 3.56-3.59 (2H, m), 3.79-3.81 (2H, m), 6.68 (2H, d, J = 6.7 Hz), 6.98 (2H, br), 7.06 (1H, d, J = 5.1 Hz), 7.09 (2H, br), 8.09 (2H, d, J = 6.7 Hz), 8.38 (1H, d, J = 5.1 Hz);
MS (EI) m/z: 368 [M⁺], 108;
Anal. Calcd for C₁₈H₂₀N₆OS·0.3 H₂O: C, 57.83; H, 5.55; N, 22.48; S, 8.58. Found: C, 57.76; H, 5.48; N, 22.46; S, 8.50.

### (Example 101) 3-amino-4-[3-(methoxymethyl) piperidin-1-yl]thieno[2,3-b]pyridine-2-carboxamide (Exemplified Compound No. 1-21)

### (101a) tert-butyl 3-(methoxymethyl)piperidine-1-carboxylate

Sodium hydride (55% oily, 0.26 g, 6 mmol) was suspended in tetrahydrofuran (5 mL) and N,N-dimethylformamide (3 mL) solution of tert-butyl 3-(hydroxymethyl)piperidine-1-carboxylate (Bioorg.Med.Chem.Lett. 8, (1998), 1595-1600) (1.08 g, 5 mmol) was added, and further methyl iodide (0.85 g, 6 mmol) was added. Water (50 mL) was added to the reaction mixture after stirring at room temperature for three days, the aqueous layer was extracted with ethyl acetate (50 mL). The extract was dried over sodium sulfate and the solvent was evaporated under reduced pressure. The residue was purified by silica gel column chromatography (hexane/ethyl acetate =10:1) and 1.03 g of the title compound was obtained (yield 90%).
IR (neat) νₘₐₓ 1696, 1422, 1151 cm⁻¹;
¹H NMR (CDCl₃, 400MHz) δ 1.16-1.51 (2H, m), 1.46 (9H, s), 1.60-1.82 (3H, m), 2.64 (1H, br), 2.82 (1H, brt, J = 13.3 Hz), 3.24 (2H, d, J = 5.9 Hz), 3.32 (3H, s), 3.87 (1H, dt, J = 4.0, 13.3 Hz), 3.94 (1H, m);
MS (EI) m/z: 229 [M⁺], 114;
Anal. Calcd for C₁₂H₂₃NO₃: C, 62.85; H, 10.11; N, 6.11. Found: C, 62.90; H, 9.77; N, 6.04.

### (101b) 3-(methoxymethyl)piperidine

tert-butyl 3-(methoxymethyl)piperidine-1-carboxylate (1.03 g, 4.5 mmol) which was produced in Example 101 (101a) was dissolved in methylene chloride (4 mL) and trifluoroacetic acid (2 mL) was added under ice-cooling. The reaction mixture was concentrated under reduced pressure after stirring at room temperature for two hours. 1N aqueous solution of sodium hydroxide (20 mL) was added to the residue and the aqueous layer was extracted with methylene chloride (3x30 mL). The extract was dried over sodium sulfate and the solvent was evaporated under reduced pressure and 0.50 g of the title compound was obtained (yield 86%) as oil. IR (neat) νₘₐₓ 3312, 1467, 1450, 1270, 1128, 1097 cm⁻¹;
¹H NMR (CDCl₃, 400MHz) δ 1.06-1.16 (1H, m), 1.40-1.51 (1H, m), 1.63-1.80 (3H, m), 2.33 (1H, t, J =11.7 Hz), 2.55 5 (1H, dt, J = 2.7, 11.7 Hz), 3.00 (1H, brd, J = 12.1 Hz), 3.11 (1H, brd, J =12.1 Hz), 3.20 (2H, d, J = 6.3 Hz), 3.31 (3H, s);
MS (EI) m/z: 129 [M⁺], 114.

### (101c) (2Z)-2-cyano-3-[3-(methoxymethyl)piperidin-1-yl]but-2-enethioamide

3-(methoxymethyl)piperidine which was produced in Example 101 (101b) was used in place of isobutylamine and the reaction was performed in a similar method as described in Example 5 (5a) and the title compound was obtained. Yield 81%.
Mp 151-152°C;
IR (KBr) νₘₐₓ 3380, 3255, 3157, 2183, 1605, 1531, 1410, 1261 cm⁻¹;
¹H NMR (DMSO-d₆, 400MHz) δ 1.25-1.35 (1H, m), 1.48-1.59 (1H, m), 1.66-1.76 (2H, m), 1.85-1.94 (1H, m), 2.26 (3H, s), 2.91-2.97 (1H, m), 3.03-3.10 (1H, m), 3.16-3.24 (2H, m), 3.22 (3H, s), 3.50-3.63 (2H, d, J = 6.3 Hz), 8.18 (1H, br), 8.90 (1H, br);
MS (EI) m/z: 253 [M⁺], 220;
Anal. Calcd for C₁₂H₁₉N₃OS.0.1 H₂O: C, 56.49; H, 7.58; N, 16.47; S, 12.57. Found: C, 56.62; H, 7.41; N, 16.39; S, 12.60.

### (101d) 4-[3-(methoxymethyl)piperidin-1-yl]-2-thioxo-1,2-dihydropyridine-3-carbonitrile

(2Z)-2-cyano-3-[3-(methoxymethyl)piperidin-1-yl]but-2-enethioamide which was produced in Example 101 (101c) (0.39 g, 1.5 mmol) and N,N-dimethylformamide dimethylacetal (0.20 g, 1.7 mmol) were dissolved in N,N-dimethylformamide (3 mL) and the mixture was stirred at room temperature for one hour, and further stirred at 100°C for one hour. The reaction solution was cooled to room temperature and water (50 mL) was added. The aqueous layer was extracted with ethyl acetate (3x50 mL) and dried over sodium sulfate and the extract was concentrated under reduced pressure. The residue was purified by silica gel column chromatography (methylene chloride/methanol =20:1) and 125 mg of the title compound was obtained (yield 31%).
Mp 154-156°C;
IR (KBr) νₘₐₓ 2208, 1622, 1550, 1517, 1249 cm⁻¹;
¹H NMR (DMSO-d₆, 400MHz) δ 1.26-1.93 (5H, m), 3.04 (1H, dd, J = 10.2, 13.3 Hz), 3.18-3.27 (3H, m), 3.24 (3H, s), 3.96-4.02 (2H, m), 6.46 (1H, d, J = 7.8 Hz), 7.46 (1H, d, J = 7.8 Hz);
MS (EI) m/z: 263 [M⁺], 248, 218;
Anal. Calcd for C₁₃H₁₇N₃OS·0.1 H₂O: C, 58.89; H, 6.54; N, 15.85; S, 12.09. Found: C, 58.93; H, 6.60; N, 16.00; S, 12.07.

### (101e) 3-amino-4-[3-(methoxymethyl)piperidin-1-yl]thieno[2,3-b]pyridine-2-carboxamide

4-[3-(methoxymethyl) piperidin-1-yl]-2-thioxo-1,2-dihydropyridine-3-carbonitrile (120 mg, 0.46 mmol) which was produced in Example 101 (101d) was dissolved in N,N-dimethylformamide (1 mL) and 8N aqueous solution of sodium hydroxide (0.2 mL) and 2-chloroacetamide (55 mg, 0.59 mmol) were added. The mixture was blended with water (50 mL) and ethyl acetate (50 mL) and partitioned after stirring at room temperature for one hour. After the organic layer was washed with a saturated saline solution (30 mL), it was dried over sodium sulfate and concentrated under reduced pressure. Ether was added to the residue and solidified, and the deposited solid was separated by filtration and further washed with ether and 138 mg of the title compound was obtained (yield 95%).
Mp 229-230°C;
IR (KBr) νₘₐₓ 3432, 3320, 3144, 1653, 1577, 1501, 1375, 1097 cm⁻¹;
¹H NMR (DMSO-d₆, 400MHz) δ 1.03-1.15 (1H, m), 1.73-1.84 (3H, m), 2.07-2.19 (1H, m), 2.35-3.44 (4H, m), 3.22 (3H, s), 6.97 (2H, br), 7.00 (1H, d, J = 5.1 Hz), 7.09 (2H, br), 8.40 (1H, d, J = 5.1 Hz);
MS (EI) m/z: 320 [M⁺];
Anal. Calcd for C₁₅H₂₀N₄O₂S: C, 56.23; H, 6.29; N, 17.49; S, 10.01. Found: C, 56.21; H, 6.45; N, 17.37; S, 9.80.

### (Example 102) 3-amino-4-(1,4-diazepan-1-yl)thieno[2,3-b]pyridine-2-carboxamide dihydrochloride (Exemplified Compound No. 3-78)

3-amino-4-(4-tert-butoxycarbonyl-1,4-diazepan-1-yl)thieno[2,3-b]pyridine-2-carboxamide (0.77 g, 2.0 mmol) which was produced in Example 55 (55c) was dissolved in 1,4-dioxane (4 mL) and 4N hydrochloric acid/1,4-dioxane (4 mL) was added. The reaction mixture was concentrated under reduced pressure after stirring at room temperature for four hours and the 0.72 g of the title compound was obtained. Mp >295°C;
IR (KBr) νₘₐₓ 3457, 3371, 3291, 1654, 1614, 1579 cm⁻¹;
¹H NMR (DMSO-d₆, 400MHz) δ 2.12-2.19 (2H, m), 3.20-3.26 (2H, m), 3.36-3.42 (2H, m), 3.58-3.64 (2H, m), 3.80-3.87 (2H, m), 7.11 (1H, d, J = 6.5 Hz), 7.27 (2H, br), 8.44 (1H, d, J = 6.5 Hz), 9.46 (2H, br);
MS (FAB) m/z: 292 [M+H]⁺;
Anal. Calcd for C₁₃H₁₇N₅OS·2(HCl)·0.3 H₂O: C, 42.24; H, 5.34; N, 18.94; S, 8.67; Cl, 19.18. Found: C, 42.39; H, 5.22; N, 19.01; S, 8.53; Cl, 18.93.

### (Example 103) 4-(4-acetyl-1,4-diazepan-1-yl)-3-aminothieno[2,3-b]pyridine-2-carboxamide (Exemplified Compound No. 3-149)

3-amino-4-(1,4-diazepan-1-yl)thieno[2,3-b]pyridine-2-carboxamide dihydrochloride (182 mg, 0.5 mmol) which was produced in Example 102 was suspended in tetrahydrofuran (3 mL) and blended with triethylamine (0.15 mL) and acetic acid anhydride (77 mg, 0.75 mmol) and the mixture was stirred at room temperature for two hours. A saturated sodium bicarbonate aqueous solution (20 mL) was added to the reaction mixture and the aqueous layer was extracted with methylene chloride (2x30 mL). After having dried in sodium sulfate in extract, the solvent was evaporated under reduced pressure and 55 mg of the title compound (yield 33%) was obtained when the obtained solid was recrystallized from ethanol (4 mL).
Mp 214-215°C;
IR (KBr) νₘₐₓ 3425, 3333, 3188, 1632, 1581, 1370, 938 cm⁻¹;
¹H NMR (DMSO-d₆, 400MHz) δ 1.97-2.08 (2H, m), 2.02 (1.5H, s), 2.05 (1.5H, s), 3.15-3.24 (4H, m), 3.55-3.62 (2H, m), 3.69-3.77 (2H, m), 7.01 (2H, br), 7.04 (0.5H, d, J = 5.5 Hz), 7.07 (0.5H, d, J = 5.5 Hz), 7.09 (2H, br), 8.38 (0.5H, d, J = 5.5 Hz),8.41 (0.5H, d, J = 5.5 Hz);
MS (FAB) m/z: 334 [M+H]⁺;
Anal. Calcd for C₁₅H₁₉N₅O₂S·0.1 H₂O: C, 53.75; H, 5.77; N, 20.89; S, 9.57. Found: C, 53.89; H, 5.75; N, 20.73; S, 9.42.

### (Example 104) 3-amino-4-[4-(methylsulfonyl)-1,4-diazepan-1-yl]thieno[2,3-b]pyridine-2-carboxamide (Exemplified Compound No. 3-151)

3-amino-4-(1,4-diazepan-1-yl)thieno[2,3-b]pyridine-2-carboxamide dihydrochloride which was produced in Example 102 (182 mg, 0.5 mmol) and potassium carbonate (221 mg, 1.6 mmol) were suspended in tetrahydrofuran (5 mL), and the resultant solution was blended with methanesulfon acid anhydride (105 mg, 0.6 mmol) and the mixture was stirred at room temperature for 15 hours. Water (20 mL) and ethyl acetate (30 mL) were added to the reaction mixture and partitioned and the aqueous layer was extracted with ethyl acetate (30 mL). After the combined organic layer was dried over sodium sulfate, the solvent was evaporated under reduced pressure and 72 mg of the title compound (yield 39%) was obtained when the obtained residue was purified by silica gel column chromatography (methylene chloride/methanol = 15: 1).
Mp 214-217°C;
IR (KBr) νₘₐₓ 3424, 3327, 3155, 1650, 1582, 1371, 1323, 1147, 934 cm⁻¹;
¹H NMR (DMSO-d₆, 400MHz) δ 2.02-2.07 (2H, m), 2.96 (3H, s), 3.26-3.84 (2H, m), 3.44-3.47 (2H, m), 3.58-3.61 (2H, m), 7.04 (2H, br), 7.10 (1H, d, J = 5.5 Hz), 7.01 (2H, br), 8.44 (1H, d, J = 5.5 Hz);
MS (FAB) m/z: 370 [M+H]⁺;
Anal. Calcd for C₁₄H₁₉N₅O₃S₂: C, 45.51; H, 5.18; N, 18.96; S, 17.36. Found: C, 45.38; H, 5.08; N, 18.64; S, 17.04.

### (Example 105) 3-amino-4-[4-(6-chloropyridazin-3-yl)-1,4-diazepan-1-yl]thieno[2,3-b]pyridine-2-carboxamide (Exemplified Compound No. 3-148)

### (105a) (2Z)-3-[4-(6-chloropyridazin-3-yl)-1,4-diazepan-1-yl]-2-cyanobut-2-enethioamide

1-(6-chloropyridazin-3-yl)-1,4-diazepane (J. Med. Chem. (2002), 45,4011-4017) was used in place of isobutylamine; and the reaction was performed in a similar method as described in Example 5 (5 a) and the title compound was obtained.
Yield 60%.
Amorphous solid
IR (KBr) νₘₐₓ 3289, 3185, 2191, 1587, 1531, 1453, 1429 cm⁻¹;
¹H NMR (DMSO-d₆, 400MHz) δ 1.89-1.95 (2H, m), 2.25 (3H, s), 3.59-3.66 (4H, m), 3.74-3.77 (2H, m), 3.97-4.00 (2H, m), 7.31 (1H, d, J = 9.8 Hz), 7.51 (1H, d, J = 9.8 Hz), 8.40 (1H, br), 9.04 (1H, br);
MS (FAB) m/z: 337 [M+H]⁺.

### (105b) 3-amino-4-[4-(6-chloropyridazin-3-yl)-1,4-diazepan-1-yl]thieno[2,3-b]pyridine-2-carboxamide

(2Z)-3-[4-(6-chloropyridazin-3-yl)-1,4-diazepan-1-yl]-2-cyanobut-2-enethioamide (0.75 g, 2.2 mmol) which was produced in Example 105 (105a) and N,N-dimethylformamide dimethylacetal (0.28 g, 2.3 mmol) were dissolved in N,N-dimethylformamide (5 mL) and the mixture was stirred at room temperature for one hour, and further stirred at 100°C for two hours. The reaction solution was cooled to room temperature and 8N aqueous solution of sodium hydroxide (0.5 mL) and 2-chloroacetamide (0.27 g, 2.9 mmol) were added. Water (30 mL) was added after the mixture was stirred at room temperature for one hour. The aqueous layer was extracted with ethyl acetate (2x50 mL) and the extract was concentrated after drying over sodium sulfate under reduced pressure. The obtained residue was purified by silica gel column chromatography (methylene chloride/methanol =20:1) and the obtained crystal was further washed with ethanol and 0.15 g of the title compound was obtained (yield 17%).
Mp 232-234°C;
IR (KBr) νₘₐₓ 3320, 1645, 1584, 1501, 1450, 1372, 939 cm⁻¹;
¹H NMR (DMSO-d₆, 400MHz) δ 2.13-2.19 (2H, m), 3.18-3.23 (2H, m), 3.30-3.35 (2H, m), 3.75-3.78 (2H, m), 4.00-4.05 (2H, m), 7.00 (2H, br), 7.05 (1H, d, J = 5.1 Hz), 7.09 (2H, br), 7.26 (1H, d, J = 9.6 Hz), 7.50 (1H, d, J = 9.6 Hz), 8.38 (1H, d, J = 5.1 Hz);
MS (FAB) m/z: 404 [M+H]⁺;
Anal. Calcd for C₁₇H₁₈N₇OSCl·0.3 H₂O: C, 49.89; H, 4.58; N, 23.95; S, 7.83; Cl, 8.66. Found: C, 49.93; H, 4.52; N, 23.93; S, 8.09; Cl, 8.39.

### (Example 106) 3-amino-4-[4-(5-methylpyridin-2-yl)-1,4-diazepan-1-yl]thieno[2,3-b]pyridine-2-carboxamide (Exemplified Compound No. 3-143)

### (106a) tert-butyl 4-(5-methylpyridin-2-yl)-1,4-diazepane-1-carboxylate

2-bromo-5-methylpyridine was used in place of 2-bromopyridine and the reaction was performed in a similar method as described in Example 99 (99a). The obtained crude product was purified by silica gel column chromatography (hexane/ethyl acetate =5:1) and the title compound was obtained at yield 81%. IR (neat) νₘₐₓ 1694, 1612, 1503, 1415, 1170, 929 cm⁻¹;
¹H NMR (CDCl₃, 400MHz) δ 1.39 (4.5H, s), 1.44 (4.5H, s), 1.92-1.98 (2H, m), 2.19 (3H, s), 3.20-3.33 (2H, m), 3.54-3.76 (6H, m), 6.45 (1H, d, J = 8.6 Hz), 7.26 (1H, dd, J = 2.0, 8.6 Hz), 7.96 (1H, d, J = 2.0 Hz);
MS (EI) m/z: 291 [M⁺], 135;
Anal. Calcd for C₁₆H₂ₛN₃O₂·0.4 H₂O: C, 64.36; H, 8.71; N, 14.07. Found: C, 64.35; H, 8.96; N, 13.98.

### (106b) 1-(5-methylpyridin-2-yl)-1,4-diazepane

The reaction was performed in a similar method as described in Example 100 (100b) and the title compound was obtained at yield 97%.
IR (neat) νₘₐₓ 3307, 1613, 1503, 1409, 804 cm⁻¹;
¹H NMR (CDCl₃, 400MHz) δ 1.85-1.91 (2H, m), 2.17 (3H, s), 2.82-2.85 (2H, m), 3.01-3.03 (2H, m), 3.68-3.72 (4H, m), 6.43 (1H, d, J = 8.6 Hz), 7.26 (1H, dd, J = 2.0, 8.6 Hz), 7.97 (1H, d, J = 2.0 Hz);
MS (FAB) m/z: 192 [M+H]⁺, 135.

### (106c) (2Z)-2-cyano-3-[(4-(5-methylpyridin-2-yl)-1,4-diazepan-1-yl)but-2-enethioamide

1-(5-methylpyridin-2-yl)-1,4-diazepane which was produced in Example 106 (106b) was used in place of isobutylamine and the reaction was performed in a similar method as described in Example 5 (5a) and the title compound was obtained.
Yield 79%.
Mp 140-145°C;
IR (KBr) νₘₐₓ 3274, 3131, 2181, 1611, 1528, 1501, 1411, 885 cm⁻¹;
¹H NMR (DMSO-d₆, 400MHz) δ 1.87-1.93 (2H, m), 2.12 (3H, s), 2.24 (3H, s), 3.53-3.67 (6H, m), 3.86-3.89 (2H, m), 6.65 (1H, d, J = 8.6 Hz), 7.34 (1H, dd, J = 2.0, 8.6 Hz), 7.91 (1H, d, J = 2.0 Hz), 8.82 (1H, br), 8.99 (1H, br);
MS (FAB) m/z: 316 [M+H]⁺;
Anal. Calcd for C₁₆H₂₁N₅S·0.3 H₂O: C, 59.90; H, 6.79; N, 21.83; S, 9.95. Found: C, 59.96; H, 6.62; N, 21.55; S, 10.19.

### (106d) 3-amino-4-[4-(5-methylpyridin-2-yl)-1,4-diazepan-1-yl]thieno[2,3-b]pyridine-2-carboxamide

(2Z)-2-cyano-3-[(4-(5-methylpyridin-2-yl)-1,4-diazepan-1-yl)but-2-enethioamide (1.26 g, 4.0 mmol) which was produced in Example 106 (106c) and N,N-dimethylformamide dimethylacetal (0.52 g, 4.4 mmol) were dissolved in N,N-dimethylformamide (6 mL) and the mixture was stirred at room temperature for one hour, and further stirred at 100°C for two hours. The reaction solution was cooled to room temperature and 8N aqueous solution of sodium hydroxide (1 mL) and 2-chloroacetamide (0.49 g, 5.2 mmol) were added. Water (6 mL) was added after the mixture was stirred at room temperature for two hours. The deposited solid was separated by filtration, and 0.87 g of a crude product was obtained when washed with water and ethanol. The crude product was suspended in 80% aqueous ethanol (10 mL), and heated and the mixture was stirred and after it was cooled, the solid was separated by filtration and purified and 0.66 g of the title compound was obtained (yield 43%).
Mp 205-208°C;
IR (KBr) νₘₐₓ 3440, 3316, 3155, 1649, 1609, 1579, 1499, 1371, 939 cm⁻¹;
¹H NMR (DMSO-d₆, 400MHz) δ 2.08-2.15 (2H, m), 2.13 (3H, s), 3.15-3.28 (4H, m), 3.64-3.67 (2H, m), 3.92-3.98 (2H, m), 6.60 (1H, d, J = 8.6 Hz), 6.98 (2H, br), 7.04 (1H, d, J = 5.1 Hz), 7.07 (2H, br), 7.34 (1H, dd, J = 2.3, 8.6 Hz), 7.90 (1H, d, J = 2.0 Hz), 8.36 (1H, d, J = 5.1 Hz);
MS (EI) m/z: 382 [M⁺];
Anal. Calcd for C₁₉H₂₂N₆SO: C, 59.66; H, 5.80; N, 21.97; S, 8.38. Found: C, 59.46; H, 5.93; N, 21.78; S, 8.41.

### (Example 107)

### 3-amino-4-methoxythieno[2,3-b]pyridine-2-carboxamide (Exemplified Compound No. 1-1)

### (107a) 2-chloro-4-methoxynicotinonitrile

A phosphorus oxychloride (10 mL) solution of 4-methoxy-2-oxo-1,2-dihydropyridine-3-carbonitrile (1.50 g, 10 mmol) which was synthesized by a method of Ogawa et al. (Heterocycles, 36,145-148,1993) was heated under reflux for two hours. The reaction liquid was concentrated and a sodium hydrogen carbonate aqueous solution (10 mL) added to the obtained residual substance and the mixture was extracted with ethyl acetate (3x10 mL), and the solvent was evaporated under reduced pressure after the extract was dried over sodium sulfate. The obtained residue was powderized with ether and the title compound was obtained (1.31 g, yield 78%).
Pale yellow powder
¹H NMR(DMSO-d₆, 400 MHz) δ 4.05 (3H, s), 7.40 (1H, d, J = 4.3 Hz), 8.55 (1H, d, J = 4.3 Hz).

### (107b) 3-amino-4-methoxythieno[2,3-b]pyridine-2-carboxamide

2-mercaptoacetamide (109 mg, 1.2 mmol) and 8M aqueous solution of sodium hydroxide (0.4 mL) were added to N,N-dimethylformamide (2 mL) solution of 2-chloro-4-methoxynicotinonitrile (169 mg, 1.0 mmol) which was produced in Example 107 (107a), and the mixture was stirred at room temperature for one hour. Water (5 mL) was added to the reaction liquid and the resulted solid was separated by filtration and the title compound was obtained (92 mg, yield 41 %).
Yellow powder
Mp 238-241 °C;
IR (KBr) νₘₐₓ 3482, 3325, 3149, 1667, 1613, 1583, 1504, 1375, 1289, 1044 cm⁻¹; ¹H NMR(DMSO-d₆, 400 MHz) δ 4.01 (3H, s), 6.95 (2H, brs), 6.98 (1H, d, J = 5.9 Hz), 7.05 (2H, brs), 8.46 (1H, d, J = 5.9 Hz);
HRMS m/z calcd for C₉H₉N₃O₂S 223.0415, found 223.0416;
MS (EI) m/z: 223 [M⁺], 205, 178, 150, 137, 122, 104, 77, 66, 45;
Anal. Calcd for C₉H₉N₃O₂S: C, 48.42; H, 4.06; N, 18.82; S, 14.36. Found: C, 48.11; H, 4.32; N, 18.76, S, 14.18.

### (Example 108) 3-amino-4-ethoxythieno[2,3-b]pyridine-2-carboxamide (Exemplified Compound No. 1-2)

### (108a) 2-chloro-4-ethoxynicotinonitrile

4-methoxy-2-oxo-1,2-dihydropyridine-3-carbonitrile, which was synthesized by a method of Ogawa et al. (Heterocycles, 36,145-148,1993) using triethyl orthoacetate in place of trimethyl orthoacetate, was used and the reaction was performed in a similar method as described in Example 107 (107a) and the title compound was synthesized.
Pale yellow powder
¹H NMR(DMSO-d₆, 500 MHz) δ 1.39 (3H, t, J = 6.8 Hz), 4.36 (2H, q, J = 6.8 Hz), 7.39 (1H, d, J = 6.4 Hz), 8.52 (1H, d, J = 6.4 Hz).

### (108b) 3-amino-4-ethoxythieno[2,3-b]pyridine-2-carboxamide

2-chloro-4-ethoxynicotinonitrile was used in place of 2-chloro-4-methoxynicotinonitrile and the reaction was performed in a similar method as described in Example 107 (107b) and the title compound was synthesized.
Pale yellow powder
Mp 241-243°C;
IR (KBr) νₘₐₓ 3446, 3331, 1645, 1584, 1506, 1375, 1294, 1046 cm⁻¹;
¹H NMR(DMSO-d₆, 400 MHz) δ 1.44 (3H, t, J = 7.1 Hz), 4.30 (2H, q, J = 7.1 Hz), 6.84 (2H, brs), 6.95 (1H, d, J = 5.9 Hz), 7.03 (2H, brs), 8.41 (1H, d, J = 5.9 Hz); HRMS m/z calcd for C₁₀H₁₁O₂N₃S 237.0572, found 237.0574;
MS (EI) m/z: 237 [M⁺], 219, 205, 192, 176, 164, 148, 137, 120, 104;
Anal. Calcd for C₁₀H₁₁N₃O₂S: C, 50.62; H, 4.67; N, 17.71; S, 13.51. Found: C, 50.62; H, 4.69; N, 17.97, S, 13.52.

### (Example 109) {[3-amino-2-(aminocarbonyl)thieno[2,3b]pyridin-4-yl]oxy} acetic acid (Exemplified Compound No. 1-5)

### (109a) 2-chloro-4-oxo-1,4-dihydropyridine-3-carbonitrile

Concentrated hydrochloric acid (5 mL) was added to acetic acid (5 mL) solution of 2-chloro-4-methoxynicotinonitrile (0.98 g, 5.81 mmol) which was produced in Example 107 (107a), and heated under reflux for two hours. Water (10 mL) was added to the reaction liquid and extracted with ethyl acetate (10 mL), and the solvent was evaporated under reduced pressure after the extract was dried over sodium sulfate. The obtained residue was powderized with ether and the title compound was obtained (0.35 g, yield 39%).
White powder
Mp 214-217°C;
¹H NMR(DMSO-d₆, 400 MHz) δ 6.96 (1H, d, J = 5.9 Hz), 8.25 (1H, d, J = 5.9 Hz). (109b) {[3-amino-2-(aminocarbonyl)thieno[2,3-b]pyridin-4-yl]oxy} acetic acid

Potassium carbonate (152 mg, 1.1 mmol) and bromoacetate tert-butyl ester (162 µL, 1.1 mmol) were added to N, N-dimethylacetamide (5 mL) solution of 2-chloro-4-oxo-1,4-dihydropyridine-3-carbonitrile (155 mg, 1.0 mmol) which was produced in Example 109 (109a) and the mixture was stirred at room temperature for five hours. Ethyl acetate (20 mL) and water (20 mL) were added to the reaction liquid and partitioned. The organic layer was washed with water (5x20 mL), a saline solution (20 mL) and the solvent was evaporated under reduced pressure after drying over sodium sulfate. The obtained residue was dissolved in N,N-dimethylformamide (2 mL) and was blended with 2-mercaptoacetamide (118 mg, 1.3 mmol) and 8N aqueous solution of sodium hydroxide (0.4 mL) and the mixture was stirred at room temperature for one hour. After 1N hydrochloric acid (4 mL) was added to the reaction liquid, it was concentrated under reduced pressure. The obtained residue was purified by reverse-phase chromatography (100% water) and the title compound was obtained (78 mg, yield 29%).
Yellow powder
Mp 248-249°C (decomposition);
IR (KBr) νₘₐₓ 3462, 3340, 3169, 1647, 1591, 1507, 1376, 1084 cm⁻¹;
¹H NMR(DMSO-d₆, 400 MHz) δ 4.97 (2H, s), 6.93 (1H, d, J = 5.9 Hz), 7.04 (2H, brs), 7.07 (2H, brs), 8.44 (1H, d, J = 5.9 Hz);
HRMS m/z calcd for C₁₀H₉O₄N₃S 267.0314, found 267.0325;
MS (FAB) m/z: 267 [M⁺], 251, 205, 187, 69, 55;
Anal. Calcd for C₁₀H₉N₃O₄S·1.96H₂O: C, 39.70; H, 4.30; N, 13.89; S, 10.60. Found: C, 39.39; H, 3.99; N, 13.89; S, 10.46.

### (Example 110) 3-amino-4-(benzyloxy)thieno[2,3-b]pyridine-2-carboxamide (Exemplified Compound No. 1-4)

Benzyl bromide was used in place of bromoacetic acid tert-butyl ester and the reaction was performed in a similar method as described in Example 109 (109b) and the title compound was synthesized.
White powder
Mp 216-221°C;
IR (KBr) νₘₐₓ 3490, 3324, 3151, 1651, 1580, 1502, 1370, 1290, 1039, 741 cm⁻¹;
¹H NMR(DMSO-d₆, 400 MHz) δ 5.44 (2H, s), 6.89 (2H, brs), 7.04 (1H, d, J = 5.5 Hz), 7.07 (2H, brs), 7.36-7.55 (5H, m), 8.42 (1H, d, J = 5.5 Hz);
HRMS m/z calcd for C₁₅H₁₃O₂N₃S 299.0728, found 299.0730;
MS (FAB) m/z: 299 [M⁺], 283, 273, 257, 200, 193, 165, 91, 65;
Anal. Calcd for C₁₅H₁₃N₃O₂S·0.12H₂O: C, 59.75; H, 4.43; N, 13.94; S, 10.63. Found: C, 60.09; H, 4.36; N, 13.63; S, 10.26.

### (Example 111) 3-amino-4-isopropoxythieno[2,3-b]pyridine-2-carboxamide (Exemplified Compound No. 1-3)

### (111a) 2,4-dichloronicotinonitrile

A phosphorus oxychloride (0.7 mL) solution of 2-chloro-4-oxo-1,4-dihydropyridine-3-carbonitrile (238 mg, 1.54 mmol) which was produced in Example 109 (109a) was heated for two hours under reflux. The reaction liquid was concentrated and a sodium hydrogen carbonate aqueous solution (10 mL) was added to the obtained residual substance and extracted with ethyl acetate (10 mL) twice, and the solvent was evaporated under reduced pressure after drying over sodium sulfate. The obtained residue was powderized in hexane and the title compound was obtained (167 mg, yield 63%).
White powder
Mp 107-109°C;
IR (KBr) νₘₐₓ 3072, 2235, 1559, 1538, 1444, 1406, 1368, 1220, 820 cm⁻¹;
¹H NMR(DMSO-d₆, 400 MHz) δ 7.91 (1H, d, J = 5.5 Hz), 8.65 (1H, d, J = 5.5 Hz); HRMS m/z calcd for C₆H₂N₂Cl₂ 171.9595, found 171.9598;
MS (EI) m/z: 172 [M⁺], 137, 110, 101, 75, 62, 51.

### (111b) 2-chloro-4-isopropoxynicotinonitrile

sodium hydride (48 mg, 1.1 mmol) and N,N-dimethylacetamide (1 mL) solution of 2,4-dichloronicotinonitrile (173 mg, 1.00 mmol) which was produced in Example 111 (111a) were added to THF (1 mL) solution of 2-propanol (84 µL, 1.1 mmol) at 0°C and the mixture was stirred at room temperature for one hour. Water (3 mL) was added to the reaction liquid and generated powder was separated by filtration and the title compound was obtained (125 mg, yield 63%).
White powder
IR (KBr) νₘₐₓ 3096, 2985, 2235, 1580, 1550, 1469, 1390, 1316, 1262, 1102, 985, 840 cm⁻¹;
¹H NMR(DMSO-d₆, 400 MHz) δ 1.36 (6H, d, J = 5.9 Hz), 4.99 (1H, quint, J = 5.9 Hz), 7.43 (1H, d, J = 6.4 Hz), 8.49 (1H, d, J = 6.4 Hz);
HRMS m/z calcd for C₉H₉ON₂Cl 196.0404, found 196.0401;
MS (EI) m/z: 196 [M⁺], 181, 154, 145, 126, 119, 111, 93, 71, 57, 44.

### (111c) 3-amino-4-isopropoxythieno[2,3-b]pyridine-2-carboxamide

2-chloro-4-isopropoxynicotinonitrile which was produced in Example 111 (111b) was used in place of 2-chloro-4-methoxynicotinonitrile and the reaction was performed in a similar method as described in Example 107 (107b) and the title compound was obtained.
White powder
Mp 238-240°C (decomposition);
IR (KBr) νₘₐₓ 3485, 3322, 3137, 1672, 1616, 1582, 1506, 1378, 1287, 1107, 995 cm⁻¹;
¹H NMR(DMSO-d₆, 400 MHz) δ 1.40 (6H, d, J = 6.3 Hz), 4.94 (1H, quint, J = 6.3 Hz), 6.83 (2H, brs), 6.98 (1H, d, J = 5.9 Hz), 7.02 (2H, brs), 8.39 (1H, d, J = 5.9 Hz); HRMS m/z calcd for C₁₀H₁₃O₂N₃S 251.0728, found 251.0742;
MS (EI) m/z: 251 [M⁺], 209, 192, 180, 164, 137, 120, 103, 92, 66, 52, 42.

### (Example 112) 3-amino-4-(cycloheptyloxy)thieno[2,3-b]pyridine-2-carboxamide (Exemplified Compound No. 1-8)

### (112a) 2-chloro-4-(cycloheptyloxy)nicotinonitrile

Cycloheptyl alcohol was used in place of 2-propanol and the reaction was performed in a similar method as described in Example 111 (111b) and the title compound was synthesized.
Colourless liquid
IR (film) νₘₐₓ 2933, 2233, 1575, 1464, 1307, 998, 821 cm⁻¹;
¹H NMR(CDCl₃, 500 MHz) δ 1.47-2.05 (12H, m), 4.64-4.69 (1H, m), 6.79 (1H, d, J = 6.3 Hz), 8.34 (1H, d, J = 6.3 Hz);
HRMS m/z calcd for C₁₃H₁₅ON₂³⁵Cl 250.0873, found 250.0880;
MS (EI) m/z: 250 [M⁺], 215, 172, 155, 137, 119, 97, 81, 55, 42;
Anal. Calcd for C₁₃H₁₅ClN₂O·0.36H₂O: C, 60.71; H, 6.16; N, 10.89. Found: C, 61.03; H, 6.51; N, 10.93.

### (112b) 3-amino-4-(cycloheptyloxy)thieno[2,3-b]pyridine-2-carboxamide

2-chloro-4-(cycloheptyloxy)nicotinonitrile which was produced in Example 112 (112a) was used in place of 2-chloro-4-methoxynicotinonitrile and the reaction was performed in a similar method as described in Example 107 (107b) and the title compound was synthesized.
White powder
Mp 179-180°C;
IR (KBr) νₘₐₓ 3491, 3329, 3163, 2927, 1654, 1582, 1504, 1371, 1288, 1012 cm⁻¹; ¹H NMR(DMSO-d₆, 400 MHz) δ 1.46-1.70 (8H, m), 1.81-1.90 (2H, m), 2.01-2.08 (2H, m), 4.83-4.89 (1H, m), 6.81 (2H, brs), 6.94 (1H, d, J = 5.5 Hz), 7.03 (2H, brs), 8.38 (1H, d, J = 5.5 Hz);
HRMS m/z calcd for C₁₅H₁₉O₂N₃S 305.1198, found 305.1197;
MS (EI) m/z: 305 [M⁺], 209, 192, 164, 120, 97, 55, 41;
Anal. Calcd for C₁₅H₁₉N₃O₂S: C, 58.99; H, 6.27; N, 13.76; S, 10.50. Found: C, 58.81; H, 6.33; N, 13.60; S, 10.30.

### (Example 113) 3-amino-4-(cyclohexyloxy)thieno[2,3-b]pyridine-2-carboxamide (Exemplified Compound No. 1-7)

### (113a) 2-chloro-4-(cyclohexyloxy)nicotinonitrile

Cyclohexyl alcohol was used in place of 2-propanol and the reaction was performed in a similar method as described in Example 111 (111b) and the title compound was synthesized.
Colourless liquid
IR (film) νₘₐₓ 2941, 2862, 2233, 1578, 1465, 1305, 1016, 987, 824 cm⁻¹;
¹H NMR(CDCl₃, 500 MHz) δ 1.42-1.96 (10H, m), 4.50-4.56 (1H, m), 6.84 (1H, d, J = 6.4 Hz), 8.35 (1H, d, J = 6.4 Hz);
HRMS m/z calcd for C₁₂H₁₃ON₂Cl 236.0717, found 236.0721;
MS (EI) m/z: 236 [M⁺], 207, 195, 181, 155, 119, 83, 67, 55, 42;
Anal. Calcd for C₁₂H₁₃ClN₂O·0.12H₂O: C, 60.34; H, 5.59; N, 11.73; Cl, 14.84. Found: C, 60.06; H, 5.85; N, 11.50; Cl, 15.24.

### (113b) 3-amino-4-(cyclohexyloxy)thieno[2,3-b]pyridine-2-carboxamide

2-chloro-4-(cyclohexyloxy)nicotinonitrile which was produced in Example 113 (113a) was used in place of 2-chlorb-4-methoxynicotinonitrile and the reaction was performed in a similar method as described in Example 107 (107b) and the title compound was synthesized.
White powder
Mp 206-208°C;
IR (KBr) νₘₐₓ 3492, 3330, 3159, 2935, 1654, 1580, 1504, 1368, 1286, 1041, 1020, 992 cm⁻¹;
¹H NMR(DMSO-d₆, 400 MHz) δ 1.28-1.77 (8H, m), 1.94-2.03 (2H, m), 4.70-4.76 (1H, m), 6.81 (2H, brs), 7.02 (1H, d, J = 5.9 Hz), 7.04 (2H, brs), 8.38 (1H, d, J = 5.9 Hz);
HRMS m/z calcd for C₁₄H₁₇O₂N₃S 291.1041, found 291.1040;
MS (EI) m/z: 291 [M⁺], 209, 192, 164, 137, 120, 55, 41;
Anal. Calcd for C₁₄H₁₇N₃O₂S·0.36H₂O: C, 56.45; H, 6.00; N, 14.11; S, 10.76. Found: C, 56.75; H, 5.74; N, 14.07; S, 10.46.

### (Example 114) 3-amino-4-(ethylthio)thieno[2,3-b]pyridine-2-carboxamide (Exemplified Compound No. 1-10)

### (114a) 2-chloro-4-(ethylthio)nicotinonitrile

N,N -dimethylacetamide (1 mL) solution of sodium thioethoxide (93 mg, 1.1 mmol) was added to N,N -dimethylacetamide (1 mL) solution of 2,4-dichloronicotinonitrile (173 mg, 1.00 mmol) which was produced in Example 111 (111a) at 0°C and the mixture was stirred at room temperature for one hour. Water (3 mL) was added to the reaction liquid, and generated powder was separated by filtration and further purified by silica gel column chromatography (hexane/ethyl acetate =3:1) and the title compound was obtained (44 mg, yield 22%).
Pale yellow powder
Mp 97-98°C;
IR (KBr) νₘₐₓ 2226, 1556, 1518, 1431, 1379, 1199, 819 cm⁻¹;
¹H NMR(CDCl₃, 500 MHz) δ 1.46 (3H, t, J = 7.3 Hz), 3.12 (2H, q, J = 7.3 Hz), 7.11 (1H, d, J = 5.9 Hz), 8.33 (1H, d, J = 5.9 Hz);
HRMS m/z calcd for C₈H₇N₂³⁵ClS 198.0018, found 198.0011;
MS (EI) m/z: 198 [M⁺], 183, 170, 165, 147, 134, 126, 108, 98, 76, 69, 64, 46.

### (114b) 3-amino-4-(ethylthio)thieno[2,3-b]pyridine-2-carboxamide

2-chloro-4-(ethylthio)nicotinonitrile which was produced in Example 114 (114a) was used in place of 2-chloro-4-methoxynicotinonitrile and the reaction was performed in a similar method as described in Example 107 (107b) and the title compound was obtained.
Pale yellow powder
Mp 230-235°C;
IR (KBr) νₘₐₓ 3460, 3297, 2141, 1666, 1589, 1494, 1365, 625 cm⁻¹;
¹H NMR(DMSO-d₆, 400 MHz) δ 1.35 (3H, t, J = 7.4 Hz), 3.23 (2H, q, J = 7.4 Hz), 6.94 (2H, brs), 7.20 (2H, brs), 7.29 (1H, d, J = 5.5 Hz), 8.39 (1H, d, J = 5.5 Hz); HRMS m/z calcd for C₁₀H₁₁ON₃S₂ 253.0344, found 253.0333;
MS (EI) m/z: 253 [M⁺], 236, 221, 208, 203, 193, 176, 148, 135, 104, 76, 45.

### (Example 115) 3-amino-4-(cyclohexylthio)thieno[2,3-b]pyridine-2-carboxamide (Exemplified Compound No. 1-13)

Mercaptocyclohexane (116 mg, 1.00 mmol) and 8N aqueous solution of sodium hydroxide (0.19 mL) were added under ice-cooling to N,N-dimethylacetamide (1 mL) solution of 2,4-dichloronicotinonitrile (173 mg, 1.00 mmol) which was produced in Example 111 (111a) and the mixture was stirred for one hour. The reaction mixture was partitioned with water and methylene chloride, and the organic layer was concentrated under reduced pressure after drying over sodium sulfate. The residue was purified by silica gel column chromatography (hexane/ethyl acetate =4:1) and a mixture (187 mg) of 2-chloro-4-(ethylthio)nicotinonitrile and 2,4-di(cyclohexylthio)nicotinonitrile was obtained. The obtained mixture (181 mg) and 2-mercaptoacetamide (66 mg) were dissolved in N,N-dimethylformamide (1.4 mL) and blended with 8N aqueous solution of sodium hydroxide (0.31 mL) and the mixture was stirred at room temperature for one hour. The solid which was deposited by adding water to the reaction mixture was separated by filtration and furthermore washed with ether and ethanol and the title compound was obtained 23 mg (yield 7%).
Yellow powder
Mp 195-197°C;
IR (KBr) νₘₐₓ 3450, 3310, 3154, 2929, 1662, 1585, 1495, 1363, 829, 620 cm-¹;
¹H NMR(DMSO-d₆, 400 MHz) δ 1.39-2.04 (10H, m), 3.60-3.71 (1H, m), 7.06 (2H, brs), 7.19 (2H, brs), 7.38 (1H, d, J = 5.5 Hz), 8.41 (1H, d, J = 5.5 Hz);
HRMS m/z calcd for C₁₄H₁₇ON₃S₂ 307.0813, found 307.0819;
MS (EI) m/z: 307 [M⁺], 289, 262, 225, 208, 180, 136, 104, 83, 55, 41.

### (Example 116) 3-amino-4-(4-{4-[(dimethylamino)carbonyl)phenyl}piperazin-1-yl)thieno[2,3-b]pyridine-2-carboxamide (Exemplified Compound No. 3-30)

### (116a) tert-butyl 4-{4-[(dimethylamino)carbonyl]phenyl}piperazine-1-carboxylate

After 1,1'-carbonyldiimidazole (0.58 g, 3.6 mmol) was added to tetrahydrofuran (10 mL) solution of 4-[4-(tert-butoxycarbonyl)piperazin-1-yl]benzoic acid (1.00 g, 3.3 mmol) and the mixture was stirred at room temperature for 30 minutes, 50% dimethylamine aqueous solution (0.45 mL) was added. The reaction liquid was stirred at room temperature for four hours and a saturated saline solution (100 mL) and ethyl acetate (100 mL) were added and partitioned. The organic layer was washed with a saturated sodium carbonate aqueous solution (30 mL) and the solvent was evaporated under reduced pressure after drying over sodium sulfate. The residue was purified by silica gel column chromatography (100% ethyl acetate) and 1.00 g of the title compound was obtained (yield 92%).
Mp 126-128°C;
IR (KBr) νₘₐₓ 1689, 1628, 1241, 1165, 835, 769 cm⁻¹;
¹H NMR (CDCl₃, 400MHz) δ 1.49 (9H, s), 3.06 (6H, brs), 3.20 (4H, t, J = 5.1 Hz), 3.58 (4H, t, J = 5.1 Hz), 6.89 (2H, d, J = 8.6 Hz), 7.38 (2H, d, J = 8.6);
MS (FAB) m/z: 333 [M⁺];
Anal. Calcd for C₁₈H₂₇N₃O₃: C, 64.84; H, 8.16; N, 12.60. Found: C, 64.82; H, 8.41; N, 12.59.

### (116b) N,N-dimethyl-4-piperazin-1-ylbenzamide

tert-butyl 4- {4-[(dimethylamino)carbonyl]phenyl}piperazine-1-carboxylate (1.00 g, 3.0 mmol) which was produced in Example 116 (116a) was dissolved in methanol (5 mL) and 4N hydrochloric acid-1,4-dioxane solution (5 mL) was added. After the reaction liquid was stirred at room temperature for two hours, it was concentrated under reduced pressure. 1N aqueous solution of sodium hydroxide (10 mL) was added to the obtained residue and the aqueous layer was extracted with methylene chloride (3x30 mL). After the extract was dried over sodium sulfate, the solvent was evaporated under reduced pressure and the title compound was obtained 0.70 g (yield 100%).
Mp 108-112°C;
IR (KBr) νₘₐₓ 3312, 1611, 1388, 1243, 830, 766 cm⁻¹;
¹H NMR (CDCl₃, 400MHz) δ 1.75 (1H, br), 3.01-3.04 (4H, m), 3.05 (6H, s), 3.16-3.21 (4H, m), 6.87 (2H, d, J = 8.6 Hz), 7.35 (2H, d, J = 8.6);
MS (EI) m/z: 233 [M⁺], 191;
Anal. Calcd for C₁₄H₂₁N₃O·0.1 H₂O: C, 66.41; H, 8.23; N, 17.87. Found: C, 66.39; H, 8.30; N, 17.74.

### (116c) 4-(4-[(1Z)-3-amino-2-cyano-1-methyl-3-thioxoprop-1-enyl]piperazin-1-yl)-N,N-dimethylbenzamide

N,N-dimethyl-4-piperazin-1-ylbenzamide which was produced in Example 116 (116b) was used in place of isobutylamine and the reaction was performed in a similar method as described in Example 5 (5a) and the title compound was obtained.
Yield 65%.
Mp 193-196°C;
IR (KBr) νₘₐₓ 3280, 3127, 2187, 1606, 1531, 992, 767 cm⁻¹;
¹H NMR (DMSO-d₆, 400MHz) δ 2.82 (3H, s), 2.95 (6H, s), 3.38-3.41 (4H, m), 3.54-3.57 (4H, m), 6.93 (2H, d, J = 7.8 Hz), 7.32 (2H, d, J = 7.8), 8.40 (1H, br), 9.12 (1H, br);
MS (FAB) m/z: 358 [M+H]⁺;
Anal. Calcd for C₁₈H₂₃N₅SO·0.2 H₂O: C, 59.88; H, 6.53; N, 19.40; S, 8.88. Found: C, 60.08; H, 6.47; N, 19.15; S, 8.72.

### (116d) 4-[4-(3-cyano-2-thioxo-1,2-dihydropyidin-4-yl)-piperazin-1-yl]-N,N-dimethylbenzamide

4-(4-[(1Z)-3-amino-2-cyano-1-methyl-3-thioxoprop-1-enyl]piperazin-1-yl)-N,N-dimethylbenzamide which was produced in Example 116 (116c) (650 mg, 1.82 mmol) and N,N-dimethylformamide dimethylacetal (238 mg, 265 µL, 2.00 mmol) were dissolved in N,N-dimethylformamide (10 mL) and the mixture was stirred at room temperature for one hour and at 60°C for a further two hours. Water (20 mL) was added to the reaction liquid and it was allowed to stand at room temperature for two hours. The deposited solid was separated by filtration and, furthermore, washed with water and ethanol and the title compound was obtained 272 mg (yield 41%).
Mp 246-249°C;
IR (KBr) νₘₐₓ 2208, 1606, 1495, 1239, 985, 765 cm⁻¹;
¹H NMR (DMSO-d₆, 400MHz) δ 2.95 (6H, s), 3.40-3.43 (4H, m), 3.80-3.83 (4H, m), 6.53 (1H, d, J = 7.4 Hz), 6.92 (2H, d, J = 8.6 Hz), 7.30 (2H, d, J = 8.6 Hz), 7.50 (1H, d, J = 7.4 Hz), 12.70 (1H, br);
MS (FAB) m/z: 368 [M+H]⁺;
Anal. Calcd for C₁₉H₂₁N₅OS·1.2 H₂O: C, 58.65; H, 6.06; N, 18.00; S, 8.24. Found: C, 58.80; H, 6.06; N, 18.08; S, 8.18.

### (116e) 3-amino-4-(4-{4-[(dimethylamino)carbonyl)phenyl}piperazin-1-yl)thieno[2,3-b]pyridine-2-carboxamide

The reaction was performed in a similar method as described in Example 5 (5c) using 4-[4-(3-cyano-2-thioxo-1,2-dihydropyridin-4-yl)-piperazin-1-yl]-N,N-dimethylbenzamide which was produced in Example 116 (116d) and the title compound was obtained. Yield 82%.
Mp >270°C;
IR (KBr) νₘₐₓ 3447, 3327, 3180, 1652, 1625, 1606, 1577, 1238, 837, 767 cm⁻¹;
¹H NMR (DMSO-d₆, 400MHz) δ 2.96 (6H, s), 2.80-3.90 (8H, m), 6.97 (2H, br), 7.04 (2H, d, J = 8.2 Hz), 7.10 (1H, d, J = 5.1 Hz), 7.16 (2H, br), 7.34 (2H, d, J = 8.2 Hz), 8.48 (1H, d, J = 5.1 Hz);
MS (FAB) m/z: 425 [M+H]⁺;
Anal. Calcd for C₂₁H₂₄N₆O₂S·0.4 H₂O: C, 58.42; H, 5.79; N, 19.47; S, 7.43. Found: C, 58.56; H, 5.73; N, 19.48; S, 7.35.

### (Example 117) 3-amino-4-(4-{4-[2-(dimethylamino)-2-oxoethyl)phenyl}piperazin-1-yl)thieno[2,3-b]pyridine-2-carboxamide (Exemplified Compound No. 3-429)

### (117a) tert-butyl 4-{4-[2-(dimethylamino)-2-oxoethyl]phenyl}piperazine-1-carboxylate

Under a nitrogen atmosphere, 2-(4-bromophenyl) -N,N-dimethyl acetamide (J. Med. Chem. 33, (1990), 2899-2905.) (1.23 g, 5.1 mmol), tert-butyl 1-piperazinecarboxylate (1.13 g, 6.1), sodium tert-butoxide (0.69 g, 7.1 mmol), tris(dibenzylideneacetone) dipalladium (= Pd2(dba)3) (23 mg, 0.03 mmol) and 2-dicyclohexylphosphino-2'-(N,N-dimethylamino) biphenyl (39 mg, 0.10 mmol) were mixed in a mixed solvent of 1,4-dioxane (10 mL) and tert-butanol (5 mL) and the mixture was stirred under reflux for two hours.
Water (100 mL) and ethyl acetate (100 mL) were added to the reaction liquid mixture and insolubles were removed with Celite. The filtrate was partitioned and the aqueous layer was extracted with ethyl acetate (50 mL). After the combined organic layer was dried over sodium sulfate, the residue obtained by evaporating the solvent under reduced pressure was purified by silica gel column chromatography (100% ethyl acetate) and 1.59 g of the title compound was obtained (yield 91%).
Mp 110-112°C;
IR (KBr) νₘₐₓ 1689, 1639, 1431, 1169, 1129, 914 cm⁻¹;
¹H NMR (CDCl₃, 400MHz) δ 1.48 (9H, s), 2.96 (3H, s), 2.99 (3H, s), 3.09-3.11 (4H, m), 3.56-3.58 (4H, m), 3.64 (2H, s), 6.88 (2H, d, J = 8.2 Hz), 7.16 (2H, d, J = 8.2); MS (FAB) m/z: 347 [M⁺];
Anal. Calcd for C₁₉H₂₉N₃O₃: C, 65.58; H, 8.41; N, 12.09. Found: C, 65.58; H, 8.29; N, 11.89.

### (117b) N,N-dimethyl-2-(4-piperazin-1-ylphenyl)acetamide

4N hydrochloric acid-1,4-dioxane solution (15 mL) was added to a methanol (15 mL) solution of tert-butyl 4-{4-[2-(dimethylamino)-2-oxoethyl]phenyl}piperazine-1-carboxylate (1.89 g, 5.4 mmol) which was produced in Example 117 (117a) and the mixture was stirred at room temperature for two hours. 1N aqueous solution of sodium hydroxide (20 mL) was added to the residue which was obtained by concentrating the reaction mixture under reduced pressure and extracted with methylene chloride (3x50 mL). After the extract was dried over sodium sulfate, the solvent was evaporated under reduced pressure and 1.30 g of the title compound was obtained (yield 97%).
Mp 90-92°C;
IR (KBr) νₘₐₓ 3269, 1637, 1517, 1241, 1128, 896 cm⁻¹;
¹H NMR (CDCl₃, 400MHz) δ 1.85 (1H, br), 2.94 (3H, s), 2.98 (3H, s), 3.01-3.03 (4H, m), 3.10-3.13 (4H, m), 3.62 (2H, s), 6.86 (2H, d, J = 8.6 Hz), 7.13 (2H, d, J = 8.6); MS (EI) m/z: 247 [M⁺], 205, 175;
Anal. Calcd for C₁₄H₂₁N₃O·0.1 H₂O: C, 67.49; H, 8.58; N, 16.87. Found: C, 67.64; H, 8.24; N, 16.51.

### (117c) 2-(4-{4-[(1Z)-3-amino-2-cyano-1-methyl-3-thioxoprop-1-enyl)piperazin-1-yl}phenyl) -N,N-dimethyl acetamide

N,N-dimethyl-2-(4-piperazin-1-ylphenyl)acetamide which was produced in Example (117b) was used in place of isobutylamine and the reaction was performed in a similar method as described in Example 5 (5a) and the title compound was obtained. Yield 90%.
Mp 174-177°C;
IR (KBr) νₘₐₓ 3283, 3141, 2189, 1622, 1537, 1518, 1236, 991 cm⁻¹;
¹H NMR (DMSO-d₆, 400MHz) δ 2.31 (3H, s), 2.81 (3H, s), 2.97 (3H, s), 3.23-3.26 (4H, m), 3.51-3.53 (4H, m), 3.56 (2H, s), 6.88 (2H, d, J = 8.0 Hz), 7.08 (2H, d, J = 8.0 Hz), 8.40 (1H, br), 9.11 (1H, br);
MS (FAB) m/z: 372 [M + H]⁺;
Anal. Calcd for C₁₉H₂₅N₅OS·0.2 H₂O: C, 60.84; H, 6.83; N, 18.67; S, 8.55. Found: C, 61.10; H, 6.65; N, 18.55; S, 8.46.

### (117d) 2-{4-[4-(3-cyano-2-thioxo-1,2-dihydropyridin-4-yl)-piperazin-1-yl]phenyl}-N,N-dimethyl acetamide

The reaction was performed in a similar method as described in Example 116 (116d) using 2-(4-{4-[(1Z)-3-amino-2-cyano-1-methyl-3-thioxoprop-1-enyl)piperazin-1-yl}phenyl)-N,N-dimethyl acetamide which was produced in Example 117 (117c). After the reaction terminated, the reaction liquid was partitioned with water (100 mL) and ethyl acetate (100 mL). The solid deposited in the aqueous layer was separated by filtration and washed with water and ethanol and 0.274 g of the title compound was obtained (yield 19%).
Mp 248-253°C;
1H NMR (DMSO-d6, 400MHz) δ 2.81 (3H, s), 2.97 (3H, s), 3.26-3.28 (4H, m), 3.57 (2H, s), 3.79-3.81 (4H, m), 6.56 (1H, d, J = 6.7 Hz), 6.90 (2H, d, J = 8.2 Hz), 7.09 (2H, d, J = 8.2 Hz), 7.53 (1H, d, J = 6.7 Hz), 12.76 (1H, br).

### (117e) 3-amino-4-(4-{4-[2-(dimethylamino)-2-oxoethyl)phenyl}piperazin-1-yl)thieno[2,3-b]pyridine-2-carboxamide

The reaction was performed in a similar method as described in Example 5 (5c) using 2-{4-[4-(3-cyano-2-thioxo-1,2-dihydropyridin-4-yl)-piperazin-1-yl]phenyl}-N,N-dimethyl acetamide which was produced in Example 117 (117d) and the title compound was obtained. Yield 94%.
Mp >280°C;
IR (KBr) νₘₐₓ 3437,3318, 1629, 1571, 1382, 1244, 978, 962 cm⁻¹;
¹H NMR (DMSO-d₆, 400MHz) δ 2.82 (3H, s), 2.98 (3H, s), 2.80-3.80 (8H, m), 3.58 (2H, s), 6.95-6.97 (4H, m), 7.09-7.11 (4H, m), 7.13 (1H, d, J = 5.1 Hz), 8.47 (1H, d, J = 5.1 Hz);
MS (FAB) m/z: 439 [M + H]⁺;
Anal. Calcd for C₂₂H₂₆N₆O₂S·0.4 H₂O: C, 59.28; H, 6.06; N, 18.85; S, 7.19. Found: C, 59.43; H, 6.02; N, 18.98; S, 7.05.

### (Example 118) 3-amino-4-(4-{3-[2-(dimethylamino)-2-oxoethyl)phenyl}piperazin-1-yl)thieno[2,3-b]pyridine-2-carboxamide (Exemplified Compound No. 3-430)

### (118a) tert-butyl 4-{3-[2-(dimethylamino)-2-oxoethyl]phenyl}piperazine-1-carboxylate

The reaction was performed in a similar method as described in Example 117 (117a) using 2-(3-bromophenyl)-N,N-dimethyl acetamide (Arch.Pharm. (Weinheim) 321,315-320 (1988)) and the title compound was obtained as a viscous oil. Yield 92%.
IR (neat) νₘₐₓ 1685, 1635, 1241, 1171, 1124, 998, 772 cm⁻¹;
¹H NMR (CDCl₃, 400MHz) δ 1.48 (9H, s), 2.96 (3H, s), 2.99 (3H, s), 3.12-3.14 (4H, m), 3.55-3.57 (4H, m), 3.68 (2H, s), 6.76 (1H, brd. J = 8.0 Hz), 6.81 (1H, dd, J = 2.0, 8.0 Hz), 6.86 (1H, brs), 7.21 (1H, t, J = 8.0 Hz);
MS (FAB) m/z: 347 [M⁺];
Anal. Calcd for C₁₉H₂₉N₃O₃·0.2 H₂O: C, 65.01; H, 8.44; N, 11.97. Found: C, 65.14; H, 8.08; N, 11.89.

### (118b) N,N-dimethyl-2-(3-piperazin-1-ylphenyl)acetamide

The reaction was performed in a similar method as described in Example 117 (117b) using tert-butyl 4-{3-[2-(dimethylamino)-2-oxoethyl]phenyl}piperazine-1-carboxylate which was produced in (118a) and an oily title compound was synthesized. Yield 99%.
IR (neat) νₘₐₓ 3456, 3316, 1640, 1244, 995, 772 cm⁻¹;
¹H NMR (CDCl₃, 400MHz) δ 2.08 (1H, br), 2.96 (3H, s), 2.99 (3H, s), 3.02-3.05 (4H, m), 3.15-3.17 (4H, m), 3.68 (2H, s), 6.74 (1H, brd, J = 7.4 Hz), 6.81 (1H, dd, J = 2.3, 8.2 Hz), 6.85 (1H, brs), 7.20 (1H, dd, J = 7.4, 8.2 Hz);
MS (EI) m/z: 247 [M⁺], 205.

### (118c) 2-{3-[4-(3-cyano-2-thioxo-1,2-dihydropyridin-4-yl)-piperazin-1-yl]phenyl}-N,N-dimethyl acetamide

N,N-dimethyl-2-(3-piperazin-1-ylphenyl)acetamide (2.68 g, 10.8 mmol) which was produced in Example 118 (118b) and (2Z)-2-cyano-3-ethoxybut-2-enethioamide (J. Org. Chem. (1962), 27,2433-2439) (1.54 g, 9.0 mmol) were mixed in ethanol (30 mL) and the mixture was stirred at room temperature for 15 hours. The residue which was obtained by evaporating the solvent under reduced pressure was dissolved in N,N-dimethylformamide (30 mL) and N,N-dimethylformamide dimethylacetal (1.29 g, 1.44 mL, 10.8 mmol) was added. The reaction mixture was stirred at room temperature for one hour and further at 80°C for two hours. After cooling to room temperature, the reaction mixture was partitioned with a saturated saline solution (100 mL) and ethyl acetate (100 mL) and the aqueous layer was extracted with methylene chloride (3x50 mL). After the extract was dried over sodium sulfate, the solvent was evaporated under reduced pressure. The obtained residue was purified by silica gel column chromatography (methylene chloride/methanol =20:1) and 0.329 g of the title compound was obtained (yield 10%).
Mp 250-254°C;
IR (KBr) νₘₐₓ, 2206, 1602, 1496, 1238, 985, 772 cm⁻¹;
¹H NMR (DMSO-d₆, 400MHz) δ 2.81 (3H, s), 2.98 (3H, s), 3.27-3.29 (4H, m), 3.61 (2H, s), 3.78-3.81 (4H, m), 6.53 (1H, d, J = 7.4 Hz), 6.65 (1H, brd, J = 7.4 Hz), 6.78-6.80 (2H, m), 7.14 (1H, dd, J = 7.4, 9.0 Hz), 7.49-7.52 (1H, m), 12.72 (1H, br);
MS (FAB) m/z: 382 [M + H]⁺;
Anal. Calcd for C₂₀H₂₃N₅OS·0.4 H₂O: C, 61.80; H, 6.17; N, 18.02; S, 8.25. Found: C, 61.77; H, 6.07; N, 18.04; S, 8.18.

### (118d) 3-amino-4-(4-{3-[2-(dimethylamino)-2-oxoethyl)phenyl}piperazin-1-yl)thieno[2,3-b]pyridine-2-carboxamide

The reaction was performed in a similar method as described in Example 5 (5c) using 2-{3-[4-(3-cyano-2-thioxo-1,2-dihydropyridin-4-yl)-piperazin-1-yl]phenyl}-N,N-dimethyl acetamide which was produced in (118c) and the title compound was obtained. Yield 78%.
Mp 273-275°C;
IR (KBr) νₘₐₓ 3439, 3316, 1632, 1579, 1370, 1240, 976, 771 cm⁻¹;
¹H NMR (DMSO-d₆, 400MHz) δ 2.83 (3H, s), 3.00 (3H, s), 2.80-3.80 (8H, m), 3.64 (2H, s), 6.59 (1H, d, J = 7.4 Hz), 6.87-6.90 (2H, m), 6.97 (2H, brs), 7.10 (1H, d, J = 5.5 Hz), 7.15 (2H, brs), 7.18 (1H, dd, J = 7.4, 8.6 Hz), 8.48 (1H, d, J = 5.5 Hz);
MS (FAB) m/z: 439 [M + H]⁺;
Anal. Calcd for C₂₂H₂₆N₆O₂S·0.1 H₂O: C, 60.01; H, 6.00; N, 19.08; S, 7.28. Found: C, 59.97; H, 5.97; N, 19.09; S, 7.07.

### (Example 119) 3-amino-4-{4-[4-(1-hydroxyethyl)phenyl]-1,4-diazepan-1-yl}thieno[2,3-b]pyridine-2-carboxamide (Exemplified Compound No. 3-1007)

Sodium borohydride (95 mg, 2.5 mmol) was added to a methanol (6 mL) suspension of 4-[4-(4-acetylphenyl)-1,4-diazepan-1-yl]-3-aminothieno[2,3-b]pyridine-2-carboxamide (103 mg, 0.25 mmol) which was produced in Example 94, and heated under reflux for two days. Water (30 mL) was added to the reaction liquid and the deposited solid was separated by filtration and the title compound was obtained (95 mg, yield 92%).
Yellow powder
Mp 244-248°C;
IR (KBr) νₘₐₓ 3450, 3337, 1646, 1609, 1518, 1368, 1187, 823 cm⁻¹;
¹H NMR(DMSO-d₆, 500 MHz) δ 1.29 (3H, d, J = 6.4 Hz), 2.12-2.14 (2H, m), 3.20 (2H, brs), 3.29 (2H, brs), 3.54 (2H, t, J = 6.3 Hz), 3.76 (2H, t, J = 5.0 Hz), 4.60 (1H, t, J = 4.9 Hz), 4.83 (1H, d, J = 4.4 Hz), 6.72 (2H, d, J = 8.2 Hz), 6.97 (2H, brs), 7.08 (1H, d, J = 5.4 Hz), 7.08 (2H, brs), 7.15 (2H, d, J = 8.8 Hz), 8.40 (1H, d, J = 5.4 Hz); HRMS m/z calcd for C₂₁H₂₆O₂N₅S 412.1807, found 412.1815;
MS (FAB) m/z: 411 [M⁺], 394, 273, 242, 183, 166, 120, 65, 51, 39, 31;
Anal. Calcd for C₂₁H₂₅N₅O₂S·0.34H₂O: C, 60.39; H, 6.20; N, 16.77; S, 7.68. Found: C, 60.32; H, 6.06; N, 16.72; S, 7.61.

### (Example 120) 3-amino-4- {4-[4-(N-hydroxyethaneimidoyl)phenyl]-1,4-diazepan-1-yl}thieno[2,3-b]pyridine-2-carboxamide (Exemplified Compound No. 3-1037)

Hydroxylamine hydrochloride (97 mg, 1.4 mmol) and sodium acetate (115 mg, 1.4 mmol) were added to a methanol (3 mL) suspension of 4-[4-(4-acetylphenyl)-1,4-diazepan-1-yl]-3-aminothieno[2,3-b]pyridine-2-carboxamide (56 mg, 0.14 mmol) which was produced in Example 94, and heated under reflux for two days. Water (10 mL) was added to the reaction liquid and the deposited solid was separated by filtration and the title compound was obtained (33 mg, yield 56%).
Pale yellow powder
Mp 238-241°C;
IR (KBr) νₘₐₓ 3431, 3317, 3169, 1643, 1601, 1521, 1370, 1200, 908, 826, 561, 482 cm⁻¹;
¹H NMR(DMSO-d₆, 500 MHz) δ 2.09 (3H, s), 2.13-2.16 (2H, m), 3.19 (2H, brs), 3.29-3.32 (2H, m), 3.59 (2H, t, J = 6.4 Hz), 3.80 (2H, t, J = 4.9 Hz), 6.78 (2H, d, J = 8.8 Hz), 6.99 (2H, brs), 7.09 (1H, d, J = 5.4 Hz), 7.09 (2H, brs), 7.49 (2H, d, J = 8.8 Hz), 8.41 (1H, d, J = 5.4 Hz), 10.71 (1H, s);
HRMS m/z calcd for C₂₁H₂₅O₂N₆S 425.1760, found 425.1761;
MS (FAB) m/z: 425 [M⁺], 407, 273, 246, 228, 182, 65;
Anal. Calcd for C₂₁H₂₄N₆O₂S·0.5H₂O: C, 58.18; H, 5.81; N, 19.39; S, 7.40. Found: C, 58.39; H, 5.80; N, 19.26; S, 7.27.

### (Example 121) 3-amino-4-[4-(4-propionylphenyl)-1,4-diazepan-1-yl]thieno[2,3-b]pyridine-2-carboxamide (Exemplified Compound No. 3-100)

### (121a) tert-butyl 4-(4-propionylphenyl)-1,4-diazepane-1-carboxylate

4-fluoropropiophenone was used in place of 4-fluoro nitrobenzene and the reaction was performed in a similar method as described in Example 59 (59a) and the title compound was obtained (yield 45%).
Yellow oil
IR (film) vₘₐₓ 2975, 2936, 1693, 1669, 1599, 1523, 1416, 1365 cm⁻¹;
¹H NMR(CDCl₃, 400 MHz) δ 1.20 (3H, t, J = 7.4 Hz), 1.35 (4.5H, s), 1.42 (4.5H, s), 1.92-2.02 (2H, m), 2.89 (2H, q, J = 7.4 Hz), 3.18-3.24 (1H, m), 3.29-3.34 (1H, m), 3.54-3.66 (6H, m), 6.66 (2H, d, J = 9.0 Hz), 7.85 (2H, d, J = 9.0 Hz);
MS (FAB) m/z: 332 M⁺, 277, 259, 231.

### (121b) 1-[4-(1,4-diazepan-1-yl)phenyl]propan-1-one

The reaction was performed in a similar method as described in Example 57 (57b) using tert-butyl 4-(4-propionylphenyl)-1,4-diazepane-1-carboxylate which was produced in Example 121 (121a) and the title compound was obtained (yield 95%). Pale brown prism crystal
Mp 97-98°C;
IR (KBr) vₘₐₓ 3343, 2935, 2821, 1652, 1597, 1524, 1407 cm⁻¹;
¹H NMR(CDCl₃, 400 MHz) δ 1.20 (3H, t, J = 7.4 Hz), 1.87-1.95 (2H, m), 2.80-2.85 (2H, m), 2.89 (2H, q, J = 7.4 Hz), 3.01-3.07 (2H, m), 3.58-3.67 (4H, m), 6.65 (2H, d, J = 9.0 Hz), 7.85 (2H, d, J = 9.0 Hz);
MS (EI) m/z: 232 M⁺, 203, 190, 176.

### (121c) 4-[4-(4-propionylphenyl)-1,4-diazepan-1-yl]-2-thioxo-1,2-dihydropyridine-3-carbonitrile

N,N-dimethylformamide (8.47 mL) solution of 1-[4-(1,4-diazepan-1-yl)phenyl]propan-1-one (984 mg, 4.24 mmol) produced in Example 121 (121b) was blended with (2Z) -2-cyano-3-ethoxybut-2-enethioamide (J.Org.Chem. (1962), 27,2433-2439) (721 mg, 4.24 mmol) and the mixture was stirred at room temperature for 15 minutes. Then N,N-dimethylformamide dimethylacetal (619 µL, 4.66 mmol) was added and after stirring for one hour, it was heated and the mixture was stirred at 80°C for 30 minutes. After the reaction liquid was allowed to be cooled to room temperature, it was blended with ethyl acetate (8.5 mL) and water (21 mL) and the mixture was stirred for 30 minutes. The deposited crystal was separated by filtration, washed sequentially with ethyl acetate and water and then dried and 508 mg of the title compound (yield 33%) was obtained.
Pale brown powder
Mp 157-158°C;
IR (KBr) νₘₐₓ 3443, 3353, 3277, 3176, 2926, 2182, 1617, 1525, 1346 cm⁻¹;
¹H NMR(DMSO-d₆, 500 MHz) δ 1.04 (2H, t, J = 7.3 Hz), 1.90-1.99 (2H, m), 2.87 (2H, q, J = 7.3 Hz), 3.63 (2H, t, J = 5.9 Hz), 3.73-3.78 (2H, m), 3.83-3.88 (2H, m), 3.95-4.00 (2H, m), 6.44 (1H, d, J = 7.8 Hz), 6.84 (2H, d, J = 8.8 Hz), 7.37 (1H, d, J = 7.8 Hz), 7.78 (2H, d, J = 8.8 Hz), 12.53 (1H, br.s);
MS (FAB) m/z: 367 [M+H]⁺, 273, 246, 228;
Anal. Calcd for C₂₀H₂₂N₄OS·0.4H₂O: C, 64.28; H, 6.15; N, 14.99; S, 8.58. Found: C, 64.15; H, 6.23; N, 15.26; S, 8.50.

### (121d) 3-amino-4-[4-(4-propionylphenyl)-1,4-diazepan-1-yl]thieno[2,3-b]pyridine-2-carboxamide

The reaction was performed in a similar method as described in Example 5 (5c) using 4-[4-(4-propionylphenyl)-1,4-diazepan-1-yl]-2-thioxo-1,2-dihydropyridine-3-carbonitrile which was produced in Example 121 (121c) and the title compound was obtained. Yield 65%.
Pale yellow powder
Mp 231-233°C;
IR (KBr) νₘₐₓ 3440, 3326, 3186, 2934, 1648, 1597, 1368 cm⁻¹;
¹H NMR(DMSO-d₆, 400 MHz) δ 1.06 (3H, t, J = 7.4 Hz), 2.11-2.21 (2H, m), 2.89 (2H, q, J = 7.4 Hz), 3.13-3.24 (2H, m), 3.25-3.34 (2H, m), 3.64 (2H, t, J = 5.1 Hz), 3.82-3.90 (2H, m), 6.82 (2H, d, J = 9.0 Hz), 6.97 (2H, br.s), 7.06 (1H, d, J = 5.1 Hz), 7.09 (2H, br.s), 7.79 (2H, d, J = 9.0 Hz), 8.38 (1H, d, J = 5.1 Hz);
MS (FAB) m/z: 424 [M+H]⁺, 423, 407;
Anal. Calcd for C₂₂H₂₅N₅O₂S·0.4H₂O: C, 61.35; H, 6.04; N, 16.26; S, 7.44. Found: C, 61.43; H, 6.23; N, 16.10; S, 7.34.

### (Example 122) 3-amino-4-(4-{4-[(2-methyl-1,3-dioxolan-2-yl)methyl]phenyl}-1,4-diazepan-1-yl)thieno[2,3-b]pyridine-2-carboxamide (Exemplified Compound No. 3-1082)

### (122a) 2-(4-bromobenzyl)-2-methyl-1,3-dioxolane

Ethylene glycol (1.67 mL, 30.0 mmol), p-toluenesulfonic acid monohydrate (380 mg, 2.0 mmol) were added to toluene (100 mL) solution of 1-(4-bromophenyl) acetone (4.26 g, 20.0 mmol) and the mixture was stirred at 120°C for nine hours. The solvent was evaporated under reduced pressure after an excess amount of triethylamine was added to the reaction liquid. The obtained residual substance was purified by silica gel column chromatography (hexane/ethyl acetate = 10:1) and the title compound was obtained (4.80 g, 93%).
Pale yellow liquid
IR (film) νₘₐₓ 2983, 2882, 1489, 1376, 1128, 1047, 832 cm⁻¹;
¹H NMR(CDCl₃, 400 MHz) δ 1.30 (3H, s), 2.87 (2H, s), 3.67-3.73 (2H, m), 3.84-3.92 (2H, m), 7.13 (2H, d, J = 8.6 Hz), 7.38 (2H, d, J = 8.6 Hz);
MS (EI) m/z: 256 [M⁺], 243, 241, 171, 169, 87, 43.

### (122b) benzyl 4-{4-[(2-methyl-1,3-dioxolan-2-yl)methyl]phenyl}-1,4-diazepane-1-carboxylate

Reaction with benzyl 1-homopiperazinecarboxylate was performed in a similar method as described in 117 (117a) using 2-(4-bromobenzyl)-2-methyl-1,3-dioxolane which was produced in Example 122 (122a) and the title compound was obtained.
Yellow liquid
IR (film) νₘₐₓ 2942, 1700, 16.14, 1520, 1421, 1222, 1118, 1046, 927 cm⁻¹;
¹H NMR(CDCl₃, 400 MHz) δ 1.29 (3H, s), 1.91-2.07 (2H, m), 2.81 (2H, s), 3.30 (1H, t, J = 5.9 Hz), 3.37 (1H, t, J = 5.9 Hz), 3.49-3.68 (6H, m), 3.76-3.83 (2H, m), 3.88-3.93 (2H, m), 5.09 (1H, s), 5.33 (1H, s), 6.62 (2H, d, J = 8.6 Hz), 7.11 (2H, d, J = 8.6 Hz), 7.26-7.39 (5H, m);
MS (FAB) m/z: 411 [M+H⁺], 323, 275, 87.

### (122c) 1-{4-[(2-methyl-1,3-dioxolan-2-yl) methyl]phenyl}-1,4-diazepane

Hydrogenolysis reaction was performed in the presence of a palladium-carbon catalyst in ethanol using benzyl 4-{4-[(2-methyl-1,3-dioxolan-2-yl)methyl]phenyl}-1,4-diazepane-1-carboxylate which was produced in Example 122 (122b), and the title compound was obtained.
Pale yellow liquid
IR (film) νₘₐₓ 3319, 2935, 1614, 1520, 1375, 1191, 1045, 813 cm⁻¹;
¹H NMR(CDCl₃, 400 MHz) δ 1.30 (3H, s), 1.82-1.93 (2H, m), 2.80 (2H, s), 2.82 (2H, t, J = 5.9 Hz), 3.02 (2H, t, J = 5.5 Hz), 3.53 (2H, t, J = 5.5 Hz), 3.56 (2H, t, J = 5.9 Hz), 3.76-3.82 (2H, m), 3.87-3.93 (2H, m), 6.63 (2H, d, J = 8.6 Hz), 7.10 (2H, d, J = 8.6 Hz);
MS (EI) m/z: 276 [M⁺], 190, 189, 87.

### (122d) 4-(4-{4-[(2-methyl-1,3-dioxolan-2-yl)methyl]phenyl}-1,4-diazepan-1-yl)-2-thioxo-1,2-dihydropyridine-3-carbonitrile

The reaction was performed following a method described in Example 121 (121c) using 1-{4-[(2-methyl-1,3-dioxolan-2-yl) methyl]phenyl}-1,4-diazepane which was produced in Example 122 (122c) and the title compound was obtained. Brown powder
Mp 204-206°C;
IR (KBr) νₘₐₓ 2949, 2206, 1625, 1518, 1353, 1247, 1139, 1043, 930, 781 cm⁻¹;
¹H NMR(DMSO-d₆, 500 MHz) δ 1.15 (3H, s), 1.90-1.97 (2H, m), 2.67 (2H, s), 3.51 (2H, t, J = 5.9 Hz), 3.57-3.64 (2H, m), 3.68-3.80 (6H, m), 3.94 (2H, t, J = 5.9 Hz), 6.41 (1H, d, J = 7.8 Hz), 6.67 (2H, d, J = 8.3 Hz), 7.01 (2H, d, J = 8.3 Hz), 7.34 (1H, d, J = 7.8 Hz), 12.50 (1H,brs);
MS (FAB) m/z: 411 [M+H⁺], 367, 338, 273, 246;
Anal. Calcd for C₂₂H₂₆N₄O₂S·0.25H₂O: C, 63.67; H, 6.44; N, 13.50. Found: C, 63.90; H, 6.18; N, 13.45.

### (122e) 3-amino-4-(4-{4-[(2-methyl-1,3-dioxolan-2-yl)methyl]phenyl}-1,4- diazepan-1-yl)thieno[2,3-b]pyridine-2-carboxamide

The reaction was performed following a method described in Example 5 (5c) using 4-(4- {4-[(2-methyl-1,3-dioxolan-2-yl)methyl]phenyl} -1,4-diazepan-1 -yl)-2-thioxo-1,2-dihydropyridine-3-carbonitrile which was produced in Example 122 (122d) and the title compound was obtained.
Pale yellow powder
Mp 227-229°C;
IR (KBr) νₘₐₓ 3446, 3333, 3143, 2923, 1643, 1575, 1515, 1372, 1212, 1044, 825 cm⁻¹;
¹H NMR(DMSO-d₆, 400 MHz) δ 1.16 (3H, s), 2.06-2.19 (2H, m), 2.70 (2H, s), 3.13-3.22 (2H, m), 3.23-3.32 (2H, m), 3.51 (2H, t, J = 5.9 Hz), 3.69-3.86 (6H, m), 6.66 (2H, d, J = 8.6 Hz), 6.98 (2H, brs), 7.02 (2H, d, J = 8.6 Hz), 7.05 (1H, d, J = 5.5 Hz), 7.07 (2H, brs), 8.37 (1H, d, J = 5.5 Hz);
MS (FAB) m/z: 468 [M+H⁺], 273, 246, 165, 93, 63;
Anal. Calcd for C₂₄H₂₉N₅O₃S·0.20H₂O: C, 61.18; H, 6.29; N, 14.86. Found: C, 61.19; H, 6.25; N, 14.81.

### (Example 123) 3-amino-4-{4-[4-(2-oxopropyl)phenyl]-1,4-diazepan-1-yl}thieno[2,3-b]pyridine-2-carboxamide (Exemplified Compound No. 3-1019)

1N hydrochloric acid (2.0 mL) was added to a methanol (2.0 mL) solution of 3-amino-4-(4- {4-[(2-methyl-1,3-dioxolan-2-yl)methyl]phenyl} -1,4-diazepan-1-yl)thieno[2,3-b]pyridine-2-carboxamide (200 mg, 428 µ mol) which was produced in Example 122 and the mixture was stirred at room temperature for two hours. The solvent was evaporated under reduced pressure after an excess amount of triethylamine was added to the reaction liquid. The obtained residual substance was blended with methylene chloride/2-propanol (4:1) (100 mL) and washed with water (50 mL) and the solvent was evaporated under reduced pressure after drying over sodium sulfate. The obtained powder was washed with ethanol and the title compound was obtained (160 mg, 88%).
Pale yellow powder
Mp 225-230°C;
IR (KBr) νₘₐₓ 3440, 3325, 3150, 2946, 1648, 1576, 1517, 1370, 1232, 939, 819 cm⁻¹; ¹H NMR(DMSO-d₆, 500 MHz) δ 2.08 (3H, s), 2.09-2.18 (2H, m), 3.17-3.23 (2H, m), 3.25-3.33 (2H, m), 3.53 (2H, t, J = 5.4 Hz), 3.57 (2H, s), 3.75 (2H, t, J = 5.4 Hz), 6.73 (2H, d, J = 8.8 Hz), 7.00 (2H, brs), 7.01 (2H, d, J = 8.8 Hz), 7.07 (1H, d, J = 5.4 Hz), 7.09 (2H, brs), 8.40 (1H, d, J = 5.4 Hz);
MS (FAB) m/z: 424 [M+H⁺], 407, 380, 273, 258, 220, 165, 63;
Anal. Calcd for C₂₂H₂₅N₅O₂S·0.32H₂O: C, 61.55; H, 6.02; N, 16.31. Found: C, 61.94; H, 6.12; N, 15.88.

### (Example 124) 3-amino-4-{4-[4-(2-hydroxyethyl)phenyl]-1,4-diazepan-1-yl}thieno[2,3-b]pyridine-2-carboxamide (Exemplified Compound No. 3-1003)

### (124a) benzyl 4-[4-(2-hydroxyethyl)phenyl]-1,4-diazepane-1-carboxylate

The reaction was performed following a method described in Example 117 (117a) using benzyl 1-homopiperazinecarboxylate and 4-bromophenethyl alcohol and the title compound was obtained.
Yellow liquid
IR (film) νₘₐₓ 3444, 2939, 1696, 1519, 1423, 1230, 1040 cm⁻¹;
¹H NMR(CDCl₃, 500 MHz) δ 1.91-2.05 (2H, m), 2.76 (2H, t, J = 6.8 Hz), 3.31 (1H, t, J = 5.9 Hz),3.38 (1H, t, J = 5.9 Hz), 3.49-3.68 (6H, m), 3.77-3.85 (2H, m), 5.09 (1H, s), 5.13 (1H, s), 6.62-6.68 (m, 2H), 7.05-7.10 (2H, m), 7.27-7.38 (5H, m);
MS (EI) m/z: 354 [M⁺], 323, 263, 233, 202, 190, 176, 91.

### (124b) 2-[4-(1,4-diazepan-1-yl)phenyl]ethanol

Hydrogenolysis reaction was performed in the presence of a palladium-carbon catalyst in ethanol using benzyl 4-[4-(2-hydroxyethyl)phenyl]-1,4-diazepane-1-carboxylate which was produced in Example 124 (124a) and the title compound was obtained.
Yellow liquid
IR (film) νₘₐₓ 3399, 2934, 1615, 1520, 1414, 1190, 1048, 808 cm⁻¹;
¹H NMR(CDCl₃, 400 MHz) δ 1.83-1.94 (2H, m), 2.76 (2H, t, J = 6.6 Hz), 2.83 (2H, t, J = 5.9 Hz), 3.02 (2H, t, J = 5.5 Hz), 3.53 (2H, t, J = 5.5 Hz), 3.56 (2H, t, J = 5.9 Hz), 3.80 (2H, t, J = 6.6 Hz), 6.66 (2H, d, J = 9.0 Hz), 7.07 (2H, d, J = 9.0 Hz);
MS (EI) m/z: 220 [M⁺], 56.

### (124c) 4-{4-[4-(2-hydroxyethyl)phenyl]-1,4-diazepan-1-yl}-2-thioxo-1,2-dihydropyridine-3-carbonitrile

The reaction was performed following a method described in Example 121 (121c) using 2-[4-(1,4-diazepan-1-yl)phenyl]ethanol which was produced in Example 124 (124b) and the title compound was synthesized.
Brown powder
Mp 180-185°C;
IR (KBr) νₘₐₓ 2939, 2206, 1625, 1517, 1354, 1250, 1042, 928, 805 cm⁻¹;
¹H NMR(DMSO-d₆, 400 MHz) δ 1.88-1.97 (2H, m), 2.57 (2H, t, J = 7.4 Hz), 3.44-3.55 (4H, m), 3.66-3.74 (4H, m), 3.90-3.97 (2H, m), 4.53 (1H, brs), 6.41 (1H, d, J = 7.4 Hz), 6.67 (2H, d, J = 8.6 Hz), 6.98 (2H, d, J = 8.6 Hz), 7.34 (1H, d, J = 7.4 Hz), 12.50 (1H, brs);
MS (FAB) m/z: 355 [M+H⁺], 273, 257, 242, 176, 165, 120, 63;
Anal. Calcd for C₁₉H₂₂N₄OS·0.25H₂O: C, 63.57; H, 6.32; N, 15.61. Found: C, 63.36; H, 6.00; N, 15.61.

### (124d) 3-amino-4-{4-[4-(2-hydroxyethyl)phenyl]-1,4-diazepan-1-yl}thieno[2,3-b]pyridine-2-carboxamide

The reaction was performed following a method described in Example 5 (5c) using 4-{4-[4-(2-hydroxyethyl)phenyl]-1,4-diazepan-1-yl}-2-thioxo-1,2-dihydropyridine-3-carbonitrile which was produced in Example 124 (124c) and the title compound was synthesized.
White powder
Mp 257-262°C;
IR (KBr) νₘₐₓ 3445, 3324, 3151, 2939, 1655, 1576, 1516, 1370, 1232, 1045, 938, 807 cm⁻¹;
¹H NMR(DMSO-d₆, 500 MHz) δ 2.07-2.16 (2H, m), 2.59 (2H, t, J = 7.4 Hz), 3.13-3.23 (2H, m), 3.24-3.30 (2H, m), 3.47-3.56 (4H, m), 3.67-3.77 (2H, m), 4.54 (1H, t, J = 5.1 Hz), 6.67 (2H, d, J = 8.6 Hz), 6.95 (2H, brs), 6.99 (2H, d, J = 8.6 Hz), 7.05 (1H, d, J = 5.5 Hz), 7.07 (2H, brs), 8.37 (1H, d, J = 5.5 Hz);
MS (FAB) m/z: 412 [M+H⁺], 242, 165, 120, 63;
Anal. Calcd for C₂₁H₂₅N₅O₂S·0.30H₂O: C, 60.50; H, 6.19; N, 16.80. Found: C, 60.43; H, 6.08; N, 16.79.

### (Example 125) 3-amino-4-{4-[4-(2-methoxyethyl)phenyl]-1,4-diazepan-1-yl}thieno[2,3-b]pyridine-2-carboxamide (Exemplified Compound No. 3-876)

### (125a) 1-bromo-4-(2-methoxyethyl)benzene

N,N-dimethylformamide (20 mL) solution of 4-bromophenethyl alcohol (2.01 g, 10.0 mmol) was cooled to 0°C and was blended with sodium hydride (55% oily, 436 mg, 10.0 mmol) and the mixture was stirred at 0°C for 10 minutes. The reaction mixture was blended with methyl iodide (0.75 mL, 12.0 mmol) and the mixture was stirred at room temperature for a further three hours. Water (50 mL) was added to the reaction liquid and extracted with ethyl acetate (50 mL) three times. The organic layers were combined and washed with water (50 mL) and a saturated saline solution (50 mL), and the solvent was evaporated under reduced pressure after drying over sodium sulfate and the title compound was obtained (2.15 g, yield 100%). Yellow liquid
IR (film) νₘₐₓ 2925, 1489, 1382, 1191, 1117, 1011, 804 cm⁻¹;
¹H NMR(CDCl₃, 400 MHz) δ 2.83 (2H, t, J = 7.0 Hz), 3.34 (3H, s), 3.58 (2H, t, J = 7.0 Hz), 7.10 (2H, d, J = 8.2 Hz), 7.41 (2H, d, J = 8.2 Hz);
MS (EI) m/z: 214 [M⁺], 171, 169, 135, 104, 90, 45.

### (125b) benzyl 4-[4-(2-methoxyethyl)phenyl]-1,4-diazepane-1-carboxylate

Reaction with benzyl 1-homopiperazinecarboxylate was performed following a method described in Example 117 (117a) using 1-bromo-4-(2-methoxyethyl)benzene which was produced in Example 125 (125a) and the title compound was synthesized.
Yellow liquid
IR (film) νₘₐₓ 2932, 1699, 1615, 1520, 1421, 1228, 928 cm⁻¹;
¹H NMR(CDCl₃, 400 MHz) δ 1.88-2.05 (2H, m), 2.78 (2H, t, J = 7.0 Hz), 3.25-3.41 (5H, m), 3.47-3.67 (8H, m), 5.08 (1H, s), 5.13 (1H, s), 6.59-6.67 (2H, m), 7.03-7.10 (2H, m), 7.27-7.38 (5H, m);
MS (EI) m/z: 368 [M⁺], 324, 323, 277, 233, 216, 190, 178, 160, 146, 118, 107, 91, 70, 44.

### (125c) 1-[4-(2-methoxyethyl)phenyl]-1,4-diazepane

Hydrogenolysis reaction was performed in the presence of a palladium-carbon catalyst in ethanol using benzyl 4-[4-(2-methoxyethyl)phenyl]-1,4-diazepane-1-carboxylate which was produced in Example 125 (125b) and the title compound was obtained.
Yellow liquid
IR (film) νₘₐₓ 3327, 2930, 1615, 1520, 1393, 1191, 1113, 807 cm⁻¹;
¹H NMR(CDCl₃, 400 MHz) δ 1.83-1.92 (2H, m), 2.77 (2H, t, J = 7.0 Hz), 2.82 (2H, t, J = 5.9 Hz), 3.01 (2H, t, J = 5.9 Hz), 3.35 (3H, s), 3.49-3.57 (6H, m), 6.62 (2H, d, J = 8.6 Hz), 7.04 (2H, d, J = 8.6 Hz);
MS (FAB) m/z: 234 [M⁺], 204, 189, 178, 164, 146, 132, 118, 105, 91, 70.

### (125d) 4-{4-[4-(2-methoxyethyl)phenyl]-1,4-diazepan-1-yl}-2-thioxo-1,2-dihydropyridine-3-carbonitrile

The reaction was performed following a method described in Example 121 (121c) using 1-[4-(2-methoxyethyl)phenyl]-1,4-diazepane which was produced in Example 125 (125c) and the title compound was synthesized.
Brown powder
Mp 182-185°C;
IR (KBr) νₘₐₓ 2930, 2204, 1625, 1518, 1353, 1241, 1109, 929, 805 cm⁻¹;
¹H NMR(DMSO-d₆, 500 MHz) δ 1.89-1.98 (2H, m), 2.65 (2H, t, J = 7.3 Hz), 3.22 (3H, s), 3.44 (2H, t, J = 7.3 Hz), 3.50 (2H, t, J = 5.9 Hz), 3.68-3.75 (4H, m), 3.92-3.97 (2H, m), 6.42 (1H, d, J = 7.8 Hz), 6.70 (2H, d, J = 8.8 Hz), 7.01 (2H, d, J = 8.8 Hz), 7.36 (1H, d, J = 7.8 Hz), 12.50 (1H, brs);
MS (FAB) m/z: 369 [M+H⁺], 336, 323, 273, 246, 165;
Anal. Calcd for C₂₀H₂₄N₄OS·0.25H₂O: C, 64.40; H, 6.62; N, 15.02. Found: C, 64.43; H, 6.52; N, 14.80.

### (125e) 3-amino-4-{4-[4-(2-methoxyethyl)phenyl]-1,4-diazepan-1-yl}thieno[2,3-b]pyridine-2-carboxamide

The reaction was performed following a method described in Example 5 (5c) using
4-{4-[4-(2-methoxyethyl)phenyl]-1,4-diazepan-1-yl}-2-thioxo-1,2-dihydropyridine-3-carbonitrile which was produced in Example 125 (125d) and the title compound was synthesized.
Pale yellow powder
Mp 230-235°C;
IR (KBr) νₘₐₓ 3443, 3169, 2935, 1655, 1573, 1518, 1452, 1369, 1231, 1102, 938, 807 cm⁻¹;
¹H NMR(DMSO-d₆, 400 MHz) δ 2.09-2.17 (2H, m), 2.68 (2H, t, J = 7.3 Hz), 3.15-3.23 (2H, m), 3.24 (3H, s), 3.26-3.31 (2H, m), 3.46 (2H, t, J = 7.3 Hz), 3.52 (2H, t, J = 5.9 Hz), 3.71-3.78 (2H, m), 6.69 (2H, d, J = 8.8 Hz), 6.98 (2H, brs), 7.03 (2H, d, J = 8.8 Hz), 7.07 (1H, d, J = 5.4 Hz), 7.08 (2H, brs), 8.40 (1H, d, J = 5.4 Hz);
MS (FAB) m/z: 426 [M+H⁺], 409, 380, 273, 230;
Anal. Calcd for C₂₂H₂₇NₛO₂S·0.25H₂O: C, 61.44; H, 6.45; N, 16.28. Found: C, 61.26; H, 6.40; N, 16.09.

### (Example 126) 3-amino-4-{4-[3-(2-hydroxyethyl)phenyl]-1,4-diazepan-1-yl} thieno[2,3-b]pyridine-2-carboxamide (Exemplified Compound No. 3-1004)

### (126a) 1-[2-(benzyloxy)ethyl]-3-bromobenzene

N,N-dimethylformamide (20 mL) solution of 3-bromophenethyl alcohol (2.04 g, 10.0 mmol) was cooled to 0°C and was blended with sodium hydride (55% oily, 436 mg, 10.0 mmol) and the mixture was stirred at 0°C for 15 minutes. Then the reaction mixture was blended with methyl iodide (1.40 mL, 12.0 mmol) and the mixture was stirred at room temperature for two hours. Water (50 mL) was added to the reaction liquid and extracted with ethyl acetate (50 mL) three times. The organic layers were combined and washed with water (50 mL) and a saturated saline solution (50 mL), the solvent was evaporated under reduced pressure after drying over sodium sulfate, the obtained residue was purified by silica gel column chromatography (hexane/toluene = 3:1) and the title compound was obtained (2.60 g, 89%).
Colourless liquid
IR (film) νₘₐₓ 2858, 1568, 1475, 1361, 1203, 1103, 695 cm⁻¹;
¹H NMR(CDCl₃, 400 MHz) δ 2.88 (2H, t, J = 7.0 Hz), 3.66 (2H, t, J = 7.0 Hz), 4.50 (2H, s), 7.11-7.15 (2H, m), 7.23-7.38 (7H, m);
MS (EI) m/z: 292, 290 [M⁺], 262, 260, 184, 169, 91.

### (126b) benzyl 4-{3-[2-(benzyloxy)ethyl]phenyl}-1,4-diazepane-1-carboxylate

Reaction with benzyl 1-homopiperazinecarboxylate was performed following a method described in Example 117 (117a) using 1-[2-(benzyloxy)ethyl]-3-bromobenzene which was produced in Example 126 (126a) and the title compound was obtained.
Brown liquid
IR (film) νₘₐₓ 2942, 1699, 1602, 1497, 1421, 1236, 1118, 928, 697 cm⁻¹;
¹H NMR(CDCl₃, 400 MHz) δ 1.88-2.04 (2H, m), 2.81-2.91 (2H, m), 3.29 (1H, t, J = 6.3 Hz), 3.37 (1H, t, J = 6.3 Hz), 3.48-3.72 (8H, m), 4.52 (2H, brs), 5.09 (1H, s), 5.14 (1H, s), 6.50-6.59 (3H, m), 7.13 (1H, m), 7.22-7.40 (10H, m);
MS (FAB) m/z: 444 [M⁺], 386, 354, 266, 246, 165.

### (126c) 2-[3-(1,4-diazepan-1-yl)phenyl]ethanol

Iodotrimethylsilane (4.8 mL, 33.7 mmol) was added to a methylene chloride (26 mL) solution of benzyl 4-{3-[2-(benzyloxy)ethyl]phenyl}-1,4-diazepane-1-carboxylate (2.30 g, 5.20 mmol) which was produced in Example 126 (126b) and the mixture was stirred at room temperature for 23 hours. Water (50 mL) was added to the reaction liquid and partitioned and the obtained aqueous layer was made basic by adding potassium carbonate. The aqueous layer was extracted with methylene chloride/2-propanol (4:1) (50 mL) three times and the solvent was evaporated under reduced pressure after the extract was dried over sodium sulfate and the title compound was obtained (1.05 g, yield 92%).
Brown liquid
IR (film) νₘₐₓ 3310, 2935, 1601, 1497, 1363, 1176, 1047, 771, 696 cm⁻¹;
¹H NMR(CDCl₃, 400 MHz) δ 1.84-1.94 (2H, m), 2.76-2.89 (4H, m), 3.03 (2H, t, J =5.5 Hz), 3.51-3.60 (4H, m), 3.85 (2H, t, J =5.9 Hz), 6.50-6.61 (3H, m), 7.16 (1H, t, J =8.2 Hz);
MS (EI) m/z: 220 [M⁺], 190, 178, 164, 152, 150, 133, 118, 91, 77, 43.

### (126d) 4-{4-[3-(2-hydroxyethyl)phenyl]-1,4-diazepan-1-yl}-2-thioxo-1,2-dihydropyridine-3-carbonitrile

The reaction was performed following a method described in Example 121 (121c) and using 2-[3-(1,4-diazepan-1-yl)phenyl]ethanol which was produced in Example 126 (126c) and the title compound was synthesized.
Brown powder
Mp 147-155°C;
IR (KBr) νₘₐₓ 2946, 2205, 1625, 1524, 1354, 1250, 1177, 1040, 774 cm⁻¹;
¹H NMR(DMSO-d₆, 500 MHz) δ 1.87-2.00 (2H, m), 2.64 (2H, t, J = 7.0 Hz), 3.48-3.61 (4H, m), 3.67-3.78 (4H, m), 3.91-4.00 (2H, m), 4.57 (1H, brs), 6.40-6.51 (2H, m), 6.56-6.66 (2H, m), 7.05 (1H, t, J = 7.8 Hz), 7.37 (1H, d, J = 7.8 Hz), 12.54 (1H, brs);
MS (FAB) m/z: 355 [M+H⁺], 273, 242, 226, 180.

### (126e) 3-amino-4-{4-[3-(2-hydroxyethyl)phenyl]-1,4-diazepan-1-yl}thieno[2,3-b]pyridine-2-carboxamide

The reaction was performed following a method described in Example 5 (5c) using 4-{4-[3-(2-hydroxyethyl)phenyl]-1,4-diazepan-1-yl}-2-thioxo-1,2-dihydropyridine-3-carbonitrile which was produced in Example 126 (126d) and the title compound was synthesized.
Pale yellow powder
Mp 120-123°C;
IR (KBr) νₘₐₓ 3436, 3325, 3185, 2939, 1649, 1598, 1499, 1450, 1370, 1234, 1047, 941, 771 cm⁻¹;
¹H NMR(DMSO-d₆, 500 MHz) δ 2.10-2.18 (2H, m), 2.65 (2H, t, J = 7.3 Hz), 3.16-3.24 (2H, m), 3.25-3.32 (2H, m), 3.50-3.61 (4H, m), 3.73-3.79 (2H, m), 4.57 (1H, brt), 6.47 (1H, d, J = 7.3 Hz), 6.57-6.63 (2H, m), 6.98 (2H, brs), 7.03-7.12 (4H, m), 8.40 (1H, d, J = 5.4 Hz);
MS (FAB) m/z: 412 [M+H⁺], 395, 353, 273, 242, 165;
Anal. Calcd for C₂₁H₂₅N₅O₂S·O₂.67H₂O: C, 59.55; H, 6.27; N, 16.54. Found: C, 59.42; H, 6.21; N, 16.66.

### (Example 127) 3-amino-4-{4-[4-(3-hydroxypropyl)phenyl]-1,4-diazepan-1-yl}thieno[2,3-b]pyridine-2-carboxamide (Exemplified Compound No. 3-1005)

### (127a) 3-(4-bromophenyl)propan-1-ol

Methyl chloroformate (1.9 mL, 24.1 mmol) was added dropwise to a tetrahydrofuran (80 mL) solution of triethylamine (3.4 mL, 24.1 mmol) and 3-(4-bromophenyl) propanoic acid (5.02 g, 21.9 mmol) at 0°C and the mixture was stirred at room temperature for one hour under nitrogen atmosphere. Insolubles were removed by filtration from the reaction liquid and an aqueous solution (40 mL) of sodium borohydride (1.24 g, 32.9 mmol) was added to the filtrate and the mixture was stirred at room temperature for two hours. Ethyl acetate (200 mL) was added to the reaction liquid and the organic layer was washed with a saturated saline solution (200 mL) twice, and the solvent was evaporated under reduced pressure after drying over sodium sulfate and the title compound was obtained (4.36 g, yield 93%).
Colourless liquid
IR (film) νₘₐₓ 3353, 2942, 1711, 1489, 1072, 1012, 833, 796 cm⁻¹;
¹H NMR(CDCl₃, 400 MHz) δ 1.83 -1.90 (2H, m), 2.67 (2H, t, J = 7.8 Hz), 3.66 (2H, t, J = 6.3 Hz), 7.06 (2H, d, J = 8.2 Hz), 7.39 (2H, d, J = 8.2 Hz);
MS (EI) m/z: 214 [M⁺], 196, 169, 117, 104, 91, 90, 77, 51, 50, 39.

### (127b) 1-[3-(benzyloxy)propyl]-4-bromobenzene

N,N-dimethylformamide (20 mL) solution of 3-(4-bromophenyl)propan-1-ol (4.22 g, 19.6 mmol) which was produced in Example 127 (127a) was blended with sodium hydride (55% oily, 1.03 g, 23.5 mmol) at 0°C under nitrogen atmosphere and the mixture was stirred for 10 minutes. The reaction mixture was blended with benzyl bromide (2.3 mL, 23.5 mmol) and the mixture was stirred at room temperature for three hours. The reaction liquid was cooled to 0°C, and the resultant solution was blended with water (10 mL) and ether (50 mL) and partitioned. The organic layer was washed with water (50 mL) twice and the solvent was evaporated under reduced pressure after drying over sodium sulfate. The obtained residue was purified by silica gel column chromatography (hexane/ethyl acetate = 5:1) and the title compound was obtained (4.78 g, yield 80%).
Colourless liquid
IR (film) νₘₐₓ 2941, 2858, 2384, 1488, 1455, 1364, 1102, 1073, 1012, 736, 698 cm⁻¹;
¹H NMR(CDCl₃, 400 MHz) δ 1.87-1.94 (2H, m), 2.67 (2H, t, J = 7.8 Hz), 3.47 (2H, t, J = 6.3 Hz), 4.50 (2H, s), 7.04 (2H, d, J = 8.6 Hz), 7.29-7.39 (7H, m);
MS (EI) m/z: 304 [M⁺], 225, 213, 183, 169, 117, 104, 91, 65.

### (127c) benzyl 4-{4-[3-(benzyloxy)propyl]phenyl}-1,4-diazepane-1-carboxylate

Reaction with benzyl 1-homopiperazinecarboxylate was performed following a method described in Example 117 (117a) using 1-[3-(benzyloxy)propyl]-4-bromobenzene which was produced in Example 127 (127b) and the title compound was obtained.
Pale yellow liquid
IR (film) νₘₐₓ 2940, 1700, 1616, 1519, 1422, 1228, 1118, 928, 737, 698 cm⁻¹;
¹H NMR(CDCl₃, 400 MHz) δ 1.85-2.02 (4H, m), 2.60 (2H, t, J = 7.8 Hz), 3.30 (1H, t, J = 5.9 Hz), 3.37 (1H, t, J = 6.3 Hz), 3.48 (2H, t, J = 6.6 Hz), 3.50.3.57 (4H, m), 3.61-3.66 (2H, m), 4.49 (2H, s), 5.07 (1H, s), 5.12 (1H, s), 6.60 (2H, d, J = 8.6 Hz), 7.01 (2H, d, J = 8.6 Hz), 7.25-7.33 (5H, m);
MS (FAB) m/z: 459 [M+H]⁺, 458, 368, 351, 260, 242, 219, 65.

### (127d) 1-{4-[3-(benzyloxy)propyl]phenyl}-1,4-diazepane

An ethanol (70 mL) solution of benzyl 4-{4-[3-(benzyloxy)propyl]phenyl}-1,4-diazepane-1-carboxylate (3.62 g, 7.9 mmol) which was produced in Example 127 (127c) was blended with 5% palladium-carbon (3.36 g, 1.6 mmol) and the mixture was stirred under hydrogen atmosphere for five hours. The reaction liquid was filtered and the solvent was evaporated under reduced pressure and a crude title compound was obtained (2.42 g, yield 95%).
Colourless liquid
IR (film) νₘₐₓ 3335, 2936, 2855, 1616, 1519, 1364, 1189, 1102, 737, 698 cm⁻¹;
¹H NMR(CDCl₃, 400 MHz) δ 1.86-1.93 (4H, m), 2.61 (2H, t, J = 7.8 Hz), 2.83 (2H, t, J = 4.7 Hz), 3.03 (2H, t, J = 5.5 Hz), 3.48-3.57 (6H, m), 4.51 (2H, s), 6.63 (2H, d, J = 8.2 Hz), 7.03 (2H, d, J = 8.2 Hz), 7.29-7.36 (5H, m);
MS (EI) m/z: 324 [M⁺], 282, 268, 254, 233, 204, 189, 176, 160, 146, 132, 118, 91, 70, 65, 43, 42.

### (127e) 3-[4-(1,4-diazepan-1-yl)-phenyl]propan-1-ol

A methylene chloride (50 mL) solution of 1-{4-[3-(benzyloxy)propyl]phenyl}-1,4-diazepane (2.16 g, 6.7 mmol) which was produced in Example 127 (127d) was blended with iodotrimethylsilane (3.8 mL, 26.6 mmol) under nitrogen atmosphere and the mixture was stirred for 10 minutes. Water (100 mL) was added to the reaction liquid and the aqueous layer was washed with methylene chloride (100 mL) twice. 4M sodium carbonate aqueous solution (50 mL) was added to the aqueous layer and extracted with methylene chloride/isopropanol (4:1) (100 mL) three times. The solvent was evaporated under reduced pressure after the extract was dried over sodium sulfate and the title compound was obtained (1.32 g, yield 73%).
Pale brown liquid
IR (film) νₘₐₓ 3310, 2934, 1616, 1519, 1365, 1189, 1058, 800 cm⁻¹;
¹H NMR(CDCl₃, 500 MHz) δ 1.83-1.90 (4H, m), 2.59 (2H, t, J = 7.8 Hz), 2.82 (2H, t, J = 5.9 Hz), 3.02 (2H, t, J = 5.5 Hz), 3.50-3.56 (4H, m), 3.67 (2H, t, J = 6.7 Hz), 6.62 (2H, d, J = 8.6 Hz), 7.03 (2H, d, J = 8.6 Hz);
MS (EI) m/z: 234 [M⁺], 204, 192, 178, 160, 146, 130, 118, 117, 91, 90, 77, 43, 42.

### (127f) 4-{4-[4-(3-hydroxypropyl)phenyl]-1,4-diazepan-1-yl}-2-thioxo-1,2-dihydropyridine-3-carbonitrile

The reaction was performed following a method described in Example 121 (121c) using 3-[4-(1,4-diazepan-1-yl)-phenyl]propan-1-ol which was produced in Example 127 (127e) and the title compound was synthesized.
Brown powder
Mp 184-186°C;
IR (KBr) νₘₐₓ 2935, 2206, 1626, 1518, 1252, 1039, 930 cm⁻¹;
¹H NMR(DMSO-d₆, 500 MHz) δ 1.64 (2H, quint, J = 7.8 Hz), 1.91-1.96 (2H, m), 2.45 (2H, t, J = 7.8 Hz), 3.36-3.40 (2H, m), 3.50 (2H, t, J = 5.9 Hz), 3.69-3.73 (4H, m), 3.94-3.96 (2H, m), 4.39 (1H, t, J = 4.9 Hz), 6.44 (1H, d, J = 7.8 Hz), 6.69 (2H, d, J = 8.8 Hz), 6.93 (2H, d, J = 8.8 Hz), 7.36 (1H, d, J = 7.8 Hz), 12.53 (1H, brs);
MS (FAB) m/z: 369 [M+H]⁺, 273, 257, 246, 176, 63.

### (127 g) 3-amino-4-{4-[4-(3-hydroxypropyl)phenyl]-1,4-diazepan-1-yl}thieno[2,3-b]pyridine-2-carboxamide

The reaction was performed following a method described in Example 5 (5c) using 4-{4-[4-(3-hydroxypropyl)phenyl]-1,4-diazepan-1-yl}-2-thioxo-1,2-dihydropyridine-3-carbonitrile which was produced in Example 127 (127f) and the title compound was synthesized.
Pale brown powder
Mp 237-241°C;
IR (KBr) νₘₐₓ 3439, 3325, 3174, 2935, 1645, 1577, 1518, 1370, 1057, 938, 900, 826, 805, 479 cm⁻¹;
¹H NMR(DMSO-d₆, 500 MHz) δ 1.66 (2H, quint, J = 7.3 Hz), 2.11-2.15 (2H, m), 2.47-2.50 (2H, m), 3.21 (2H, brs), 3.29 (2H, brs), 3.41 (2H, q, J = 6.8 Hz), 3.52 (2H, t, J = 5.9 Hz), 3.74 (2H, t, J = 4.4 Hz), 4.40 (1H; t, J = 4.9 Hz), 6.70 (2H, d, J = 8.3 Hz), 6.97 (2H, brs), 7.00 (2H, d, J = 8.3 Hz), 7.08 (1H, d, J = 5.4 Hz), 7.09 (2H, brs), 8.40 (1H, d, J = 5.4 Hz);
HRMS m/z calcd for C₂₂H₂₈O₂N₅S 426.1964, found 426.1994;
MS (ESI) m/z: 426 [M+H]⁺, 360;
Anal. Calcd for C₂₂H₂₇N₂O₂S·0.3H₂O: C, 61.32; H, 6.46; N, 16.25; S, 7.44. Found: C, 61.07; H, 6.38; N, 16.49; S, 7.52.

### (Example 128) 3-amino-4-(4-{4-[(dimethylamino)carbonyl]phenyl}-1,4-diazepan-1-yl)thieno[2,3-b]pyridine-2-carboxamide (Exemplified Compound No. 3-107)

### (128a) benzyl 4-[4-(trifluoroacetyl)phenyl]-1,4-diazepane-1-carboxylate

The reaction was performed in a similar method as described in Example 59 (59a) using benzyl 1-homopiperazinecarboxylate and 2,2,2,4'-tetrafluoroacetophenone and the title compound (quantitatively) was obtained. Slightly orange oil
IR (film) νₘₐₓ 2959, 1697, 1595, 1528, 1423, 1166 cm⁻¹;
¹H NMR(CDCl₃, 500 MHz) δ 1.93-2.06 (2H, m), 3.33-3.38 (1H, m), 3.40-3.46 (1H, m), 3.60-3.74 (6H, m), 5.07 (1H, s), 5.13 (1H, s), 6.67-6.74 (2H, m), 7.25-7.37 (5H, m), 7.91-7.98 (2H, m);
MS (FAB) m/z: 407 [M+H]⁺, 406, 315, 289.

### (128b) 4-{4-[(benzyloxy)carbonyl]-1,4-diazepan-1-yl}benzoic acid

benzyl 4-[4-(trifluoroacetyl)phenyl]-1,4-diazepane-1-carboxylate (7.18 g, 17.7 mmol) which was produced in Example 128 (128a) was dissolved in N,N-dimethylformamide (35.4 mL) and was blended with 8N aqueous solution of sodium hydroxide (4.42 mL, 35.3 mmol) and heated and the mixture was stirred at 80°C for one hour. The reaction liquid was cooled, diluted with water (70 mL) and washed with ethyl acetate (70 mL). After the aqueous layer was made acidic with 1N hydrochloric acid and extracted with ethyl acetate (10 mLx3), the organic layers were combined and the solvent was evaporated under reduced pressure after drying over magnesium sulfate. The residual substance was blended with ether (50 mL) to be crystallized, separated by filtration, washed with ether and dried and 5.82 g of the title compound was obtained (yield 69%).
Colourless prism crystal
Mp 135-137°C;
IR (KBr) νₘₐₓ 3063, 2947, 2667, 2562, 1699, 1667, 1600, 1417 cm⁻¹;
¹H NMR(CDCl₃, 400 MHz) δ 1.92-2.07 (2H, m), 3.30-3.36 (1H, m), 3.41 (1H, t, J = 5.9 Hz), 3.58-3.71 (6H, m), 5.08 (1H, s), 5.14 (1H, s), 6.63-6.72 (2H, m), 7.24-7.38 (5H, m), 7.91-7.99 (2H, m);
MS (EI) m/z: 354 M⁺, 263, 202.

### (128c) benzyl 4-{4-[(dimethylamino)carbonyl]phenyl}-1,4-diazepane-carboxylate

Synthesis was performed with reference to J. Org. Chem. (1990), 55, 6252-6259 by the following method. 1,1'-carbonyldiimidazole (3.89 g, 24 mmol) was added to a tetrahydrofuran (80 mL) solution of 4-{4-[(benzyloxy)carbonyl]-1,4-diazepan-1-yl}benzoic acid (7.09 g, 20 mmol) which was produced in Example 128 (128b) under ice-cooling and the mixture was stirred for 30 minutes. The reaction liquid was warmed to room temperature and was blended with a tetrahydrofuran solution (2.0M, 15 mL, 30 mmol) of dimethylamine and the mixture was stirred for 30 minutes. A saturated sodium hydrogen carbonate aqueous solution (150 mL) was added to the reaction liquid and extracted with ethyl acetate (100 mLx3). The organic layers were combined, washed with a saturated saline solution (50 mL) and dried over anhydrous sodium sulfate and the solvent was evaporated under reduced pressure. The residual substance was purified by silica gel column chromatography (eluent: ethyl acetate) and 7.63 g of the title compound was obtained (quantitative). Pale yellow oil
IR (film) νₘₐₓ 3475, 2939, 1699, 1608, 1493, 1423, 1391 cm⁻¹;
¹H NMR(CDCl₃, 400 MHz) δ 1.90-2.06 (2H, m), 3.07 (6H, s), 3.26-3.33 (1H, m), 3.34-3.41 (1H, m), 3.52-3.70 (6H, m), 5.10 (1H, s), 5.14 (1H, s), 6.59-6.71 (2H, m), 7.22-7.42 (7H, m);
MS (FAB) m/z: 382 {M+H}⁺, 337, 246.
(128d) 4-(1,4-diazepan-1-yl)-N,N-dimethylbenzamide

10% palladium-carbon (water content: 53 wt%, 1.10 g) was added to an ethanol (11 ml) solution of benzyl 4-{4-[(dimethylamino)carbonyl]phenyl}-1,4-diazepane-carboxylate (1.10 g, 2.88 mmol) which was produced in Example 128 (128c) and the mixture was stirred under hydrogen atmosphere for one hour. The reaction liquid was filtered and after the catalyst was washed with ethanol, 671 mg of the title compound was obtained (yield 94%) by evaporating the solvent from the obtained filtrate under reduced pressure.
Colourless oil
IR (film) νₘₐₓ 3444, 3321, 2931, 1672, 1608, 1493, 1390 cm⁻¹;
¹H NMR(CDCl₃, 400 MHz) δ 1.85-1.94 (2H, m), 2.79-2.85 (2H, m), 3.00-3.05 (2H, m), 3.07 (6H, m), 3.54-3.64 (4H, m), 6.66 (2H, d, J = 8.6 Hz), 7.36 (2H, d, J = 8.6 Hz);
MS (EI) m/z: 247 M⁺., 203, 191, 160.

### (128e) 4-[4-(3-cyano-2-thioxo-1,2-dihydropyridin-4-yl)-1,4-diazepan-1-yl]-N,N-dimethyl benzamide

The reaction was performed following a method described in Example 121 (121c) using 4-(1,4-diazepan-1-yl)-N,N-dimethylbenzamide which was produced in Example 128 (128d) and the title compound was synthesized. Yield 24%.
Slightly brown powder
Mp 239-241 °C;
IR (KBr) νₘₐₓ 3180, 3147, 3045, 2960, 2918, 2208, 1605, 1525, cm⁻¹;
¹H NMR(DMSO-d₆, 500 MHz) δ 1.92-2.00 (2H, m), 2.94 (6H, s), 3.54-3.61 (2H, m), 3.71-3.81 (4H, m), 3.95-4.01 (2H, m), 6.44 (1H, d, J = 7.8 Hz), 6.79 (2H, d, J = 8.8 Hz), 7.26 (2H, d, J = 8.8 Hz), 7.36 (2H, d, J = 7.8 Hz), 12.50 (1H, br.s);
MS (FAB) m/z: 382 [M+H]⁺, 337, 273;
Anal. Calcd for C₂₀H₂₃N₅OS·0.6H₂O: C, 61.23; H, 6.22; N, 17.85; S, 8.17. Found: C, 61.32; H, 6.18; N, 17.95; S, 8.12.

### (128f) 3-amino-4-(4-{4-[(dimethylamino)carbonyl]phenyl}-1,4-diazepan-1-yl)thieno[2,3-b]pyridine-2-carboxamide

The reaction was performed following a method described in Example 5 (5c) using 4-[4-(3-cyano-2-thioxo-1,2-dihydropyridin-4-yl)-1,4-diazepan-1-yl]-N,N-dimethyl benzamide which was produced in Example 128 (128e) and the title compound was synthesized. Yield 83%.
Slightly brown powder
Mp 159-161°C;
IR (KBr) νₘₐₓ 3437, 3327, 3187, 2930, 2847, 1606, 1497, 1387 cm⁻¹;
¹H NMR(DMSO-d₆, 400 MHz) δ 2.11-2.20 (2H, m), 2.97 (6H, s), 3.16-3.34 (2H, m), 3.58 (2H, t, J = 6.3 Hz), 3.78-3.84 (2H, m), 6.76 (2H, d, J = 9.0 Hz), 6.97 (2H, br.s), 7.04-7.12 (3H, m), 7.28 (2H, d, J = 9.0 Hz), 8.38 (1H, d, J = 5.1 Hz);
MS (FAB) m/z: 439 [M+H]⁺, 394, 273;
Anal. Calcd for C₂₂H₂₆N₆O₂S·1.2H₂O: C, 57.42; H, 6.22; N, 18.26; S, 6.97. Found: C, 57.17; H, 6.03; N, 18.45; S, 6.83.

### (Example 129) 3-amino-4-{4-[4-(azetidin-1-ylcarbonyl)phenyl]-1,4-diazepan-1-yl}thieno[2,3-b]pyridine-2-carboxamide (Exemplified Compound No. 3-940)

### (129a) benzyl 4-[4-(azetidin-1-ylcarbonyl)phenyl]-1,4-diazepane-1-carboxylate

The reaction was performed following a method described in Example 128 (128c) using 4-{4-[(benzyloxy)carbonyl]-1,4-diazepan-1-yl}benzoic acid which was produced in Example 128 (128b) and azetidine and the title compound was (quantitatively) synthesized.
Slightly yellow oil
IR (film) νₘₐₓ 3459, 2952, 2886, 1698, 1605, 1428, 1234, 1175 cm⁻¹;
¹H NMR(CDCl₃, 400 MHz) δ 1.90-2.06 (2H, m), 2.32 (2H, quint, J = 7.8 Hz), 3.31 (2H, t, J = 6.3 Hz), 3.35-3.41 (2H, m), 3.52-3.69 (6H, m), 4.13-4.42 (4H, m), 5.08 (1H, s), 5.13 (1H, s), 6.60-6.69 (2H, m), 7.24-7.38 (5H, m), 7.52-7.62 (2H, m);
MS (FAB) m/z: 394 [M+H]⁺, 337, 258, 246.

### (129b) 1-[4-(azetidin-1-ylcarbonyl)phenyl]-1,4-diazepane

The reaction was performed following a method described in Example 128 (128d) using benzyl 4-[4-(azetidin-1-ylcarbonyl)phenyl]-1,4-diazepane-1-carboxylate which was produced in Example 129 (129a) and azetidine and the title compound was synthesized (yield 98%).
Colourless oil
IR (film) νₘₐₓ 3416, 3321, 2941, 1606, 1433, 1406, 1177 cm⁻¹;
¹H NMR(CDCl₃, 400 MHz) δ 1.89 (2H, quint, J = 5.9 Hz), 2.26-2.37 (2H, m), 2.78-2.86 (2H, m), 2.99-3.07 (2H, m), 3.53-3.66 (4H, m), 4.11-4.45 (4H, m), 6.65 (2H, d, J = 9.0 Hz), 7.56 (2H, d, J = 9.0 Hz);
MS (EI) m/z: 259 M⁺, 217, 203, 189.

### (129c) 4-{4-[4-(azetidin-1-ylcarbonyl)phenyl}-1,4-diazepan-1-yl-2-thioxo-1,2-dihydropyridine-3-carbonitrile

The reaction was performed following a method described in Example 121 (121c) using 1-[4-(azetidin-1-ylcarbonyl)phenyl]-1,4-diazepane which was produced in Example 129 (129b) and azetidine and the title compound was synthesized.
Yield 25%.
White powder
Mp 136-138°C;
IR (KBr) νₘₐₓ 3441, 2950, 2882, 2204, 1605, 1522, 1432, 1402 cm⁻¹;
¹H NMR(DMSO-d₆, 500 MHz) δ 1.90-1.99 (2H, m), 2.23 (2H, quint, J = 7.8 Hz), 3.59 (2H, t, J = 5.9 Hz), 3.72-3.83 (4H, m), 3.88-4.41 (6H, m), 6.44 (1H, d, J = 7.8 Hz), 6.79 (2H, d, J = 8.8 Hz), 7.36 (1H, d, J = 7.8 Hz), 7.48 (2H, d, J = 8.8 Hz), 12.53 (1H, br.s);
MS (FAB) m/z: 394 [M+H]⁺, 378, 337, 273;
Anal. Calcd for C₂₁H₂₃N₅OS·0.84H₂O: C, 61.72; H, 6.09; N, 17.14; S, 7.85. Found: C, 61.88; H, 5.82; N, 17.10; S, 7.74.

### (129d) 3-amino-4-{4-[4-(azetidin-1-ylcarbonyl)phenyl]-1,4-diazepan-1-yl}thieno[2,3-b]pyridine-2-carboxamide

The reaction was performed following a method described in Example 5 (5c) using 4-{4-[4-(azetidin-1-ylcarbonyl)phenyl}-1,4-diazepan-1-yl}-2-thioxo-1,2-dihydropyridine-3-carbonitrile which was produced in Example 129 (129c) and azetidine and the title compound was synthesized. Yield 77%.
Slightly brown powder
Mp 141-143°C;
IR (KBr) νₘₐₓ 3438, 3325, 3189, 2952, 2882, 1648, 1604, 1432, 1399, cm⁻¹;
¹H NMR(DMSO-d₆, 400 MHz) δ 2.10-2.30 (4H, m), 3.14-3.25 (2H, m), 3.26-3.38 (2H, m), 3.55-3.65 (2H, m), 3.79-3.88 (2H, m), 3.92-4.42 (4H, m), 6.79 (2H, d, J = 8.6 Hz), 6.98 (2H, br.s), 7.08 (1H, d, J = 5.5 Hz), 7.11 (2H, br.s), 7.50 (2H, d, J = 8.6 Hz), 8.40 (1H, d, J = 5.5 Hz);
MS (FAB) m/z: 451 [M+H]⁺, 434, 394, 273;
Anal. Calcd for C₂₃H₂₆N₆OS·1.2H₂O: C, 58.81; H, 6.06; N, 17.80; S, 6.79. Found: C, 58.61; H, 6.00; N, 17.50; S, 6.64.

### (Example 130) 3-amino-4-{4-[4-(morpholin-4-ylcarbonyl)phenyl]-1,4-diazepan-1-yl}thieno[2,3-b]pyridine-2-carboxamide (Exemplified Compound No. 3-970)

### (130a) benzyl 4-[4-(morpholin-4-ylcarbonyl)phenyl]-1,4-diazepane-1-carboxylate

The reaction was performed following a method described in Example 128 (128c) using 4-{4-[(benzyloxy)carbonyl]-1,4-diazepan-1-yl}benzoic acid which was produced in Example 128 (128b) and morpholine and the title compound was synthesized (quantitative).
Slightly brown oil
IR (film) νₘₐₓ 3485, 2957, 2928, 2857, 1698, 1607, 1424 cm⁻¹;
¹H NMR(CDCl₃, 400 MHz) δ 1.91-2.06 (2H, m), 3.31 (1H, t, J = 6.3 Hz), 3.38 (1H, t, J = 6.3 Hz), 3.54-3.74 (14H, m), 5.09 (1H, s), 5.13 (1H, s), 6.63-6.71 (2H, m), 7.28-7.39 (7H, m);
MS (FAB) m/z: 424 [M+H]⁺, 337, 273.

### (130b) 1-[4-(morpholin-4-ylcarbonyl)phenyl]-1,4-diazepane

The reaction was performed following a method described in Example 128 (128d) using benzyl 4-[4-(morpholin-4-ylcarbonyl)phenyl]-1,4-diazepane-1-carboxylate which was produced in Example 130 (130a) and the title compound was synthesized (quantitative).
Colourless oil
IR (film) νₘₐₓ 3417, 3314, 2929, 2855, 1607, 1524, 1457, 1431 cm⁻¹;
¹H NMR(CDCl₃, 400 MHz) δ 1.89 (2H, q, J = 5.9 Hz), 2.78-2.86 (2H, m), 2.99-3.07 (2H, m), 3.53-3.77 (12H, m), 6.66 (2H, d, J = 9.0 Hz), 7.34 (2H, d, J = 9.0 Hz);
MS (EI) m/z: 289 M⁺, 247, 233, 203.

### (130c) 4-{4-[4-(morpholin-4-ylcarbonyl)phenyl]-l,4-diazepan-1-yl}-2-thioxo-1,2-dihydropyridine-3-carbonitrile

The reaction was performed following a method described in Example 121 (121c) using 1-[4-(morpholin-4-ylcarbonyl)phenyl]-1,4-diazepane which was produced in Example 130 (130b) and the title compound was synthesized. Yield 46%.
Brown foam
IR (KBr) νₘₐₓ 3434, 3198, 3132, 2958, 2922, 2854, 2204, 1606, 1520 cm⁻¹;
¹H NMR(CDCl₃, 400 MHz) δ 2.07-2.16 (2H, m), 3.54-3.79 (12H, m), 3.82-3.89 (2H, m), 4.11-4.18 (2H, m), 6.20 (1H, d, J = 7.4 Hz), 6.70 (2H, d, J = 8.6 Hz), 7.23 (1H, d, J = 7.4 Hz), 7.36 (2H, d, J = 8.6 Hz), 11.57 (1H, br.s);
MS (FAB) m/z: 424 [M+H]⁺, 337, 273.

### (130d) 3-amino-4-{4-[4-(morpholin-4-ylcarbonyl)phenyl]-1,4-diazepan-1-yl}thieno[2,3-b]pyridine-2-carboxamide

The reaction was performed following a method described in Example 5 (5c) using 4-{4-[4-(morpholin-4-ylcarbonyl)phenyl]-1,4-diazepan-1-yl}-2-thioxo-1,2-dihydropyridine-3-carbonitrile which was produced in Example 130 (130c) and the title compound was synthesized. Yield 43%.
Slightly brown powder
Mp 274-275°C;
IR (KBr) νₘₐₓ 3437, 3323, 3189, 2957, 2921, 2852, 1606, 1367 cm⁻¹;
¹H NMR(DMSO-d₆, 500 MHz) δ 2.12-2.20 (2H, m), 3.18-3.25 (2H, m), 3.27-3.35 (2H, m), 3.48-3.55 (4H, m), 3.57-3.64 (6H, m), 3.80-3.86 (2H, m), 6.80 (2H, d, J = 8.8 Hz), 6.96 (2H, br.s), 7.06-7.13 (3H, s), 7.29 (2H, d, J = 8.8 Hz), 8.41 (1H, d, J = 5.4 Hz);
MS (FAB) m/z: 481 [M+H]⁺, 273, 258, 242;
Anal. Calcd for C₂₄H₂₈N₆O₃S: C, 59.98; H, 5.87; N, 17.49; S, 6.67. Found: C, 59.89; H, 5.89; N, 17.28; S, 6.70.

### (Example 131) 3-amino-4-(4-{4-[2-(dimethylamino)-2-oxoethyl]phenyl}-1,4-diazepan-1-yl)thieno[2,3-b]pyridine-2-carboxamide (Exemplified Compound No. 3-925)

### (131a) benzyl 4-{4-[2-(dimethylamino)-2-oxoethyl]phenyl}-1,4-diazepane-1-carboxylate

The reaction was performed following a method described in Example 117 (117a) using benzyl 1-homopiperazinecarboxylate in place of tert-butyl 1-piperazinecarboxylate and the title compound was obtained. Yield 80%.
Yellow oil
IR (film) νₘₐₓ 3481, 2939, 1699, 1642, 1520, 1423 cm⁻¹;
¹H NMR(CDCl₃, 400 MHz) δ 1.90-2.06 (2H, m), 2.95 (3H, s), 3.00 (3H, s), 3.27-3.33 (1H, m), 3.34-3.40 (1H, m), 3.50-3.67 (8H, m), 5.08 (1H, s), 5.13 (1H, s), 6.60-6.68 (2H, m), 7.10 (2H, d, J = 8.2 Hz), 7.27-7.40 (5H, m);
MS (FAB) m/z: 396 [M+H]⁺, 323, 260.

### (131b) 2-[4-(1,4-diazepan-1-yl)phenyl]-N,N-dimethylacetamide

The reaction was performed following a method described in Example 128 (128d) using benzyl 4-{4-[2-(dimethylamino)-2-oxoethyl]phenyl}-1,4-diazepane-1-carboxylate which was produced in Example 131 (131a) and the title compound was obtained. Yield 94%.
Pale yellow oil
IR (film) νₘₐₓ 3430, 2934, 1629, 1520, 13.98 cm⁻¹;
¹H NMR(CDCl₃, 400 MHz) δ 1.88 (2H, quint, J = 5.9 Hz), 2.79-2.84 (2H, m), 2.94 (3H, s), 2.97-3.04 (5H, m), 3.47 (2H, s), 3.49-3.57 (4H, m), 6.62 (2H, d, J = 9.0 Hz), 7.07 (2H, d, J = 9.0 Hz);
MS (FAB) m/z: 262 [M+H]⁺, 261, 219, 189.

### (131c) 2-{4-[4-(3-cyano-2-thioxo-1,2-dihydropyridin-4-yl)-1,4-diazepan-1-yl]phenyl}-N,N-dimethylacetamide

The reaction was performed following a method described in Example 121 (121c) using 2-[4-(1,4-diazepan-1-yl)phenyl]-N,N-dimethylacetamide which was produced in Example 131 (131b) and the title compound was synthesized (yield 47%).
Brown amorphous
IR (KBr) νₘₐₓ 3124, 3034, 2938, 2203, 1616, 1519 cm⁻¹;
¹H NMR(CDCl₃, 400 MHz) δ 2.04-2.16 (2H, m), 2.96 (3H, s), 3.02 (3H, s), 3.52-3.63 (4H, m), 3.69-3.82 (4H, m), 4.08-4.16 (2H, m), 6.19 (1H, d, J = 7.8 Hz), 6.66 (2H, d, J = 8.6 Hz), 7.12 (2H, d, J = 8.6 Hz), 7.22 (1H, d, J = 7.8 Hz), 11.94 (1H, br.s);
MS (FAB) m/z: 396 [M+H]⁺, 350, 323, 262.

### (131d) 3-amino-4-(4-{4-[2-(dimethylamino)-2-oxoethyl]phenyl}-1,4-diazepan-1-yl)thieno[2,3-b]pyridine-2-carboxamide

The reaction was performed following a method described in Example 5 (5c) using 2-{4-[4-(3-cyano-2-thioxo-1,2-dihydropyridin-4-yl)-1,4-diazepan-1-yl]phenyl} -N,N-dimethylacetamide which was produced in Example 131 (131c) and the title compound was synthesized (yield 56%).
Slightly yellow powder
Mp 133-135°C;
IR (KBr) νₘₐₓ 3435, 3326, 3189, 2935, 2839, 1634, 1579, 1519 cm⁻¹;
¹H NMR(DMSO-d₆, 500 MHz) δ 2.10-2.18 (2H, m), 2.82 (3H, s), 2.98 (3H, s), 3.15-3.23 (2H, m), 3.25-3.33 (2H, m), 3.49-3.56 (4H, m), 3.72-3.78 (2H, m), 6.71 (2H, d, J = 8.8 Hz), 7.00 (2H, br.s), 7.03 (2H, d, J = 8.8 Hz), 7.07 (1H, d, J = 5.4 Hz), 7.09 (2H, br.s), 8.39 (1H, d, J = 5.4 Hz);
MS (FAB) m/z: 453 [M+H]⁺, 452, 436, 380;
Anal. Calcd for C₂₃H₂₈N₆O₂S·1.16H₂O: C, 58.35; H, 6.45; N, 17.75. Found: C, 58.10; H, 6.19; N, 18.03.

### (Example 132) 3-amino-4-(4-{4-[3-(dimethylamino)-3-oxopropyl]phenyl}-1,4-diazepan-1-yl)thieno[2,3-b]pyridine-2-carboxamide (Exemplified Compound No. 3-927)

### (132a) 3-(4-bromophenyl)-N,N-dimethylpropanamide

According to a method described in Example 128 (128c), the title compound was obtained (yield 99%) using 3-(4-bromophenyl)propionic acid.
Colourless oil
IR (neat) νₘₐₓ 1648, 1488, 1400, 1012, 816 cm⁻¹;
¹H NMR (CDCl₃, 400MHz) δ 2.58 (2H, t, J = 7.8 Hz), 2.92 (2H, t, J = 7.8 Hz), 2.93 (6H, s), 7.08 (2H, d, J = 8.5 Hz), 7.38 (2H, d, J = 8.5);
MS (EI) m/z: 255 [M⁺], 169;
Anal. Calcd for C₁₁H₁₄NOBr·0.1 H₂O: C, 51.22; H, 5.55; N, 5.43; Br, 30.98. Found: C, 51.03; H, 5.67; N, 5.48; Br, 31.18.

### (132b) benzyl 4-{4-[3-(dimethylamino)-3-oxopropyl]phenyl}-1,4-diazepane-1-carboxylate

The reaction was performed following a method described in Example 117 (117a) using 3-(4-bromophenyl)-N,N-dimethylpropanamide which was produced in Example 132 (132a) and benzyl 1-homopiperazinecarboxylate and the title compound was obtained (yield 77%).
Slightly yellow oil
IR (film) νₘₐₓ 3550, 3488, 2939, 1699, 1645, 1519 cm⁻¹;
¹H NMR(CDCl₃, 400 MHz) δ 1.90-2.04 (2H, m), 2.52-2.59 (2H, m), 2.81-2.88 (2H, m), 2.92 (3H, s), 2.93 (3H, s), 3.30 (1H, t, J = 6.3 Hz), 3.34-3.39 (1H, m), 3.49-3.59 (4H, m), 3.60-3.67 (2H, m), 5.07 (1H, s), 5.12 (1H, s), 6.58-6.64 (2H, m), 7.05 (2H, d, J = 8.2 Hz), 7.25-7.35 (5H, m);
MS (FAB) m/z: 410 [M+H]⁺, 409, 323, 274.

### (132c) 3-[4-(1,4-diazepan-1-yl)phenyl]-N,N-dimethylpropanamide

The title compound was obtained (quantitatively) by performing the reaction following a method described in Example 128 (128d) using benzyl 4-{4-[3-(dimethylamino)-3-oxopropyl]phenyl}-1,4-diazepane-1-carboxylate which was produced in Example 132 (132b).
Slightly yellow oil
IR (film) νₘₐₓ 3445, 3319, 2931, 1635, 1520 cm⁻¹;
¹H NMR(CDCl₃, 400 MHz) δ 1.88 (2H, quint, J = 5.9 Hz), 2.53-2.60 (2H, m), 2.78-2.88 (4H, m), 2.93 (3H, s), 2.94 (3H, s), 2.98-3.03 (2H, m), 3.49-3.57 (4H, m), 6.61 (2H, d, J = 8.6 Hz), 7.04 (2H, d, J = 8.6 Hz);
MS (EI) m/z: 275 M⁺, 233, 219.

### (132d) 3-{4-[4-(3-cyano-2-thioxo-1,2-dihydropyridin-4-yl)-1,4-diazepan-1-yl]phenyl}-N, N-dimethylpropanamide

The reaction was performed following a method described in Example 121 (121c) using 3-[4-(1,4-diazepan-1-yl)phenyl]-N,N-dimethylpropanamide which was produced in Example 132 (132c) and the title compound was synthesized. Yield 49%.
Brown foam
IR (KBr) νₘₐₓ 3124, 2932, 2204, 1732, 1615, 1518 cm⁻¹;
¹H NMR(DMSO-d₆, 400 MHz) δ 2.06-2.14 (2H, m), 2.53-2.60 (2H, m), 2.81-2.88 (2H, m), 2.94 (6H, s), 3.51-3.57 (2H, m), 3.70-3.80 (4H, m), 4.08-4.13 (2H, m), 6.21 (1H, d, J = 7.8 Hz), 6.63 (2H, d, J = 8.6 Hz), 7.07 (2H, d, J = 8.6 Hz), 7.26 (1H, d, J = 7.8 Hz), 12.27 (1H, br.s);
MS (FAB) m/z: 410 [M+H]⁺, 219, 176.

### (132e) 3-amino-4-(4-{4-[3-(dimethylamino)-3-oxopropyl]phenyl}-1,4-diazepan-1-yl)thieno[2,3-b]pyridine-2-carboxamide

The reaction was performed following a method described in Example 5 (5c) using 3-{4-[4-(3-cyano-2-thioxo-1,2-dihydropyridin-4-yl)-1,4-diazepan-1-yl]phenyl}-N, N-dimethylpropanamide which was produced in Example 132 (132d) and the title compound was synthesized. Yield 62%.
Slightly brown powder
Mp 241-243°C;
IR (KBr) νₘₐₓ 3445, 3328, 3173, 2941, 2840, 1637, 1578, 1518, 1368 cm⁻¹;
¹H NMR(DMSO-d₆, 400 MHz) δ 2.06-2.16 (2H, m), 2.49-2.56 (2H, m), 2.63-2.70 (2H, m), 2.80 (3H, s), 2.93 (3H, s), 3.14-3.33 (4H, m), 3.48-3.54 (2H, m), 3.70-3.77 (2H, m), 6.67 (2H, d, J = 8.6 Hz), 6.93 (2H, br.s), 7.02 (2H, d, J = 8.6 Hz), 7.05 (1H, d, J = 5.5 Hz), 7.06 (2H, br.s), 8.37 (1H, d, J = 5.5 Hz);
MS (FAB) m/z: 467 [M+H]⁺, 466, 394, 380;
Anal. Calcd for C₂₄H₃₀N₆O₂S·0.3H₂O: C, 61.07; H, 6.53; N, 17.80; S, 6.79. Found: C, 61.02; H, 6.42; N, 18.03; S, 6.74.

### (Example 133) 3-amino-4-(4-{4-[2-(dimethylamino)ethyl]phenyl}-1,4-diazepan-1-yl)thieno[2,3-b]pyridine-2-carboxamide (Exemplified Compound No. 3-979)

### (133a) N- {2-[4-(1,4-diazepan-1-yl)phenyl] ethyl}-N,N-dimethylamine

A tetrahydrofuran (20 mL) solution of 2-[4-(1,4-diazepan-1-yl)phenyl]-N,N-dimethylacetamide (1.32 g, 5.06 mmol) which was produced in Example 131 (131b) was slowly added dropwise to a tetrahydrofuran (5 mL) suspension of lithium aluminum hydride (576 mg, 15.2 mmol) under ice-cooling. After the dropwise addition was completed, the reaction liquid was stirred at room temperature for one hour and tetrahydrofuran (25 mL) was added. The reaction liquid was ice-cooled and carefully blended with 1N aqueous solution of sodium hydroxide (2,880 µL) and the mixture was stirred at room temperature for 30 minutes. Subsequently anhydrous sodium sulfate was added, and after the mixture was stirred at room temperature for a further 30 minutes, insolubles were removed by Celite filtration. The insolubles were washed with tetrahydrofuran and the solvent was concentrated under reduced pressure from the collected filtrate. Toluene was added to the residual substance and the solvent was azeotropically distilled under reduced pressure and 1.16 g of the title compound was obtained (yield 92%).
Yellow oil
IR (film) νₘₐₓ 3315, 2938, 2858, 2818, 2778, 1616, 1520, 1461 cm⁻¹;
¹H NMR(CDCl₃, 400 MHz) δ 1.88 (2H, quint, J = 5.9 Hz), 2.28 (6H, s), 2.44-2.50 (2H, m), 2.63-2.69 (2H, m), 2.79-2.84 (2H, m), 2.98-3.03 (2H, m), 3.48-3.57 (4H, m), 6.62 (2H, d, J = 8.6 Hz), 7.02 (2H, d, J = 8.6 Hz);
MS (EI) m/z: 247 M⁺, 202, 189.

### (133b) 3-amino-4-(4-{4-[2-(dimethylamino)ethyl]phenyl}-1,4-diazepan-1-yl)thieno[2,3-b]pyridine-2-carboxamide

(2Z)-2-cyano-3-ethoxybut-2-enethioamide (J. Org. Chem. (1962), 27,2433-2439) (797 mg, 4.68 mmol) was added to N,N-dimethylformamide (9.36 mL) solution of N-{2-[4-(1,4-diazepan-1-yl)phenyl]ethyl}-N,N-dimethylamine (1.16 g, 4.68 mmol) which was produced in Example 133 (133a) and the resultant mixture was stirred at room temperature for 15 minutes. Subsequently N,N-dimethylformamide dimethylacetal (684 µL, 5.15 mmol) was added, the mixture was stirred for one hour and then heated and the mixture was stirred at 80°C for 30 minutes. After the reaction liquid was allowed to be cooled to room temperature, the resultant solution was blended with 2-chloroacetamide (526 mg, 5.62 mmol) and 8N-aqueous solution of sodium hydroxide (1.75 mL, 14.0 mmol) and the mixture was stirred at room temperature for two hours. Furthermore, water (9.36 mL) and ethanol (4.68 mL) were added and the mixture was stirred for 30 minutes and the deposited crystal was separated by filtration, washed with 50% ethanol aqueous solution and then dried and 297 mg of the title compound was obtained (yield 14%). Yellow powder
Mp 200-203°C;
IR (KBr) νₘₐₓ 3442, 3328, 3164, 2939, 2824, 1650, 1578, 1518 cm⁻¹;
¹H NMR(DMSO-d₆, 400 MHz) δ 2.05-2.23 (2H, m), 2.17 (6H, s), 2.34-2.41 (2H, m), 2.52-2.61 (2H, m), 3.13-3.35 (4H, m), 3.47-3.55 (2H, m), 3.70-3.77 (2H, m), 6.69 (2H, d, J = 8.6 Hz), 6.98 (2H, br.s), 7.02 (2H, d, J = 8.6 Hz), 7.07 (1H, d, J = 5.4 Hz), 7.09 (2H, br.s), 8.40 (2H, d, J = 5.4 Hz);
MS (FAB) m/z: 439 [M+H]⁺, 273, 258, 242;
Anal. Calcd for C₂₃H₃₀N₆OS: C, 62.99; H, 6.89; N, 19.16; S, 7.31. Found: C, 62.59; H, 6.91; N, 19.04;S, 7.07.

### (Example 134) 4-[4-(5-acetylpyridin-2-yl)-1,4-diazepan-1-yl]-3-aminothieno[2,3-b]pyridine-2-carboxamide (Exemplified Compound No. 3-142)

### (134a) tert-butyl 4-(5-acetylpyridin-2-yl)-1,4-diazepane-1-carboxylate

An N-butanol (18 mL) suspension of 5-acetyl-2-bromopyridine (Chem. Ber. (1992), 1169-1190) (1.12 g, 5.6 mmol), tert-butyl 1-homopiperazinecarboxylate (1.23 g, 6.2) and sodium carbonate (0.77 g, 7.3) was stirred under heat reflux for 11 hours.
Insolubles were removed by filtration, and the residue which was obtained by evaporating the solvent was partitioned with ethyl acetate (100 mL) and water (50 mL). The solvent was evaporated under reduced pressure after the organic layer was dried over sodium sulfate. Residue was purified by silica gel column chromatography (hexane/ethyl acetate =1:1) and 1.57 g of the title compound was obtained (yield 88%).
Mp 120-122°C;
IR (KBr) νₘₐₓ 1677, 1666, 1603, 1167 cm⁻¹;
¹H NMR (CDCl₃, 400MHz) δ 1.39 (4.5H, s), 1.43 (4.5H, s), 1.93-1.99 (2H, m), 2.49 (3H, s), 3.23-3.37 (2H, m), 3.55-3.59 (2H, m), 3.66-3.74 (2H, m), 3.82-3.87 (2H, m), 6.51 (1H, d, J = 9.0 Hz), 8.00 (1H, dd, J = 2.4, 9.0 Hz), 8.73 (1H, d, J = 2.4 Hz);
MS (EI) m/z: 319 [M⁺], 163, 57;
Anal. Calcd for C₁₇H₂₅N₃O₃: C, 63.93; H, 7.89; N, 13.16. Found: C, 63.74; H, 7.91; N, 13.08.

### (134b) 1-[6-(1,4-diazepan-1-yl)pyridin-3-yl]ethanone

4N hydrochloric acid-1,4-dioxane solution (10 mL) was added to 1,4-dioxane (25 mL) solution of tert-butyl 4-(5-acetylpyridin-2-yl)-1,4-diazepane-1-carboxylate (1.55 g, 4.9) which was produced in Example 134 (134a) and the mixture was stirred at room temperature for three hours. 1N aqueous solution of sodium hydroxide (20 mL) was added to the residue which was obtained by concentrating the reaction mixture under reduced pressure and the aqueous layer was extracted with methylene chloride (2x50 mL). 1.05 g (99%) of the title compound was obtained by evaporating the solvent under reduced pressure after the extract was dried over sodium sulfate.
IR (neat) νₘₐₓ 3324, 1663, 1597, 1285, 957, 813 cm⁻¹;
¹H NMR (CDCl₃, 400MHz) δ 1.63 (1H, br), 1.87-1.93 (2H, m), 2.50 (3H, s), 2.84-2.87 (2H, m), 3.03-3.06 (2H, m), 3.75-3.84 (4H, m), 6.52 (1H, d, J = 9.0 Hz), 8.01 (1H, dd, J = 2.0, 9.0 Hz), 8.76 (1H, d, J = 2.0 Hz);
MS (EI) m/z: 219 [M⁺], 163, 149;
Anal. Calcd for C₁₂H₁₇N₃O·0.36 H₂O: C, 63.84; H, 7.91; N, 18.61. Found: C, 64.04; H, 7.93; N, 18.25.

### (134c) (2Z)-3-[4-(5-acetylpyridin-2-yl)-1,4-diazepan-1-yl]-2-cyanobut-2-enethioamide

The reaction was performed following a method described in Example 5 (5a) using 1-[6-(1,4-diazepan-1-yl)pyridin-3-yl]ethanone which was produced in Example 134 (134b) in place of isobutylamine and the title compound was synthesized.
Yield 87%.
Mp 184-187°C (dec.);
IR (KBr) vₘₐₓ 3298, 3178, 2180, 1661, 1655, 1597, 1273 cm⁻¹;
¹H NMR (DMSO-d₆, 400MHz) δ 1.89-1.95 (2H, m), 2.24 (3H, s), 2.45 (3H, s), 3.59-4.03 (8H, m), 6.83 (1H, d, J = 9.0 Hz), 7.97 (1H, dd, J = 2.0, 9.0 Hz), 8.41 (1H, br), 8.73 (1H, d, J = 2.0 Hz), 9.06 (1H, br);
MS (FAB) m/z: 344 [M + H]⁺;
Anal. Calcd for C₁₇H₂₁N₅SO·0.16 H₂O: C, 58.96; H, 6.20; N, 20.22; S, 9.26. Found: C, 59.28; H, 6.29; N, 19.89; S, 8.94.

### (134d) 4-[4-(5-acetylpyridin-2-yl)-1,4-diazepan-1-yl]-2-thioxo-1,2-dihydropyridine-3-carbonitrile

[(2Z)-3-[4-(5-acetylpyridin-2-yl)-1,4-diazepan-1-yl]-2-cyanobut-2-enethioamide (1.00 g, 3.4 mmol) which was produced in Example 134 (134c) and N,N-dimethylformamide dimethylacetal (0.49 g, 0.54 mL, 4.1 mmol) were dissolved in N,N-dimethylformamide (10 mL) and the resultant solution was stirred at room temperature for one hour. Furthermore, after reaction was conducted at 100°C for two hours, the reaction mixture was concentrated under reduced pressure. 1N aqueous solution of sodium hydroxide (10 mL) and ethyl acetate (50 mL) were added to the residue and partitioned. The aqueous layer was neutralized by adding 1N hydrochloric acid (10 mL) and extracted with ethyl acetate (3x50 mL). The extract was dried over sodium sulfate and the solvent was evaporated under reduced pressure. Ethanol was added to the obtained residue and a crude product (0.23 g) of the title compound was obtained by separating the deposited solid by filtration and washing it with ethanol.
¹H NMR (DMSO-d₆, 400MHz) δ 1.90-1.96 (2H, m), 2.44 (3H, s), 3.74-4.06 (8H, m), 6.45 (1H, d, J = 7.4 Hz), 6.84 (1H, d, J = 9.4 Hz), 7.35-7.38 (1H, m), 7.95 (1H, brd, J = 9.4 Hz), 8.41 (1H, br), 8.71 (1H, brs), 12.59 (1H, br).

### (134e) 4-[4-(5-acetylpyridin-2-yl)-1,4-diazepan-1-yl]-3-aminothieno[2,3-b]pyridine-2-carboxamide

The title compound was obtained by performing the reaction following a method described in Example 5 (5c) using a crude product of 4-[4-(5-acetylthien-2-yl)-1,4-diazepan-1-yl]-2-thioxo-1,2-dihydropyridine-3-carbonitrile which was produced in Example 134 (134d). Yield 10% from (2Z)-3-[4-(5-acetylpyridin-2-yl)-1,4-diazepan-1-yl]-2-cyanobut-2-enethioamide.
Mp >250°C;
IR (KBr) νₘₐₓ 3442, 3329, 3170, 1647, 1596, 1277 cm-¹_{;}
¹H NMR (DMSO-d₆, 400MHz) δ 2.12-2.20 (2H, m), 2.46 (3H, s), 3.17-3.35 (4H, m), 3.78-3.84 (2H, m), 4.04-4.13 (2H, m), 6.78 (1H, d, J = 9.0 Hz), 6.98 (2H, br), 7.05 (1H, d, J = 5.5 Hz), 7.09 (2H, br), 7.97 (1H, dd, J = 2.4, 9.0 Hz), 8.37 (1H, d, J = 5.5 Hz), 8.72 (1H, d, J = 2.4 Hz);
MS (FAB) m/z: 411 [M + H]⁺;
Anal. Calcd for C₁₇H₂₁N₅SO·0.16 H₂O: C, 58.96; H, 6.20; N, 20.22; S, 9.26. Found: C, 59.28; H, 6.29; N, 19.89; S, 8.94.

### (Example 135) 4-[4-(5-acetylthiophen-2-yl)-1,4-diazepan-1-yl]-3-aminothieno[2,3-b]pyridine-2-carboxamide (Exemplified Compound No. 3-1111)

### (135a) 1-[5-(1,4-diazepan-1-yl)thiophen-2-yl]ethanone

After 2-acetyl-5-bromothiophene (1.03 g, 5 mmol) and homopiperazine (1.50 g, 15 mmol) was mixed in water (5 mL), the mixture was stirred under heat reflux for ten hours. 1N aqueous solution of sodium hydroxide (10 mL) was added to the reaction mixture and the resultant solution was extracted with methylene chloride (2x30 mL). After the extract was dried over sodium sulfate, crude crystal which was obtained by evaporating the solvent under reduced pressure was recrystallized from ethyl acetate (30 mL) and 0.80 g (71 %) of the title compound was obtained.
Mp 130-132°C;
IR (KBr) νₘₐₓ 3322, 1599, 1497, 1328, 788 cm⁻¹;
¹H NMR (CDCl₃, 400MHz) δ 1.77 (1H, br), 1.90-1.96 (2H, m), 2.40 (3H, s), 2.87-2.90 (2H, m), 3.04-3.07 (2H, m), 3.53-3.62 (4H, m), 5.85 (1H, d, J = 4.3 Hz), 7.44 (1H, d, J = 4.3 Hz);
MS (EI) m/z: 224 [M⁺], 182, 168;
Anal. Calcd for C₁₁H₁₆N₂SO·0.2 H₂O: C, 57.97; H, 7.25; N, 12.29; S, 14.07. Found: C, 58.09; H, 7.31; N, 12.31; S, 13.86.

### (135b) (2Z)-3-[4-(5-acetylthiophen-2-yl)-1,4-diazepan-1-yl]-2-cyanobut-2-enethioamide

The reaction was performed following a method described in Example 5 (5a) using 1-[5-(1,4-diazepan-1-yl)thiophen-2-yl]ethanone which was produced in Example 135 (135a) in place of isobutylamine and the title compound was obtained.
Yield 62%.
Mp 183-185°C;
IR (KBr) νₘₐₓ 3331, 3288, 3171, 2186, 1601, 1536, 1493, 1440, 1100 cm⁻¹;
¹H NMR (DMSO-d₆, 400MHz) δ 1.95-2.02 (2H, m), 2.26 (3H, s), 2.31 (3H, s), 3.55-3.79 (8H, m), 6.13 (1H, d, J = 4.0 Hz), 7.65 (1H, d, J = 4.0 Hz), 8.48 (1H, br), 9.12 (1H, br);
MS (FAB) m/z: 349 [M + H]⁺;
Anal. Calcd for C₁₆H₂₀N₄S₂O·0.1 H₂O: C, 54.86; H, 5.81; N, 15.99; S, 18.31. Found: C, 54.75; H, 6.01; N, 15.76; S, 18.33.

### (135c) 4-[4-(5-acetylthiophen-2-yl)-1,4-diazepan-1-yl]-2-thioxo-1,2-dihydropyridine-3-carbonitrile

(2Z)-3-[4-(5-acetylthiophen-2-yl)-1,4-diazepan-1-yl]-2-cyanobut-2-enethioamide (0.65 g, 1.9 mmol) which was produced in Example 135 (135b) and

N,N-dimethylformamide dimethylacetal (0.24 g, 0.27 mL, 2.1 mmol) were dissolved in N,N-dimethylformamide (6 mL) and the resultant solution was stirred at room temperature for one hour. Furthermore, the reaction mixture was cooled to room temperature after having reacted at 100°C for two hours. Ethyl acetate and water were added to the reaction liquid and the deposited solid was separated by filtration and a crude product (0.11 g) of the title compound was obtained by further washing the deposited solid with water and ethanol.
¹H NMR (DMSO-d₆, 400MHz) δ 1.94-2.00 (2H, m), 2.28 (3H, s), 3.59-3.61 (2H, m), 3.73-3.75 (2H, m), 3.81-3.83 (2H, m), 4.01-4.04 (2H, m), 6.11 (1H, d, J = 4.7 Hz), 6.46 (1H, d, J = 7.4 Hz), 7.36-7.39 (1H, m), 7.59 (1H, d, J = 4.7 Hz).

### (135d) 4-[4-(5-acetylthiophen-2-yl)-1,4-diazepan-1-yl]-3-aminothieno[2,3-b]pyridine-2-carboxamide

The title compound was obtained by performing the reaction following a method described in Example 5 (5c) using a crude product of 4-[4-(5-acetylthiophen-2-yl)-1,4-diazepan-1-yl]-2-thioxo-1,2-dihydropyridine-3-carbonitrile which was produced in Example 135 (135c). Yield 10% from (2Z)-3-[4-(5-acetylthiophene-2-yl)-1,4-diazepan-1-yl]-2-cyanobut-2-enethioamide.
Mp 239-241°C;
IR (KBr) νₘₐₓ 3439, 3326, 3188, 1646, 1580, 1486, 1448, 1093 cm⁻¹;
¹H NMR (DMSO-d₆, 400MHz) δ 2.17-2.23 (2H, m), 2.30 (3H, s), 3.18-3.24 (2H, m), 3.32-3.36 (2H, m), 3.56-3.60 (2H, m), 3.80-3.84 (2H, m), 6.07 (1H, d, J = 4.5 Hz), 6.99 (2H, br), 7.06 (1H, d, J = 5.5 Hz), 7.10 (2H, br), 7.64 (1H, d, J = 4.5 Hz), 8.40 (1H, d, J = 5.5 Hz);
MS (FAB) m/z: 416 [M + H]⁺;
Anal. Calcd for C₁₉H₂₁N₅S₂O₂·2.3 H₂O: C, 49.94; H, 5.65; N, 15.33; S, 14.03. Found: C, 50.05; H, 5.40; N, 15.46; S, 13.83.

### (Example 136) 3-amino-4-(4-{4-[(dimethylamino)carbonyl]-1,3-thiazol-2-yl}-1,4-diazepan-1-yl)thieno[2,3-b]pyridine-2-carboxamide (Exemplified Compound No. 3-1116)

### (136a) tert-butyl 4-[(benzoylamino)carbonothioyl]-1,4-diazepane-1-carboxylate

Benzoyl isothiocyanate (1.6 mL, 12 mmol) was added to a tetrahydrofuran (20 mL) solution of tert-butyl 1,4-diazepane-1-carboxylate (1.97 mL, 10 mmol) and the resultant mixture was stirred for ten hours. The reaction liquid was concentrated, and the obtained residue was purified by silica gel column chromatography (hexane/ethyl acetate =1:1) and the title compound was obtained (3.46 g, yield 95%).
Pale red foam
IR (KBr) νₘₐₓ 3244, 2974, 1693, 1526, 1416, 1246, 1166, 709 cm⁻¹;
¹H NMR(CDCl₃, 500 MHz) δ 1.48 (9H, s), 2.09-2.17 (2H, m), 3.48-3.63 (4H, m), 3.79-3.88 (2H, m), 4.10-4.17 (2H, m), 7.49-7.51 (2H, m), 7.58-7.60 (1H, m), 7.84 (2H, d, J = 7.8 Hz), 8.26-8.29 (1H, m);
HRMS m/z calcd for C₁₈H₂₅O₃N₃SNa 386.1514, found 386.1508;
MS (FAB) m/z: 364 [M+H]⁺, 348, 332, 308, 274, 264, 246, 230, 214, 187, 165, 105, 93, 89, 77, 65, 57.

### (136b) tert-butyl 4-(aminocarbonothioyl)-1,4-diazepane-1-carboxylate

Sodium methylate (4.9M methanol solution) (2.1 mL, 10.5 mmol) was added to methanol (50 mL) solution of tert-butyl 4-[(benzoylamino)carbonothioyl]-1,4-diazepane-1-carboxylate (3.46 g, 9.5 mmol) which was produced in Example (136a) and the resultant mixture was heated under reflux for two days. The reaction liquid was concentrated and the obtained residue was purified by silica gel column chromatography (ethyl acetate) and the title compound was obtained (2.52 g, yield 93%).
White foam
IR (KBr) νₘₐₓ 3370, 3199, 2976, 1681, 1641, 1492, 1420, 1365, 1168 cm⁻¹;
¹H NMR(CDCl₃, 500 MHz) δ 1.47 (9H, s), 1.96-1.99 (2H, m), 3.38-3.44 (2H, m), 3.57-3.61 (6H, m), 5.71 (2H, brs);
HRMS m/z calcd for C₁₁H₂₁O₂N₃SNa 282.1252, found 282.1228;
MS (ESI) m/z: 282 [M+Na]⁺, 260;
Anal. Calcd for C₁₁H₂₁N₃O₂S: C, 50.94; H, 8.16; N, 16.20; S, 12.36. Found: C, 51.01; H, 8.18; N, 16.02, S, 12.21.

### (136c) tert-butyl 4-[4-(ethoxycarbonyl)-1,3-thiazol-2-yl]-1,4-diazepane-1-carboxylate

Triethylamine (2.2 mL, 15.8 mmol) and ethyl bromopyruvate (2.0 mL, 15.8 mmol) were added to an ethanol (20 mL) solution of tert-butyl 4-(aminocarbonothioyl)-1,4-diazepane-1-carboxylate (2.04 g, 7.9 mmol) which was produced in Example (136b) and the resultant mixture was heated under reflux for 30 minutes. The reaction liquid was concentrated, and the obtained residue was purified by silica gel column chromatography (hexane/ethyl acetate = 2:1) and the title compound was obtained (2.80 g, yield 99%).
Pale yellow liquid
IR (film) νₘₐₓ 2978, 1695, 1550, 1416, 1368, 1212, 1168 cm⁻¹;
¹H NMR(CDCl₃, 500 MHz) δ1.36 (3H, t, J = 7.3 Hz), 1.43 (4.5H, s), 1.44 (4.5H, s), 2.01 (2H, quint, J = 5.9 Hz), 3.35 (1H, t, J = 5.9 Hz), 3.43 (1H, t, J = 4.4 Hz), 3.61-3.77 (6H, m), 4.34 (2H, q, J = 7.3 Hz), 7.38 (1H, s);
MS (FAB) m/z: 356 [M+H]⁺, 273, 242, 226, 165, 65.

### (136d) tert-butyl 4-{4-[(dimethylamino)carbonyl]-1,3-thiazol-2-yl]-1,4-diazepane-1-carboxylate

Trimethylaluminum (2.0M toluene solution, 7.9 mL, 15.8 mmol) was added dropwise to a toluene (16 mL) solution of dimethylamine hydrochloride (1.28 g, 15.8 mmol) under nitrogen atmosphere and the resultant mixture was stirred for two hours. The above dimethylaluminum amide solution was added dropwise to a toluene (80 mL) solution of tert-butyl 4-[4-(ethoxycarbonyl)-1,3-thiazol-2-yl]-1,4-diazepane-1-carboxylate (2.80 g, 7.9 mmol) which was produced in Example (136c) and the resultant solution was heated under reflux for 50 minutes. An ammonium chloride aqueous solution (50 mL) and ethyl acetate (50 mL) were added to the reaction liquid and the resultant solution was filtered with Celite. The obtained filtrate was extracted with ethyl acetate (50 mL) twice and the solvent was evaporated under reduced pressure after drying the extract over sodium sulfate. The obtained residue was purified by silica gel column chromatography (hexane/ethyl acetate =1:1) and the title compound was obtained (1.69 g, yield 60%).
Pale yellow liquid
IR (film) νₘₐₓ 3492, 2932, 1693, 1626, 1546, 1414, 1169 cm⁻¹;
¹H NMR(CDCl₃, 500 MHz) δ 1.44 (9H, s), 2.00 (2H, quint, J = 5.9 Hz), 3.06 (3H, brs), 3.24 (3H, brs), 3.33.(1H, t, J = 5.9 Hz), 3.42 (1H, t, J = 5.9 Hz), 3.57-3.68 (6H, m), 7.02 (1H, s);
MS (FAB) m/z: 355 [M+H]⁺, 255, 242, 210, 57.

### (136e) 2-(1,4-diazepan-1-yl)-N,N-dimethyl-1,3-thiazole-4-carboxamide

The reaction was performed following a method described in Example 57 (57b) using tert-butyl 4-{4-[(dimethylamino)carbonyl]-1,3-thiazol-2-yl}-1,4-diazepane-1-carboxylate which was produced in Example 136 (136d) and the title compound was obtained.
Pale yellow liquid
IR (film) νₘₐₓ 3442, 2936, 1618, 1548, 1453, 1398, 1319, 1175, 719 cm⁻¹;
¹H NMR(CDCl₃, 500 MHz) δ 1.93 (2H, quint, J = 5.9 Hz), 2.90 (2H, t, J = 5.9 Hz), 3.04-3.06 (5H, m), 3.25 (3H, brs), 3.65-3.69 (4H, m), 6.99 (1H, s);
MS (EI) m/z: 254 [M⁺], 211, 198, 185, 172, 167, 139, 128, 112, 83, 70, 56, 44.

### (136f) 2-[4-(3-cyano-2-thioxo-1,2-dihydropyridin-4-yl)-1,4-diazepan-1-yl]-N,N-dimethyl-1,3-thiazole-4-carboxamide

The reaction was performed following a method described in Example 118 (118c) using 2-(1,4-diazepan-1-yl)-N,N-dimethyl-1,3-thiazole-4-carboxamide which was produced in Example 136 (136e) and the title compound was synthesized. Brown foam
IR (KBr) νₘₐₓ 2929, 1621, 1543, 1236, 1174, 1141, 922, 777 cm⁻¹;
¹H NMR(DMSO-d₆, 500 MHz) δ 2.17 (2H, quint, J = 5.4 Hz), 3.07 (3H, brs), 3.21 (3H, brs), 3.67 (2H, t, J = 6.3 Hz), 3.86 (2H, t, J = 5.9 Hz), 3.96 (2H, t, J = 5.4 Hz), 4.14 (2H, t, J = 4.9 Hz), 6.25 (1H, d, J = 7.8 Hz), 7.05 (1H, s), 7.33 (1H, d, J = 7.8 Hz), 12.35 (1H, brs);
MS (FAB) m/z: 389 [M+H]⁺, 273, 242, 226, 1665, 65.

### (136 g) 3-amino-4-(4-{4-[(dimethylamino)carbonyl]-1,3-thiazol-2-yl}-1,4-diazepan-1-yl)thieno [2,3-b]pyridine-2-carboxamide

The reaction was performed following a method described in Example 5 (5c) using 2-[4-(3-cyano-2-thioxo-1,2-dihydropyridin-4-yl)-1,4-diazepan-1-yl]-N,N-dimethyl-1,3-thiazole-4-carboxamide which was produced in Example 136 (136f) and the title compound was synthesized.
White powder
Mp 285-287°C(dec.);
IR (KBr) νₘₐₓ 3441, 3326, 3165, 1669, 1603, 1532, 1370, 1174, 922, 664, 628 cm⁻¹; ¹H NMR(DMSO-d₆, 400 MHz) δ 2.20-2.22 (2H, m), 2.93 (3H, brs), 3.12 (3H, brs), 3.24 (2H, brs), 3.38 (2H, brs), 3.65 (2H, t, J = 6.3 Hz), 3.94-3.96 (2H, m), 7.03 (2H, brs), 7.08-7.10 (2H, m), 7.12 (2H, brs), 8.42 (1H, d, J = 5.5 Hz);
HRMS m/z calcd for C₁₉H₂₄O₂N₇S₂ 446.1433, found 446.1409;
MS (ESI) m/z: 446 [M+H]⁺, 429;
Anal. Calcd for C₁₉H₂₃N₇O₂S₂·0.6H₂O: C, 50.00; H, 5.34; N, 21.48. Found: C, 50.01; H, 5.38; N,21.37.

### (Example 137) 4-[4-(4-acetyl-1,3-thiazol-2-yl)-1,4-diazepan-1-yl]-3-aminothieno[2,3-b]pyridine-2-carboxamide (Exemplified Compound No. 3-1110)

### (137a) tert-butyl 4-(4-acetyl-1,3-thiazol-2-yl)-1,4-diazepane-1-carboxylate

Methyl magnesium chloride (3M tetrahydrofuran solution) (1.2 mL, 3.6 mmol) was added dropwise to a tetrahydrofuran (25 mL) solution of tert-butyl 4-{4-[(dimethylamino)carbonyl]-1,3-thiazol-2-yl}-1,4-diazepan-1-carboxylate (1.05 g, 3.0 mmol) which was produced in Example 136 (136d) at 0°C and the resultant solution was stirred for three hours. An ammonium chloride aqueous solution (30 mL) was added to the reaction liquid and the resultant solution was extracted with ethyl acetate (50 mL) twice, and the solvent was evaporated under reduced pressure after drying the extract over sodium sulfate. The obtained residue was purified by silica gel column chromatography (hexane/ethyl acetate = 1:1) and the title compound was obtained (0.66 g, yield 69%).
Colourless liquid
IR (film) νₘₐₓ 2975, 1687, 1549, 1416, 1167, 927, 606 cm⁻¹;
¹H NMR(CDCl₃, 500 MHz) δ 1.43 (4.5H, s), 1.44 (4.5H, s), 2.01 (2H, quint, J = 6.4 Hz), 2.53 (3H, s), 3.35 (1H, t, J = 5.9 Hz), 3.43 (1H, t, J = 4.9 Hz), 3.63-3.71 (6H, m), 7.35 (1H, s);
MS (FAB) m/z: 326 [M+H]⁺, 270, 252, 224, 169, 57.

### (137b) 1-[2-(1,4-diazepan-1-yl)-1,3-thiazol-4-yl]ethanone

The reaction was performed following a method described in Example 57 (57b) using tert-butyl 4-(4-acetyl-1,3-thiazol-2-yl)-1,4-diazepane-1-carboxylate which was produced in Example 137 (137a) and the title compound was synthesized. Yellow liquid
IR (film) νₘₐₓ 3329, 2938, 1679, 1550, 1355, 1211, 915, 695, 605 cm⁻¹;
¹H NMR(CDCl₃, 400 MHz) δ 1.94 (2H, quint, J = 4.9 Hz), 2.54 (3H, s), 2.92 (2H, t, J = 5.5 Hz), 3.07 (2H, t, J = 5.5 Hz), 3.69-3.73 (4H, m), 7.34 (1H, s);
MS (EI) m/z: 225 [M⁺], 210, 197, 183, 169, 155, 141, 128, 83, 70, 56, 43.

### (137c) 4-[4-(4-acetyl-1,3-thiazol-2-yl)-1,4-diazepan-1-yl]-2-thioxo-1,2-dihydropyridine-3-carbonitrile

The reaction was performed following a method described in Example 121 (121c) using 1-[2-(1,4-diazepan-1-yl)-1,3-thiazol-4-yl]ethanone which was produced in Example 137 (137b) and the title compound was synthesized.
Brown solid
IR (KBr) νₘₐₓ 3097, 2959, 2204, 1672, 1623, 1545, 1354, 1242, 1143, 924, 776, 604 cm⁻¹;
¹H NMR(DMSO-d₆, 500 MHz) δ 1.96-2.01 (2H, m), 2.42 (3H, s), 3.68 (2H, t, J = 5.9 Hz), 3.84-3.88 (4H, m), 4.09 (2H, t, J = 5.4 Hz), 6.47 (1H, d, J = 7.8 Hz), 7.38 (1H, d, J = 7.8 Hz), 7.64 (1H, s), 12.54 (1H, brs);
MS (FAB) m/z: 360 [M+H]⁺, 344, 328, 273, 242, 226, 165, 65;
Anal. Calcd for C₁₆H₁₇N₅OS₂·0.33H₂O: C, 52.58; H, 4.87; N, 19.16; S, 17.55. Found: C, 52.38; H, 4.68; N, 19.37; S, 17.48.

### (137d) 4-[4-(4-acetyl-1,3-thiazol-2-yl)-1,4-diazepan-1-yl]-3-aminothieno[2,3-b]pyridine-2-carboxamide

The reaction was performed following a method described in Example 5 (5c) using 4-[4-(4-acetyl-1,3-thiazol-2-yl)-1,4-diazepan-1-yl]-2-thioxo-1,2-dihydropyridine-3-carbonitrile which was produced in Example 137 (137c) and the title compound was synthesized.
Pale yellow powder
Mp 217-219°C;
IR (KBr) νₘₐₓ 3427, 3318, 3172, 1673, 1581, 1543, 1365, 1211, 937, 602 cm⁻¹;
¹H NMR(DMSO-d₆, 400 MHz) δ 2.19-2.24 (2H, m), 2.45 (3H, s), 3.23 (2H, brs), 3.39 (2H, brs), 3.67 (2H, t, J = 5.9 Hz), 3.97 (2H, brs), 7.01 (2H, brs), 7.08 (1H, d, J = 5.5 Hz), 7.11 (2H, brs), 7.66 (1H, s), 8.04 (1H, d, J = 5.5 Hz);
HRMS m/z calcd for C₁₈H₂₁O₂N₆S₂ 417.1167, found 417.1173;
MS (ESI) m/z: 417 [M+H]⁺, 400;
Anal. Calcd for C₁₈H₂₀N₆O₂S₂·0.33H₂O: C, 51.17; H, 4.93; N, 19.89; S, 15.18. Found: C, 51.18; H, 4.85; N, 20.16; S, 14.83.

### (Example 138) 3-amino-4-[4-(2-methyl-1-oxo-2,3-dihydro-1H-isoindol-5-yl)-1,4-diazepan-1-yl]thieno[2,3-b]pyridine-2-carboxamide (Exemplified Compound No. 3-1098)

### (138a) ethyl 4-bromo-2-methylbenzoate

4N hydrochloric acid-1,4-dioxane solution was added to an ethanol solution of 4-bromo 2-methyl-benzoic acid (4.30 g, 20.0 mmol) and the resultant solution was stirred at 80°C for nine hours. The reaction liquid was concentrated and methylene chloride (200 mL) was added to the obtained residual substance and the resultant mixture was washed with 1N aqueous solution of sodium hydroxide (50 mL) and a saturated sodium chloride aqueous solution (50 mL), and the solvent was evaporated under reduced pressure after drying the extract over sodium sulfate and the title compound was obtained (4.54 g, yield 93%).
Yellow liquid
IR (film) vₘₐₓ 2981, 1722, 1589, 1445, 1253, 1082, 863, 771 cm⁻¹;
¹H NMR(CDCl₃, 400 MHz) δ 1.38 (3H, t, J = 7.8 Hz), 2.57 (3H, s), 4.34 (2H, q, J = 7.8 Hz), 7.37 (1H, d, J = 8.2 Hz), 7.40 (1H, s), 7.77 (1H, d, J = 8.2 Hz);
MS (EI) m/z: 242 [M⁺], 199, 197, 171, 169, 163, 90, 63.

### (138b) ethyl 4-bromo-2-(bromomethyl)benzoate

N-bromosuccinimide (3.62 g, 20.0 mmol), 2,2'-azobis (isobutyronitrile) (608 mg, 3.70 mmol) were sequentially added to carbon tetrachloride (90 ml) solution of ethyl 4-bromo-2-methylbenzoate (4.50 g, 18.5 mmol) which was produced in Example 138 (138a) and the resultant mixture was stirred at 90°C for three hours. The reaction liquid was concentrated and hexane was added to the obtained residual substance and the resultant mixture was filtered. The filtrate was concentrated and a crude title compound was obtained (5.90 g, yield 99%).
Yellow liquid

### (138c) 5-bromo-2-methylisoindolin-1-one

2M methylamine methanol solution was added to ethyl 4 -bromo-2-(bromomethyl)benzoate (5.62 g, 17.5 mmol) which was produced in Example 138 (138b) and the resultant mixture was stirred at 70°C for 23 hours. The reaction liquid was concentrated and the obtained residual substance was purified by silica gel column chromatography (hexane/ethyl acetate =1:2 to 1:4) and the title compound was obtained (2.70 g, yield 68%).
White powder
Mp 132-134°C;
IR (KBr) νₘₐₓ 2914, 1680, 1400, 1275, 1043, 769 cm⁻¹;
¹H NMR(CDCl₃, 400 MHz) δ 3.19 (3H, s), 4.36 (2H, s), 7.58-7.63 (2H, m), 7.70 (1H, d, J = 8.6 Hz);
MS (EI) m/z: 225 [M⁺], 199, 198, 197, 196, 171, 169, 146, 112, 98, 89, 75, 59;
Anal. Calcd for C₉H₈BrNO: C, 47.82; H, 3.57; N, 6.20. Found: C, 47.80; H, 3.59; N, 6.22.

### (138d) benzyl 4-(2-methyl-1-oxo-2,3-dihydro-1H-isoindol-5-yl)-1,4-diazepane-1-carboxylate

The reaction was performed following a method described in Example 117 (117a) using 5-bromo-2-methylisoindolin-1-one which was produced in Example 138 (138c) and benzyl 1-homopiperazinecarboxylate and the title compound was synthesized.
Pale yellow liquid
IR (film) νₘₐₓ 3451, 2947, 1679, 1479, 1239, 1120, 927, 754 cm⁻¹;
¹H NMR(CDCl₃, 500 MHz) δ 1.93-2.05 (2H, m), 3.14 (3H, s), 3.33 (1H, t, J = 5.9 Hz), 3.41 (1H, t, J = 5.9 Hz), 3.58-3.70 (6H, m), 4.21 (1H, s), 4.26 (1H, m), 5.05 (1H, s), 5.13 (1H, s), 6.64 (1H, m), 6.73 (1H, m), 7.26-7.86 (5H, m), 7.65 (1H, m);
MS (FAB) m/z: 380 [M+H⁺], 379, 353, 273, 246, 244.

### (138e) 5-(1,4-diazepan-1-yl)-2-methylisoindoline-1-one

Hydrogenolysis reaction was performed using benzyl 4-(2-methyl-1-oxo-2,3-dihydro-1H-isoindol-5-yl)-1,4-diazepane-1-carboxylate which was produced in Example 138 (138d) following a method described in Example 128 (128d) and the title compound was obtained.
Yellow liquid
IR (film) νₘₐₓ 3421, 1658, 1480, 1399, 1250, 1111, 768 cm⁻¹;
¹H NMR(CDCl₃, 400 MHz) δ 1.85-1.96 (2H, m), 2.83 (2H, t, J = 5.9 Hz), 3.05 (2H, t, J = 5.5 Hz), 3.14 (3H, s), 3.60 (2H, t, J = 5.5 Hz), 3.64 (2H, t, J = 5.9 Hz), 4.27 (2H, s), 6.65 (1H, s), 6.74 (1H, d, J = 8.6 Hz), 7.64 (1H, d, J = 8.6 Hz);
MS (EI) m/z: 245 [M⁺], 203, 189, 177, 146, 83, 70.

### (138f) 4-[4-(2-methyl-1-oxo-2,3-dihydro-1H-isoindol-5-yl)-1,4-diazepan-1-yl]-2-thioxo-1,2-dihydropyridine-3-carbonitrile

The reaction was performed following a method described in Example 121 (121c) using 5-(1,4-diazepan-1-yl)-2-methylisoindolin-1-one which was produced in Example 138 (138e) and the title compound was synthesized.
Brown powder
Mp 257-265°C;
IR (KBr) νₘₐₓ 2950, 2203, 1660, 1615, 1522, 1246, 926, 767 cm⁻¹;
¹H NMR(DMSO-d₆, 500 MHz) δ 1.93-2.00 (2H, m), 3.31 (3H, s), 3.62 (2H, t, J = 5.9 Hz), 3.75 (2H, t, J = 5.9 Hz), 3.82 (2H, t, J = 5.4 Hz), 4.00 (2H, t, J = 5.4 Hz), 4.31 (2H, s), 6.44 (1H, d, J = 7.8 Hz), 6.86 (1H, d, J = 8.8 Hz), 6.92 (1H, s), 7.36 (1H, d, J = 7.8 Hz), 7.41 (1H, d, J = 8.8 Hz), 12.55 (1H, brs);
MS (FAB) m/z: 380 [M+H⁺], 273, 257, 238, 165, 85, 63;
Anal. Calcd for C₂₀H₂₁N₅OS·1.75H₂O: C, 58.45; H, 6.01; N, 17.04. Found: C, 58.21; H, 5.66; N, 16.82.

### (138 g) 3-amino-4-[4-(2-methyl-1-oxo-2,3-dihydro-1H-isoindol-5-yl)-1,4-diazepan-1-yl]thieno[2,3-b]pyridine-2-carboxamide

The reaction was performed following a method described in Example 5 (5c) using 4-[4-(2-methyl-1-oxo-2,3-dihydro-1H-isoindol-5-yl)-1,4-diazepan-1-yl]-2-thioxo-1,2-dihydropyridine-3-carbonitrile which was produced in Example 138 (138f) and the title compound was synthesized.
Pale yellow powder
Mp 293-295°C;
IR (KBr) νₘₐₓ 3442, 3324, 3171, 2918, 1653, 1503, 1366, 1235, 1110, 767 cm⁻¹;
¹H NMR(DMSO-d₆, 500 MHz) δ 2.13-2.24 (2H, m), 3.00 (3H, s), 3.14-3.24 (2H, m), 3.28-3.33 (2H, m), 3.63 (2H, t, J = 6.4 Hz), 3.85 (2H, t, J = 4.4 Hz), 4.32 (2H, s), 6.86 (1H, d, J = 8.8 Hz), 6.90 (1H, s), 7.01 (2H, brs), 7.07 (1H, d, J = 5.4 Hz), 7.11 (2H, brs), 7.44 (1H, d, J = 8.8 Hz), 8.40 (1H, d, J = 5.4 Hz);
MS (FAB) m/z: 437 [M+H⁺], 420, 273, 246, 165, 93, 63;
Anal. Calcd for C₂₂H₂₄N₆O₂S·0.67H₂O: C, 58.91; H, 5.69; N, 18.74. Found: C, 58.83; H, 5.65; N, 18.74.

### (Example 139) 3-amino-4-[4-(1,3-benzoxazol-6-yl)-1,4-diazepan-1-yl]thieno[2,3-b]pyridine-2-carboxamide (Exemplified Compound No. 3-1099)

### (139a) tert-butyl 4-(3-hydroxy-4-nitrophenyl)-1,4-diazepane-1-carboxylate

The reaction was performed following a method described in Example 59 (59a) using 5-fluoro-2-nitrophenol and tert-butyl 1-homopiperazinecarboxylate and the title compound was synthesized.
Yellow powder
Mp 106-109°C;
IR (KBr) νₘₐₓ 3431, 2973, 1690, 1625, 1566, 1415, 1325, 1262, 1166, 927, 754 cm⁻¹;
¹H NMR(CDCl₃, 500 MHz) δ 1.39 (4.5H, s), 1.43 (4.5H, s), 1.95-1.99 (2H, m), 3.27 (1H, t, J = 5.9 Hz), 3.37 (1H, t, J = 5.9 Hz), 3.57-3.68 (6H, m), 6.18 (1H, brs), 6.31 (1H, brd, J = 9.3 Hz), 7.95 (1H, d, J = 9.3 Hz), 11.31 (1H, brs);
MS (EI) m/z: 337 [M⁺], 280, 264, 236, 220, 193, 181, 70, 57;
Anal. Calcd for C₁₆H₂₃N₃O₅: C, 56.96; H, 6.87; N, 12.46. Found: C, 56.97; H, 6.85; N, 12.39.

### (139b) tert-butyl 4-(1,3-benzoxazol-6-yl)-1,4-diazepane-1-carboxylate

Trimethyl orthoformate (80 mL), 10% palladium-carbon (5.2 g) were sequentially added to an ethanol (80 mL) solution of tert-butyl 4-(3-hydroxy-4-nitrophenyl)-1,4-diazepane-1-carboxylate (5.20 g, 15.4 mmol) which was produced in Example 139 (139a) and the resultant mixture was stirred under normal pressure hydrogen atmosphere at room temperature for five hours and then stirred at 90°C for 17 hours. The reaction mixture was filtered with Celite and the filtrate was concentrated. The obtained residual substance was purified by silica gel column chromatography (hexane/ethyl acetate =2:1 to 1:1 with one) and the title compound was obtained (611 mg, yield 13%).
Yellow liquid
IR (film) νₘₐₓ 2974, 1688, 1630, 1500, 1416, 1246, 1168, 1057, 929 cm⁻¹;
¹H NMR(CDCl₃, 500 MHz) δ 1.32 (4.5H, s), 1.42 (4.5H, s), 1.96-2.04 (2H, m), 3.21 (1H, t, J = 5.9 Hz), 3.33 (1H, t, J = 5.9 Hz), 3.57-3.65 (6H, m), 6.77 (1H, dd, J = 2.0, 8.8 Hz), 6.82 (1H, brs), 7.57 (1H, d, J = 8.8 Hz), 7.87 (1H, s);
MS (FAB) m/z: 317 [M⁺], 262, 242, 216, 165, 63.

### (139c) 6-(1,4-diazepan-1-yl)-1,3-benzoxazole

A methylene chloride (10 mL) solution of tert-butyl 4-(1,3-benzoxazol-6-yl)-1,4-diazepane-1-carboxylate (534 mg, 1.68 mmol) which was produced in Example 139 (139b) was cooled to 0°C and was sequentially blended with 2,6-lutidine (0.5 mL, 4.30 mmol), trimethylsilyl trifluoromethanesulfonate (0.5 mL, 2.76 mmol) and the resultant mixture was stirred at 0°C for one hour. The reaction liquid was concentrated and the obtained residual substance was purified by silica gel column chromatography (methylene chloride/methanol =1:0 to 20:1) and the title compound was obtained (267 mg, yield 73%).
Yellow liquid
IR (film) vₘₐₓ 3396, 2932, 1624, 1510, 1358, 1176, 925 cm⁻¹;
¹H NMR(CDCl₃, 400 MHz) δ 1.91-1.97 (2H, m), 2.84 (2H, t, J = 5.9 Hz), 3.07 (2H, t, J = 5.4 Hz), 3.60 (2H, t, J = 5.4 Hz), 3.64 (2H, t, J = 6.3 Hz), 6.77 (1H, dd, J = 2.0, 8.6 Hz), 6.82 (1H, d, J = 2.0 Hz), 7.57 (1H, d, J = 8.6 Hz), 7.87 (1H, s);
MS (FAB) m/z: 218 [M+H⁺], 161, 63.

### (139d) (2Z)-3-[4-(1,3-benzoxazol-6-yl)-1,4-diazepan-1-yl]-2-cyanobut-2-enethioamide

The reaction was performed following a method described in Example 5 (5a) using 6-(1,4-diazepan-1-yl)-1,3-benzoxazole which was produced in Example 139 (139c) in place of isobutylamine and the title compound was obtained.
Yellow powder
Mp 177-180°C;
IR (KBr) νₘₐₓ 3288, 3170, 2182, 1627, 1517, 1442, 1358, 1289, 1208, 1060, 876, 801 cm⁻¹;
¹H NMR(DMSO-d₆, 500 MHz) δ 1.95-2.01 (2H, m), 2.24 (3H, s), 3.53 (2H, t, J = 5.4 Hz), 3.62 (2H, t, J = 5.9 Hz), 3.66 (2H, t, J = 5.4 Hz), 3.81 (2H, t, J = 5.9 Hz), 6.88 (1H, dd, J = 2.0, 8.8 Hz), 7.11 (1H, d, J = 2.0 Hz), 7.54 (1H, d, J = 8.8 Hz), 8.37 (1H, brs), 8.41 (1H, s), 9.02 (1H, brs);
MS (FAB) m/z: 342 [M+H⁺], 326, 273, 258, 242, 226, 180, 165, 63;
Anal. Calcd for C₁₇H₁₉N₅OS.0.30H₂O: C, 58.91; H, 5.69; N, 20.21. Found: C, 59.26; H, 5.56; N, 19.92.

### (139e) 4-[4-(1,3-benzoxazol-6-yl)-1,4-diazepan-1-yl]-2-thioxo-1,2-dihydropyridine-3-carbonitrile

The reaction was performed following a method described in Example 116 (116d) using (2Z)-3-[4-(1,3-benzoxazol-6-yl)-1,4-diazepan-1-yl]-2-cyanobut-2-enethioamide which was produced in Example 139 (139d) and the title compound was obtained.
Brown powder
Mp 245-250°C;
IR (KBr) νₘₐₓ 3119, 2955, 2204, 1625, 1517, 1354, 1252, 1062, 928, 803 cm⁻¹;
¹H NMR(DMSO-d₆, 400 MHz) δ 1.94-2.02 (2H, m), 3.61 (2H, t, J = 5.9 Hz), 3.73 (2H, t, J = 5.5 Hz), 3.81 (2H, m), 4.00 (2H, t, J = 5.5 Hz), 6.44 (1H, d, J = 7.8 Hz), 6.89 (1H, d, J = 8.6 Hz), 7.13 (1H, brs), 7.36 (1H, d, J = 7.8 Hz), 7.53 (1H, d, J = 8.6 Hz),8.41 (1H, s), 12.53 (1H, brs);
MS (FAB) m/z: 352 [M+H⁺], 273, 258, 242, 226, 165, 83, 63;
Anal. Calcd for C₁₈H₁₇N₅OS·0.56H₂O: C, 59.82; H, 5.05; N, 19.38. Found: C, 59.49; H, 4.76; N, 19.86.

### (139f) 3-amino-4-[4-(1,3-benzoxazol-6-yl)-1,4-diazepan-1-yl]thieno[2,3-b]pyridine-2-carboxamide

The reaction was performed following a method described in Example 5 (5c) using 4-[4-(1,3-benzoxazol-6-yl)-1,4-diazepan-1-yl]-2-thioxo-1,2-dihydropyridine-3-carbonitrile of Example 139 (139e) and the title compound was obtained.
Pale yellow powder
Mp 159-162°C;
IR (KBr) νₘₐₓ 3441, 3323, 2948, 1643, 1580, 1500, 1455, 1368, 1204, 1058, 942, 803 cm⁻¹;
¹H NMR(DMSO-d₆, 500 MHz) δ 2.14-2.22 (2H, m), 3.16-3.23 (2H, m), 3.29-3.36 (2H, m), 3.62 (2H, t, J = 5.9 Hz), 3.84 (2H, t, J = 4.4 Hz), 6.89 (1H, dd, J = 2.4, 8.8 Hz), 7.00 (2H, brs), 7.06-7.09 (4H, m), 7.55 (1H, t, J = 8.8 Hz), 8.40 (1H, d, J = 5.4 Hz), 8.41 (1H, s);
MS (FAB) m/z: 409 [M+H⁺], 273, 258, 242, 226, 213, 180, 165, 63;
Anal. Calcd for C₂₀H₂₀N₆O₂S·0.40H₂O: C, 57.79; H, 5.04; N, 20.22. Found: C, 57.69; H, 4.79; N, 20.40.

### (Example 140) 3-amino-4-[4-(4-hydroxyimino-3,4-dihydro-2H-chromen-7-yl)-1,4-diazepan-1-yl]thieno[2,3-b]pyridine-2-carboxamide (Exemplified Compound No. 3-1100)

### (140a) benzyl 4-(4-acetyl-3-hydroxyphenyl)-1,4- diazepane-1-carboxylate

The reaction was performed following a method described in Example 59 (59a) using 4'-fluoro-2'-hydroxyacetophenone and benzyl 1-homopiperazinecarboxylate and the title compound was obtained.
Yellow liquid
IR (film) νₘₐₓ 2948, 1699, 1631, 1521, 1424, 1370, 1331, 1232, 928, 754 cm⁻¹;
¹H NMR(CDCl₃, 500 MHz) δ 1.93-2.02 (2H, m), 2.49 (3H, s), 3.33 (1H, t, J = 6.3 Hz), 3.40 (1H, t, J = 5.9 Hz), 3.56-3.64 (6H, m), 5.10 (1H, s), 5.14 (1H, s), 6.11-6.12 (1H, m), 6.20-6.23 (1H, m), 7.30-7.36 (5H, m), 7.52 (0.5H, d, J = 8.8 Hz), 7.54 (0.5H, d, J = 8.8 Hz), 12.85 (0.5H, s), 12.86 (0.5H, s);
MS (FAB) m/z: 369 [M+H]⁺, 353, 327, 273, 242, 226, 165, 65.

### (140b) benzyl 4-{4-[(2E)-3-(dimethylamino)prop-2-enoyl]-3-hydroxyphenyl}-1,4-diazepane-1-carboxylate

An N,N-dimethylformamide dimethylacetal (13 mL, 99 mmol) solution of benzyl 4-(4-acetyl-3-hydroxyphenyl)-1,4- diazepane-1-carboxylate (3.64 g, 9.9 mmol) which was produced in Example 140 (140a) was stirred at 100°C for two hours. Ethyl acetate (100 mL) was added to the reaction liquid, and the organic layer was washed with water (100 mL) twice, and the solvent was evaporated under reduced pressure after drying over sodium sulfate. The obtained residue was purified by silica gel column chromatography (100% ethyl acetate) and the title compound was obtained (3.67 g, yield 88%).
Yellow foam
IR (KBr) νₘₐₓ 2936, 1698, 1622, 1545, 1361, 1241, 1115 cm⁻¹;
¹H NMR(CDCl₃, 500 MHz) δ 1.95-2.03 (2H, m), 2.84-3.12 (6H, m), 3.31 (1H, t, J = 6.4 Hz), 3.38 (1H, t, J = 5.9 Hz), 3.54-3.64 (6H, m), 5.10 (1H, s), 5.14 (1H, s), 5.65 (1H, d, J = 12.2 Hz), 6.14-6.17 (2H, m), 7.30-7.36 (5H, m), 7.53-7.55 (1H, m), 7.79 (1H, d, J =12.2 Hz);
MS (FAB) m/z: 424 [M+H]⁺, 353, 273, 242, 226, 165, 65;
Anal. Calcd for C₂₄H₂₉N₃O₄·0.5H₂O: C, 66.65; H, 6.99; N, 9.72. Found: C, 66.52; H, 6.64; N, 9.46.

### (140c) benzyl 4-(4-oxo-4H-chromen-7-yl)-1,4-diazepane-1-carboxylate

80% acetic acid aqueous solution (10 mL) of benzyl 4-{4-[(2E)-3-(dimethylamino)prop-2-enoyl]-3-hydroxyphenyl}-1,4-diazepane-1-carboxylate (3.67 g, 8.7 mmol) which was produced in Example 140 (140b) was stirred at 100°C for two hours. Ethyl acetate (100 mL) was added to the reaction liquid and the organic layer was washed with water (100 mL) and a sodium hydrogen carbonate aqueous solution (100 mL), and the solvent was evaporated under reduced pressure after drying over sodium sulfate. The obtained residue was purified by silica gel column chromatography (ethyl acetate/methanol =20:1) and the title compound was obtained (3.19 g, yield 97%).
Yellow liquid
IR (film) νₘₐₓ 2951, 1697, 1626, 1588, 1446, 1411, 1230, 928, 754 cm⁻¹;
¹H NMR(CDCl₃, 500 MHz) δ 1.95-2.04 (2H, m), 3.55 (1H, t, J = 6.3 Hz), 3.43 (1H, t, J = 6.4 Hz), 3.61-3.69 (6H, m), 5.07 (1H, s), 5.13 (1H, s), 6.20 (1H, d, J = 6.4 Hz), 6.48-6.50 (1H, m), 6.73-6.77 (1H, m), 7.28-7.33(5H, m), 7.68(1H, d, J = 6.4 Hz), 8.00-8.03 (1H, m);
MS (FAB) m/z: 379 [M+H]⁺, 353, 273, 243, 226, 165, 91, 65;
Anal. Calcd for C₂₂H₂₂N₂O₄·0.5H₂O: C, 68.20; H, 5.98; N, 7.23. Found: C, 68.41; H, 5.89; N, 7.15.

### (140d) 7-(1,4-diazepan-1-yl)-2,3-dihydro-4H-chromen-4-one

benzyl 4-(4-oxo-4H-chromen-7-yl)-1,4-diazepane-1-carboxylate (1.98 g, 5.2 mmol) which was produced in Example 140 (140c) was subjected to hydrogenolysis in ethanol (50 mL) solvent under normal pressure hydrogen atmosphere in the presence of 10% palladium-carbon catalyst (2.23 g, 1.0 mmol) for six hours. The catalyst was removed from the reaction liquid and the title compound was obtained (1.07 g, yield 84%) by evaporating the solvent under reduced pressure.
Orange liquid
IR (film) νₘₐₓ 3322, 2934, 1663, 1601, 1406, 1143, 824 cm⁻¹;
¹H NMR(CDCl₃, 500 MHz) δ 1.89 (2H, quint, J = 5.9 Hz), 2.70 (2H, t, J = 6.4 Hz), 2.83 (2H, t, J = 5.4 Hz), 3.02 (2H, t, J = 5.4 Hz), 3.57 (2H, t, J = 5.4 Hz), 3.62 (2H, t, J = 5.9 Hz), 4.47 (2H, t, J = 6.8 Hz), 6.10 (1H, d, J = 2.4 Hz), 6.39 (1H, dd, J = 2.4, 8.8 Hz), 7.77 (1H, d, J = 8.8 Hz);
MS (EI) m/z: 246 [M⁺], 204, 190, 178, 176, 162, 123, 119, 69, 56, 44, 43.

### (140e) 4-[4-(4-oxo-3,4-dihydro-2H-chromen-7-yl)-1,4-diazepan-1-yl]-2-thioxo-1,2-dihydropyridine-3-carbonitrile

The reaction was performed following a method described in Example 121 (121c) using 7-(1,4-diazepan-1-yl)-2,3-dihydro-4H-chromen-4-one which was produced in Example 140 (140d) and the title compound was synthesized.
Brown solid
Mp 190-201 °C(dec.);
IR (KBr) νₘₐₓ 2957, 2207, 1602, 1516, 1405, 1253, 1141, 926 cm⁻¹;
¹H NMR(DMSO-d₆, 400 MHz) δ 1.88-1.93 (2H, m), 2.59 (2H, t, J = 5.9 Hz), 3.60 (2H, t, J = 5.5 Hz), 3.75 (2H, t, J = 5.9 Hz), 3.81 (2H, t, J = 5.1 Hz), 3.92-3.95 (2H, m), 4.41 (2H, t, J = 5.9 Hz), 6.24 (1H, d, J = 2.4 Hz), 6.43 (1H, d, J = 7.4 Hz), 6.53 (1H, dd, J = 2.0, 9.0 Hz), 7.36 (1H, d, J = 7.4 Hz), 7.53 (1H, d, J = 9.0 Hz), 12.55 (1H, brs);
MS (FAB) m/z: 381 [M+H]⁺, 273, 258, 242, 226, 216, 165, 63, 52.

### (140f) 4-[4-(4-hydroxyimino-3,4-dihydro-2H-chromen-7-yl)-1,4-diazepan-1-yl]-2-thioxo-1,2-dihydropyridine-3-carbonitrile

The reaction was performed following a method described in Example 120 using 4-[4-(4-oxo-3,4-dihydro-2H-chromen-7-yl)-1,4-diazepan-1-yl]-2-thioxo-1,2-dihydropyridine-3-carbonitrile which was produced in Example 140 (140e) and the title compound was synthesized.
Brown solid
Mp >290°C;
IR (KBr) νₘₐₓ 2948, 2210, 1625, 1552; 1520, 1256, 1144, 1063, 776 cm⁻¹;
¹H NMR(DMSO-d₆, 500 MHz) δ 1.90-1.93 (2H, m), 2.74 (2H, t, J = 5.9 Hz), 3.53 (2H, t, J = 5.9 Hz), 3.74 (4H, brs), 3.92-3.94 (2H, m), 4.41 (2H, t, J = 5.9 Hz), 6.21 (1H, d, J = 2.4 Hz), 6.44 (1H, d, J = 7.8 Hz), 6.47 (1H, dd, J = 2.4, 8.8 Hz), 7.38 (1H, d, J = 7.8 Hz), 7.56 (1H, d, J = 8.8 Hz), 10.68 (1H, s);
MS (FAB) m/z: 396 [M+H]⁺, 378, 273, 242, 226, 65.

### (140 g) 3-amino-4-[4-(4-hydroxyimino-3,4-dihydro-2H-chromen-7-yl)-1,4-diazepan-1-yl]thieno[2,3-b]pyridine-2-carboxamide

The reaction was performed following a method described in Example 5 (5c) using 4-[4-(4-hydroxyimino-3,4-dihydro-2H-chromen-7-yl)-1,4-diazepan-1-yl]-2-thioxo-1,2-dihydropyridine-3-carbonitrile which was produced in Example 140 (140f) and the title compound was synthesized.
White powder
Mp 178-183°C (dec.);
IR (KBr) νₘₐₓ 3441, 3327, 1606, 1513, 1369, 1183, 1061, 919, 828 cm⁻¹;
¹H NMR(DMSO-d₆, 400 MHz) δ 2.11-2.15 (2H, m), 2.75 (2H, t, J = 6.3 Hz), 3.17 (2H, brs), 3.26 (2H, brs), 3.53-3.56 (2H, m), 3.75-3.77 (2H, m), 4.11 (2H, t, J = 6.3 Hz), 6.19 (1H, d, J = 2.7 Hz), 6.45 (1H, dd, J = 2.7, 9.0 Hz), 6.97 (2H, brs), 7.07 (1H, d, J = 5.4 Hz), 7.08 (2H, brs), 7.56 (1H, d, J = 9.0 Hz), 8.38 (1H, d, J = 5.4 Hz), 10.63 (1H, s);
HRMS m/z calcd for C₂₂H₂₅O₃N₆S 453.1709, found 453.1718;
MS (FAB) m/z: 453 [M+H]⁺, 452, 435, 418, 273, 242, 176, 65.

### (Example 141) 3-amino-6-methyl-4-(4-phenyl-1,4-diazepan-1-yl)thieno[2,3-b]pyridine-2-carboxamide (Exemplified Compound No. 3-161)

### (141a) [1-(4-phenyl-1,4-diazepan-1-yl)ethylidene]malononitrile

The reaction was performed following a method described in Example 75 (75b) using 1-phenyl homopiperazine in place of 1-phenylpiperazine and the title compound was obtained.
White powder
Mp 113-114°C;
IR (KBr) νₘₐₓ 2957, 2204, 1598, 1562, 1504, 1464, 1356, 928, 754 cm⁻¹;
¹H NMR(DMSO-d₆, 400 MHz) δ 1.94 (2H, brs), 2.22 (3H, brs), 3.57 (2H, t, J = 5.5 Hz), 3.61-4.02 (6H, m), 6.62 (1H, t, J = 7.0 Hz), 6.78 (2H, d, J = 8.2 Hz), 7.16 (2H, dd, J = 7.0, 9.0 Hz);
HRMS m/z calcd for C₁₆H₁₈N₄ 266.1533, found 266.1525;
MS (EI) m/z: 266 [M⁺], 265, 237, 210, 201, 184, 160, 146, 132, 120, 106, 91, 77, 42, 41;
Anal. Calcd for C₁₆H₁₈N₄·0.1H₂O: C, 71.67; H, 6.84; N, 20.89. Found: C, 71.55; H, 6.76; N, 21.00.

### (141b)[(2E)-3-(dimethylamino)-1-(4-phenyl-1,4-diazepan-1-yl)but-2-enylidene]malononitrile

The reaction was performed following a method described in Example 75 (75c) using [1-(4-phenyl-1,4-diazepan-1-yl)ethylidene]malononitrile which was produced in Example 141 (141a) and the title compound was obtained.
Pale brown powder
Mp 236-238°C (dec.);
IR (film) νₘₐₓ 2933, 2195, 1722, 1645, 1505, 1440, 1026, 752 cm⁻¹;
¹H NMR(CDCl₃, 400 MHz) δ 2.01-2.07 (2H, m), 2.19 (3H, s), 2.97 (6H, s), 3.57 (2H, t, J = 5.9 Hz), 3.63 (2H, t, J = 5.9 Hz), 3.71 (2H, t, J = 5.1 Hz), 3.83 (2H, t, J = 5.9 Hz), 4.30 (1H, s), 6.70-6.73 (3H, m), 7.22 (2H, d, J = 8.6 Hz);
HRMS m/z calcd for C₂₀H₂₅N₅ 335.2109, found 335.2122;
MS (EI) m/z: 335 [M⁺], 320, 292, 291, 229, 203, 168, 159, 120, 91, 85, 72, 56;
Anal. Calcd for C₂₀H₂₅N₅: C, 71.61; H, 7.51; N, 20.88. Found: C, 71.85; H, 7.56; N, 20.57.

### (141c) 6-methyl-2-oxo-4-(4-phenyl-1,4-diazepan-1-yl)-1,2-dihydropyridine-3-carbonitrile

The reaction was performed following a method described in Example 75 (75d) using [(2E)-3-(dimethylamino)-1-(4-phenyl-1,4-diazepan-1-yl)but-2-enylidene]malononitrile which was produced in Example 141 (141b) and the title compound was obtained.
White powder
Mp 132-133°C;
IR (KBr) νₘₐₓ 2957, 2199, 1625, 1503, 1459, 1351, 1216, 931, 751, 692 cm⁻¹;
¹H NMR(DMSO-d₆, 500 MHz) δ 1.91-1.95 (2H, m), 2.08 (3H, s), 3.51 (2H, t, J = 6.3 Hz), 3.65-3.70 (4H, m), 3.91 (2H, t, J = 5.4 Hz), 5.88 (1H, s), 6.60 (1H, t, J = 7.3 Hz), 6.78 (2H, d, J = 7.3 Hz), 7.16 (2H, t, J = 7.3 Hz), 11.20 (1H, brs);
HRMS m/z calcd for C₁₈H₂₁ON₄ 309.1715, found 309.1713;
MS (FAB) m/z: 309, 273, 258, 246, 226, 216, 202, 189, 176, 165, 120, 65.

### (141 d) 2-chloro-6-methyl-4-(4-phenyl-1,4-diazepan-1-yl)nicotinonitrile

The reaction was performed following a method described in Example 75 (75e) using 6-methyl-2-oxo-4-(4-phenyl-1,4-diazepan-1-yl)-1,2-dihydropyridine-3-carbonitrile which was produced in Example 141 (141c) and the title compound was obtained.
White powder
Mp 132-133°C;
IR (KBr) νₘₐₓ 3438, 2211, 1593, 1504, 1355, 1220, 1040, 929, 750 cm⁻¹;
¹H NMR(CDCl₃, 400 MHz) δ 2.14 (2H, quint, J = 5.9 Hz), 2.41 (3H, s), 3.57 (2H, t, J = 6.3 Hz), 3.71 (2H, t, J = 5.9 Hz), 3.78 (2H, t, J = 4.9 Hz), 3.98 (2H, t, J = 3.9 Hz), 6.42 (1H, s), 6.72-6.75 (3H, m), 7.24 (2H, d, J = 7.3 Hz);
HRMS m/z calcd for C₁₈H₂₀N₄Cl 327.1377, found 327.1371;
MS (FAB) m/z: 327 [M+H]⁺, 326, 273, 242, 226, 165, 120, 65.

### (141e) 6-methyl-4-(4-phenyl-1,4-diazepan-1-yl)-2-thioxo-1,2-dihydropyridine-3-carbonitrile

An ethanol (200 mL) suspension of 2-chloro-6-methyl-4-(4-phenyl-1,4-diazepan-1-yl)nicotinonitrile (6.67 g, 20.4 mmol) which was produced in Example 141 (141d) and thiourea (7.77 g, 102.1 mmol) was heated under reflux for three hours. The reaction liquid was cooled to room temperature and the generated crystal was filtered and the title compound was obtained (5.91 g, yield 89%).
White powder
Mp 250-253°C;
IR (KBr) νₘₐₓ 2962, 2204, 1628, 1598, 1547, 1504, 1351, 1207, 926, 750 cm⁻¹;
¹H NMR(DMSO-d₆, 400 MHz) δ 1.93-1.98 (2H, m), 2.19 (3H, s), 3.53 (2H, t, J = 5.5 Hz), 3.70-3.74 (4H, m), 3.94 (2H, t, J = 5.9 Hz), 6.33 (1H, s), 6.60 (1H, t, J = 8.2 Hz), 6.78 (2H, d, J = 8.2 Hz), 7.16 (2H, t, J = 8.2 Hz), 12.49 (1H, brs);
HRMS m/z calcd for C₁₈H₂₂N₄S 325.1487, found 325.1501;
MS (FAB) m/z: 325 [M+H]⁺, 324, 273, 192, 178, 165, 65, 51, 39.

### (141f) 3-amino-6-methyl-4-(4-phenyl-1,4-diazepan-1-yl)thieno[2,3-b]pyridine-2-carboxamide

The reaction was performed following a method described in Example 5 (5c) using 6-methyl-4-(4-phenyl-1,4-diazepan-1-yl)-2-thioxo-1,2-dihydropyridine-3-carbonitrile which was produced in Example 141 (141e) and the title compound was synthesized.
Pale yellow powder
Mp 283-286°C;
IR (KBr) νₘₐₓ 3441, 3323, 1645, 1596, 1504, 1367, 1203, 749, 692 cm⁻¹;
¹H NMR(DMSO-d₆, 500 MHz) δ 2.12-2.14 (2H, m), 2.45 (3H, s), 3.17 (2H, brs), 3.27-3.31 (2H, m), 3.55 (2H, t, J = 6.4 Hz), 3.77 (2H, t, J = 4.4 Hz), 6.61 (1H, t, J = 7.3 Hz), 6.77 (2H, d, J = 7.3 Hz), 6.95 (1H, s), 6.96 (2H, brs), 7.00 (2H, brs), 7.17 (2H, t, J = 7.3 Hz);
HRMS m/z calcd for C₂₀H₂₄ON₅S 382.1701, found 382.1696;
MS (EI) m/z: 382 [M+H]⁺, 365, 363, 325, 323, 244, 232, 218, 158, 146, 120, 106, 77, 75, 57, 45;
Anal. Calcd for C₂₀H₂₃N₅OS·0.5H₂O: C, 61.51; H, 6.19; N, 17.93; S, 8.21. Found: C, 61.35; H, 5.85; N, 17.92; S, 8.14.

### (Example 142) 3-amino-4-(4-{4-[(dimethylamino)carbonyl]phenyl}-1,4-diazepan-1-yl)-6-methylthieno[2,3-b]pyridine-2-carboxamide (Exemplified Compound No. 3-182)

### (142a) 4-[4-(2,2-dicyano-1-methylvinyl)-1,4-diazepan-1-yl]-N,N-dimethylbenzamide

The reaction was performed following a method described in Example 75 (75b) using 4-(1,4-diazepan-1-yl)-N,N-dimethylbenzamide which was produced in Example 128 (128d) in place of 1-phenylpiperazine and the title compound was obtained.
Colourless liquid
IR (film) νₘₐₓ 3439, 2935, 2206, 1608, 1562, 1493, 1396, 1190 cm⁻¹;
¹H NMR(CDCl₃, 400 MHz) δ 2.11 (2H, brs), 2.31 (3H, s), 3.07 (6H, s), 3.55-3.97 (8H, m), 6.68 (2H, d, J = 8.6 Hz), 7.38 (2H, d, J = 8.6 Hz);
MS (EI) m/z: 337 [M⁺], 293, 266, 265, 203, 174, 160, 146, 132, 118, 104, 77, 72, 40.

### (142b) 4-{4-[(2E)-1-(dicyanomethylene)-3-(dimethylamino)but-2-en-1-yl]-1,4-diazepan-1-yl} -N,N-dimethylbenzamide

The reaction was performed following a method described in Example 75 (75c) using 4-[4-(2,2-dicyano-1-methylvinyl)-1,4-diazepan-1-yl]-N,N-dimethylbenzamide which was produced in Example 142 (142a) and the title compound was obtained.
Yellow foam
IR (film) νₘₐₓ 2926, 2194, 1607, 1561, 1493, 1440, 1390, 1187, 1025, 763, 554 cm⁻¹; ¹H NMR(CDCl₃, 500 MHz) δ 2.03-2.06 (2H, m), 2.20 (3H, s), 2.99 (6H, s), 3.07 (6H, s), 3.60-3.63 (4H, m), 3.75-3.78 (2H, m), 3.83-3.84 (2H, m), 4.31 (1H, s), 6.69 (2H, d, J = 8.8 Hz), 7.38 (2H, d, J = 8.8 Hz);
MS (FAB) m/z: 407 [M+H]⁺, 406, 362, 273, 242, 203, 165, 39, 31.

### (142c) 4-[4-(3-cyano-6-methyl-2-oxo-1,2-dihydropyridin-4-yl)-1,4-diazepan-1-yl]-N,N-dimethylbenzamide

The reaction was performed following a method described in Example 75 (75d) using 4-{4-[(2E)-1-(dicyanomethylene)-3-(dimethylamino)but-2-en-1-yl]-1,4-diazepan-1-yl}-N,N-dimethylbenzamide which was produced in Example 142 (142b) and the title compound was obtained.
Brown powder
Mp 220-223°C;
IR (KBr) νₘₐₓ 3434, 2939, 2199, 1621, 1526, 1494, 1454, 1391, 1355, 1189 cm⁻¹;
¹H NMR(DMSO-d₆, 500 MHz) δ 1.90-1.96 (2H, m), 2.08 (3H, s), 2.95 (6H, s), 3.55-3.57 (2H, m), 3.67-3.69 (2H, m), 3.72-3.74 (2H, m), 3.91-3.93 (2H, m), 5.89 (1H, s), 6.68 (2H, d, J = 8.8 Hz), 7.27 (2H, d, J = 8.8 Hz), 11.20 (1H, brs);
MS (FAB) m/z: 380 [M+H]⁺, 349, 335, 273, 258, 242, 93, 65.

### (142d) 4-[4-(2-chloro-3-cyano-6-methylpyridin-4-yl)-1,4-diazepan-1-yl]-N,N-dimethylbenzamide

The reaction was performed following a method described in Example 75 (75e) using 4-[4-(3-cyano-6-methyl-2-oxo-1,2-dihydropyridin-4-yl)-1,4-diazepan-1-yl]-N,N-dimethylbenzamide which was produced in Example 142 (142c) and the title compound was obtained.
White powder
Mp 199-201°C;
IR (KBr) νₘₐₓ 2946, 2210, 1618, 1590, 1515, 1392, 1219, 1189, 1038, 926, 830, 763 cm⁻¹;
¹H NMR(CDCl₃, 500 MHz) δ 2.14 (2H, quint, J = 5.9 Hz), 2.42 (3H, s), 3.07 (6H, s), 3.60 (2H, t, J = 5.9 Hz), 3.69 (2H, t, J = 5.9 Hz), 3.82 (2H, t, J = 4.9 Hz), 3.98 (2H, t, J = 4.9 Hz), 6.43 (1H, s), 6.70 (2H, d, J = 8.8 Hz), 7.38 (2H, d, J = 8.8 Hz);
MS (FAB) m/z: 398 [M+H]⁺, 397, 353, 246, 242, 182.

### (142e) 4-[4-(3-cyano-6-methyl-2-thioxo-1,2-dihydropyridin-4-yl)-1,4-diazepan-1-yl]-N,N-dimethylbenzamide

The reaction was performed following a method described in Example 141 (141e) using 4-[4-(2-chloro-3-cyano-6-methylpyridin-4-yl)-1,4-diazepan-1-yl]-N,N-dimethylbenzamide which was produced in Example 142 (142d) and the title compound was obtained.
White powder
Mp 175-178°C;
IR (KBr) νₘₐₓ 3205, 2969, 2201, 1607, 1551, 1485, 1352, 1185, 929 cm⁻¹;
¹H NMR(DMSO-d₆, 400 MHz) δ 1.92-1.97 (2H, m), 2.19 (3H, s), 2.95 (6H, s), 3.56-5.58 (2H, m), 3.72-3.77 (4H, m), 3.95-3.96 (2H, m), 6.34 (1H, s), 6.79 (2H, d, J = 8.6 Hz), 7.27 (2H, d, J = 8.6 Hz), 12.51 (1H, brs);
MS (FAB) m/z: 396 [M+H]⁺, 367, 351, 335, 273, 246, 242, 165.

### (142f) 3-amino-4-(4-{4-[(dimethylamino)carbonyl)phenyl}-1,4-diazepan-1-yl)-6-methylthieno[2,3-b]pyridine-2-carboxamide

The reaction was performed following a method described in Example 5 (5c) using 4-[4-(3-cyano-6-methyl-2-thioxo-1,2-dihydropyridin-4-yl)-1,4-diazepan-1-yl]-N,N-dimethylbenzamide which was produced in Example 142 (142e) and the title compound was synthesized.
White powder
Mp 158-162°C;
IR (KBr) νₘₐₓ 3436, 3322, 3182, 1607, 1492, 1371, 1189 cm⁻¹;
¹H NMR(DMSO-d₆, 500 MHz) δ 2.14-2.16 (2H, m), 2.45 (3H, s), 2.97 (6H, s), 3.16 (2H, brs), 3.28 (2H, brs), 3.59 (2H, t, J = 5.9 Hz), 3.82 (2H, t, J = 4.9 Hz), 6.78 (2H, d, J = 8.8 Hz), 6.96 (1H, s), 6.96 (2H, brs), 7.02 (2H, brs), 7.30 (2H, d, J = 8.8 Hz); HRMS m/z calcd for C₂₃H₂₉O₂N₆S 453.2027, found 453.2044;
MS (ESI) m/z: 453 [M+H]⁺, 440;
Anal. Calcd for C₂₃H₂₈N₆O₂S·4.32H₂O: C, 52.08; H, 6.96; N, 15.84; S, 6.05. Found: C, 51.75; H, 6.72; N, 15.84; S, 5.91.

### (Example 143) 3-amino-4-(3-methoxypiperidin-1-yl)thieno[2,3-b]pyridine-2-carboxamide (Exemplified Compound No. 1-28)

### (143a) 4-(3-methoxypiperidin-1-yl)-2-thioxo-1,2-dihydropyridine-3-carbonitrile

The reaction was performed following a method described in Example 118 (118c) using 3-methoxy piperidine (J. Med.Chem. 8,766, (1965)) and the title compound was obtained.
Brown powder
Mp 148-152°C;
IR (KBr) νₘₐₓ 2943, 2207, 1622, 1515, 1303, 1250, 1167, 1097, 985, 778 cm⁻¹;
¹H NMR(DMSO-d₆, 400 MHz) δ 1.47-1.62 (2H, m), 1.76-1.83 (1H, m), 1.87-1.93 (1H, m), 3.25 (3H, s), 3.35-3.40 (1H, m), 3.44-3.52 (2H, m), 3.58-3.63 (1H, m), 3.86 (2H, dd, J = 2.4, 13.3 Hz), 6.48 (1H, d, J = 7.4 Hz), 7.44 (1H, d, J = 7.4 Hz), 12.60 (1H, brs);
HRMS m/z calcd for C₁₂H₁₅ON₃S 249.0935, found 249.0919;
MS (FAB) m/z: 250 [M+H]⁺, 218, 180, 39.

### (143b) 3-amino-4-(3-methoxypiperidin-1-yl)thieno[2,3-b]pyridine-2-carboxamide

The reaction was performed following a method described in Example 5 (5c) using 4-(3-methoxypiperidin-1-yl)-2-thioxo-1,2-dihydropyridine-3-carbonitrile which was produced in Example 143 (143a) and the title compound was obtained. Pale yellow powder
Mp 182-185°C;
IR (KBr) νₘₐₓ 3412, 3321, 3146, 2936, 1660, 1585, 1502, 1373, 1246, 1099, 977 cm⁻¹; ¹H NMR(DMSO-d₆, 500 MHz) δ 1.49-2.09 (4H, m), 2.81-3.46 (4H, m), 3.32 (3H, s), 3.54-3.58 (1H, m), 6.99 (2H, brs), 7.05 (1H, d, J = 5.4 Hz), 7.09 (2H, brs), 8.44 (1H, d, J = 5.4 Hz);
HRMS m/z calcd for C₁₄H₁₈O₂N₄S 306.1151, found 306.1154;
MS (EI) m/z: 306 [M⁺], 274, 256, 229, 218, 202, 176, 175, 148, 147, 105, 104, 58, 41;
Anal. Calcd for C₁₄H₁₈N₄O₂S: C, 54.88; H, 5.92; N, 18.29; S, 10.47. Found: C, 54.81; H, 5.71; N, 18.11, 10.47.

### (Example 144) 3-amino-4-[3-(ethoxymethyl)piperidin-1-yl]thieno[2,3-b]pyridine-2-carboxamide (Exemplified Compound No. 1-22)

### (144a) tert-butyl 3-(ethoxymethyl)piperidine-1-carboxylate

The reaction was performed following a method described in Example 101 (101 a) using ethyl iodide in place of methyl iodide and the title compound was obtained as an oil. Yield 82%.
IR (neat) νₘₐₓ 1697, 1423, 1152 cm⁻¹;
¹H NMR (CDCl₃, 400MHz) δ 1.16-1.27 (1H, m), 1.19 (3H, t, J = 7.0 Hz), 1.38-1.50 (1H, m), 1.46 (9H, s), 1.60-1.66 (1H, m), 1.72-1.83 (2H, m), 2.48-2.91 (2H, m), 3.28 (2H, d, J = 6.3 Hz), 3.41-3.51 (2H, m), 3.80-4.15 (2H, m);
MS (EI) m/z: 243 [M⁺], 114, 57, 41.

### (144b) 3-(ethoxymethyl)piperidine

4N hydrochloric acid-1,4-dioxane solution (8 mL) was added to a methanol (4 mL) solution of tert-butyl 3-(ethoxymethyl)piperidine-1-carboxylate (2.00 g, 8.2 mmol) which was produced in Example 144 (144a). The reaction solution was concentrated under reduced pressure after being stirred at room temperature for two hours. The obtained residue was treated with 1N aqueous solution of sodium hydroxide (20 mL) and the aqueous layer was extracted with methylene chloride (3x30 mL). The extract was dried over sodium sulfate, the solvent was evaporated under reduced pressure and 1.01 g of the title compound was obtained (yield 86%) as an oil.
IR (neat) νₘₐₓ 3310, 1625, 1553, 1270, 1113 cm⁻¹;
¹H NMR (CDCl₃, 400MHz) δ 1.04-1.15 (1H, m), 1.18 (3H, t, J = 7.0 Hz), 1.39-1.50 (1H, m), 1.60 (1H, br), 1.59-1.83 (3H, m), 2.32 (1H, dd, J = 10.2, 12.1 Hz), 2.55 (1H, dt, J = 3.1, 11.7 Hz), 2.99 (1H, brd, J = 12.1 Hz), 3.12 (1H, brd, J = 11.7 Hz), 3.23 (2H, d, J = 6.3 Hz), 3.39-3.50 (2H, m);
MS (EI) m/z: 143 [M⁺], 114, 44.

### (144c) (2Z)-2-cyano-3-[3-(ethoxymethyl)piperidin-1-yl]but-2-enethioamide

The reaction was performed following a method described in Example 5 (5a) using 3-(ethoxymethyl)piperidine which was produced in Example 144 (144b) in place of isobutylamine and the title compound was obtained. Yield 86%.
Mp 146-148°C;
IR(KBr) νₘₐₓ 3335, 3277, 3142, 2189, 1627, 1542, 1396, 1110 cm⁻¹;
¹H NMR (DMSO-d₆, 400MHz) δ 1.10 (3H, t, J = 7.0 Hz), 1.26-1.93 (5H, m), 2.27 (3H, s), 2.93 (1H, dd, J = 10.2, 13.7 Hz), 3.05-3.12 (1H, m), 3.20-3.30 (2H, m), 3.34-3.46 (2H, m), 3.51-3.62 (2H, m), 8.19 (1H, br), 8.93 (1H, br);
MS (EI) m/z: 267 [M⁺], 234, 204;
Anal. Calcd for C₁₃H₂₁N₃SO: C, 58.39; H, 7.92; N, 15.71; S, 11.99. Found: C, 58.40; H, 7.99; N, 15.60; S, 12.06.

### (144d) 4-[3-(ethoxymethyl)piperidin-1-yl]-2-thioxo-1,2-dihydropyridine-3-carbonitrile

The reaction was performed following a method described in Example 5 (5b) using (2Z)-2-cyano-3-[3-(ethoxymethyl)piperidin-1-yl]but-2-enethioamide which was produced in Example 144 (144c) and a crude crystal of the title compound was obtained.
Mp 151-154°C;
¹H NMR (DMSO-d₆, 400MHz) δ 1.10 (3H, t, J = 7.0 Hz), 1.26-1.90 (5H, m), 3.08 (1H, dd, J = 10.2, 13.3 Hz), 3.19-3.45 (5H, m), 3.91-3.99 (2H, m), 6.45 (1H, d, J = 7.4 Hz), 7.42-7.45 (1H, m), 12.59 (1H, br).

### (144e) 3-amino-4-[3-(methoxymethyl)piperidin-1-yl]thieno[2,3-b]pyridine-2-carboxamide

The reaction was performed following a method described in Example 5 (5c) using a crude crystal of 4-[3-(ethoxymethyl)piperidin-1-yl]-2-thioxo-1,2-dihydropyridine-3-carbonitrile which was produced in Example 144 (144d) and the title compound was obtained. Yield 34% from (2Z)-2-cyano-3-[3-(ethoxymethyl)piperidin-1-yl]but-2-enethioamide.
Mp 125-126°C;
IR (KBr) νₘₐₓ 3439, 3323, 3174, 1650, 1580, 1501, 1372, 1106 cm⁻¹;
¹H NMR (DMSO-d₆, 400MHz) δ 1.01-1.17 (1H, m), 1.08 (3H, t, J = 7.0 Hz), 1.73-1.85 (3H, m), 2.05-2.16 (1H, m), 2.34-2.70 (2H, m), 3.21-3.46 (6H, m), 6.92 (2H, br), 7.00 (1H, d, J = 5.5 Hz), 7.09 (2H, br), 8.41 (1H, d, J = 5.5 Hz);
MS (FAB) m/z: 335 [M + H]⁺;
Anal. Calcd for C₁₆H₂₂N₄SO₂·0.6 H₂O: C, 55.66; H, 6.77; N, 16.23; S, 9.29. Found: C, 55.44; H, 6.63; N, 16.33; S, 9.41.

### (Example 145) 3-amino-4-{3-[(2-methoxyethoxy)methyl]piperidin-1-yl}thieno[2,3-b]pyridine-2-carboxamide (Exemplified Compound No. 1-112)

### (145a) tert-butyl 3-[(2-methoxyethoxy)methyl]piperidine-1-carboxylate

A toluene (2 mL) solution of 3-(hydroxymethyl)piperidine-1-carboxylate (Bioorg. Med. Chem. Lett. 8, (1998), 1595-1600) (215 mg, 1 mmol) and 1-bromo-2-methoxyethane (278 mg, 0.19 mL, 2 mmol) was blended with tetra-n-butylammonium bisulfate (68 mg, 0.2 mmol) and 50% aqueous solution of sodium hydroxide (1 mL) and the resultant mixture was stirred at room temperature for 20 hours. The reaction mixture was partitioned with water (30 mL) and ethyl acetate (50 mL) and the organic layer was dried over sodium sulfate after being washed with a saturated saline solution (30 mL), and the solvent was evaporated under reduced pressure. The obtained residue was purified by silica gel chromatography (hexane/ethyl acetate, 2:1) and 177 mg of the title compound was obtained (65%) as an oil.
IR (neat) νₘₐₓ 1695, 1423, 1152 cm⁻¹;
¹H NMR (CDCl₃, 400MHz) δ 1.14-1.24 (1H, m), 1.38-1.49 (1H, m), 1.45 (9H, s), 1.59-1.66 (1H, m), 1.76-1.85 (2H, m), 2.52-2.85 (2H, m), 3.32 (2H, d, J = 5.9 Hz), 3.38 (3H, s), 3.50-3.58 (4H, m), 3.82-4.04 (2H, m);
MS (EI) m/z: 273 [M⁺], 172, 114, 57;
Anal. Calcd for C₁₄H₂₇NO₄·0.1 H₂O: C, 61.11; H, 9.96; N, 5.09. Found: C, 61.01; H, 9.57; N, 5.13.

### (145b)3-[(2-methoxyethoxy)methyl]piperidine

A methanol (2 mL) solution of tert-butyl 3-[(2-methoxyethoxy)methyl]piperidine-1-carboxylate (0.87 g, 3.2 mmol) which was produced in Example 145 (145a) was blended with 4N hydrochloric acid-1,4-dioxane solution (3 mL) and the resultant solution was stirred at room temperature for two hours. 1N aqueous solution of sodium hydroxide (10 mL) was added to the residue which was obtained by concentrating the reaction mixture under reduced pressure and the aqueous layer was extracted with methylene chloride (3x30 mL). After the extract was dried over sodium sulfate, the solvent was evaporated under reduced pressure and 0.51 g of the title compound was obtained (92%) as an oil.
IR (neat) νₘₐₓ 3413, 1626, 1271, 1111 cm⁻¹;
¹H NMR (CDCl₃, 400MHz) δ 1.05-1.15 (1H, m), 1.40-1.51 (1H, m), 1.62-1.86 (3H, m), 1.76 (1H, br), 2.33 (1H, dd, J = 9.8, 12.1 Hz), 2.56 (1H, ddd, J = 2.7, 11.7, 12.1 Hz), 3.00 (1H, brd, J = 12.1 Hz), 3.14 (1H, brd, J = 11.7 Hz), 3.3 (2H, d, J = 6.3 Hz), 3.39 (3H, s), 3.48-3.60 (4H, m);
MS (EI) m/z: 173 [M⁺], 114, 44;
Anal. Calcd for C₉H₁₉NO₂·0.2 H₂O: C, 61.12; H, 11.06; N, 7.92. Found: C, 61.15; H, 10.67; N, 7.86.

### (145c) 3-amino-4-{3-[(2-methoxyethoxy)methyl]piperidin-1-yl}thieno[2,3-b]pyridine-2-carboxamide

3-[(2-methoxyethoxy)methyl]piperidine (0.50 g, 2.9 mmol) which was produced in Example 145 (145b) and (2Z)-2-cyano-3-ethoxybut-2-enethioamide (J.Org.Chem. (1962), 27,2433-2439) (0.41 g, 2.4 mmol) were mixed with ethanol (3 mL) and the resultant mixture was stirred at room temperature for one hour. The residue obtained by concentrating the reaction mixture under reduced pressure was dissolved in toluene (8 mL) and was blended with N,N-dimethylformamide dimethylacetal (0.86 g, 0.96 mL, 7.2 mmol) and the resultant mixture was stirred under reflux for 30 minutes. After the solvent was evaporated under reduced pressure, 1N aqueous solution of sodium hydroxide (8 mL) was added to the residue and the mixture was stirred and heated under reflux for one hour. After cooling, the reaction mixture was partitioned with water and ether. The obtained aqueous layer was neutralized with 1N hydrochloric acid (8 mL) and extracted with ethyl acetate (2x50 mL). The solvent was evaporated under reduced pressure after the extract was dried over sodium sulfate. The residue was dissolved in N,N-dimethylformamide (5 mL) and the resultant solution was blended with 2-chloroacetamide (0.15 g, 1.6 mmol) and 8N aqueous solution of sodium hydroxide (0.5 mL) and the mixture was stirred at room temperature for one hour. The reaction mixture was blended with water (50 mL) and extracted with ethyl acetate (2x50 mL). After the extract was dried over sodium sulfate, the residue which was obtained by evaporating the solvent under reduced pressure was purified by silica gel column chromatography (100% ethyl acetate) and 0.25 g of the title compound was obtained (yield 28%).
Mp 138-139°C;
IR (KBr) vₘₐₓ 3433, 3323, 3178, 1651, 1579, 1501, 1372, 1088 cm⁻¹;
¹H NMR (DMSO-d₆, 400MHz) δ 1.00-1.15 (1H, m), 1.73-1.83 (3H, m), 2.07-2.16 (1H, m), 2.31-2.70 (2H, m), 3.21 (3H, s), 3.25-3.53 (8H, m), 6.92 (2H, br), 7.00 (1H, d, J = 5.5 Hz), 7.09 (2H, br), 8.41 (1H, d, J = 5.5 Hz);
MS (FAB) m/z: 365 [M + H]⁺.

### (Example 146) 3-amino-4-{3-[(2-ethoxyethoxy)methyl]piperidin-1-yl}thieno[2,3-b]pyridine-2-carboxamide (Exemplified Compound No. 1-123)

### (146a) tert-butyl 3-[(2-ethoxyethoxy)methyl]piperidine-1-carboxylate

The reaction was performed following a method described in Example 101 (101a) using 2-bromoethyl ethyl ether in place of methyl iodide and the title compound was obtained as an oil. Yield 56%.
IR (neat) νₘₐₓ 1696, 1423, 1152 cm⁻¹;
¹H NMR (CDCl₃, 400MHz) δ 1.14-1.25 (1H, m), 1.21 (3H, t, J = 7.0 Hz), 1.37-1.51 (1H, m), 1.45 (9H, s), 1.60-1.67 (1H, m), 1.75-1.85 (2H, m), 2.50-2.86 (2H, m), 3.30-3.37 (2H, m), 3.54 (2H, q, J = 7.0 Hz), 3.50-3.67 (4H, m), 3.84-4.08 (2H, m); MS (FAB) m/z: 288 [M + H]⁺.

### (146b) 3-[(2-methoxyethoxy)methyl]piperidine

The reaction was performed following a method described in Example 144 (144b) using tert-butyl 3-[(2-ethoxyethoxy)methyl]piperidine-1-carboxylate which was produced in Example 146 (146a) and the title compound was obtained as an oil.
Yield 92%.
IR (neat) νₘₐₓ 3410, 1640, 1273, 1114 cm⁻¹;
¹H NMR (CDCl₃, 400MHz) δ 1.05-1.15 (1H, m), 1.21 (3H, t, J = 7.0 Hz), 1.40-1.51 (1H, m), 1.62-1.85 (3H, m), 1.75 (1H, br), 2.33 (1H, dd, J = 10.0, 12.0 Hz), 2.56 (1H, dt, J = 3.0, 12.0 Hz), 3.01 (1H, brd, J = 12.0 Hz), 3.15 (1H, brd, J = 12.0 Hz), 3.27-3.83 (2H, m), 3.54 (2H, q, J = 7.0 Hz), 3.51-3.62 (4H, m);
MS (EI) m/z: 187 [M⁺], 114, 44.

### (146c) 4-{3-[(2-ethoxyethoxy)methyl]piperidin-1-yl}-2-thioxo-1,2-dihydropyridine-3-carbonitrile

3-[(2-ethoxyethoxy)methyl]piperidine (0.48 g, 2.6 mmol) which was produced in Example 146 (146b) and (2Z)-2-cyano-3-ethoxybut-2-enethioamide (J. Org. Chem. (1962), 27,2433-2439) (0.40 g, 2.3 mmol) were mixed with ethanol (5 mL) and the resultant mixture was stirred at room temperature for two hours. The residue obtained by concentrating the reaction mixture under reduced pressure was dissolved in toluene (5 mL) and the mixture was blended with N,N-dimethylformamide dimethylacetal (0.82 g, 0.92 mL, 6.9 mmol) and the mixture was stirred under reflux for 30 minutes. After the solvent was evaporated under reduced pressure, 1N aqueous solution of sodium hydroxide (5 mL) was added to the residue and the mixture was stirred under heat reflux for one hour. After cooling the mixture, the reaction mixture was partitioned with water (10 mL) and ether (50 mL). The obtained aqueous layer was neutralized with 1N hydrochloric acid (5 mL) and extracted with ethyl acetate (2x50 mL). After the extract was dried over sodium sulfate, the solvent was evaporated under reduced pressure. The residue was purified by silica gel column chromatography (methylene chloride/methanol =30:1) and 0.10 g of the title compound was obtained (yield 13%).
Mp 119-123°C;
IR (KBr) νₘₐₓ 2213, 1625, 1552, 1525, 1250, 1120, 1111 cm⁻¹;
¹H NMR (DMSO-d₆, 400MHz) δ 1.09 (3H, t, J = 7.0 Hz), 1.26-1.94 (5H, m), 3.09 (1H, dd, J = 9.8, 13.3 Hz), 3.20-3.52 (9H, m), 3.92-4.00 (2H, m), 6.47 (1H, d, J = 7.4 Hz), 7.43-7.47 (1H, m), 12.63 (1H, br);
MS (FAB) m/z: 322 [M + H]⁺;
Anal. Calcd for C₁₆H₂₃N₃SO₂: C, 59.78; H, 7.21; N, 13.07; S, 9.98. Found: C, 59.72; H, 7.10; N, 13.09; S, 9.87.

### (146d) 3-amino-4-{3-[(2-ethoxyethoxy)methyl]piperidin-1-yl}thieno[2,3-b]pyridine-2-carboxamide

4-{3-[(2-ethoxyethoxy)methyl]piperidin-1-yl}2-thioxo-1,2-dihydropyridine-3-carbonitrile (100 mg, 0.31 mmol) which was produced in Example 146 (146c) was dissolved in N,N-dimethylformamide (1 mL) and the resultant solution was blended with 2-chloroacetamide (38 mg, 0.40 mmol) and 8N aqueous solution of sodium hydroxide (0.1 mL) and the mixture was stirred at room temperature for one hour. Water (30 mL) was added to the reaction mixture and the mixture was extracted with ethyl acetate (2x50 mL). After the extract was dried over sodium sulfate, the solvent was evaporated under reduced pressure The obtained residue was recrystallized from ethanol (2 mL) and 62 mg of the title compound was obtained (yield 53%). -
Mp 117-118°C;
IR (KBr) νₘₐₓ 3434, 3323, 3180, 1650, 1579, 1501, 1371, 1118 cm⁻¹;
¹H NMR (DMSO-d₆, 400MHz) δ 1.00-1.15 (1H, m), 1.06 (3H, t, J = 7.0 Hz), 1.70-1.87 (3H, m), 2.06-2.19 (1H, m), 2.30-2.70 (2H, m), 3.41 (2H, q, J = 7.0 Hz), 3.25-3.54 (8H, m), 6.94 (2H, br), 7.02 (1H, d, J = 5.5 Hz), 7.10 (2H, br), 8.44 (1H, d, J = 5.5 Hz);
MS (FAB) m/z: 379 [M + H]⁺;
Anal. Calcd for C₁₈H₂₆N₄SO₃·0.3 H₂O: C, 56.32; H, 6.98; N, 14.59; S, 8.35. Found: C, 56.16; H, 6.69; N, 14.72; S, 8.36.

### (Example 147) 3-amino-4-[(3S)-3-(methoxymethyl)piperidin-1-yl]thieno[2,3-b]pyridine-2-carboxamide (Exemplified Compound No. 1-21)

### (147a) tert-butyl (3S)-3-(hydroxymethyl)piperidine-1-carboxylate

A tetrahydrofuran (50 mL) solution of di-tert-butyl dicarbonate (11.73 g, 53.7 mmol) was added dropwise to a tetrahydrofuran (100 mL) suspension of ethyl (S)-nipecotate D-tartrate (15.72 g, 51.2 mmol) and triethylamine (17.08 g, 23.5 mL, 168.8 mmol) under ice-cooling for 30 minutes. The reaction liquid was warmed to room temperature and further stirred for 15 hours. The filtrate which was obtained by removing insolubles by filtration was concentrated under reduced pressure. The residue was dissolved in ethyl acetate (200 mL) and washed sequentially with water (50 mL), 1N hydrochloric acid (50 mL) and saturated sodium bicarbonate aqueous solution (50 mL). After the organic layer was dried over sodium sulfate, the residue which was obtained by evaporating the solvent under reduced pressure was dissolved in tetrahydrofuran (50 mL). This solution was dripped to a tetrahydrofuran (150 mL) suspension of lithium aluminum hydride (1.94 g, 51.2 mmol) under ice-cooling for 30 minutes. After the dropwise addition was completed, the reaction liquid was warmed to room temperature and further stirred at room temperature for one hour. The reaction container was transferred onto an ice bath and a little amount of ethyl acetate and saturated ammonium chloride aqueous solution were added to the reaction liquid and the resultant liquid was stirred at room temperature further for one hour. Ethyl acetate was added to the mixture and insolubles were removed by decantation (3x100 mL). The obtained organic layer was concentrated and the residue was blended with water (100 mL) and extracted with ethyl acetate (2x100 mL). After the extract was dried over sodium sulfate, a crude crystal (10.77 g) which was obtained by evaporating the solvent under reduced pressure was recrystallized from a mixed solvent of ethyl acetate and hexane (1:5) and 9.03 g of the title compound was obtained (yield 82%).
Mp 94-97°C;
IR (KBr) νₘₐₓ 3489, 3466, 1674, 1434, 1154 cm⁻¹;
¹H NMR (CDCl₃, 400MHz) δ 1.20-1.35 (1H, m), 1.35-1.50 (1H, m), 1.46 (9H, s), 1.58-1.83 (3H, m), 2.12 (1H, br), 2.75-3.24 (2H, m), 3.50 (2H, brs), 3.40-3.90 (2H, m);
MS (EI) m/z: 215 [M⁺], 57.
Anal. Calcd for C₁₁H₂₁NO₃: C, 61.37; H, 9.83; N, 6.51. Found: C, 61.30; H, 9.75; N, 6.48;
[α]²⁰_{D}: +18.6° (C = 1.11, EtOH).

### (147b) (3S)-3-(methoxymethyl)piperidine hydrochloride

After washing in hexane (3x10 mL), sodium hydride (55% oily, 3.89 g, 89 mmol) was suspended in N,N-dimethylformamide (80 mL) and methyl iodide (14.47 g, 6.35 mL, 102 mmol) was added thereto at 0°C and subsequently a tetrahydrofuran (80 mL) solution of tert-butyl (3S)-3-(hydroxymethyl)piperidine-1-carboxylate (18.28 g, 85 mmol) which was produced in Example 147 (147a) was added dropwise for 30 minutes. The reaction mixture was warmed to room temperature and further stirred at room temperature overnight. Water (200 mL) was added to the reaction liquid and the aqueous layer was extracted with ethyl acetate (2x150 mL). The extract was washed with a saturated saline solution (50 mL) and the solvent was evaporated under reduced pressure after drying the extract over sodium sulfate. The residue was dissolved in methanol (50 mL) and 4N hydrochloric acid-1,4-dioxane solution (50 mL) were added thereto. The reaction mixture was concentrated under reduced pressure after being stirred at room temperature for two hours. Ethyl acetate (50 mL) was added to the residue and was suspended and after the crystal was filtered, it was washed with ethyl acetate and the title compound was obtained 12.45 g (88%).
Mp 197-199°C;
IR (KBr) νₘₐₓ 2944, 1591, 1450, 1129, 1097, 964 cm⁻¹;
¹H NMR (DMSO-d₆, 400MHz) δ 1.14-1.25 (1H, m), 1.59-1.78 (3H, m), 1.97-2.08 (1H, m), 2.55-2.61 (1H, m), 2.66-2.76 (1H, m), 3.22 (3H, s), 3.14-3.26 (4H, m), 8.85-9.15 (2H, br);
MS (EI) m/z: 129 [M⁺], 114;
[α]²⁰_{D}: -12.1° (C = 1.02, MeOH).

### (147c) (2Z)-2-cyano-3-[(3S)-3-(methoxymethyl)piperidin-1-yl]but-2-enethioamide

1N aqueous solution of sodium hydroxide (150 mL) was added to (3S)-3-(methoxymethyl)piperidine hydrochloride (10.76 g, 65 mmol) which was produced in Example 147 (147b) and the aqueous layer was extracted with methylene chloride (3x100 mL). The extract was dried over sodium sulfate and the solvent was evaporated under reduced pressure. The obtained crude product of (3S)-3-(methoxymethyl)piperidine (7.56 g) was dissolved in ethanol (120 mL) and (2Z)-2-cyano-3-ethoxybut-2-enethioamide (J.Org.Chem. (1962), 27,2433-2439) (9.47 g, 56 mmol) was added. After the reaction mixture was stirred at room temperature for 18 hours, the deposited crystal was filtered and washed with ethanol and the title compound was obtained (yield 84%).

Mp 149-151°C.

### (147d) 4-[(3S)-3-(methoxymethyl)piperidin-1-yl]-2-thioxo-1,2-dihydropyridine-3-carbonitrile

(2Z)-2-cyano-3-[(3S)-3-(methoxymethyl)piperidin-1-yl]but-2-enethioamide (7.83 g, 31 mmol) which was produced in Example 147 (147c) and N,N-dimethylformamide dimethylacetal (4.04 g, 4.5 mL, 34 mmol) were dissolved in N,N-dimethylformamide (60 mL) and the resultant solution was stirred at room temperature for three hours and was stirred at 60°C for a further two hours. The reaction liquid was blended with a saturated saline solution (150 mL) and the aqueous layer was extracted with methylene chloride (4x100 mL). After the extract was dried over sodium sulfate, the solvent was evaporated under reduced pressure. The residue was purified by silica gel column chromatography (ethyl acetate/methanol =15:1) and 2.42 g of the title compound was obtained (yield 30%). Mp 176-177°C.

### (147e) 3-amino-4-[(3S)-3-(methoxymethyl)piperidin-1-yl]thieno[2,3-b]pyridine-2-carboxamide

The reaction was performed following a method described in Example 5 (5c) using 4-[(3S)-3-(methoxymethyl)piperidin-1-yl]-2-thioxo-1,2-dihydropyridine-3-carbonitrile which was produced in Example 147 (147d) and the title compound was synthesized. Yield 85%.
Mp 194-196°C;
[α]²⁰D: +61.3° (C = 0.90, MeOH).

### (Example 148) 3-amino-4-{(3S)-[(2-methoxyethoxy)methyl]piperidin-1-yl}thieno[2,3-b]pyridine-2-carboxamide hydrochloride (Exemplified Compound No. 1-112)

The reaction was performed following a method described in Example 145 using tert-butyl (3S)-3-(hydroxymethyl)piperidine-1-carboxylate which was produced in Example 147 (147a).

3-amino-4- {(3S)-[(2-methoxyethoxy)methyl]piperidin-1-yl} thieno [2,3-b]pyridine-2-carboxamide (410 mg, 1.1 mmol) obtained was dissolved in ethanol (5 mL) and 1N hydrochloric acid (3 mL) was added. The residue which was obtained by concentrating the mixture under reduced pressure was washed with ethanol and 320 mg of the title compound (71 %) was obtained as a yellow solid.
Mp 183-188°C;
IR (KBr) νₘₐₓ 1647, 1608, 1104 cm⁻¹;
¹H NMR (DMSO-d₆, 400MHz) δ 1.17-1.28 (1H, m), 1.68-1.83 (3H, m), 2.02-2.12 (1H, m), 2.78-3.05 (2H, m), 3.22 (3H, s), 3.27-3.63 (8H, m), 7.11 (1H, d, J = 5.5 Hz), 7.24 (2H, br), 8.45 (1H, d, J = 5.5 Hz);
MS (FAB) m/z: 365 [M + H]⁺;
Anal. Calcd for C₁₇H₂₄N₄SO₃·HCl·0.1 H₂O: C, 50.70; H, 6.31; N, 13.91; S, 7.96; Cl, 8.80. Found: C, 50.53; H, 6.16; N, 13.81; S, 8.08; Cl, 8.96;
[α]²⁰_{D}: +82.3° (C = 1.00, MeOH).

### (Example 149) 3-amino-4-{(3S)-3-[(3-methoxypropoxy)methyl]piperidin-1-yl}thieno[2,3-b]pyridine-2-carboxamide (Exemplified Compound No. 1-115)

### (149a) tert-butyl (3S)-3-[(3-methoxypropoxy)methyl]piperidine-1-carboxylate

The reaction was performed following a method described in Example 145 (145a) using tert-butyl (3S)-3-(hydroxymethyl)piperidine-1-carboxylate which was produced in Example 147 (147a) and using 1-bromo-3-methoxypropane in place of 1-bromo-2-methoxyethane and the title compound was obtained as an oil. Yield 63%.
IR (neat) νₘₐₓ 1696, 1423, 1152, 1119 cm⁻¹;
¹H NMR (CDCl₃, 400MHz) δ 1.13-1.25 (1H, m), 1.33-1.50 (1H, m), 1.45 (9H, s), 1.59-1.67 (1H, m), 1.72-1.85 (4H, m), 2.54-2.62 (1H, m), 2.75-2.82 (1H, m), 3.22-3.29 (2H, m), 3.32 (3H, s), 3.42-3.50 (2H, m), 3.45 (2H, t, J = 6.3 Hz), 3.86-4.02 (2H, m);
MS (FAB) m/z: 288 [M+H]⁺, 232, 188;
Anal. Calcd for C₁₅H₂₉NO₄: C, 62.69; H, 10.17; N, 4.87. Found: C, 62.79; H, 9.89; N, 4.91.

### (149b) (3S)-3-[(3-methoxypropoxy)methyl]piperidine

The reaction was performed following a method described in Example 144 (144b) using tert-butyl (3S)-3-[(3-methoxypropoxy)methyl]piperidine-1-carboxylate which was produced in Example 149 (149a) and the title compound was obtained as an oil. Yield 92%.
IR (neat) νₘₐₓ 3316, 1621, 1555, 1119 cm⁻¹;
¹H NMR (CDCl₃, 400MHz) δ 1.05-1.15 (1H, m), 1.39-1.50 (1H, m), 1.62 (1H, br), 1.62-1.68 (1H, m), 1.70-1.85 (4H, m), 2.33 (1H, dd, J = 9.8, 11.7 Hz), 2.54 (1H, dt, J = 3.1, 11.7 Hz), 2.99 (1H, brd, J = 11.7 Hz), 3.11 (1H, brd, J = 11.7 Hz), 3.19-3.26 (2H, m), 3.32 (3H, s), 3.41-3.50 (4H, m);
MS (EI) m/z: 188 [M+H]⁺, 172, 114, 99;
Anal. Calcd for C₁₀H₂₁NO₂·0.1 H₂O: C, 63.52; H, 11.30; N, 7.41. Found: C, 63.70; H, 11.23; N, 7.36.

### (149c) 4-{(3S)-3-[(3-methoxypropoxy)methyl]piperidin-1-yl}-2-thioxo-1,2-dihydropyridine-3-carbonitrile

(3S)-3-[(3-methoxypropoxy)methyl]piperidine (2.05 g, 10.7 mmol) which was produced in Example 149 (149b) and (2Z)-2-cyano-3-ethoxybut-2-enethioamide (J.Org.Chem. (1962), 27,2433-2439) (1.77 g, 10.4 mmol) were mixed with ethanol (30 mL) and the resultant mixture was stirred at room temperature for two hours. The residue obtained by concentrating the reaction mixture under reduced pressure was dissolved in N,N-dimethylformamide (30 mL) and the resultant solution was blended with N,N-dimethylformamide dimethylacetal (1.30 g, 1.45 mL, 10.9 mmol) and the mixture was stirred at room temperature for one hour and the mixture was stirred at 60°C for a further one hour. After cooling the solution, a saturated saline solution (200 mL) was added to the reaction mixture and the aqueous layer was extracted with ethyl acetate (3x100 mL). The organic layer combined was washed with a saturated saline solution (100 mL), and the solvent was evaporated under reduced pressure after drying over sodium sulfate. The residue was purified by silica gel column chromatography (ethyl acetate/methanol =15:1) and 0.54 g of the title compound was obtained (yield 16%).
Mp 137-140°C;
IR (KBr) νₘₐₓ 2212, 1623, 1552, 1522, 1250, 1115 cm⁻¹;
¹H NMR (DMSO-d₆, 400MHz) δ 1.27-1.37 (1H, m), 1.49-1.60 (1H, m), 1.69-1.93 (5H, m), 3.09 (1H, dd, J = 10.2, 12.5 Hz), 3.22 (3H, s), 3.20-3.45 (7H, m), 3.92-4.02 (2H, m), 6.47 (1H, d, J = 7.5 Hz), 7.46 (1H, d, J = 7.5 Hz), 12.62 (1H, br);
MS (EI) m/z: 321 [M⁺], 306, 218;
Anal. Calcd for C₁₆H₂₃N₃O₂S: C, 59.78; H, 7.21; N, 13.07; S, 9.98. Found: C, 59.99; H, 7.15; N, 13.13; S, 9.96.

### (149d) 3-amino-4-{(3S)-3-[(3-methoxypropoxy)methyl]piperidin-1-yl}thieno[2,3-b]pyridine-2-carboxamide

The reaction was performed following a method described in Example 5 (5c) using 4-{(3S)-3-[(3-methoxypropoxy)methyl]piperidin-1-yl}-2-thioxo-1,2-dihydropyridine-3-carbonitrile which was produced in Example 149 (149c) and the title compound was obtained. Yield 72%.
Mp 123-124°C;
IR (KBr) νₘₐₓ 3421, 3324, 3154, 1656, 1580, 1372, 1111 cm⁻¹;
¹H NMR (DMSO-d₆, 400MHz) δ 1.01-1.16 (1H, m), 1.66-1.73 (2H, m), 1.74-1.82 (3H, m), 2.07-2.17 (1H, m), 2.35-2.67 (2H, m), 3.17 (3H, s), 3.23-3.45 (8H, m), 6.92 (2H, br), 7.00 (1H, d, J = 5.5 Hz), 7.08 (2H, br), 8.41 (1H, d, J = 5.5 Hz);
MS (FAB) m/z: 379 [M + H]⁺;
Anal. Calcd for C₁₈H₂₆N₄SO₃: C, 57.12; H, 6.92; N, 14.80; S, 8.47. Found: C, 56.94;
H, 6.77; N, 14.68; S, 8.42;
[α]²⁰_{D}: +79.2° (C = 1.16, MeOH).

### (Example 150) 3-amino-4-[(3S)-3-(methoxymethyl)piperidin-1-yl]-6-methylthieno[2,3-b]pyridine-2-carboxamide (Exemplified Compound No. 1-68)

### (150a) {1-[(3S)-3-(methoxymethyl)piperidin-1-yl]ethylidene}malononitrile

A methanol (5 ml) solution of (1-ethoxyethylidene)malononitrile (136 mg, 1 mmol) which was produced in Example 75 (75a) was blended with (3S)-3-(methoxymethyl)piperidine hydrochloride (166 mg, 1 mmol) which was produced in Example 147 (147b) and triethylamine (139 µL, 1 mmol) and the resultant mixture was stirred at room temperature for 15 hours. The reaction liquid was concentrated and the residue was blended with ethyl acetate (10 ml) and washed with water (10 ml) twice, and the solvent was evaporated under reduced pressure after drying the washed solution over sodium sulfate. The obtained residue was purified by silica gel column chromatography (hexane/ethyl acetate =1:1) and the title compound was obtained (169 mg, yield 77%).
Pale yellow liquid
IR (KBr) νₘₐₓ 2933, 2206, 1563, 1417, 1110, 967, 616 cm-¹;
¹H NMR(CDCl₃, 400 MHz) δ 1.39-1.49 (1H, m), 1.62-1:72 (1H, m), 1.83-1.99 (3H, m), 2.29 (3H, s), 3.13 (1H, dd, J = 9.8, 13.3 Hz), 3.23 (1H, dd, J = 7.3, 9.3 Hz), 3.32 (3H, s), 3.31-3.34 (2H, m), 4.01-4.14 (2H, m);
MS (EI) m/z: 219 [M⁺], 204, 186, 174, 172, 147, 135, 134, 122, 119, 78, 67, 45, 41, 39.

### (150b) {(2E)-3-(dimethylamino)-1-[(3S)-3-(methoxymethyl)piperidin-1-yl]but-2-enylidene} malononitrile

A xylene (7 ml) solution of {1-[(3S)-3-(methoxymethyl)piperidin-1-yl]ethylidene}malononitrile (1.13 g, 5.2 mmol) which was produced in Example 150 (150a) was blended with N,N-dimethylacetamide dimethylacetal (7 ml) and the resultant mixutre was heated under reflux for three hours. Ethyl acetate (50 ml) was added to the residue which was obtained by concentrating the reaction liquid and the organic layer was washed with water (50 ml) twice, and the solvent was evaporated under reduced pressure after drying the washed solution over sodium sulfate. The obtained residue was purified by silica gel column chromatography (100% ethyl acetate) and the title compound was obtained (1.37 g, yield 91%).
Yellow paste-like material
IR (film) νₘₐₓ 2929, 2195, 1562, 1508, 1441, 1625, 1127, 1026, 758, 550 cm⁻¹;
¹H NMR(CDCl₃, 400 MHz) δ 1.31-1.39 (1H, m), 1.59-1.68 (1H, m), 1.76-1.90 (3H, m), 2.22 (3H, s), 2.92-2.98 (2H, m), 3.01 (6H, s), 3.27 (2H, t, J = 7.0 Hz), 3.31 (3H, s), 3.90-3.96 (2H, m), 4.37 (1H, s);
MS (EI) m/z: 288 [M⁺], 273, 248, 243, 223, 212, 189, 160, 146, 135, 128, 85, 70, 56, 44, 42.

### (150c) 2-chloro-4-[(3S)-3-(methoxymethyl)piperidin-1-yl]-6-methylnicotinonitrile

A methanol (15 ml) solution of {(2E)-3-(dimethylamino)-1-[(3S)-3-(methoxymethyl)piperidin-1-yl]but-2-enylidene}malononitrile (1.37 g, 4.8 mmol) which was produced in Example 150 (150b) was blended with oxalyl chloride (3.5 ml, 48 mmol) at 0°C and the resultant mixture was heated under reflux for 10 minutes. A sodium hydrogen carbonate aqueous solution (50 ml) was added to the residual substance which was obtained by concentrating the reaction liquid and the aqueous layer was extracted with ethyl acetate (20 ml) twice. After the extract was dried over sodium sulfate, the solvent was evaporated under reduced pressure. The obtained residue was purified by silica gel column chromatography (hexane/ethyl acetate =3:1) and the title compound was obtained (1.00 g, yield 75%).
Yellow liquid
IR (film) νₘₐₓ 2929, 2857, 2218, 1590, 1511, 1449, 1215, 1126, 966, 855, 593 cm⁻¹; ¹H NMR(CDCl₃, 400 MHz) δ 1.27-1.37 (1H, m), 1.66-1.77 (1H, m), 1.80-1.88 (2H, m), 1.99-2.07 (1H, m), 2.44 (3H, s), 2.90 (1H, dd, J = 9.08, 12.5 Hz), 3.10 (1H, dt, J = 3.1, 11.0 Hz), 3.25-3.35 (2H, m), 3.34 (3H, s), 3.87-3.94 (2H, m), 6.53 (1H, s); MS (EI) m/z: 279 [M⁺], 264, 246, 234, 232, 197, 194, 180, 168, 152, 142, 116, 115, 90,71,67,45,41.

### (150d) 4-[(3S)-(methoxymethyl)piperidin-1-yl]-6-methyl-2-thioxo-1,2-dihydropyridine-3-carbonitrile]

An ethanol (30 ml) solution of 2-chloro-4-[(3S)-3-(methoxymethyl)piperidin-1-yl]-6-methylnicotinonitrile (1.00 g, 3.57 mmol) which was produced in Example 150 (150c) and thiourea (1.36 g, 17.9 mmol) were heated under reflux for 24 hours. The reaction liquid was concentrated and blended with water (20 ml) and the deposited crystal was filtered and the title compound was obtained (0.83 g, yield 83%).
White powder
Mp 137-145°C;
IR (KBr) νₘₐₓ 3366, 3267, 3183, 2216, 1618, 1543, 1453, 1310, 1209, 1093, 723, 626 cm⁻¹;
¹H NMR(DMSO-d₆, 400 MHz) δ 1.26-1.35 (1H, m), 1.52-1.58 (1H, m), 1.73-1.79 (2H, m), 1.83-1.90 (1H, m), 2.22 (3H, s), 2.98 (1H, dd, J = 10.6, 13.7 Hz), 3.10-3.18 (1H, m), 3.21-3.24 (2H, m), 3.23 (3H, s), 3.91-3.97 (2H, m), 6.35 (1H, s), 12.55 (1H, brs);
MS (EI) m/z: 277 [M⁺], 262, 247, 232, 192, 178, 150, 144, 90, 71, 45.

### (150e)3-amino-4-[(3S)-3-(methoxymethyl)piperidin-1-yl]-6-methylthieno[2,3-b]pyridine-2-carboxamide

The reaction was performed following a method described in Example 5 (5c) using 4-[(3S)-(methoxymethyl)piperidin-1-yl]-6-methyl-2-thioxo-1,2-dihydropyridine-3-carbonitrile which was produced in Example 150 (150d) and the title compound was obtained.
White powder
Mp 124-132°C;
IR (KBr) νₘₐₓ 3439, 3324, 3169, 2924, 1652, 1585, 1546, 1489, 1369, 1122, 1087, 476 cm⁻¹;
¹H NMR(CDCl₃, 500 MHz) δ 1.10-1.19 (1H, m), 1.74-1.93 (3H, m), 2.09 (1H, brs), 2.43-2.48 (1H, m), 2.54-2.59 (1H, m), 2.59 (3H, s), 3.27-3.48 (4H, m), 3.34 (3H, s), 5.23 (2H, brs), 6.75 (1H, s), 6.97 (2H, brs);
HRMS m/z calcd for C₁₆H₂₃O₂N₄S 335.1542, found 335.1538;
MS (ESI) m/z: 335 [M+H]⁺;
Anal. Calcd for C₁₆H₂₂N₄O₂S·1.0H₂O: C, 54.33; H, 6.86; N, 15.90; S, 9.10. Found: C, 54.85; H, 6.55; N, 16.25; S, 8.74.
[α]²⁰D: +46.9° (C = 1.00, MeOH).

### (Example 151) 3-amino-4-{3-(4-hydroxybutoxy)methyl]piperidin-1-yl}thieno[2,3-b]pyridine-2-carboxamide (Exemplified Compound No. 1-105)

### (151a) tert-butyl 3-{[4-(benzyloxy)butoxy]methyl}piperidine-1-carboxylate

Sodium hydride (55% oily, 464 mg, 10.6 mmol) was suspended in tetrahydrofuran (15 mL) under nitrogen atmosphere and an N,N-dimethylformamide (6 mL) solution of tert-butyl 3-(hydroxymethyl)piperidine-1-carboxylate (Bioorg.Med.Chem.Lett. 8, (1998) 1595-1600) (1.91 g, 8.87 mmol) was added dropwise thereto. After being stirred at room temperature for one hour, the resultant mixture was blended dropwise with tetrahydrofuran (9 mL) solution of benzyl 3-bromobutyl ether (2.5 mL, 13.31 mmol) and the mixture was stirred at room temperature overnight. A saturated ammonium chloride solution was added to the reaction liquid and the resultant liquid was extracted with ethyl acetate. The organic layer was washed with water and a saturated saline solution, and the solvent was evaporated after drying the washed solution over sodium sulfate. The obtained residue was subjected to silica gel column chromatography (ethyl acetate/hexane) and the title compound was obtained (1.60 g, 48%) as a colourless oil.
IR (Liquid film) νₘₐₓ 2932, 2856, 1694, 1423, 1366, 1266, 1152, 1115 cm⁻¹;
¹H NMR(CDCl₃, 500MHz) :δ 1.14-1.23 (1H, m), 1.45 (9H, s), 1.60-1.71 (5H, m), 1.73-1.81 (2H, m), 2.58 (1H, br), 2.79 (1H, t, J=12.2 Hz), 3.25 (2H, dd, J=1.7, 6.1 Hz), 3.38-3.42 (2H, m), 3.49 (2H, t, J=6.1 Hz), 3.89 (2H, d, J=13.2 Hz), 3.98 (1H, dd, J=5.5, 11.0 Hz), 4.50 (2H, s), 7.26-7.30 (2H, m), 7.32-7.35 (3H, m);
MS(FAB) m/z: 378 [M + H]⁺.

### (151b) tert-butyl 3-[(4-hydroxybutoxy)methyl]piperidine-1-carboxylate

tert-butyl 3-{[4-(benzyloxy)butoxy]methyl}piperidine-1-carboxylate (1.71 g, 4.53 mmol) which was produced in Example 151 (151a) was dissolved in ethanol (10 mL) and the resultant solution was blended with palladium hydroxide (200 mg) and the mixture was stirred at room temperature under normal pressure hydrogen atmosphere for two hours. The reaction liquid was filtered with Celite and the solvent was evaporated under reduced pressure and the title compound was obtained (1.30 g, yield 100%) as an oil.
IR (Liquid film) νₘₐₓ 3741, 2976, 2935, 2859, 1745, 1694, 1426, 1268, 1242, 1154 cm⁻¹;
¹H NMR(CDCl₃, 400MHz) :δ 1.16-1.25 (1H, m), 1.45 (9H, s), 1.40-1.50 (2H, m), 1.60-1.69 (5H, m), 1.75-1.82 (2H, m), 2.78-2.84 (1H, m), 3.29 (2H, d, J=6.3 Hz), 3.41-3.47 (2H, m), 3.64 (2H, t, J=5.7 Hz), 3.81-3.91 (2H, br), 3.97 (1H, dd, J=5.5, 11.0 Hz);
MS(FAB) m/z: 288 [M + H]⁺.

### (151c) 4-(piperidin-3-ylmethoxy)butyl acetate

tert-butyl 3-[(4-hydroxybutoxy)methyl]piperidine-1-carboxylate (775 mg, 2.70 mmol) which was produced in Example (151b) was blended with 4N hydrochloric acid - ethyl acetate solution (5 mL) and the resultant mixture was stirred at room temperature for 2.5 hours. After the reaction terminated, the residue which was obtained by evaporating the solvent was blended with methylene chloride and 1N aqueous solution of sodium hydroxide and partitioned. The obtained organic layer was washed with a saturated saline solution and dried over anhydrous sodium sulfate, and the solvent was evaporated under reduced pressure, and the title compound was obtained (516 mg, 83%) as an oil.
¹H NMR(CDCl₃, 500MHz) :δ 1.06-1.15 (1H, m), 1.41-1.51 (1H, m), 1.59-1.85 (8H, m), 2.05 (3H, s), 2.33 (1H, dd, J=10.0, 12.2 Hz), 2.55 (1H, dt, J=2.9, 12.2 Hz), 3.00 (1H, d, J=11.7 Hz), 3.12 (1H, d, J=10.7 Hz), 3.22-3.24 (2H, m), 3.38-3.43 (2H, m), 4.08 (2H, t, J=6.6 Hz);
MS(FAB) m/z: 230 [M + H]⁺.

### (151d) 4-([1-(3-cyano-2-thioxo-1,2-dihydropyridin-4-yl)piperidin-3-yl]methoxy)butyl acetate

4-(piperidin-3-ylmethoxy)butyl acetate (504 mg, 2.20 mmol) which was produced in Example 151 (151c) was dissolved in N,N-dimethylformamide (2 mL) and the mixture was blended with (2Z)-2-cyano-3-ethoxybut-2-enethioamide (J.Org.Chem. (1962), 27,2433-2439) (299 mg, 1.76 mmol) and the mixture was stirred at room temperature for 30 minutes. The reaction liquid was blended with N,N-dimethylformamide dimethylacetal (294 µL, 2.20 mmol) and the mixture was stirred at room temperature overnight and the mixture was stirred at 80°C for one hour. After the reaction terminated, the reaction liquid was diluted with water and extracted with ethyl acetate. The obtained organic layer was washed with a saturated saline solution and dried over anhydrous sodium sulfate, and the solvent was evaporated under reduced pressure. The obtained residue was purified by silica gel column chromatography (methylene chloride/methanol =40:1 to 20:1) and an oil (135 mg) mainly containing the title compound was obtained.
¹H NMR(CDCl₃, 400MHz) :δ 1.36-1.44 (1H, m), 1.57-1.74 (6H, m), 1.83-1.91 (2H, m), 2.05 (3H,s), 3.09 (1H, dd, J=10.0, 13.1 Hz), 3.25-3.45 (7H, m), 4.08 (2H, t, J=6.6 Hz), 6.29 (1H, d, J=7.4 Hz), 7.27 (1H, d, J=7.4 Hz), 11.9 (1H, br).

### (151e) 3-amino-4-{3-[(4-hydroxybutoxy)methyl]piperidin-1-yl}thieno[2,3-b]pyridine-2-carboxamide

4-([1-(3-cyano-2-thioxo-1,2-dihydropyridin-4-yl)piperidin-3-yl]methoxy)butyl acetate (134 mg) which was produced in Example 151 (151d) was dissolved in N,N-dimethylformamide (1.5 mL) and the resultant solution was blended with 2-chloroacetamide (44.8 mg, 0.479 mmol) and 8N aqueous solution of sodium hydroxide (0.3 mL) and the mixture was stirred at room temperature overnight. After the reaction terminated, the reaction liquid was diluted with water and extracted with ethyl acetate. The obtained organic layer was washed with a saturated saline solution and dried over anhydrous sodium sulfate, and the solvent was evaporated under reduced pressure. The residue was purified by thin layer chromatography (methylene chloride/methanol =10:1) and the title compound was obtained (42.2 mg, yield 30% from 4-(piperidin-3-ylmethoxy)butyl acetate) as a pale yellow solid.
Mp 140-141 °C;
IR (KBr) νₘₐₓ 3436, 3325, 3183, 2933, 2859, 1651, 1581, 1501, 1373, 1347 cm⁻¹;
¹H NMR(CDCl₃, 500MHz) :δ 1.09-1.18 (1H, m), 1.64-1.69 (4H, m), 1.76-1.84 (1H, m), 1.86-1.94 (2H, br), 2.08-2.17 (1H, m), 2.35 (1H, br s), 2.41 (1H, t, J=11.2 Hz), 2.64 (1H, t, J=10.5 Hz), 3.28-3.34 (1H, m), 3.36-3.51 (4H, m), 3.52-3.58 (1H, m), 3.64 (2H, d, J=2.4 Hz), 5.32 (2H, br s), 6.89 (1H, d, J=5.4 Hz), 6.98 (2H, br s), 8.46 (1H, d, J=5.4 Hz);
MS(FAB) m/z: 379 [M + H]⁺;
Anal. calcd for C₁₈H₂₆N₄O₃S·3/10H₂O : C,56.32; H, 6.98; N,14.59; S,8.35. Found C,56.33; H,6.71; N,14.60, S, 8.31.

### (Example 152) 3-amino-4-{3-[(3-hydroxypropoxy)methyl]piperidin-l-yl}thieno[2,3-b]pyridine-2-carboxamide (Exemplified Compound No. 1-102)

### (152a) benzyl 3-{[3-(benzyloxy)propoxy]methyl}piperidine-1-carboxylate

The reaction was performed following a method described in Example 151 (151a) using benzyl 3-(hydroxymethyl)piperidine-1-carboxylate (Arch.Pharm.(Weinheim Ger.) 323,1990,9-12) (2.03 g, 8.14 mmol) in place of tert-butyl 3-(hydroxymethyl)piperidine-1-carboxylate and using benzyl 4-bromopropyl ether in place of benzyl 3-bromobutyl ether and the title compound was obtained (538 mg, yield 17%) as an oil.
IR (Liquid film) νₘₐₓ 3462, 2932, 2859, 1699, 1433, 1262, 1237, 1113 cm⁻¹;
¹H NMR(CDCl₃, 400MHz) :δ 1.14-1.24 (1H, m), 1.45 (1H, br s), 1.60-1.68 (1H, m), 1.71-1.80 (2H, m), 1.85 (2H, qu, J=6.3 Hz), 2.64 (1H, br d), 2.81-2.88 (1H, m), 3.25 (2H, d, J= 5.5 Hz), 3.43-3.49 (2H, m), 3.51-3.57 (2H, m), 3.98 (1H, dt, J=3.9, 12.9 Hz), 4.06 (1H, br s), 4.47 (2H, s), 5.10 (2H, d, J=3.9 Hz), 7.23-7.35 (10H, m); MS(FAB) m/z: 398 [M + H]⁺.

### (152b) benzyl 3-{[3-(benzyloxy)propoxy]methyl}piperidine-1-carboxylate

benzyl 3-{[3-(benzyloxy)propoxy]methyl}piperidine-1-carboxylate (1.90 g, 4.78 mmol) which was produced in Example 152 (152a) was dissolved in ethanol (3 mL) and the resultant solution was blended with 10% palladium-carbon (180 mg) and the mixture was stirred at room temperature under normal pressure hydrogen atmosphere for 1.5 hours. After the reaction terminated, the reaction liquid was filtered with Celite and the solvent was evaporated under reduced pressure. The obtained residual substance (1.24 g) was dissolved in methylene chloride (7 mL) and the mixture was blended with di-tert-butyl dicarbonate (1.28 mL, 5.57 mmol) and the mixture was stirred at room temperature under nitrogen atmosphere for 2.5 hours. After the reaction liquid was concentrated under reduced pressure, the obtained residue was purified by silica gel column chromatography (hexane:ethyl acetate =3:1) and the title compound was obtained (1.62 g, yield 95%) as an oil.
IR (Liquid film) νₘₐₓ 2975, 2931, 2858, 1694, 1424, 1366, 1267, 1242, 1179, 1118 cm⁻¹;
¹H NMR(CDCl₃, 400MHz) :δ 1.12-1.26 (1H, m), 1.45 (9H, s), 1.60-1.66 (1H, m), 1.71-1.80 (2H, m), 1.84-1.90 (2H, qu, J=6.3 Hz), 2.57 (1H, br), 2.77 (1H, t, J=11.4 Hz), 3.25 (2H, m), 3.45-3.51 (2H, m), 3.55 (2H, t, J=6.3 Hz), 3.88 (2H, dt, J=3.7, 12.5 Hz), 3.97 (1H, br), 4.49 (2H, s), 7.24-7.28 (2H, m), 7.30-7.32 (3H, m); MS(FAB) m/z: 364 [M + H]⁺.

### (152c) tert-butyl 3-[(3-hydroxypropoxy)methyl]piperidine-1-carboxylate

The reaction was performed following a method described in Example 151 (151b) using benzyl 3-{[3-(benzyloxy)propoxy]methyl}piperidine-1-carboxylate (1.61 g, 4.43 mmol) which was produced in Example 152 (152b) and the title compound was obtained (1.17 g, yield 96%) as a colourless solid.
Mp 78°C;
IR (KBr) νₘₐₓ 3466, 2974, 2938, 2861, 1679, 1473, 1438, 1272, 1156, 1121 cm⁻¹; ¹H NMR(CDCl₃, 400MHz) :δ 1.18-1.27 (1H, m), 1.45 (9H, s), 1.56-1.66 (1H, m), 1.67-1.83 (5H, m), 2.36 (1H, br), 2.78 (2H, br), 2.92 (1H, br), 3.26-3.33 (2H, m), 3.54-3.63 (2H, m), 3.77 (2H, br), 3.86 (1H, d, J=12.1 Hz);
MS(FAB) m/z: 274 [M + H]⁺;
Anal. calcd for C₁₄H₂₇NO₄·1/10H₂O : C,61.11; H,9.96; N,5.09. Found C, 61.05; H,10.03; N,4.99.

### (152d) 3-(piperidin-3-ylmethoxy)propyl acetate

The reaction was performed following a method described in Example 151 (151c) using tert-butyl 3-[(3-hydroxypropoxy)methyl]piperidine-1-carboxylate (932 mg, 3.41 mmol) which was produced in Example 152 (152c) and the title compound was obtained (655 mg, yield 89%) as an oil.
IR (Liquid film) νₘₐₓ 3315, 2930, 2856, 1739, 1368, 1244, 1125, 1052 cm⁻¹;
¹H NMR(CDCl₃, 500MHz) :δ 1.06-1.15 (1H, m), 1.41-1.51 (1H, m), 1.62-1.69 (1H, m), 1.72-1.85 (3H, m), 1.86-1.91 (2H, m), 2.05 (3H, s), 2.33 (1H, t, J=11.3 Hz), 2.55 (1H, t, J=12.1 Hz), 3.00 (1H, d, J=12.1 Hz), 3.11 (1H, d, J=11.3 Hz), 3.24 (2H, d, J=5.9 Hz), 3.44-3.48 (2H, m), 4.15 (2H, t, J=6.4 Hz);
MS(EI) m/z: 215 M⁺.

### (152e) 4-([1-(3-cyano-2-thioxo-1,2-dihydropyridin-4-yl)piperidin-3-yl)methoxy)propyl acetate

The reaction was performed following a method described in Example 151 (151d) using 3-(piperidin-3-ylmethoxy)propyl acetate (640 mg, 2.97 mmol) which was produced in Example 152 (152d) and an oil (234 mg) mainly containing the title compound was obtained.
¹H NMR(CDCl₃, 400MHz) :δ 1.35-1.46 (1H, m), 1.57-1.74 (4H, m), 1.83-1.92 (2H, m), 2.05 (3H,s), 3.09 (1H, dd, J=9.8, 13.2 Hz), 3.26-3.50 (7H, m), 4.14 (2H, t, J=6.7 Hz), 6.29 (1H, d, J=7.8 Hz), 7.27 (1H, d, J=7.8 Hz), 11.9 (1H, br).

### (152f) 3-amino-4-{3-[(3-hydroxypropoxy)methyl]piperidin-1-yl}thieno[2,3-b]pyridine-2-carboxamide

The reaction was performed following a method described in Example 151 (151e) using 4-([1-(3-cyano-2-thioxo-1,2-dihydropyridin-4-yl)piperidin-3-yl]methoxy)propyl acetate which was produced in Example 152 (152d) and the title compound was obtained (109 mg, yield 10.0% from 3-(piperidin-3-ylmethoxy)propyl acetate) as a pale brown solid.
Mp 166-168°C;
IR (KBr) νₘₐₓ 3436, 3325, 3180, 2931, 2859, 1652, 1581, 1501, 1373, 1347 cm⁻¹; ¹H NMR(CDCl₃, 400MHz) :δ 1.09-1.18 (1H, m), 1.76-1.86 (3H, m), 1.86-1.94 (2H, br), 2.08-2.17 (1H, m), 2.31-2.36 (1H, m), 2.42 (1H, t, J=11.5 Hz), 2.62 (1H, t, J=11.5 Hz), 3.31-3.45 (3H, m), 3.48-3.54 (1H, m), 3.55-3.66 (2H, m), 3.75 (2H, q, J=5.5 Hz), 5.33 (2H, br s), 6.86 (1H, d, J=5.5 Hz), 6.93 (2H, br s), 8.44 (1H, d, J=5.5 Hz);
MS(FAB) m/z: 365 [M + H]⁺;
Anal. calcd for C₁₇H₂₄N₄O₃S·4/5H₂O : C,53.89; H,6.81; N,14.79; S,8.46. Found C,53.93; H,6.51; N,14.76, S,8.22.

### (Example 153) 3-amino-4-[3-(3-hydroxypropyl)piperidin-1-yl]thieno[2,3-b]pyridine-2-carboxamide (Exemplified Compound No. 1-96)

### (153a) 3-piperidin-3-ylpropyl acetate.

The reaction was performed following a method described in Example 151 (151c) using tert-butyl 3-(3-hydroxypropyl)piperidine-1-carboxylate (626 mg, 2.57 mmol) which was synthesized according to a method described in J. Med. Chem. (1996), 41,2709-2719 and the title compound was obtained (400 mg, yield 84%) as an oil.
IR (Liquid film) νₘₐₓ 3321, 2930, 2852, 1738, 1547, 1451, 1367, 1244, 1051, 1037 cm⁻¹;
¹H NMR(CDC1₃, 400MHz) :δ 0.96-1.06 (1H, m), 1.15-1.28 (2H, m), 1.35-1.49 (2H, m), 1.53-1.70 (4H, m), 1.78-1.86 (1H, m), 2.04 (3H, s), 2.23 (1H, t, J=11.0 Hz), 2.52 (1H, dt, J=2.7, 12.0 Hz), 2.97-3.04 (2H, m), 4.03 (2H, t, J=6.9 Hz);
MS(FAB) m/z: 186 [M + H]⁺.

### (153b) 3-[1-(3-cyano-2-thioxo-1,2-dihydropyridin-4-yl)piperidin-3-yl]propyl acetate

The reaction was performed following a method described in Example 151 (151d) using 3-piperidin-3-ylpropyl acetate (400 mg, 2.16 mmol) which was produced in Example 153 (153a) and an oil (80 mg) mainly containing the title compound was obtained.
¹H NMR(CDCl₃, 400MHz): δ 1.19-1.42 (3H, m), 1.63-1.77 (4H, m), 1.84-1.92 (1H, m), 2,1.94-2.01 (1H, m), 2.05 (3H,s), 2.85 (1H, dd, J=10.6, 13.2 Hz), 3.12-3.19 (1H, m), 4.05 (2H, dt, J=2.0, 6.5 Hz), 4.12 (2H, t, J=12.5 Hz), 6.25 (1H, d, J=7.4 Hz), 7.29 (1H, d, J=7.4 Hz), 12.2 (1H, br).

### (153c) 3-amino-4-[3-(3-hydroxypropyl)piperidin-1-yl]thieno[2,3-b]pyridine-2-carboxamide

The reaction was performed following a method described in Example 151 (151e) using 3-[1-(3-cyano-2-thioxo-1,2-dihydropyridin-4-yl)piperidin-3-yl]propyl acetate which was produced in Example 153 (153b) and the title compound was obtained (68.0 mg, yield 9.4% from 3-piperidin-3-ylpropyl acetate) as yellow amorphous.
IR (KBr) νₘₐₓ 3440, 3326, 3189, 2931, 2852, 1648, 1581, 1502, 1373, 1346 cm⁻¹;
¹H NMR(CDCl₃, 400MHz): δ 0,98-1.08 (1H, m), 1.30-1.36 (2H, m), 1.54-1.82 (4H, m), 1.84-2.03 (3H, m), 2.28 (1H, t, J=11.2 Hz), 2.62 (1H, t, J=11.2 Hz), 3.38 (2H, t, J=9.6 Hz), 3.65 (2H, t, J=6.3 Hz), 5.52 (2H, br s), 6.84 (1H, d, J=5.1 Hz), 6.97 (2H, br s), 8.41 (1H, d, J=5.1 Hz);
MS(FAB) m/z: 335 [M + H]⁺.

### (Example 154) 3-amino-4-[3-(3-methoxypropyl)piperidin-1-yl]thieno[2,3-b]pyridine-2-carboxamide (Exemplified Compound No. 1-109)

### (154a) tert-butyl 3-(3-methoxypropyl)piperidine-1-carboxylate

tert-butyl 3-(3-hydroxypropyl)piperidine-1-carboxylate (J. Med. Chem. (1996), 41, 2709-2719) (617 mg, 2.54 mmol) was dissolved in tetrahydrofuran (10 mL) and the resultant solution was blended with sodium hydride (55% oily, 230 mg, 5.27 mmol) and the mixture was stirred under nitrogen atmosphere at room temperature for 30 minutes. The reaction liquid was blended with methyl iodide (0.60 mL, 9.64 mmol) and the mixture was stirred at room temperature for 30 minutes. Furthermore, the reaction liquid was blended with sodium hydride (200 mg, 4.58 mmol) and methyl iodide (1.2 mL, 19.2 mmol) and after it was stirred at room temperature for one hour, was further blended with a saturated ammonium chloride aqueous solution and the mixture was extracted with ethyl acetate. The extract was washed with a saturated saline solution and dried over anhydrous sodium sulfate and the solvent was evaporated under reduced pressure. The residue was purified by silica gel column chromatography (hexane: ethyl acetate = 6: 1) and the title compound was obtained (618 mg, yield 95%) as an oil.
IR (Liquid film) νₘₐₓ 2976, 2933, 2856, 1696, 1423, 1366, 1267, 1241, 1177, 1150 cm⁻¹;
¹H NMR(CDCl₃, 500MHz) :δ 1.02-1.11 (1H, m), 1.18-1.32 (2H, m), 1.36-1.49 (2H, m), 1.45 (9H, s), 1.57-1.66 (3H, m), 1.77-1.86 (1H, m), 2.30-2.60 (1H, br), 2.74 (1H, t, J=11.7 Hz), 3.33 (3H, s), 3.36 (2H, t, J=6.6 Hz), 3.92 (1H, d, J=12.7 Hz), 3.80-4.10 (1H, br);
MS(EI) m/z: 257 M⁺.

### (154b) 3-(3-methoxy propyl)piperidine

The reaction was performed following a method described in Example 151 (151c) using tert-butyl 3-(3-methoxypropyl)piperidine-1-carboxylate (607 mg, 2.36 mmol) which was produced in Example 154 (154a) and the title compound was obtained (362 mg, yield 98%) as an oil.
IR (Liquid film) νₘₐₓ 3401, 3311, 2929, 2852, 2737, 1645, 1543, 1451, 1274, 1118 cm⁻¹ ;
¹H NMR(CDCl₃, 400MHz) :δ 0.96-1.06 (1H, m), 1.13-1.28 (2H, m), 1.35-1.49 (2H, m), 1.53-1.67 (3H, m), 1.79-1.86 (2H, m), 2.22 (1H, t, J=11.0 Hz), 2.51 (1H, dt, J=2.5, 11.8 Hz), 2.96-3.06 (2H, m), 3.32 (3H, s), 3.34 (2H, t, J=6.7 Hz);
MS(FAB) m/z: 158 [M + H]⁺.

### (154c) 4-[3-(3-methoxypropyl)piperidin-1-yl]-2-thioxo-1,2-dihydropyridine-3-carbonitrile

3-(3-methoxypropyl)piperidine (355 mg, 2.26 mmol) which was produced in Example 154 (154b) was dissolved in N,N-dimethylformamide (2 mL) and the resultant solution was blended with (2Z)-2-cyano-3-ethoxybut-2-enethioamide (J.Org.Chem. (1962), 27,2433-2439) (312 mg, 1.83 mmol) and the mixture was stirred at room temperature for one hour. The reaction liquid was blended with N,N-dimethylformamide dimethylacetal (266 µL, 1.99 mmol), stirred at room temperature overnight and then diluted with water and the aqueous layer was extracted with ethyl acetate. The extract was washed with a saturated saline solution and dried over anhydrous sodium sulfate and the solvent was evaporated under reduced pressure. The residue was purified by silica gel column chromatography (methylene chloride:methanol = 30:1-20:1) and thin layer chromatography (100% ethyl acetate) and the title compound was obtained (59.6 mg, yield 9.0%) as a yellow amorphous substance.
IR (KBr) νₘₐₓ 3115, 2935, 2854, 2209, 1624, 1552, 1519, 1310, 1250, 1116 cm⁻¹;
¹H NMR(CDCl₃, 400MHz) :δ 1.18-1.28 (1H, m), 1.29-1.40 (2H, m), 1.55-1.75 (4H, m), 1.83-1.90 (1H, m), 1.94-2.02 (1H, m), 2.85 (1H, dd, J=10.8, 13.1 Hz), 3.14-3.21 (1H, m), 3.33 (3H, s), 3.36-3.41 (2H, m), 4.08-4.18 (2H, m), 6.27 (1H, d, J=7.4 Hz), 7.30 (1H, d, J=7.4 Hz), 12.23 (1H, br);
MS(FAB) m/z: 292 [M + H]⁺.

### (154d) 3-amino-4-[3-(3-methoxypropyl)piperidin-1-yl]thieno[2,3-b]pyridine-2-carboxamide

4-[3-(3-methoxypropyl)piperidin-1-yl]-2-thioxo-1,2-dihydropyridine-3-carbonitrile (58.6 mg, 0.201 mmol) which was produced in Example 154 (154c) was dissolved in N,N-dimethylformamide (1 mL) and the resultant solution was blended with 2-chloroacetamide (32.8 mg, 0.350 mmol) and 8N aqueous solution of sodium hydroxide (0.2 mL) and the mixture was stirred at room temperature for two hours. After the reaction terminated, the reaction liquid was diluted with water and extracted with ethyl acetate. The obtained organic layer was washed with a saturated saline solution and dried over anhydrous sodium sulfate, and the solvent was evaporated under reduced pressure. The residue was purified by thin layer chromatography (100% ethyl acetate) and the title compound was obtained (65.5 mg, yield 93%) as yellow amorphous.
IR (KBr) νₘₐₓ 3438, 3323, 3178, 2932, 2852, 1651, 1580, 1556, 1501, 1371 cm⁻¹;
¹H NMR(CDCl₃, 400MHz) : δ 0,98-1.08 (1H, m), 1.29-1.35 (2H, m), 1.56-1.68 (2H, m), 1.69-1.82 (2H, m), 1.84-1.92 (1H, m), 1.93-2.01 (1H, m), 2.31 (1H, t, J=11.0 Hz), 2.62 (1H, t, J=11.7 Hz), 3.32 (3H, s), 3.35-3.42 (2H, m), 3.37 (2H, t, J=6.5 Hz), 5.29 (2H, br s), 6.85 (1H, d, J=5.5 Hz), 6.99 (2H, br s), 8.43 (1H, d, J=5.5 Hz);
MS(FAB) m/z: 349 [M + H]⁺;
Anal. calcd for C₁₇H₂₄N₄O₂S·1/5H₂O: C,58.00; H,6.99; N,15.91; S, 9.11. Found C,58.06; H,6.98; N,15.70; S,8.85.

### (Example 155) 3-amino-4-[3-(4-hydroxybutyl)piperidin-1-yl]thieno[2,3-b]pyridine-2-carboxamide (Exemplified Compound No. 1-97)

### (155a) tert-butyl 3-(3-oxopropyl)piperidine-1-carboxylate

tert-butyl 3-(3-hydroxypropyl)piperidine-1-carboxylate (J. Med. Chem. (1996), 41,2709-2719) (1.08 g, 4.44 mmol) was dissolved in methylene chloride (10 mL) and the resultant solution was blended with Dess-Martin periodinane (2.3 g, 5.42 mmol) and the mixture was stirred under nitrogen atmosphere at room temperature for two hours. Ether was added to the reaction liquid and the organic layer was washed sequentially with 1N aqueous solution of sodium hydroxide and a saturated saline solution and dried over anhydrous sodium sulfate and the solvent was evaporated under reduced pressure and the title compound was obtained (1.00 g, yield 93%).
IR (Liquid film) νₘₐₓ 2976, 2932, 2857, 1726, 1692, 1425, 1366, 1268, 1174, 1150 cm⁻¹;
¹H NMR(CDCl₃, 500MHz) :δ 1.05-1.14 (1H, m), 1.37-1.60 (4H, m), 1.45 (9H, s), 1.60-1.67 (1H, m), 1.78-1.85 (1H, m), 2.40-2.60 (1H, br), 2.49 (2H, t, J=7.3 Hz), 2.80 (1H, t, J=11.2 Hz), 3.88 (1H, dt, J=3.5, 13.1 Hz), 3.98 (1H, br), 9.79 (1H, s); MS(FAB) m/z: 242 [M + H]⁺.

### (155b) tert-butyl 3-[(3)-4-methoxybut-3-enyl]piperidine-1-carboxylate

(Methoxymethyl) triphenylphosphonium chloride (1.76 g, 5.13 mmol) was dissolved in tetrahydrofuran (5 mL) and the resultant solution was blended with a hexane solution (1.50M, 3.0 mL, 4.5 mmol) ofN-butyllithium under ice-cooling and the mixture was stirred under nitrogen atmosphere at 0°C for 30 minutes. A tetrahydrofuran (5 mL) solution of tert-butyl 3-(3-oxopropyl)piperidine-l-carboxylate (675 mg, 2.80 mmol) which was produced in Example 155 (155a) was added dropwise to the reaction mixture and the resultant mixture was stirred at 0°C for one hour and the mixture was stirred at room temperature for 3 hours. A saturated ammonium chloride aqueous solution was added to the reaction liquid and the aqueous layer was extracted with ethyl acetate. The obtained organic layer was washed with a saturated saline solution and dried over anhydrous sodium sulfate, and the solvent was evaporated under reduced pressure. The residue was purified by silica gel column chromatography (hexane:ethyl acetate =8:1) and the title compound was obtained (475 mg, yield 63%) as an oil.
IR (Liquid film) νₘₐₓ: 2976, 2932, 2853, 1695, 1656, 1423, 1366, 1267, 1176, 1109 cm⁻¹;
¹H NMR(CDCl₃, 400MHz) : δ 0.98-1.12 (1H, br), 1.15-1.33 (2H, m), 1.37-1.56 (2H, m), 1.45 (9H, s), 1.58-1.66 (1H, m), 1.76-1.87 (1H, m), 1.95 (1H, q, J=7.0 Hz), 2.08 (1H, dq, J=1.3, 7.5 Hz), 2.26-2.63 (1H, br), 2.75 (1H, t, J= 10.8 Hz), 3.49 (3/2H, s), 3.56 (3/2H, s), 3.74-4.07 (1H, br), 3.85-3.92 (1H, m), 4.30 (1/2H, q, J=7.0 Hz), 4.68 (1/2H, dt, J=7.5, 12.5 Hz), 5.85 (1/2H, d, J=6.3 Hz), 6.27 (1/2H, d, J=12.5 Hz); MS(EI) m/z: 269 M⁺.

### (155c) tert-butyl 3-(4-hydroxybutyl)piperidine-1-carboxylate

tert-butyl 3-[(3)-4-methoxybut-3-enyl]piperidine-1-carboxylate (465 mg, 1.73 mmol) which was produced in Example 155 (155b) was dissolved in methanol (2 mL) and water (2 mL) and the resultant solution was blended with sulfuric acid (0.2 mL) and the mixture was stirred under nitrogen atmosphere at room temperature for seven hours. The reaction liquid was blended with a sodium hydrogen carbonate aqueous solution and extracted with ethyl acetate. The extract was concentrated under reduced pressure and the obtained residue was dissolved in acetone (4 mL), and the resultant solution was blended with amberlite (IR - 120PLUS, 1 g), and the mixture was stirred overnight at room temperature. The reaction liquid was filtered and the filtrate was dried over anhydrous sodium sulfate and the solvent was evaporated under reduced pressure and a crude aldehyde compound (237 mg) was obtained. The obtained compound was dissolved in methanol (5 mL) and the solution was blended with an excess amount of sodium borohydride under ice-cooling, and the mixture was stirred under nitrogen atmosphere at room temperature for 30 minutes. The reaction liquid was blended with water and then a saturated ammonium chloride aqueous solution and extracted with ethyl acetate. The extract was washed with a saturated saline solution and dried over anhydrous sodium sulfate and the solvent was evaporated under reduced pressure. The residue was purified by silica gel column chromatography (hexane:ethyl acetate =3:1) and the title compound was obtained (197 mg, yield 44%) as an oil.
IR (Liquid film) νₘₐₓ 3443, 2976, 2932, 2859, 1694, 1672, 1428, 1367, 1269, 1152 cm⁻¹;
¹H NMR(CDCl₃, 500MHz) : δ 1.03-1.11 (1H, m), 1.14-1.33 (3H, m), 1.35-1.47 (4H, m), 1.45 (9H, s), 1.52-1.65 (3H, m), 1.78-1.84 (1H, m), 2.48 (1H, br), 2.77-2.82 (1H, m), 3.64 (2H, q, J=5.4 Hz), 3.71-4.04 (2H, br);
MS(EI) m/z: 257 M⁺.

### (155d) 4-piperidin-3-ylbutyl acetate

The reaction was performed following a method described in Example 151 (151c) using tert-butyl 3-(4-hydroxybutyl)piperidine-1-carboxylate (301 mg, 1.17 mmol) which was produced in Example 155 (155c) and the title compound was obtained (195 mg, yield 84%) as an oil.
IR (Liquid film) νₘₐₓ 3323, 2930, 2851, 1739, 1645, 1459, 1445, 1367, 1243, 1038 cm⁻¹;
¹H NMR(CDCl₃, 400MHz) : δ 0.95-1.05 (1H, m), 1.12-1.25 (2H, m), 1.31-1.49 (4H, m), 1.57-1.66 (3H, m), 1.69-1.85 (2H, m), 2.04 (3H, s), 2.21 (1H, dd, J=10.5, 12.0 Hz), 2.52 (1H, dt, J=2.7, 12.0 Hz), 2.96-3.05 (2H, m), 4.04 (2H, t, J=6.7 Hz); MS(EI) m/z: 198 (M - H)⁺.

### (155e) 4-[1-(3-cyano-2-thioxo-1,2-dihydropyridin-4-yl)piperidin-3-yl]butyl acetate

The reaction was performed following a method described in Example 151 (151d) using 4-piperidin-3-ylbutyl acetate (185 mg, 0.928 mmol) which was produced in Example 155 (155d) and an oil (36 mg) mainly containing the title compound was obtained.
¹H NMR(CDCl₃, 500MHz) :δ 1.16-1.50 (5H, m), 1.59-1.78 (4H, m), 1.84-2.00 (2H, m), 2.06 (3H, s), 3.13-3.19 (1H, m), 3.49-3.55 (1H, m), 4.07 (2H, t, J=6.4 Hz), 4.09-4.15 (2H, m), 6.25 (1H, d, J=7.8 Hz), 7.31 (1H, d, J=7.8 Hz), 12.1 (1H, br).

### (155f) 3-amino-4-[3-(4-hydroxybutyl)piperidin-1-yl]thieno-[2,3-b]pyridine-2-carboxamide

The reaction was performed following a method described in Example 151 (151e) using 4-[1-(3-cyano-2-thioxo-1,2-dihydropyridin-4-yl)piperidin-3-yl]butyl acetate which was produced in Example 155 (155e) and the title compound was obtained (15.5 mg, yield 4.8% from 4-piperidin-3-ylbutyl acetate) as a yellow amorphous substance.
IR (KBr) νₘₐₓ 3441, 3326, 3187, 2931, 2855, 1649, 1581, 1502, 1374, 1247 cm⁻¹;
¹H NMR(CDCl₃, 500MHz) :δ 0,99-1.07 (1H, m), 1.25-1.33 (2H, m), 1.36-1.50 (3H, m), 1.54-1.69 (2H, m), 1.70-1.81 (2H, m), 1.85-1.91 (1H, m), 1.96 (1H, d, J=11.7 Hz), 2.29 (1H, t, J=11.0 Hz), 2.64 (1H, t, J=11.7 Hz), 3.40 (2H, t, J=11.0 Hz), 3.65 (2H, t, J=6.6 Hz), 5.34 (2H, br s), 6.87 (1H, d, J=5.1 Hz), 7.01 (2H, br s), 8.45 (1H, d, J=5.1 Hz);
MS(FAB) m/z: 349 [M + H]⁺.

### (Example 156) 3-amino-4-{3-[(2-cyanoethoxy)methyl]piperidin-1-yl}thieno[2,3-b]pyridine-2-carboxamide (Exemplified Compound No. 1-152)

### (156a) benzyl 3-[(2-cyanoethoxy)methyl]piperidine-1-carboxylate

benzyl 3-(hydroxymethyl)piperidine-1-carboxylate (Arch.Pharm.(Weinheim Ger.) 323,1990,9-12) (6.30 g, 25.3 mmol) was dissolved in tetrahydrofuran (50 mL) and the resultant solution was blended with acrylonitrile (3.31 mL, 50.5 mmol) and sodium hydroxide (160 mg, 2.85 mmol) and the mixture was stirred under nitrogen atmosphere at room temperature for nine hours. Furthermore, the reaction liquid was blended with sodium hydroxide (160 mg, 2.85 mmol) and the mixture was stirred at room temperature overnight. Water was added to the reaction liquid and the aqueous layer was extracted with ethyl acetate. After the extract was washed with a saturated saline, it was dried over anhydrous sodium sulfate solution and the solvent was evaporated under reduced pressure. The residue was purified by silica gel column chromatography (hexane:ethyl acetate =3:1 to 2:1) and the title compound was obtained (6.91 g, yield 90%).
IR (Liquid film) νₘₐₓ 2937, 2863, 2251, 1698, 1471, 1433, 1262, 1236, 1155, 1117 cm⁻¹;
¹H NMR(CDCl₃, 400MHz) :δ 1.22-1.33 (1H, m), 1.40-1.53 (1H, m), 1.62-1.70 (1H, m), 1.76-1.87 (2H, br), 2.55 (2H, br s), 2.66-2.87 (1H, br), 2.93 (1H, t, J=11.0 Hz), 3.35 (2H, d, J=5.1 Hz), 3.59 (2H, br s), 3.92 (1H, dt, J=4.2, 13.3 Hz), 4.01 (1H, br s), 5.08-5.17 (2H, m), 7.27-7.35 (5H, m);
MS(EI) m/z: 303 [M + H]⁺.

### (156b) 3-(piperidin-3-ylmethoxy) propanenitrile

Hydrogenolysis reaction was performed using benzyl 3-[(2-cyanoethoxy)methyl]piperidine-1-carboxylate (1.03 g, 3.40 mmol) which was produced in Example 156 (156a) in the presence of a palladium-carbon catalyst in ethanol and the title compound was obtained (564 mg, yield 99%) as an oil.
IR (Liquid film) νₘₐₓ 3389, 3326, 2932, 2860, 2251, 1640, 1545, 1418, 1273, 1113 cm⁻¹;
¹H NMR(CDCl₃, 400MHz) :δ 1.08-1.18 (1H, m), 1.40-1.51 (1H, m), 1.62-1.69 (2H, m), 1.75-1.83 (2H, m), 2.36 (1H, dd, J=10.0, 11.7 Hz), 2.55 (1H, dd, J=2.7, 11.7 Hz), 2.59 (2H, t, J=6.4 Hz), 2.99 (1H, dt, J=3.3, 12.1 Hz), 3.12 (1H, d, J=12.1 Hz), 3.32 (2H, d, J=6.3 Hz), 3.58-3.67 (2H, m);
MS(FAB) m/z: 169 [M + H]⁺. (156c) 4-(3-[(2-cyanoethoxy)methyl]piperazin-1-yl)-2-thioxo-1,-dihydropyridine-3-carbonitrile

The reaction was performed following a method described in Example 151 (151d) using 3-(piperidin-3-ylmethoxy)propanenitrile (550 mg, 3.27 mmol) which was produced in Example 156 (156b) and an oil (178 mg) mainly containing the title compound was obtained.
¹H NMR(CDCl₃, 400MHz) :δ 1.38-1.48 (1H, m), 1.67-1.91 (3H, m), 2.03-2.11 (1H, m), 2.61-2.64 (2H, m), 3.15 (1H, dd, J=9.8, 13.3 Hz), 3.33-3.41 (2H, m), 3.47 (1H, dd, J=4.7, 9.8 Hz), 3.60-3.66 (2H, m), 4.08 (2H, t, J=14.5 Hz), 6.33 (1H, d, J=7.8 Hz), 7.33 (1H, d, J=7.8 Hz), 12.2 (1H, br).

### (156d) 3-amino-4-{3-[(2-cyanoethoxy)methyl]piperidin-1-yl}thieno[2,3-b]pyridine-2-carboxamide

The reaction was performed following a method described in Example 151 (151e) using 4-(3-[(2-cyanoethoxy)methyl]piperazin-1-yl)-2-thioxo-1,-dihydropyridine-3-carbonitrile which was produced in Example 156 (156c) and the title compound was obtained (42.8 mg, yield 3.6% from 3-(piperidin-3-ylmethoxy)propanenitrile) as a yellow solid.
Mp 155-156°C;
IR (KBr) νₘₐₓ 3439, 3330, 3169, 2932, 2250, 1649, 1580, 1501, 1371, 1346 cm⁻¹;
¹H NMR(CDCl₃, 400MHz) :δ 1.11-1.26 (1H, m), 1.72-1.97 (3H, m), 2.12 (1H, br s), 2.48 (1H, t, J=11.2 Hz), 2.54-2.64 (1H, m), 2.59 (2H, t, J=6.1 Hz), 3.34-3.50 (4H, m), 3.60-3.69 (2H, m), 5.46 (2H, br s), 6.87 (1H, d, J=4.9 Hz), 6.96 (2H, br s), 8.44 (1H, d, J=4.9 Hz);
MS(FAB) m/z: 360 [M + H]⁺;
Anal. calcd for C₁₇H₂₁N₅O₂S·1/2H₂O : C,55.42; H,6.02; N,19.01; S,8.70. Found C,55.44; H,6.00; N,18.88, S,8.48.

### (Example 157) 3-amino-4-(3-{[3-(dimethylamino)propoxy]methyl}piperidin-1-yl)thieno[2,3-b]pyridine-2-carboxamide (Exemplified Compound No. 1-144)

### (157a) benzyl 3-[(3-aminopropoxy)methyl]piperidine-1-carboxylate

benzyl 3-[(2-cyanoethoxy)methyl]piperidine-1-carboxylate (2.06 g, 6.81 mmol) which was produced in Example 156 (156a) was dissolved in methanol (30 mL) and the resultant solution was blended with cobalt (II) chloride (excess amount) and sodium borohydride (excess amount) divided into several times and the mixture was stirred under nitrogen atmosphere at 0°C for 3.5 hours. The reaction liquid was blended with water and methylene chloride and partitioned. The obtained organic layer was dried over anhydrous sodium sulfate and the solvent was evaporated under reduced pressure. The residue was purified by silica gel column chromatography (hexane:ethyl acetate =1:1 to 0:1) and the title compound was obtained (1.31 g, yield 63%) as an oil.
IR (Liquid film) νₘₐₓ 2935, 2858, 1699, 1470, 1432, 1261, 1236, 1154, 1116, 699 cm⁻¹;
¹H NMR(CD₃OD, 400MHz) :δ 1.20-1.32 (1H, m), 1.38-1.51 (1H, m), 1.62-1.81 (5H, m), 2.55-2.83 (3H, m), 2.92 (1H, br s), 3.20-3.33 (2H, m), 3.38-3.51 (2H, m), 3.91 (1H, d, J=13.3 Hz), 4.04 (1H, d, J=12.5 Hz), 5.09 (2H, s), 7.26-7.33 (5H, m); MS(FAB) m/z: 307 [M + H]⁺.

### (157b) benzyl 3-{[3-(dimethylamino)propoxy]methyl}piperidine-1-carboxylate

benzyl 3-[(3-amino propoxy)methyl]piperidine-1-carboxylate (890 mg, 2.90 mmol) which was produced in Example 157 (157a) was mixed with formic acid (5 mL) and formaldehyde (5 mL) and the mixture was stirred under nitrogen atmosphere at 100°C for two hours. After the reaction liquid was diluted with ethyl acetate and washed with water, it was washed with a saturated saline solution and dried over anhydrous sodium sulfate, and the solvent was evaporated under reduced pressure and the title compound was obtained (933 mg, 96%) as an oil.
IR (Liquid film) vₘₐₓ 2939, 2858, 1700, 1469, 1432, 1366, 1261, 1236, 1154, 1117 cm⁻¹;
¹H NMR(CD₃OD, 400MHz) :δ 1.19-1.30 (1H, m), 1.38-1.50 (1H, m), 1.62-1.81 (5H, m), 2.15-2.27 (6H, br), 2.31-2.45 (2H, m), 2.60-2.84 (2H, br), 2.91 (1H, t, J=12.3 Hz), 3.20-3.28 (1H, m), 3.34-3.46 (2H, m), 3.93 (1H, br s), 4.03-4.09 (1H, m), 5.09 (2H, s), 7.25-7.34 (5H, m);
MS(FAB) m/z: 335 [M + H]⁺.

### (157c) N,N-dimethyl-N-[3-(piperidin-3-yhnethoxy)propyl]amine

Hydrogenolysis reaction was performed using benzyl 3-{[3-(dimethylamino)propoxy]methyl}piperidine-1-carboxylate (926 mg, 2.79 mmol) which was produced in Example 157 (157b) in the presence of a palladium-carbon catalyst in ethanol and the title compound was obtained (535 mg, yield 96%) as an oil.
IR (Liquid film) νₘₐₓ 3400, 2936, 2858, 2796, 1655, 1544, 1468, 1273, 1164, 1115 cm⁻¹;
¹H NMR(CD₃OD, 400MHz) :δ 1.10-1.21 (1H, m), 1.6-1.58 (1H, m), 1.66-1.83 (5H, m), 2.24 (6H, s), 2.33 (1H, dd, J=10.6, 12.1 Hz), 2.35-2.42 (2H, m), 2.53 (1H, dt, J=3.1, 12.1 Hz), 2.98 (1H, d, J=13.3 Hz), 3.09 (1H, d, J=12.1 Hz), 3.20-3.30 (2H, m), 3.39-3.47 (2H, m);
MS(EI) m/z: 200 [M⁺]. (157d) 4-(3-([3-(dimethylamino)propoxy]methyl)piperidin-1-yl)-2-thioxo-1,2-dihydropyridine-3-carbonitrile

The reaction was performed following a method described in Example 151 (151d) using N,N-dimethyl-N-[3-(piperidin-3-ylmethoxy)propyl]amine (520 mg, 2.60 mmol) which was produced in Example 157 (157c) and an oil (78.7 mg) mainly containing the title compound was obtained.
¹H NMR(CDCl₃, 400MHz): δ 1.34-1.42 (1H, m), 1.70-1.90 (6H, m), 2.23 (3H, s), 2.25 (3H, s), 2.38 (2H, t, J=7.4), 3.08 (1H, dd, J=9.8, 13.1 Hz), 3.23-3.48 (5H, m), 4.02-4.14 (2H, m), 6.30 (1H, d, J=7.4 Hz), 7.34 (1H, d, J=7.4 Hz), 7.86 (1H, br).

### (157e) 3-amino-4-(3-{[3-(dimethylamino)propoxy]methyl}piperidin-1-yl)thieno[2,3-b]pyridine-2-carboxamide

The reaction was performed following a method described in Example 151 (151e) using 4-(3-([3-(dimethylamino)propoxy]methyl)piperidin-1-yl)-2-thioxo-1,2-dihydropyridine-3-carbonitrile which was produced in Example 157 (157d) and the title compound was obtained (44.1 mg, yield 4.3% from N,N-dimethyl-N-[3-(piperidin-3-ylmethoxy)propyl]amine) as a yellow solid.
Mp 174°C;
IR (KBr) νₘₐₓ 3450, 3311, 3167, 2936, 2858, 1649, 1579, 1504, 1368, 1345 cm⁻¹;
¹H NMR(CDCl₃, 500MHz) :δ 1.08-1.20 (1H, m), 1.70-1.86 (3H, m), 1.86-1.95 (2H, m), 2.10 (1H, br s), 2.21 (6H, s), 2.32 (2H, t, J=7.3 Hz), 2.46 (1H, t, J=10.7 Hz), 2.59 (1H, t, J=11.2 Hz), 3.27-3.39 (2H, m), 3.41-3.50 (4H, m), 5.30 (2H, br s), 6.90 (1H, d, J=5.4 Hz), 7.01 (2H, br s), 8.46 (1H, d, J=5.4 Hz);
MS(FAB) m/z: 392 [M + H]⁺.

### (Example 158) 3-amino-4-{3-[(cyanomethoxy)methyl]piperidin-1-yl}thieno[2,3-b]pyridine-2-carboxamide (Exemplified Compound No. 1-149)

### (158) benzyl 3-[(cyanomethoxy)methyl]piperidine-1-carboxylate

benzyl 3-(hydroxymethyl)piperidine-1-carboxylate (Arch. Pharm. (Weinheim Ger.) 323, 1990, 9-12) (1.01 g, 4.05 mmol) was dissolved in tetrahydrofuran (10 mL), and the resultant solution was blended with sodium hydride (55% oily, 216 mg, 4.95 mmol) under ice-cooling and the mixture was stirred under nitrogen atmosphere at room temperature for 30 minutes. A tetrahydrofuran (7 mL) solution of brombacetonitrile (0.38 mL, 5.67 mmol) was added dropwise to the reaction liquid for more than two hours. The reaction liquid was blended with water after stirring at room temperature for two hours, and the mixture was extracted with ethyl acetate. The extract was washed with a saturated saline solution and dried over anhydrous sodium sulfate and the solvent was evaporated under reduced pressure. The residue was purified by silica gel column chromatography (hexane:ethyl acetate =5:1 to 4:1) and the title compound was obtained (655 mg, yield 56%) as an oil.
IR (Liquid film) νₘₐₓ 3469, 2939, 2862, 1698, 1471, 1434, 1263, 1236, 1155, 1110 cm⁻¹;
¹H NMR(CDC1₃, 400MHz) :δ 1.23-1.33 (1H, m), 1.41-1.54 (1H, m), 1.62-1.71 (1H, m), 1.75-1.90 (2H, m), 2.90-2.97 (1H, m), 2.69-2.87 (1H, br), 3.38-3.50 (2H, m), 3.94-4.10 (1H, br), 3.93 (1H, dt, J=4.0, 13.4 Hz), 4.18 (2H, br s), 5.07-5.18 (2H, m), 7.27-7.37 (5H, m);
MS(EI) m/z: 289 [M + H]⁺.

### (158b) (piperidin-3-ylmethoxy)acetonitrile

benzyl 3-[(cyanomethoxy)methyl]piperidine-1-carboxylate (732 mg, 2.54 mmol) which was produced in Example 158 (158a) was dissolved in ethanol (4 mL), and the resultant solution was blended with ammonium acetate (106 mg, 1.38 mmol) and 10% palladium-carbon (90 mg) and the mixture was stirred at room temperature under normal pressure hydrogen atmosphere for 1.5 hours. The reaction liquid was filtered with Celite after the reaction terminated and the solvent was evaporated under reduced pressure. The residue was dissolved in methylene chloride, washed with 1N aqueous solution of sodium hydroxide and then with a saturated saline solution and dried over anhydrous sodium sulfate, the solvent was evaporated under reduced pressure, and the title compound was obtained (294 mg, yield 75%) as an oil. IR (Liquid film) νₘₐₓ 3323, 2929, 2856, 2810, 2742, 1470, 1443, 1357, 1271, 1106 cm⁻¹;
¹H NMR(CDCl₃, 400MHz) :δ 1.10-1.20 (1H, m), 1.41-1.52 (1H, m), 1.62-1.72 (2H, m), 1.74-1.85 (2H, m), 2.36 (1H, dd, J=9.8, 11.7 Hz), 2.55 (1H, dt, J=3.1, 11.7 Hz), 2.99 (1H, dt, J=3.5, 12.0 Hz), 3.08-3.12 (1H, m), 3.38-3.45 (2H, m), 4.22 (2H, s); MS(FAB) m/z: 155 [M + H]⁺.

### (158c) 4-{3-[(cyanomethoxy)methyl]piperidin-1-yl}-2-thioxo-1,2-dihydropyridine-3-carbonitrile

The reaction was performed following a method described in Example 151 (151d) using (piperidin-3-ylmethoxy)acetonitrile (292 mg, 1.89 mmol) which was produced in Example 158 (158b) and an oil (111 mg) mainly containing the title compound was obtained.
¹H NMR(CDCl₃, 500MHz) :δ 1.32-1.42 (1H, m), 1.70-1.93 (4H, m), 3.42-3.59 (4H, m), 4.08-4.15 (2H, m), 4.29 (2H, s), 6.29 (1H, d, J=7.5 Hz), 7.36 (1H, d, J=7.5 Hz), 12.2 (1H, br).

### (158d) 3-amino-4-{3-[(cyanomethoxy)methyl]piperidin-1-yl}thieno[2,3-b]pyridine-2-carboxamide

The reaction was performed following a method described in Example 151 (151e) using 4-{3-[(cyanomethoxy)methyl]piperidin-1-yl}-2-thioxo-1,2-dihydropyridine-3-carbonitrile which was produced in Example 158 (158c) and the title compound was obtained (11.4 mg, yield 1.7% from (piperidin-3-ylmethoxy)acetonitrile) as a yellow solid.
Mp 217-218°C;
IR (KBr) νₘₐₓ 3426, 3328, 3156, 2932, 1649, 1581, 1504, 1371, 1346, 1111 cm⁻¹;
¹H NMR(CDCl₃, 400MHz) :δ 1.13-1.25 (1H, m), 1.75-1.87 (1H, m), 1.88-1.96 (2H, m), 2.11-2.22 (1H, br), 2.47 (1H, t, J=11.0 Hz), 2.61 (1H, t, J=11.0 Hz), 3.40-3.58 (4H, m), 4.24 (2H, s), 5.28 (2H, br s), 6.88 (1H, d, J=5.5 Hz), 6.96 (2H, br s), 8.45 (1H, d, J=5.5 Hz);
MS(FAB) m/z: 346 [M + H]⁺;
Anal. calcd for C₁₆H₁₉N₅O₂S·7/10H₂O : C,53.68; H,5.74; N,19.56; S,8.96. Found C,53.67; H,5.56; N,19.34, S,8.96.

### (Example 159) 3-amino-4-{3-[(2-hydroxyethoxy)methyl]piperidin-1-yl}thieno[2,3-b]pyridine-2-carboxamide (Exemplified Compound No. 1-99)

### (159a) benzyl 3-[(2-tert-butoxy-2-oxoethoxy)hydroxymethyl]piperidine-1-carboxylate

50% aqueous solution of sodium hydroxide (18 mL) divided into several times was added to a benzene (18 mL) suspension of benzyl 3-(hydroxymethyl)piperidine-1-carboxylate (Arch. Pharm. (Weinheim Ger.), 323, 1990, 9-12) (11.97 g, 48.01 mmol), tert-butyl bromoacetate (16.3 mL, 110 mmol) and tetra-N-butylammonium bisulfate (3.8 g, 11 mmol) under ice-cooling. The reaction mixture was stirred at 0°C for 15 minutes and stirred further overnight at room temperature and the resultant solution was blended with ethyl acetate (30 mL) and partitioned. The organic layer was washed with a saturated saline solution (3x60 mL) and after drying over sodium sulfate, it was filtered and concentrated. The obtained residue was purified by silica gel column chromatography (hexane: ethyl acetate =5:1 to 4:1) and the title compound was obtained (16.88 g, yield 96%). Colourless liquid
IR (film) νₘₐₓ 3033, 2935, 2858, 1747, 1701, 1431, 1368, 1260, 1232, 1138, 979, 850, 734, 699 cm⁻¹;
¹H NMR (CDCl₆, 400 MHz) δ 1.28 (1H, br s), 1.47 (10H, s), 1.67 (1H, br s), 1.84 (2H, br s), 2.76 (1H, br s), 2.88 (1H, t, J = 10.3 Hz), 3.30-3.44 (2H, br m), 3.92 (2H, s), 3.98 (1H, br s), 4.10 (1H, br s), 5.13 (2H, s), 7.27-7.39 (5H, m);
HRMS m/z: found [M + H]⁺ 364.2111, calcd for C₂₀H₃₀NO₅ [M + H]⁺ 364.2124.

### (159b) ({1-[(benzyloxy)carbonyl]piperidin-3-yl}methoxy)acetic acid

A methylene chloride solution (30 mL) of benzyl 3-[(2-tert-butoxy-2-oxoethoxy)hydroxymethyl]piperidine-1-carboxylate (5.04 g, 13.9 mmol) which was produced in Example 159 (159a) was ice-cooled and blended with trifluoroacetic acid (10 mL). The reaction mixture was stirred at 0°C for 15 minutes and after further stirring overnight at room temperature, it was concentrated. The obtained residue was purified by silica gel column chromatography (hexane:ethyl acetate =6:4 to 1:5) and the title compound was obtained (3.86 g, yield 91 %).
Colourless liquid
IR (film) νₘₐₓ 3065, 2937, 1698, 1441, 1263, 1139, 977, 699 cm⁻¹;
¹H NMR(CDCl₃, 400 MHz) δ 1.24-1.40 (1H, m), 1.41-1.56 (1H, m), 1.57-1.72 (1H, m), 1.73-2.00 (2H, m), 2.78-3.30 (2H, m), 3.31-3.48 (2H, m), 3.57-4.15 (4H, m), 5.13 (2H, br s), 7.27-7.40 (5H, m);
MS (FAB) m/z: 308 [M+H]⁺, 264, 246, 176.

### (159c) benzyl 3-[(2-hydroxymethoxy)methyl]piperidine-1-carboxylate

Boron trifluoride ether complex (3.5 mL, 27.7 mmol) was slowly added dropwise to a tetrahydrofuran (20 mL) suspension of sodium borohydride (0.785 g, 20.8 mmol) under ice-cooling and the mixture was stirred under nitrogen atmosphere for 30 minutes. A tetrahydrofuran (22 mL) solution of ({1-[(benzyloxy)carbonyl]piperidin-3-yl}methoxy)acetic acid (5.317 g, 17.3 mmol) which was produced in Example 159 (159b) was added dropwise to the reaction mixture and after it was stirred at 0°C for 3 hours, it was blended with methanol (5 mL). Insolubles were filtered by Celite and washed with ethyl acetate (100 mL). The crude product which was obtained by concentrating the filtrate was purified by silica gel column chromatography (hexane:ethyl acetate =6: 4 to 100% ethyl acetate) and the title compound was obtained (4.546 g, yield 90%).
Colourless liquid
IR (film) νₘₐₓ 3446, 2933, 1698, 1435, 1261, 1154, 1072, 699 cm⁻¹;
¹H NMR(CDCl₃, 400 MHz) δ 1.20-1.34 (1H, m), 1.40-1.55 (1H, m), 1.57-1.72 (1H, m), 1.74-1.94 (2H, m), 2.75-3.15 (2H, m), 3.29-3.37 (2H, m), 3.42-3.60 (2H, m), 3.64-4.03 (4H, m), 5.12 (2H, br s), 7.25-7.37 (5H, m);
MS (FAB) m/z: 294 [M+H]⁺, 250, 246, 226, 165.

### (159d) 2-(piperidin-3-ylmethoxy)ethanol

An ethanol (7 mL) solution of benzyl 3-[(2-hydroxymethoxy)methyl]piperidine-1-carboxylate (1.767 g, 6.02 mmol) which was produced in Example 159 (159c) was blended with 10% palladium-carbon catalyst (150 mg) under nitrogen atmosphere and the mixture was stirred under normal pressure hydrogen atmosphere for four hours. After the reaction terminated, the crude product which was obtained by removing the catalyst by Celite filtration and evaporating the solvent was purified by silica gel column chromatography (Chromatorex, (NH), Fuji Silysia Chemical LTD.) (100% ethyl acetate) and the title compound was obtained (836 mg, yield 87%).
Colourless liquid
¹H NMR(CDCl₆, 400 MHz) δ 1.05-1.19 (1H, m), 1.39-1.51 (1H, m), 1.61-1.70 (1H, m), 1.73-1.86 (2H, m), 2.11 (2H, br s), 2.37 (2H, t, J = 10.2 Hz), 2.57 (2H, t, J = 11.7 Hz), 2.99 (2H, d, J = 12.1 Hz), 3.12 (2H, d, J = 12.1 Hz), 3.325 (2H, d, J = 6.3 Hz), 3.46-3.58 (2H, m), 3.69-3.77 (2H, m).

### (159e) 4- {3-[(2-hydroxyethoxy)methyl]piperidin-1-yl}-2-thioxo-1,2-dihydropyridine-3-carbonitrile

2-(piperidin-3-ylmethoxy)ethanol (749 mg, 4.70 mmol) which was produced in Example 159 (159d) and (2Z)-2-cyano-3-ethoxybut-2-enethioamide (J. Org. Chem., (1962), 27,2433-2439) (780 mg, 4.58 mmol) were dissolved in N,N-dimethylformamide (8 mL) and the mixture was stirred at room temperature for two hours. Furthermore, N,N-dimethylformamide dimethylacetal (0.73 mL, 5.50 mmol) was added to the reaction liquid and the mixture was stirred at room temperature overnight. The residue which was obtained by concentrating the reaction mixture was purified by silica gel column chromatography (methylene chloride: methanol =98:2 to 85:15) and thin layer column chromatography (methylene chloride:methanol =98:2) and the title compound was obtained (283 mg). Yield 21%.
Amorphous
IR (film) νₘₐₓ 3193, 3148, 3043, 2932, 2859, 2207, 1621, 1571, 1448, 1361, 1249, 1167, 1121, 1059, 781, 599 cm⁻¹;
¹H NMR(CDCl₆, 400 MHz) δ 1.27-1.41 (1H, m), 1.62-1.76 (1H, m), 1.86 (2H, br d, J = 9 Hz), 2.08 (1H, br s), 2.70-2.98 (1H, br s), 3.065 (1H, dd, J = 9.8, 13.3 Hz), 3.24-3.38 (2H, m), 3.445 (1H, dd, J = 4.7, 9.8 Hz), 3.48-3.60 (3H, m), 3.74 (2H, t, J = 4.7 Hz), 4.03 (2H, br d, J = 13.7 Hz), 4.325 (2H, br d, J = 13.7 Hz), 6.36 (1H, d, J = 7.4 Hz), 7.395 (1H, d, J = 7.4 Hz);
HRMS m/z: found [M + H]⁺ 294.1254, calcd for C₁₄H₂₀N₃O₂S [M + H]⁺ 294.1276.

### (159f) 3-amino-4-{3-[(2-hydroxyethoxy)methyl]piperidin-1-yl}thieno[2,3-b]pyridine-2-carboxamide

The reaction was performed following a method described in Example 5 (5c) using 4-{3-[(2-hydroxyethoxy)methyl]piperidin-1-yl}-2-thioxo-1,2-dihydropyridine-3-carbonitrile which was produced in Example 159 (159e) and the title compound was synthesized. Yield 55%.
Amorphous
IR (KBr) νₘₐₓ 3438, 3326, 3191, 3148, 2931, 2856, 1648, 1581, 1502, 1448, 1372, 1247, 1123, 1059, 960, 825, 737, 605 cm⁻¹;
¹H NMR(CDCl₆, 400 MHz) δ 1.07-1.22 (1H, m), 1.72-2.03 (4H, m), 2.15 (1H, br s), 2.46 (1H, br t, J = 10.2 Hz), 2.61 (1H, br t, J = 10.2 Hz), 3.31-3.59 (6H, m), 3.72 (2H, br s), 5.30 (2H, br s), 6.875 (1H, d, J = 5.4 Hz), 6.97 (2H, br s), 7.835 (1H, d, J = 5.4 Hz);
HRMS m/z: found [M + H]⁺ 351.1496, calcd for C₁₆H₂₃N₄O₃S [M + H]⁺ 351.1490.

### (Example 160) tert-butyl 2-({1-[3-amino-2-(aminocarbonyl)thieno[2,3-b]pyridin-4-yl]piperidin-3-yl}methoxy)ethylcarbamate (Exemplified Compound No. 1-182) (160a) benzyl 3-({2-[(methylsulfonyl)oxy]ethoxy}methyl)piperidine-1-carboxylate

A methylene chloride (50 mL) solution of triethylamine (1.70 mL, 12.1 mmol), methanesulfonyl chloride (0.87 mL, 11.2 mmol) and benzyl 3-[(2-hydroxy methoxy)methyl]piperidine-1-carboxylate (2.74 g, 9.34 mmol) which was produced in Example 159 (159c) was stirred overnight under nitrogen atmosphere. After having added a saturated saline solution (15 mL), extraction of the reaction liquid was performed. The extract was dried over sodium sulfate and the crude product which was obtained by evaporating the solvent after filtration was purified by silica gel column chromatography (hexane:ethyl acetate = 4:1 to 2:1) and the title compound was obtained (3.25 g, yield 94%).
Pale yellow liquid
IR (film) νₘₐₓ 2937, 1697, 1433, 1354, 1236, 1175, 1019, 922, 807 cm⁻¹;
¹H NNR(CDCl₃, 500 MHz) δ 1.16-1.28 (1H, m), 1.41-1.55 (1H, m), 1.62-1.73 (1H, m), 1.74-1.87 (2H, m), 2.62-2.82 (1H, m), 2.83-3.13 (4H, m), 3.27-3.41 (2H, m), 3.64 (2H, br s), 3.93-4.17 (2H, m), 4.31 (2H, br s), 5.07-5.18 (2H, m), 7.28-7.41 (5H, m);
HRMS m/z: found [M + H]⁺ 372.1448, calcd for C₁₇H₂₆N₁O₆S [M + H]⁺ 372.1480.

### (160b) benzyl 3-[(2-azidoethoxy)methyl]piperidine-1-carboxylate

N,N-dimethylformamide (10 mL) solution of sodium azide (723 mg, 11.12 mmol) and benzyl 3-({2-[(methylsulfonyl)oxy]ethoxy}methyl)piperidine-1-carboxylate (3.178 g, 8.56 mmol) which was produced in Example 160 (160a) was heated and the mixture was stirred at 80°C for six hours. The reaction mixture was blended with ethyl acetate (50 mL) and water (20 mL) and partitioned. The organic layer was washed with a saturated saline solution (2x15 mL) and dried over sodium sulfate, and the crude product which was obtained by evaporating the solvent after filtration was purified by silica gel column chromatography (hexane:ethyl acetate =7:3) and the title compound was obtained (2.721 g, yield 100%).
Pale yellow liquid
IR (film) νₘₐₓ 2934, 2103, 1699, 1432, 1235, 1153, 699 cm⁻¹;
¹H NMR(CDCl₃, 500 MHz) δ 1.22-1.34 (1H, m), 1.40-1.54 (1H, m), 1.62-1.72 (1H, m), 1.74-1.89 (2H, m), 2.64-2.95 (2H, m), 3.26-3.41 (4H, m), 3.53-3.63 (2H, m), 3.94-4.15 (2H, m), 5.07-5.19 (2H, m), 7.28-7.39 (5H, m); MS (FAB) m/z: 319 [M+H]⁺, 275, 242, 211, 183, 91, 63.

### (160c) benzyl 3-({2-[(tert-butoxycarbonyl)amino]ethoxy}methyl)piperidine-1-carboxylate

A tetrahydrofuran (8 mL) solution of benzyl 3-[(2-azidoethoxy)methyl]piperidine-1-carboxylate (2.635 g, 8.28 mmol) which was produced in Example 160 (160b) was blended with triphenylphosphine (2.280 g, 8.69 mmol) and the mixture was stirred at room temperature for 20 hours. The reaction liquid was blended with water (0.3 mL) and the mixture was stirred for 20 hours and then blended with di-tert-butyl dicarbonate (1.987 g, 9.10 mmol) and the mixture was stirred for a further 40 hours. The crude product which was obtained by concentrating the reaction mixture was purified by silica gel column chromatography (hexane:ethyl acetate =8:2) and the title compound was obtained (3.218 g, yield 99%).
Colourless oil
IR (film) νₘₐₓ 3355, 2933, 2861 , 1701, 1520, 1433, 1260, 1237, 1155, 1121, 985, 867, 764, 699 cm⁻¹;
¹H NMR(CDCl₃, 400 MHz) δ 1.17-1.30 (1H, m), 1.44 (10H, s), 1.58-1.70 (1H, m), 1.72-1.83 (1H, m), 2.75 (1H, br s), 2.92 (1H, t, J = 10.9 Hz), 3.28 (4H, d, J = 5.9 Hz), 3.37-3.48 (2H, m), 3.87-4.06 (2H, m), 4.68-5.05 (1H, m), 5.12 (2H; m), 7.25-7.37 (5H, m);
HRMS m/z: found [M + H]⁺ 393.2429, calcd for C₂₁H₂₉N₁₀O₂ [M + H]⁺ 393.2389.

### (160d) tert-butyl 2-(piperidin-3-ylmethoxy)ethyl carbamate

An ethanol (14 mL) solution of benzyl 3-({2-[(tert-butoxycarbonyl)amino]ethoxy}methyl)piperidine-1-carboxylate (3.158 g, 8.04 mmol) which was produced in Example 160 (160c) was blended with 10% palladium-carbon catalyst (257 mg) under nitrogen atmosphere and the mixture was stirred overnight under normal pressure hydrogen atmosphere. The catalyst was removed by Celite filtration and it was washed with ethanol. The crude product which was obtained by concentrating the filtrate was purified by silica gel column chromatography (Chromatorex (NH), Fuji Silysia Chemical LTD.) (100% ethyl acetate) and the title compound was obtained (2.018 g, yield 97%).
A white solid
IR (film) νₘₐₓ 3303, 3205, 2978, 2939, 2857, 2804, 1690, 1560, 1367, 1275, 1170, 1126, 979, 949, 869, 777, 608 cm⁻¹;
¹H NMR(CDC1₃, 400 MHz) δ 1.03-1.16 (1H, m), 1.44 (9H, s), 1.56-1.69 (2H, br s), 1.70-1.81 (2H, br s), 2.32 (1H, t, J = 10.9 Hz), 2.54 (1H, t, J = 10.9 Hz), 2.99 (1H, d, J = 12.2 Hz), 3.10 (1H, d, J = 12.2 Hz), 3.20-3.32 (4H, m), 3.43 (2H, br s), 4.86 (1H, br s);
HRMS m/z: found [M + H]⁺ 259.1998, calcd for C₁₃H₂₇N₂O₃ [M + H]⁺ 259.2021.

### (160e) tert-butyl 2-(piperidin-3-ylmethoxy)ethylcarbamate

The reaction was performed following a method described in Example 146 (146c) using tert-butyl [2-(piperidin-3-ylmethoxy)ethyl]carbamate which was produced in Example 160 (160d) and the title compound was obtained. Yield 44%. Pale yellowish white amorphous
IR (KBr) νₘₐₓ 3344, 3190, 3130, 2974, 2933, 2861, 2208, 1703,1620, 1515, 1450, 1365, 1302, 1249, 1170, 1167, 1120, 999, 964, 856, 781 cm⁻¹ ;
¹H NMR(CDCl₃, 400 MHz) δ 1.30-1.40 (1H, m), 1.44 (9H, s), 1.63-1.74 (1H, br s), 1.80-1.91 (2H, br d), 2.02 (1H, br s), 3.00-3.10 (1H, m), 3.22-3.33 (4H, m), 3.38-3.50 (3H, m), 4.03-4.17 (2H, m), 4.89 (1H, br s), 6.28 (1H, d, J = 7.0 Hz), 7.28 (1H, d, J = 7.0 Hz);
HRMS m/z: found [M + H]⁺ 393.1998, calcd for C₁₉H₂₉N₄O₃S [M + H]⁺ 393.1960.

### (160f) tert-butyl 2-({1-[3-amino-2-(aminocarbonyl)thieno[2,3-b]pyridin-4-yl]piperidin-3-yl}methoxy)ethylcarbamate

The reaction was performed following a method described in Example 5 (5c) using tert-butyl 2-(piperidin-3-ylmethoxy)ethylcarbamate which was produced in Example 160 (160e) and the title compound was synthesized. Yield 79%.
Pale yellow amorphous
IR (KBr) νₘₐₓ 3440, 3326, 3186, 2973, 2931, 2859, 1706, 1648, 1581, 1502, 1451, 1367, 1248, 1169, 1122, 1056, 995, 961, 865, 736, 557 cm⁻¹;
¹H NMR(CDCl₆, 400 MHz) δ 1.05-1..22 (1H, m), 1.42 (9H, s), 1.71-1.95 (3H, m), 2.10 (1H, br s), 2.46 (1H, br t, J = 10.2 Hz), 2.59 (1H, br t, J = 10.2 Hz), 3.21-3.55 (8H, m), 4.82 (1H, br s), 5.33 (2H, br s), 6.90 (1H, d, J = 5.5 Hz), 6.99 (2H, br s), 8.46 (1H, d, J = 5.5 Hz);
HRMS m/z: found [M + H]⁺ 450.2160, calcd for C₂₁H₃₂N₅O₄S [M + H]⁺ 450.2175.

### (Example 161) 3-amino-4-{3-[(2-aminoethoxy)methyl]piperidin-1-yl}thieno[2,3-b]pyridine-2-carboxamide dihydrochloride (Exemplified Compound No. 1-133)

tert-butyl 2-({1-[3-amino-2-(aminocarbonyl)thieno[2,3-b]pyridin-4-yl]piperidin-3-yl}methoxy)ethylcarbamate (259 mg, 0.58 mmol) which was produced in Example 160 was suspended in 1,4-dioxane (4 mL) and was blended with 4N hydrochloric acid-ethyl acetate (3 mL) and the mixture was stirred overnight. The solvent was evaporated and the obtained yellow solid was dried under reduced pressure and the title compound was obtained.
IR (KBr) νₘₐₓ 3309, 3169, 2967, 2932, 2859, 1649, 1513, 1482, 1385, 1257, 1107, 1044, 963, 803, 729, 555, 476 cm⁻¹;
¹H NMR(CD₃OD, 400 MHz) δ 1.31-1.46 (1H, m), 1.75-1.95 (3H, m), 2.20 (1H, br s), 3.02-3.18 (3H, m), 3.20-3.31 (1H, m), 3.38-3.50 (2H, m), 3.57-3.70 (2H, m), 3.92 (1H, br d, J = 12.5 Hz), 4.04 (1H, br d, J = 12.5 Hz), 7.23 (1H, d, J = 7.0 Hz), 8.35 (1H, d, J = 7.0 Hz);
HRMS m/z: found 350.1657 [M - 2HCl + H]⁺, calcd for C₁₆H₂₄N₅O₂S [M - 2HCl + H]⁺ 350.1650.

### (Example 162) 3-amino-4-(3-{[2-(dimethylamino)-2-oxoethoxy]methyl}piperidin-1-yl)thieno[2,3-b]pyridine-2-carboxamide (Exemplified Compound No. 1-155)

### (162a) benzyl 3-{2-[(dimethylamino)-2-oxoethoxy]methyl}piperidine-1-carboxylate

A methylene chloride (25 mL) solution of ({1-[(benzyloxy)carbonyl]piperidin-3-yl}methoxy)acetic acid (1.062 g, 3.45 mmol) which was produced in Example 159 (159b) was blended with triethylamine (0.53 mL, 3.8 mmol) and ethyl chloroformate (0.36 mL, 3.8 mmol) under ice-cooling and the mixture was stirred for one hour. Furthermore, triethylamine (0.6 mL, 4.32 mmol) and dimethylamine hydrochloride (352 mg, 3.8 mmol) were added to the reaction liquid and it was stirred for one hour and then stirred overnight at room temperature. A saturated saline solution (15 mL) was added to the reaction mixture, and extraction was performed and the extracted organic layer was dried over sodium sulfate and the crude product which was obtained by concentrating the organic layer after filtration was purified by silica gel column chromatography (hexane:ethyl acetate =1:1) and the title compound was obtained (961 mg, yield 83%).
Pale yellow liquid
IR (film) νₘₐₓ 3567, 3495, 2934, 2858, 1698, 1664, 1498, 1432, 1260, 1235, 1152, 1113, 980, 764, 700 cm⁻¹;
¹H NMR(CDCl₃, 400 MHz) δ 1.17-1.32 (1H, m), 1.46 (1H, br s), 1.60-1.72 (1H, m), 1.75-1.91 (2H, m), 2.72 (1H, br s), 2.81-3.09 (7H, m), 3.27-3.44 (2H, m), 3.86-4.13 (4H, m), 4.68-5.05 (2H, m), 5.04-5.16 (2H, m), 7.25-7.37 (5H, m);
HRMS m/z: found [M + H]⁺ 335.1964, calcd for C₁₇H₂₈N₂O₄ [M + H]⁺ 335.1970.

### (162b) N,N-dimethyl-2-(piperidin-3-ylmethoxy) acetamide

An ethanol (7 mL) solution of benzyl 3-{2-[(dimethylamino)-2-oxoethoxy]methyl}piperidine-1-carboxylate (936 mg, 2.80 mmol) which was produced in Example 162 (162a) was blended with 10% palladium-carbon (102.2 mg) and the mixture was stirred overnight under normal pressure hydrogen atmosphere. Palladium was removed by Celite filtration and it was washed with ethanol. The crude product which was obtained after concentration of the filtrate was purified by silica gel column chromatography (Chromatorex, (NH), Fuji Silysia Chemical LTD.) (100% ethyl acetate) and the title compound was obtained (506 mg, yield 90%).
Pale yellow liquid
IR (film) νₘₐₓ 3460, 3309, 2930, 2854, 1651, 1505, 1447, 1404, 1346, 1265, 1109, 1023, 886, 859, 806, 709, 574 cm⁻¹;
¹H NMR(CDCl₃, 400 MHz) δ 1.07-1.17 (1H, m), 1.38-1.51 (1H, m), 1.61-1.80 (1H, m), 1.70-1.86 (2H, m), 2.36 (1H, dd, J = 9.8, 11.7 Hz), 2.54 (1H, dt, J = 2.7, 11.7 Hz), 2.94 (3H, s), 2.97 (1H, br s), 3.01 (3H, s), 3.14 (1H, br d, J = 12.1 Hz), 3.29-3.36 (2H, m), 4.10 (2H, s);
HRMS m/z: found [M + H]⁺ 201.1611, calcd for C₁₀H₂₁N₂O₂ [M + H]⁺ 201.2890.

### (162c) 2-{[1-(3-cyano-2-thioxo-1,2-dihydropyridin-4-yl)piperidin-3-yl]methoxy}-N,N-dimethylacetamide

N,N-dimethyl-2-(piperidin-3-ylmethoxy)acetamide which was produced in Example 162 (162b) (5.695 g, 28.44 mmol) and (2Z)-2-cyano-3-ethoxybut-2-enethioamide (J. Org. Chem., (1962), 27, 2433-2439) (4.825 g, 28.34 mmol) were suspended in ethanol (100 mL) and the mixture was stirred at room temperature for 13 hours. After the solvent was evaporated, N,N-dimethylformamide (60 mL) and N,N-dimethylformamide dimethylacetal (4.16 mL, 31.27 mmol) were added and the mixture was stirred overnight at room temperature. The crude product which was obtained by concentrating the reaction mixture was purified by silica gel column chromatography (methylene chloride:methanol =98:2 to 85:15) and the title compound was obtained (2.455 g). Yield 26%.
Light greenish white amorphous
IR (KBr) νₘₐₓ 3138, 3036, 2933, 2858, 2206, 1649,1618, 1511, 1446, 1300, 1244, 1170, 1164, 1113, 1004, 964, 855, 782 cm⁻¹;
¹H NMR(CDCl₃, 400 MHz) δ 1.29-1.43 (1H, m), 1.59-1.81 (3H, m), 1.99-2.12 (2H, m), 2.95 (3H, s), 2.97 (3H, s), 3.07-3.11 (1H, m), 3.21-3.31 (1H, m), 3.36-3.53 (2H, m), 4.05-4.18 (4H, m), 6.32 (1H, d, J = 7.4 Hz), 7.31 (1H, d, J = 7.4 Hz);
HRMS m/z: found [M + H]⁺ 335.1545, calcd for C₁₆H₂₃N₄O₂S [M + H]⁺ 335.1541.

### (162d) 3-amino-4-(3-{[2-(dimethylamino)-2-oxoethoxy]methyl}piperidin-1-yl)thieno[2,3-b]pyridine-2-carboxamide

The reaction was performed following a method described in Example 5 (5c) using 2-{[1-(3-cyano-2-thioxo-1,2-dihydropyridin-4-yl)piperidin-3-yl]methoxy}-N,N-dimethylacetamide which was produced in Example 162 (162c) and the title compound was synthesized. Yield 79%.
A pale brown solid
IR (KBr) νₘₐₓ 3436, 3324, 3187, 2930, 2855, 1646, 1579, 1501, 1466, 1371, 1354, 1248, 1118, 1058, 961, 825, 737, 475 cm⁻¹;
¹H NMR(CDCl₆, 400 MHz) δ 1.07-1.24 (1H, m), 1.68-1.95 (3H, m), 2.15 (1H, br s), 2.42-2.64 (2H, m), 2.93 (3H, s), 2.98 (3H, s), 3.32-3.57 (4H, m), 4.12 (2H, s), 5.27 (2H, br s), 6.90 (1H, d, J = 5.5 Hz), 6.87 (2H, br s), 8.43 (1H, d, J = 5.5 Hz);
HRMS m/z: found [M + H]⁺ 392.1754, calcd for C₁₈H₂₆N₅O₃S [M + H]⁺ 392.1756; Anal. Calcd for C₁₈H₂₅N₅O₃S·H₂O: C,52.79; H,6.65; N,17.10. Found: C,53.12; H,6.25; N,17.27.

### (Example 163) 3-amino-4-(3-{[2-(diethylamino)-2-oxoethoxy]methyl}piperidin-1-yl)thieno[2,3-b]pyridine-2-carboxamide (Exemplified Compound No. 1-164)

### (163a) benzyl 3-{2-[(diethylamino)-2-oxoethoxy]methyl}piperidine-1-carboxylate

A methylene chloride (30 mL) solution of ({1-[(benzyloxy)carbonyl]piperidin-3-yl}methoxy)acetic acid (920 mg, 2.99 mmol) which was produced in Example 159 (159b) was blended with triethylamine (0.44 mL, 3.14 mmol) and ethyl chloroformate (0.3 mL, 3.14 mmol) under ice-cooling. After the reaction liquid was stirred for one hour, diethylamine (0.61 mL, 5.98 mmol) was added to the reaction liquid and the mixture was stirred for 30 minutes and the mixture was stirred overnight at room temperature. A saturated saline solution (15 mL) was added to the reaction liquid, and extraction was performed and the extracted organic layer was dried over sodium sulfate and the crude product which was obtained by concentrating the organic layer after filtration was purified by silica gel column chromatography (hexane:ethyl acetate =1:1) and the title compound was obtained (958 mg, yield 88%).
Colourless liquid
IR (film) νₘₐₓ 3553, 3496, 2935, 2858, 1698, 1664, 1468, 1433, 1261, 1235, 1153, 1115, 983, 765, 699 cm⁻¹;
¹H NMR(CDCl₃, 400 MHz) δ 1.04-1.32 (7H, m), 1.46 (1H, br s), 1.58-1.74 (1H, m), 1.82 (2H, br s), 2.71 (1H, br s), 2.86 (1H, br t, J = 10.9 Hz), 3.16-3.46 (6H, m), 3.96 (1H, br s), 4.06 (3H, br s), 5.02-5.18 (2H, m), 7.22-7.37 (5H, m);
HRMS m/z: found [M + H]⁺ 363.2290, calcd for C₂₀H₃₁N₂O₄ [M + H]⁺ 363.2283.

### (163b) N,N-diethyl-2-(piperidin-3-ylmethoxy)acetamide

An ethanol (7 mL) solution of benzyl 3-{2-[(diethylamino)-2-oxoethoxy]methyl}piperidine-1-carboxylate (946 mg, 2.61 mmol) which was produced in Example 163 (163a) was blended with 10% palladium-carbon (110 mg) under nitrogen atmosphere and the mixture was stirred overnight under normal pressure hydrogen atmosphere. Palladium was removed by Celite filtration and it was washed with ethanol. The crude product which was obtained after concentration of the filtrate was purified by silica gel column chromatography (Chromatorex, (NH), Fuji Silysia Chemical LTD.) (100% ethyl acetate) and the title compound was obtained (549 mg, yield 92%).
Colourless liquid
IR (film) νₘₐₓ 3464, 3310, 2932, 2853, 1643, 1464, 1440, 1380, 1268, 1221, 1123, 1109, 1035, 858, 796, 590 cm⁻¹;
¹H NMR(CDCl₃, 400 MHz) δ 1.08-1.22 (7H, m), 1.38-1.51 (1H, m), 1.57-1.71 (2H, m), 1.75-1.88 (2H, m), 2.36 (1H, t, J = 11.7 Hz), 2.54 (1H, t, J = 11.7 Hz), 2.99 (1H, d, J = 11.7 Hz), 3.14 (1H, d, J = 11.7 Hz), 3.28-3.43 (6H, m), 4.10 (2H, s);
MS (EI) m/z: found [M + H]⁺ 229.19, calcd for C₁₂H₂₅N₂O₂ [M + H]⁺ 229.34.

### (163c) 2-{[1-(3-cyano-2-thioxo-1,2-dihydropyridin-4-yl)piperidin-3-yl]methoxy}-N,N-diethylacetamide

N,N-diethyl-2-(piperidin-3-ylmethoxy) acetamide which was produced in Example 163 (163b) (524 mg, 2.3 mmol) and (2Z)-2-cyano-3-ethoxybut-2-enethioamide (J. Org. Chem., (1962), 27,2433-2439) (389 mg, 2.3 mmol) were dissolved in N,N-dimethylformamide (100 mL) and the mixture was stirred at room temperature for 2.5 hours. The reaction liquid was blended with N,N-dimethylformamide dimethylacetal (0.365 mL, 2.8 mmol) and the mixture was stirred overnight at room temperature. The crude product which was obtained by concentrating the reaction mixture was purified by silica gel column chromatography (methylene chloride:methanol =98:2 to 85:15) and thin layer chromatography-(methylene chloride:methanol =95:5) and a mixture (84 mg) mainly containing the title compound was obtained.
¹H NMR(CDCl₃, 400 MHz) δ 1.15 (3H, t, J = 7.0 Hz), 1.21 (3H, t, J = 7.0 Hz), 1.26-1.54 (1H, m), 1.64-2.03 (3H, m), 2.03-2.06 (2H, m), 3.05-3.14 (1H, m), 3.23-3.58 (6H, m), 4.03-4.22 (4H, m), 6.3 (1H, d, J = 7.4 Hz), 7.38 (1H, d, J = 7.4 Hz).

### (163d) 3-amino-4-(3-{[2-(diethylamino)-2-oxoethoxy]methyl}piperidin-1-yl)thieno[2,3-b]pyridine-2-carboxamide

A mixture (84 mg) containing 2-{[1-(3-cyano-2-thioxo-1,2-dihydropyridin-4-yl)-piperidin-3-yl]methoxy}-N,N-diethylacetamide which was produced in Example 163 (163c) was mixed with 2-chloroacetamide (22 mg, 0.23 mmol), 8N aqueous solution of sodium hydroxide (1 mL) and N,N-dimethylformamide (1 mL) and the mixture was stirred for 3 hours. The reaction mixture was blended with ethyl acetate (25 mL) and a saturated saline solution (10 mL) and partitioned. The aqueous layer was extracted with ethyl acetate (15 mL) and after the combined organic layer was dried over sodium sulfate, it was filtered and concentrated. The obtained crude product was purified by thin layer chromatography (ethyl acetate:methanol =95:5) and the title compound was obtained (55 mg). Yield 5% from (2Z)-2-cyano-3-ethoxybut-2-enethioamide.
IR (KBr) νₘₐₓ 3425, 3327, 3178, 2967, 2933, 2857, 1644, 1579, 1501, 1458, 1371, 1354, 1247, 1122, 1108, 1059, 962, 823, 770, 596, 477 cm⁻¹;
¹H NMR(CDCl₆, 400 MHz) δ 1.05-1.21 (7H, m), 1.70-1.96 (3H, m), 2.14 (1H, br s), 2.43-2.64 (2H, m), 3.20-3.59 (8H, m), 4.10 (2H, s), 5.25 (2H, br s), 6.87 (1H, d, J = 5.1 Hz), 6.97 (2H, br s), 8.43 (1H, d, J = 5.1 Hz);
HRMS m/z: found [M + H]⁺ 420.2075, calcd for C₁₈H₂₆N₅O₃S [M + H]⁺ 420.2069.

### (Example 164) 4-(3-{[2-(acetylamino)ethoxy]methyl}piperidin-1-yl)-3-aminothieno[2,3-b]pyridine-2-carboxamide (Exemplified Compound No. 1-173)

A methylene chloride (2 mL) solution of 3-amino-4-{3-[(2-aminoethoxy)methyl]pipehdin-1-yl}thieno[2,3-b]pyridine-2-carboxamide dihydrochloride (38 mg, 0.089 mmol) which was produced in Example 161 was blended with triethylamine (46 µL, 0.331 mmol) and acetyl chloride (8 µL, 0.114 mmol) and the mixture was stirred overnight. The reaction liquid was blended with methylene chloride (10 mL) and a saturated saline solution (10 mL) and partitioned. The organic layer was dried over sodium sulfate and the crude product which was obtained by concentration after filtration was purified by thin layer chromatography (methylene chloride:methanol =96:4) and the title compound was obtained (29.3 mg, yield 84%).
Amorphous
IR (KBr) νₘₐₓ 3437, 3319, 3194, 2930, 2855, 1648, 1555, 1501, 1439, 1372, 1246, 1122, 1056, 996, 960, 824, 736, 475 cm⁻¹;
¹H NMR(CDCl₆, 400 MHz) δ 1.06-1.19 (1H, m), 1.64-2.01 (6H, m), 2.13 (1H, br s), 2.40 (1H, br t, J = 10.7 Hz), 2.64 (1H, br t, J = 10.7 Hz), 3.24-3.61 (8H, m), 5.43 (2H, br s), 5.90 (1H, br s), 6.90 (1H, d, J = 5.4 Hz), 6.99 (2H, br s), 8.47 (1H, d, J = 5.4 Hz);
HRMS m/z: found [M + H]⁺ 392.1769, calcd for C₁₈H₂₆N₅O₃S [M + H]⁺ 392.1756.

### (Example 165) 3-amino-4-(3-{[2-(hexanoylamino)ethoxy]methyl}piperidin-1-yl)thieno[2,3-b]pyridine-2-carboxamide (Exemplified Compound No. 1-181)

A methylene chloride (30 mL) solution of hexanoic acid (1.03 mL, 8.27 mmol), N-hydroxysuccinimide (1.0 g, 8.69 mmol) and 1-ethyl-3-(3-dimethylaminopropyl)-carbodiimide hydrochloride (1.66 g, 8.68 mmol) was stirred overnight at room temperature. A saturated saline solution (30 mL) was added to the reaction mixture and extraction was performed. After the extract was dried over sodium sulfate, it was filtered and concentrated and a crude product of active ester was obtained. A methylene chloride (2 mL) solution of the crude active ester (16.6 mg, 0.078 mmol) and 3-amino-4-{3-[(2-aminoethoxy)methyl]piperidin-1-yl}thieno[2,3-b]pyridine-2-carboxamide dihydrochloride (32.9 mg, 0.078 mmol) which was produced in Example 161 was blended with triethylamine (38 µL, 0.273 mmol) and the mixture was stirred overnight. The reaction mixutre was blended with methylene chloride (15 mL) and a saturated saline solution (5 mL) and partitioned. The organic layer was dried over sodium sulfate and the crude product which was obtained by concentration after filtration was purified by thin layer chromatography (ethylacetate:methanol =95:5) and the title compound was obtained (23.3 mg, yield 67%).
Amorphous
IR (KBr) vₘₐₓ 3440, 3321, 3191, 2929, 2858, 1645, 1579, 1502, 1457, 1373, 1346, 1247, 1122, 1057, 960, 824, 736, 557 cm⁻¹;
¹H NMR(CDCl₆, 400 MHz) δ 0.86 (3H, t, J = 7.0 Hz), 1.06-1.18 (1H, m), 1.20-1.33 (4H, m), 1.53-1.64 (2H, m), 1.72-1.96 (3H, m), 2.06-2.18 (3H, m), 2.41 (1H, br t, J = 10.7 Hz), 2.62 (1H, br t, J = 10.7 Hz), 3.25-3.56 (8H, m), 5.43 (2H, br s), 5.84 (1H, br s), 6.87 (1H, d, J = 5.1 Hz), 6.97 (2H, br s), 8.44 (1H, d, J = 5.1 Hz);
HRMS m/z: found [M + H]⁺ 448.2383, calcd for C₂₂H₃₄N₅O₃S [M + H]⁺ 448.2382.

### (Example 166) 3-amino-4-{3-[3-(2-hydroxyethoxy)propyl]piperidin-1-yl}thieno[2,3-b]pyridine-2-carboxamide (Exemplified Compound No. 1-101)

### (166a) benzyl 3-(3-hydroxypropyl)piperidine-1-carboxylate

3-piperidine-3-ylpropan-1-ol (9.86 g, 68.8 mmol) which was synthesized with reference to J. Org. Chem., 1964, 29, 1736-1738 and triethylamine (11.5 mL, 82.6 mmol) were dissolved in methylene chloride (69 mL), and a methylene chloride (35 mL) solution of benzyl chloroformate (10.8 mL, 75.7 mmol) was added dropwise under ice-cooling. After dropwise addition was completed, the reaction liquid was stirred at room temperature for two hours, and was blended with water (70 mL) and partitioned. The aqueous layer was extracted with methylene chloride (50 mLx2), and after the combined extract was dried over magnesium sulfate, the solvent was evaporated under reduced pressure. The residual substance was purified by silica gel column chromatography (hexane/ethyl acetate = 3:1 to 1/1) and 10.13 g of the title compound was obtained (yield 53%).
Slightly yellow oil
IR (film) νₘₐₓ 3445, 2935, 2857, 1699, 1435, 1261, 1238 cm⁻¹;
¹H NMR(CDCl₃, 400 MHz) δ 1.01-1.72 (8H, m), 1.75-1.90 (1H, m), 2.41-2.66 (1H, m), 2.76-2.91 (1H, m), 3.54-3.69 (2H, m), 3.87-4.12 (2H, m), 5.12 (2H, br.s), 7.26-7.42 (5H, m);
MS (FAB) m/z: 278 [M+H]⁺, 258, 242, 234.

### (166b) benzyl 3-[3-(2-tert-butoxy-2-oxoethoxy)propyl]piperidine-1-carboxylate

The reaction was performed following a method described in Example 159 (159a) using benzyl 3-(3-hydroxypropyl)piperidine-1-carboxylate which was produced in Example 166 (166a) in place of benzyl 3-(3-hydroxymethyl)piperidine-1-carboxylate and the title compound was obtained.
Colourless liquid
IR (film) νₘₐₓ 2936, 1749, 1701, 1431, 1237, 1136 cm⁻¹;
¹H NMR(CDCl₃, 400 MHz) δ 1.08-1.86 (9H, m), 1.47 (9H, s), 2.80 (1H, t, J = 11.7 Hz), 3.46-3.49 (2H, m), 3.92 (2H, s), 3.98-4.11 (3H, m), 5.10 (2H, brs), 7.26-7.33 (5H, m);
MS (FAB) m/z: 392 [M+H]⁺, 390, 336, 25.8, 256, 91.

### (166c) (3-{1-[(benzyloxy)carbonyl]piperidin-3-yl}propoxy)acetic acid

A methanol/water (2:1) (15 mL) suspension of benzyl 3-[3-(2-tert-butoxy-2-oxoethoxy)propyl]piperidine-1-carboxylate (1.80 g, 4.60 mmol) which was produced in Example 166 (166b) was blended with lithium hydroxide and the mixture was stirred at room temperature for 17 hours. The reaction liquid was concentrated and the residue was blended with water (100 mL) and methylene chloride (50 mL) and partitioned. The obtained aqueous layer was blended with 1N hydrochloric acid (30 mL) and extracted with methylene chloride (100 mL) three times. The organic layer were combined and the solvent was evaporated under reduced pressure after drying over sodium sulfate and a crude product (1.54 g) of the title compound was obtained.
Yellow liquid
IR (film) νₘₐₓ 3065, 2937, 1760, 1698, 1440, 1263, 1137, 699 cm⁻¹;
¹H NMR(CDCl₃, 400 MHz) δ 1.03-1.17 (1H, m), 1.18-1.54 (4H, m), 1.56-1.74 (3H, m), 1.76-1.89 (1H, m), 2.47-2.65 (1H, m), 2.78-2.95 (1H, m), 3.44-3.61 (2H, m), 3.90-4.11 (2H, m), 4.08 (2H, s), 5.12 (2H, s), 7.27-7.40 (5H, m);
MS (FAB) m/z: 336 [M+H⁺], 273, 242, 226, 180.

### (166d) benzyl 3-[3-(2-hydroxyethoxy)propyl]piperidine-1-carboxylate

The reaction was performed following a method described in Example 159 (159c) using (3-{1-[(benzyloxy)carbonyl]piperidin-3-yl}propoxy)acetic acid which was produced in Example 166 (166c) and the title compound was obtained. Colourless liquid
IR (film) νₘₐₓ 3457, 2935, 1698, 1434, 1362, 1237, 1119, 764, 699 cm⁻¹;
¹H NMR(CDCl₃, 400 MHz) δ 1.02-1.16 (1H, m), 1.18-1.36 (2H, m), 1.37-1.53 (2H, m), 1.54-1.72 (3H, m), 1.80-1.90 (1H, m), 2.40-2.65 (1H, m), 2.74-2.88 (1H, m), 3.34-3.56 (4H, m), 3.68-3.76 (2H, m), 3.89-4.18 (2H, m), 5.12 (2H, s), 7.27-7.43 (5H, m);
MS (FAB) m/z: 322 [M+H⁺], 278, 242, 226, 186.

### (166e) 2-(3-piperidin-3-ylpropoxy)ethanol

The reaction was performed following a method described in Example 159 (159d) using benzyl 3-[3-(2-hydroxyethoxy)propyl]piperidine-1-carboxylate which was produced in Example 166 (166d) and the title compound was obtained.
Yellow liquid
IR (film) νₘₐₓ 3296, 2930, 1450, 1112, 893 cm⁻¹;
¹H NMR(CDCl₃, 500 MHz) δ 0.96-1.08 (1H, m), 1.16-1.29 (2H, m), 1.39-1.50 (2H, m), 1.53-1.68 (3H, m), 1.73-1.87 (1H, m), 2.23 (1H, t, J = 10.3 Hz), 2.53 (1H, dt, J = 2.9, 12.2 Hz), 3.00 (1H, d, J = 12.2 Hz), 3.06 (1H, d, J = 11.7 Hz), 3.43-3.50 (2H, m), 3.51-3.55 (2H, m), 3.73 (2H, t, J = 4.9 Hz);
MS (FAB) m/z: 188 [M+H]⁺, 126, 96, 44.

### (166f) 4-{3-[3-(2-hydroxyethoxy)propyl]piperidin-1-yl}-2-thioxo-1,2-dihydropyridine-3-carbonitrile

The reaction was performed following a method described in Example 121 (121c) using 2-(3-piperidin-3-ylpropoxy)ethanol which was produced in Example 166 (166e) and the title compound was obtained.
Brown powder
Mp 112-116°C;
IR (KBr) νₘₐₓ 3526, 3117, 2940, 2210, 1625, 1547, 1461, 1313, 1249, 1128, 953, 800 cm⁻¹;
¹H NMR(CDCl₃, 500 MHz) δ 1.17-1.46 (3H, m), 1.56-1.77 (4H, m), 1.83-1.90 (1H, m), 1.94-2.02 (1H, m), 2.15 (1H, brs), 2.85 (1H, dd, J = 10.5, 13.2 Hz), 3.16 (1H, dt,
J = 2.9, 13.2 Hz), 3.40 (2H, t, J = 6.4 Hz), 3.55 (2H, t, J = 4.4 Hz), 3.74 (2H, t, J = 4.4 Hz), 4.07-4.18 (2H, m), 6.27 (1H, d, J = 7.3 Hz), 7.33 (1H, d, J = 7.3 Hz), 12.15 (1H, brs);
MS (FAB) m/z: 322 [M+H]⁺, 273, 242, 226, 180.

### (166 g) 3-amino-4-{3-[3-(2-hydroxyethoxy)propyl]piperidin-1-yl}thieno[2,3-b]pyridine-2-carboxamide

The reaction was performed following a method described in Example 5 (5c) using 4-{3-[3-(2-hydroxyethoxy)propyl]piperidin-1-yl}-2-thioxo-1,2-dihydropyridine-3-carbonitrile which was produced in Example 166 (166f) and the title compound was obtained.
A pale yellow foam
IR (KBr) νₘₐₓ 3326, 2933, 1649, 1580, 1502, 1448, 1373, 1248, 1114, 1057, 960 cm⁻¹;
¹H NMR(CDCl₃, 400 MHz) δ 0.96-1.14 (1H, m), 1.27-1.41 (2H, m), 1.57-1.83 (4H, m), 1.84-2.03 (3H, m), 2.24-2.36 (1H, m), 2.58-2.71 (1H, m), 3.34-3.45 (2H, m), 3.48 (2H, t, J = 6.7 Hz), 3.51-3.55 (2H, m), 3.70-3.75 (2H, m), 5.27 (2H, brs), 6.86 (1H, d, J = 5.5 Hz), 6.98 (2H, brs), 8.44 (1H, d, J = 5.5 Hz);
MS (FAB) m/z: 379 [M+H⁺], 362, 258, 242, 226, 165;
Anal. Calcd for C₁₈H₂₆N₄O₃S·0.50H₂O: C, 55.79; H, 7.02; N, 14.46; S, 8.27. Found: C, 55.56; H, 6.87; N, 14.31, S, 8.24.

### (Example 167) 3-amino-4-(3-{3-[2-(dimethylamino)-2-oxoethoxy]propyl}piperidin-1-yl)thieno[2,3-b]pyridine-2-carboxamide (Exemplified Compound No. 1-157) (167a) benzyl 3-{3-[2-(dimethylamino)-2-oxoethoxy]propyl}piperidine-1-carboxylate

The reaction was performed following a method described in Example 116 (116a) using (3-{1-[(benzyloxy)carbonyl]piperidin-3-yl}propoxy)acetic acid which was produced in Example 166 (166c) and the title compound was obtained. Colourless liquid
IR (film) νₘₐₓ 2935, 1699, 1433, 1261, 1112 cm⁻¹;
¹H NMR(CDCl₃, 400 MHz) δ 1.06-1.86 (9H, m), 2.81 (1H, dt, J = 3.0, 11.7 Hz), 2.95-3.00 (1H, m), 2.95 (3H, s), 3.00 (3H, s), 3.48 (2H, t, J = 6.3 Hz), 3.99-4.15 (2H, m), 4.12 (2H, s), 5.12 (2H, brs), 7.35-7.36 (5H, m);
MS (FAB) m/z: 363 [M+H]⁺, 319, 273, 255, 227, 165, 91, 63.

### (167b) N,N-dimethyl-2-(3-piperidin-3-ylpropoxy)acetamide

The reaction was performed following a method described in Example 162 (162b) using benzyl 3-{3-[2-(dimethylamino)-2-oxoethoxy]propyl}piperidine-1-carboxylate which was produced in Example 167 (167a) and the title compound was obtained.
Pale yellow liquid
IR (film) νₘₐₓ 3432, 2932, 1650, 1415, 1271, 1114 cm⁻¹;
¹H NMR(CDCl₃, 500 MHz) δ 0.99-1.84 (9H, m), 2.25 (1H, t, J = 11.7 Hz), 2.54 (1H, dt, J = 2.9, 11.7 Hz), 2.96-3.07 (2H, m), 2.96 (3H, s), 3.01 (3H, s), 3.48 (2H, t, J = 6.4 Hz), 4.12 (2H, s);
MS (FAB) m/z: 229 [M+H]⁺, 165, 63.

### (167c) 2-{3-[1-(3-cyano-2-thioxo-1,2-dihydropyridin-4-yl)piperidin-3-yl]propoxy}-N,N-dimethylacetamide

The reaction was performed following a method described in Example 121 (121c) using N,N-dimethyl-2-(3-piperidin-3-ylpropoxy)acetamide which was produced in Example 167 (167b) and the title compound was obtained.
Yellow powder
Mp 186-187°C;
IR (KBr) νₘₐₓ 2935, 2205, 1619, 1514, 1302, 1244, 1114, 781 cm⁻¹;
¹H NMR(CDCl₃, 500 MHz) δ 1.19-1.99 (9H, m), 2.84 (1H, dd, J = 10.7, 13.2 Hz), 2.96 (3H, s), 3.00 (3H, s), 3.17 (1H, dt, J = 2.9, 13.2 Hz), 3.52 (2H, t, J = 6.4 Hz), 4.10-4.16 (2H, m), 4.14 (2H, s), 6.28 (1H, d, J = 7.3 Hz), 7.28 (1H, d, J = 7.3 Hz), 11.45 (1H, brs);
MS (FAB) m/z: 363 [M+H]⁺, 347, 273, 246, 219, 165;
Anal. Calcd for C₁₈H₂₆N₄O₂S·0.04H₂O: C, 59.52; H, 7.24; N, 15.43; S, 8.83. Found: C, 59.28; H, 7.09; N, 15.42; S, 8.76.

### (167d) 3-amino-4-(3-{3-[2-(dimethylamino)-2-oxoethoxy)propyl}piperidin-1-yl)thieno[2,3-b]pyridine-2-carboxamide

The reaction was performed following a method described in Example 5 (5c) using 2- {3-[1-(3-cyano-2-thioxo-1,2-dihydropyridin-4-yl)piperidin-3-yl]propoxy}-N,N-dimethylacetamide which was produced in Example 167 (167c) and the title compound was obtained.
White foam
IR (KBr) νₘₐₓ 3437, 3324, 2933, 1646, 1579, 1500, 1371, 1112, 960 cm⁻¹;
¹H NMR(CDCl₃, 500 MHz) δ 0.99-1.07 (1H, m), 1.83-1.87 (2H, m), 1.63-1.81 (4H, m), 1.85-1.90 (1H, m), 1.95-2.00 (1H, m), 2.27-2.83 (1H, m), 2.62 (1H, t, J = 10.7 Hz), 2.95 (3H, s), 3.01 (3H, s), 3.39 (2H, brd, J = 9.8 Hz), 3.51 (2H, t, J = 6.4 Hz), 4.13 (2H, s), 5.27 (2H, brs), 6.88 (1H, d, J = 5.4 Hz), 7.01 (1H, brs), 8.46 (1H, d, J = 5.4 Hz);
HRMS m/z calcd for C₂₀H₃₀O₃N₅S 420.2069, found 420.2058;
MS (ESI) m/z: 420 [M+H]⁺, 403;
Anal. Calcd for C₂₀H₂₉N₅O₃S·0.86H₂O: C, 55.22; H, 7.12; N, 16.10; S, 7.37. Found: C, 55.21; H, 6.93; N, 16.11; S, 7.10.

### (Example 168) 3-amino-4(3-{3-[2-(dimethylamino)ethoxy]propyl}piperidin-1-yl)thieno[2,3-b]pyridine-2-carboxamide hydrochloride (Exemplified Compound No. 1-143)

### (168a) N,N-dimethyl-2-(3-piperidin-3-ylpropoxy)ethanamine

A tetrahydrofuran (20 ml) solution of N,N-dimethyl-2-(3-piperidin-3-ylpropoxy)acetamide (974 mg, 4.3 mmol) which was produced in Example 167 (167b) was added dropwise to a tetrahydrofuran (5 ml) suspension of lithium aluminum hydride (490 mg, 12.9 mmol) at 0°C and the mixture was stirred at room temperature for one hour. The reaction liquid was blended with tetrahydrofuran (25 ml) and 1N sodium hydroxide (2.5 ml) at 0°C and dried over sodium sulfate after stirring for 30 minutes. Insolubles were filtered by Celite, and the filtrate was concentrated under reduced pressure and a crude title compound was obtained (900 mg, yield 98%).
Colourless liquid
IR (film) νₘₐₓ 2934, 2854, 1466, 1271, 1118, 666 cm⁻¹;
¹H NMR(CDCl₃, 400 MHz) δ 0.97-1.85 (9H, m), 2.26 (6H, s), 2.19-2.26 (1H, m), 2.49 (2H, t, J = 5.9 Hz), 2.47-2.54 (1H, m), 3.00 (2H, t, J = 13.7 Hz), 3.40 (2H, t, J = 7.0 Hz), 3.50 (2H, t, J = 5.9 Hz);
MS (EI) m/z: 213 [M-H]⁺, 205, 182, 170, 144, 142, 126, 124, 96, 84, 71, 58, 41, 40.

### (168b) 3-amino-4(3-{3-[2-(dimethylamino)ethoxy]propyl}piperidin-1-yl)thieno[2,3-b]pyridine-2-carboxamide hydrochloride

The reaction was performed following a method described in Example 133 (133b) using N,N-dimethyl-2-(3-piperidin-3-ylpropoxy)ethanamine which was produced in Example 168 (168a) to obtain 3-amino-4(3-{3-[2-(dimethylamino)ethoxy]propyl} piperidin-1-yl)thieno [2,3-b]pyridine-2-carboxamide and the title compound was obtained by mixing it with 4N hydrochloric acid - ethyl acetate solution and concentrating the mixture.
Yellow foam
IR (KBr) νₘₐₓ 3319, 3166, 2942, 1650, 1609, 1480, 1387, 1259, 1115, 753 cm⁻¹;
¹H NNR(CD₃OD, 500 MHz) δ 1.31-1.97 (9H, m), 2.91 (6H, s), 2.86-2.94 (1H, s), 3.31-3.36 (3H, m), 3.53-3.55 (2H, m), 3.74-3.76 (2H, m), 3.93-3.96 (2H, m), 7.25 (1H, d, J = 6.8 Hz), 7.37 (1H, d, J = 6.8 Hz);
HRMS m/z calcd for C₂₀H₃₂O₂N₅S 406.2277, found 406.2280;
MS (ESI) m/z: 406 [M+H]⁺, 363;
Anal. Calcd for C₂₀H₃₁N₅O₂S·2.6HCl.2H₂O: C, 44.58; H, 7.05; N, 13.00; Cl, 17.55. Found: C, 44.22; H, 7.94; N, 12.52; Cl, 17.68.

### (Example 169) 3-amino-4-(3-{3-[3-(dimethylamino)propoxy]propyl}piperidin-1-yl)thieno[2,3-b]pyridine-2-carboxamide (Exemplified Compound No. 1-146) (169a) benzyl 3-[3-(2-cyanoethoxy)propyl]piperidine-1-carboxylate

The reaction was performed following a method described in Example 156 (156a) using benzyl 3-(3-hydroxypropyl)piperidine-1-carboxylate which was produced in Example 166 (166a) and the title compound was synthesized. Yield 73%.
Colourless oil
IR (film) νₘₐₓ 2937, 2860, 2251, 1699, 1432, 1261, 1237 cm⁻¹;
¹H NMR(CDCl₃, 500 MHz) δ 1.03-1.14 (1H, m), 1.18-1.34 (2H, m), 1.37-1.51 (2H, m), 1.56-1.70 (3H, m), 1.80-1.88 (1H, m), 2.42-2.62 (3H, m), 2.78-2.87 (1H, m), 3.40-3.51 (2H, m), 3.57-3.67 (2H, m), 3.91-4.15 (2H, m), 5.12 (2H, br.s), 7.29-7.38 (5H, m);
MS (FAB) m/z: 331 [M+H]⁺, 223, 195.

### (169b) benzyl 3-[3-(3-aminopropyl)propyl]piperidine-1-carboxylate

The reaction was performed following a method described in Example 157 (157a) using benzyl 3-[3-(2-cyanoethoxy)propyl]piperidine-1-carboxylate which was produced in Example 169 (169a) and the title compound was obtained (quantitative). Pale brown oil
IR (film) νₘₐₓ 3376, 2936, 2858, 1699, 1432 cm⁻¹;
¹H NMR(CDCl₃, 400 MHz) δ 0.96-1.72 (8H, m), 1.77-1.98 (3H, m), 2.38-2.56 (1H, m), 2.72-2.88 (1H, m), 2.94-3.12 (1H, m), 3.28-3.60 (5H, m), 3.86-4.12 (2H, m), 5.10 (2H, br.s), 7.20-7.38 (5H, m);
MS (FAB) m/z: 335 [M+H]⁺, 316, 273.

### (169c) benzyl 3-{3-[3-(dimethylamino)propoxy]propyl}piperidine-1-carboxylate

The reaction was performed following a method described in Example 157 (157b) using benzyl 3-[3-(3-aminopropyl)propyl]piperidine-1-carboxylate which was produced in Example 169 (169b) and the title compound was obtained. Yield 94%. Colourless oil
IR (film) νₘₐₓ 3287, 2935, 2854, 2764, 1620, 1558, 1464 cm⁻¹;
¹H NMR(CDCl₃, 400 MHz) δ 0.99-1.90 (11H, m), 2.22 (6H, s), 2.33 (2H, t, J = 7.4 Hz), 2.38-2.61 (1H, m), 2.74-2.86 (1H, m), 3.31-3.48 (4H, m), 3.92-4.17 (2H, m), 5.12 (2H, br.s), 7.25-7.41 (5H, m);
MS (FAB) m/z: 363 [M+H]⁺, 349, 273, 227.

### (169d) N,N-dimethyl-3-(3-piperidin-3-ylpropoxy)propan-1-amine

The reaction was performed following a method described in Example 128 (128d) using benzyl 3-{3-[3-(dimethylamino)propoxy]propyl}piperidine-1-carboxylate which was produced in Example 169 (169c) and the title compound was obtained. Yield 94%.
Colourless oil
IR (film) νₘₐₓ 3287, 2935, 2854, 2764, 1620, 1558, 1464 cm⁻¹;
¹H NMR(CDCl₃, 400 MHz) δ 0.95-1.07 (1H, m), 1.12-1.28 (2H, m), 1.33-1.50 (2H, m), 1.51-1.67 (2H, m), 1.68-1.87 (4H, m), 2.15-2.26 (1H, m), 2.23 (6H, s), 2.29-2.37 (2H, m), 2.52 (1H, dt, J = 11.7, 2.7 Hz), 2.95-3.08 (2H, m), 3.39 (2H, t, J = 6.6 Hz), 3.44 (2H, t, J = 6.6 Hz);
MS (EI) m/z: 228 M⁺, 219, 184.

### (169e) 3-amino-4-(3-{3-[3-(dimethylamino)propoxy]propyl}piperidin-1-yl)thieno[2,3-b]pyridine-2-carboxamide

The reaction was performed following a method described in Example 133 (133b) using N,N-dimethyl-3-(3-piperidin-3-ylpropoxy)propan-1-amine which was produced in Example 169 (169d) and the title compound was obtained. Yield 15%. Brown amorphous
IR (film) νₘₐₓ 3439, 3321, 3172, 2936, 2855, 2816, 1652, 1580, 1500 cm⁻¹;
¹H NMR(CDCl₃; 400 MHz) δ 0.97-1.11 (1H, m), 1.27-1.38 (2H, m), 1.54-1.68 (1H, m), 1.69-1.83 (4H, m), 1.84-1.92 (1H, m), 1.93-2.02 (1H, m), 2.22 (6H, s), 2.26-2.37 (3H, m), 2.57-2.67 (1H, m), 3.35-3.47 (6H, m), 5.33 (2H, br.s), 6.87 (1H, d, J = 5.4 Hz), 7.02 (2H, br.s), 8.46 (1H, d, J = 5.4 Hz);
MS (FAB) m/z: 420 [M+H]⁺, 377, 335, 273;
Anal. Calcd for C₂₁H₃₃N₅O₂S·0.5H₂O: C, 58.85; H, 8.00; N, 16.34, S, 7.48. Found: C, 59.17; H, 7.64; N, 16.12, S, 7.12.

### (Example 170) 3-amino-4-[2-(methoxymethyl)morpholin-4-yl]thieno[2,3-b]pyridine-2-carboxamide (Exemplified Compound No. 1-190)

### (170a) 4-[2-(methoxymethyl)morpholin-4-yl]-2-thioxo-1,2-dihydropyridine-3-carbonitrile

A reaction similar to that in Example 121 (121c) was performed using 2-(methoxymethyl) morpholine which can be obtained by using 2-(methoxymethyl)morpholine hydrochloride (J. Med. Chem., 37, 2791, (1994)) with treatment with base and the title compound was obtained.
Pale yellow powder
Mp 200-202°C;
IR (KBr) νₘₐₓ 3122, 2895, 2208, 1621, 1545, 1514, 1445, 1303, 1251, 1099, 1011, 783, 424 cm⁻¹;
¹H NMR(DMSO-d₆, 400 MHz) δ 3.08 (1H, dd, J = 2.7, 13.7 Hz), 3.23-3.28 (1H, m), 3.27 (3H, s), 3.37 (1H, dd, J = 4.3, 10.2 Hz), 3.42 (1H, dd, J = 5.1, 10.2 Hz), 3.56 (1H, dt, J = 2.0, 11.4 Hz), 3.64-3.68 (1H, m), 3.90-4.01 (3H, m), 6.49 (1H, d, J = 7.8 Hz), 7.50 (1H, d, J = 7.8 Hz), 12.73 (1H, brs);
MS (FAB) m/z: 266 [M+H]⁺, 265, 232, 220, 214, 180, 65, 63, 51, 39, 31, 23.

### (170b) 3-amino-4-[2-(methoxymethyl)morpholin-4-yl]thieno[2,3-b]pyridine-2-carboxamide

The reaction was performed following a method described in Example 5 (5c) using 4-[2-(methoxymethyl)morpholin-4-yl]-2-thioxo-1,2-dihydropyridine-3-carbonitrile which was produced in Example 170 (170a) and the title compound was obtained.
White powder
Mp 109-112°C;
IR (KBr) νₘₐₓ 3420, 3319, 3171, 2885, 1651, 1580, 1500, 1371, 1245, 1099, 1003, 825, 743 cm⁻¹_{;}
¹H NMR((DMSO-d₆, 400 MHz) δ 2.56-2.65 (1H, m), 2.80-2.88 (1H, m), 3.20 (1H, d, J = 12.5 Hz), 3.26 (3H, s), 3.29-3.31 (1H, m), 3.35 (1H, dd, J = 5.5, 10.2 Hz), 3.41 (1H, dd, J = 5.5, 10.6 Hz), 3.83-4.00 (3H, m), 6.92 (2H, brs), 7.02 (1H, d, J = 5.5 Hz), 7.12 (2H, brs), 8.43 (1H, d, J = 5.5 Hz);
HRMS m/z calcd for C₁₄H₁₉O₃N₄S 323.1178, found 323.1178;
MS (ESI) m/z: 323 [M+H]⁺, 306;
Anal. Calcd for C₁₄H₁₈N₄O₃S·0.44H₂O: C, 50.91; H, 5.76; N, 16.96; S, 9.71. Found: C, 51.03; H, 5.53; N, 17.35; S, 9.32.

### (Example 171) 3-amino-4-{3-[(2-morpholin-4-yl-2-oxoethoxy)methyl]piperidin-1-yl}thieno[2,3-b]pyridine-2-carboxamide (Exemplified Compound No. 1-229)

### (171a) benzyl 3-[(2-morpholin-4-yl-2-oxoethoxy)methyl]piperidine-1-carboxylate

The reaction was performed following a method described in Example 163 (163a) using morpholine in place of diethylamine and the title compound was obtained.
Colourless liquid
IR (film) νₘₐₓ 3507, 1698, 1434, 1360, 1236, 1115 cm⁻¹;
¹H NMR(CDCl₃, 500 MHz) δ 1.18-1.30 (1H, m), 1.41-1.57 (1H, m), 1.62-1.72 (1H, m), 1.75-1.90 (2H, m), 2.66-2.79 (1H, m), 2.84-2.96 (1H, m), 3.30-3.74 (10H, m), 3.91-4.16 (4H, m), 5.12 (2H, s), 7.27-7.40 (5H, m);
MS (FAB) m/z: 377 [M+H⁺], 333, 269, 241, 230, 186.

### (171b) 4-[(piperidin-3-ylmethoxy)acetyl]morpholine

The reaction was performed following a method described in Example 163 (163b) using benzyl 3-[(2-morpholin-4-yl-2-oxo ethoxy)methyl]piperidine-1-carboxylate which was produced in Example 171 (171 a) and the title compound was obtained.
Pale yellow liquid
IR (film) νₘₐₓ 3320, 2924, 1650, 1441, 1274, 1115 cm⁻¹;
¹H NMR(CDCl₃, 400 MHz) δ 1.05-1.23 (1H, m), 1.39-1.55 (1H, m), 1.60-1.86 (3H, m), 2.37 (1H, dd, J = 10.1, 11.7 Hz), 2.55 (1H, dt, J = 2.7, 11.7 Hz), 3.00 (1H, brd, J = 12.1 Hz), 3.12 (1H, brd, J = 12.1 Hz), 3.30-3.35 (2H, m), 3.50-3.56 (2H, m), 3.57-3.63 (2H, m), 3.64-3.71 (4H, m), 4.11 (2H, s);
MS (FAB) m/z: 243 [M+H]⁺, 165, 96.

### (171c) 4-{3-[(2-morpholin-4-yl-2-oxoethoxy)methyl]piperidin-1-yl}-2-thioxo-1,2-dihydropyridine-3-carbonitrile

The reaction was performed in a similar method as described in Example 163 (163c) using 4-[(piperidin-3-ylmethoxy)acetyl]morpholine which was produced in Example 171 (171b) and the title compound was obtained.
Yellow powder
Mp 172-177°C;
IR (KBr) νₘₐₓ 3195, 2926, 2205, 1619, 1516, 1446, 1301, 1241 , 115, 781 cm⁻¹;
¹H NMR(CDCl₃, 500 MHz) δ 1.33-1.44 (1H, m), 1.65-1.77 (1H, m), 1.84-1.93 (2H, m), 2.03-2.14 (1H, m), 3.10 (1H, dd, J = 10.3, 13.2 Hz), 3.27 (1H, brt, J = 11.2 Hz), 3.41 (1H, t, J = 8.8 Hz), 3.45-3.53 (3H, m), 3.58-3.65 (2H, m), 3.66-3.73 (4H, m), 4.09 (1H, d, J = 13.2 Hz), 4.14-4.22 (3H, m), 6.32 (1H, d, J = 7.3 Hz), 7.29 (1H, d, J = 7.3 Hz), 11.74 (1H, brs);
MS (FAB) m/z: 377 [M+H]⁺, 345, 262, 232, 230;
Anal. Calcd for C₁₈H₂₄N₄O₃S: C, 57.43; H, 6.43; N, 14.88; S, 8.52. Found: C, 57.18; H, 6.47; N, 14.84, S, 8.44.

### (171d) 3-amino-4-{3-[(2-morpholin-4-yl-2-oxoethoxy)methyl]piperidin-1-yl}thieno[2,3-b]pyridine-2-carboxamide

The reaction was performed in a similar method as described in Example 5 (5c) using 4-{3-[(2-morpholin-4-yl-2-oxoethoxy)methyl]piperidin-1-yl}-2-thioxo-1,2-dihydropyridine-3-carbonitrile which was produced in Example 171 (171c) and the title compound was obtained.
Pale yellow powder
Mp 163-165°C;
IR (KBr) νₘₐₓ 3451, 3156, 2931, 1649, 1579, 1501, 1366, 1116 cm⁻¹;
¹H NMR(CDCl₃, 500 MHz) δ 1.09-1.23 (1H, m), 1.73-1.97 (3H, m), 2.10-2.20 (1H, m), 2.40-2.52 (1H, m), 2.57-2.68 (1H, m), 3.33-3.74 (12H, m), 4.14 (2H, d, J = 3.9 Hz), 5.29 (2H, brs), 6.89 (1H, d, J = 5.4 Hz), 6.99 (2H, brs), 8.47 (1H, d, J = 5.4 Hz); MS (FAB) m/z: 434 [M+H⁺], 417, 391, 320, 272, 244, 202, 165;
Anal. Calcd for C₂₀H₂₇N₅O₄S: C, 55.41; H, 6.28; N, 16.15; S, 7.40. Found: C, 55.07; H, 6.30; N, 16.10, S, 7.37.

### (Preparation Examples)

### (Preparation Example 1) Powder

Powdered pharmaceutical composition can be obtained by mixing 5 g of a compound of Example 94, 895 g of lactose and 100 g of corn starch in a blender. (Preparation Example 2) Granule

After mixing 5 g of a compound of Example 74, 865 g of lactose and 100 g of low substituted hydroxypropyl cellulose, 300 g of 10% hydroxypropyl cellulose aqueous solution is added and blended. Granules can be obtained by extruding this with an extrusion granulating machine followed by drying.

### (Preparation Example 3) Tablet

Tablets can be obtained by mixing 5 g of a compound of Example 66, 90 g of lactose, 34 g of corn starch, 20 g of crystalline cellulose and 1 g of magnesium stearate in a blender and punching with a tabletting machine.

### (Test Examples)

### (Test Example 1) Osteoblast differentiation test

ST2 cells (available from Institute of Physical and Chemical Research) which are stroma cells derived from mouse marrow were used. In this test, α-MEM medium (available from GIBCO BRL Cat. No. 10370-021) with which 10% (v/v) inactivated fetal bovine serum (available from Hyclone Company, FBS) and 1% (v/v) Penicillin-Streptomycin, Liquid (available from GIBCO BRL Cat. No. 15140-122) are mixed (hereinbelow abbreviated as 10% -FBS-α-MEM) was used. Every culture in this test was conducted in a CO₂ incubator (37°C, 95% humidity, 5% CO₂).

The cells mentioned above were harvested with 2 mL of 0.25% trypsin solution (available from GIBCO BRL Cat. No. 15050-065) and after they were dispersed by adding 10 mL of 10% -FBS-αMEM, the cells were collected by centrifugal separation (25°C, 800 rpm, 5 minutes). A cell suspension containing 40,000 cells/mL was prepared from the collected cells using 10% -FBS-α-MEM.

The cell suspension was dispensed to the wells of a 96 well microplate (Falcon company) in an amount of 100 µL per well so as to achieve 4,000 cells/well and cultured for 24 hours. Compounds were dispensed to the wells in final concentrations of 0.01, 0.03, 0.1, 0.3 µg/ml except the following control group. DMSO was dispensed to the wells of the control group in a final concentration of 0.1% (v/v). Alkaline phosphatase (ALP) activity was measured for each group after culturing for four days.

The measurement of ALP activity was performed as follows. That is, after the whole medium was removed from each well of the culturing plate, each well was washed twice by dispensing and removing 100 µL of Dulbecco's phosphate buffer (available from GIBCO BRL Cat. No. 14190-144). Cell lysing solution containing 10 mM MgCl₂ and 2%(v/v) TritonX-100 (Sigma company) was prepared and 50 µL/well of the cell lysing solution was dispensed and the mixture was stirred at room temperature for five minutes. An ALP substrate solution containing 50 mM of diethanolamine (Wako Pure Chemical Industries, Ltd. Cat.No.099-03112) and 20 mM of p-nitrophenyl phosphite (Wako pure Chemical Industries, Ltd. Cat. No. 147-02343) was prepared and 50 µL/well of the ALP substrate solution was dispensed and after allowing to stand at room temperature for 10 minutes, absorbance was measured using a microplate reader (Bio-rad company). Alkaline phosphatase activity increase rate (%) in the group blended with test compounds was calculated assuming the observed value of the control group of each plate to be 100% and evaluated as the osteoblast differentiation promoting activity.

In this test, the compounds of Examples 14, 16, 17, 24, 29, 57, 58, 59, 64, 65, 66, 68, 72, 74, 76, 77, 83, 84, 93, 94, 96, 97, 98, 99, 101 and 106 exhibited an alkaline phosphatase activity increase rate of more than 150% at 0.03 µg/mL.

### (Test Example 2) Osteoclast formation suppression test

18 day-old ICR mice were purchased from Japan SLC, Inc. and used in the following experiments. The mice were sacrificed by cervical dislocation and the right and left femurs and tibias were extracted. After the tissue around the extracted femurs and tibias was removed, they were finely minced with scissors. 10 mL of 15%-FBS-α MEM was added to the minced femurs and tibias and the supernatant was collected after agitation for one minute and filtering with a cell strainer (Becton Dickinson company). A cell suspension containing 500,000 cells/mL was prepared with 15%-FBS-α MEM. The cell suspension was dispensed to the wells of a 96 well microplate (Falcon company) in an amount of 100 µL per well so as to achieve 50,000 cells/well and cultured for 24 hours. Active form vitamin D3 (Sigma company, Cat.No.D1530) was dispensed to the wells in final concentrations of 20 nM. Compounds were dispensed to the wells in final concentrations of 0.01, 0.03, 0.1, 0.3 µg/ml except the following control group. DMSO was dispensed to the wells of the control group in a final concentration of 0.1 % (v/v). Tartrate-resistant acid phosphatase (TRAP) activity was measured for each group after culturing for five days.

The measurement of TRAP activity was performed as follows. That is, after the whole medium was removed from each well of the culturing plate, each well was washed twice by dispensing and removing 100 µL of Dulbecco's phosphate buffer (available from GIBCO BRL Cat. No. 14190-144). After retaining with a mixture of acetone-ethanol (1:1) for one minute, the retention liquid was removed and the wells stained at 37°C with Leukocyte acid phosphatase kit (Sigma company,

Cat.No.387-A) for 30 minutes. The staining solution was removed, then 100 µL of 10% sodium dodecylsulfate (Wako Pure Chemical Industries, Ltd. Cat. No. 191-07145) was dispensed and the mixture stirred for five minutes, and then absorbance was measured using a microplate reader (Bio-rad company). TRAP activity decrease rate (%) in the group blended with test compounds was calculated assuming the observed value of the control group of each plate to be 100% and evaluated as the osteoclast formation suppression activity.

In this test, the compounds of Examples 57, 65, 66, 72, 83, 94, 97, 99, 101 and 106 exhibited an excellent osteoclast formation suppression effect.

### (Test Example 3) Effect on bone mineral density

8 week-old female F344 rats were purchased from Charles River Laboratories and used in the following experiment. After ketamine (12.5 mg/ml)/xylazine (2.5 mg/ml) liquid mixture was intraperitoneally injected in an amount of 0.25 ml/100 g for anesthesia, ovariectomy or a sham operation was performed. From the next day of the operation, 0.5% carboxymethyl cellulose sodium salt solution (Wako Pure Chemical Industries, Ltd. Cat.No.039-01335) in which a test compound was suspended was orally administered once per day and six days a week. Six weeks after the administration started, the animals were painlessly killed by removing the whole blood from the aorta abdominalis under ketamine xylazine anesthesia and the right and left femurs were extracted.

After soft tissue was removed from the extracted femurs, the bone density was measured by DXA device DCS-600R (Aloka Co., Ltd.). The bone density was determined for the whole femur as well as three evenly divided parts, i.e., proximal, midshaft and distal end.

In this test, the compound of Example 57 significantly increased the bone density at 10 mg/kg.

### (Test Example 4) Effect on fracture healing

12 week-old female F344 rats were purchased from Charles River Laboratories and used in the following experiment. An operation for fracturing bone was performed following a method of Li et al. (J. Bone Miner. Res 1999, 14: 966-979) under ketamine xylazine anesthesia. From the next day of the operation, 0.5% carboxymethyl cellulose sodium salt solution (Wako Pure Chemical Industries, Ltd. Cat.No.039-01335) in which a test compound was suspended was orally administered once per day and six days a week. 39 days after the administration started, the animals were painlessly killed by removing the whole blood from the aorta abdominalis under ketamine xylazine anesthesia and the femurs were extracted.

After soft tissue was removed from the extracted femurs, they were measured by bone strength measuring apparatus MZ-500D (Malto Co., Ltd.). 3-point bending tests were performed and determined by maximum load.

In this test, the compound of Example 57 significantly improved fracture healing.

### Industrial Applicability

Since the compound having a general formula (I) of the present invention or pharmacologically acceptable salt thereof has effects for promoting osteogenesis, suppressing bone resorption and/or improving bone density, it is useful as a pharmaceutical composition [particularly a pharmaceutical composition for prevention or treatment of osteopathy [for example, osteoporosis (for example, postmenopausal osteoporosis, senile osteoporosis or secondary osteoporosis caused by the use of steroids or immunosuppressants), osteopenia or bone destruction associated with rheumatoid arthritis, Paget's disease of bone, bone fracture or dysostosis due to dwarfism] or osteoarthritis}.

## Claims

1. A compound having the following general formula (I) 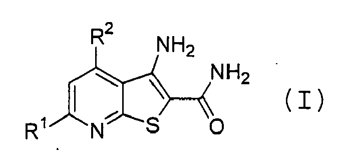 [wherein
R¹ represents a hydrogen atom, a cyclopropyl group or a C₁-C₆ alkyl group,
R² represents R^{a}S-, R^{a}O-, R^{a}NH-, R^{a}(R^{b})N- or a group 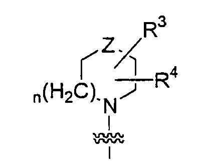 wherein R^{a} and R^{b} are the same or different and independently represent a C₁-C₆ alkyl group which may be substituted with one or more groups selected from Substituent Group α and Substituent Group γ; a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms,
R³ and R⁴ are the same or different and independently represent a hydrogen atom; a group selected from Substituent Group α, Substituent Group β and Substituent Group γ; a C₁-C₆ alkyl group substituted with one or more groups selected from Substituent Group γ; or a C₁-C₆ alkoxy group substituted with one or more groups selected from Substituent Group γ,
or when R³ and R⁴ are bonded to adjacent carbon atoms, R³ and R⁴ together with the carbon atoms to which they are bonded may form a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms,
Z represents a single bond; a double bond; an oxygen atom; a sulfur atom; sulfinyl; sulfonyl; or a group having the formula R⁵N<;
R⁵ represents a hydrogen atom; a C₁-C₆ alkyl group which may be substituted with one or more groups selected from Substituent Group α and Substituent Group γ; a
C₂-C₆ alkenyl group which may be substituted with one or more groups selected from Substituent Group α and Substituent Group γ; a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; a 5- to 7-membered heteroaryl group which may be substituted with one or more groups selected from Substituent Group α, Substituent Group β and Substituent Group γ and which contains 1 to 3 sulfur, oxygen and/or nitrogen atoms; a formyl group; a C₂-C₇ alkylcarbonyl group which may be substituted with one or more groups selected from Substituent Group α and Substituent Group γ; a 5- to 7-membered heterocyclylcarbonyl group which may be substituted with one or more groups selected from Substituent Group α, Substituent Group β and Substituent Group γ and which contains 1 to 3 sulfur, oxygen and/or nitrogen atoms; a C₇-C₁₁ arylcarbonyl group which may be substituted with one or more groups selected from Substituent Group α, Substituent Group β and Substituent Group γ; a 5- to 7-membered heteroarylcarbonyl group which may be substituted with one or more groups selected from Substituent Group α, Substituent Group β and Substituent Group γ and which contains 1 to 3 sulfur, oxygen and/or nitrogen atoms; a C₁-C₆ alkylsulfonyl group which may be substituted with one or more groups selected from Substituent Group α and Substituent Group γ; a C₆-C₁₀ arylsulfonyl group which may be substituted with one or more groups selected from Substituent Group α, Substituent Group β and Substituent Group γ; a 5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms; a C₂-C₇ alkoxycarbonyl group which may be substituted with one or more groups selected from Substituent Group α and Substituent Group γ; a C₇-C₁₁ aryloxycarbonyl group which may be substituted with one or more groups selected from Substituent Group α, Substituent Group β and Substituent Group γ; or a group having the formula R^{c}(R ^{d})N-CO- (wherein R^{c} and R^{d} are the same or different and independently represent a hydrogen atom or a C₁-C₆ alkyl group which may be substituted with one or more groups selected from Substituent Group α and Substituent Group γ),
n represents an integer of 1 to 4,
Substituent Group α represents the group consisting of a halogen atom; a nitro group; a cyano group; a hydroxy group; a group having the formula R⁶-CO-, the formula R^{e}(R^{f})N-, the formula R^{e}(R^{f})N-CO- or the formula R^{e}(R^{f})N-SO₂- (wherein R⁶ represents a hydrogen atom, a C₁-C₆ alkyl group, a C₁-C₆ halogenated alkyl group, a C₃-C₈ cycloalkyl group, a hydroxy group, a C₁-C₆ alkoxy group, a C₆-C₁₀ aryl group or a C₆-C₁₀ aryloxy group and R^{e} and R^{f} are the same or different and independently represent a hydrogen atom; a C₁-C₆ alkyl group; a C₁-C₆ alkoxy group; a C₆-C₁₀ aryl group; a 5- to 7-membered heteroaryl group which contains 1 to 3 sulfur, oxygen and/or nitrogen atoms; a formyl group; a C₂-C₇ alkylcarbonyl group; a C₂-C₇ alkoxycarbonyl group; a C₇-C₁₁ arylcarbonyl group; a 5- to 7-membered heteroarylcarbonyl group which contains 1 to 3 sulfur, oxygen and/or nitrogen atoms; a C₁-C₆ alkylsulfonyl group; a C₆-C₁₀ arylsulfonyl group; or a 5- to 7-membered heteroarylsulfonyl group which contains 1 to 3 sulfur, oxygen and/or nitrogen atoms, or alternatively R^{e} and R^{f} together with the nitrogen atom to which they are bonded form a 4- to 7-membered heterocyclyl group which contains 1 to 3 sulfur, oxygen and/or nitrogen atoms (wherein the heterocyclyl group may have 1 or 2 substituent groups selected from a hydroxy group and a methyl group)); a hydroxyimino group; a C₁-C₆ alkoxyimino group; a C₁-C₆ alkoxy group; a C₃-C₈ cycloalkyloxy group; a C₁-C₆ halogenated alkoxy group; a C₁-C₆ alkylthio group; a C₁-C₆ alkylsulfinyl group; and a C₁-C₆ alkylsulfonyl group, Substituent Group β represents the group consisting of a C₁-C₆ alkyl group which may be substituted with one or more groups selected from Substituent Group α; and a C₁-C₆ alkyl group substituted with a 5- to 7-membered heterocyclyl group which may be substituted with one or more groups selected from Substituent Group α and a C₁-C₆ alkyl group and which contains 1 to 3 sulfur, oxygen and/or nitrogen atoms, and Substituent Group γ represents the group consisting of a C₁-C₆ alkoxy group substituted with one or more groups selected from Substituent Group α; a C₁-C₆ alkylthio group substituted with one or more groups selected from Substituent Group α; a C₃-C₈ cycloalkyl group which may be substituted with one or more groups selected from Substituent Group α and Substituent Group β; a 5- to 7-membered heterocyclyl group which may be substituted with one or more groups selected from Substituent Group α and Substituent Group β and which contains 1 to 3 sulfur, oxygen and/or nitrogen atoms; a C₆-C₁₀ aryl group which may be substituted with one or more groups selected from Substituent Group α and Substituent Group β; a 5-to 7-membered heteroaryl group which may be substituted with one or more groups selected from Substituent Group α and Substituent Group β and which contains 1 to 3 sulfur, oxygen and/or nitrogen atoms; a C₃-C₈ cycloalkyloxy group which may be substituted with one or more groups selected from Substituent Group α and
Substituent Group β; a 5- to 7-membered heterocyclyloxy group which may be substituted with one or more groups selected from Substituent Group α and Substituent Group β and which contains 1 to 3 sulfur, oxygen and/or nitrogen atoms; a C₆-C₁₀ aryloxy group which may be substituted with one or more groups selected from Substituent Group α and Substituent Group β; a 5- to 7-membered heteroaryloxy group which may be substituted with one or more groups selected from Substituent Group α and Substituent Group β and which contains 1 to 3 sulfur, oxygen and/or nitrogen atoms; and a C₆-C₁₀ aryl - C₁-C₆ alkoxy group in which the aryl moiety may be substituted with one or more groups selected from Substituent Group α and Substituent Group β] or a pharmacologically acceptable salt thereof.

2. The compound or pharmacologically acceptable salt thereof according to claim 1 wherein R¹ is a hydrogen atom, a cyclopropyl group or a C₁-C₄ alkyl group.

3. The compound or pharmacologically acceptable salt thereof according to claim 1 wherein R¹ is a hydrogen atom, methyl, ethyl, propyl or cyclopropyl.

4. The compound or pharmacologically acceptable salt thereof according to claim 1 wherein R¹ is a hydrogen atom or methyl.

5. The compound or pharmacologically acceptable salt thereof according to any one of claims 1 to 4 wherein R² is a group R^{a}(R^{b})N-, and R^{a} and R^{b} are the same or different and independently represent a C₁-C₆ alkyl group which may be substituted with one or more groups selected from Substituent Group α and Substituent Group γ.

6. The compound or pharmacologically acceptable salt thereof according to claim 5 wherein R^{a} is a C₁-C₆ alkyl group which may be substituted with one group selected from Substituent Group α and Substituent Group γ, R^{b} is a C₁-C₆ alkyl group, and Substituent Group α is the group consisting of a C₁-C₆ alkoxy group, and Substituent Group γ is the group consisting of a C₁-C₆ alkoxy group substituted with one or more groups selected from Substituent Group α; a C₆-C₁₀ aryloxy group which may be substituted with one or more groups selected from Substituent Group α and Substituent Group β; and a 5- to 7-membered heteroaryloxy group which may be substituted with one or more groups selected from Substituent Group α and Substituent Group β and which contains 1 to 3 sulfur, oxygen and/or nitrogen atoms.

7. The compound or pharmacologically acceptable salt thereof according to any one of claims 1 to 4 wherein R² is a group 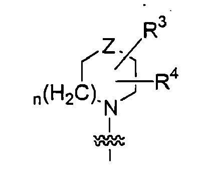 wherein R⁴ is a hydrogen atom or together with R³ forms a C₃-C₈ cycloalkyl group which may be substituted with one or more groups selected from Substituent Group α, Substituent Group β and Substituent Group γ; a 5- to 7-membered heterocyclyl group which may be substituted with one or more groups selected from Substituent Group α, Substituent Group β and Substituent Group γ and which contains 1 to 3 sulfur, oxygen and/or nitrogen atoms; a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms.

8. The compound or pharmacologically acceptable salt thereof according to claim 7 wherein R² is a group 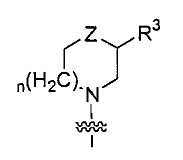 wherein Z represents a single bond, an oxygen atom, a sulfur atom or a group having the formula R⁵N<,
R⁵ represents a C₆-C₁₀ aryl group which may be substituted with one or more groups selected from Substituent Group α, Substituent Group β and Substituent Group γ; a 5- to 7-membered heteroaryl group which may be substituted with one or more groups selected from Substituent Group α, Substituent Group β and Substituent Group γ and which contains 1 to 3 sulfur, oxygen and/or nitrogen atoms; a formyl group; a C₂-C₇ alkylcarbonyl group which may be substituted with one or more groups selected from Substituent Group α and Substituent Group γ; a C₁-C₆ alkylsulfonyl group which may be substituted with one or more groups selected from Substituent Group α and Substituent Group γ; a C₆-C₁₀ arylsulfonyl group which may be substituted with one or more groups selected from Substituent Group α, Substituent Group β and Substituent Group γ; a 5- to 7-membered heteroarylsulfonyl group which may be substituted with one or more groups selected from Substituent Group α, Substituent Group β and Substituent Group γ and which contains 1 to 3 sulfur, oxygen and/or nitrogen atoms; a C₂-C₇ alkoxycarbonyl group which may be substituted with one or more groups selected from Substituent Group α and Substituent Group γ; or a group having the formula R^{c}(R^{d})N-CO-, and n is an integer of 1 to 3.

9. The compound or pharmacologically acceptable salt thereof according to claim 8 wherein R³ is a C₁-C₆ alkoxy group; a C₁-C₆ alkyl group which may be substituted with one or more groups selected from Substituent Group α; a C₁-C₆ alkoxy group substituted with one or more groups selected from Substituent Group α; a C₆-C₁₀ aryloxy group which may be substituted with one or more groups selected from Substituent Group α, Substituent Group β and Substituent Group γ; a C₁-C₆ alkyl group substituted with one or more groups selected from Substituent Group γ; or a C₁-C₆ alkoxy group substituted with one or more groups selected from Substituent Group γ,
Z is a single bond, and n is 2.

10. The compound or pharmacologically acceptable salt thereof according to claim 8 wherein R³ is a hydrogen atom, Z is a sulfur atom, and n is 1.

11. The compound or pharmacologically acceptable salt thereof according to claim 8 wherein R³ is a hydrogen atom, Z is a group having the formula R⁵N<, R⁵ represents a C₆-C₁₀ aryl group which may be substituted with one or more groups selected from Substituent Group α, Substituent Group β and Substituent Group γ; or a 5- to 7-membered heteroaryl group which may be substituted with one or more groups selected from Substituent Group α, Substituent Group β and Substituent Group γ and which contains 1 to 3 sulfur, oxygen and/or nitrogen atoms: n is 2.

12. The compound or pharmacologically acceptable salt thereof according to claim 11 wherein the compound is selected from the following:
· 3-amino-4-[(3S)-3-(methoxymethyl)piperidin-1-yl]thieno[2,3-b]pyridine-2-carboxamide,
· 3-amino-4-[(3S)-3-(methoxymethyl)piperidin-1-yl]-6-methylthieno[2,3-b]pyridine-2-carboxamide,
· 3-amino-4-{3-[3-(2-hydroxyethoxy)propyl]piperidin-1-yl}thieno[2,3-b]pyridine-2-carboxamide,
· 3-amino-4-{(3S)-[(2-methoxyethoxy)methyl]piperidin-1-yl}thieno[2,3-b]pyridine-2-carboxamide,
· 3-amino-4-{(3S)-3-[(3-methoxypropoxy)methyl]piperidin-1-yl}thieno[2,3-b]pyridine-2-carboxamide,
· 3-amino-4-(3-{[2-(dimethylamino)-2-oxoethoxy]methyl}piperidin-1-yl)thieno[2,3-b]pyridine-2-carboxamide,
· 3-amino-4-(3- {3-[2-(dimethylamino)-2-oxoethoxy]propyl}piperidin-1-yl)thieno [2,3-b]pyridine-2-carboxamide,
· 4-[4-(4-acetylphenyl)-1,4-diazepan-1-yl]-3-aminothieno[2,3-b]pyridine-2-carboxamide,
· 3-amino-4-[4-(4-propionylphenyl)-1,4-diazepan-1-yl]thieno[2,3-b]pyridine-2-carboxamide,
· 3-amino-4-{4-[4-(dimethylamino)phenyl]-1,4-diazepan-1-yl}thieno[2,3-b]pyridine-2-carboxamide,
· 3-amino-4-(4-{4-[(dimethylamino)carbonyl]phenyl}-1,4-diazepan-1-yl)thieno[2,3-b]pyridine-2-carboxamide,
· 4-[4-(5-acetylpyridin-2-yl)-1,4-diazepan-1-yl]-3-aminothieno[2,3-b]pyridine-2-carboxamide,
· 3-amino-4-(4-{4-[(dimethylaimno)carbonyl]phenyl}-1,4-diazepan-1-yl)-6-methylthieno[2,3-b]pyridine-2-carboxamide,
· 3-amino-4- {4-[4-(2-methoxyethyl)phenyl]-1,4-diazepan-1-yl}thieno[2,3-b]pyridine-2-carboxamide,
· 3-amino-4-(4-{4-[2-(dimethylamino)-2-oxoethyl]phenyl}-1,4-diazepan-1-yl)thieno[2,3-b]pyridine-2-carboxamide,
· 3-amino-4-(4-{4-[3-(dimethylamino)-3-oxopropyl]phenyl}-1,4-diazepan-1-yl)thieno[2,3-b]pyridine-2-carboxamide,
· 3-amino-4-{4-[4-(azetidin-1-ylcarbonyl)phenyl]-1,4-diazepan-1-yl}thieno[2,3-b]pyridine-2-carboxamide,
· 3-amino-4-{4-[4-(morpholin-4-ylcarbonyl)phenyl]-1,4-diazepan-1-yl}thieno[2,3-b]pyridine-2-carboxamide,
· 3-amino-4-(4-{4-[2-(dimethylamino)ethyl]phenyl}-1,4-diazepan-1-yl)thieno[2,3-b]pyridine-2-carboxamide,
· 3-amino-4-{4-[4-(2-hydroxyethyl)phenyl]-1,4-diazepan-1-yl}thieno[2,3-b]pyridine-2-carboxamide,
· 3-amino-4-{4-[3-(2-hydroxyethyl)phenyl]-1,4-diazepan-1-yl}thieno[2,3-b]pyridine-2-carboxamide,
· 3-amino-4-{4-[4-(3-hydroxypropyl)phenyl]-1,4-diazepan-1-yl}thieno[2,3-b]pyridine-2-carboxamide,
· 3-amino-4-{4-[4-(1-hydroxyethyl)phenyl]-1,4-diazepan-1-yl}thieno[2,3-b]pyridine-2-carboxamide,
· 3-amino-4-{4-[4-(2-oxopropyl)phenyl]-1,4-diazepan-1-yl}thieno[2,3-b]pyridine-2-carboxamide,
· 3-amino-4-{4-[4-(N-hydroxyethaneimidoyl)phenyl]-1,4-diazepan-1-yl}thieno[2,3-b]pyridine-2-carboxamide,
· 3-amino-4-(4-{4-[(2-methyl-1,3-dioxolan-2-yl)methyl]phenyl}-1,4-diazepan-1-yl)thieno[2,3-b]pyridine-2-carboxamide,
· 3-amino-4-[4-(2-methyl-1-oxo-2,3-dihydro-1H-isoindol-5-yl)-1,4-diazepan-1-yl]thieno[2,3-b]pyridine-2-carboxamide,
· 3-amino-4-[4-(1,3-benzoxazol-6-yl)-1,4-diazepan-1-yl]thieno[2,3-b]pyridine-2-carboxamide,
· 3-amino-4-[4-(4-hydroxyimino-3,4-dihydro-2H-chromen-7-yl)-1,4-diazepan-1-yl]thieno[2,3-b]pyridine-2-carboxamide,
· 4-[4-(4-acetyl-1,3-thiazol-2-yl)-1,4-diazepan-1-yl]-3-aminothieno[2,3-b]pyridine-2-carboxamide,
· 4-[4-(5-acetylthiophen-2-yl)-1,4-diazepan-1-yl]-3-aminothieno[2,3-b]pyridine-2-carboxamide, and
· 3-amino-4-(4-{4-[(dimethylamino)carbonyl]-1,3-thiazol-2-yl}-1,4-diazepan-1-yl)thieno[2,3-b]pyridine-2-carboxamide.

13. A pharmaceutical composition which comprises the compound or pharmacologically acceptable salt thereof according to any one of claims 1 to 12 as an active ingredient.

14. The pharmaceutical composition according to claim 13 for prevention or treatment of osteopathy or osteoarthritis.

15. The pharmaceutical composition according to claim 14 wherein the osteopathy is osteoporosis, osteopenia or bone destruction associated with rheumatoid arthritis, Paget's disease of bone, bone fracture or dysostosis due to dwarfism.

16. The pharmaceutical composition according to claim 15 wherein the osteoporosis is postmenopausal osteoporosis, senile osteoporosis or secondary osteoporosis caused by the use of steroids or immunosuppressants.

17. Use of the compound or pharmacologically acceptable salt thereof according to any one of claims 1 to 12 as an active ingredient for preparation of a pharmaceutical composition.

18. The use according to claim 17 wherein the pharmaceutical composition is a pharmaceutical composition for prevention or treatment of osteopathy or osteoarthritis.

19. The use according to claim 18 wherein the osteopathy is osteoporosis, osteopenia or bone destruction associated with rheumatoid arthritis, Paget's disease of bone, bone fracture or dysostosis due to dwarfism.

20. The use according to claim 19 wherein the osteoporosis is postmenopausal osteoporosis, senile osteoporosis or secondary osteoporosis caused by the use of steroids or immunosuppressants.

21. A method for promoting osteogenesis, suppressing bone resorption and/or improving bone density comprising administering an effective amount of the compound or pharmacologically acceptable salt thereof according to any one of claims 1 to 12 to a mammal or a bird.

22. The method according to claim 21 for prevention or treatment of osteopathy or osteoarthritis.

23. The method according to claim 22 wherein the osteopathy is osteoporosis, osteopenia or bone destruction associated with rheumatoid arthritis, Paget's disease of bone, bone fracture or dysostosis due to dwarfism.

24. The method according to claim 23 wherein the osteoporosis is postmenopausal osteoporosis, senile osteoporosis or secondary osteoporosis caused by the use of steroids or immunosuppressants.

25. The method according to any one of claims 21 to 24 wherein the mammal is a human, horse, cow or pig and the bird is a chicken.

26. The method according to any one of claims 21 to 24 comprising administering an effective amount of the compound or pharmacologically acceptable salt thereof according to any one of claims 1 to 12 to a human.
